(19) Europäisches Patentamt
European Patent Office
Office européen des brevets

(11) **EP 4 736 955 A2**

(12) **EUROPEAN PATENT APPLICATION**

(43) Date of publication:
**06.05.2026 Bulletin 2026/19**

(21) Application number: **26151295.8**

(22) Date of filing: **23.10.2019**

(51) International Patent Classification (IPC):
***A61P 35/00*** *(2006.01)*

(52) Cooperative Patent Classification (CPC):
**A61P 35/00; A61K 47/68033; A61K 47/68035; A61K 47/6849; A61K 47/6851; A61K 47/6857; C07K 16/30;** A61K 2039/505; C07K 2317/24; C07K 2317/30; C07K 2317/33; C07K 2317/70; C07K 2317/92

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2018 US 201862751530 P**
**27.03.2019 US 201962824539 P**
**10.05.2019 US 201962846297 P**

(62) Document number(s) of the earlier application(s) in accordance with Art. 76 EPC:
**19876033.2 / 3 873 512**

(71) Applicants:
- **ImmunoGen, Inc.**
  **North Chicago, IL 60064 (US)**
- **Cytomx Therapeutics, Inc.**
  **South San Francisco, CA 94080 (US)**

(72) Inventors:
- **LIU, Yimao**
  **Acton, 01720 (US)**
- **HICKS, Stuart, W.**
  **North Andover, 01845 (US)**
- **GUIDI, Cynthia, J.**
  **Littleton, 01460 (US)**
- **KOHLI, Neeraj**
  **Arlington, 02474 (US)**
- **CHITTENDEN, Thomas**
  **Sudbury, 01776 (US)**
- **LAMBERT, John**
  **Cambridge, 02139 (US)**
- **PAIDHUNGAT, Madan, M.**
  **South San Francisco, 94080 (US)**
- **SAGERT, Jason, Gary**
  **South San Francisco, 94080 (US)**
- **TIPTON, Kimberly, Ann**
  **South San Francisco, 94080 (US)**
- **CHAN, Chanty, Mariategue**
  **South San Francisco, 94080 (US)**
- **FOX, Ellaine Anne, Mariano**
  **South San Francisco, 94080 (US)**

(74) Representative: **Carpmaels & Ransford LLP**
**One Southampton Row**
**London WC1B 5HA (GB)**

Remarks:
- The complete document including Reference Table(s) and the Sequence Listing(s) can be downloaded from the EPO website
- This application was filed on 12-01-2026 as a divisional application to the application mentioned under INID code 62.
- Claims filed after the date of receipt of the divisional application (Rule 68(4) EPC).

# (54) EPCAM ANTIBODIES, ACTIVATABLE ANTIBODIES, AND IMMUNOCONJUGATES, AND USES THEREOF

(57) The disclosure generally relates to antibodies and antibody fragments that specifically bind human EpCAM, EpCAM activatable antibodies, and immunoconjugates thereof, as well as, methods of making and using the antibodies, antibody fragments, activatable antibodies, and immunoconjugates, for the diagnosis and treatment of diseases such as cancer.

FIG. 1A

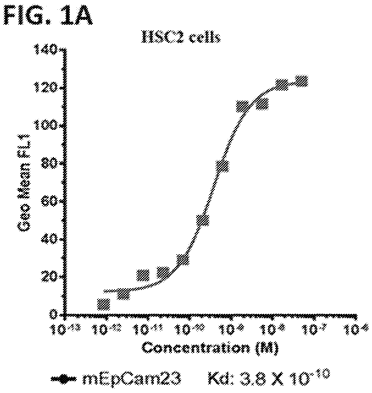


EP 4 736 955 A2

## Description

## BACKGROUND

The disclosure generally relates to antibodies and antibody fragments that specifically bind human EpCAM, EpCAM activatable antibodies, and immunoconjugates thereof, as well as, methods of making and using the antibodies, antibody fragments, activatable antibodies, and immunoconjugates, for the diagnosis and treatment of diseases such as cancer.

Epithelial cell adhesion molecule (EpCAM), is a type I trans-membrane glycoprotein comprising an extracellular domain, a transmembrane domain, and a single intracellular domain. EpCAM expression in human is epithelia-specific. The majority of epithelial cells express EpCAM, except squamous epithelium and some specific epithelium cell types, such as epidermal keratinocytes, hepatocytes, gastric parietal cells, and myoepithelial cells (Balzar et al., J. Mol. Med. 77:699-712 (1999); Momburg et al., Cancer Res 47:2883-2891 (1987)).

EpCAM is abundantly and homogeneously expressed on human carcinomas of different origins (Went et al., Br. J. Cancer 94:128-35 (2006); Herlyn et al., Proc Natl. Acad. Sci. USA 76:1438-1442 (1979); Went et al., Hum. Pathol. 35:122-128 (2004)). EpCAM is overexpressed in the vast majority of epithelial cancers, including for example, ovarian cancers, colon cancers, stomach cancers, prostate cancers, and lung cancers. In addition, EpCAM has been shown to be expressed on the majority of primary, metastatic, and disseminated NSCLC (non-small cell lung cancer cells) (Passlick, Int. J. Cancer 87:548-552 (2000)), on gastric and gastro-esophageal junction adenocarcinomas (Martin, J. Clin. Pathol. 52:701-704 (1999)) and in cell lines derived from colorectal, pancreatic carcinomas and breast carcinomas (Szala, Proc. Natl. Acad. Sci. USA 87:3542-3546 (1990), Packeisen, Hybridoma 18:37-40 (1999)). In another study, immunohistochemical analysis of 108 samples of secondary tumors has found that only 4% lacked EpCAM expression. EpCAM is overexpressed also in cancer-initiating or cancer stem cells isolated from colon, breast, pancreas and prostate carcinomas (O'Brien et al., Nature 445:106-110 (2007); Marhaba et al., Curr. Mol. Med. 8:784-804 (2008)).

Normal cells express EpCAM on the basolateral side of the epithelial membrane, whereas cancer cells heavily express EpCAM on the apical surface. Antibody-based therapeutics have been designed to exploit this characteristic of EpCAM expression, as normal cellular EpCAM is less prominent and less exposed, meaning healthy cells may not be as susceptible to binding by therapeutic anti-EpCAM antibodies.

A number of antibodies to EpCAM have been used in the clinic but failed for various reasons. The EpCAM antibodies tested take a number of formats, including naked antibodies, immunotoxins and bi- or tri-specific antibodies (Baeuerle, Br. J. Cancer, 96:417-423 (2007)). For example, adecatumumab (MT201), a naked anti-EpCAM antibody has been tested in clinical studies of treatment in colorectal, prostate and breast cancers. Safety issues facing the current anti-EpCAM antibody-based approaches include systemic intolerability and acute pancreatitis. Thus, although there have been several attempts to develop therapeutic antibodies to EpCAM, there is a significant need for the development of novel therapeutic EpCAM antibodies that overcome the shortcomings and limitations of the previously developed antibodies.

## BRIEF SUMMARY

The disclosure provides antibodies and antibody fragments that specifically bind human EpCAM, as well as EpCAM activatable antibodies, and immunoconjugates comprising the antibodies, antibody fragments and activatable antibodies. Polynucleotides comprising nucleic acid sequences encoding the EpCAM antibodies, EpCAM-binding antibody fragments and activatable antibodies are also provided, as are vectors comprising the polynucleotides, and cells comprising the polynucleotides and vectors. The disclosure also provides compositions such as pharmaceutical compositions comprising the EpCAM antibodies, EpCAM-binding antibody fragments and activatable antibodies. Methods of making and using the EpCAM antibodies, EpCAM-binding antibody fragments, activatable antibodies, and compositions are further provided. Such methods include using the antibodies, antibody fragments, activatable antibodies, immunoconjugates and other compositions to inhibit tumor growth, as well as, methods of making and using the antibodies, antibody fragments, activatable antibodies, immunoconjugates, and compositions for the diagnosis and treatment of diseases such as cancer.

In some embodiments, the disclosure provides:

[1] an EpCAM antibody or EpCAM-binding antibody fragment, wherein the antibody or antibody fragment comprises:

(a) a heavy chain CDR1 (VH-CDR1) comprising the sequence $X_1YX_3X_4H$, wherein $X_1$ is selected from N and S, $X_3$ is selected from Y, N, F, S, H, D, L, I, and W, and $X_4$ is selected from I and M (SEQ ID NO:5);
(b) a heavy chain CDR2 (VH-CDR2) comprising the sequence $WX_2X_3PGX_6VYIQYX_{12}X_{13}KFX_{17}G$, wherein $X_2$ is selected from I and F, $X_3$ is selected from Y and N, $X_6$ is selected from N and D, $X_{12}$ is selected from N and S, $X_{13}$ is selected from E and Q, and $X_{17}$ is selected from K and Q (SEQ ID NO:7);

(c) heavy chain CDR3 (VH-CDR3) comprising the sequence $X_1GX_3X_4FAY$, wherein $X_1$ is selected from D and E, $X_3$ is selected from P, A, S, Y, F, G, T, and V, and $X_4$ is selected from Y and W (SEQ ID NO:8);
(d) a light chain CDR1 (VL-CDR1) comprising the sequence $RSSX_4SLLHSX_{10}GX_{12}TY LX_{16}$, wherein $X_4$ is selected from R and K, $X_{10}$ is selected from N and D, $X_{12}$ is selected from F and I, and $X_{16}$ is selected from Y and S (SEQ ID NO:10);
(e) a light chain CDR2 (VL-CDR2) comprising the sequence QTSNLAS (SEQ ID NO:40); and
(f) a light chain CDR3 (VL-CDR3) comprising the sequence $X_1QX_3LELPX_8T$, wherein $X_1$ is selected from A, L, and Q, $X_3$ is selected from S. G, Y, and N, and $X_8$ is selected from N and W (SEQ ID NO:11);

[2] the antibody or antibody fragment of [1], wherein the antibody or antibody fragment comprises:

(a) a heavy chain CDR1 (VH-CDR1) comprising the sequence $NYX_3IH$, wherein $X_3$ is selected from Y, N, F, S, H, D, L, I, and W (SEQ ID NO:6);
(b) a heavy chain CDR2 (VH-CDR2) comprising the sequence $WX_2X_3PGX_6VYIQYX_{12}X_{13}KFX_{17}G$, wherein $X_2$ is selected from I and F, $X_3$ is selected from Y and N, $X_6$ is selected from N and D, $X_{12}$ is selected from N and S, $X_{13}$ is selected from E and Q. and $X_{17}$ is selected from K and Q (SEQ ID NO:7);
(c) heavy chain CDR3 (VH-CDR3) comprising the sequence $DGPX_4FAY$, wherein $X_4$ is selected from Y and W (SEQ ID NO:9);
(d) a light chain CDR1 (VL-CDR1) comprising the sequence $RSSX_4SLLHSX_{10}GX_{12}TYLX_{16}$, wherein $X_4$ is selected from R and K, $X_{10}$ is selected from N and D, $X_{12}$ is selected from F and I, and $X_{16}$ is selected from Y and S (SEQ ID NO:10);
(e) a light chain CDR2 (VL-CDR2) comprising the sequence QTSNLAS (SEQ ID NO:40): and
(f) a light chain CDR3 (VL-CDR3) comprising the sequence $AQX_3LELPNT$, wherein $X_3$ is selected from S, G, Y, and N (SEQ ID NO:12);

[3] the EpCAM antibody or EpCAM-binding antibody fragment of [1] or [2], wherein the antibody or antibody fragment comprises a VH-CDR1, VH-CDR2, VH-CDR3, VL-CDR1, VL-CDR2, and VL-CDR3 having the sequences selected from the group:

(a) SEQ ID NOs: 13-15, 42, 40, and 41, respectively;
(b) SEQ ID NOs: 13-15, and 39-41, respectively;
(c) SEQ ID NOs: 13, 26, 15, and 39-41, respectively; and
(d) SEQ ID NOs: 13, 24, 15, 42, 40, and 41, respectively;

[4] the EpCAM antibody or EpCAM-binding antibody fragment of any one of [1]-[3], wherein the antibody or antibody fragment binds with a KD of 3.0 nM or less to both human EpCAM and cynomolgus EpCAM;
[5] the EpCAM antibody or EpCAM-binding antibody fragment of [4], wherein the antibody or antibody fragment specifically binds to an epitope within the extracellular domain of human EpCAM having the amino acid sequence of SEQ ID NO:2;
[6] the antibody or antibody fragment of [5], wherein the antibody or antibody fragment comprises:

(a) a VH-CDR1 comprising the sequence NYYIH (SEQ ID NO:13);
(b) a VH-CDR2 comprising the sequence W1YPGNVYIQYNEKFKG (SEQ ID NO:14);
(c) a VH-CDR3 comprising the sequence DGPWFAY (SEQ ID NO:15);
(d) a VL-CDR1 comprising the sequence RSSRSLLHSDGFTYLY (SEQ ID NO:42);
(e) a VL-CDR2 comprising the sequence QTSNLAS (SEQ ID NO:40); and
(f) and a VL-CDR3 comprising the sequence AQNLELPNT (SEQ ID NO:41);

[7] the antibody or antibody fragment of [4], wherein the antibody or antibody fragment comprises:

(a) a VH-CDR1 comprising the sequence NYYIH (SEQ ID NO:13);
(b) a VH-CDR2 comprising the sequence WIYPGNVYIQYNEKFKG (SEQ ID NO:14);
(c) a VH-CDR3 comprising the sequence DGPWFAY (SEQ ID NO:15);
(d) a VL-CDR1 comprising the sequence RSSKSLLHSDGFTYLY (SEQ ID NO:39);
(e) a VL-CDR2 comprising the sequence QTSNLAS (SEQ ID NO:40); and
(f) a VL-CDR3 comprising the sequence AQNLELPNT (SEQ ID NO:41);

[8] the EpCAM antibody or EpCAM-binding antibody fragment of [3], wherein the antibody or antibody fragment

comprises a VH and a VL selected from:

(a) a VH having a sequence of SEQ ID NO:54, and a VL having a sequence of SEQ ID NO:89;
(b) a VH having a sequence of SEQ ID NO:54, and a VL having a sequence of SEQ ID NO:87;
(c) a VH having a sequence of SEQ ID NO:55, and a VL having a sequence of SEQ ID NO:87;
(d) a VH having a sequence of SEQ ID NO:56, and a VL having a sequence of SEQ ID NO:88;
(e) a VH having a sequence of SEQ ID NO:55, and a VL having a sequence of SEQ ID NO:89; and
(f) a VH having a sequence of SEQ ID NO:56, and a VL having a sequence of SEQ ID NO:89;

[9] the antibody or antibody fragment of [8], wherein the antibody or antibody fragment comprises a VH of SEQ ID NO:54 and a VL of SEQ ID NO:89;
[10] the antibody or antibody fragment of [8], wherein the antibody or antibody fragment comprises a VH of SEQ ID NO:54 and a VL of SEQ ID NO:87;
[11] the EpCAM antibody or EpCAM-binding antibody fragment of [8], wherein the antibody or antibody fragment comprises a heavy chain and a light chain selected from

(a) a HC having a sequence of SEQ ID NO:103, and a LC having a sequence of SEQ ID NO:140;
(b) a HC having a sequence of SEQ ID NO:103, and a LC having a sequence of SEQ ID NO:138;
(c) a HC having a sequence of SEQ ID NO:105, and a LC having a sequence of SEQ ID NO:139;
(d) a HC having a sequence of SEQ ID NO:106, and a LC having a sequence of SEQ ID NO:139;
(e) a HC having a sequence of SEQ ID NO:105, and a LC having a sequence of SEQ ID NO:140; and
(f) a HC having a sequence of SEQ ID NO:106, and a LC having a sequence of SEQ ID NO:140;

[12] the antibody or antibody fragment of [11], wherein the antibody or antibody fragment comprises a HC of SEQ ID NO:103 and a LC of SEQ ID NO:140;
[13] the antibody or antibody fragment of [11], wherein the antibody or antibody fragment comprises a HC of SEQ ID NO: 103 and a LC of SEQ ID NO: 138;
[14] the EpCAM antibody or EpCAM-binding antibody fragment of [1] or [2], wherein the antibody or antibody fragment comprises a VH-CDR1, VH-CDR2, VH-CDR3, VL-CDR1, VL-CDR2, and VL-CDR3 having the sequences selected from the group consisting of:

(a) SEQ ID NOs: 22, 14, 15, 42, 40, and 41, respectively;
(b) SEQ ID NOs: 13, 14, 33, 42, 40, and 41, respectively;
(c) SEQ ID NOs: 23, 14, 15, 42, 40, and 41, respectively; and
(d) SEQ ID NOs: 25, 14, 15, 42, 40, and 41, respectively;

[15] the antibody or antibody fragment of [14], wherein the antibody or antibody fragment comprises:

(a) a VH-CDR1 comprising the sequence NYHIH (SEQ ID NO:22);
(b) a VH-CDR2 comprising the sequence WIYPGNVYIQYNEKFKG (SEQ ID NO:14);
(c) a VH-CDR3 comprising the sequence DGPWFAY (SEQ ID NO:15);
(d) a VL-CDR1 comprising the sequence RSSRSLLHSDGFTYLY (SEQ ID NO:42);
(e) a VL-CDR2 comprising the sequence QTSNLAS (SEQ ID NO:40); and
(f) a VL-CDR3 comprising the sequence AQNLELPNT (SEQ ID NO:41);

[16] the antibody or antibody fragment of [14], wherein the antibody or antibody fragment comprises:

(a) a VH-CDR1 comprising the sequence NYYIH (SEQ ID NO:13);
(b) a VH-CDR2 comprising the sequence WIYPGNVYIQYNEKFKG (SEQ ID NO:14);
(c) a VH-CDR3 comprising the sequence DGYWFAY (SEQ ID NO:33);
(d) a VL-CDR1 comprising the sequence RSSRSLLHSDGFTYLY (SEQ ID NO:42);
(e) a VL-CDR2 comprising the sequence QTSNLAS (SEQ ID NO:40); and
(f) a VL-CDR3 comprising the sequence AQNLELPNT (SEQ ID NO:41);

[17] the EpCAM antibody or EpCAM-binding antibody fragment of [14], wherein the antibody or antibody fragment comprises a VH and a VL selected from:

(a) a VH having a sequence of SEQ ID NO:75, and a VL having a sequence of SEQ ID NO:89;

(b) a VH having a sequence of SEQ ID NO:77, and a VL having a sequence of SEQ ID NO:89;
(c) a VH having a sequence of SEQ ID NO:76, and a VL having a sequence of SEQ ID NO:89; and
(d) a VH having a sequence of SEQ ID NO:84, and a VL having a sequence of SEQ ID NO:89;

[18] the antibody or antibody fragment of [17], wherein the antibody or antibody fragment comprises a VH of SEQ ID NO: 75 and a VL of SEQ ID NO: 89;
[19] the antibody or antibody fragment of [17], wherein the antibody or antibody fragment comprises a VH of SEQ ID NO: 77 and a VL of SEQ ID NO: 89;
[20] the EpCAM antibody or EpCAM-binding antibody fragment of [17], wherein the antibody or antibody fragment comprises a heavy chain and a light chain selected from

(a) a HC having a sequence of SEQ ID NO:125. and a LC having a sequence of SEQ ID NO:140;
(b) a HC having a sequence of SEQ ID NO:127, and a LC having a sequence of SEQ ID NO: 140;
(c) a BHC having a sequence of SEQ ID NO:126, and a LC having a sequence of SEQ ID NO:140; and
(d) a HC having a sequence of SEQ ID NO:134, and a LC having a sequence of SEQ ID NO:140;

[21] the antibody or antibody fragment of [20], wherein the antibody or antibody fragment comprises a HC of SEQ ID NO: 125 and a LC of SEQ ID NO: 140;
[22] the antibody or antibody fragment of [20], wherein the antibody or antibody fragment comprises a HC of SEQ ID NO: 127 and a LC of SEQ ID NO: 140;
[23] the antibody or antibody fragment of any one of [1]-[22], wherein the antibody or antibody fragment is a murine, non-human mammal, chimeric, humanized, or human antibody;
[24] the antibody or antibody fragment of any one of [1]-[23], wherein the antibody is a full-length antibody;
[25] the full-length antibody of [24], wherein the antibody is human IgG1;
[26] the antibody or antibody fragment of any one of [1]-[23], wherein antibody fragment is an Fab, Fab', $F(ab')_2$, Fd, single chain Fv or scFv, disulfide linked $F_v$, V-NAR domain, IgNar, intrabody, $IgG\Delta CH_2$, minibody, $F(ab')_3$, tetrabody, triabody, diabody, single-domain antibody, DVD-Ig, Fcab, $mAb_2$, $(scFv)_2$, or scFv-Fc;
[27] an activatable antibody comprising the antibody or antibody fragment of any one of [1]-[26], wherein the activatable antibody further comprises

(a) a cleavable moiety coupled to the antibody or antibody fragment, wherein the cleavable moiety is a polypeptide that functions as a substrate for a protease; and
(b) a masking moiety coupled to the antibody or antibody fragment, wherein the masking moiety inhibits the binding of the antibody or antibody fragment to EpCAM when the activatable antibody is in an uncleaved state,

wherein the activatable antibody in the uncleaved state has the structural arrangement from N-terminus to C-terminus as follows: (masking moiety)-(cleavable moiety)-(antibody or antibody fragment) or (antibody or antibody fragment)-(cleavable moiety)-(masking moiety);
[28] an EpCAM activatable antibody comprising:

(a) an EpCAM antibody or EpCAM-binding antibody fragment comprising a VH-CDR1, VH-CDR2, VH-CDR3, VL-CDR1, VL-CDR2, and VL-CDR3 having the sequences of a member selected from the group:

(i) SEQ ID NOs: 13-15, 42, 40, and 41, respectively;
(ii) SEQ ID NOs: 13-15, and 39-41, respectively;
(iii) SEQ ID NOs: 13, 26, 15, and 39-41, respectively; and
(iv) SEQ ID NOs: 13, 24, 15, 42, 40, and 41, respectively;

(b) a masking moiety coupled to the EpCAM antibody or EpCAM-binding antibody fragment, wherein the masking moiety inhibits the binding of the antibody or antibody fragment to EpCAM when the activatable antibody is in an uncleaved state; and
(c) a cleavable moiety coupled to the EpCAM antibody or EpCAM-binding antibody fragment, wherein the cleavable moiety is a polypeptide that functions as a substrate for a protease;

wherein the activatable antibody in the uncleaved state has the structural arrangement from N-terminus to C-terminus as follows: (masking moiety)-(cleavable moiety)-(antibody or antibody fragment) or (antibody or antibody fragment)-(cleavable moiety)-(masking moiety);
[29] the EpCAM activatable antibody of [28], wherein the activatable antibody comprises an EpCAM antibody or

EpCAM-binding antibody fragment comprising a VH-CDR1, VH-CDR2, VH-CDR3, VL-CDR1, VL-CDR2, and VL-CDR3 having the sequences of SEQ ID NOs: 13-15, 42, 40, and 41, respectively;
[30] the EpCAM activatable antibody of [29], wherein the activatable antibody comprises an EpCAM antibody or EpCAM-binding antibody fragment comprising a VH having the sequence of SEQ ID NO: 54 and a VL having the sequence of SEQ ID NO: 89;
[31] the EpCAM activatable antibody of [30], wherein the activatable antibody comprises an EpCAM antibody or EpCAM-binding antibody fragment comprising a heavy chain having the sequence of SEQ ID NO: 103 and a LC having the sequence of SEQ ID NO: 140;
[32] the EpCAM activatable antibody of any one of [29]-[31], wherein the activatable antibody comprises a masking moiety having an amino acid sequence selected from SEQ ID NOs: 151-157;
[33] the EpCAM activatable antibody of [32], wherein the activatable antibody comprises a masking moiety having the amino acid sequence of SEQ ID NO: 155;
[34] the EpCAM activatable antibody of any one of [29]-[33], wherein the activatable antibody comprises a cleavable moiety comprising the amino acid sequence of SEQ ID NO:168 or 169;
[35] an EpCAM activatable antibody comprising:

(a) an EpCAM antibody or EpCAM-binding antibody fragment comprising a VH-CDR1, VH-CDR2, VH-CDR3, VL-CDR1, VL-CDR2, and VL-CDR3 having the sequences selected from the group consisting of:

(i) SEQ ID NOs: 22. 14, 15, 42, 40, and 41, respectively;
(ii) SEQ ID NOs: 13, 14, 33, 42, 40, and 41, respectively;
(iii) SEQ ID NOs: 23, 14, 15, 42, 40, and 41, respectively, and;
(iv) SEQ ID NOs: 25, 14, 15, 42, 40, and 41, respectively;

(b) a masking moiety coupled to the EpCAM antibody or EpCAM-binding antibody fragment, wherein the masking moiety inhibits the binding of the antibody or antibody fragment to EpCAM when the activatable antibody is in an uncleaved state; and

(c) a cleavable moiety coupled to the EpCAM antibody or EpCAM-binding antibody fragment, wherein the cleavable moiety is a polypeptide that functions as a substrate for a protease;

wherein the activatable antibody in the uncleaved state has the structural arrangement from N-terminus to C-terminus as follows: (masking moiety)-(cleavable moiety)-(antibody or antibody fragment) or (antibody or antibody fragment)-(cleavable moiety)-(masking moiety);
[36] the EpCAM activatable antibody of [35], wherein the activatable antibody comprises an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 22, 14, 15, 42, 40, and 41, respectively;
[37] the EpCAM activatable antibody of [36], wherein the activatable antibody comprises an EpCAM antibody or EpCAM-binding antibody fragment comprising a VH having the sequence of SEQ ID NO: 75 and a VL having the sequence of SEQ ID NO: 89;
[38] the EpCAM activatable antibody of [37], wherein the activatable antibody comprises an EpCAM antibody or EpCAM-binding antibody fragment comprising a heavy chain having the sequence of SEQ ID NO: 125 and a light chain having the sequence of SEQ ID NO: 140;
[39] the EpCAM activatable antibody of any one of [36]-[38], wherein the activatable antibody comprises a masking moiety having an amino acid sequence selected from SEQ ID NOs: 151-157 and 162-167;
[40] the EpCAM activatable antibody of [39], wherein the activatable antibody comprises a masking moiety having an amino acid sequence selected from SEQ ID NO: 162-167;
[41] the EpCAM activatable antibody of any one of [36]-[40], wherein the activatable antibody comprises a cleavable moiety comprising the amino acid sequence of SEQ ID NO:168 or 169;
[42] the EpCAM activatable antibody of [35], wherein the activatable antibody comprises an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 13, 14, 33, 42, 40, and 41, respectively;
[43] the EpCAM activatable antibody of [42], wherein the activatable antibody comprises an EpCAM antibody or EpCAM-binding antibody fragment comprising a VH having the sequence of SEQ ID NO: 77 and a VL having the sequence of SEQ ID NO: 89;
[44] the EpCAM activatable antibody of [43], wherein the activatable antibody comprises an EpCAM antibody or EpCAM-binding antibody fragment comprising a heavy chain having the sequence of SEQ ID NO: 127 and a light chain having the sequence of SEQ ID NO: 140;

[45] the EpCAM activatable antibody of any one of [42]-[44], wherein the activatable antibody comprises a masking moiety having an amino acid sequence selected from SEQ ID NOs: 151-161;
[46] the EpCAM activatable antibody of [45], wherein the activatable antibody comprises a masking moiety having an amino acid sequence selected from of SEQ ID NO: 158-161;
[47] the EpCAM activatable antibody of any one of [42]-[46], wherein the activatable antibody comprises a cleavable moiety comprising the amino acid sequence of SEQ ID NO:168 or 169;
[48] a cell producing the antibody or EpCAM-binding antibody fragment of any one of [1]-[26] or the activatable antibody of any one of [27]-[47];
[49] a method of producing an EpCAM antibody or EpCAM-binding antibody fragment, or an EpCAM activatable antibody, comprising:

(a) culturing the cell of [48]; and,
(b) isolating the EpCAM antibody, EpCAM-binding antibody fragment, or EpCAM activatable antibody from the cell or cell culture;

[50] the method of [49], wherein the cell is a eukaryotic cell;
[51] the method of [50], wherein the cell is a CHO cell;
[52] a diagnostic reagent comprising the antibody or antibody fragment of any one of [1]-[26] or the EpCAM activatable antibody of any one of [27]-[47];
[53] the diagnostic reagent of [52], wherein the antibody, antibody fragment, or activatable antibody is labeled;
[54] the diagnostic reagent of [53], wherein the label is selected from: a radiolabel, a fluorophore, a chromophore, an imaging agent and a metal ion;
[55] a polynucleotide or set of polynucleotides encoding the antibody or antibody fragment of any of [1]-[26] or the activatable antibody of any one of [27]-[47];
[56] a vector or set of vectors comprising the polynucleotide of [55];
[57] a host cell comprising the polynucleotide or set of polynucleotides of [55] or the vector of [56];
[58] an immunoconjugate represented by the following formula:

$$\mathrm{EpBA}-\left(-\mathrm{Cy}^{L1}\right)_{W_L},$$

wherein:

EpBA is an EpCAM antibody, EpCAM antibody fragment, or EpCAM activatable antibody according to any of [1]-[47], that is covalently linked to $Cy^{L1}$ through a lysine residue;

$W_L$ is an integer from 1 to 20; and
$Cy^{L1}$ is represented by the following formula:

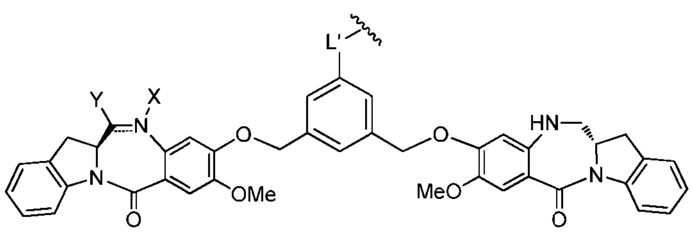
, or

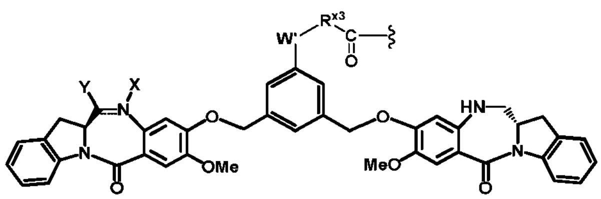

or a pharmaceutically acceptable salt thereof, wherein:

the double line $\overline{- -}$ between N and C represents a single bond or a double bond, provided that when it is a double bond, X is absent and Y is -H or a $(C_1-C_4)$alkyl; and when it is a single bond, X is -H or an amine protecting moiety, and Y is -OH or $-SO_3H$ or a pharmaceutically acceptable salt thereof;
W' is $-NR^{e'}$,
$R^{e'}$ is $-(CH_2-CH_2-O)_n-R^k$;
n is an integer from 2 to 6;
$R^k$ is -H or -Me;
$R^{x3}$ is a $(C_1-C_6)$alkyl;
L' is represented by the following formula:

$$-NR_5-P-C(=O)-(CR_aR_b)_m-C(=O)- \quad (B1');$$

or

$$-NR_5-P-C(=O)-(CR_aR_b)_m-S-Z^{s1}- \quad (B2');$$

$R_5$ is -H or a $(C_1-C_3)$alkyl;
P is an amino acid residue or a peptide containing between 2 to 20 amino acid residues;
$R_a$ and $R_b$, for each occurrence, are each independently -H, $(C_1-C_3)$alkyl, or a charged substituent or an ionizable group Q;
m is an integer from 1 to 6; and
$Z^{s1}$ is selected from any one of the following formulas:

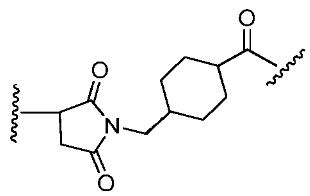 (b1); 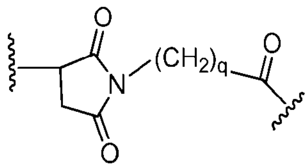 (b2); 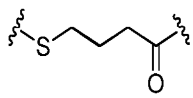 (b3);

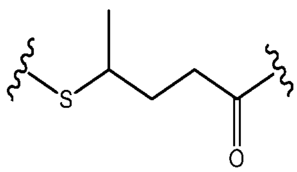 (b4); 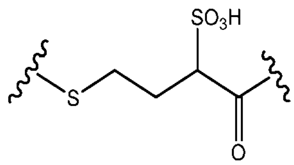 (b5),

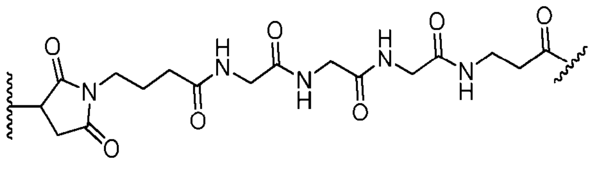 (b6),

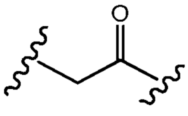 (b7); 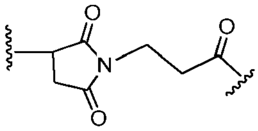 (b8); 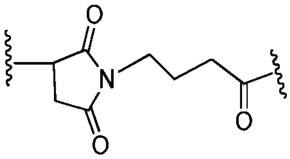 (b9); and

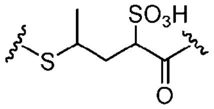 (b10),

wherein q is an integer from 1 to 5;

[59] the immunoconjugate of [58], wherein $R_a$ and $R_b$ are both H; and $R_5$ is H or Me;

[60] the immunoconjugate of [58] or [59], wherein P is a peptide containing 2 to 5 amino acid residues;

[61] the immunoconjugate of any one of [58]-[60], wherein P is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-$N^9$-tosyl-Arg, Phe-$N^9$-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:215), β-Ala-Leu-Ala-Leu (SEQ ID NO:216), Gly-Phe-Leu-Gly (SEQ ID NO:217), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, Met-Ala, Gln-Val, Asn-Ala, Gln-Phe and Gln-Ala. More specifically, P is Gly-Gly-Gly, Ala-Val, Ala-Ala, Ala-D-Ala, D-Ala-Ala, or D-Ala-D-Ala;

[62] the immunoconjugate of [61], wherein P is Gly-Gly-Gly, Ala-Val, Ala-Ala, Ala-D-Ala, D-Ala-Ala, or D-Ala-D-Ala;

[63] the immunoconjugate of any one of [58]-[62], wherein Q is -SO3H or a pharmaceutically acceptable salt thereof;

[64] the immunoconjugate of [58], wherein the immunoconjugate is represented by the following formula:

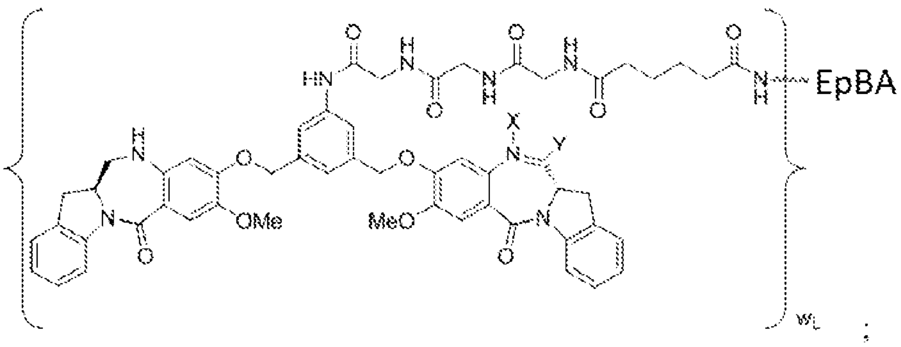
;

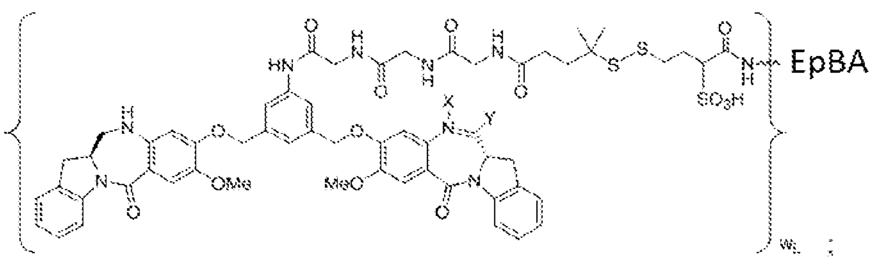
;

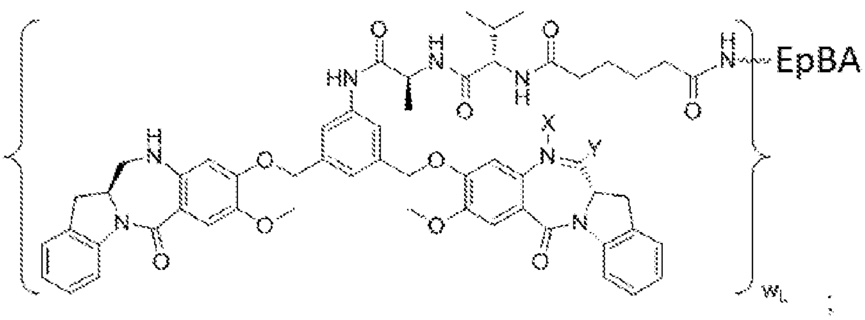
;

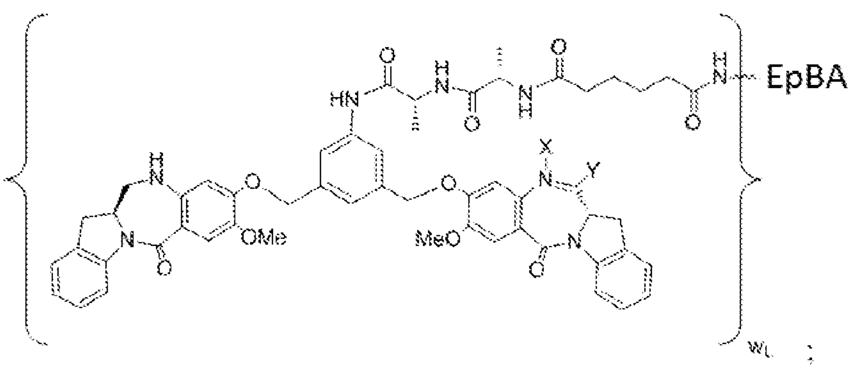
;

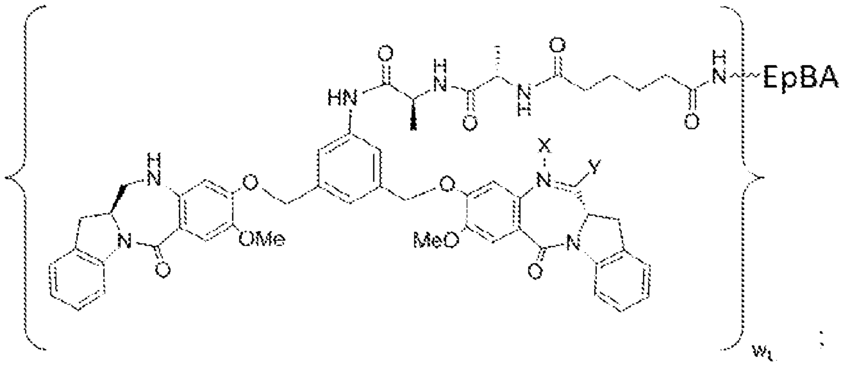

;

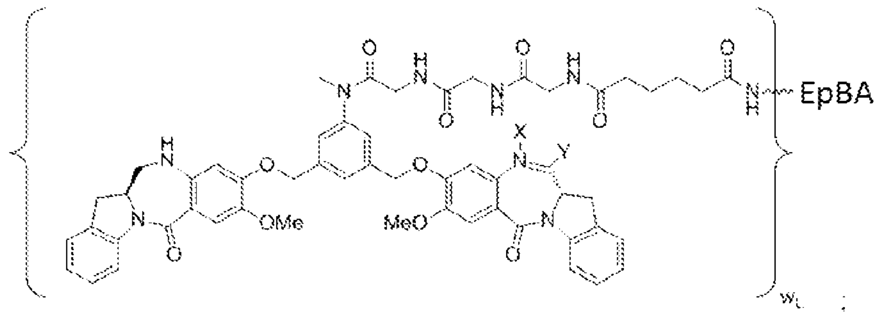

;

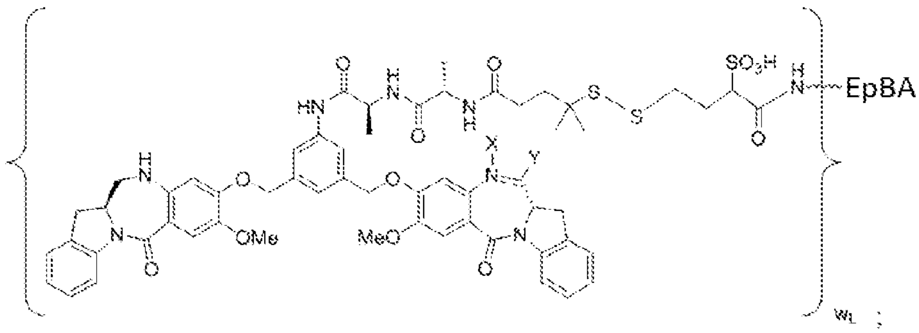

;

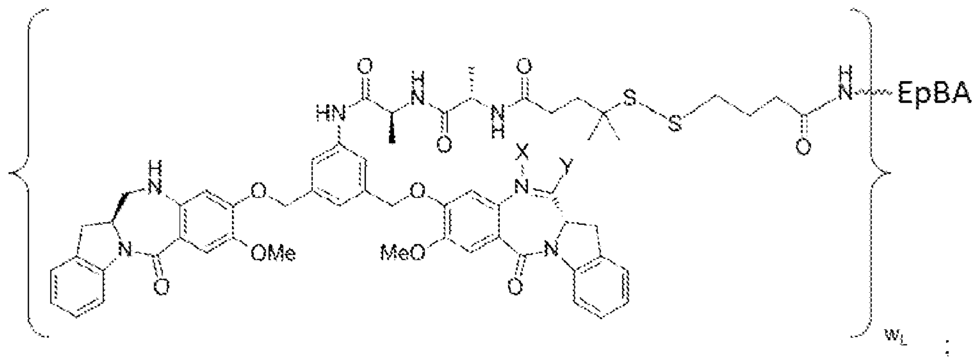

;

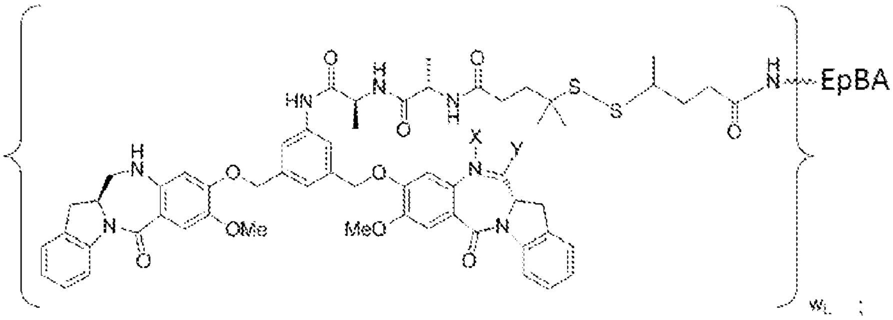
;

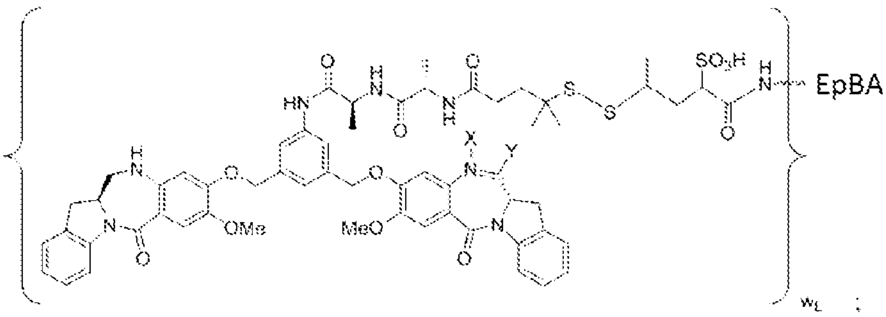
;

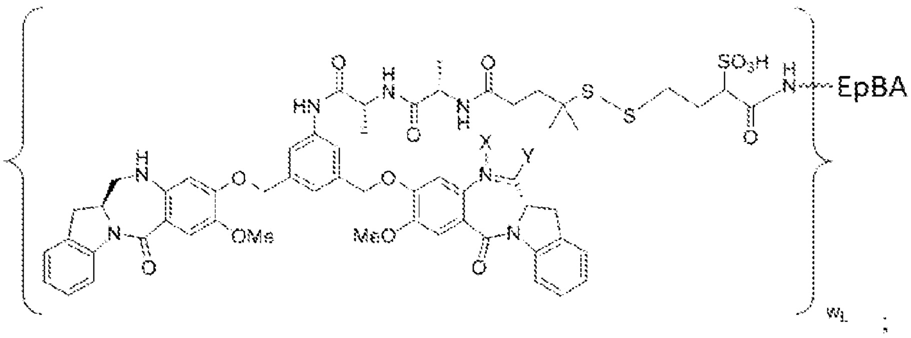
;

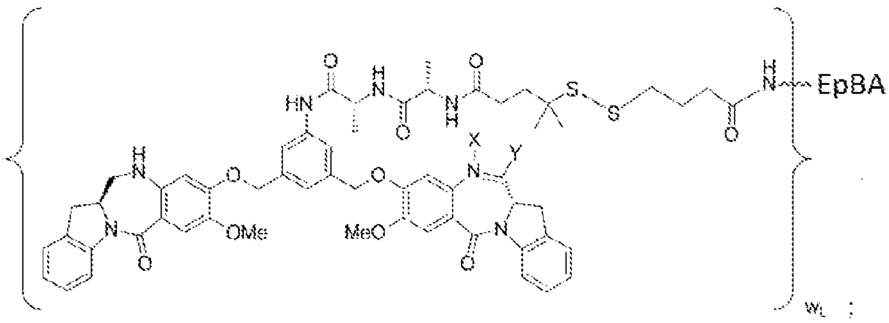
;

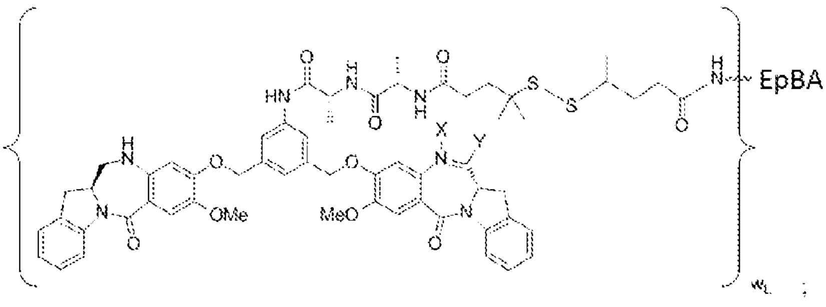
;

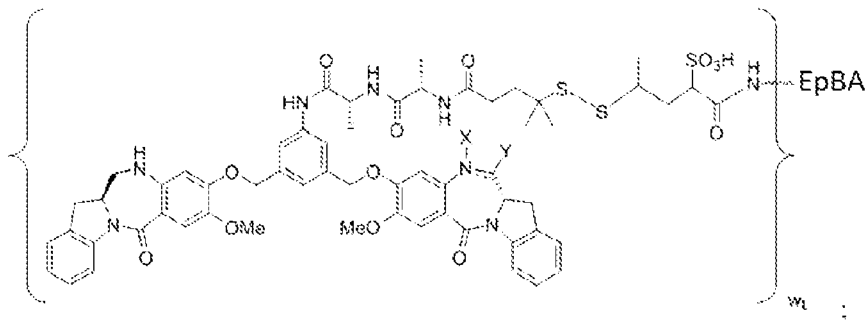

;

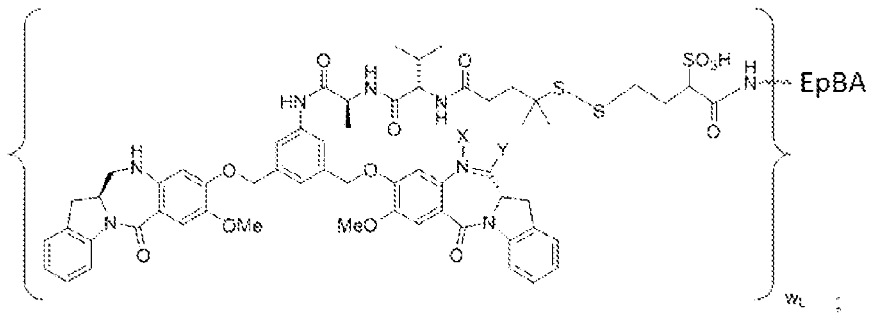

;

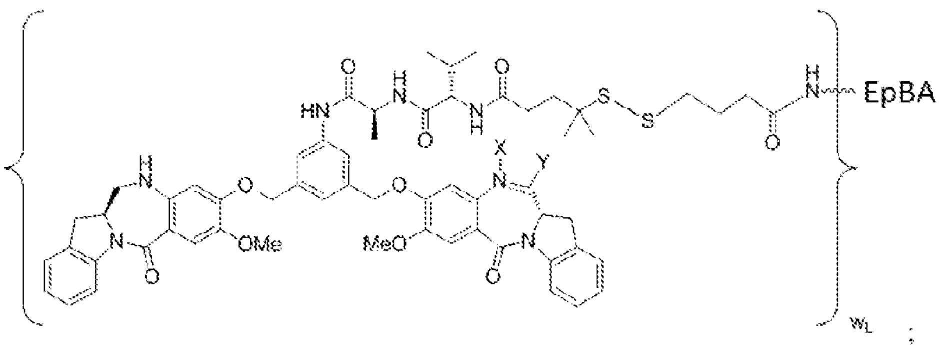

;

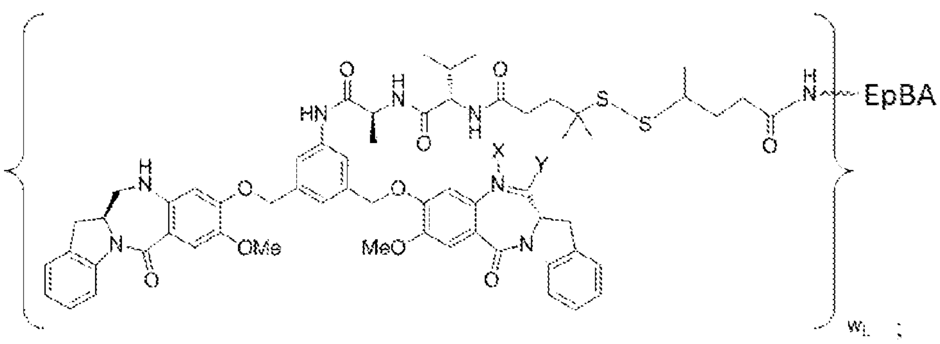

;

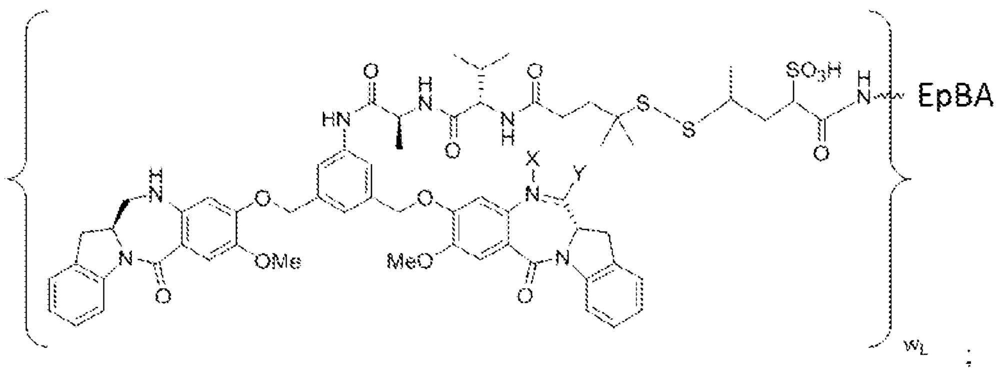

or

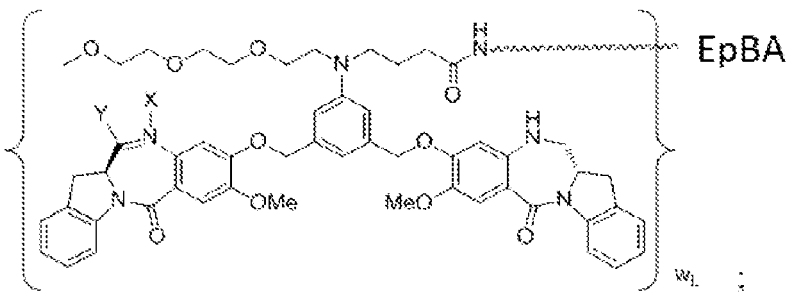

or a pharmaceutically acceptable salt thereof, wherein

$W_L$ is an integer from 1 to 10; the double line $\overline{--}$ between N and C represents a single bond or a double bond, provided that when it is a double bond, X is absent and Y is -H; and

when it is a single bond, X is -H, and Y is -OH or $-SO_3H$ or a pharmaceutically acceptable salt thereof;

[65] the immunoconjugate of any one of [58]-[64], wherein the double line $\overline{--}$ between N and C represents a double bond, X is absent and Y is -H;

[66] the immunoconjugate of any one of [58]-[64], wherein the double line $\overline{--}$ between N and C represents a single bond, X is -H, and Y is -SO3H or a pharmaceutically acceptable salt thereof;

[67] the immunoconjugate of [66], wherein Y is -SO3H, -SO3Na or -SO3K;

[68] the immunoconjugate of [66], wherein Y is -SO3Na;

[69] an immunoconjugate represented by the following formula:

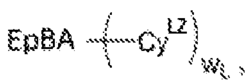

wherein:

EpBAis an EpCAM antibody, EpCAM antibody fragment, or EpCAM activatable antibody according to any of [1]-[47] that is covalently linked to $Cy^{L2}$ through a lysine residue;

$W_L$ is an integer from 1 to 20;

$Cy^{L2}$ is represented by the following formula:

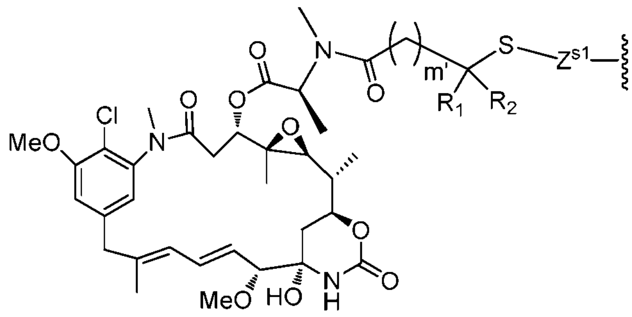

,

m' is 1 or 2;
$R_1$ and $R_2$, are each independently H or a $(C_1$-$C_3)$alkyl; and
$Z^{s1}$ is selected from any one of the following formulas:

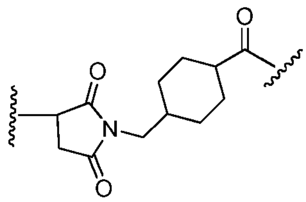 (b1); 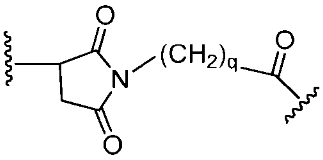 (b2); 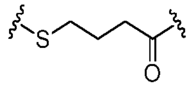 (b3);

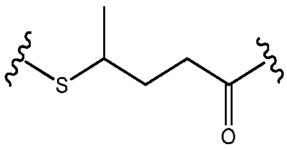 (b4); 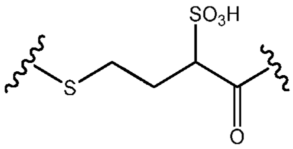 (b5),

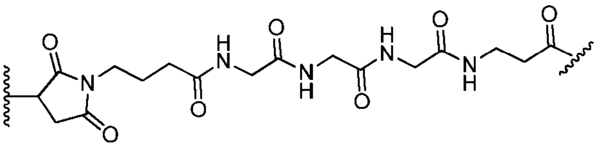 (b6),

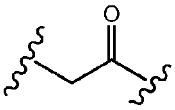 (b7); 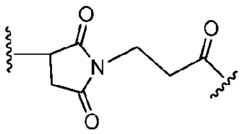 (b8); 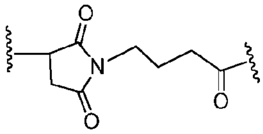 (b9), and 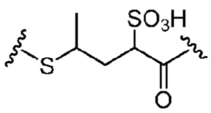

(b10),

wherein q is an integer from 1 to 5;

[70] the immunoconjugate of [69], wherein m' is 1, and $R_1$ and $R_2$ are both H;
[71] the immunoconjugate of [69], wherein m' is 2, and $R_1$ and $R_2$ are both Me;
[72] the immunoconjugate of [69], wherein the immunoconjugate is represented by the following formula:

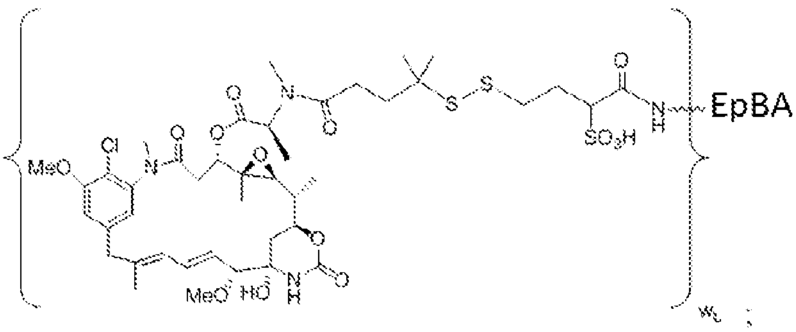

;

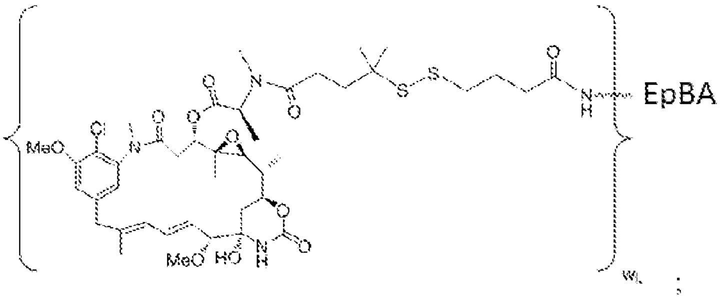

;

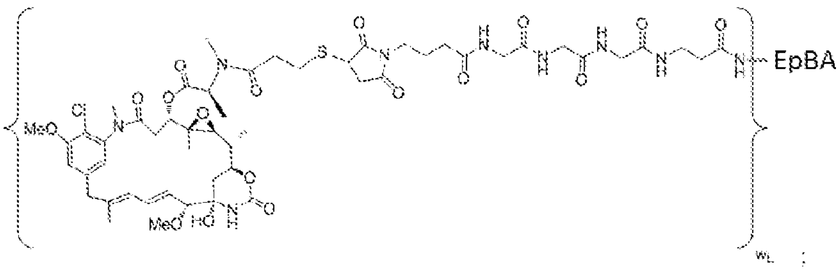

;

or

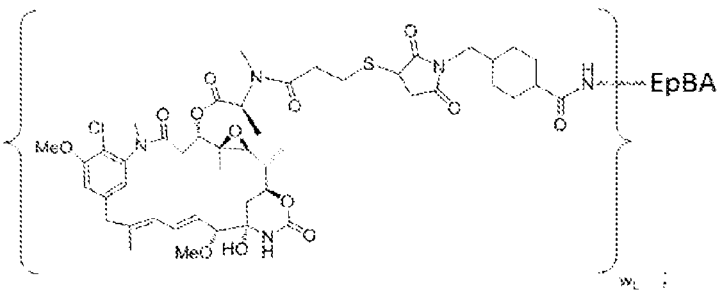

;

or a pharmaceutically acceptable salt thereof, wherein $W_L$ is an integer from 1 to 10;
[73] the immunoconjugate of [69], wherein the immunoconjugate is represented by the following formula:

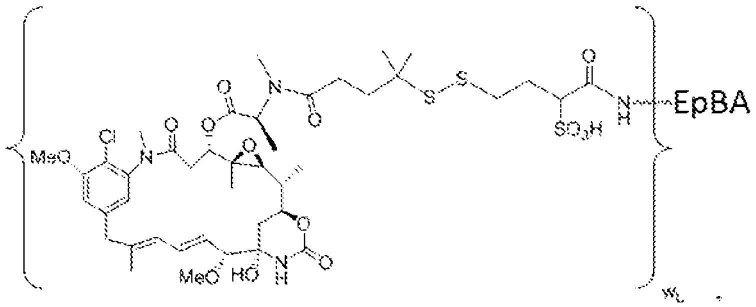

.

or a pharmaceutically acceptable salt thereof.

[74] an immunoconjugate represented by the following formula:

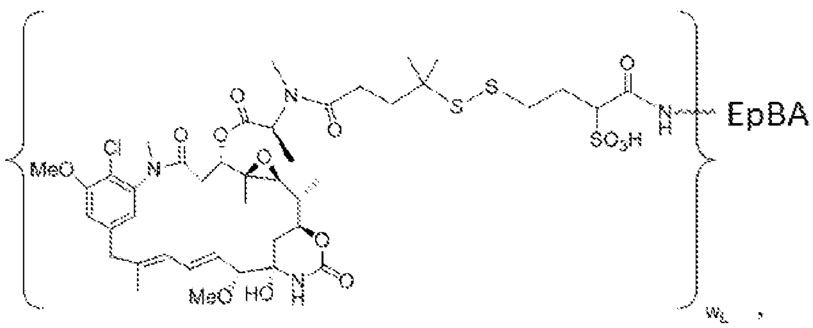

or a pharmaceutically acceptable salt thereof, wherein:

$W_L$ is an integer from 1 to 10;
EpBA is an EpCAM antibody, EpCAM antibody fragment, or EpCAM activatable antibody comprising a VH-CDR1, VH-CDR2, VH-CDR3, VL-CDR1, VL-CDR2, and VL-CDR3 having the sequences of SEQ ID NOs:13-15, 42, 40, and 41, respectively;

[75] the immunoconjugate of [74], wherein the isolated antibody, or EpCAM-binding antibody fragment comprises a heavy chain variable region (VH) and a light chain variable region (VL) having sequences of SEQ ID NO:54 and SEQ ID NO:89, respectively;
[76] the immunoconjugate of [74], wherein the isolated antibody comprises a heavy chain and a light chain having the sequences of SEQ ID NO:103 and SEQ ID NO:140, respectively;
[77] the immunoconjugate of any one of [74]-[76], wherein the immunoconjugate comprises a activatable antibody comprising a masking moiety having an amino acid sequence selected from SEQ ID NOs:151-157;
[78] the immunoconjugate of [77], wherein the masking moiety has the amino acid sequence of SEQ ID NO:155;
[79] the immunoconjugate of [77] or [78], wherein the immunoconjugate comprises a activatable antibody further comprising a cleavable moiety of SEQ ID NO:168 or SEQ ID NO:169;
[80] an immunoconjugate comprising an EpBA coupled to a maytansinoid compound DM21L (also referred to as LDL-DM) represented by the following structural formula:

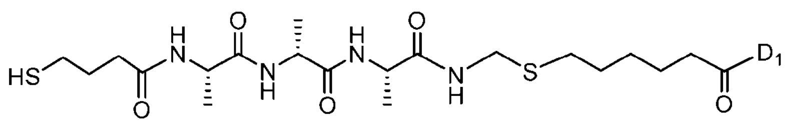 (D-2);

via γ-maleimidobutyric acid N-succinimidyl ester (GMBS) or a
N-(y-maleimidobutryloxy)sulfosuccinimide ester (sulfo-GMBS or sGMBS) linker, wherein EpBA is an EpCAM antibody, EpCAM antibody fragment, or EpCAM activatable antibody according to any of [1]-[47];

[81] the immunoconjugate of [80], wherein the GMBS and sulfo-GMBS (or sGMBS) linkers are represented by the following formula:

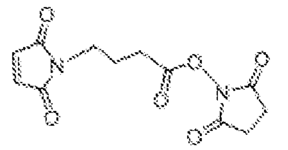 GMBS 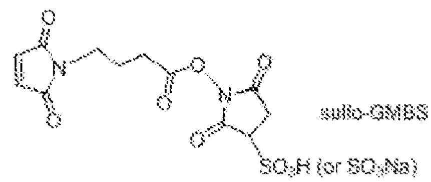 sulfo-GMBS

[82] the immunoconjugate of [80] or [81], represented by the following formula:

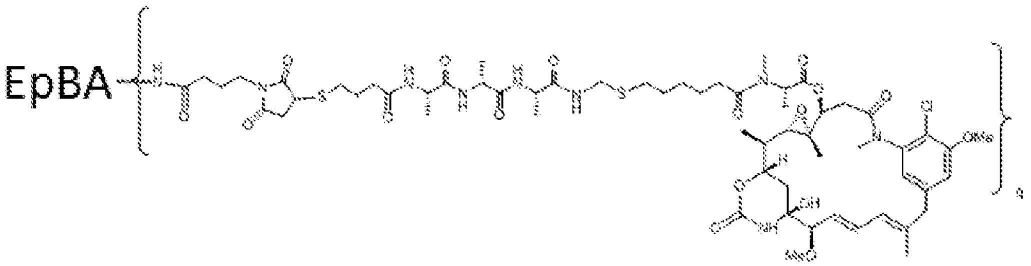

,

wherein:

EpBA is connected to the maytansinoid compound through a Lys amine group, wherein q is an integer from 1 or 10;

[83] the immunoconjugate of any of [80]-[82], wherein the EpBA comprises a VH-CDR1, VH-CDR2, VH-CDR3, VL-CDR1, VL-CDR2, and VL-CDR3 comprising the sequences of SEQ ID NOs: 13-15, 42, 40, and 41, respectively;

[84] the immunoconjugate of [83], wherein the EpBA comprises a heavy chain variable region (VH) and a light chain variable region (VL) having sequences of SEQ ID NO:54 and SEQ ID NO:89, respectively;

[85] the immunoconjugate of [84], wherein the EpBA comprises a heavy chain and a light chain having the sequences of SEQ ID NO:103 and SEQ ID NO:140, respectively;

[86] the immunoconjugate of any one of [83]-[85], wherein the immunoconjugate comprises a activatable antibody comprising a masking moiety having an amino acid sequence selected from SEQ ID NOs:151-157;

[87] the immunoconjugate of [86], wherein the masking moiety has the amino acid sequence of SEQ ID NO:155;

[88] the immunoconjugate of [86] or [87], wherein the immunoconjugate comprises a activatable antibody further comprising a cleavable moiety of SEQ ID NO:168 or SEQ ID NO:169;

[89] the immunoconjugates of any one of [58]-[88], wherein the pharmaceutically acceptable salt is a sodium or a potassium salt;

[90] a pharmaceutical composition comprising the antibody, antigen-binding fragment, or activatable antibody of any one of [1]-[47] and a pharmaceutically acceptable carrier;

[91] a pharmaceutical composition comprising the immunoconjugate of any of [58]-[88] and a pharmaceutically acceptable carrier;

[92] A method of killing a cancer cell that comprises contacting the cancer cell with an effective amount of an antibody, antigen-binding fragment, or activatable antibody of any one of [1]-[47], immunoconjugate of any of [58]-[88], or pharmaceutical composition of [90] or [91];

[93] the method of [92] wherein the cancer cell is an epithelial cancer cell, a breast cancer cell, lung cancer cell, stomach cancer cell, colorectal cancer, prostate cancer cell, ovarian cancer cell, colon cancer cell, rectal cancer cell or a cancer stem cell;

[94] the method of [92] wherein the cancer cell is an ovarian cancer cell, a uterine cancer cell, a gastric cancer cell, a pancreatic cancer cell, or a colorectal cancer cell;

[95] a method of treating cancer that expresses EpCAM, the method comprising administering a therapeutically effective amount of an antibody or antigen-binding fragment of any one of [1]-[47], immunoconjugate of any of [58]-[88], or pharmaceutical composition of [90] or [91], to a subject in need thereof.

[96] the method of treatment according to [95], wherein the cancer is an epithelial cancer;

[97] the method of treatment according to [95], wherein the cancer is breast cancer, lung cancer, stomach cancer, colorectal cancer, prostate cancer, ovarian cancer, colon cancer, esophageal cancer, tracheal cancer, gastric cancer bladder cancer, uterine cancer, rectal cancer, cancer of the small intestine, or a metastases thereof;

[98] the method of treatment according to [95], wherein the cancer is ovarian cancer, uterine cancer, gastric cancer, pancreatic cancer, colorectal cancer, or a metastases thereof;

[99] the method of treatment of [97], wherein the cancer is lung cancer;

[100] the method of treatment of [99], wherein the lung cancer is non-small cell lung cancer;

[101] the method of treatment of [100], wherein the non-small cell lung cancer is non-squamous non-small cell lung cancer;

[102] the method of treatment of [95], wherein the cancer is ovarian cancer;

[103] the method of treatment of [95], wherein the cancer is triple negative breast cancer;

[104] the method of treatment of [95], wherein the cancer is colorectal cancer;

[105] the method of treatment of [95], wherein the cancer is esophageal cancer;

[106] the method of treatment of [95], wherein the cancer is gastric cancer;

[107] the method of treatment of [95], wherein the cancer is uterine cancer;

[108] the method of treatment of [95], wherein the cancer is pancreatic cancer;

[109] an EpCAM antibody, EpCAM-binding antibody fragment, or an immunoconjugate thereof comprising (i) a heavy

chain comprising the same amino acid sequence as the amino acid sequence of the heavy chain encoded by the plasmid deposited with the ATCC® as PTA-125343 and (ii) a light chain comprising the same amino acid sequence as the amino acid sequence of the light chain variable region encoded by the plasmid deposited with the ATCC® as PTA-125342;

[110] an EpCAM activatable antibody, EpCAM-binding activatable antibody fragment, or an immunoconjugate thereof comprising (i) a heavy chain comprising the same amino acid sequence as the amino acid sequence of the heavy chain encoded by the plasmid deposited with the ATCC® as PTA-125343 and (ii) a light chain comprising the same amino acid sequence as the amino acid sequence of the light chain variable region encoded by the plasmid deposited with the ATCC® as PTA-125344; and/or

[111] an EpCAM activatable antibody, EpCAM-binding activatable antibody fragment, or an immunoconjugate thereof comprising (i) a heavy chain comprising the same amino acid sequence as the amino acid sequence of the heavy chain encoded by the plasmid deposited with the ATCC® as PTA-125343 and (ii) a light chain comprising the same amino acid sequence as the amino acid sequence of the light chain variable region encoded by the plasmid deposited with the ATCC® as PTA-125345.

## BRIEF DESCRIPTION OF THE DRAWINGS/FIGURES

**FIGs. 1A and 1B** show binding curves of the murine EpCAM antibody mEpCAM23 to HSC2 cells **(FIG. 1A)** and cyno kidney epithelial cells **(FIG. 1B).**

**FIGs. 2A** and **2B** show sequence alignments between the variable regions of the original muEpCAM-23 light chain and heavy chain sequences and their closest human germline matches. Based on the alignment results, the human germline sequences selected as the acceptor frameworks for the $V_L$ and $V_H$ domains of EpCAM-23 antibody were IGKV2D-29*02 **(FIG. 2A)** and IGHV1-3*01 **(FIG. 2B),** respectively.

**FIGs. 3A-3F** show binding curves of various humanized antibodies of mEpCAM23. In particular, **FIG. 3A** shows binding of mEpCAM23 to HSC2 cells, **FIG. 3B** shows binding of mEpCAM23 to cyno primary kidney epithelial cells, **FIG. 3C** shows binding of various humanized EpCAM23 antibodies to HSC2 cells, and **FIG. 3D** shows binding of various humanized EpCAM23 antibodies to cyno primary kidney epithelial cells. **FIG. 3E** shows binding of the site-specific modified antibody huEpCAM23Gv4.2-C442 to HSC2 cells and **FIG. 3F** shows binding of the same antibody to cyno primary kidney epithelial cells.

**FIGs. 4A** and **4B** show the sequence alignment of the variable regions of the original murine EpCAM-23 antibody, grafted version Gv2.2, and grafted version Gv4.2. In particular, **FIG. 4A** shows the alignment of the variable regions of the antibody light chains, and **FIG. 4B** shows the alignment of the variable regions of the antibody heavy chains.

**FIGs. 5A-5N** show binding curves for various affinity variants of huEpCAM23Gv4.2 on HSC2 cells and cyno primary kidney epithelial cells. In particular, **FIG. 5A** shows binding of Gv4a.2, Gv4b.2, and Gv4c.2 to huEpCAM-mFc; **FIG. 5B** shows binding of Gv4a.2, Gv4b.2, and Gv4c.2 to cynoEpCAM-mFc; **FIG. 5C** shows binding of Gv4.2a, Gv4.2b, Gv4.2c, and Gv4.2d to huEpCAM-mFc; **FIG. 5D** shows binding of Gv4.2a, Gv4.2b, Gv4.2c, and Gv4.2d to cynoEpCAM-mFc; **FIG. 5E** shows binding of Gv4a.2a, Gv4b.2a, and Gv4c.2a to HSC2 cells; **FIG. 5F** shows binding of Gv4a.2a, Gv4b.2a, and Gv4c.2a to cyno primary kidney epithelial cells; **FIG. 5G** shows binding of Gv4a.2b, Gv4a.2d, Gv4b.2b, Gv4b.2d, Gv4c.2b, and Gv4c.2d to HSC2 cells; and **FIG. 5H** shows binding of Gv4a.2b, Gv4a.2d, Gv4b.2b, Gv4b.2d, Gv4c.2b, and Gv4c.2d to cyno primary kidney epithelial cells. **FIG. 5I** shows binding of Gv4.2H, Gv4.2K, Gv4.2L, Gv4.2O, Gv4.2P, Gv4.2Q, Gv4.2R, and Gv4.2S on HSC2 cells; **FIG. 5J** shows binding of 1361-D, 1361-H, 1361-I, and 1361-L on HSC2 cells; and **FIG. 5K** shows binding of 1565-A, 1565-F, 1565-G, 1565-S, 1565-T, 1565-V, and 1565-Y on HSC2 cells. **FIG. 5L** shows binding of Gv4.2H, Gv4.2K, Gv4.2L, Gv4.2O, Gv4.2P, Gv4.2Q, Gv4.2R, and Gv4.2S on cyno primary kidney epithelial cells; **FIG. 5M** shows binding of 1361-D, 1361-H, 1361-I, and 1361-L on cyno primary kidney epithelial cells; and **FIG. 5N** shows binding of 1565-A, 1565-F, 1565-G, 1565-S, 1565-T, 1565-V, and 1565-Y on cyno primary kidney epithelial cells.

**FIGs. 6A** and **6B** show binding curves of intact and uPA-activated huEpCAM23Gv4.2 activatable antibodies with various masks on HSC2 cells and cyno primary kidney epithelial cells. In particular, **FIG. 6A** shows binding of intact and uPA-activated huEpCAM23 with substrate 3014 and with masks Ep01-2, Ep02, Ep03, Ep04, Ep05, Ep07, and Ep11 on HSC2 cells, and **FIG. 6B** shows binding of intact and uPA-activated huEpCAM23 with substrate 3014 and masks Ep02, Ep03, Ep04, Ep05, and Ep07 on cyno primary kidney epithelial cells.

**FIGs. 7A** and **7B** show binding curves of various uPA-treated activatable antibody-drug conjugates of huEpCAM23 on HSC2 cells. In particular, **FIG. 7A** shows binding of uPA-treated huEpCAM23Gv4.2-sSPDB-DM4 with substrate 3014 and with masks Ep01-2, Ep02, Ep03, Ep04, Ep05, Ep07, and Ep11 on HSC2 cells, and **FIG. 7B** shows binding of uPA-activated huEpCAM23Gv4.2-lys-DGN549 with substrate 3014 and with masks Ep01-2, Ep02, Ep03, Ep04, Ep05, Ep07, and Ep11 on HSC2 cells.

**FIGs. 8A-8D** show binding curves of various activatable antibody-drug conjugates with the substrate 2014 or 3014 on HSC2 cells. In particular, **FIG. 8A** shows binding of intact and uPA-activated huEpCAM23Gv4.2-Ep05-2014-sSPDB-DM4 on HSC2 cells, **FIG. 8B** shows binding of intact and uPA-activated huEpCAM23Gv4.2-Ep05-3014-sSPDB-DM4 on HSC2 cells, **FIG. 8C** shows binding of intact and uPA-activated huEpCAM23Gv4.2-Ep05-3014-lys-DGN549 on HSC2 cells, and **FIG. 8D** shows binding of intact and uPA-activated huEpCAM23Gv4.2-Ep05-3014-GMBS-DM21L and intact and uPA-activated huEpCAM23Gv4.2-Ep05-2014-GMBS-DM21L on HSC2 cells.

**FIG. 9** shows an alignment of the extracellular domain of human EpCAM (SEQ ID NO: 1) and mouse EpCAM (SEQ ID NO: 210), following cleavage of the signal peptide.

**FIGs. 10A** and **10B** show an alignment of the extracellular region of human EpCAM (SEQ ID NO: 1), mouse EpCAM (SEQ ID NO:210), and various chimerized EpCAM variants (SEQ ID NOs:211-214). **FIG. 10A** shows an alignment between amino acid residues 1-160, and **FIG. 10B** shows an alignment between amino acid residues 161-243.

**FIG. 11** shows binding curves of huEpCAM23Gv4.2 to the extracellular domains of human EpCAM and mouse EpCAM.

**FIGs. 12A-12K** show dose-response curves of antibody-drug conjugates (ADCs) of huEpCAM23Gv4.2 in various cell lines. In particular, **FIGs. 12A-12E** show the dose-response curves of huEpCAM23Gv4.2-sSPDB-DM4 in H1568 cells **(FIG. 12A),** in H292 cells **(FIG. 12B),** in H2110 cells **(FIG. 12C),** in LoVo cells **(FIG. 12D),** and in Detroit562 cells **(FIG. 12E). FIG. 12F** shows the dose-response curves of huEpCAM23Gv4.2-lys-DGN549 and huEpCAM23Gv4.2-C442-DGN549 in H2110 cells. **FIGs. 12G-12K** show the dose-response curves of huEpCAM23Gv4.2-GMBS-DM21L in Calu3 cells **(FIG. 12G),** Detroit562 cells **(FIG. 12H),** EBC-1 cells **(FIG. 12I),** H2110 cells **(FIG. 12J),** and H441 cells **(FIG. 12K).**

**FIGs. 13A-13D** show the dose-response curves of intact and uPA-activated activatable antibody-drug conjugates (AADCs) of huEpCAM23Gv4.2-sSPDB-DM4 in various cell lines. In particular, the dose-response curves of intact and uPA-activated huEpCAM23Gv4.2-Ep05-2014-sSPDB-DM4 and intact and uPA-activated huEpCAM23Gv4.2-Ep05-3014-sSPDB-DM4 are shown in Calu3 cells **(FIG. 13A),** OV90 cells **(FIG.13B),** EBC-1 cells **(FIG. 13C),** and H2110 cells **(FIG. 13D).**

**FIGs. 14A-14D** show the dose-response curves of intact and uPA-activated AADCs of huEpCAM23Gv4.2-GMBS-DM21L in various cell lines. In particular, the dose-response curves of intact and uPA-activated huEpCAM23Gv4.2-Ep05-2014-GMBS-DM21L and intact and uPA-activated huEpCAM23Gv4.2-Ep05-3014-GMBS-DM21L are shown in Calu3 cells **(FIG. 14A),** Detroit562 cells **(FIG.14B),** EBC-1 cells **(FIG. 14C),** and H2110 cells **(FIG. 14D).**

**FIGs. 15A-15D** show the dose-response curves of intact and uPA-activated AADCs of huEpCAM23-lys-DGN549 in various cell lines. In particular, the dose-response curves of intact and uPA-activated huEpCAM23Gv4.2-Ep05-3014-lys-DGN549 are shown in H2110 cells **(FIG. 15A),** EBC-1 cells **(FIG. 15B),** Calu3 cells **(FIG. 15C),** and OV90 cells **(FIG. 15D).**

**FIG. 16** shows the anti-tumor activity of huEpCAM23Gv4.2-lys-DGN549 in the H2110 non-small cell lung cancer xenograft model.

**FIGs. 17A-17F** show the anti-tumor activity of various AADCs of huEpCAM23Gv4.2 in the H2110 non-small cell lung cancer xenograft model. In particular, **FIG. 17A** shows the anti-tumor activity of huEpCAM23-lys-DGN549 with masks Ep02, Ep01, Ep11, and Ep04, **FIG. 17B** shows the anti-tumor activity of huEpCAM23-lys-DGN549 with masks Ep03, Ep05, Ep07, and Ep04, and **FIG. 17C** shows the anti-tumor activity of huEpCAM23-Ep05-3014-lys-DGN549 and huEpCAM23-Ep07-3014-lys-DGN549 compared to non-cleavable conjugates. **FIG. 17D** shows the anti-tumor activity of huEpCAM23Gv4.2-Ep01-02-2014-sSPDB-DM4 compared to a non-cleavable conjugate and to the ADC huEpCAM23Gv4.2-sSPDB-DM4, **FIG. 17E** shows the anti-tumor activity of huEpCAM23Gv4.2-Ep05-2014-sSPDB-DM4 and huEpCAM23Gv4.2-Ep05-3014-sSPDB-DM4 compared to a non-cleavable conjugate, and **FIG. 17F** shows the anti-tumor activity of huEpCAM23Gv4.2-Ep05-2014-lys-DGN549 and huEpCAM23Gv4.2-Ep05-3014-lys-DGN549 compared to a non-cleavable conjugate.

**FIG. 18** shows the anti-tumor activity of various AADCs of huEpCAM23Gv4.2-sSPDB-DM4 in the Calu3 non-small cell lung cancer xenograft model.

**FIG. 19** shows the anti-tumor activity of various AADCs of huEpCAM23Gv4.2-sSPDB-DM4 in the H292 non-small cell lung cancer xenograft model.

**FIG. 20** shows the anti-tumor activity of Ep05-3014-DM4, Ep05-2014-DM4, Ep05-3014-DM21, and Ep05-2014-DM21 in C.B-17 SCID mice bearing the Detroit 562 HNSCC xenograft.

**FIG. 21** shows the anti-tumor activity of Ep05-3014-DM21 and Ep05-2014-DM21 in C.B-17 SCID mice bearing the NCI-H441 NSCLC xenograft.

**FIG. 22** shows the anti-tumor activity of Ep05-2014-DM21 in C.B-17 SCID mice bearing the OV-90 EOC xenograft.

**FIG. 23** shows the anti-tumor activity of Ep05-2014-DM21 in C.B-17 SCID mice bearing the Calu-3 NSCLC xenograft.

**FIG. 24** shows the tolerability of an EpCAM-targeting ADC (huEpCAM23Gv4.2-sSPDB-DM4 (Anti-EpCAM-DM4)) and EpCAM-targeting AADCs (huEpCAM23Gv4.2-Ep05-2014-sSPDB-DM4 (M05-2014-DM4)) and huEpCAM23Gv4.2-Ep05-3014-sSPDB-DM4 (M05-3014-DM4)) in cynomolgus monkeys.

**FIGs. 25A-25E** show the serum chemistry of cynomolgus monkeys administered an EpCAM-targeting ADC (huEpCAM23Gv4.2-sSPDB-DM4) or an EpCAM-targeting AADC (huEpCAM23Gv4.2-Ep05-2014-sSPDB-DM4). In particular, **FIG. 25A** shows A/G ratios, **FIG. 25B** shows urea nitrogen concentrations, **FIG. 25C** shows creatinine concentrations, **FIG. 25D** shows lipase concentrations, and **FIG. 25E** shows amylase concentrations.

**FIG. 26** shows the pharmacokinetic profile of cynomolgus monkeys receiving 8 mg/kg of an EpCAM-targeting ADC (huEpCAM23Gv4.2-sSPDB-DM4 (Anti-EpCAM-DM4 ADC)) or 8 mg/kg of an EpCAM-targeting AADC (huEpCAM23Gv4.2-Ep05-2014-sSPDB-DM4 (M05-2014-DM4 AADC)), or huEpCAM23Gv4.2-Ep05-3014-sSPDB-DM4 (M05-3014-DM4 AADC)).

**FIG. 27** shows the tolerability of huEpCAM23Gv4.2-Ep05-2014-DM21L (Ep05-2014-DM21L) and huEpCAM23Gv4.2-Ep05-3014-DM21L (Ep05-3014-DM21L) in cynomolgus monkeys.

**FIGs. 28A-28E** show the serum chemistry of cynomolgus monkeys administered 12 mg/kg of huEpCAM23Gv4.2-Ep05-2014-DM21L (Ep05-2014-DM21L) and huEpCAM 23Gv4.2-Ep05-3014-DM21L (Ep05-3014-DM21L). In particular, **FIG. 28A** shows A/G ratios, **FIG. 28B** shows urea nitrogen concentrations, **FIG. 28C** shows creatinine concentrations, **FIG. 28D** shows lipase concentrations, and **FIG. 28E** shows amylase concentrations.

**FIG. 29** shows the pharmacokinetic profile of cynomolgus monkeys receiving 12 mg/kg huEpCAM23Gv4.2-Ep05-2014-DM21L (Ep05-2014-DM21).

**FIG. 30** shows an expanded analysis of EpCAM expression in indication-specific tissue microarrays.

## DETAILED DESCRIPTION

The disclosure provides antibodies and antigen-binding antibody fragments that specifically bind human EpCAM, including activatable antibodies (activatable forms of the EpCAM antibodies or EpCAM-binding antibody fragments), and immunoconjugates comprising the antibodies, antibody fragments, and activatable antibodies. EpCAM is known to be associated with cell-cell adhesion in epithelia and to be involved in cell signaling, differentiation, proliferation, and migration. The overexpression of EpCAM has been implicated in the pathogenesis of diseases and disorders, such as cancer. For example, EpCAM is highly expressed in a variety of cancer types such as, for example, breast cancer, lung cancer, liver cancer, stomach cancer, head & neck cancer, prostate cancer, pancreatic cancer, ovarian cancer, colon cancer, and kidney cancer, and most cancers (and metastases) of epithelial origin. EpCAM is also highly expressed in tumor initiating/cancer stem cells. The provided EpCAM antibodies, EpCAM-binding antibody fragments, EpCAM activatable antibodies, and immunoconjugates have uses that include treating such diseases and cancers.

### Definitions

To facilitate an understanding, a number of terms and phrases are defined below.

The terms "epithelial cell adhesion molecule" or "EpCAM", as used herein, refers to any native human EpCAM unless otherwise indicated. The term also encompasses naturally occurring variants of EpCAM, *e.g.,* splice variants, allelic variants and isoforms. EpCAM polypeptides can be isolated from a variety of sources, such as from human or cynomolgous tissue or other biological samples, or prepared by known recombinant or synthetic methods. EpCAM is also known as CD326, 17-1A antigen, HEA125, MK-1, EGP-2, EGP314, EGP40, GA733-2, KSA, TACSTD1, TROP1, KS1/4, M4S1, DIAR5, MIC18, HNPCC8, and ESA. Examples of EpCAM sequences include, but are not limited to NCBI reference number NP_002345.2 (amino acid residues 24-314 correspond to mature EpCAM, amino acids 24-265 correspond to the extracellular region of mature EpCAM (SEQ ID NO:1)). The extracellular region of mature EpCAM can further be divided into three domains: D1 (amino acids 1-36 of SEQ ID NO:1 (SEQ ID NO:2)), D2 (amino acids 43-112 of SEQ ID NO:1 (SEQ ID NO:3)), and D3 (amino acids 113-243 of SEQ ID NO:1 (SEQ ID NO:4)).

The terms "antibody" and "antigen-binding antibody fragment" and the like, as used herein, include any protein or peptide containing molecule that comprises at least a portion of an immunoglobulin molecule, such as, but not limited to, at least one complementarity determining region (CDR) of a heavy or light chain or an antigen binding portion thereof. Such antibody optionally further affects at least one EpCAM activity, such as, but not limited to, where such antibody modulates, decreases, increases, antagonizes, agonizes, partially agonizes, partially antagonizes, mitigates, alleviates, blocks, inhibits, abrogates and/or interferes with at least one EpCAM activity or binding *in vitro, in situ, in vivo* and/or *ex vivo.* In some embodiments, the EpCAM antibody or EpCAM-binding antibody fragment affects at least one EpCAM-mediated activity or function selected from: ligand binding, receptor signaling, membrane association, cell migration, cell proliferation, receptor binding activity, RNA, DNA or protein production and/or synthesis.

Antibodies are heterotetrameric glycoproteins, composed of two identical light chains (LC) and two identical heavy chains (HC). Typically, each light chain is linked to a heavy chain by one covalent disulfide bond, while the number of disulfide linkages varies between the heavy chains of different immunoglobulin isotypes. Each heavy and light chain also has spaced intrachain disulfide bridges. Each heavy chain has at one end a variable region (VH) followed by a number of constant domains. Each light chain has a variable region at one end (VL) and a constant domain at its other end; the

constant domain of the light chain is aligned with the first constant domain of the heavy chain and the light chain variable region is aligned with the variable region of the heavy chain. Antibody light chains of any vertebrate species can be assigned to one of two clearly distinct types, namely kappa and lambda, based on the amino acid sequences of their constant domains. Immunoglobulins can be assigned to five major classes, namely IgA, IgD, IgE, IgG and IgM, depending on the heavy chain constant domain amino acid sequence. IgA and IgG are further sub-classified as the isotypes IgA1, IgA2, IgG1, IgG2, IgG3 and IgG4.

The term "antibody" also includes fragments, specified portions and variants thereof, including antibody mimetics or comprising portions of antibodies that mimic the structure and/or function of an antibody or specified fragment or portion thereof, including single chain antibodies and antigen (*e.g.*, EpCAM)-binding antibody fragments. Functional fragments include antigen-binding fragments that bind to a mammalian antigen, such as EpCAM, alone or in combination with other antigens. For example, antibody fragments capable of binding to antigen or portions thereof, including, but not limited to, Fab (*e.g.,* by papain digestion), Fab' (*e.g.,* by pepsin digestion and partial reduction) and F(ab')2 (*e.g.,* by pepsin digestion), facb (*e.g.,* by plasmin digestion), pFc' (*e.g.,* by pepsin or plasmin digestion), Fd (*e.g.,* by pepsin digestion, partial reduction and reaggregation), Fv or scFv (*e.g.,* by molecular biology techniques) fragments, are encompassed by the disclosure (*see, e.g.,* Colligan, Immunology).

Such fragments can be produced by enzymatic cleavage, synthetic or recombinant techniques, as known in the art and/or as disclosed herein. Antibodies can also be produced in a variety of truncated forms using antibody genes in which one or more stop codons have been introduced upstream of the natural stop site. For example, a combination gene encoding a F(ab')2 heavy chain portion can be designed to include DNA sequences encoding the CH1 domain and/or hinge region of the heavy chain. The various portions of antibodies can be joined together chemically by conventional techniques, or can be prepared as a contiguous protein using genetic engineering techniques.

The term "antibody fragment" refers to a portion of an intact antibody, generally the antigen binding or variable region of an intact antibody. Examples of antibody fragments include, but are not limited to Fab, Fab', F(ab')2, single chain (scFv) and Fv fragments, diabodies; linear antibodies; single-chain antibody molecules; single Fab arm "one arm" antibodies and multispecific antibodies formed from antibody fragments, among others.

Antibody fragments include any protein or peptide containing molecule that comprises at least a portion of an immunoglobulin molecule, such as but not limited to, at least one complementarity determining region (CDR) of a heavy or light chain or a ligand binding portion thereof, a heavy chain or light chain variable region, a heavy chain or light chain constant region, a framework region, or any portion thereof, or at least one portion of an antigen or antigen receptor or binding protein, which can be incorporated into an EpCAM antibody provided herein.

The term "variable" refers to the fact that certain portions of the variable regions of antibodies differ extensively in sequence among antibodies and are used in the binding and specificity of each particular antibody for its particular antigen. However, the variability is not evenly distributed throughout the variable regions of antibodies. The variability is concentrated in three segments called complementarity-determining regions (CDRs) or hypervariable regions both in the light-chain and the heavy-chain variable regions. The more highly conserved portions of variable regions are called the framework (FR). The variable regions of native heavy and light chains each comprise four FR regions, largely adopting a beta-sheet configuration, connected by three CDRs, which form loops connecting, and in some cases forming part of, the beta-sheet structure. The CDRs in each chain are held together in close proximity by the FR regions and, with the CDRs from the other chain, contribute to the formation of the antigen-binding site of antibodies. The constant domains are not involved directly in binding an antibody to an antigen, but exhibit various effector functions, such as participation of the antibody in antibody-dependent cellular toxicity. There are at least two techniques for determining CDRs: (1) an approach based on cross-species sequence variability (*i.e.,* Kabat et al., Sequences of Proteins of Immunological Interest, (5th ed., 1991, National Institutes of Health, Bethesda Md.)); and (2) an approach based on crystallographic studies of antigen-antibody complexes (Al-lazikani et al., J. Molec. Biol. 273:927-948 (1997)). In addition, combinations of these two approaches are sometimes used in the art to determine CDRs.

The Kabat numbering system is generally used when referring to a residue in the variable region (approximately residues 1-107 of the light chain and residues 1-113 of the heavy chain) (*e.g.,* Kabat et al., Sequences of Immunological Interest. 5th Ed. Public Health Service, National Institutes of Health, Bethesda, Md. (1991)).

The amino acid position numbering as in Kabat, refers to the numbering system used for heavy chain variable regions or light chain variable regions of the compilation of antibodies in Kabat et al., Sequences of Proteins of Immunological Interest, 5th Ed. Public Health Service, National Institutes of Health, Bethesda, Md. (1991). Using this numbering system, the actual linear amino acid sequence can contain fewer or additional amino acids corresponding to a shortening of, or insertion into, a FR or CDR of the variable region. For example, a heavy chain variable region can include a single amino acid insert (residue 52a according to Kabat) after residue 52 of H2 and inserted residues (*e.g.*, residues 82a, 82b, and 82c, etc. according to Kabat) after heavy chain FR residue 82. The Kabat numbering of residues can be determined for a given antibody by alignment at regions of homology of the sequence of the antibody with a "standard" Kabat numbered sequence. Chothia refers instead to the location of the structural loops (Chothia et al., J. Mol. Biol. 196:901-917 (1987)). The end of the Chothia CDR-H1 loop when numbered using the Kabat numbering convention varies

between H32 and H34 depending on the length of the loop (this is because the Kabat numbering scheme places the insertions at H35A and H35B; if neither 35A nor 35B is present, the loop ends at 32; if only 35A is present, the loop ends at 33; if both 35A and 35B are present, the loop ends at 34). The AbM hypervariable regions represent a compromise between the Kabat CDRs and Chothia structural loops, and are used by Oxford Molecular's AbM antibody modeling software.

The terms "EpCAM antibody", "EpCAM antibody", "antibody that specifically binds to EpCAM", "EpCAM-binding antibody fragment", an "antibody fragment that specifically binds EpCAM" refers to an antibody that is capable of binding EpCAM with sufficient affinity such that the antibody is useful as a diagnostic and/or therapeutic agent in targeting EpCAM. The extent of binding of an EpCAM antibody to an unrelated, non-EpCAM protein is less than about 10% of the binding of the antibody to EpCAM as measured, *e.g.*, by a radioimmunoassay (RIA).

**Table 1.**

| Loop | Kabat | AbM | Chothia |
|---|---|---|---|
| L1 | L24-L34 | L24-L34 | L24-L34 |
| L2 | L50-L56 | L50-L56 | L50-L56 |
| L3 | L89-L97 | L89-L97 | L89-L97 |
| H1 | H31-H35B | H26-H35B | H26-H32..34 |
| | | (Kabat Numbering) | |
| H1 | H31-H35 | H26-H35 | H26-H32 |
| | | (Chothia Numbering) | |
| H2 | H50-H65 | H50-H58 | H52-H56 |
| H3 | H95-H102 | E95-H102 | H95-H102 |

The term "epitope" refers to a protein determinant capable of specific binding to an antibody. Epitopes usually consist of chemically active surface groupings of molecules such as amino acids or sugar side chains and usually have specific three dimensional structural characteristics, as well as specific charge characteristics. When the antigen is a polypeptide, epitopes can be formed both from contiguous amino acids and noncontiguous amino acids juxtaposed by tertiary folding of a protein. Epitopes formed from contiguous amino acids are typically retained upon protein denaturing, whereas epitopes formed by tertiary folding are typically lost upon protein denaturing. An epitope typically includes at least 3, and more usually, at least 5 or 8-10 amino acids in a unique spatial conformation.

"Blocking" antibody is one which inhibits or reduces the biological activity of the antigen it binds such as EpCAM. Preferred blocking antibodies substantially or completely inhibit the biological activity of the antigen. Desirably, the biological activity is reduced by 10%, 20%, 30%, 50%, 70%, 80%, 90%, 95%, or even 100%. In one embodiment, the blocking antibody reduces the EpCAM associated tyrosine kinase activity 10%, 20%, 30%, 50%, 70%, 80%, 90%, 95%, or even 100%.

An "isolated" antibody is one separated and/or recovered from its natural environment. Contaminant components of its natural environment are materials which would interfere with diagnostic or therapeutic uses for the antibody, and may include enzymes, hormones, and other proteinaceous or non-proteinaceous solutes. In preferred aspects, the antibody will be purified (1) to greater than 95% by weight of antibody as determined by, for example, the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE (sodium dodecyl sulfate polyacrylamide gel electrophoresis) under reducing or non-reducing conditions using Coomassie blue or, preferably, silver stain. Isolated antibody includes the EpCAM antibody in situ within recombinant cells since at least one component of the antibody's natural environment will not be present. Ordinarily, however, isolated antibody will be prepared by at least one purification step.

A "human antibody" refers to an antibody produced by a human or an antibody having an amino acid sequence corresponding to an antibody produced by a human made using any technique known in the art. This definition of a human antibody includes intact or full-length antibodies, activatable antibodies, antigen (*e.g.*, human or cynomolgous EpCAM)-binding antibody fragments, and/or antibodies comprising at least one human heavy and/or light chain polypeptide such as, for example, an antibody comprising murine light chain and human heavy chain polypeptides.

The term "chimeric antibodies," as used herein, refer to antibodies wherein the sequence of the immunoglobulin molecule is derived from two or more species. Typically, the variable region of both light and heavy chains corresponds to the variable region of antibodies derived from one species of mammals (*e.g.*, mouse, rat, rabbit, etc.) with the desired specificity, affinity, and capability while the constant regions are homologous to the sequences in antibodies derived from another (usually human) to avoid eliciting an immune response in that species.

The term "humanized antibody," as used herein, refers to forms of non-human (*e.g.*, murine) antibodies that are specific immunoglobulin chains, chimeric immunoglobulins, or antigen-binding antibody fragments that contain minimal non-human (*e.g.*, murine) sequences. Typically, humanized antibodies are human immunoglobulins in which residues from the complementarity determining region (CDR) are replaced by residues from the CDR of a non-human species (*e.g.*, mouse, rat, rabbit, hamster) that have the desired specificity, affinity, and capability (Jones et al., Nature 321:522-525 (1986); Riechmann et al., Nature 332:323-327 (1988); Verhoeyen et al., Science 239:1534-1536 (1988)). In some instances, the Fv framework region (FR) residues of a human immunoglobulin are replaced with the corresponding residues in an antibody from a non-human species that has the desired specificity, affinity, and capability. The humanized antibody can be further modified by the substitution of additional residues either in the Fv framework region and/or within the replaced non-human residues to refine and optimize antibody specificity, affinity, and/or capability. In general, the antibody will comprise substantially all of at least one, and typically two or three, variable regions containing all or substantially all of the CDR regions that correspond to the non-human immunoglobulin whereas all or substantially all of the FR regions are those of a human immunoglobulin consensus sequence. The antibody can also comprise at least a portion of an immunoglobulin constant region or domain (Fc), typically that of a human immunoglobulin. Examples of methods used to generate humanized antibodies are described in U.S. Pat. No. 5,225,539.

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity (CDC); Fc receptor binding; antibody-dependent cell-mediated cytotoxicity (ADCC); and antibody-dependent cell-mediated phagocytosis (ADCP).

"Human effector cells" are leukocytes which express one or more FcRs and perform effector functions. In certain aspects, the cells express at least FcγRIII and perform ADCC or ADCP effector function(s). Examples of human leukocytes which mediate ADCC or ADCP include peripheral blood mononuclear cells (PBMC), natural killer (NK) cells, monocytes, cytotoxic T cells and neutrophils. The effector cells may be isolated from a native source, *e.g.,* from blood.

The term "Fc region" as used herein includes the polypeptides comprising the constant region of an antibody excluding the first constant region immunoglobulin domain. Thus Fc refers to the last two constant region immunoglobulin domains of IgA, IgD, and IgG, and the last three constant region immunoglobulin domains of IgE and IgM, and the flexible hinge N-terminal to these domains. For IgA and IgM, Fc may include the J chain. For IgG, Fc comprises immunoglobulin domains Cγ2 and Cγ3 (Cγ2 and Cγ3) and the hinge between Cγ1 (Cγ1) and Cγ2 (Cγ2). Although the boundaries of the Fc region may vary, the human IgG heavy chain Fc region is usually defined to comprise residues C226 or P230 to its carboxyl-terminus, wherein the numbering is according to the EU index as in Kabat *et al.,* (1991, NIH Publication 91-3242, National Technical Information Service, Springfield, Va.). The "EU index as set forth in Kabat" refers to the residue numbering of the human IgG1 EU antibody as described in Kabat *et al., supra.* Fc may refer to this region in isolation, or this region in the context of an antibody, antibody fragment, or Fc fusion protein. An Fc variant protein may be an antibody, Fc fusion, or any protein or protein domain that comprises an Fc region. Particularly preferred are proteins comprising variant Fc regions, which are non-naturally occurring variants of an Fc. Polymorphisms have been observed at a number of Fc positions, including, but not limited to, Kabat 270, 272, 312, 315, 356, and 358, and thus slight differences between the presented sequence and sequences in the prior art may exist and would be understood by one skilled in the art based on the present teachings.

The term "conjugate", "immunoconjugate", "ADC", or "AADC" as used herein, refers to a compound or a derivative thereof that is linked to a cell binding agent (*i.e.,* an EpCAM antibody, EpCAM-binding antibody fragment, or EpCAM activatable antibody) and is defined by a generic formula: C-L-A, wherein C= compound, L= linker, and A= EpCAM-binding agent (EpBA) (*e.g.,* an EpCAM antibody, EpCAM-binding antibody fragment, or EpCAM activatable antibody, as disclosed herein). In some embodiments, the generic formula: D-L-A, wherein D=drug, L=linker and A=cell binding agent (*e.g.,* an EpCAM antibody, EpCAM-binding antibody fragment, or EpCAM activatable antibody), may also be used in the same manner.

A "linker" is any chemical moiety that is capable of linking a compound, usually a drug, such as a maytansinoid or an indolinobenzodiazepine compounds, to a cell-binding agent such as an anti EpCAM antibody or an EpCAM-binding antibody fragment in a stable, covalent manner. Linkers can be susceptible to or be substantially resistant to acid-induced cleavage, light-induced cleavage, peptidase-induced cleavage, esterase-induced cleavage, and disulfide bond cleavage, at conditions under which the compound or the antibody remains active. Suitable linkers are well known in the art and include, for example, disulfide groups, thioether groups, acid labile groups, photolabile groups, peptidase labile groups and esterase labile groups. Linkers also include peptide linkers and charged linkers, and hydrophilic forms thereof, as disclosed herein and know in the art.

"Aberrant cell proliferation", as used herein, unless otherwise indicated, refers to cell growth that is independent of normal regulatory mechanisms (*e.g.*, loss of contact inhibition). This includes, for example, the abnormal growth of: (1) tumor cells (tumors) that proliferate by expressing a mutated tyrosine kinase or over expression of a receptor tyrosine kinase; (2) benign and malignant cells of other proliferative diseases in which aberrant tyrosine kinase activation occurs; (3) any tumors that proliferate by receptor tyrosine kinases; (4) any tumors that proliferate by aberrant serine/threonine

kinase activation; (5) benign and malignant cells of other proliferative diseases in which aberrant serine/threonine kinase activation occurs, and (6) benign and malignant cells of other proliferative diseases.

The terms "cancer" and "cancerous" refer to or describe the physiological condition in mammals that is typically characterized by unregulated cell growth. A "tumor" comprises one or more cancerous cells. Examples of cancer include, but are not limited to, carcinoma, blastoma, sarcoma, myeloma, leukemia or lymphoid malignancies. The term "cancer" or "cancerous" as defined herein, includes "pre-cancerous" conditions that, if not treated, can evolve into a cancerous condition. In particular embodiments, the cancer is an epithelial cancer. Examples of epithelial cancers include, breast cancer, lung cancer, liver cancer, stomach cancer, head & neck cancer, prostate cancer, pancreatic cancer, ovarian cancer, colon cancer, and kidney cancer. Additional cancers that can be diagnosed or treated with the provided EpCAM antibodies, EpCAM-binding antibody fragments, EpCAM activatable antibodies, or immunoconjugates, include esophageal cancer, tracheal cancer, brain cancer, carcinoma, cholangiocellular cancer, endometrial cancer, cervical cancer, gastric cancer, bladder cancer, uterine cancer, testicular cancer, rectal cancer, skin cancer (melanoma), cancer of the small intestine, gall bladder cancer, cancer of the bile duct, salivary gland cancer. In some embodiments, the cancer is ovarian cancer, uterine cancer, gastric cancers, pancreatic cancer, or colorectal cancer.

The terms "cancer cell," "tumor cell," and grammatical equivalents refer to the total population of cells derived from a tumor or a pre-cancerous lesion, including both non-tumorigenic cells, which comprise the bulk of the tumor cell population, and tumorigenic stem cells (cancer stem cells).

As used herein, the term "cytotoxic agent" refers to a substance that inhibits or prevents one or more cellular functions and/or causes cell death.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, methods and compositions provided herein are useful in attempts to delay development of a disease or disorder.

Terms such as "treating" or "treatment" or "to treat" or "alleviating" or "to alleviate" refer to both 1) therapeutic measures that cure, slow down, lessen symptoms of, and/or halt progression of a diagnosed pathologic condition or disorder and 2) prophylactic or preventative measures that prevent and/or slow the development of a targeted pathologic condition or disorder. Thus, those in need of treatment include those already with the disorder; those prone to have the disorder; and those in whom the disorder is to be prevented. In certain embodiments, a subject is successfully "treated" for cancer according to the methods provided herein if the patient shows one or more of the following: a reduction in the number of or complete absence of cancer cells; a reduction in the tumor size; inhibition of or an absence of cancer cell infiltration into peripheral organs including, for example, the spread of cancer into soft tissue and bone; inhibition of or an absence of tumor metastasis; inhibition or an absence of tumor growth; relief of one or more symptoms associated with the specific cancer; reduced morbidity and mortality; improvement in quality of life; reduction in tumorigenicity, tumorigenic frequency, or tumorigenic capacity, of a tumor; reduction in the number or frequency of cancer stem cells in a tumor; differentiation of tumorigenic cells to a non-tumorigenic state; or some combination of effects.

An "effective amount" of an antibody as disclosed herein is an amount sufficient to carry out a specifically stated purpose. A "therapeutically effective amount" refers to an amount effective, at dosages and for periods of time necessary, to achieve the desired therapeutic result. A "therapeutically effective amount" of a therapeutic agent (*e.g.,* a conjugate or immunoconjugate) may vary according to factors such as the disease state, age, sex, and weight of the individual, and the ability of the antibody to elicit a desired response in the individual. A therapeutically effective amount is also one in which any toxic or detrimental effects of the therapeutic agent are outweighed by the therapeutically beneficial effects.

A "therapeutic agent" encompasses both a biological agent such as an antibody, a peptide, a protein, an enzyme, a chemotherapeutic agent, or a conjugate or immunoconjugate.

The terms "subject," "individual," "animal," "patient," and "mammal," refer to any subject, particularly a mammalian subject, for whom diagnosis, prognosis, or therapy is desired. Mammalian subjects include but are not limited to humans, non-human primates, domestic animals, farm animals, rodents, and the like, which is to be the recipient of a particular treatment.

The terms "polynucleotide" or "nucleic acid", as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide can comprise modified nucleotides, such as methylated nucleotides and their analogs. If present, modification to the nucleotide structure can be imparted before or after assembly of the polymer. The sequence of nucleotides can be interrupted by non-nucleotide components. A polynucleotide can be further modified after polymerization, such as by conjugation with a labeling component. Other types of modifications include, for example, "caps", substitution of one or more of the naturally occurring nucleotides with an analog, internucleotide modifications such as, for example, those with uncharged linkages (*e.g.*, methyl phosphonates, phosphotriesters, phosphoamidates, carbamates,

*etc.*) and with charged linkages (*e.g.,* phosphorothioates, phosphorodithioates, *etc.*), those containing pendant moieties, such as, for example, proteins (*e.g.,* nucleases, toxins, antibodies, signal peptides, ply-L-lysine, *etc.*), those with intercalators (*e.g.,* acridine, psoralen, *etc.*), those containing chelators (*e.g.,* metals, radioactive metals, boron, oxidative metals, *etc.*), those containing alkylators, those with modified linkages (*e.g.,* alpha anomeric nucleic acids, *etc.*), as well as unmodified forms of the polynucleotide(s). Further, any of the hydroxyl groups ordinarily present in the sugars can be replaced, for example, by phosphonate groups, phosphate groups, protected by standard protecting groups, or activated to prepare additional linkages to additional nucleotides, or can be conjugated to solid supports. The 5' and 3' terminal OH can be phosphorylated or substituted with amines or organic capping group moieties of from 1 to 20 carbon atoms. Other hydroxyls can also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2' azido-ribose, carbocyclic sugar analogs, alpha-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages can be replaced by alternative linking groups. These alternative linking groups include, but are not limited to, embodiments, wherein phosphate is replaced by P(O)S ("thioate"), P(S)S ("dithioate"), "(O)NR2 ("amidate"), P(O)R, P(O)OR', CO or CH2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

The term "vector" means a construct, which is capable of delivering, and optionally expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

The terms "polypeptide", "peptide", and "protein" are used interchangeably herein to refer to polymers of amino acids of any length. The polymer can be linear or branched, it can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc.*), as well as other modifications known in the art.

The term "identical" or percent "identity", as known in the art, is a measure of the relationship between two polynucleotides or two polypeptides, as determined by comparing their sequences. Identity or similarity with respect to a sequence is defined herein as the percentage of amino acid residues in the candidate sequence that are identical (*i.e.,* same residue) or similar (*i.e.,* amino acid residue from the same group based on common side-chain properties, see below) to EpCAM antibody residues, after aligning the sequences and introducing gaps, if necessary, to achieve the maximum percent sequence identity. None of N-terminal, C-terminal, or internal extensions, deletions, or insertions into the antibody sequence outside of the variable region shall be construed as affecting sequence identity or similarity. In general, the two sequences to be compared are aligned to give a maximum correlation between the sequences. The alignment of the two sequences is examined and the number of positions giving an exact amino acid or nucleotide correspondence between the two sequences determined, divided by the total length of the alignment and multiplied by 100 to give a % identity figure. This % identity Figure may be determined over the whole length of the sequences to be compared, which is particularly suitable for sequences of the same or very similar length and which are highly homologous, or over shorter defined lengths, which is more suitable for sequences of unequal length or which have a lower level of homology. Likewise percent similarity can be determined in an analogous manner based on the presence of both identical and similar residues.

The percent identity can be measured using sequence comparison software or algorithms or by visual inspection. Various algorithms and software known in the art that can be used to obtain alignments of amino acid or nucleotide sequences. One such non-limiting example of a sequence alignment algorithm is the algorithm described in Karlin et al., Proc. Natl. Acad. Sci. 87:2264-2268 (1990), as modified in Karlin et al., Proc. Natl. Acad. Sci. 90:5873-5877 (1993), and incorporated into the NBLASTand XBLAST programs (Altschul et al., Nucleic Acids Res., 25:3389-3402 (1991)). In certain embodiments, Gapped BLAST can be used as described in Altschul et al., Nucleic Acids Res. 25:3389-3402 (1997). BLAST-2, WU-BLAST-2 (Altschul et al., Meth. Enzym. 266:460-480 (1996)), ALIGN, ALIGN-2 (Genentech, South San Francisco, California) or Megalign (DNASTAR®) are additional publicly available software programs that can be used to align sequences. In certain embodiments, the percent identity between two nucleotide sequences is determined using the GAP program in GCG software (*e.g.*, using a NWSgapdna.CMP matrix and a gap weight of 40, 50, 60, 70, or 90 and a length weight of 1, 2, 3, 4, 5, or 6). In certain alternative embodiments, the GAP program in the GCG software package, which incorporates the algorithm of Needleman and Wunsch (J. Mol. Biol. (48):444-453 (1970)) can be used to determine the percent identity between two amino acid sequences (*e.g.*, using either a Blossum 62 matrix or a PAM250 matrix, and a

gap weight of 16, 14, 12, 10, 8, 6, or 4, and a length weight of 1, 2, 3, 4, 5). Alternatively, in certain embodiments, the percent identity between nucleotide or amino acid sequences is determined using the algorithm of Myers and Miller (CABIOS 4:11-17 (1989)). For example, the percent identity can be determined using the ALIGN program (version 2.0) and using a PAM120 with residue Table, a gap length penalty of 12 and a gap penalty of 4. Appropriate parameters for maximal alignment by particular alignment software can be determined by one skilled in the art. In certain embodiments, the default parameters of the alignment software used. In certain embodiments, the percentage identity "X" of a first amino acid sequence to a second sequence amino acid is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the length of a first sequence is longer than the second sequence, the percent identity of the first sequence to the second sequence will be longer than the percent identity of the second sequence to the first sequence.

As a non-limiting example, whether any particular polynucleotide has a certain percentage sequence identity (*e. g.*, is at least 80% identical, at least 85% identical, at least 90% identical, and in some embodiments, at least 95%, 96%, 97%, 98%, or 99% identical) to a reference sequence can, in certain embodiments, be determined using the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman, Advances in Applied Mathematics 2: 482-489 (1981), to find the best segment of homology between two sequences. When using Bestfit or any other sequence alignment program to determine whether a particular sequence is, for instance, 95% identical to a reference sequence as provided herein, the parameters are set such that the percentage of identity is calculated over the full length of the reference nucleotide sequence and that gaps in homology of up to 5% of the total number of nucleotides in the reference sequence are allowed.

A "conservative amino acid substitution" is one in which one amino acid residue is replaced with another amino acid residue having a similar side chain. Families of amino acid residues having similar side chains have been defined in the art, including, for example, basic side chains (*e.g.,* lysine, arginine, histidine), acidic side chains (*e.g.,* aspartic acid, glutamic acid), uncharged polar side chains (*e.g.*, glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.*, threonine, valine, isoleucine) and aromatic side chains (*e.g.*, tyrosine, phenylalanine, tryptophan, histidine). For example, substitution of a phenylalanine for a tyrosine is a conservative substitution. In some embodiments, conservative substitutions in the sequences of the polypeptides and antibodies provided herein do not abrogate the binding of the polypeptide or antibody containing the amino acid sequence, to the antigen(s), to which the polypeptide or antibody binds. Methods of identifying nucleotide and amino acid conservative substitutions which do not eliminate antigen binding are well- known in the art (*see, e.g.,* Brummell et al., Biochem. 32:1180-1187 (1993); Kobayashi et al., Protein Eng. 12(10):879-884 (1999); and Burks et al., Proc. Natl. Acad. Sci. USA 94:412-417 (1997)).

"Alkyl" as used herein refers to a saturated linear or branched-chain monovalent hydrocarbon radical of one to twenty carbon atoms. Examples of alkyl include, but are not limited to, methyl, ethyl, 1-propyl, 2-propyl, 1-butyl, 2-methyl-1-propyl, -CH2CH(CH3)2), 2 butyl, 2-methyl-2-propyl, 1-pentyl, 2-pentyl 3-pentyl, 2-methyl-2-butyl, 3-methyl-2-butyl, 3 methyl-1-butyl, 2-methyl-1-butyl, 1-hexyl), 2-hexyl, 3-hexyl, 2-methyl-2-pentyl, 3-methyl-2 pentyl, 4-methyl-2-pentyl, 3-methyl-3-pentyl, 2-methyl-3-pentyl, 2,3-dimethyl-2-butyl, 3,3 dimethyl-2-butyl, 1-heptyl, 1-octyl, and the like. Preferably, the alkyl has one to ten carbon atoms. More preferably, the alkyl has one to four carbon atoms.

The number of carbon atoms in a group can be specified herein by the prefix "Cx-xx", wherein x and xx are integers. For example, "C1-4alkyl" is an alkyl group having from 1 to 4 carbon atoms.

The term "compound" or "cytotoxic compound," or "cytotoxic agent" are used interchangeably. They are intended to include compounds for which a structure or formula or any derivative thereof has been disclosed herein or a structure or formula or any derivative thereof that has been incorporated by reference. The term also includes, stereoisomers, geometric isomers, tautomers, solvates, metabolites, and salts (*e.g.*, pharmaceutically acceptable salts) of a compound of all the formulae disclosed herein. The term also includes any solvates, hydrates, and polymorphs of any of the foregoing. The specific recitation of "stereoisomers," "geometric isomers," "tautomers," "solvates," "metabolites," "salt", "conjugates," "conjugates salt," "solvate," "hydrate," or "polymorph" in certain embodiments, provided herein shall not be interpreted as an intended omission of these forms in other disclosed embodiments, where the term "compound" is used without recitation of these other forms.

The term "imine reactive reagent" refers to a reagent that is capable of reacting with an imine group. Examples of imine reactive reagent includes, but is not limited to, sulfites (H2SO3, H2SO2 or a salt of HSO3-, SO32- or HSO2- formed with a cation), metabisulfite (H2S2O5 or a salt of S2O52- formed with a cation), mono, di, tri, and tetra- thiophosphates (PO3SH3, PO2S2H3, POS3H3, PS4H3 or a salt of PO3S3-, PO2S23-, POS33- or PS43-formed with a cation), thio phosphate esters ((RiO)2PS(ORi), RiSH, RiSOH, RiSO2H, RiSO3H), various amines (hydroxyl amine (*e.g.,* NH2OH), hydrazine (*e.g.,* NH2NH2), NH2O-Ri, Ri'NH-Ri, NH2-Ri), NH2-CO-NH2, NH2-C(=S)-NH2, thiosulfate (H2S2O3 or a salt of S2O32- formed with a cation), dithionite (H2S2O4 or a salt of S2O42- formed with a cation), phosphorodithioate (P(=S)(ORk)(SH)(OH) or a salt thereof formed with a cation), hydroxamic acid (RkC(=O)NHOH or a salt formed with a cation),

hydrazide (RkCONHNH2), formaldehyde sulfoxylate (HOCH2SO2H or a salt of HOCH2SO2- formed with a cation, such as HOCH2SO2-Na+), glycated nucleotide (such as GDP-mannose), fludarabine or a mixture thereof, wherein Ri and Ri' are each independently a linear or branched alkyl having 1 to 10 carbon atoms and are substituted with at least one substituent selected from N(Rj)2, -CO2H, SO3H, and PO3H; Ri and Ri' can be further optionally substituted with a substituent for an alkyl disclosed herein; Rj is a linear or branched alkyl having 1 to 6 carbon atoms; and Rk is a linear, branched or cyclic alkyl, alkenyl or alkynyl having 1 to 10 carbon atoms, aryl, heterocyclyl or heteroaryl (preferably, Rk is a linear or branched alkyl having 1 to 4 carbon atoms; more preferably, Rk is methyl, ethyl or propyl). Preferably, the cation is a monovalent cation, such as Na+ or K+. Preferably, the imine reactive reagent is selected from sulfites, hydroxyl amine, urea and hydrazine. More preferably, the imine reactive reagent is NaHSO3 or KHSO3.

The term "cation" refers to an ion with positive charge. The cation can be monovalent (*e.g.,* Na+, K+, NH4+ *etc.*), bi-valent (*e.g.,* Ca2+, Mg2+, *etc.*) or multi-valent (*e.g.,* Al3+ *etc.*). Preferably, the cation is monovalent.

The phrase "pharmaceutically acceptable salt" as used herein, refers to pharmaceutically acceptable organic or inorganic salts of a compound provided herein. Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate "mesylate," ethanesulfonate, benzenesulfonate, p-toluenesulfonate, pamoate (*i.e.,* 1,1'-methylene-bis-(2-hydroxy-3-naphthoate)) salts, alkali metal (*e.g.,* sodium and potassium) salts, alkaline earth metal (*e.g.*, magnesium) salts, and ammonium salts. A pharmaceutically acceptable salt can involve the inclusion of another molecule such as an acetate ion, a succinate ion or other counter ion. The counter ion can be any organic or inorganic moiety that stabilizes the charge on the parent compound. Furthermore, a pharmaceutically acceptable salt can have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

If the compound provided herein is a base, the desired pharmaceutically acceptable salt can be prepared by any suitable method available in the art, for example, treatment of the free base with an inorganic acid, such as hydrochloric acid, hydrobromic acid, sulfuric acid, nitric acid, methanesulfonic acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like.

If the compound provided herein is an acid, the desired pharmaceutically acceptable salt can be prepared by any suitable method, for example, treatment of the free acid with an inorganic or organic base, such as an amine (primary, secondary or tertiary), an alkali metal hydroxide or alkaline earth metal hydroxide, or the like. Illustrative Examples of suitable salts include, but are not limited to, organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium.

As used herein, the term "solvate" means a compound that further includes a stoichiometric or non-stoichiometric amount of solvent such as water, isopropanol, acetone, ethanol, methanol, DMSO, ethyl acetate, acetic acid, and ethanolamine dichloromethane, 2 propanol, or the like, bound by non-covalent intermolecular forces. Solvates or hydrates of the compounds are readily prepared by addition of at least one molar equivalent of a hydroxylic solvent such as methanol, ethanol, 1-propanol, 2-propanol or water to the compound to result in solvation or hydration of the imine moiety.

A "metabolite" or "catabolite" is a product produced through metabolism or catabolism in the body of a specified compound, a derivative thereof, or a conjugate thereof, or salt thereof. Metabolites of a compound, a derivative thereof, or a conjugate thereof, can be identified using routine techniques known in the art and their activities determined using tests such as those disclosed herein. Such products can result for example from the oxidation, hydroxylation, reduction, hydrolysis, amidation, deamidation, esterification, deesterification, enzymatic cleavage, and the like, of the administered compound. Accordingly, the disclosure includes metabolites of compounds, a derivative thereof, or a conjugate thereof, of the disclosed EpCAM compositions disclosed herein, including compounds, derivatives thereof, or conjugates thereof, produced by a process comprising contacting a disclosed EpCAM compound, a derivative thereof, or a conjugate thereof, with a mammal for a period of time sufficient to yield a metabolic product thereof.

The phrase "pharmaceutically acceptable" indicates that the substance or composition must be compatible chemically and/or toxicologically, with the other ingredients comprising a formulation, and/or the mammal being treated therewith.

The term "protecting group" or "protecting moiety" refers to a substituent that is commonly employed to block or protect a particular functionality while reacting other functional groups on the compound, a derivative thereof, or a conjugate thereof. For example, an "amine-protecting group" or an "amino-protecting moiety" is a substituent attached to an amino group that blocks or protects the amino functionality in the compound. Such groups are well known in the art (*see,* for example, P. Wuts and T. Greene, 2007, Protective Groups in Organic Synthesis, Chapter 7, J. Wiley & Sons, NJ) and

exemplified by carbamates such as methyl and ethyl carbamate, FMOC, substituted ethyl carbamates, carbamates cleaved by 1,6-β-elimination (also termed "self immolative"), ureas, amides, peptides, alkyl and aryl derivatives. Suitable amino-protecting groups include acetyl, trifluoroacetyl, t-butoxycarbonyl (BOC), benzyloxycarbonyl (CBZ) and 9-fluorenylmethylenoxycarbonyl (Fmoc). For a general description of protecting groups and their use, see P. G.M. Wuts & T. W. Greene, Protective Groups in Organic Synthesis, John Wiley & Sons, New York, 2007.

The term "amino acid" refers to naturally occurring amino acids or non-naturally occurring amino acid. In one embodiment, the amino acid is represented by NH2-C(Raa'Raa)-C(=O)OH, wherein Raa and Raa' are each independently H, an optionally substituted linear, branched or cyclic alkyl, alkenyl or alkynyl having 1 to 10 carbon atoms, aryl, heteroaryl or heterocyclyl or Raa and the N-terminal nitrogen atom can together form a heteroycyclic ring (*e.g.,* as in proline). The term "amino acid residue" refers to the corresponding residue when one hydrogen atom is removed from the amine and/or carboxy end of the amino acid, such as -NH-C(Raa'Raa)-C(=O)O-.

The term "peptide" refers to short chains of amino acid monomers linked bypeptide (amide) bonds. In some embodiments, the peptides contain 2 to 20 amino acid residues. In other embodiments, the peptides contain 2 to 10 amino acid residues. In yet other embodiments, the peptides contain 2 to 5 amino acid residues. As used herein, when a peptide is a portion of a cytotoxic agent or a linker disclosed herein represented by a specific sequence of amino acids, the peptide can be connected to the rest of the cytotoxic agent or the linker in both directions. For example, a dipeptide X1-X2 includes X1-X2 and X2-X1. Similarly, a tripeptide X1-X2-X3 includes X1-X2-X3 and X3-X2-X1 and a tetrapeptide X1-X2-X3-X4 includes X1-X2-X3-X4 and X4-X2-X3-X1. X1, X2, X3 and X4 represents an amino acid residue.

The term "reactive ester group" refers to a group an ester group that can readily react with an amine group to form amide bond. Exemplary reactive ester groups include, but are not limited to, N-hydroxysuccinimide esters, N-hydroxyphthalimide esters, N-hydroxy sulfo-succinimide esters, para-nitrophenyl esters, dinitrophenyl esters, pentafluorophenyl esters and their derivatives, wherein the derivatives facilitate amide bond formation. In certain embodiments, the reactive ester group is a N-hydroxysuccinimide ester or a N hydroxy sulfo-succinimide ester.

The term "amine reactive group" refers to a group that can react with an amine group to form a covalent bond. Exemplary amine reactive groups include, but are not limited to, reactive ester groups, acyl halides, sulfonyl halide, imidoester, or a reactive thioester groups. In certain embodiments, the amine reactive group is a reactive ester group. In one embodiment, the amine reactive group is a N-hydroxysuccinimide ester or a N-hydroxy sulfo-succinimide ester.

The term "thiol-reactive group" refers to a group that can react with a thiol (-SH) group to form a covalent bond. Exemplary thiol-reactive groups include, but are not limited to, maleimide, haloacetyl, aloacetamide, vinyl sulfone, vinyl sulfonamide or vinyal pyridine. In one embodiment, the thiol-reactive group is maleimide.

As used in the present disclosure and claims, the singular forms "a," "an," and "the" include plural forms unless the context clearly dictates otherwise.

The term "and/or" where used herein is to be taken as specific disclosure of each of the two or more specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Wherever embodiments, are disclosed herein with the language "comprising," otherwise analogous embodiments, described in terms of "consisting of" and/or "consisting essentially of" are also provided.

## EpCAM Antibodies and EpCAM-Binding Antibody fragments

Proteins that specifically bind human EpCAM are provided. In some embodiments, the proteins are referred to herein as "EpCAM-binding agents" or EpBAs."

In additional embodiments, the EpCAM-binding agent is an EpCAM antibody, an EpCAM-binding antibody fragment, or an EpCAM activatable antibody. In some embodiments, the EpBA is a full-length EpCAM antibody (*i.e.,* a full-length antibody that specifically binds EpCAM). In some embodiments, the EpCAM antibody is a monoclonal antibody. In some embodiments, the EpCAM antibody is a recombinant antibody, a human antibody, a humanized antibody, a chimeric antibody, a multi-specific antibody (*e.g.,* a bi-specific antibody), or an EpCAM-binding antibody fragment thereof. In some embodiments, the EpCAM antibody specifically binds human EpCAM. In further embodiments, the EpCAM antibody specifically binds human EpCAM and cyno EpCAM. In some embodiments, the EpCAM antibody or EpCAM-binding antibody fragment is a mouse, other rodent, chimeric, humanized or fully human monoclonal antibody.

In some embodiments, the EpCAM antibody is an EpCAM-binding antibody fragment. In additional embodiments, the EpCAM-binding antibody fragment is a: Fab, Fab', $F(ab')_2$, Fv fragment, diabody, or single chain antibody molecule. In additional aspects, the EpCAM antibody is an EpCAM-binding antibody fragment is a Fd, single chain Fv (scFv), disulfide linked Fv, V-NAR domain, IgNar, intrabody, IgGΔCH2, minibody, $F(ab')_3$, scAb, dAb, tetrabody, triabody, diabody, single-domain heavy chain antibody, single-domain light chain antibody, DVD-Ig, Fcab, $mAb^2$, $(scFv)_2$, scFv-Fc or bis-scFv.

The EpCAM antibodies and EpCAM-binding antibody fragments provided herein optionally bind EpCAM (*e.g.*, human EpCAM and/or murine EpCAM), with a wide range of affinities ($K_D$). In a preferred embodiment, the antibody binds human EpCAM with high affinity. For example, a human or human engineered or humanized or resurfaced mAb can bind human antigen with a $K_D$ equal to or less than about $10^{-7}$ M, such as but not limited to, 0.1-9.9 (or any range or value therein between) x $10^{-7}$, $10^{-8}$, $10^{-9}$, $10^{-10}$, $10^{-11}$, or $10^{-12}$, or any range or value therein, as determined by flow cytometry base assays, enzyme-linked immunoabsorbent assay (ELISA), surface plasmon resonance (SPR) or the KinExA® method using standard operating procedures. In some embodiments, the EpCAM antibodies bind with a Kd of about $10^{-9}$ M or less, more specifically about $10^{-9}$ to $10^{-10}$ M.

The affinity or avidity of an antibody or antibody fragment for EpCAM can be determined experimentally using any suitable method known in the art, *e.g.,* flow cytometry, enzyme-linked immunoabsorbent assay (ELISA), or radioimmunoassay (RIA), or kinetics (*e.g.*, BIACORE™ analysis), using standard operating procedures. Direct binding assays as well as competitive binding assay formats can be routinely employed. (*see, e.g.,* Berzofsky, et al., "Antibody-Antigen Interactions," In Fundamental Immunology, Paul, W. E., Ed., Raven Press: New York, N.Y. (1984); Kuby, Janis Immunology, W. H. Freeman and Company: New York, N.Y. (1992); and methods disclosed herein. The measured affinity of a particular antibody-EpCAM interaction can vary if measured under different conditions (*e.g.*, salt concentration, pH, temperature). Thus, measurements of affinity and other EpCAM-binding parameters (*e.g.*, KD or Kd, Kon, Koff) are preferably made with standardized solutions of antibody and EpCAM, and a standardized buffer, as known in the art and such as the buffer disclosed herein.

In one embodiment, binding assays are performed using flow cytometry on cells expressing the EpCAM antigen on the surface. For example, such EpCAM-positive cells are incubated with varying concentrations of EpCAM antibodies using 1 x$10^5$ cells per sample in 100 μL FACS buffer (RPMI-1640 medium supplemented with 2% normal goat serum). Then, the cells are pelleted, washed, and incubated for 1 h with 100 μL of FITC-conjugated goat antimouse IgG-antibody (such as obtainable from Jackson ImmunoResearch) in FACS buffer. The cells are pelleted again, washed with FACS buffer and resuspended in 200 μL of PBS containing 1% formaldehyde. Samples are acquired, for example, using a FACSCalibur™ flow cytometer with the HTS multiwell sampler and analyzed using CellQuest® Pro (all from BD Biosciences, San Diego, US). For each sample the mean fluorescence intensity for FL1 (MFI) is exported and plotted against the antibody concentration in a semi-log plot to generate a binding curve. A sigmoidal dose-response curve is fitted for binding curves and $EC_{50}$ values are calculated using programs such as GraphPad Prism v4 with default parameters (GraphPad software, San Diego, CA). $EC_{50}$ values can be used as a measure for the apparent dissociation constant "Kd" or "KD" for each antibody.

In certain embodiments, the EpCAM antibodies are modified to alter their binding affinity for EpCAM and/or EpCAM antigenic fragments. Binding properties may be determined by a variety of *in vitro* assay methods and employing standard operating procedures known in the art, including for example, enzyme-linked immunoabsorbent assay (ELISA), radioimmunoassay (RIA)), or kinetics (*e.g.,* BIACORE™ analysis).

In one embodiment, the EpCAM antibody or EpCAM-binding antibody fragment specifically binds human or cynomolgus EpCAM and/or EpCAM antigenic fragments with a dissociation constant or KD or Kd (koff/kon) of less than $10^{-5}$ M, or of less than $10^{-6}$ M, or of less than $10^{-7}$ M, or of less than $10^{-8}$ M, or of less than $10^{-9}$ M, or of less than $10^{-10}$ M, or of less than $10^{-11}$ M, or of less than $10^{-12}$ M, or of less than $10^{-13}$ M. In some embodiments, the EpCAM antibody or EpCAM-binding antibody fragment specifically binds human or cynomolgus EpCAM and/or EpCAM antigenic fragments with a KD of 1.0 x $10^{-9}$ M or less, 2.0 x $10^{-9}$ M or less, 3.0 x $10^{-9}$ M or less, 4.0 x $10^{-9}$ M or less, 5.0 x $10^{-9}$ M or less, 6.0 x $10^{-9}$ M or less, 7.0 x $10^{-9}$ M or less, 8.0 x $10^{-9}$ M or less, or 9.0 x $10^{-9}$ M or less. In certain embodiments, the EpCAM antibody or EpCAM-binding antibody fragment binds to both human and cynomolgus EpCAM and/or EpCAM antigenic fragments with a KD of 3.0 x $10^{-9}$ M or less. In some embodiments, the EpCAM antibody or EpCAM-binding antibody fragment binds to human EpCAM with a KD of about 0.4 x $10^{-9}$. In some embodiments, the EpCAM antibody or EpCAM-binding antibody fragment binds to human EpCAM with a KD of about 0.8 x $10^{-9}$. In some embodiments, the EpCAM antibody or EpCAM-binding antibody fragment binds to cynomolgus EpCAM with a KD of about 0.8 x $10^{-9}$. In some embodiments, the EpCAM antibody or EpCAM-binding antibody fragment binds to cynomolgus EpCAM with a KD of about 2.2 x $10^{-9}$. In some embodiments, the EpCAM antibody or EpCAM-binding antibody fragment binds to cynomolgus EpCAM with a KD of about 2.8 x $10^{-9}$.

In some embodiments, the EpCAM antibody or EpCAM-binding antibody fragment specifically binds to an epitope within the extracellular region of human EpCAM (SEQ ID NO:1). The extracellular region of human EpCAM may be further divided into three distinct domains: D1 (SEQ ID NO:2), D2 (SEQ ID NO:3), and D3 (SEQ ID NO:4). In certain embodiments, the EpCAM antibody or EpCAM-binding antibody fragment specifically binds to an epitope within the first extracellular domain (D1) of human EpCAM.

In one embodiment, the EpCAM antibody or EpCAM-binding antibody fragment comprises a VH-CDR1 comprising $X_1YX_3X_4H$, wherein $X_1$ is selected from N and S, $X_3$ is selected from Y, N, F, S, H, D, L, I, and W, and $X_4$ is selected from I and M (SEQ ID NO:5); a VH-CDR2 comprising $WX_2X_3PGX_6VYIQYX_{12}X_{13}KFX_{17}G$, wherein $X_2$ is selected from I and F, $X_3$ is selected from Y and N, $X_6$ is selected from N and D, $X_{12}$ is selected from N and S, $X_{13}$ is selected from E and Q, and $X_{17}$ is selected from K and Q (SEQ ID NO:7); and a VH-CDR3 comprising $X_1GX_3X_4FAY$, wherein $X_1$ is

selected from D and E, $X_3$ is selected from P, A, S, Y, F, G, T, and V, and $X_4$ is selected from Y and W (SEQ ID NO:8).

In additional embodiments, the disclosure provides an EpCAM antibody or EpCAM-binding antibody fragment, comprising a light chain CDR1 (VL-CDR1) comprising RSS$X_4$SLLHS$X_{10}$G$X_{12}$TYL$X_{16}$, wherein $X_4$ is selected from R and K, $X_{10}$ is selected from N and D, $X_{12}$ is selected from F and I, and $X_{16}$ is selected from Y and S (SEQ ID NO:10); a light chain VL-CDR2 comprising QTSNLAS (SEQ ID NO:40); and a VL-CDR3 comprising $X_1$Q$X_3$LELP$X_8$T, wherein $X_1$ is selected from A, L, and Q, $X_3$ is selected from S, G, Y, and N, and $X_8$ is selected from N and W (SEQ ID NO:11). In some embodiments, the disclosure provides an EpCAM antibody or EpCAM-binding antibody fragment comprising a heavy chain CDR1 (VH-CDR1) comprising the sequence of SEQ ID NO:13; a heavy chain CDR2 (VH-CDR2) comprising the sequence of SEQ ID NO:14; a heavy chain CDR3 (VH-CDR3) comprising the sequence of SEQ ID NO:15; a light chain CDR1 (VL-CDR1) comprising the sequence of SEQ ID NO:42; a light chain CDR2 (VL-CDR2) comprising the sequence of SEQ ID NO:40; and a light chain CDR3 (VL-CDR3) comprising the sequence of SEQ ID NO:41.

In some embodiments, the VH-CDR1 comprises the sequence NY$X_3$IH, wherein $X_3$ is selected from Y, N, F, S, H, D, L, I, and W (SEQ ID NO:6). In some embodiments, the VH-CDR3 comprises the sequence DGP$X_4$FAY, wherein $X_4$ is selected from Y and W (SEQ ID NO:9). In some embodiments, the VL-CDR3 comprises the sequence AQ$X_3$LELPNT, wherein $X_3$ is selected from S, G, Y, and N (SEQ ID NO:12). In some embodiments, the disclosure provides an EpCAM antibody or EpCAM-binding antibody fragment comprising a heavy chain CDR1 (VH-CDR1) comprising the sequence of SEQ ID NO:13; a heavy chain CDR2 (VH-CDR2) comprising the sequence of SEQ ID NO:14; a heavy chain CDR3 (VH-CDR3) comprising the sequence of SEQ ID NO:15; a light chain CDR1 (VL-CDR1) comprising the sequence of SEQ ID NO:42; a light chain CDR2 (VL-CDR2) comprising the sequence of SEQ ID NO:40; and a light chain CDR3 (VL-CDR3) comprising the sequence of SEQ ID NO:41.

In some embodiments, the EpCAM antibody or EpCAM-binding antibody fragment comprises a set of complementarity determining regions (CDRs): heavy chain variable region (VH)-CDR1, VH-CDR2, VH-CDR3, light chain variable region (VL) CDR1, VL-CDR2 and VL-CDR3, wherein the heavy chain CDRs are disclosed in Table 2.

**Table 2. Exemplary heavy chain CDR sequences of EpCAM antibodies.**

| | VH- CDR1 | VH-CDR2 | VH-CDR3 |
|---|---|---|---|
| **Murine and Chimeric** | | | |
| **muEpcam23** | NYYIH (SEQ ID NO:13) | WIYPGNVYIQYNEKFKG (SEQ ID NO:14) | DGPWFAY (SEQ ID NO:15) |
| **chEpcam23** | NYYIH (SEQ ID NO:13) | WIYPGNVYIQYNEKFKG (SEQ ID NO:14) | DGPWFAY (SEQ ID NO:15) |
| **Humanized Variants** | | | |
| | **VH- CDR1** | **VH-CDR2** | **VH-CDR3** |
| **huEpCAM23_VHG v1** | NYYIH (SEQ ID NO:13) | WIYPGNVYIQYSQKFQG (SEQ ID NO:26) | DGPWFAY (SEQ ID NO:15) |
| **huEpCAM23_VHG v2** | NYYIH (SEQ ID NO:13) | WIYPGNVYIQYNEKFKG (SEQ ID NO:14) | DGPWFAY (SEQ ID NO:15) |
| **huEpCAM23_VHG v3** | NYYIH (SEQ ID NO:13) | WIYPGNVYIQYNEKFKG (SEQ ID NO:14) | DGPWFAY (SEQ ID NO:15) |
| **huEpCAM23_VHG v4** | NYYIH (SEQ ID NO:13) | WIYPGNVYIQYSQKFQG (SEQ ID NO:26) | DGPWFAY (SEQ ID NO:15) |
| **Affinity Variants** | | | |
| **huEpCAM23HCGv 2a** | NYYIH (SEQ ID NO:13) | WIYPGNVYIQYNEKFKG (SEQ ID NO:14) | DGPWFAY (SEQ ID NO:15) |
| **huEpCAM23HCGv 2b** | SYYIH (SEQ ID NO:16) | WIYPGNVYIQYNEKFKG (SEQ ID NO:14) | DGPWFAY (SEQ ID NO:15) |
| **huEpCAM23HCGv 2c** | NYNIH (SEQ ID NO:17) | WIYPGNVYIQYNEKFKG (SEQ ID NO:14) | DGPWFAY (SEQ ID NO:15) |
| **huEpCAM23HCGv 2d** | NYYIH (SEQ ID NO:13) | WIYPGDVYIQYNEKFKG (SEQ ID NO:27) | DGPWFAY (SEQ ID NO:15) |

(continued)

| Affinity Variants | | | |
|---|---|---|---|
| **huEpCAM23HCGv 2e** | NYFIH (SEQ ID NO:18) | WIYPGNVYIQYNEKFKG (SEQ ID NO:14) | DGPWFAY (SEQ ID NO:15) |
| **huEpCAM23HCGv 2f** | NYSIH (SEQ ID NO:19) | WIYPGNVYIQYNEKFKG (SEQ ID NO:14) | DGPWFAY (SEQ ID NO:15) |
| **huEpCAM23HCGv 2g** | NYWIH (SEQ ID NO:20) | WIYPGNVYIQYNEKFKG (SEQ ID NO:14) | DGPWFAY (SEQ ID NO:15) |
| **huEpCAM23HCGv 2h** | NYYIH (SEQ ID NO:13) | WFYPGNVYIQYNEKFKG (SEQ ID NO:28) | DGPWFAY (SEQ ID NO:15) |
| **huEpCAM23HCGv 2i/2o** | NYYIH (SEQ ID NO:13) | WIYPGNVYIQYNEKFKG (SEQ ID NO:14) | DGPWFAY (SEQ ID NO:15) |
| **huEpCAM23HCGv 2j** | NYYIH (SEQ ID NO:13) | WINPGNVYIQYNEKFKG (SEQ ID NO:29) | DGPWFAY (SEQ ID NO:15) |
| **huEpCAM23HCGv 2k** | NYYIH (SEQ ID NO:13) | WIYPGNVYIQYNEKFKG (SEQ ID NO:14) | EGPWFAY (SEQ ID NO:30) |
| **huEpCAM23HCGv 2l** | NYYIH (SEQ ID NO:13) | WIYPGNVYIQYNEKFKG (SEQ ID NO:14) | DGPYFAY (SEQ ID NO:31) |
| **huEpCAM23HCGv2 m/ huEp-Cam23 HG2-1565-A Heavy Chain** | NYYIH (SEQ ID NO:13) | WIYPGNVYIQYNEKFKG (SEQ ID NO:14) | DGAWFAY (SEQ ID NO:32) |
| **huEpCAM23HCGv 2n** | NYYMH (SEQ ID NO:21) | WIYPGNVYIQYNEKFKG (SEQ ID NO:14) | DGPWFAY (SEQ ID NO:15) |
| **huEpCAM23HCGv 2p** | NYYIH (SEQ ID NO:13) | WIYPGNVYIQYNEKFKG (SEQ ID NO:14) | DGPWFAY (SEQ ID NO:15) |
| **huEpCAM23HCGv 2q** | NYYIH (SEQ ID NO:13) | WIYPGNVYIQYNEKFKG (SEQ ID NO:14) | DGPWFAY (SEQ ID NO:15) |
| **huEpCAM23HCGv 2r** | NYYIH (SEQ ID NO:13) | WIYPGNVYIQYNEKFKG (SEQ ID NO:14) | DGPWFAY (SEQ ID NO:15) |
| | **VH- CDR1** | **VH-CDR2** | **VH-CDR3** |
| **huEpCAM23HCGv 2s** | NYYIH (SEQ ID NO:13) | WIYPGNVYIQYNEKFKG (SEQ ID NO:14) | DGPWFAY (SEQ ID NO:15) |
| **huEpCam23HG2-1361-H Heavy Chain** | NYHIH (SEQ ID NO:22) | WIYPGNVYIQYNEKFKG (SEQ ID NO:14) | DGPWFAY (SEQ ID NO:15) |
| **huEpCam23HG2-1361-D Heavy Chain** | NYDIH (SEQ ID NO:23) | WIYPGNVYIQYNEKFKG (SEQ ID NO:14) | DGPWFAY (SEQ ID NO:15) |
| **huEpCam23HG2-1565-Y Heavy Chain** | NYYIH (SEQ ID NO:13) | WIYPGNVYIQYNEKFKG (SEQ ID NO:14) | DGYWFAY (SEQ ID NO:33) |
| **huEpCam23HG2-1565-S Heavy Chain** | NYYIH (SEQ ID NO:13) | WIYPGNVYIQYNEKFKG (SEQ ID NO:14) | DGSWFAY (SEQ ID NO:34) |
| **huEpCam23HG2-1565-F Heavy Chain** | NYYIH (SEQ ID NO:13) | WIYPGNVYIQYNEKFKG (SEQ ID NO:14) | DGFWFAY (SEQ ID NO:35) |
| **huEpCam23HG2-1565-G Heavy Chain** | NYYIH (SEQ ID NO:13) | WIYPGNVYIQYNEKFKG (SEQ ID NO:14) | DGGWFAY (SEQ ID NO:36) |
| **huEpCam23HG2-1565-T Heavy Chain** | NYYIH (SEQ ID NO:13) | WIYPGNVYIQYNEKFKG (SEQ ID NO:14) | DGTWFAY (SEQ ID NO:37) |
| **huEpCam23HG2-1565-V Heavy Chain** | NYYIH (SEQ ID NO:13) | WIYPGNVYIQYNEKFKG (SEQ ID NO:14) | DGVWFAY (SEQ ID NO:38) |

(continued)

| | VH- CDR1 | VH-CDR2 | VH-CDR3 |
|---|---|---|---|
| **huEpCam23HG2-1361-I Heavy Chain** | NYIIH (SEQ ID NO:24) | WIYPGNVYIQYNEKFKG (SEQ ID NO:14) | DGPWFAY (SEQ ID NO:15) |
| **huEpCam23HG2-1361-L Heavy Chain** | NYLIH (SEQ ID NO:25) | WIYPGNVYIQYNEKFKG (SEQ ID NO:14) | DGPWFAY (SEQ ID NO:15) |

In some embodiments, the disclosure provides an EpCAM antibody or EpCAM-binding antibody fragment comprising the heavy chain CDRs of a single row in Table 2. In some embodiments, the disclosure provides an EpCAM antibody or EpCAM-binding antibody fragment comprising a VH-CDR1 selected from SEQ ID NOs:13, and 16-25; a VH-CDR2 slected from SEQ ID NOs:14, and 26-29; and a VH-CDR3 selected from SEQ ID NOs:15, and 30-38. In some embodiments, the disclosure provides an EpCAM antibody or EpCAM-binding antibody fragment comprising a VH-CDR1 of SEQ ID NO:13; a VH-CDR2 of SEQ ID NO:14; and a VH-CDR3 of SEQ ID NO: 15. In some embodiments, the disclosure provides an EpCAM antibody or EpCAM-binding antibody fragment comprising a VH-CDR1 of SEQ ID NO:13; a VH-CDR2 of SEQ ID NO:26; and a VH-CDR3 of SEQ ID NO:15.

In one embodiment, the disclosure provides an EpCAM antibody, EpCAM-binding antibody fragment, or EpCAM activatable antibody comprising, a VH-CDR1 comprising NYYIH (SEQ ID NO:13), or a variant thereof comprising 1, 2, 3, or 4, conservative amino acid substitutions; a VH-CDR2 comprising WIYPGNVYIQYNEKFKG (SEQ ID NO:14), or a variant thereof comprising 1, 2, 3, or 4, amino conservative acid substitutions; and a heavy chain CDR3 comprising DGPWFAY (SEQ ID NO: 15), or a variant thereof comprising 1, 2, 3, or 4, conservative amino acid substitutions.

In some embodiments, the disclosure provides an EpCAM antibody or EpCAM-binding antibody fragment comprising a VH-CDR1 of SEQ ID NO:22; a VH-CDR2 of SEQ ID NO:14; and a VH-CDR3 of SEQ ID NO: 15. In some embodiments, the disclosure provides an EpCAM antibody or EpCAM-binding antibody fragment comprising a VH-CDR1 of SEQ ID NO:13; a VH-CDR2 of SEQ ID NO:14; and a VH-CDR3 of SEQ ID NO:33. In some embodiments, the disclosure provides an EpCAM antibody or EpCAM-binding antibody fragment comprising a VH-CDR1 of SEQ ID NO:23; a VH-CDR2 of SEQ ID NO:14; and a VH-CDR3 of SEQ ID NO: 15. In some embodiments, the disclosure provides an EpCAM antibody or EpCAM-binding antibody fragment comprising a VH-CDR1 of SEQ ID NO:25; a VH-CDR2 of SEQ ID NO: 14; and a VH-CDR3 of SEQ ID NO: 15.

In one embodiment, the disclosure provides an EpCAM antibody, EpCAM-binding antibody fragment, or EpCAM activatable antibody comprising, a VH-CDR1 comprising NYHIH (SEQ ID NO:22), or a variant thereof comprising 1, 2, 3, or 4, conservative amino acid substitutions; a VH-CDR2 comprising WIYPGNVYIQYNEKFKG (SEQ ID NO:14), or a variant thereof comprising 1, 2, 3, or 4, amino conservative acid substitutions; and a heavy chain CDR3 comprising DGPWFAY (SEQ ID NO: 15), or a variant thereof comprising 1, 2, 3, or 4, conservative amino acid substitutions. In one embodiment, the disclosure provides an EpCAM antibody, EpCAM-binding antibody fragment, or EpCAM activatable antibody comprising, a VH-CDR1 comprising NYYIH (SEQ ID NO: 13), or a variant thereof comprising 1, 2, 3, or 4, conservative amino acid substitutions; a VH-CDR2 comprising WIYPGNVYIQYNEKFKG (SEQ ID NO:14), or a variant thereof comprising 1, 2, 3, or 4, amino conservative acid substitutions; and a heavy chain CDR3 comprising DGYWFAY (SEQ ID NO:33), or a variant thereof comprising 1, 2, 3, or 4, conservative amino acid substitutions.

In some embodiments, the EpCAM antibody or EpCAM-binding antibody fragment comprises a set of complementarity determining regions (CDRs): heavy chain variable region (VH)-CDR1, VH-CDR2, VH-CDR3, light chain variable region (VL) CDR1, VL-CDR2 and VL-CDR3, wherein the light chain CDRs are disclosed in Table 3.

**Table 3. Exemplary light chain CDR sequences of EpCAM antibodies.**

| Antibody | VL- CDR1 | VL-CDR2 | VL-CDR3 |
|---|---|---|---|
| **Murine and Chimeric** | | | |
| **muEpcam23** | RSSKSLLHSDGFTYLY (SEQ ID NO:39) | QTSNLAS (SEQ ID NO:40) | AQNLELPNT (SEQ ID NO:41) |
| **chEpcam23** | RSSKSLLHSDGFTYLY (SEQ ID NO:39) | QTSNLAS (SEQ ID NO:40) | AQNLELPNT (SEQ ID NO:41) |
| **Humanized Variants** | | | |
| **huEpCAM23_V LG v1** | RSSRSLLHSDGFTYLY (SEQ ID NO:42) | QTSNLAS (SEQ ID NO:40) | AQNLELPNT (SEQ ID NO:41) |

(continued)

| Humanized Variants | | | |
|---|---|---|---|
| **huEpCAM23_V LG v2** | RSSKSLLHSDGFTYLY (SEQ ID NO:39) | QTSNLAS (SEQ ID NO:40) | AQNLELPNT (SEQ ID NO:41) |
| **huEpCAM23_V LG v3** | RSSKSLLHSDGFTYLY (SEQ ID NO:39) | QTSNLAS (SEQ ID NO:40) | AQNLELPNT (SEQ ID NO:41) |
| **huEpCAM23_V LG v4** | RSSRSLLHSDGFTYLY (SEQ ID NO:42) | QTSNLAS (SEQ ID NO:40) | AQNLELPNT (SEQ ID NO:41) |
| **Affinity Variants** | | | |
| **huEpCAM23LC Gv4a** | RSSRSLLHSNGFTYLY (SEQ ID NO:43) | QTSNLAS (SEQ ID NO:40) | AQNLELPNT (SEQ ID NO:41) |
| **huEpCAM23LC Gv4b** | RSSRSLLHSDGITYLY (SEQ ID NO:44) | QTSNLAS (SEQ ID NO:40) | AQNLELPNT (SEQ ID NO:41) |
| **huEpCAM23LC Gv4c** | RSSRSLLHSDGFTYLY (SEQ ID NO:42) | QTSNLAS (SEQ ID NO:40) | AQNLELPWT (SEQ ID NO:46) |
| **huEpCAM23LC Gv4e** | RSSRSLLHSDGFTYLS (SEQ ID NO:45) | QTSNLAS (SEQ ID NO:40) | AQNLELPNT (SEQ ID NO:41) |
| **huEpCAM23LC Gv4 f** | RSSRSLLHSDGFTYLY (SEQ ID NO:42) | QTSNLAS (SEQ ID NO:40) | AQNLELPNT (SEQ ID NO:41) |
| **huEpCAM23LC Gv4g** | RSSRSLLHSDGFTYLY (SEQ ID NO:42) | QTSNLAS (SEQ ID NO:40) | QQNLELPNT (SEQ ID NO:47) |
| **huEpCAM23LC Gv4h** | RSSRSLLHSDGFTYLY (SEQ ID NO:42) | QTSNLAS (SEQ ID NO:40) | LQNLELPNT (SEQ ID NO:48) |
| **huEpCAM23LC Gv4i** | RSSRSLLHSDGFTYLY (SEQ ID NO:42) | QTSNLAS (SEQ ID NO:40) | AQYLELPNT (SEQ ID NO:49) |
| **huEpCAM23LC Gv4 j** | RSSRSLLHSDGFTYLY (SEQ ID NO:42) | QTSNLAS (SEQ ID NO:40) | AQGLELPNT (SEQ ID NO:50) |
| **huEpCAM23LC Gv4k** | RSSRSLLHSDGFTYLY (SEQ ID NO:42) | QTSNLAS (SEQ ID NO:40) | AQSLELPNT (SEQ ID NO:51) |

In some embodiments, the disclosure provides an EpCAM antibody or EpCAM-binding antibody fragment comprising the light chain CDRs of a single row in Table 3. In some embodiments, the disclosure provides an EpCAM antibody or EpCAM-binding antibody fragment comprising a VL-CDR1 selected from SEQ ID NOs:39, and 42-45; a VL-CDR2 of SEQ ID NO:40; and a VL-CDR3 selected from SEQ ID NOs:41, and 46-51. In some embodiments, the disclosure provides an EpCAM antibody or EpCAM-binding antibody fragment comprising a VL-CDR1 of SEQ ID NO:42;a VL-CDR2 of SEQ ID NO:40; and a VL-CDR3 of SEQ ID NO:41. In some embodiments, the disclosure provides an EpCAM antibody or EpCAM-binding antibody fragment comprising a VL-CDR1 of SEQ ID NO:39; a VL-CDR2 of SEQ ID NO:40; and a VL-CDR3 of SEQ ID NO:41.

In one embodiment, the disclosure provides an EpCAM antibody, EpCAM-binding antibody fragment, or EpCAM activatable antibody comprising: a VL-CDR1 comprising RSSRSLLHSDGFTYLY (SEQ ID NO:42), or a variant thereof comprising 1, 2, 3, or 4, conservative amino acid substitutions; a VL-CDR2 comprising QTSNLAS (SEQ ID NO:40), or a variant thereof comprising 1, 2, 3, or 4, conservative amino acid substitutions; and a VL-CDR3 comprising AQNLELPNT (SEQ ID NO:41), or a variant thereof comprising 1, 2, 3, or 4, conservative amino acid substitutions. In one embodiment, the disclosure provides an EpCAM antibody, EpCAM-binding antibody fragment, or EpCAM activatable antibody comprising: a VL-CDR1 comprising RSSKSLLHSDGFTYLY (SEQ ID NO:39), or a variant thereof comprising 1, 2, 3, or 4, conservative amino acid substitutions; a VL-CDR2 comprising QTSNLAS (SEQ ID NO:40), or a variant thereof comprising 1, 2. 3. or 4. conservative amino acid substitutions; and a VL-CDR3 comprising AQNLELPNT (SEQ ID NO:41), or a variant thereof comprising 1, 2, 3, or 4, conservative amino acid substitutions.

In some embodiments, the disclosure provides an EpCAM antibody or EpCAM-binding antibody fragment comprising a VH-CDR1 selected from SEQ ID NOs:13, and 16-25; a VH-CDR2 selected from SEQ ID NOs:14, and 26-29; a VH-CDR3 selected from SEQ ID NOs:15, and 30-38; a VL-CDR1 selected from SEQ ID NOs:39, and 42-45; a VL-CDR2

of SEQ ID NO:40; and a VL-CDR3 selected from SEQ ID NOs:41, and 46-51. In some embodiments, the disclosure provides an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 13-15, 42, 40, and 41, respectively. In some embodiments, the disclosure provides an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 13-15, and 39-41, respectively. In some embodiments, the disclosure provides an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 13, 26, 15, and 39-41, respectively. In some embodiments, the disclosure provides an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 13, 26, 15, 42, 40, and 41, respectively.

In some embodiments, the disclosure provides an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 22, 14, 15, 42, 40, and 41, respectively. In some embodiments, the disclosure provides an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 13, 14, 33, 42, 40, and 41, respectively. In some embodiments, the disclosure provides an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 23, 14, 15, 42, 40, and 41, respectively. In some embodiments, the disclosure provides an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 25, 14, 15, 42, 40, and 41, respectively.

In some embodiments, the EpCAM antibody or EpCAM-binding antibody fragment comprises a heavy chain variable region (VH) sequence disclosed in Table 4. In some embodiments, the EpCAM antibody or EpCAM-binding antibody fragment comprises a VH sequence that is at least 90%, 91%, 92%, 93%, 94%, 95%, 96%, 97%, 98%, or 99%, identical to a VH sequence disclosed in Table 4.

**Table 4. Exemplary Heavy Chain Variable Sequences of EpCAM Antibodies.**

| Antibody name | Sequence |
|---|---|
| **Murine and Chimeric** | |
| **muEpCAM23** | EVKLEESGPALVKPGASVRISCKASGYTFTNYYIHWVKQRPGQGLDY IGWIYPGNVYIQYNEKFKGKATLTADKSSSTAFMQLSSLTSEDSAVYF CARDGPWFAYWGQGTLVTVSS (SEQ ID NO:52) |
| **chEpCAm23** | EVKLEESGPALVKPGASVRISCKASGYTFTNYYIHWVKQRPGQGLDY IGWIYPGNVYIQYNEKFKGKATLTADKSSSTAFMQLSSLTSEDSAVYF CARDGPWFAYWGQGTLVTVSS (SEQ ID NO:52) |
| **Humanized Variants** | |
| **huEpCAM23_VHGv1** | QVQLVQSGAEVKKPGASVKVSCKASGYTFTNYYIHWVRQAPGQRLE WMGWIYPGNVYIQYSQKFQGRVTITRDTSASTAYMELSSLRSEDTAV YYCARDGPWFAYWGQGTLVTVSS (SEQ ID NO:53) |
| **huEpCAM23_VHGv2** | QVQLVQSGAEVKKPGASVKVSCKASGYTFTNYYIHWVRQAPGQRLE YIGWIYPGNVYIQYNEKFKGRATLTADKSASTAYMELSSLRSEDTAV YYCARDGPWFAYWGQGTLVTVSS (SEQ ID NO:54) |
| **huEpCAM23_VHGv3** | QVQLVQSGAEVKKPGASVKVSCKASGYTFTNYYIHWVRQAPGQRLE WMGWIYPGNVYIQYNEKFKGRVTITRDTSASTAYMELSSLRSEDTAV YYCARDGPWFAYWGQGTLVTVSS (SEQ ID NO:55) |
| **huEpCAM23_VHGv4** | QVQLVQSGAEVKKPGASVKVSCKASGYTFTNYYIHWVRQAPGQRLE YIGWIYPGNVYIQYSQKFQGRVTITADKSASTAYMELSSLRSEDTAVY YCARDGPWFAYWGQGTLVTVSS (SEQ ID NO:56) |

(continued)

| Affinity Variants | |
|---|---|
| huEpCAM23HC Gv2a | QVQLVESGAEVKKPGASVKVSCKASGYTFTNYYIHWVRQAPGQRLE YIGWIYPGNVYIQYNEKFKGRATLTADKSASTAYMELSSLRSEDTAV YYCARDGPWFAYWGQGTLVTVSS (SEQ ID NO:57) |
| huEpCAM23HC Gv2b | QVQLVQSGAEVKKPGASVKVSCKASGYTFTSYYIHWVRQAPGQRLE YIGWIYPGNVYIQYNEKFKGRATLTADKSASTAYMELSSLRSEDTAV YYCARDGPWFAYWGQGTLVTVSS (SEQ ID NO:58) |
| huEpCAM23HC Gv2c | QVQLVQSGAEVKKPGASVKVSCKASGYTFTNYNIHWVRQAPGQRLE YIGWIYPGNVYIQYNEKFKGRATLTADKSASTAYMELSSLRSEDTAV YYCARDGPWFAYWGQGTLVTVSS (SEQ ID NO:59) |
| huEpCAM23HC Gv2d | QVQLVQSGAEVKKPGASVKVSCKASGYTFTNYYIHWVRQAPGQRLE YIGWIYPGDVYIQYNEKFKGRATLTADKSASTAYMELSSLRSEDTAV YYCARDGPWFAYWGQGTLVTVSS (SEQ ID NO:60) |
| huEpCAM23HC Gv2e | QVQLVQSGAEVKKPGASVKVSCKASGYTFTNYFIHWVRQAPGQRLE YIGWIYPGNVYIQYNEKFKGRATLTADKSASTAYMELSSLRSEDTAV YYCARDGPWFAYWGQGTLVTVSS (SEQ ID NO:61) |
| huEpCAM23HC Gv2f | QVQLVQSGAEVKKPGASVKVSCKASGYTFTNYSIHWVRQAPGQRLE YIGWIYPGNVYIQYNEKFKGRATLTADKSASTAYMELSSLRSEDTAV YYCARDGPWFAYWGQGTLVTVSS (SEQ ID NO:62) |
| huEpCAM23HC Gv2g | QVQLVQSGAEVKKPGASVKVSCKASGYTFTNYWIHWVRQAPGQRLE YIGWIYPGNVYIQYNEKFKGRATLTADKSASTAYMELSSLRSEDTAVY YCARDGPWFAYWGQGTLVTVSS (SEQ ID NO:63) |
| huEpCAM23HC Gv2h | QVQLVQSGAEVKKPGASVKVSCKASGYTFTNYYIHWVRQAPGQRLE YIGWFYPGNVYIQYNEKFKGRATLTADKSASTAYMELSSLRSEDTAV YYCARDGPWFAYWGQGTLVTVSS (SEQ ID NO:64) |
| huEpCAM23HC Gv2i | QVQLVQSGAEVKKPGASVKVSCKASGYTFTNYYIHWVRQAPGQRLE WIGWIYPGNVYIQYNEKFKGRATLTADKSASTAYMELSSLRSEDTAV YYCARDGPWFAYWGQGTLVTVSS (SEQ ID NO:65) |
| huEpCAM23HC Gv2j | QVQLVQSGAEVKKPGASVKVSCKASGYTFTNYYIHWVRQAPGQRLE YIGWINPGNVYIQYNEKFKGRATLTADKSASTAYMELSSLRSEDTAV YYCARDGPWFAYWGQGTLVTVSS (SEQ ID NO:66) |
| huEpCAM23HC Gv2k | QVQLVQSGAEVKKPGASVKVSCKASGYTFTNYYIHWVRQAPGQRLE YIGWIYPGNVYIQYNEKFKGRATLTADKSASTAYMELSSLRSEDTAV YYCAREGPWFAYWGQGTLVTVSS (SEQ ID NO:67) |
| huEpCAM23HC Gv2l | QVQLVQSGAEVKKPGASVKVSCKASGYTFTNYYIHWVRQAPGQRLE YIGWIYPGNVYIQYNEKFKGRATLTADKSASTAYMELSSLRSEDTAV YYCARDGPYFAYWGQGTLVTVSS(SEQ ID NO:68) |
| huEpCAM23HCG v2m/ huEpCam 23HG2-1565-A Hea-vy Chain | QVQLVQSGAEVKKPGASVKVSCKASGYTFTNYYIHWVRQAPGQRLE YIGWIYPGNVYIQYNEKFKGRATLTADKSASTAYMELSSLRSEDTAV YYCARDGAWFAYWGQGTLVTVSS (SEQ ID NO:69) |

(continued)

| Affinity Variants | |
|---|---|
| huEpCAM23HC Gv2n | QVQLVQSGAEVKKPGASVKVSCKASGYTFTNYYMHWVRQAPGQRLEYIGWIYPGNVYIQYNEKFKGRATLTADKSASTAYMELSSLRSEDTAVYYCARDGPWFAYWGQGTLVTVSS (SEQ ID NO:70) |
| huEpCAM23HC Gv2p | QVQLVQSGAEVKKPGASVKVSCKASGYTFTNYYIHWVRQAPGQRLEYIGWVYPGNVYIQYNEKFKGRATLTADKSASTAYMELSSLRSEDTAVYYCARDGPWFAYWGQGTLVTVSS (SEQ ID NO:71) |
| huEpCAM23HC Gv2q | QVQLVQSGAEVKKPGASVKVSCKASGYTFTNYYIHWVRQAPGQRLEYIGWIYPGNVYIQYNEKFKGRATITADKSASTAYMELSSLRSEDTAVYYCARDGPWFAYWGQGTLVTVSS (SEQ ID NO:72) |
| huEpCAM23HC Gv2r | QVQLVQSGAEVKKPGASVKVSCKASGYTFTNYYIHWVRQAPGQRLEYIGWIYPGNVYIQYNEKFKGRATLTRDKSASTAYMELSSLRSEDTAVYYCARDGPWFAYWGQGTLVTVSS (SEQ ID NO:73) |
| huEpCAM23HC Gv2s | QVQLVQSGAEVKKPGASVKVSCKASGYTFTNYYIHWVRQAPGQRLEYIGWIYPGNVYIQYNEKFKGRVTLTADKSASTAYMELSSLRSEDTAVYYCARDGPWFAYWGQGTLVTVSS (SEQ ID NO:74) |
| huEpCam23HG2 -1361-H Heavy Chain | QVQLVQSGAEVKKPGASVKVSCKASGYTFTNYHIHWVRQAPGQRLEYIGWIYPGNVYIQYNEKFKGRATLTADKSASTAYMELSSLRSEDTAVYYCARDGPWFAYWGQGTLVTVSS (SEQ ID NO:75) |
| huEpCam23HG2 -1361-D Heavy Chain | QVQLVQSGAEVKKPGASVKVSCKASGYTFTNYDIHWVRQAPGQRLEYIGWIYPGNVYIQYNEKFKGRATLTADKSASTAYMELSSLRSEDTAVYYCARDGPWFAYWGQGTLVTVSS (SEQ ID NO:76) |
| huEpCam23HG2 -1565-Y Heavy Chain | QVQLVQSGAEVKKPGASVKVSCKASGYTFTNYYIHWVRQAPGQRLEYIGWIYPGNVYIQYNEKFKGRATLTADKSASTAYMELSSLRSEDTAVYYCARDGYWFAYWGQGTLVTVSS (SEQ ID NO:77) |
| huEpCam23HG2 -1565-S Heavy Chain | QVQLVQSGAEVKKPGASVKVSCKASGYTFTNYYIHWVRQAPGQRLEYIGWIYPGNVYIQYNEKFKGRATLTADKSASTAYMELSSLRSEDTAVYYCARDGSWFAYWGQGTLVTVSS (SEQ ID NO:78) |
| huEpCam23HG2 -1565-F Heavy Chain | QVQLVQSGAEVKKPGASVKVSCKASGYTFTNYYIHWVRQAPGQRLEYIGWIYPGNVYIQYNEKFKGRATLTADKSASTAYMELSSLRSEDTAVYYCARDGFWFAYWGQGTLVTVSS (SEQ ID NO:79 |
| huEpCam23HG2 -1565-G Heavy Chain | QVQLVQSGAEVKKPGASVKVSCKASGYTFTNYYIHWVRQAPGQRLEYIGWIYPGNVYIQYNEKFKGRATLTADKSASTAYMELSSLRSEDTAVYYCARDGGWFAYWGQGTLVTVSS (SEQ ID NO:80) |
| huEpCam23HG2 -1565-T Heavy Chain | QVQLVQSGAEVKKPGASVKVSCKASGYTFTNYYIHWVRQAPGQRLEYIGWIYPGNVYIQYNEKFKGRATLTADKSASTAYMELSSLRSEDTAVYYCARDGTWFAYWGQGTLVTVSS (SEQ ID NO:81) |
| huEpCam23HG2 -1565-V Heavy Chain | QVQLVQSGAEVKKPGASVKVSCKASGYTFTNYYIHWVRQAPGQRLEYIGWIYPGNVYIQYNEKFKGRATLTADKSASTAYMELSSLRSEDTAVYYCARDGVWFAYWGQGTLVTVSS (SEQ ID NO:82) |

(continued)

| Affinity Variants | |
|---|---|
| huEpCam23HG2 -1361-I Heavy Chain | QVQLVQSGAEVKKPGASVKVSCKASGYTFTNYIIHWVRQAPGQRLE YIGWIYPGNVYIQYNEKFKGRATLTADKSASTAYMELSSLRSEDTAV YYCARDGPWFAYWGQGTLVTVSS (SEQ ID NO:83) |
| huEpCam23HG2 -1361-L Heavy Chain | QVQLVQSGAEVKKPGASVKVSCKASGYTFTNYLIHWVRQAPGQRLE YIGWIYPGNVYIQYNEKFKGRATLTADKSASTAYMELSSLRSEDTAV YYCARDGPWFAYWGQGTLVTVSS (SEQ ID NO:84) |

In some embodiments, the EpCAM antibody or EpCAM-binding antibody fragment comprises a VH sequence having a total of one, two, three, four, five, six, seven, eight, nine, ten, fewer than fifteen, or zero, amino acid substitutions, deletions, and/or insertions from a reference VH sequence selected from SEQ ID NOs: 53-84. In some embodiments, the insertions, substitutions, deletions, and/or insertions are in framework regions(s) of the reference sequence. In some embodiments, the substitutions are conservative. In other embodiments, the substitutions are non-conservative.

In some embodiments, the EpCAM antibody or EpCAM-binding antibody fragment comprises a VH having a sequence selected from SEQ ID NOs:53-84. In some embodiments, the EpCAM antibody or EpCAM-binding antibody fragment comprises a VH having a sequence selected from SEQ ID NOs:53-56. In some embodiments, the EpCAM antibody or EpCAM-binding antibody fragment comprises a VH having the sequence of SEQ ID NO:54 . In some embodiments, the EpCAM antibody or EpCAM-binding antibody fragment comprises a VH sequence that is at least 90%, 91%, 92%, 93%, 94%, 95%, 96%, 97%, 98%, or 99%, identical to a sequence selected from SEQ ID NOs: 53-56. In some embodiments, the EpCAM antibody or EpCAM-binding antibody fragment comprises a VH sequence that is at least 90%, 91%, 92%, 93%, 94%, 95%, 96%, 97%, 98%, and 99%, identical to the sequence of SEQ ID NO: 54.

In some embodiments, the EpCAM antibody or EpCAM-binding antibody fragment comprises a VH having a sequence selected from SEQ ID NOs: 75-77, and 84. In some embodiments, the EpCAM antibody or EpCAM-binding antibody fragment comprises a VH having the sequence of SEQ ID NO:75. In some embodiments, the EpCAM antibody or EpCAM-binding antibody fragment comprises a VH having the sequence of SEQ ID NO:77. In some embodiments, the EpCAM antibody or EpCAM-binding antibody fragment comprises a VH sequence that is at least 90%, 91%, 92%, 93%, 94%, 95%, 96%, 97%, 98%, or 99%, identical to a sequence selected from SEQ ID NOs:75-77, and 84. In some embodiments, the EpCAM antibody or EpCAM-binding antibody fragment comprises a VH sequence that is at least 90%, 91%, 92%, 93%, 94%, 95%, 96%, 97%, 98%, and 99%, identical to the sequence of SEQ ID NO: 75. In some embodiments, the EpCAM antibody or EpCAM-binding antibody fragment comprises a VH sequence that is at least 90%, 91%, 92%, 93%, 94%, 95%, 96%, 97%, 98%, and 99%, identical to the sequence of SEQ ID NO: 77.

In some embodiments, the EpCAM antibody or EpCAM-binding antibody fragment comprises a light chain variable region (VL) sequence disclosed in Table 5. In some embodiments, the EpCAM antibody or EpCAM-binding antibody fragment comprises a VL sequence that is at least 90%, 91%, 92%, 93%, 94%, 95%, 96%, 97%, 98%, or 99%, identical to a VL sequence disclosed in Table 5.

**Table 5. Exemplary Light Chain Variable Sequences of EpCAM Antibodies.**

| Antibody name | Sequence |
|---|---|
| Murine and Chimeric | |
| muEpcam23 | DIVLTQTPFSNPVTLGTSASISCRSSKSLLHSDGFTYLYWFLQKPGQSPH LLIYQTSNLASGVPDRFSSSGSGTDFTLRISRVEAEDVGVYYCAQNLELP NTFGGGTKLEIK (SEQ ID NO:85) |
| chEpcam23 | DIVLTQTPFSNPVTLGTSASISCRSSKSLLHSDGFTYLYWFLQKPGQSPH LLIYQTSNLASGVPDRFSSSGSGTDFTLRISRVEAEDVGVYYCAQNLELP NTFGGGTKLEIK (SEQ ID NO:85) |
| Humanized Variants | |
| huEpCAM23_ VLGv1 | DIVMTQTPLSLSVTPGQPASISCRSSRSLLHSDGFTYLYWYLQKPGQSPQ LLIYQTSNLASGVPDRFSGSGSGTDFTLKISRVEAEDVGVYYCAQNLEL PNTFGQGTKLEIK (SEQ ID NO:86) |

(continued)

| Humanized Variants | |
|---|---|
| huEpCAM23_ VLGv2 | DIVLTQTPLSLSVTPGQPASISCRSSKSLLHSDGFTYLYWFLQKPGQSPQ LLIYQTSNLASGVPDRFSSSGSGTDFTLKISRVEAEDVGVYYCAQNLELP NTFGQGTKLEIK (SEQ ID NO:87) |
| huEpCAM23_ VLGv3 | DIVMTQTPLSLSVTPGQPASISCRSSKSLLHSDGFTYLYWFLQKPGQSPQ LLIYQTSNLASGVPDRFSGSGSGTDFTLKISRVEAEDVGVYYCAQNLEL PNTFGQGTKLEIK (SEQ ID NO:88) |
| huEpCAM23_ VLGv4 | DIVLTQTPLSLSVTPGQPASISCRSSRSLLHSDGFTYLYWFLQKPGQSPQ LLIYQTSNLASGVPDRFSSSGSGTDFTLKISRVEAEDVGVYYCAQNLELP NTFGQGTKLEIK (SEQ ID NO:89) |
| Affinity Variants | |
| huEpCAM23L CGv4a | DIVLTQTPLSLSVTPGQPASISCRSSRSLLHSNGFTYLYWFLQKPGQSPQL LIYQTSNLASGVPDRFSSSGSGTDFTLKISRVEAEDVGVYYCAQNLELP NTFGQGTKLEIK (SEQ ID NO:90) |
| huEpCAM23L CGv4b | DIVLTQTPLSLSVTPGQPASISCRSSRSLLHSDGITYLYWFLQKPGQSPQL LIYQTSNLASGVPDRFSSSGSGTDFTLKISRVEAEDVGVYYCAQNLELP NTFGQGTKLEIK (SEQ ID NO:91) |
| Antibody name | Sequence |
| huEpCAM23L CGv4c | DIVLTQTPLSLSVTPGQPASISCRSSRSLLHSDGFTYLYWFLQKPGQSPQ LLIYQTSNLASGVPDRFSSSGSGTDFTLKISRVEAEDVGVYYCAQNLELP WTFGQGTKLEIK (SEQ ID NO:92) |
| huEpCAM23L CGv4e | DIVLTQTPLSLSVTPGQPASISCRSSRSLLHSDGFTYLSWFLQKPGQSPQL LIYQTSNLASGVPDRFSSSGSGTDFTLKISRVEAEDVGVYYCAQNLELP NTFGQGTKLEIK (SEQ ID NO:93) |
| huEpCAM23L CGv4f | DIVLTQTPLSLSVTPGQPASISCRSSRSLLHSDGFTYLYWYLQKPGQSPQL LIYQTSNLASGVPDRFSSSGSGTDFTLKISRVEAEDVGVYYCAQNLELP NTFGQGTKLEIK (SEQ ID NO:94) |
| huEpCAM23L CGv4g | DIVLTQTPLSLSVTPGQPASISCRSSRSLLHSDGFTYLYWFLQKPGQSPQ LLIYQTSNLASGVPDRFSSSGSGTDFTLKISRVEAEDVGVYYCQQNLELP NTFGQGTKLEIK (SEQ ID NO:95) |
| huEpCAM23L CGv4h | DIVLTQTPLSLSVTPGQPASISCRSSRSLLHSDGFTYLYWFLQKPGQSPQ LLIYQTSNLASGVPDRFSSSGSGTDFTLKISRVEAEDVGVYYCLQNLELP NTFGQGTKLEIK (SEQ ID NO:96) |
| huEpCAM23L CGv4i | DIVLTQTPLSLSVTPGQPASISCRSSRSLLHSDGFTYLYWFLQKPGQSPQ LLIYQTSNLASGVPDRFSSSGSGTDFTLKISRVEAEDVGVYYCAQYLELP NTFGQGTKLEIK (SEQ ID NO:97) |
| huEpCAM23L CGv4j | DIVLTQTPLSLSVTPGQPASISCRSSRSLLHSDGFTYLYWFLQKPGQSPQ LLIYQTSNLASGVPDRFSSSGSGTDFTLKISRVEAEDVGVYYCAQGLELP NTFGQGTKLEIK (SEQ ID NO:98) |
| huEpCAM23L CGv4k | DIVLTQTPLSLSVTPGQPASISCRSSRSLLHSDGFTYLYWFLQKPGQSPQ LLIYQTSNLASGVPDRFSSSGSGTDFTLKISRVEAEDVGVYYCAQSLELP NTFGQGTKLEIK (SEQ ID NO:99) |

In some embodiments, the EpCAM antibody or EpCAM-binding antibody fragment comprises a VL sequence having a total of one, two, three, four, five, six, seven, eight, nine, ten, fewer than fifteen, or zero, amino acid substitutions, deletions, and/or insertions from a reference VH sequence selected from SEQ ID NOs: 86-99. In some embodiments, the insertions, substitutions, deletions, and/or insertions are in framework regions(s) of the reference sequence. In some embodiments, the substitutions are conservative. In other embodiments, the substitutions are non-conservative.

In some embodiments, the EpCAM antibody or EpCAM-binding antibody fragment comprises a VL having a sequence selected from SEQ ID NOs:86-99. In some embodiments, the EpCAM antibody or EpCAM-binding antibody fragment comprises a VL having a sequence selected from SEQ ID NOs:86-89. In some embodiments, the EpCAM antibody or EpCAM-binding antibody fragment comprises a VL having the sequence of SEQ ID NO:89. In some embodiments, the EpCAM antibody or EpCAM-binding antibody fragment comprises a VL having the sequence of SEQ ID NO:87. In some embodiments, the EpCAM antibody or EpCAM-binding antibody fragment comprises a VH sequence that is at least 90%, 91%, 92%, 93%, 94%, 95%, 96%, 97%, 98%, or 99%, identical to a sequence selected from SEQ ID NOs: 86-89. In some embodiments, the EpCAM antibody or EpCAM-binding antibody fragment comprises a VL sequence that is at least 90%, 91%, 92%, 93%, 94%, 95%, 96%, 97%, 98%, and 99%, identical to the sequence of SEQ ID NO: 89. In some embodiments, the EpCAM antibody or EpCAM-binding antibody fragment comprises a VL sequence that is at least 90%, 91%, 92%, 93%, 94%, 95%, 96%, 97%, 98%, and 99%, identical to the sequence of SEQ ID NO: 87.

In some embodiments, the EpCAM antibody or EpCAM-binding antibody fragment comprises a VH comprising a sequence selected from SEQ ID NOs:53-84 and a VL comprising a sequence selected from SEQ ID NOs: 86-89. In some embodiments, the EpCAM antibody or EpCAM-binding antibody fragment comprises a VH comprising the sequence of SEQ ID NO:54 and a VL comprising the sequence of SEQ ID NO: 89. In some embodiments, the EpCAM antibody or EpCAM-binding antibody fragment comprises a VH comprising the sequence of SEQ ID NO:54 and a VL comprising the sequence of SEQ ID NO: 87. In some embodiments, the EpCAM antibody or EpCAM-binding antibody fragment comprises a VH comprising the sequence of SEQ ID NO:55, and a VL comprising the sequence of SEQ ID NO: 87. In some embodiments, the EpCAM antibody or EpCAM-binding antibody fragment comprises a VH comprising the sequence of SEQ ID NO:56, and a VL comprising the sequence of SEQ ID NO: 88. In some embodiments, the EpCAM antibody or EpCAM-binding antibody fragment comprises a VH comprising the sequence of SEQ ID NO:55, and a VL comprising the sequence of SEQ ID NO: 89. In some embodiments, the EpCAM antibody or EpCAM-binding antibody fragment comprises a VH comprising the sequence of SEQ ID NO:56, and a VL comprising the sequence of SEQ ID NO: 89.

In some embodiments, the EpCAM antibody or EpCAM-binding antibody fragment comprises a VH comprising the sequence of SEQ ID NO:75, and a VL comprising the sequence of SEQ ID NO: 89. In some embodiments, the EpCAM antibody or EpCAM-binding antibody fragment comprises a VH comprising the sequence of SEQ ID NO:77, and a VL comprising the sequence of SEQ ID NO: 89. In some embodiments, the EpCAM antibody or EpCAM-binding antibody fragment comprises a VH comprising the sequence of SEQ ID NO:76, and a VL comprising the sequence of SEQ ID NO: 89. In some embodiments, the EpCAM antibody or EpCAM-binding antibody fragment comprises a VH comprising the sequence of SEQ ID NO:84, and a VL comprising the sequence of SEQ ID NO: 89.

In some embodiments, the EpCAM antibody or EpCAM-binding antibody fragment competes for binding to human EpCAM with an antibody comprising a VH and a VL sequence disclosed in Tables 4 and 5, respectively.

In one embodiment, the disclosure provides an EpCAM antibody or EpCAM-binding antibody fragment that competes for binding to human EpCAM with an antibody comprising a heavy chain variable region (VH) and a light chain variable region (VL) selected from:

(a) a heavy chain variable region (VH) of SEQ ID NO:54 and a light chain variable region (VL) of SEQ ID NO:89;
(b) a VH of SEQ ID NO:54 and a VL of SEQ ID NO: 87;
(c) a VH of SEQ ID NO:75 and a VL of SEQ ID NO: 89; and
(d) a VH of SEQ ID NO:77 and a VL of SEQ ID NO: 89.

An EpCAM antibody or EpCAM-binding antibody fragment is said to "compete" with a reference molecule for binding to EpCAM if it binds to human EpCAM to the extent that it blocks, to some degree, binding of the reference molecule to human EpCAM. The ability of proteins to compete for binding to EpCAM and thus to interfere with, block or "cross-block" one anothers' binding to EpCAM can be determined by any standard competitive binding assay known in the art including, for example, a competition ELISA assay, surface plasmon resonance (SPR; BIACORE®, Biosensor, Piscataway, N.J.) or according to methods described by Scatchard et al. (Ann. N.Y. Acad. Sci. 51:660-672 (1949)). An antibody may be said to competitively inhibit binding of the reference EpCAM antibody to human EpCAM, for example, by at least 90%, at least 80%, at least 70%, at least 60%, or at least 50%.

In some embodiments, the EpCAM antibody or EpCAM-binding antibody fragment further includes a heavy chain constant region or fragment thereof. In some aspects, the antibody or antibody fragment comprises a heavy chain immunoglobulin constant region selected from the group consisting of: (a) a human IgA constant region, or fragment

thereof; (b) a human IgD constant region, or fragment thereof; (c) a human IgE constant domain, or fragment thereof; (d) a human IgG1 constant region, or fragment thereof; (e) a human IgG2 constant region, or fragment thereof; (f) a human IgG3 constant region, or fragment thereof; (g) a human IgG4 constant region, or fragment thereof; and (h) a human IgM constant region, or fragment thereof. In certain embodiments, the EpCAM antibody or EpCAM-binding antibody fragment comprises a heavy chain constant region or fragment thereof, *e.g.,* a human IgG constant region or fragment thereof. In further embodiments, the EpCAM antibody or EpCAM-binding antibody fragment comprises a heavy chain immunoglobulin constant domain that has, or has been mutated to have altered effector function and/or half-life.

In some embodiments, the EpCAM antibody or EpCAM-binding antibody fragment comprises a heavy chain sequence disclosed in Table 6.

**Table 6. Exemplary heavy chain full-length sequences of EpCAM antibodies.**

| Antibody name | Sequence |
|---|---|
| **Murine and Chimeric** | |
| **muEpCAM-23HC** | EVKLEESGPALVKPGASVRISCKASGYTFTNYYIHWVKQRPGQGLDYI<br>GWIYPGNVYIQYNEKFKGKATLTADKSSSTAFMQLSSLTSEDSAVYFC<br>ARDGPWFAYWGQGTLVTVSSAKTTPPSVYPLAPGCGDTTGSSVTLGC<br>LVKGYFPESVTVTWNSGSLSSSVHTFPALLQSGLYTMSSSVTVPSSTW<br>PSQTVTCSVAHPASSTTVDKKLEPSGPISTINPCPPCKECHKCPAPNLEG<br>GPSVFIFPPNIKDVLMISLTPKVTCVVVDVSEDDPDVRISWFVNNVEVH<br>TAQTQTHREDYNSTIRVVSALPIQHQDWMSGKEFKCKVNNKDLPSPIE<br>RTISKIKGLVRAPQVYILPPPAEQLSRKDVSLTCLVVGFNPGDISVEWTS<br>NGHTEENYKDTAPVLDSDGSYFIYSKLDIKTSKWEKTDSFSCNVRHEG<br>LKNYYLKKTISRSPGK (SEQ ID NO:100) |
| **chEpcam23 HC** | EVKLEESGPALVKPGASVRISCKASGYTFTNYYIHWVKQRPGQGLDYI<br>GWIYPGNVYIQYNEKFKGKATLTADKSSSTAFMQLSSLTSEDSAVYFC<br>ARDGPWFAYWGQGTLVTVSSASTKGPSVFPLAPSSKSTSGGTAALGC<br>LVKDYFPEPVTVSWNSGALTSGVHTFPAVLQSSGLYSLSSVVTVPSSSL<br>GTQTYICNVNHKPSNTKVDKKVEPKSCDKTHTCPPCPAPELLGGPSVF<br>LFPPKPKDTLMISRTPEVTCVVVDVSHEDPEVKFNWYVDGVEVHNAK<br>TKPREEQYNSTYRVVSVLTVLHQDWLNGKEYKCKVSNKALPAPIEKTI<br>SKAKGQPREPQVYTLPPSRDELTKNQVSLTCLVKGFYPSDIAVEWESN<br>GQPENNYKTTPPVLDSDGSFFLYSKLTVDKSRWQQGNVFSCSVMHEA<br>LHNHYTQKSLSLSPG (SEQ ID NO:101) |
| **Humanized variants** | |
| **huEpCAM2 3 VHGv1** | QVQLVQSGAEVKKPGASVKVSCKASGYTFTNYYIHWVRQAPGQRLE<br>WMGWIYPGNVYIQYSQKFQGRVTITRDTSASTAYMELSSLRSEDTAV<br>YYCARDGPWFAYWGQGTLVTVSSASTKGPSVFPLAPSSKSTSGGTAA<br>LGCLVKDYFPEPVTVSWNSGALTSGVHTFPAVLQSSGLYSLSSVVTVP<br>SSSLGTQTYICNVNHKPSNTKVDKKVEPKSCDKTHTCPPCPAPELLGG |
| **Antibody name** | **Sequence** |
| | PSVFLFPPKPKDTLMISRTPEVTCVVVDVSHEDPEVKFNWYVDGVEVH<br>NAKTKPREEQYNSTYRVVSVLTVLHQDWLNGKEYKCKVSNKALPAPI<br>EKTISKAKGQPREPQVYTLPPSRDELTKNQVSLTCLVKGFYPSDIAVE<br>WESNGQPENNYKTTPPVLDSDGSFFLYSKLTVDKSRWQQGNVFSCSV<br>MHEALHNHYTQKSLSLSPG (SEQ ID NO:102) |

(continued)

| Antibody name | Sequence |
|---|---|
| huEpCAM2 3 VHGv2 (lysine-linked) | QVQLVQSGAEVKKPGASVKVSCKASGYTFTNYYIHWVRQAPGQRLE<br>YIGWIYPGNVYIQYNEKFKGRATLTADKSASTAYMELSSLRSEDTAVY<br>YCARDGPWFAYWGQGTLVTVSSASTKGPSVFPLAPSSKSTSGGTAAL<br>GCLVKDYFPEPVTVSWNSGALTSGVHTFPAVLQSSGLYSLSSVVTVPS<br>SSLGTQTYICNVNHKPSNTKVDKKVEPKSCDKTHTCPPCPAPELLGGP<br>SVFLFPPKPKDTLMISRTPEVTCVVVDVSHEDPEVKFNWYVDGVEVH<br>NAKTKPREEQYNSTYRVVSVLTVLHQDWLNGKEYKCKVSNKALPAPI<br>EKTISKAKGQPREPQVYTLPPSRDELTKNQVSLTCLVKGFYPSDIAVE<br>WESNGQPENNYKTTPPVLDSDGSFFLYSKLTVDKSRWQQGNVFSCSV<br>MHEALHNHYTQKSLSLSPG (SEQ ID NO:103) |
| huEpCAM2 3 VHGv2-C442 (site-specific) | QVQLVQSGAEVKKPGASVKVSCKASGYTFTNYYIHWVRQAPGQRLE<br>YIGWIYPGNVYIQYNEKFKGRATLTADKSASTAYMELSSLRSEDTAVY<br>YCARDGPWFAYWGQGTLVTVSSASTKGPSVFPLAPSSKSTSGGTAAL<br>GCLVKDYFPEPVTVSWNSGALTSGVHTFPAVLQSSGLYSLSSVVTVPS<br>SSLGTQTYICNVNHKPSNTKVDKKVEPKSCDKTHTCPPCPAPELLGGP<br>SVFLFPPKPKDTLMISRTPEVTCVVVDVSHEDPEVKFNWYVDGVEVH<br>NAKTKPREEQYNSTYRVVSVLTVLHQDWLNGKEYKCKVSNKALPAPI<br>EKTISKAKGQPREPQVYTLPPSRDELTKNQVSLTCLVKGFYPSDIAVE<br>WESNGQPENNYKTTPPVLDSDGSFFLYSKLTVDKSRWQQGNVFSCSV<br>MHEALHNHYTQKSLCLSPG SLSPG (SEQ ID NO:104) |
| huEpCAM2 3 VHGv3 | QVQLVQSGAEVKKPGASVKVSCKASGYTFTNYYIHWVRQAPGQRLE<br>WMGWIYPGNVYIQYNEKFKGRVTITRDTSASTAYMELSSLRSEDTAV<br>YYCARDGPWFAYWGQGTLVTVSSASTKGPSVFPLAPSSKSTSGGTAA<br>LGCLVKDYFPEPVTVSWNSGALTSGVHTFPAVLQSSGLYSLSSVVTVP<br>SSSLGTQTYICNVNHKPSNTKVDKKVEPKSCDKTHTCPPCPAPELLGG<br>PSVFLFPPKPKDTLMISRTPEVTCVVVDVSHEDPEVKFNWYVDGVEVH<br>NAKTKPREEQYNSTYRVVSVLTVLHQDWLNGKEYKCKVSNKALPAPI<br>EKTISKAKGQPREPQVYTLPPSRDELTKNQVSLTCLVKGFYPSDIAVE<br>WESNGQPENNYKTTPPVLDSDGSFFLYSKLTVDKSRWQQGNVFSCSV<br>MHEALHNHYTQKSLSLSPG (SEQ ID NO:105) |
| huEpCAM2 3 VHGv4 | QVQLVQSGAEVKKPGASVKVSCKASGYTFTNYYIHWVRQAPGQRLE<br>YIGWIYPGNVYIQYSQKFQGRVTITADKSASTAYMELSSLRSEDTAVY<br>YCARDGPWFAYWGQGTLVTVSSASTKGPSVFPLAPSSKSTSGGTAAL<br>GCLVKDYFPEPVTVSWNSGALTSGVHTFPAVLQSSGLYSLSSVVTVPS<br>SSLGTQTYICNVNHKPSNTKVDKKVEPKSCDKTHTCPPCPAPELLGGP<br>SVFLFPPKPKDTLMISRTPEVTCVVVDVSHEDPEVKFNWYVDGVEVH<br>NAKTKPREEQYNSTYRVVSVLTVLHQDWLNGKEYKCKVSNKALPAPI<br>EKTISKAKGQPREPQVYTLPPSRDELTKNQVSLTCLVKGFYPSDIAVE<br>WESNGQPENNYKTTPPVLDSDGSFFLYSKLTVDKSRWQQGNVFSCSV<br>MHEALHNHYTQKSLSLSPG (SEQ ID NO:106) |

(continued)

| Affinity variants | |
|---|---|
| huEpCAM2 3HCGv2a | QVQLVESGAEVKKPGASVKVSCKASGYTFTNYYIHWVRQAPGQRLEYIGWIYPGNVYIQYNEKFKGRATLTADKSASTAYMELSSLRSEDTAVYYCARDGPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:107) |
| huEpCAM2 3HCGv2b | QVQLVQSGAEVKKPGASVKVSCKASGYTFTSYYIHWVRQAPGQRLEYIGWIYPGNVYIQYNEKFKGRATLTADKSASTAYMELSSLRSEDTAVYYCARDGPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:108) |
| huEpCAM2 3HCGv2c | QVQLVQSGAEVKKPGASVKVSCKASGYTFTNYNIHWVRQAPGQRLEYIGWIYPGNVYIQYNEKFKGRATLTADKSASTAYMELSSLRSEDTAVYYCARDGPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:109) |
| huEpCAM2 3HCGv2d | QVQLVQSGAEVKKPGASVKVSCKASGYTFTNYYIHWVRQAPGQRLEYIGWIYPGDVYIQYNEKFKGRATLTADKSASTAYMELSSLRSEDTAVYYCARDGPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:110) |
| huEpCAM2 3HCGv2e | QVQLVQSGAEVKKPGASVKVSCKASGYTFTNYFIHWVRQAPGQRLEYIGWIYPGNVYIQYNEKFKGRATLTADKSASTAYMELSSLRSEDTAVYYCARDGPWFAYWGQGTLVTVSSASTKGPSVFPLAPSSKSTSGGTAALGCLVKDYFPEPVTVSWNSGALTSGVHTFPAVLQSSGLYSLSSVVTVPSSS |

(continued)

| Affinity variants | |
|---|---|
| | LGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:111) |
| huEpCAM2 3HCGv2f | QVQLVQSGAEVKKPGASVKVSCKASGYTFTNYSIHWVRQAPGQRLEYIGWIYPGNVYIQYNEKFKGRATLTADKSASTAYMELSSLRSEDTAVYYCARDGPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:112) |
| huEpCAM2 3HCGv2g | QVQLVQSGAEVKKPGASVKVSCKASGYTFTNYWIHWVRQAPGQRLEYIGWIYPGNVYIQYNEKFKGRATLTADKSASTAYMELSSLRSEDTAVYYCARDGPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:113) |
| huEpCAM2 3HCGv2h | QVQLVQSGAEVKKPGASVKVSCKASGYTFTNYYIHWVRQAPGQRLEYIGWFYPGNVYIQYNEKFKGRATLTADKSASTAYMELSSLRSEDTAVYYCARDGPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:114) |
| huEpCAM2 3HCGv2i /2o | QVQLVQSGAEVKKPGASVKVSCKASGYTFTNYYIHWVRQAPGQRLEWIGWIYPGNVYIQYNEKFKGRATLTADKSASTAYMELSSLRSEDTAVYYCARDGPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:115) |

(continued)

| Affinity variants | |
|---|---|
| huEpCAM2 3HCGv2j | QVQLVQSGAEVKKPGASVKVSCKASGYTFTNYYIHWVRQAPGQRLEYIGWINPGNVYIQYNEKFKGRATLTADKSASTAYMELSSLRSEDTAVYYCARDGPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:116) |
| huEpCAM2 3HCGv2k | QVQLVQSGAEVKKPGASVKVSCKASGYTFTNYYIHWVRQAPGQRLEYIGWIYPGNVYIQYNEKFKGRATLTADKSASTAYMELSSLRSEDTAVYYCAREGPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:117) |
| huEpCAM2 3HCGv2L | QVQLVQSGAEVKKPGASVKVSCKASGYTFTNYYIHWVRQAPGQRLEYIGWIYPGNVYIQYNEKFKGRATLTADKSASTAYMELSSLRSEDTAVYYCARDGPYFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:118) |
| huEpCAM2 3HCGv2m/ huEp-Cam23 HG2-1565-A Heavy Chain | QVQLVQSGAEVKKPGASVKVSCKASGYTFTNYYIHWVRQAPGQRLEYIGWIYPGNVYIQYNEKFKGRATLTADKSASTAYMELSSLRSEDTAVYYCARDGAWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG (SEQ ID NO:119) |
| huEpCAM2 3HCGv2n | QVQLVQSGAEVKKPGASVKVSCKASGYTFTNYYMHWVRQAPGQRLEYIGWIYPGNVYIQYNEKFKGRATLTADKSASTAYMELSSLRSEDTAVYYCARDGPWFAYWGQGTLVTVSSASTKGPSVFPLAPSSKSTSGGTAAL |

(continued)

| Affinity variants | |
|---|---|
| | GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:120) |
| **huEpCAM2 3HCGv2p** | QVQLVQSGAEVKKPGASVKVSCKASGYTFTNYYIHWVRQAPGQRLE YIGWVYPGNVYIQYNEKFKGRATLTADKSASTAYMELSSLRSEDTAV YYCARDGPWFAYWGQGTLVTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:121) |
| **huEpCAM2 3HCGv2q** | QVQLVQSGAEVKKPGASVKVSCKASGYTFTNYYIHWVRQAPGQRLE YIGWIYPGNVYIQYNEKFKGRATITADKSASTAYMELSSLRSEDTAVY YCARDGPWFAYWGQGTLVTVSSASTKGPSVFPLAPSSKSTSGGTAAL GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:122) |
| **huEpCAM2 3HCGv2r** | QVQLVQSGAEVKKPGASVKVSCKASGYTFTNYYIHWVRQAPGQRLE YIGWIYPGNVYIQYNEKFKGRATLTRDKSASTAYMELSSLRSEDTAVY YCARDGPWFAYWGQGTLVTVSSASTKGPSVFPLAPSSKSTSGGTAAL GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:123) |
| **huEpCAM2 3HCGv2s** | QVQLVQSGAEVKKPGASVKVSCKASGYTFTNYYIHWVRQAPGQRLE YIGWIYPGNVYIQYNEKFKGRVTLTADKSASTAYMELSSLRSEDTAVY YCARDGPWFAYWGQGTLVTVSSASTKGPSVFPLAPSSKSTSGGTAAL GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE |

(continued)

| Affinity variants | |
|---|---|
| | WESNGQPENNYKTTPPVLDSDGSFFLYSKLTVDKSRWQQGNVFSCSV MHEALHNHYTQKSLSLSPG (SEQ ID NO:124) |
| huEpCam23 HG2-1361-H Heavy Chain | QVQLVQSGAEVKKPGASVKVSCKASGYTFTNYHIHWVRQAPGQRLE YIGWIYPGNVYIQYNEKFKGRATLTADKSASTAYMELSSLRSEDTAVY YCARDGPWFAYWGQGTLVTVSSASTKGPSVFPLAPSSKSTSGGTAAL GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:125) |
| huEpCam23 HG2-1361-D Heavy Chain | QVQLVQSGAEVKKPGASVKVSCKASGYTFTNYDIHWVRQAPGQRLE YIGWIYPGNVYIQYNEKFKGRATLTADKSASTAYMELSSLRSEDTAVY YCARDGPWFAYWGQGTLVTVSSASTKGPSVFPLAPSSKSTSGGTAAL GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:126) |
| huEpCam23 HG2-1565-Y Heavy Chain | QVQLVQSGAEVKKPGASVKVSCKASGYTFTNYYIHWVRQAPGQRLE YIGWIYPGNVYIQYNEKFKGRATLTADKSASTAYMELSSLRSEDTAVY YCARDGYWFAYWGQGTLVTVSSASTKGPSVFPLAPSSKSTSGGTAAL GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:127) |
| huEpCam23 HG2-1565-S Heavy Chain | QVQLVQSGAEVKKPGASVKVSCKASGYTFTNYYIHWVRQAPGQRLE YIGWIYPGNVYIQYNEKFKGRATLTADKSASTAYMELSSLRSEDTAVY YCARDGSWFAYWGQGTLVTVSSASTKGPSVFPLAPSSKSTSGGTAAL GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:128) |
| huEpCam23 HG2-1565-F | QVQLVQSGAEVKKPGASVKVSCKASGYTFTNYYIHWVRQAPGQRLE YIGWIYPGNVYIQYNEKFKGRATLTADKSASTAYMELSSLRSEDTAVY YCARDGFWFAYWGQGTLVTVSSASTKGPSVFPLAPSSKSTSGGTAAL |

(continued)

| Affinity variants | |
|---|---|
| Heavy Chain | GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:129) |
| huEpCam23 HG2-1565-G Heavy Chain | QVQLVQSGAEVKKPGASVKVSCKASGYTFTNYYIHWVRQAPGQRLE YIGWIYPGNVYIQYNEKFKGRATLTADKSASTAYMELSSLRSEDTAVY YCARDGGWFAYWGQGTLVTVSSASTKGPSVFPLAPSSKSTSGGTAAL GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:130) |
| huEpCam23 HG2-1565-T Heavy Chain | QVQLVQSGAEVKKPGASVKVSCKASGYTFTNYYIHWVRQAPGQRLE YIGWIYPGNVYIQYNEKFKGRATLTADKSASTAYMELSSLRSEDTAVY YCARDGTWFAYWGQGTLVTVSSASTKGPSVFPLAPSSKSTSGGTAAL GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:131) |
| huEpCam23 HG2-1565-V Heavy Chain | QVQLVQSGAEVKKPGASVKVSCKASGYTFTNYYIHWVRQAPGQRLE YIGWIYPGNVYIQYNEKFKGRATLTADKSASTAYMELSSLRSEDTAVY YCARDGVWFAYWGQGTLVTVSSASTKGPSVFPLAPSSKSTSGGTAAL GCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPG (SEQ ID NO:132) NGQPENNYKTTPPVLDSDGSFFLYSKLTVDKSRWQQGNVFSCSVMHE ALHNHYTQKSLSLSPG (SEQ ID NO:133) |
| huEpCam23 HG2-1361-I Heavy Chain | QVQLVQSGAEVKKPGASVKVSCKASGYTFTNYIIHWVRQAPGQRLEYI GWIYPGNVYIQYNEKFKGRATLTADKSASTAYMELSSLRSEDTAVYY CARDGPWFAYWGQGTLVTVSSASTKGPSVFPLAPSSKSTSGGTAALG CLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNA KTKPREEQYNSTYRVVSVLTVLHQDWLNGKEYKCKVSNKALPAPIEK TISKAKGQPREPQVYTLPPSRDELTKNQVSLTCLVKGFYPSDIAVEWES |

(continued)

| Affinity variants | |
|---|---|
| **huEpCam23 HG2-1361-L Heavy Chain** | QVQLVQSGAEVKKPGASVKVSCKASGYTFTNYLIHWVRQAPGQRLEY IGWIYPGNVYIQYNEKFKGRATLTADKSASTAYMELSSLRSEDTAVYY CARDGPWFAYWGQGTLVTVSSASTKGPSVFPLAPSSKSTSGGTAALG CLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNA KTKPREEQYNSTYRVVSVLTVLHQDWLNGKEYKCKVSNKALPAPIEK TISKAKGQPREPQVYTLPPSRDELTKNQVSLTCLVKGFYPSDIAVEWES NGQPENNYKTTPPVLDSDGSFFLYSKLTVDKSRWQQGNVFSCSVMHE ALHNHYTQKSLSLSPG (SEQ ID NO:134) |

In some embodiments, the EpCAM antibody or EpCAM-binding antibody fragment comprises a heavy chain (HC) sequence selected from SEQ ID NOs:102-134. In some embodiments, the EpCAM antibody or EpCAM-binding antibody fragment comprises a heavy chain (HC) sequence selected from SEQ ID NOs:102-106. In a specific embodiment, the EpCAM antibody or EpCAM-binding antibody fragment comprises a HC sequence of SEQ ID NO:103. In alternative embodiments, the EpCAM antibody or EpCAM-binding antibody fragment comprises a HC sequence selected from SEQ ID NOs:125-127 and 134. In a specific embodiment, the EpCAM antibody or EpCAM-binding antibody fragment comprises a HC sequence of SEQ ID NO:125. In a specific embodiment, the EpCAM antibody or EpCAM-binding antibody fragment comprises a HC sequence of SEQ ID NO:127.

In additional aspects, the EpCAM antibody or EpCAM-binding antibody fragment comprises a light chain immunoglobulin constant region. In a further aspect, the antibody comprises a human Ig kappa constant region or a human Ig lambda constant region.

In some embodiments, the EpCAM antibody or EpCAM-binding antibody fragment comprises a light chain sequence disclosed in Table 7.

**Table 7. Exemplary light chain full-length sequences of EpCAM antibodies.**

| Antibody name | Sequence |
|---|---|
| **Murine and Chimeric** | |
| **muEpcam23LC** | DIVLTQTPFSNPVTLGTSASISCRSSKSLLHSDGFTYLYWFLQKPGQ SPHLLIYQTSNLASGVPDRFSSSGSGTDFTLRISRVEAEDVGVYYCA QNLELPNTFGGGTKLEIKRADAAPTVSIFPPSSEQLTSGGASVVCFL NNFYPKDINVKWKIDGSERQNGVLNSWTDQDSKDSTYSMSSTLTL TKDEYERHNSYTCEATHKTSTSPIVKSFNRNEC (SEQ ID NO:135) |
| **chEpCam23LC** | DIVLTQTPFSNPVTLGTSASISCRSSKSLLHSDGFTYLYWFLQKPGQS PHLLIYQTSNLASGVPDRFSSSGSGTDFTLRISRVEAEDVGVYYCAQ NLELPNTFGGGTKLEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC (SEQ ID NO:136) |
| **Humanized variants** | |
| **huEpCAM23_V LGv1** | DIVMTQTPLSLSVTPGQPASISCRSSRSLLHSDGFTYLYWYLQKPGQ SPQLLIYQTSNLASGVPDRFSGSGSGTDFTLKISRVEAEDVGVYYCA QNLELPNTFGQGTKLEIKRTVAAPSVFIFPPSDEQLKSGTASVVCLLN NFYPREAKVQWKVDNALQSGNSQESVTEQDSKDSTYSLSSTLTLSK ADYEKHKVYACEVTHQGLSSPVTKSFNRGEC (SEQ ID NO:137) |

(continued)

| Humanized variants | |
|---|---|
| huEpCAM23_V LGv2 | DIVLTQTPLSLSVTPGQPASISCRSSKSLLHSDGFTYLYWFLQKPGQS PQLLIYQTSNLASGVPDRFSSSGSGTDFTLKISRVEAEDVGVYYCAQ NLELPNTFGQGTKLEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC (SEQ ID NO:138) |
| huEpCAM23_V LGv3 | DIVMTQTPLSLSVTPGQPASISCRSSKSLLHSDGFTYLYWFLQKPGQS PQLLIYQTSNLASGVPDRFSGSGSGTDFTLKISRVEAEDVGVYYCAQ NLELPNTFGQGTKLEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC (SEQ ID NO:139) |
| huEpCAM23_V LGv4 | DIVLTQTPLSLSVTPGQPASISCRSSRSLLHSDGFTYLYWFLQKPGQ SPQLLIYQTSNLASGVPDRFSSSGSGTDFTLKISRVEAEDVGVYYCA QNLELPNTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:140) |
| **Affinity variants** | |
| huEpCAM23LC Gv4a | DIVLTQTPLSLSVTPGQPASISCRSSRSLLHSNGFTYLYWFLQKPGQ SPQLLIYQTSNLASGVPDRFSSSGSGTDFTLKISRVEAEDVGVYYCA QNLELPNTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:141) |
| huEpCAM23LC Gv4b | DIVLTQTPLSLSVTPGQPASISCRSSRSLLHSDGITYLYWFLQKPGQS PQLLIYQTSNLASGVPDRFSSSGSGTDFTLKISRVEAEDVGVYYCA QNLELPNTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:142) |
| huEpCAM23LC Gv4c | DIVLTQTPLSLSVTPGQPASISCRSSRSLLHSDGFTYLYWFLQKPGQ SPQLLIYQTSNLASGVPDRFSSSGSGTDFTLKISRVEAEDVGVYYCA QNLELPWTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:143) |
| **Antibody name** | **Sequence** |
| huEpCAMGvL C4e | DIVLTQTPLSLSVTPGQPASISCRSSRSLLHSDGFTYLSWFLQKPGQS PQLLIYQTSNLASGVPDRFSSSGSGTDFTLKISRVEAEDVGVYYCA QNLELPNTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:144) |
| huEpCAMGvL C4f | DIVLTQTPLSLSVTPGQPASISCRSSRSLLHSDGFTYLYWYLQKPGQ SPQLLIYQTSNLASGVPDRFSSSGSGTDFTLKISRVEAEDVGVYYCA QNLELPNTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:145) |

(continued)

| Antibody name | Sequence |
|---|---|
| huEpCAMGvL C4g | DIVLTQTPLSLSVTPGQPASISCRSSRSLLHSDGFTYLYWFLQKPGQ SPQLLIYQTSNLASGVPDRFSSSGSGTDFTLKISRVEAEDVGVYYCQ QNLELPNTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:146) |
| huEpCAMGvL C4h | DIVLTQTPLSLSVTPGQPASISCRSSRSLLHSDGFTYLYWFLQKPGQ SPQLLIYQTSNLASGVPDRFSSSGSGTDFTLKISRVEAEDVGVYYCL QNLELPNTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:147) |
| huEpCAMGvL C4i | DIVLTQTPLSLSVTPGQPASISCRSSRSLLHSDGFTYLYWFLQKPGQ SPQLLIYQTSNLASGVPDRFSSSGSGTDFTLKISRVEAEDVGVYYCA QYLELPNTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:148) |
| huEpCAMGvL C4j | DIVLTQTPLSLSVTPGQPASISCRSSRSLLHSDGFTYLYWFLQKPGQ SPQLLIYQTSNLASGVPDRFSSSGSGTDFTLKISRVEAEDVGVYYCA QGLELPNTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:149) |
| huEpCAMGvL C4k | DIVLTQTPLSLSVTPGQPASISCRSSRSLLHSDGFTYLYWFLQKPGQ SPQLLIYQTSNLASGVPDRFSSSGSGTDFTLKISRVEAEDVGVYYCA QSLELPNTFGQGTKLEIKRTVAAPSVFIFPPSDEQLKSGTASVVCLL NNFYPREAKVQWKVDNALQSGNSQESVTEQDSKDSTYSLSSTLTL SKADYEKHKVYACEVTHQGLSSPVTKSFNRGEC (SEQ ID NO:150) |

In some embodiments, the EpCAM antibody or EpCAM-binding antibody fragment comprises a light chain (LC) sequence selected from SEQ ID NOs:137-150. In some embodiments, the EpCAM antibody or EpCAM-binding antibody fragment comprises a light chain (LC) sequence selected from SEQ ID NOs:137-140. In a specific embodiment, the EpCAM antibody or EpCAM-binding antibody fragment comprises a LC sequence of SEQ ID NO: 140. In another embodiment, the EpCAM antibody or EpCAM-binding antibody fragment comprises a LC sequence of SEQ ID NO:138.

In additional embodiments, the EpCAM antibody or EpCAM-binding antibody fragment comprises a HC having a sequence selected from SEQ ID NOs:102-134 and an LC having a sequence selected from SEQ ID NOs:137-150. In additional embodiments, the EpCAM antibody or EpCAM-binding antibody fragment comprises a HC having the sequence of SEQ ID NO:103 and an LC having the sequence of SEQ ID NO:140. In additional embodiments, the EpCAM antibody or EpCAM-binding antibody fragment comprises a HC having the sequence of SEQ ID NO:103 and an LC having the sequence of SEQ ID NO:138. In additional embodiments, the EpCAM antibody or EpCAM-binding antibody fragment comprises a HC having the sequence of SEQ ID NO:105 and an LC having the sequence of SEQ ID NO:138. In additional embodiments, the EpCAM antibody or EpCAM-binding antibody fragment comprises a HC having the sequence of SEQ ID NO:106 and an LC having the sequence of SEQ ID NO:139. In additional embodiments, the EpCAM antibody or EpCAM-binding antibody fragment comprises a HC having the sequence of SEQ ID NO:105 and an LC having the sequence of SEQ ID NO:140. In additional embodiments, the EpCAM antibody or EpCAM-binding antibody fragment comprises a HC having the sequence of SEQ ID NO:106 and an LC having the sequence of SEQ ID NO:140. In some embodiments, the EpCAM antibody or EpCAM-binding antibody fragment comprises a HC having the sequence of SEQ ID NO:125 and an LC having the sequence of SEQ ID NO:140. In additional embodiments, the EpCAM antibody or EpCAM-binding antibody fragment comprises a HC having the sequence of SEQ ID NO:127 and an LC having the sequence of SEQ ID NO:140. In additional embodiments, the EpCAM antibody or EpCAM-binding antibody fragment comprises a HC having the sequence of SEQ ID NO:126 and an LC having the sequence of SEQ ID NO:140. In additional embodiments, the EpCAM antibody or EpCAM-binding antibody fragment comprises a HC having the sequence of SEQ ID

NO:134 and an LC having the sequence of SEQ ID NO:140.

In some embodiments, the EpCAM antibody comprises an altered (e.g., mutated or engineered) Fc region. For example, in some aspects, the Fc region has been altered to reduce or enhance the effector functions of the antibody, alter serum half-life or other functional properties of the antibody. Reduction or elimination of effector function is desirable in certain cases, for example in the case of antibodies whose mechanism of action involves blocking or antagonism, but not killing of the cells bearing a target antigen. Increased effector function is generally desirable when directed to undesirable cells, such as tumor and foreign cells, where the FcγRs are expressed at low levels, for example, tumor-specific B cells with low levels of FcγRIIB (e.g., non-Hodgkin's lymphoma, CLL, and Burkitt's lymphoma). Immunoconjugates of the invention possessing such conferred or altered effector function activity are useful for the treatment and/or prevention of a disease, disorder or infection in which an enhanced efficacy of effector function activity is desired. In some aspects, the Fc region is an isotype selected from IgM, IgA, IgG, IgE, or other isotype.

Although the Fc Region of the EpCAM antibodies and EpCAM-binding antibody fragments may possess the ability to bind to one or more Fc receptors (e.g., FcγR(s)), in certain embodimnents the antibody or antibody fragment comprises a variant Fc region having an altered binding to FcγRIA (CD64), FcγRIIA (CD32A), FcγRIIB (CD32B), FcγRIIIA (CD16a) or FcγRIIIB (CD16b) (relative to the binding exhibited by a wild-type Fc Region), *e.g.,* will have enhanced binding to an activating receptor and/or will have substantially reduced or no ability to bind to inhibitory receptor(s). Thus, the Fc region of the the EpCAM antibody or EpCAM-binding antibody fragment may include some or all of the CH2 domain and/or some or all of the CH3 domain of a complete Fc region, or may comprise a variant CH2 and/or a variant CH3 sequence (that may include, for example, one or more insertions and/or one or more deletions with respect to the CH2 or CH3 domains of a complete Fc Region). Such Fc regions may comprise non-Fc polypeptide portions, or may comprise portions of non-naturally complete Fc regions, or may comprise non-naturally occurring orientations of CH2 and/or CH3 domains (such as, for example, two CH2 domains or two CH3 domains, or in the N-terminal to C-terminal direction, a CH3 domain linked to a CH2 domain, *etc.*).

Fc Region modifications identified as altering effector function are known in the art, including modifications that increase binding to activating receptors (*e.g.*, FcγRIIA (CD16A) and reduce binding to inhibitory receptors (*e.g.,* FcγRIIB (CD32B) (see, *e.g.,* Stavenhagen, et al., Cancer Res. 57(18):8882-8890 (2007)). **Table 8** lists exemplary single, double, triple, quadruple and quintuple substitutions (numbering is that of the EU index as in Kabat, and substitutions are relative to the amino acid sequence of **SEQ ID NO:304)** of exemplary modification that increase binding to activating receptors and/or reduce binding to inhibitory receptors.

| **Table 8<br>Variations of Preferred Activating Fc Regions** | | | |
|---|---|---|---|
| **Single-Site Variations** | | | |
| F243L | R292G | D270E | R292P |
| Y300L | P396L | | |
| **Double-Site Variations** | | | |
| F243L and R292P | F243L and Y300L | F243L and P396L | R292P and Y300L |
| D270E and P396L | R292P and V305I | P396L and Q419H | P247L and N421K |
| R292P and P396L | Y300L and P396L | R255L and P396L | R292P and P305I |
| K392T and P396L | | | |
| **Triple-Site Variations** | | | |
| F243L, P247L and N421K | | P247L, D270E and N421K | |
| F243L, R292P and Y300L | | R255L, D270E and P396L | |
| F243L, R292P and V305I | | D270E, G316D and R416G | |
| F243L, R292P and P396L | | D270E, K392T and P396L | |
| F243L, Y300L and P396L | | D270E, P396L and Q419H | |
| V284M, R292L and K370N | | R292P, Y300L and P396L | |
| **Quadruple-Site Variations** | | | |
| L234F, F243L, R292P and Y300L | | F243L, P247L, D270E and N421K | |
| L234F, F243L, R292P and Y300L | | F243L, R255L, D270E and P396L | |

(continued)

| Quadruple-Site Variations | |
|---|---|
| L235I, F243L, R292P and Y300L | F243L, D270E, G316D and R416G |
| L235Q, F243L, R292P and Y300L | F243L, D270E, K392T and P396L |
| P247L, D270E, Y300L and N421K | F243L, R292P, Y300L, and P396L |
| R255L, D270E, R292G and P396L | F243L, R292P, V305I and P396L |
| R255L, D270E, Y300L and P396L | F243L, D270E, P396L and Q419H |
| D270E, G316D, P396L and R416G | |
| **Quintuple-Site Variations** | |
| L235V, F243L, R292P, Y300L and P396L | F243L, R292P, V305I, Y300L and P396L |
| L235P, F243L, R292P, Y300L and P396L | |

Exemplary variants of human IgG1 Fc Regions with reduced binding to CD32B and/or increased binding to CD16A contain F243L, R292P, Y300L, V305I or P396L substitutions, wherein the numbering is that of the EU index as in Kabat. These amino acid substitutions may be present in a human IgG1 Fc Region in any combination. In one embodiment, the variant human IgG1 Fc Region contains a F243L, R292P and Y300L substitution. In another embodiment, the variant human IgG1 Fc Region contains a F243L, R292P, Y300L, V305I and P396L substitution.

In some embodiments, the EpCAM antibody or EpCAM-binding antibody fragment comprises an immunoglobulin heavy chain constant region containing a modification that decreases effector function (*see, e.g.,* Idusogie et al., J. Immunol. 166:2571-2575 (2001); Sazinsky et al., PNAS USA 105:20167-20172 (2008); Davis et al., J. Rheumatol. 34:2204-2210 (2007); Bolt et al., Eur. J. Immunol. 23:403-411 (1993); Alegre et al., Transplantation 57:1537-1543 (1994); Xu et al., Cell Immunol. 200:16-26 (2000); Cole et al., Transplantation 68:563-571 (1999); Hutchins et al., PNAS USA 92:11980-11984 (1995); Reddy et al., J. Immunol. 164:1925-1933 (2000); WO97/11971, and WO07/106585; U.S. Appl. Publ. 2007/0148167A1; McEarchern et al., Blood 109:1185-1192 (2007); Strohl, Curr. Op. Biotechnol. 20:685-691 (2009); and Kumagai et al., J. Clin. Pharmacol. 47:1489-1497 (2007), the contents of each of which is herein incorporated by reference in its entirety).

In some embodiments, it is preferred for the Fc region of the EpCAM antibody or EpCAM-binding antibody fragment to exhibit decreased (or substantially no) binding to an effector receptor selected from the group consisting of: FcγRIA (CD64), FcγRIIA (CD32A)(allotypes R131 and H131), FcγRIIB (CD32B), FcγRIIIA (CD16a) (allotype V158 and F158) and FcγRIIIB (CD16b)(allotype FcγIIIb-NA1 and FcγIIIb-NA2); relative to the binding exhibited by the wild-type IgG Fc Region (SEQ ID NO:304). In some embodiments, the EpCAM antibody or EpCAM-binding antibody fragment Fc region variant effector receptor binding affinity has been reduced to 1/10 or less, 1/50 or less, or 1/100 or less as, compared to the binding affinity of the corresponding antibody or antibody binding fragment comprising the wildtype Fc region of the corresponding immunoglobulin.

In a specific embodiment, the EpCAM antibody or EpCAM-binding antibody fragment comprises an IgG Fc region that exhibits reduced effector function (*e.g.*, reduced ADCC) and comprise a modification at one or more amino acid positions selected from the group consisting of 233, 234, 235, 236, 237, 238, 239, 265, 266, 267, 269, 270, 271, 295, 296, 297, 298, 300, 324, 325, 327, 328, 329, 331, and 332, wherein the amino acid position numbering is according to the EU index as set forth in Kabat. In one embodiment, the CH2-CH3 domain of the EpCAM antibody include any 1, 2, 3, or 4 of the substitutions: L234A, L235A, D265A, N297Q, N297A, and N297G, wherein the numbering is that of the EU index as in Kabat. In another embodiment, the CH2-CH3 domains contain an N297Q substitution, an N297A substitution, or L234A and L235A substitutions, as these mutations abolish FcR binding. Alternatively, the EpCAM antibody or EpCAM-binding antibody fragment compreses a CH2-CH3 domain of a naturally occurring Fc region that inherently exhibits decreased (or substantially no) binding to FcγRIIIA (CD16a) and/or reduced effector function (relative to the binding and effector function exhibited by the wild-type IgG1 Fc region (SEQ ID NO:304). In a specific embodiment, the Fc constant region of the EpCAM antibody comprises an IgG2 Fc region (SEQ ID NO:305) or an IgG4 Fc region (SEQ ID:NO:306). Since the N297A, N297G, N297Q, L234A, L235A and D265A substitutions abolish effector function, in circumstances in which effector function is desired, these substitutions would preferably not be employed.

A preferred IgG1 sequence for the CH2 and CH3 Domains of the Fc region-containing EpCAM antibody or EpCAM-binding antibody fragment that has reduced or abolished effector function comprises the substitutions L234A/L235A (shown underlined) (SEQ ID NO:307):

APE**AA**GGPSV FLFPPKPKDT LMISRTPEVT CVVVDVSHED PEVKFNWYVD
GVEVHNAKTK PREEQYNSTY RVVSVLTVLH QDWLNGKEYK CKVSNKALPA
PIEKTISKAK GQPREPQVYT LPPSRDELTK NQVSLTCLVK GFYPSDIAVE
WESNGQPENN YKTTPPVLDS DGSFFLYSKL TVDKSRWQQG NVFSCSVMHE
ALHNHYTQKS LSLSPG

A preferred IgG1 sequence for the CH2 and CH3 Domains of the Fc region-containing EpCAM antibody or EpCAM-binding antibody fragment that has reduced or abolished effector function comprises the substitution N297A (shown underlined) (SEQ ID NO:308):

APELLGGPSV FLFPPKPKDT LMISRTPEVT CVVVDVSHED PEVKFNWYVD
GVEVHNAKTK PREEQY**A**STY RVVSVLTVLH QDWLNGKEYK CKVSNKALPA
PIEKTISKAK GQPREPQVYT LPPSRDELTK NQVSLTCLVK GFYPSDIAVE
WESNGQPENN YKTTPPVLDS DGSFFLYSKL TVDKSRWQQG NVFSCSVMHE
ALHNHYTQKS LSLSPG

A preferred IgG 1 sequence for the CH2 and CH3 Domains of the Fc region-containing EpCAM antibody or EpCAM-binding antibody fragment that has reduced or abolished effector function comprises the substitution N297Q (shown underlined) (SEQ ID NO:309):

APELLGGPSV FLFPPKPKDT LMISRTPEVT CVVVDVSHED PEVKFNWYVD
GVEVHNAKTK PREEQY**Q**STY RVVSVLTVLH QDWLNGKEYK CKVSNKALPA

PIEKTISKAK GQPREPQVYT LPPSRDELTK NQVSLTCLVK GFYPSDIAVE
WESNGQPENN YKTTPPVLDS DGSFFLYSKL TVDKSRWQQG NVFSCSVMHE
ALHNHYTQKS LSLSPG

In some embodiments, the EpCAM antibody or EpCAM-binding antibody fragment EpCAM antibody or EpCAM-binding antibody fragment comprises one or more modifications corresponding to: IgG1-C220S, C226S, C229S, P238S; IgG1-C226S, C229S; IgG1-C226S, C229S, E233P, L234V, L235A; IgG1-L234A, L235A; IgG1-L234F, L235E, P331S; IgG1-L234F, L235E, P331S; IgG1-H268Q, A330S, P331S; IgG1-G236R, L328R; IgG1-L235G, G236R, IgG1-N297A; IgG1-N325A, L328R; IgG1-N325L, L328R; IgG1-K326W, E333S; IgG2-V234A, G237A; IgG2-E333S; IgG2 H268Q, V309L, A330S, A331S; IgG4-S228P, L236E; IgG4-F234A, L235A; IgG4-F234A, G237A, E318A; IgG4-L235A, G237A, E318A; IgG4-L236E; IgG2-EU sequence 118-260; and IgG4-EU sequence 261-447; wherein the position numbering is according to the EU index as in Kabat.

In some embodiments, the EpCAM antibody or EpCAM-binding antibody fragment comprises a heavy chain immunoglobulin constant domain that has reduced CDC activity. In particular aspects, EpCAM antibody or EpCAM-binding antibody fragment comprises an IgG1 heavy chain constant region containing a mutation that decreases CDC activity (*see, e.g.,* WO 1997/11971 and WO 2007/106585; U.S. Appl. Publ. 2007/0148167A1; McEarchern et al., Blood 109:1185-1192 (2007); Hayden-Ledbetter et al., Clin. Cancer 15:2739-2746 (2009); Lazar et al., PNAS USA 103:4005-4010 (2006); Bruckheimer et al., Neoplasia 11:509-517 (2009); Strohl, Curr. Op. Biotechnol. 20:685-691 (2009); and Sazinsky et al., PNAS USA 105:20167-20172 (2008); each of which is herein incorporated by reference in its entirety). Examples of heavy chain constant domain sequence modifications that decrease CDC include one or more modifications corresponding to: IgG1-C226S, C229S, E233P, L234V, L235A; IgG1-C226S, P230S; IgG1-L234F, L235E, P331S; IgG1-S239D, A330L, I332E; IgG2 EU sequence 118-260; IgG4-EU sequence 261-447; and IgG2-H268Q, V309L, A330S, A331S, according to the EU index

In some embodiments, the provided EpCAM antibody or EpCAM-binding antibody fragment comprises a heavy chain immunoglobulin constant domain that contains one or more half-life extending amino acid modifications (e.g., substitutions). Numerous mutations capable of increasing the half-life of an Fc region-containing molecule are known in the art and are encompassed as components of the EpCAM antibodies and EpCAM-binding antibody fragments provided herein. *See, e.g.,* U.S. Patent Nos. 6,277,375; 7,083,784; 7,217,797, and 8,088,376; U.S. Publ. Nos. 2002/0147311; and 2007/0148164; and PCT Publication Nos. WO 1998/23289; WO 2009/058492; and WO 2010/033279, the contents of each of which is herein incorporated by reference in its entirety.

The serum half-life of proteins comprising Fc regions may be increased by increasing the binding affinity of the Fc Region for FcRn. The term "half-life" as used herein means a pharmacokinetic property of a molecule that is a measure of the mean survival time of the molecules following their administration. Half-life can be expressed as the time required to eliminate fifty percent (50%) of a known quantity of the molecule from a subject's (e.g., a human patient or other mammal) body or a specific compartment thereof, for example, as measured in serum, *i.e.,* circulating half-life, or in other tissues. In general, an increase in half-life results in an increase in mean residence time (MRT) in circulation for the administered molecule.

In some embodiments, the EpCAM antibody or EpCAM-binding antibody fragment comprises a half-life extending amino acid substitution at one or more positions selected from the group consisting of: 238, 250, 252, 254, 256, 257, 256, 265, 272, 286, 288, 303, 305, 307, 308, 309, 311, 312, 317, 340, 356, 360, 362, 376, 378, 380, 382, 413, 424, 428, 433, 434, 435, and 436, wherein the amino acid position numbering is according to the EU index. In some embodiments, the EpCAM antibody or EpCAM-binding antibody fragment contains one or more amino acid substitutions of amino acid residues at positions 251-257, 285-290, 308-314, 385-389, and 428-436, wherein the amino acid position numbering is according to the EU index. In some embodiments, the EpCAM antibody or EpCAM-binding antibody fragment contains one or more of a substitution of the amino acid at Kabat position 252 with Tyr, Phe, Trp, or Thr; a substitution of the amino acid at Kabat position 254 with Thr; a substitution of the amino acid at Kabat position 256 with Ser, Arg, Gln, Glu, Asp, or Thr; a substitution of the amino acid at Kabat position 257 with Leu; a substitution of the amino acid at Kabat position 309 with Pro; a substitution of the amino acid at Kabat position 311 with Ser; a substitution of the amino acid at Kabat position 428 with Thr, Leu, Phe, or Ser; a substitution of the amino acid at Kabat position 433 with Arg, Ser, Iso, Pro, or Gln; or a substitution of the amino acid at Kabat position 434 with Trp, Met, Ser, His, Phe, or Tyr. More specifically, the EpCAM antibody or EpCAM-binding antibody fragment domain can contain amino acid substitutions relative to a wild-type human IgG constant domain including a substitution of the amino acid at Kabat position 252 with Tyr, a substitution of the amino acid at Kabat position 254 with Thr, and a substitution of the amino acid at Kabat position 256 with Glu.

In some embodiments, the EpCAM antibody or EpCAM-binding antibody fragment comprises a least one substitution selected from: T250Q, M252Y, S254T, T256E, K288D, T307Q, V308P, A378V, M428L, N434A, N434S, N434H, N434Y, H435K, and Y436I, wherein the numbering is that of the EU index as in Kabat. In further embodiments, the EpCAM antibody or EpCAM-binding antibody fragment comprises substitutions selected from: (a) M252Y, S254T and T256E; (b) M252Y and S254T; (c) M252Y and T256E; (d) T250Q and M428L; (e) T307Q and N434A; (f) A378V and N434A; (g) N434A and Y436I; (h) V308P and N434A; and (i) K288D and H435K.

In a preferred embodiment, the EpCAM antibody or EpCAM-binding antibody fragment contains a variant IgG Fc Region comprising any 1, 2, or 3 of the substitutions: M252Y, S254T and T256E. The disclosure further provides EpCAM antibody or EpCAM-binding antibody fragments possessing variant Fc regions comprising: (a) one or more mutations which alter effector function and/or FcγR; and (b) one or more mutations which extend serum half-life.

## EpCAM Activatable Antibodies

In additional embodiments, the disclosure provides EpCAM activatable antibodies (e.g., activatable EpCAM antibodies and activatable EpCAM-binding antibody fragments). In some embodiments, the EpCAM activatable antibody comprises an EpCAM antibody or EpCAM-binding antibody fragment that specifically binds EpCAM (e.g., human EpCAM) coupled to a masking moiety (MM), such that coupling of the MM reduces the ability of the EpCAM antibody or EpCAM-binding antibody fragment to bind EpCAM. In some embodiments, the MM is coupled *via* a sequence that includes a substrate for a protease, for example, a protease that is active in diseased tissue and/or a protease that is co-localized with EpCAM at a treatment site in a subject. The EpCAM activatable antibodies are preferably stable in circulation, activated at intended sites of therapy and/or diagnosis but not in normal, *e.g.,* healthy tissue or other tissue not targeted for treatment and/or diagnosis, and, when activated, exhibit binding to EpCAM that is at least comparable to the corresponding, unmodified antibody. Immunoconjugates comprising the EpCAM activatable antibody are also provided, as are nucleic acids or sets of nucleic acids encoding the EpCAM activatable antibodies, and vectors and host cells comprising the nucleic acids. Pharmaceutical compositions comprising the activatable antibodies, immunoconjugates, nucleic acids, vectors, and host cells, are also provided.

In some embodiments, the EpCAM activatable antibody or antibody fragment further comprises

(a) a cleavable moiety coupled to the antibody or antibody fragment, wherein the cleavable moiety is a polypeptide that functions as a substrate for a protease; and
(b) a masking moiety coupled to the antibody or antibody fragment, wherein the masking moiety inhibits the binding of the antibody or antibody fragment to EpCAM when the activatable antibody is in an uncleaved state,

wherein the activatable antibody in the uncleaved state has the structural arrangement from N-terminus to C-terminus as follows: (masking moiety)-(cleavable moiety)-(antibody or antibody fragment) or (antibody or antibody fragment)-(cleavable moiety)-(masking moiety).

An EpCAM activatable antibody comprising:

(a) an EpCAM antibody or EpCAM-binding antibody fragment comprising a VH-CDR1, VH-CDR2, VH-CDR3, VL-CDR1, VL-CDR2, and VL-CDR3 having the sequences of a member selected from the group:

(i) SEQ ID NOs: 13-15, 42, 40, and 41, respectively;
(ii) SEQ ID NOs: 13-15, and 39-41, respectively;
(iii) SEQ ID NOs: 13, 26, 15, and 39-41, respectively; and
(iv) SEQ ID NOs: 13, 24, 15, 42, 40, and 41, respectively;

(b) a masking moiety coupled to the EpCAM antibody or EpCAM-binding antibody fragment, wherein the masking moiety inhibits the binding of the antibody or antibody fragment to EpCAM when the activatable antibody is in an uncleaved state; and
(c) a cleavable moiety coupled to the EpCAM antibody or EpCAM-binding antibody fragment, wherein the cleavable moiety is a polypeptide that functions as a substrate for a protease;

wherein the activatable antibody in the uncleaved state has the structural arrangement from N-terminus to C-terminus as follows: (masking moiety)-(cleavable moiety)-(antibody or antibody fragment) or (antibody or antibody fragment)-(cleavable moiety)-(masking moiety).

The EpCAM activatable antibodies in an activated state bind human EpCAM and include (i) an EpCAM antibody or a EpCAM-binding antibody fragment (Ab) that specifically binds to human EpCAM (*e.g.,* as disclosed herein); (ii) a masking moiety (MM) that, when the EpCAM activatable antibody, is in an uncleaved state, inhibits the binding of the EpCAM activatable antibody to EpCAM; and (c) a cleavable moiety (CM) coupled to the EpCAM antibody or EpCAM-binding antibody fragment, wherein the CM is a polypeptide that functions as a substrate for a protease. In some embodiments, the EpCAM activatable antibody, in the uncleaved state has the structural arrangement from N-terminus to C-terminus as follows: MM-CM-Ab or Ab-CM-MM. In some embodiments, the EpCAM activatable antibody, comprises a linking peptide between the MM and the CM. In some embodiments, the EpCAM activatable antibody, comprises a linking peptide between the CM and the Ab.

In some embodiments, the EpCAM activatable antibody, in an uncleaved state specifically binds to mammalian EpCAM with a dissociation constant less than or equal to 1 nM, less than or equal to 5 nM, less than or equal to 10 nM, less than or equal to 15 nM, less than or equal to 20 nM, less than or equal to 25 nM, less than or equal to 50 nM, less than or equal to 100 nM, less than or equal to 150 nM, less than or equal to 250 nM, less than or equal to 500 nM, less than or equal to 750 nM, less than or equal to 1000 nM, and 122. /or less than or equal to 2000 nM.

In some embodiments, the EpCAM activatable antibody, in an uncleaved state specifically binds to mammalian EpCAM (e.g., human EpCAM or cynomolgous EpCAM) with a dissociation constant greater than or equal to 1 nM, greater than or equal to 5 nM, greater than or equal to 10 nM, greater than or equal to 15 nM, greater than or equal to 20 nM, greater than or equal to 25 nM, greater than or equal to 50 nM, greater than or equal to 100 nM, greater than or equal to 150 nM, greater than or equal to 250 nM, greater than or equal to 500 nM, greater than or equal to 750 nM, greater than or equal to 1000 nM, and 122. /or greater than or equal to 2000 nM.

In some embodiments, the EpCAM activatable antibody, in an uncleaved state specifically binds to the mammalian EpCAM (e.g., human EpCAM or cynomolgous EpCAM) with a dissociation constant in the range of 1 nM to 2000 nM, 1 nM to 1000 nM, 1 nM to 750 nM, 1 nM to 500 nM, 1 nM to 250 nM, 1 nM to 150 nM, 1 nM to 100 nM, 1 nM to 50 nM, 1 nM to 25 nM, 1 nM to 15 nM, 1 nM to 10 nM, 1 nM to 5 nM, 5 nM to 2000 nM, 5 nM to 1000 nM, 5 nM to 750 nM, 5 nM to 500 nM, 5 nM to 250 nM, 5 nM to 150 nM, 5 nM to 100 nM, 5 nM to 50 nM, 5 nM to 25 nM, 5 nM to 15 nM, 5 nM to 10 nM, 10 nM to 2000 nM, 10 nM to 1000 nM, 10 nM to 750 nM, 10 nM to 500 nM, 10 nM to 250 nM, 10 nM to 150 nM, 10 nM to 100 nM, 10 nM to 50 nM, 10 nM to 25 nM, 10 nM to 15 nM, 15 nM to 2000 nM, 15 nM to 1000 nM, 15 nM to 750 nM, 15 nM to 500 nM, 15 nM to 250 nM, 15 nM to 150 nM, 15 nM to 100 nM, 15 nM to 50 nM, 15 nM to 25 nM, 25 nM to 2000 nM, 25 nM to 1000 nM, 25 nM to 750 nM, 25 nM to 500 nM, 25 nM to 250 nM, 25 nM to 150 nM, 25 nM to 100 nM, 25 nM to 50 nM, 50 nM to 2000 nM, 50 nM to 1000 nM, 50 nM to 750 nM, 50 nM to 500 nM, 50 nM to 250 nM, 50 nM to 150 nM, 50 nM to 100 nM, 100 nM to 2000 nM, 100 nM to 1000 nM, 100 nM to 750 nM, 100 nM to 500 nM, 100 nM to 250 nM, 100 nM to 150 nM, 150 nM to 2000 nM, 150 nM to 1000 nM, 150 nM to 750 nM, 150 nM to 500 nM, 150 nM to 250 nM, 250 nM to 2000 nM, 250 nM to 1000 nM, 250 nM to 750 nM, 250 nM to 500 nM, 500 nM to 2000 nM, 500 nM to 1000 nM, 500 nM to 750 nM, 500 nM to 500 nM, 500 nM to 250 nM, 500 nM to 150 nM, 500 nM to 100 nM, 500 nM to 50 nM, 750 nM to 2000 nM, 750 nM to 1000 nM, or 1000 nM to 2000 nM.

In some embodiments, the EpCAM activatable antibody, in an activated state specifically binds to mammalian EpCAM (e.g., human EpCAM or cynomolgous EpCAM) with a dissociation constant that is less than or equal to 0.01 nM, 0.05 nM, 0.1 nM, 0.5 nM, 1 nM, 5 nM, or 10 nM. In some embodiments, the EpCAM activatable antibody, in an activated state specifically binds to mammalian EpCAM with a dissociation constant is greater than or equal to 0.01 nM, 0.05 nM, 0.1

nM, 0.5 nM, 1 nM, 5 nM, or 10 nM.

In some embodiments, the EpCAM activatable antibody, in an activated state specifically binds to the mammalian EpCAM (e.g., human EpCAM or cynomolgous EpCAM) with a dissociation constant in the range of 0.01 nM to 100 nM, 0.01 nM to 10 nM, 0.01 nM to 5 nM, 0.01 nM to 1 nM, 0.01 to 0.5 nM, 0.01 nm to 0.1 nM, 0.01 nm to 0.05 nM, 0.05 nM to 100 nM, 0.05 nM to 10 nM, 0.05 nM to 5 nM, 0.05 nM to 1 nM, 0.05 to 0.5 nM, 0.05 nm to 0.1 nM, 0.1 nM to 100 nM, 0.1 nM to 10 nM, 0.1 nM to 5 nM, 0.1 nM to 1 nM, 0.1 to 0.5 nM, 0.5 nM to 100 nM, 0.5 nM to 10 nM, 0.5 nM to 5 nM, 0.5 nM to 1 nM, 1 nM to 100 nM, 1 nM to 10 nM, 1 nM to 5 nM, 5 nM to 100 nM, 5 nM to 10 nM, or 10 nM to 100 Nm.

In some embodiments, the EpCAM activatable antibody specifically binds to human EpCAM with a dissociation constant of less than 1 nM. In some embodiments, the EpCAM activatable antibody specifically binds to cynomolgus EpCAM with a dissociation constant of less than 1 nM. In some embodiments, the EpCAM activatable antibody specifically binds to human EpCAM and cynomolgus EpCAM with a dissociation constant of less than 1 nM.

In some embodiments, the serum half-life of the EpCAM activatable antibody is longer than that of the corresponding antibody; *e.g.*, the pK of the EpCAM activatable antibody is longer than that of the corresponding antibody. In some embodiments, the serum half-life of the EpCAM activatable antibody is similar to that of the corresponding antibody.

In some embodiments, the EpCAM activatable antibody comprises an EpCAM antibody or EpCAM-binding antibody fragment comprising a VH-CDR1, a VH-CDR2, and a VH-CDR3 having the sequences set forth in one row of Table 2. In some embodiments, the EpCAM activatable antibody comprises an EpCAM antibody or EpCAM-binding antibody fragment comprising a VL-CDR1, a VL-CDR2, and a VL-CDR3 having the sequences set forth in one row of Table 3.

In some embodiments, the EpCAM activatable antibody comprises an EpCAM antibody or EpCAM-binding antibody fragment comprising a VH-CDR1 selected from SEQ ID NOs:13, and 16-25; a VH-CDR2 selected from SEQ ID NOs:14, and 26-29; a VH-CDR3 selected from SEQ ID NOs:15, and 30-38; a VL-CDR1 selected from SEQ ID NOs:39, and 42-45; a VL-CDR2 of SEQ ID NO:40; and a VL-CDR3 selected from SEQ ID NOs:41, and 46-51.

In some embodiments, the EpCAM activatable antibody comprises an EpCAM antibody or EpCAM-binding antibody fragment comprising a VH-CDR1, VH-CDR2, VH-CDR3, VL-CDR1, VL-CDR2, and VL-CDR3 having the sequences selected from the group consisting of: (i) SEQ ID NOs: 13-15, 42, 40, and 41, respectively; (ii) SEQ ID NOs: 13-15, and 39-41, respectively; (iii) SEQ ID NOs: 13, 26, 15, and 39-41, respectively; and (iv) SEQ ID NOs: 13, 26, 15, 42, 40, and 41, respectively. In some embodiments, the EpCAM activatable antibody comprises an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 13-15, 42, 40, and 41, respectively.

In some embodiments, the EpCAM activatable antibody comprises an EpCAM antibody or EpCAM-binding antibody fragment comprising a VH-CDR1, VH-CDR2, VH-CDR3, VL-CDR1, VL-CDR2, and VL-CDR3 having the sequences selected from the group consisting of: (i) SEQ ID NOs: 22, 14, 15, 42, 40, and 41, respectively; (ii) SEQ ID NOs: 13, 14, 33, 42, 40, and 41, respectively; (iii) SEQ ID NOs: 23, 14, 15, 42, 40, and 41, respectively, and; (iv) SEQ ID NOs: 25, 14, 15, 42, 40, and 41, respectively. In some embodiments, the EpCAM activatable antibody comprises an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 22, 14, 15, 42, 40, and 41, respectively. In some embodiments, the EpCAM activatable antibody comprises an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 13, 14, 33, 42, 40, and 41, respectively.

In some embodiments, the EpCAM activatable antibody comprises a VH disclosed in Table 4. In some embodiments, the EpCAM activatable antibody comprises a VL disclosed in Table 5. In some embodiments, the EpCAM activatable antibody comprises a VH having the sequence of SEQ ID NO: 54 and a VL having the sequence of SEQ ID NO: 89. In some embodiments, the EpCAM activatable antibody comprises an EpCAM antibody or EpCAM-binding antibody fragment comprising a VH having the sequence of SEQ ID NO: 75 and a VL having the sequence of SEQ ID NO: 89. In some embodiments, the EpCAM activatable antibody comprises an EpCAM antibody or EpCAM-binding antibody fragment comprising a VH having the sequence of SEQ ID NO: 77 and a VL having the sequence of SEQ ID NO: 89.

In some embodiments, the EpCAM activatable antibody comprises a VH sequence that is at least 90%, 91%, 92%, 93%, 94%, 95%, 96%, 97%, 98% or 99% identical to a sequence selected from SEQ ID NO: 54, 75, and 77. In some embodiments, the EpCAM activatable antibody comprises a VL sequence that is at least 90%, 91%, 92%, 93%, 94%, 95%, 96%, 97%, 98% or 99% identical to an sequence comprising SEQ ID NO:89.

In some embodiments, the EpCAM activatable antibody comprises a HC disclosed in Table 6. In some embodiments, the EpCAM activatable antibody comprises a LC disclosed in Table 7. In some embodiments, the EpCAM activatable antibody comprises a HC having the sequence of SEQ ID NO: 103 and a LC having the sequence of SEQ ID NO: 140. In some embodiments, the EpCAM activatable antibody comprises a HC having the sequence of SEQ ID NO: 125 and a LC having the sequence of SEQ ID NO: 140. In some embodiments, the EpCAM activatable antibody comprises a HC having the sequence of SEQ ID NO: 127 and a light chain having the sequence of SEQ ID NO: 140.

In some embodiments, the EpCAM activatable antibody comprises an EpCAM antibody or EpCAM-binding antibody fragment that specifically binds to an epitope within the extracellular region of human EpCAM (SEQ ID NO:1). In certain embodiments, the EpCAM activatable antibody comprises an EpCAM antibody or EpCAM-binding antibody fragment that specifically binds to an epitope within the first extracellular domain (D1) of human EpCAM (SEQ ID NO:2).

In some embodiments, the EpCAM activatable antibody comprises a VH-CDR1 comprising $X_1YX_3X_4H$, wherein $X_1$ is selected from N and S, $X_3$ is selected from Y, N, F, S, H, D, L, I, and W, and $X_4$ is selected from I and M (SEQ ID NO:5); a VH-CDR2 comprising $WX_2X_3PGX_6VYIQYX_{12}X_{13}KFX_{17}G$, wherein $X_2$ is selected from I and F, $X_3$ is selected from Y and N, $X_6$ is selected from N and D, $X_{12}$ is selected from N and S, $X_{13}$ is selected from E and Q, and $X_{17}$ is selected from K and Q (SEQ ID NO:7); and a VH-CDR3 comprising $X_1GX_3X_4FAY$, wherein $X_1$ is selected from D and E, $X_3$ is selected from P, A, S, Y, F, G, T, and V, and $X_4$ is selected from Y and W (SEQ ID NO:8). In some embodiments, the EpCAM activatable antibody comprises a VL-CDR1 comprising $RSSX_4SLLHSX_{10}G$ $X_{12}TYLX_{16}$, wherein $X_4$ is selected from R and K, $X_{10}$ is selected from N and D, $X_{12}$ is selected from F and I, and $X_{16}$ is selected from Y and S (SEQ ID NO:10); a light chain VL-CDR2 comprising QTSNLAS (SEQ ID NO:40); and a VL-CDR3 comprising $X_1QX_3LELPX_8T$, wherein $X_1$ is selected from A, L, and Q, $X_3$ is selected from S, G, Y, and N, and $X_8$ is selected from N and W (SEQ ID NO:11). In some embodiments, the EpCAM activatable antibody comprises a VH-CDR1 comprising the sequence of SEQ ID NO: 13; a VH-CDR2 comprising the sequence of SEQ ID NO:14; a VH-CDR3 comprising the sequence of SEQ ID NO:15; a VL-CDR1 comprising the sequence of SEQ ID NO:42; a VL-CDR2 comprising the sequence of SEQ ID NO:40; and a VL-CDR3 comprising the sequence of SEQ ID NO:41.

In some embodiments, the VH-CDR1 of the EpCAM activatable antibody comprises the sequence $NYX_3IH$, wherein $X_3$ is selected from Y, N, F, S, H, D, L, I, and W (SEQ ID NO:6). In some embodiments, the VH-CDR3 of the EpCAM activatable antibody comprises the sequence $DGPX_4FAY$, wherein $X_4$ is selected from Y and W (SEQ ID NO:9). In some embodiments, the VL-CDR3 of the EpCAM activatable antibody comprises the sequence $AQX_3LELPNT$, wherein $X_3$ is selected from S, G, Y, and N (SEQ ID NO:12). In some embodiments, the EpCAM activatable antibody comprises a VH-CDR1 comprising the sequence of SEQ ID NO: 13; a VH-CDR2 comprising the sequence of SEQ ID NO:14; a VH-CDR3 comprising the sequence of SEQ ID NO:15; a VL-CDR1 comprising the sequence of SEQ ID NO:42; a VL-CDR2 comprising the sequence of SEQ ID NO:40; and a VL-CDR3 comprising the sequence of SEQ ID NO:41.

Suitable components of the disclosed EpCAM activatable antibody also include an EpCAM antibody or EpCAM-binding antibody fragment, that cross-competes for binding to human EpCAM and/or cynomolgus EpCAM with an EpCAM antibody comprising a VH having the sequence of SEQ ID NO: 54 and a VL having the sequence of SEQ ID NO: 89. Additional suitable EpCAM activatable antibodies cross-compete for binding to human EpCAM and/or cynomolgus EpCAM with an EpCAM antibody comprising a VH having the sequence of SEQ ID NO: 75 and a VL having the sequence of SEQ ID NO: 89. Additional suitable EpCAM activatable antibodies cross-compete for binding to human EpCAM and/or cynomolgus EpCAM with an EpCAM antibody comprising a VH having the sequence of SEQ ID NO: 77 and a VL having the sequence of SEQ ID NO: 89.

The EpCAM activatable antibodies provided herein include a masking moiety (MM). In some embodiments, the masking moiety (or "mask") is an amino acid sequence that is coupled or otherwise attached to the EpCAM antibody and is positioned within the EpCAM activatable antibody construct such that the masking moiety reduces the ability of the EpCAM antibody to specifically bind EpCAM. Suitable masking moieties are identified using any of a variety of known techniques. For example, peptide masking moieties are identified using the methods described in WO 2009/025846, the contents of which is herein incorporated by reference in its entirety.

In some embodiments, the MM of the activatable antibody has a dissociation constant for binding to the Ab which is greater than the dissociation constant of the Ab to EpCAM. In some embodiments, the MM has a dissociation constant for binding to the Ab which is no more than the dissociation constant of the Ab to EpCAM.

In some embodiments, the MM has a dissociation constant for binding to the Ab which is less than the dissociation constant of the Ab to EpCAM.

In some embodiments, the dissociation constant (Kd) of the MM towards the Ab is no more than 2, 3, 4, 5, 10, 25, 50, 100, 250, 500, 1,000, 2,500, 5,000, 10,000, 50,000, 100,000, 500,000, 1,000,000, 5,000,000, 10,000,000, 50,000,000 times or greater, or between 1-5, 5-10, 10-100, 10-1,000, 10-10,000, 10-100,000, 10-1,000,000, 10-10,000,000, 100-1,000, 100-10,000, 100-100,000, 100-1,000,000, 100-10,000,000, 1,000- 10,000, 1,000-100,000, 1,000-1,000,000, 1000-10,000,000, 10,000-100,000, 10,000-1,000,000, 10,000-10,000,000, 100,000-1,000,000, or 100,000-10,000,000 times or greater than the dissociation constant of the Ab towards the target.

In some embodiments, the MM does not interfere or compete with the Ab for binding to EpCAM when the EpCAM activatable antibody is in a cleaved state. In some embodiments, the MM is a polypeptide of about 2 to 40 amino acids in length. In some embodiments, the MM is a polypeptide of up to about 40 amino acids in length.

In some embodiments, the MM polypeptide sequence is different from that of EpCAM. In some embodiments, the MM polypeptide sequence is no more than 50% identical to any natural binding partner of the Ab. In some embodiments, the MM polypeptide sequence is different from that of EpCAM and is no more than 40%, 30%, 25%, 20%, 15%, or 10% identical to any natural binding partner of the Ab.

In some embodiments, the coupling of the MM to the Ab reduces the ability of the Ab to bind EpCAM such that the dissociation constant (IQ) of the Ab when coupled to the MM towards EpCAM is at least 2, 5, 10, 20, 40, 100, 1,000, 10,000 greater than the IQ of the Ab when not coupled to the MM towards EpCAM.

In some embodiments, in the presence of EpCAM, the MM reduces the ability of the Ab to bind EpCAM by at least 90% when the CM is uncleaved, as compared to when the CM is cleaved when assayed *in vitro* using a target displacement assay such as, for example, the assay described in WO 2010/081173, the contents of which are hereby incorporated by reference in its entirety.

When the Ab is modified with a MM and is in the presence of human EpCAM, specific binding of the Ab to human EpCAM is reduced or inhibited, as compared to the specific binding of the Ab not modified with an MM or the specific binding of the parental Ab to human EpCAM.

The IQ of the Ab modified with a MM towards human EpCAM is at least 5, 10, 25, 50, 100, 250, 500, 1,000, 2,500, 5,000, 10,000, 50,000, 100,000, 500,000, 1,000,000, 5,000,000, 10,000,000, 50,000,000 or greater, or between 5-10, 10-100, 10-1,000, 10-10,000, 10-100,000, 10-1,000,000, 10-10,000,000, 100-1,000, 100-10,000, 100-100,000, 100-1,000,000, 100-10,000,000, 1,000-10,000, 1,000-100,000, 1,000-1,000,000, 1000-10,000,000, 10,000-100,000, 10,000-1,000,000, 10,000-10,000,000, 100,000-1,000,000, or 100,000-10,000,000 times greater than the IQ of the Ab not modified with an MM or of the parental Ab towards human EpCAM. Conversely, the binding affinity of the Ab modified with a MM towards human EpCAM is at least 2, 3, 4, 5, 10, 25, 50, 100, 250, 500, 1,000, 2,500, 5,000, 10,000, 50,000, 100,000, 500,000, 1,000,000, 5,000,000, 10,000,000, 50,000,000 or greater, or between 5-10, 10-100, 10-1,000, 10-10,000, 10-100,000, 10-1,000,000, 10-10,000,000, 100-1,000, 100-10,000, 100-100,000, 100-1,000,000, 100-10,000,000, 1,000-10,000, 1,000-100,000, 1,000-1,000,000, 1000-10,000,000, 10,000-100,000, 10,000-1,000,000, 10,000-10,000,000, 100,000-1,000,000, or 100,000-10,000,000 times lower than the binding affinity of the Ab not modified with an MM or of the parental Ab towards human EpCAM.

In some embodiments, the dissociation constant (Kd) of the MM towards the Ab is approximately equal to the Kd of the Ab towards human EpCAM. In some embodiments, the dissociation constant (Kd) of the MM towards the Ab is no more than the dissociation constant of the Ab towards human EpCAM. In some embodiments, the dissociation constant (Kd) of the MM towards the Ab is less than the dissociation constant of the Ab towards human EpCAM. In some embodiments, the dissociation constant (Kd) of the MM towards the Ab is greater than the dissociation constant of the Ab towards human EpCAM.

In some embodiments, the MM has a Kd for binding to the Ab that is no more than the Kd for binding of the Ab to human EpCAM.

In some embodiments, the MM has a Kd for binding to the Ab that is no less than the Kd for binding of the Ab to human EpCAM. In some embodiments, the MM has a Kd for binding to the Ab that is approximately equal to the Kd for binding of the Ab to human EpCAM. In some embodiments, the MM has a Kd for binding to the Ab that is less than the Kd for binding of the Ab to human EpCAM. In some embodiments, the MM has a Kd for binding to the Ab that is greater than the Kd for binding of the Ab to human EpCAM. In some embodiments, the MM has a Kd for binding to the Ab that is no more than 2, 3, 4, 5, 10, 25, 50, 100, 250, 500, or 1,000 fold greater than the Kd for binding of the Ab to human EpCAM. In some embodiments, the MM has a Kd for binding to the Ab that is between 1-5, 2-5, 2-10, 5-10, 5-20, 5-50, 5-100, 10-100, 10-1,000, 20-100, 20-1000, or 100-1,000 fold greater than the Kd for binding of the Ab to human EpCAM.

In some embodiments, the MM has an affinity for binding to the Ab that is less than the affinity of binding of the Ab to human EpCAM. In some embodiments, the MM has an affinity for binding to the Ab that is no more than the affinity of binding of the Ab to human EpCAM. In some embodiments, the MM has an affinity for binding to the Ab that is approximately equal of the affinity of binding of the Ab to human EpCAM. In some embodiments, the MM has an affinity for binding to the Ab that is no less than the affinity of binding of the Ab to human EpCAM. In some embodiments, the MM has an affinity for binding to the Ab that is greater than the affinity of binding of the Ab to human EpCAM.

In some embodiments, the MM has an affinity for binding to the Ab that is 2, 3, 4, 5, 10, 25, 50, 100, 250, 500, or 1,000 less than the affinity of binding of the Ab to human EpCAM. I In some embodiments, the MM has an affinity for binding to the Ab that is between 1-5, 2-5, 2-10, 5-10, 5-20, 5-50, 5-100, 10-100, 10-1,000, 20-100, 20-1000, or 100- 1,000 fold less than the affinity of binding of the Ab to human EpCAM. In some embodiments, the MM has an affinity for binding to the Ab that is 2 to 20 fold less than the affinity of binding of the Ab to human EpCAM. In some embodiments, a MM not covalently linked to the Ab and at equimolar concentration to the EpCAM activatable antibody does not inhibit the binding of the Ab to human EpCAM.

When the Ab is modified with a MM and is in the presence of human EpCAM specific binding of the Ab to human EpCAM is reduced or inhibited, as compared to the specific binding of the Ab not modified with an MM or the specific binding of the parental Ab to human EpCAM. When compared to the binding of the Ab not modified with an MM or the binding of the parental Ab to human EpCAM, the Ab's ability to bind human EpCAM when modified with an MM can be reduced by at least 50%, 60%, 70%, 80%, 90%, 92%, 93%, 94%, 95%, 96%, 97%, 98%, 99% and even 100% for at least 2, 4, 6, 8, 12, 28, 24, 30, 36, 48, 60, 72, 84, or 96 hours, or 5, 10, 15, 30, 45, 60, 90, 120, 150, or 180 days, or 1, 2, 3, 4, 5, 6, 7, 8, 9, 10, 11, or 12 months or more when measured *in vivo* or in an *in vitro* assay.

The MM inhibits the binding of the Ab to human EpCAM. The MM binds the antigen binding domain of the Ab and inhibits binding of the Ab to human EpCAM. The MM can sterically inhibit the binding of the Ab to human EpCAM. The MM can allosterically inhibit the binding of the Ab to its target. In these embodiments, when the Ab is modified or coupled to a MM and in the presence of target there is no binding or substantially no binding of the Ab to human EpCAM, or no more than 0.001%, 0.01%, 0.1%, 1%, 2%, 3%, 4%, 5%, 6%, 7%, 8%, 9%, 10%, 15%, 20%, 25%, 30%, 35%, 40%, or 50% binding of the Ab to human EpCAM, as compared to the binding of the Ab not modified with an MM, the parental Ab, or the Ab not coupled to an MM to human EpCAM, for at least 2, 4, 6, 8, 12, 28, 24, 30, 36, 48, 60, 72, 84, or 96 hours, or 5, 10, 15, 30, 45, 60, 90, 120, 150, or 180 days, or 1, 2, 3, 4, 5, 6, 7, 8, 9, 10, 11, or 12 months or longer when measured *in vivo* or in an *in vitro* assay.

When an Ab is coupled to or modified by a MM, the MM 'masks' or reduces or otherwise inhibits the specific binding of the Ab to human EpCAM. When an Ab is coupled to or modified by a MM, such coupling or modification can effect a structural change that reduces or inhibits the ability of the Ab to specifically bind its target.

An Ab coupled to or modified with an MM can be represented by the following formulae (in order from an amino (N) terminal region to carboxyl (C) terminal region:

(MM)-(Ab)

(Ab)-(MM)

(MM)-L-(Ab)

(Ab)-L-(MM)

where MM is a masking moiety, the Ab is an EpCAM antibody, EpCAM-bindng antigen fragment, and the L is a linker. In many embodiments, it may be desirable to insert one or more linkers, *e.g.*, flexible linkers, into the composition so as to provide for flexibility.

In certain embodiments, the MM is not a natural binding partner of the Ab. In some embodiments, the MM contains no or substantially no homology to any natural binding partner of the Ab. In some embodiments, the MM is no more than 5%, 10%, 15%, 20%, 25%, 30%, 35%, 40%, 45%, 50%, 55%, 60%, 65%, 70%, 75%, or 80% similar to any natural binding partner of the Ab. In some embodiments, the MM is no more than 5%, 10%, 15%, 20%, 25%, 30%, 35%, 40%, 45%, 50%, 55%, 60%, 65%, 70%, 75%, or 80% identical to any natural binding partner of the Ab. In some embodiments, the MM is no more than 25% identical to any natural binding partner of the Ab. In some embodiments, the MM is no more than 50% identical to any natural binding partner of the Ab. In some embodiments, the MM is no more than 20% identical to any natural binding partner of the Ab. In some embodiments, the MM is no more than 10% identical to any natural binding partner of the Ab.

In some embodiments, MM comprises a sequence disclosed in Table 9. In some embodiments, the MM comprises a sequence selected from SEQ ID NOs:151-157. In some embodiments, the MM comprises a sequence selected from SEQ ID NOs: 158-161. In some embodiments, the MM comprises a sequence selected from SEQ ID NOs:162-167. In some embodiments, the MM comprises the sequence of SEQ ID NO: 155.

**Table 9. Exemplary Masking Moiety Sequences.**

| Mask name | Sequence |
|---|---|
| **Masks for EpCAM23Gv4.2 humanized antibody** | |
| **Ep01-2** | PLMTCSDYYTCLNNL (SEQ ID NO:151) |
| **Ep02** | LSCTHSRYDMHCPHM (SEQ ID NO:152) |
| **Ep03** | HYCHSRTDTITHCNA (SEQ ID NO:153) |
| **Ep04** | WCPRLFDRPSMGCPT (SEQ ID NO:154) |
| **Ep05** | WWPPCQGGAWCEQRI (SEQ ID NO:155) |
| **Ep07** | HSGCPRLFDRCSAPA (SEQ ID NO:156) |
| **Ep11** | FICPTLYDRPHCMHT (SEQ ID NO:157) |
| **Masks for 1565-Y affinity variant** | |
| **Ep101** | SWCHSATDTILPCSN (SEQ ID NO:158) |
| **Ep102** | SPACSDRYYQTCVLN (SEQ ID NO:159) |

(continued)

| Masks for 1565-Y affinity variant | |
|---|---|
| **Ep103** | MSCVVDRFDRQCPHL (SEQ ID NO:160) |
| **Ep104** | TTRCEHYWFTCPLSP (SEQ ID NO:161) |
| **Masks for 1361-H affinity variant** | |
| **Ep105** | DCTGYSPSVLPACRV (SEQ ID NO:162) |
| **Ep106** | FCSGYSPSVLPSCLM (SEQ ID NO:163) |
| **Ep107** | SKPCSYMHPYCFYNS (SEQ ID NO:164) |
| **Ep108** | LTRCTIAHPYCYYNY (SEQ ID NO:165) |
| **Ep109** | PNTCMSERRICSLTY (SEQ ID NO:166) |
| **Ep110** | PRPHCAILRQCLAAT (SEQ ID NO:167) |

The EpCAM activatable antibodies provided herein include a cleavable moiety. In some embodiments, the cleavable moiety (or "substrate") includes an amino acid sequence that is a substrate for a protease, usually an extracellular protease. Suitable substrates are identified using any of a variety of known techniques. For example, peptide substrates are identified using the methods described in US Patent Nos. 7,666,817 and 8,563,269; and WO 2014/026136, the contents of each of which is herein incorporated by reference in its entirety. (*See also,* Boulware et al., Biotechnol Bioeng. 106(3):339-346 (2010)).

In some embodiments, the EpCAM activatable antibodies include an Ab that is modified by an MM and also includes one or more cleavable moieties (CM). Such EpCAM activatable antibodies exhibit activatable/switchable binding, to human EpCAM. The EpCAM activatable antibodies generally include an antibody or antigen-binding antibody fragment (Ab), modified by or coupled to a masking moiety (MM) and a modifiable or cleavable moiety (CM). In some embodiments, the CM contains an amino acid sequence that serves as a substrate for at least one protease.

The elements of the EpCAM activatable antibodies are arranged so that the MM and CM are positioned such that in a cleaved (or relatively active) state and in the presence of human EpCAM, the EpCAM activatable antibody binds human EpCAM, but when the EpCAM activatable antibody is in an uncleaved (or relatively inactive) state in the presence of human EpCAM, specific binding of the EpCAM activatable antibody to human EpCAM is reduced or inhibited. The specific binding of the EpCAM activatable antibody to human EpCAM can be reduced due to the inhibition or masking of the EpCAM activatable antibody's ability to specifically bind human EpCAM by the MM.

The IQ of the EpCAM activatable antibody modified with a MM and a CM towards human EpCAM is at least 5, 10, 25, 50, 100, 250, 500, 1,000, 2,500, 5,000, 10,000, 50,000, 100,000, 500,000, 1,000,000, 5,000,000, 10,000,000, 50,000,000 or greater, or between 5-10, 10-100, 10- 1,000, 10-10,000, 10-100,000, 10-1,000,000, 10-10,000,000, 100-1,000, 100-10,000, 100- 100,000, 100-1,000,000, 100-10,000,000, 1,000-10,000, 1,000-100,000, 1,000-1,000,000, 1000-10,000,000, 10,000-100,000, 10,000-1,000,000, 10,000-10,000,000, 100,000- 1,000,000, or 100,000-10,000,000 times greater than the Kj of the EpCAM activatable antibody not modified with an MM and a CM or of the parental Ab towards human EpCAM. Conversely, the binding affinity of the Ab modified with a MM and a CM towards human EpCAM is at least 5, 10, 25, 50, 100, 250, 500, 1,000, 2,500, 5,000, 10,000, 50,000, 100,000, 500,000, 1,000,000, 5,000,000, 10,000,000, 50,000,000 or greater, or between 5-10, 10-100, 10-1,000, 10-10,000, 10- 100,000, 10-1,000,000, 10-10,000,000, 100-1,000, 100-10,000, 100-100,000, 100- 1,000,000, 100-10,000,000, 1,000-10,000, 1,000-100,000, 1,000-1,000,000, 1000- 10,000,000, 10,000-100,000, 10,000-1,000,000, 10,000-10,000,000, 100,000-1,000,000, or 100,000-10,000,000 times lower than the binding affinity of the EpCAM activatable antibody not modified with an MM and a CM or of the parental Ab towards human EpCAM.

When the EpCAM activatable antibody is modified with a MM and a CM and is in the presence of human EpCAM but not in the presence of a modifying agent (for example at least one protease), specific binding of the EpCAM activatable antibody to human EpCAM is reduced or inhibited, as compared to the specific binding of the EpCAM activatable antibody not modified with an MM and a CM or of the parental Ab to human EpCAM. When compared to the binding of the parental Ab or the binding of an EpCAM activatable antibody not modified with an MM and a CM to human EpCAM, the EpCAM activatable antibody's ability to bind human EpCAM when modified with an MM and a CM can be reduced by at least 50%, 60%, 70%, 80%, 90%, 92%, 93%, 94%, 95%, 96%, 97%, 98%, 99% and even 100% for at least 2, 4, 6, 8, 12, 28, 24, 30, 36, 48, 60, 72, 84, or 96 hours or 5, 10, 15, 30, 45, 60, 90, 120, 150, or 180 days, or 1, 2, 3, 4, 5, 6, 7, 8, 9, 10, 11, or 12 months or longer when measured *in vivo* or in an *in vitro* assay.

As used herein, the term cleaved state refers to the condition of the EpCAM activatable antibodies following modification of the CM by at least one protease. The term uncleaved or intact state, as used herein, refers to the condition of

the EpCAM activatable antibodies in the absence of cleavage of the CM by a protease. As discussed above, the term "activatable antibody" or "activatable antibody" is used herein to refer to an EpCAM activatable antibody, in both its uncleaved (native or intact) state, as well as in its cleaved state. It will be apparent to ordinarily skilled artisan that in some embodiments, a cleaved EpCAM activatable antibody, may lack an MM due to cleavage of the CM by protease, resulting in release of at least the MM (*e.g.*, where the MM is not joined to the EpCAM activatable antibody, by a covalent bond (*e.g.,* a disulfide bond between cysteine residues).

By activatable or switchable is meant that the EpCAM activatable antibody, exhibits a first level of binding to a target when the EpCAM activatable antibody, is in a inhibited, masked, intact or uncleaved state (*i.e.,* a first conformation), and a second level of binding to human EpCAM in the uninhibited, unmasked and/or cleaved state (*i.e.,* a second conformation), where the second level of target binding is greater than the first level of binding. In general, the access of human EpCAM to the Ab of the EpCAM activatable antibody, is greater in the presence of a cleaving agent capable of cleaving the CM, *i.e.,* a protease, than in the absence of such a cleaving agent. Thus, when the EpCAM activatable antibody is in the uncleaved state, the Ab is inhibited from binding human EpCAM and can be masked from human EpCAM-binding (*i.e.,* the first conformation is such that the Ab cannot bind human EpCAM), and in the cleaved state the Ab is not inhibited or is unmasked to target binding.

The CM and Ab of the EpCAM activatable antibodies are selected so that the Ab represents a binding moiety for a given target, and the CM represents a substrate for a protease. In some embodiments, the protease is co-localized with human EpCAM at a treatment site or diagnostic site in a subject. As used herein, co-localized refers to being at the same site or relatively close nearby. In some embodiments, a protease cleaves a CM yielding an activated antibody that binds to a target located nearby the cleavage site. The EpCAM activatable antibodies disclosed herein find particular use where, for example, a protease capable of cleaving a site in the CM, *i.e.,* a protease, is present at relatively higher levels in target-containing tissue of a treatment site or diagnostic site than in tissue of non-treatment sites (for example in healthy tissue). In some embodiments, a CM of the disclosure is also cleaved by one or more other proteases. In some embodiments, it is the one or more other proteases that is co-localized with human EpCAM and that is responsible for cleavage of the CM *in vivo.*

In some embodiments, EpCAM activatable antibodies provide for reduced toxicity and/or adverse side effects that could otherwise result from binding of the EpCAM activatable antibodies at non- treatment sites if the EpCAM activatable antibodies were not masked or otherwise inhibited from binding to human EpCAM.

In general, an EpCAM activatable antibody, can be designed by selecting an Ab of interest and constructing the remainder of the EpCAM activatable antibody, so that, when conformationally constrained, the MM provides for masking of the EpCAM activatable antibodies or reduction of binding of the EpCAM activatable antibodies to human EpCAM. Structural design criteria can be to be taken into account to provide for this functional feature.

EpCAM activatable antibodies exhibiting a switchable phenotype of a desired dynamic range for target binding in an inhibited versus an uninhibited conformation are provided. Dynamic range generally refers to a ratio of (a) a maximum detected level of a parameter under a first set of conditions to (b) a minimum detected value of that parameter under a second set of conditions. For example, in the context of an EpCAM activatable antibody, the dynamic range refers to the ratio of (a) a maximum detected level of target protein binding to an EpCAM activatable antibody, in the presence of at least one protease capable of cleaving the CM of the EpCAM activatable antibodies to (b) a minimum detected level of target protein binding to an EpCAM activatable antibody, in the absence of the protease. The dynamic range of an EpCAM activatable antibody, can be calculated as the ratio of the dissociation constant of an EpCAM activatable antibody, cleaving agent (*e.g.*, enzyme) treatment to the dissociation constant of the EpCAM activatable antibodies cleaving agent treatment. The greater the dynamic range of an EpCAM activatable antibody, the better the switchable phenotype of the EpCAM activatable antibody.

EpCAM activatable antibodies having relatively higher dynamic range values (*e.g.*, greater than 1) exhibit more desirable switching phenotypes such that target protein binding by the EpCAM activatable antibodies occurs to a greater extent (*e.g.*, predominantly occurs) in the presence of a cleaving agent (*e.g.,* enzyme) capable of cleaving the CM of the EpCAM activatable antibodies than in the absence of a cleaving agent.

EpCAM activatable antibodies can be provided in a variety of structural configurations. Exemplary formulae for EpCAM activatable antibodies are provided below. It is specifically contemplated that the N- to C-terminal order of the Ab, MM and CM may be reversed within an EpCAM activatable antibody. It is also specifically contemplated that the CM and MM may overlap in amino acid sequence, *e.g.*, such that the CM is contained within the MM.

For example, EpCAM activatable antibodies can be represented by the following formula (in order from an amino (N) terminal region to carboxyl (C) terminal region:

(MM)-(CM)-(Ab)

(Ab)-(CM)-(MM)

where MM is a masking moiety, CM is a cleavable moiety, and Ab is an EpCAM antibody or an EpCAM-binding antibody

fragment. It should be noted that although MM and CM are indicated as distinct components in the formulae above, in all exemplary embodiments, (including formulae) disclosed herein it is contemplated that the amino acid sequences of the MM and the CM could overlap, *e.g.,* such that the CM is completely or partially contained within the MM. In addition, the formulae above provide for additional amino acid sequences that may be positioned N-terminal or C-terminal to the EpCAM activatable antibodies elements.

In some embodiments, the EpCAM activatable antibody comprises a CM that is cleavable by a protease. In some embodiments, the protease that cleaves the CM is active, *e.g.,* up- regulated or otherwise unregulated, in diseased tissue, and the protease cleaves the CM in the EpCAM activatable antibody, when the EpCAM activatable antibody, is exposed to the protease.

In some embodiments, the protease is co-localized with EpCAM in a tissue, and the protease cleaves the CM in the EpCAM activatable antibody, when the EpCAM activatable antibody, is exposed to the protease.

In some embodiments, the CM is positioned in the EpCAM activatable antibody, such that when the EpCAM activatable antibody, is in the uncleaved state, binding of the EpCAM activatable antibody, to EpCAM is reduced to occur with a dissociation constant that is at least 2, 5, 10, 20, 40, 50, 100, or 200, greater than the dissociation constant of an unmodified Ab binding to EpCAM, whereas in the cleaved state (*i.e.,* when the EpCAM activatable antibody, is in the cleaved state), the Ab binds EpCAM.

In some embodiments, the CM is a polypeptide of up to 15 amino acids in length.

In some embodiments, the CM is a polypeptide that includes a first cleavable moiety (CMl) that is a substrate for at least one matrix metalloprotease (MMP) and a second cleavable moiety (CM2) that is a substrate for at least one serine protease (SP). In some embodiments, each of the CM1 substrate sequence and the CM2 substrate sequence of the CM1-CM2 substrate is independently a polypeptide of up to 15 amino acids in length.

In some embodiments, the CM is a substrate for at least one protease that is or is believed to be up-regulated or otherwise unregulated in cancer. In some embodiments, the CM is a substrate for at least one protease that is or is believed to be up-regulated in inflammation. In some embodiments, the CM is a substrate for at least one protease that is or is believed to be up-regulated or otherwise unregulated in autoimmunity.

In some embodiments, the CM is a substrate for at least one protease selected from a matrix metalloprotease (MMP), thrombin, a neutrophil elastase, a cysteine protease, legumain, and a serine protease, such as matriptase (MT-SPl), and urokinase (uPA). Without being bound by theory, it is believed that these proteases are up-regulated or otherwise unregulated in at least one of cancer, inflammation, and/or autoimmunity.

Exemplary substrates include but are not limited to substrates cleavable by one or more of the following enzymes or proteases: an ADAMS/ADAMTS, (*e.g.*, ADAM8, ADAM9, ADAM 10, ADAM 12, ADAM 15, ADAM 17/TACE, ADAMDEC1, ADAMTS1, ADAMTS4, ADAMTS5); an aspartate protease (*e.g.*, BACE, Renin); an aspartic cathepsin (*e.g.,* Cathepsin D and Cathepsin E); a caspase (*e.g.,* Caspase 1-10, and Caspase 14); a cysteine cathepsin (*e.g.*, Cathepsin B, Cathepsin C, Cathepsin K, Cathepsin L, Cathepsin S, Cathepsin V/L2, and Cathepsin X/Z/P); a cysteine proteinase (*e.g.*, Cruzipain, Legumain, and Otubain-2); a KLK (*e.g.,* KLK4-8, KLK10, KLK 11, KLK 13, and KLK14 ); a metalloproteinase (*e.g.,* Meprin, Neprilysin, PSMA, and BMP1); an MMP (*e.g.*, MMP1-3, MMP 7-17, MMP 19,, MMP 20, MMP 23, MMP 24, MMP 26, and MMP 27); a serine protease (*e.g.*, activated protein C, Cathepsin A, Cathepsin C, Chymase, and a coagulation factor protease such as FVIIa, FIXa, FXa, FXIa, and FXIIa), an Elastase (*e.g.*, human Neutrophil Elastase); Granzyme B; Guanidinobenzoatase; HtrAl; Lactoferrin; Marapsin; NS3/4A; PACE4; Plasmin; PSA, tPA; Thrombin; Tryptase; uPA; a Type II Transmembrane Serine Protease (TTSP) (*e.g.*, DESC1, DPP-4, FAP, Hepsin, Matriptase-2, MT-SPl/Matriptase, and TMPRSS2-4).

In some embodiments, the CM is selected for use with a specific protease, for example a protease that is known to be co-localized with the target of the EpCAM activatable antibody.

In some embodiments, the CM is a substrate for at least one MMP. Examples of MMPs include MMP1-3, MMP 7-17, MMP19, MMP20, MMP23, MMP24, MMP26, and MMP27. In some embodiments, the CM is a substrate for a protease selected from MMP 9, MMP 14, MMP1, MMP3, MMP13, MMP 17, MMP11, and MMP19. In some embodiments, the CM is a substrate for MMP9. In some embodiments, the CM is a substrate for MMP14.

Suitable CM that can routinely be incorporated into the provided activatable antibodies are known in the art. *See,* for example, WO 2016/179285, e.g., pages 40-47, the contents of which is herein incorporated by reference in its entirety.

In some embodiments, the CM is a substrate for a neutrophil elastase. In some embodiments, the CM is a substrate for a serine protease. In some embodiments, the CM is a substrate for legumain. In some embodiments, the CM is a substrate for matriptase. In some embodiments, the CM is a substrate for a cysteine protease. In some embodiments, the CM is a substrate for a cysteine protease, such as a cathepsin. In other embodiments, the CM is a substrate for a uPA.

In particular embodiments, the CM is a substrate for uPA. In some embodiments, the CM comprises a sequence disclosed in Table 10.

**Table 10. Exemplary CM Sequences.**

| Substrate name | Sequence |
|---|---|
| **3014** | AVGLLAPPGGLSGRSDNI (SEQ ID NO:168) |
| **2014** | ISSGLLSGRSDNI (SEQ ID NO:169) |

In some embodiments, the CM comprises the sequence AVGLLAPPGGLSGRSDNI (SEQ ID NO:168).

In some embodiments, the CM comprises the sequence ISSGLLSGRSDNI (SEQ ID NO:169).

In some embodiments, the CM is a substrate for at least two proteases. In some embodiments, each protease is selected from an ADAMS/ADAMTS, (*e.g.*, ADAM8, ADAM9, ADAM 10, ADAM 12, ADAM 15, ADAM 17/TACE, ADAMDEC1, ADAMTS1, ADAMTS4, ADAMTS5); an aspartate protease (*e.g.*, BACE, Renin); an aspartic cathepsin (*e.g.,* Cathepsin D and Cathepsin E); a caspase (*e.g.,* Caspase 1-10, and Caspase 14); a cysteine cathepsin (*e.g.*, Cathepsin B, Cathepsin C, Cathepsin K, Cathepsin L, Cathepsin S, Cathepsin V/L2, and Cathepsin X/Z/P); a cysteine proteinase (*e.g.*, Cruzipain, Legumain, and Otubain-2); a KLK (*e.g.,* KLK4-8, KLK10, KLK 11, KLK 13, and KLK14 ); a metalloproteinase (*e.g.,* Meprin, Neprilysin, PSMA, and BMP1); an MMP (*e.g.,* MMP1-3, MMP 7-17, MMP 19,, MMP 20, MMP 23, MMP 24, MMP 26, and MMP 27); a serine protease (*e.g.*, activated protein C, Cathepsin A, Cathepsin C, Chymase, and a coagulation factor protease such as FVIIa, FIXa, FXa, FXIa, and FXIIa), an Elastase (*e.g.*, human Neutrophil Elastase); Granzyme B; Guanidinobenzoatase; HtrAl; Lactoferrin; Marapsin; NS3/4A; PACE4; Plasmin; PSA, tPA; Thrombin; Tryptase; uPA; a Type II Transmembrane Serine Protease (TTSP) (*e.g.,* DESC1, DPP-4, FAP, Hepsin, Matriptase-2, MT-SPl/Matriptase, and TMPRSS2-4. In some embodiments, the CM is a substrate for at least two proteases, wherein one of the proteases is selected from: a MMP, thrombin, a neutrophil elastase, a cysteine protease, uPA, legumain and matriptase and the other protease is selected from those listed above. In some embodiments, the CM is a substrate for at least two proteases selected from the group: a MMP, thrombin, a neutrophil elastase, a cysteine protease, uPA, legumain and matriptase.

In some embodiments, the EpCAM activatable antibody includes at least a first CM and a second CM. In some embodiments, the first CM and the second CM are each polypeptides of no more than 15 amino acids long. In some embodiments, the first CM and the second CM in the EpCAM activatable antibody, in the uncleaved state have the structural arrangement from N-terminus to C-terminus as follows: MM-CM1-CM2-Ab or Ab-CM2-CM1-MM. In some embodiments, at least one of the first CM and the second CM is a polypeptide that functions as a substrate for a protease selected from a MMP, thrombin, a neutrophil elastase, a cysteine protease, uPA, legumain, and matriptase. In some embodiments, the first CM is cleaved by a first cleaving agent selected from MMP, thrombin, a neutrophil elastase, a cysteine protease, uPA, legumain, and matriptase in a target tissue and the second CM is cleaved by a second cleaving agent in a target tissue. In some embodiments, the other protease is selected from the list presented in the preceding paragraph. In some embodiments, the first cleaving agent and the second cleaving agent are the same protease selected from a MMP, thrombin, a neutrophil elastase, a cysteine protease, uPA, legumain, and matriptase, and the first CM and the second CM are different substrates for the enzyme. In some embodiments, the first cleaving agent and the second cleaving agent are the same protease selected from the list in the preceding paragraph. In some embodiments, the first cleaving agent and the second cleaving agent are different proteases. In some embodiments, the first cleaving agent and the second cleaving agent are co-localized in the target tissue. In some embodiments, the first CM and the second CM are cleaved by at least one cleaving agent in the target tissue.

In some embodiments, the EpCAM activatable antibody, also includes a signal peptide. In some embodiments, the signal peptide is conjugated to the EpCAM activatable antibody, *via* a spacer. In some embodiments, the spacer is conjugated to the EpCAM activatable antibody, in the absence of a signal peptide. In some embodiments, the spacer is joined directly to the MM of the EpCAM activatable antibody. In some embodiments, the spacer is joined directly to the MM of the EpCAM activatable antibody, in the structural arrangement from N-terminus to C- terminus of spacer-MM-CM-Ab.

Suitable spacers and spacer technology is known in the art and can routinely be used to incorporate spacers in some embodiments of the provided activatable antibodies. *See,* for example, WO 2016/179285 (*e.g.,* at pages 52-53), the contents of which is herein incorporated by reference in its entirety.

In many embodiments, it may be desirable to insert one or more linkers, *e.g.*, flexible linkers, into the EpCAM activatable antibody, construct so as to provide for flexibility at one or more of the MM-CM junction, the CM-Ab junction, or both. For example, the Ab, MM, and/or CM may not contain a sufficient number of residues (*e.g.*, Gly, Ser, Asp, Asn, especially Gly and Ser, particularly Gly) to provide the desired flexibility. As such, the switchable phenotype of such EpCAM activatable antibody, constructs may benefit from introduction of one or more amino acids to provide for a flexible linker. In addition, as described below, where the EpCAM activatable antibody, is provided as a conformationally constrained construct, a flexible linker can be operably inserted to facilitate formation and maintenance of a cyclic structure in the uncleaved EpCAM activatable antibody.

For example, in certain embodiments, an EpCAM activatable antibody, comprises one of the following formulae

(where the formula below represent an amino acid sequence in either N- to C-terminal direction or C- to N-terminal direction):

(MM)-L1-(CM)-(Ab)

(MM)-(CM)-L2-(Ab)

(MM)-L1-(CM)-L2-(Ab)

wherein MM, CM, and Ab are as defined above; wherein LI and L2 are each independently and optionally present or absent, are the same or different flexible linkers that include at least 1 flexible amino acid (*e.g.,* Gly). In addition, the formulae above provide for additional amino acid sequences that may be positioned N-terminal or C-terminal to the EpCAM activatable antibodies elements. Examples include, but are not limited to, targeting moieties (*e.g.*, a ligand for a receptor of a cell present in a target tissue) and serum half-life extending moieties (*e.g.*, polypeptides that bind serum proteins, such as immunoglobulin (*e.g.*, IgG) or serum albumin (*e.g.,* human serum albumin (HSA)).

In some embodiments, the EpCAM activatable antibody is exposed to and cleaved by a protease such that, in the activated or cleaved state, the activated antibody includes a light chain sequence that includes at least a portion of LP2 and/or CM sequence after the protease has cleaved the CM.

The CM is specifically cleaved by at least one protease at a rate of about 0.001-1500 x $10^4$ $M^{-1}S^{-1}$ or at least 0.001, 0.005, 0.01, 0.05, 0.1, 0.5, 1, 2.5, 5, 7.5, 10, 15, 20, 25, 50, 75, 100, 125, 150, 200, 250, 500, 750, 1000, 1250, or 1500 x $10^4$ $M^{-1}S^{-1}$. In some embodiments, the CM is specifically cleaved at a rate of about 100,000 $M^{-1}S^{-1}$. In some embodiments, the CM is specifically cleaved at a rate from about 1x$10^2$ to about 1 x $10^6$ $M^{-1}S^{-1}$ (*i.e.,* from about 1 x$10^2$ to about 1 x $10^6$ $M^{-1}S^{-1}$).

For specific cleavage by an enzyme, contact between the enzyme and CM is made. When the EpCAM activatable antibody, comprising an Ab (*e.g.,* an EpCAM antibody or EpCAM-binding antibody fragment) coupled to a MM and a CM is in the presence of EpCAM and sufficient enzyme activity, the CM can be cleaved. Sufficient enzyme activity can refer to the ability of the enzyme to make contact with the CM and effect cleavage. It can readily be envisioned that an enzyme may be in the vicinity of the CM but unable to cleave because of other cellular factors or protein modification of the enzyme.

Linkers suitable for use in EpCAM activatable antibody, compositions disclosed herein are generally ones that provide flexibility of the modified Ab (*e.g.,* an EpCAM antibody or EpCAM-binding antibody fragment) or the EpCAM activatable antibody, to facilitate the inhibition of the binding of the EpCAM activatable antibody to human EpCAM. Such linkers are generally referred to as flexible linkers. Suitable linkers can be readily selected and can be of any of a suitable of different lengths, such as from 1 amino acid (*e.g.,* Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and may be 1, 2, 3, 4, 5, 6, 7, 8, 9, 10, 11, 12, 13, 14, 15, 16, 17, 18, 19, or 20 amino acids in length.

Exemplary flexible linkers for the activatable antibodies, antibodies, and antibody fragments provided herein include, glycine polymers (G)n, glycine-serine polymers (including, for example, (GS)n. Suitable linkers and linker technology are known in the art and can routinely be used to incorporate spacers in some embodiments of the provided activatable antibodies. *See,* for example, WO 2016/179285 (*e.g.,* at pages 26, 113-116), the contents of which is herein incorporated by reference in its entirety. The ordinarily skilled artisan will recognize that design of an EpCAM activatable antibodies can include linkers that are all or partially flexible, such that the linker can include a flexible linker as well as one or more portions that confer less flexible structure to provide for a desired EpCAM activatable antibodies structure.

In some embodiments, the EpCAM activatable antibody comprises a first linking peptide (LP1) and a second linking peptide (LP2), and wherein the EpCAM activatable antibody, in the uncleaved state has the structural arrangement from N-terminus to C-terminus as follows: MM-LP1-CM-LP2-Ab or Ab-LP2-CM-LP1-MM. In some embodiments, the two linking peptides need not be identical to each other.

In some embodiments, the EpCAM activatable antibody, comprises a first linking peptide (LP1) and a second linking peptide (LP2), and wherein the EpCAM activatable antibody, in the uncleaved state has the structural arrangement from N-terminus to C-terminus as follows: MM-LP1-CM-LP2-Ab or Ab-LP2-CM-LP1-MM. In some embodiments, the two linking peptides need not be identical to each other.

In some embodiments, at least one of LP1 or LP2 of the EpCAM activatable antibody comprises a flexible linker. Suitable linkers and linker technology are known in the art and can routinely be used to incorporate spacers in some embodiments of the provided activatable antibodies. *See,* for example, WO 2016/179285 (*e.g.,* at pages 26, 113-116), the contents of which is herein incorporated by reference in its entirety.

In some embodiments, the EpCAM activatable antibody comprises a light chain having a sequence disclosed in Table 11. In some embodiments, the activatable antibody comprises a light chain having the sequence of SEQ ID NO:174.

In some embodiments, the activatable antibody comprises a light chain having the sequence of SEQ ID NO:179.

**Table 11. Exemplary light chain activatable antibody sequences.**

| Activatable antibody name | Sequence |
|---|---|
| **For use with heavy chain of HuEpCAM23Gv4.2, 1361-H, or 1565-Y** | |
| **Ep01-2 3014 Lv** | QGQSGQGPLMTCSDYYTCLNNLGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:170) |
| **Ep02 3014 Lv** | QGQSGQGLSCTHSRYDMHCPHMGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:171) |
| **Ep03 3014 Lv** | QGQSGQGHYCHSRTDTITHCNAGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:172) |
| **Ep04 3014 Lv** | QGQSGQGWCPRLFDRPSMGCPTGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:173) |
| **Ep05 3014 Lv** | QGQSGQGWWPPCQGGAWCEQRIGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:174) |
| **Ep07 3014 Lv** | QGQSGQGHSGCPRLFDRCSAPAGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:175) |
| **Ep11 3014 Lv** | QGQSGQGFICPTLYDRPHCMHTGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:176) |

(continued)

| Activatable antibody name | Sequence |
|---|---|
| For use with heavy chain of HuEpCAM23Gv4.2, 1361-H, or 1565-Y | |
| Ep01-2 2014 Lv | QGQSGQGPLMTCSDYYTCLNNLGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:177) |
| Ep04 2014 Lv | QGQSGQGWCPRLFDRPSMGCPTGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:178) |
| Ep05 2014 Lv | QGQSGQGWWPPCQGGAWCEQRIGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:179) |
| Ep07 2014 Lv | QGQSGQGHSGCPRLFDRCSAPAGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:180) |
| For use with heavy chain of 1565-Y | |
| Ep101 2014 Lv | QGQSGQGSWCHSATDTILPCSNGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:181) |
| Ep102 2014 Lv | QGQSGQGSPACSDRYYQTCVLNGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:182) |
| Ep103 2014 Lv | QGQSGQGMSCVVDRFDRQCPHLGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:183) |

(continued)

| For use with heavy chain of 1565-Y | |
|---|---|
| **Ep104 2014 Lv** | QGQSGQGTTRCEHYWFTCPLSPGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:184) |
| **Ep1013014 Lv** | QGQSGQGSWCHSATDTILPCSNGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:185) |
| **Ep102 3014 Lv** | QGQSGQGSPACSDRYYQTCVLNGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:186) |
| **Ep103 3014 Lv** | QGQSGQGMSCVVDRFDRQCPHLGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:187) |
| **fEp104 3014 Lv** | QGQSGQGTTRCEHYWFTCPLSPGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQS |
| | GNSQESVTEQDSKDSTYSLSSTLTLSKADYEKHKVYACEVTHQGLSSPVTKSFNRGEC (SEQ ID NO:188) |
| **For use with heavy chain of 1361-H** | |
| **Ep105 2014 Lv** | QGQSGQGDCTGYSPSVLPACRVGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:189) |
| **Ep106 2014 Lv** | QGQSGQGFCSGYSPSVLPSCLMGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:190) |

(continued)

| For use with heavy chain of 1361-H | |
|---|---|
| Ep107 2014 Lv | QGQSGQGSKPCSYMHPYCFYNSGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:191) |
| Ep108 2014 Lv | QGQSGQGLTRCTIAHPYCYYNYGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:192) |
| Ep109 2014 Lv | QGQSGQGPNTCMSERRICSLTYGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:193) |
| Ep110 2014 Lv | QGQSGQGPRPHCAILRQCLAATGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:194) |
| Ep105 3014 Lv | QGQSGQGDCTGYSPSVLPACRVGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:195) |
| Ep106 3014 Lv | QGQSGQGFCSGYSPSVLPSCLMGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLE |
| | IKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:196) |
| Ep107 3014 Lv | QGQSGQGSKPCSYMHPYCFYNSGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:197) |

(continued)

| For use with heavy chain of 1361-H | |
|---|---|
| Ep108 3014 Lv | QGQSGQGLTRCTIAHPYCYYNYGGGSSGGAVGLLAPPGGLSGRSDNIGGSDIVLTQT PLSLSVTPGQPASISCRSSRSLLHSDGFTYLYWFLQKPGQSPQLLIYQTSNL ASGVPDRFSSSGSGTDFTLKISRVEAEDVGVYYCAQNLELPNTFGQGTKLE IKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVT KSFNRGEC (SEQ ID NO:198) |
| Ep109 2014 Lv | QGQSGQGPNTCMSERRICSLTYGGGSSGGAVGLLAPPGGLSGRSDNIGGSDIVLTQT PLSLSVTPGQPASISCRSSRSLLHSDGFTYLYWFLQKPGQSPQLLIYQTSNL ASGVPDRFSSSGSGTDFTLKISRVEAEDVGVYYCAQNLELPNTFGQGTKLE IKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVT KSFNRGEC (SEQ ID NO:199) |
| Ep110 3014 Lv | QGQSGQGPRPHCAILRQCLAATGGGSSGGAVGLLAPPGGLSGRSDNIGGSDIVLTQT PLSLSVTPGQPASISCRSSRSLLHSDGFTYLYWFLQKPGQSPQLLIYQTSNL ASGVPDRFSSSGSGTDFTLKISRVEAEDVGVYYCAQNLELPNTFGQGTKLE IKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVT KSFNRGEC (SEQ ID NO:200) |

In some embodiments, the EpCAM activatable antibody comprises a light chain having a sequence selected from SEQ ID NOs: 170-180 and a heavy chain having a sequence selected from SEQ ID NOs: 103, 125, and 127. In some embodiments, the EpCAM activatable antibody comprises a light chain having the sequence selected from SEQ ID NOs: 170-180 and a heavy chain having the sequence of SEQ ID NO: 103. In some embodiments, the EpCAM activatable antibody comprises a light chain having the sequence of SEQ ID NO:174 and a heavy chain having the sequence of SEQ ID NO: 103. In some embodiments, the EpCAM activatable antibody comprises a light chain having the sequence of SEQ ID NO:179 and a heavy chain having the sequence of SEQ ID NO: 103.

In some embodiments, the EpCAM activatable antibody comprises a light chain having a sequence selected from SEQ ID NOs: 181-188 and a heavy chain having the sequence of SEQ ID NO:127. In some embodiments, the EpCAM activatable antibody comprises a light chain having a sequence selected from SEQ ID NOs: 189-200 and a heavy chain having the sequence of SEQ ID NO:125.

In certain embodiments, the huEpCAM23 antibody is encoded by the plasmids deposited with the American Type Culture Collection (ATCC), located at 10801 University Boulevard, Manassas, Va. 20110 on October 4, 2018 under the terms of the Budapest Treaty and having ATCC deposit nos. PTA-125343 and PTA-125344 or PTA-125345. Examples of EpCAM antibody, EpCAM-binding antibody fragment, and EpCAM activatable antibody, immunoconjugates are provided herein.

## Polynucleotides, Vectors, Host cells and Recombinant Methods

The disclosure further provides polynucleotides comprising a nucleotide sequence encoding the EpCAM antibodies, EpCAM-binding antibody fragments, and EpCAM activatable antibodies disclosed herein.

The polynucleotides may be obtained, and the nucleotide sequence of the polynucleotides determined, using methods known in the art. For example, if the nucleotide sequence of the antibody is known, a polynucleotide encoding the antibody may be assembled from chemically synthesized oligonucleotides (*e.g.,* as described in Kutmeier et al., BioTechniques 17:242 (1994)) which, briefly, involves the synthesis of overlapping oligonucleotides containing portions of the sequence encoding the antibody, annealing and ligation of those oligonucleotides, and then amplification of the ligated oligonucleotides by PCR.

In some embodiments, a polynucleotide of the disclosure comprises a sequence set forth in Table 12.

**Table 12. Exemplary Nucleic Acid Sequences.**

| Antibody name | Sequence |
|---|---|
| **Chimeric Antibody 23** | |
| **Ch Epcam2 3HC** | gaggtgaagctggaggagagcggccccgccctggtgaagcctggagcttccgtgaggatcagctgtaaggcctctggcta caccttttacaaactactatatccattgggtgaagcagcggccaggacagggcctggactacatcggctggatctatcccggca acgtgtacatccagtataatgagaagttcaagggcaaggccaccctgacagctgataagtccagctctaccgcttttatgcagc tgtccagcctgacaagcgaggactctgccgtgtacttctgcgctagggatggcccttggtttgcctattggggccagggcacc ctggtgacagtgtcttccgcttccaccaagggcccatcagttttccccttggctccaagttctaaatccacaagcggtggaacag ctgcactgggatgcctcgttaaagattatttccctgagcctgtgacagtgagctggaatagcggagcattgacttcaggtgtgca |
| | cacttttcccgctgtgttgcagtcctccggtctgtactcactgtccagtgtcgtaaccgtcccttctagcagcttgggaacccaga cctacatctgtaacgtcaaccataaaccatccaacacaaaggtggataagaaggttgaaccaaagagctgtgataagacacat acatgccctccttgtcctgcaccagagctcctcggaggtccatctgtgttcctgtttcccccccaaacccaaggacactcttatgat ctctcgtactccagaggtcacctgtgttgttgtcgacgtgagccatgaagatcccgaggttaaattcaactggtacgtggatgga gtcgaggttcacaatgccaagaccaagcccagggaggagcaatataattctacatatcgggtagtgagcgttctgaccgtgct ccaccaagattggctcaatggaaaagagtacaagtgcaaggtgtccaacaaggctcttcccgctcccattgagaaaactatct ccaaagccaaggggcagccacgggaaccccaggtgtatacattgcccccatctagagacgagctgaccaagaaccaggtg agtctcacttgtctggtcaaggggttttacccttctgacattgctgtagagtgggagtctaacggacagccagaaaacaactaca agacaactcccccagtgctggacagcgacgggagcttcttcctctactccaagttgactgtagacaagtctagatggcagcaa ggaaacgttttctcctgctcagtaatgcatgaggctctgcacaatcactatacccagaaatcactgtcccttagcccaggg (SEQ ID NO:201) |
| **Ch EpCam2 3LC** | gatattgtgctgacccagactccattctccaatcccgtcactcttggaacatcagcttccatctcctgcaggtctagtaagagtctc ctacatagtgatggcttcacttatttgtattggtttctgcagaagccaggccagtctcctcatctcctgatttatcagacgtccaacct tgcctcaggagtcccagacaggttcagtagcagtgggtcaggaactgatttcacactgagaatcagcagagtggaggctgaa gatgtgggtgtttattactgtgctcaaaatctagaacttcccaacacgttcggagggggggaccaagctggaaatcaaacgtacg gtggctgcaccatctgtcttcatcttcccgccatctgatgagcagttgaaatctggaactgcctctgttgtgtgcctgctgaataac ttctatcccagagaggccaaagtacagtggaaggtggataacgccctccaatcgggtaactcccaggagagtgtcacagagc aggacagcaaggacagcacctacagcctcagcagcaccctgacgctgagcaaagcagactacgagaaacacaaagtctac gcctgcgaagtcacccatcagggcctgagctcgcccgtcacaaagagcttcaacaggggagagtgt (SEQ ID NO:202) |
| **Humanized variant 23Gv4.2** | |

(continued)

| Antibody name | Sequence |
|---|---|
| **Chimeric Antibody 23** | |
| **Hu EpCAM 23_VHG v2** | caggtgcagctggtgcagagcggagctgaggtgaagaagccaggagcttccgtgaaggtgagctgtaaggcctctggcta caccttcacaaactactatatccattgggtgaggcaggctccaggacagcggctggagtacatcggatggatctatcctggca acgtgtacatccagtataatgagaagtttaagggcagggccaccctgacagctgacaagagcgcctctaccgcttacatgga gctgtccagcctgagatctgaggacacagccgtgtactattgcgctcgcgatggcccttggtttgcctattggggccagggca ccctggtgacagtgtcttccgcttccaccaagggcccatcagttttccccttggctccaagttctaaatccacaagcggtggaac agctgcactgggatgcctcgttaaagattatttccctgagcctgtgacagtgagctggaatagcggagcattgacttcaggtgt gcacacttttcccgctgtgttgcagtcctccggtctgtactcactgtccagtgtcgtaaccgtcccttctagcagcttgggaaccc agacctacatctgtaacgtcaaccataaaccatccaacacaaaggtggataagaaggttgaaccaaagagctgtgataagac acatacatgccctccttgtcctgcaccagagctcctcggaggtccatctgtgttcctgtttccccccaaacccaaggacactctta tgatctctcgtactccagaggtcacctgtgttgttgtcgacgtgagccatgaagatcccgaggttaaattcaactggtacgtggat ggagtcgaggttcacaatgccaagaccaagcccagggaggagcaatataattctacatatcgggtagtgagcgttctgaccg tgctccaccaagattggctcaatggaaaagagtacaagtgcaaggtgtccaacaaggctcttcccgctcccattgagaaaact atctccaaagccaaggggcagccacgggaaccccaggtgtatacattgcccccatctagagacgagctgaccaagaacca ggtgagtctcacttgtctggtcaaggggttttacccttctgacattgctgtagagtgggagtctaacggacagccagaaaacaa ctacaagacaactcccccagtgctggacagcgacgggagcttcttcctctactccaagttgactgtagacaagtctagatggca gcaaggaaacgttttctcctgctcagtaatgcatgaggctctgcacaatcactatacccagaaatcactgtcccttagcccaggg (SEQ ID NO:203) |
| **Hu EpCAM 23_VLG v4** | gacatcgtgctgacccagacaccactgtctctgtccgtgaccccaggacagcctgctagcatctcttgtaggtccagcaggtc cctgctgcatagcgatggcttcacctacctgtattggtttctgcagaagccaggccagtctccccagctgctgatctaccagaca tctaacctggcttccggcgtgcctgacaggttctcttccagcggcagcggcaccgacttcaccctgaagatctctcgggtgga ggctgaggacgtgggcgtgtactattgcgctcagaacctggagctgccaaatacctttggccagggcacaaagctggagatc aagcgtacggtggctgcaccatctgtcttcatcttcccgccatctgatgagcagttgaaatctggaactgcctctgttgtgtgcct gctgaataacttctatcccagagaggccaaagtacagtggaaggtggataacgccctccaatcgggtaactcccaggagagt gtcacagagcaggacagcaaggacagcacctacagcctcagcagcaccctgacgctgagcaaagcagactacgagaaac acaaagtctacgcctgcgaagtcacccatcagggcctgagctcgcccgtcacaaagagcttcaacaggggagagtgt (SEQ ID NO:204) |

(continued)

| Affinity variants (Heavy Chain) | |
|---|---|
| Hu EpCam2 3HG2-1361-H Heavy Chain | caggtgcagctggtgcagagcggagctgaggtgaagaagccaggagcttccgtgaaggtgagctgtaaggcctctggctacaccttcacaaactaccacatccattgggtgaggcaggctccaggacagcggctggagtacatcggatggatctatcctggcaacgtgtacatccagtataatgagaagtttaagggcagggccaccctgacagctgacaagagcgcctctaccgcttacatggagctgtccagcctgagatctgaggacacagccgtgtactattgcgctcgcgatggcccttggtttgcctattggggccagggcaccctggtgacagtgtcttccgcttccaccaagggcccatcagttttccccttggctccaagttctaaatccacaagcggtggaacagctgcactgggatgcctcgttaaagattatttccctgagcctgtgacagtgagctggaatagcggagcattgacttcaggtgtgcacacttttcccgctgtgttgcagtcctccggtctgtactcactgtccagtgtcgtaaccgtcccttctagcagcttgggaacccagacctacatctgtaacgtcaaccataaaccatccaacacaaaggtggataagaaggttgaaccaaagagctgtgataagacacatacatgccctccttgtcctgcaccagagctcctcggaggtccatctgtgttcctgtttcccccaaacccaaggacactcttatgatctctcgtactccagaggtcacctgtgttgttgtcgacgtgagccatgaagatcccgaggttaaattcaactggtacgtggatggagtcgaggttcacaatgccaagaccaagcccagggaggagcaatataattctacatatcgggtagtgagcgttctgaccgtgctccaccaagattggctcaatggaaaagagtacaagtgcaaggtgtccaacaaggctcttcccgctcccattgagaaaactatctccaaagccaaggggcagccacgggaaccccaggtgtatacattgcccccatctagagacgagctgaccaagaaccaggtgagtctcacttgtctggtcaaggggttttacccttctgacattgctgtagagtgggagtctaacggacagccagaaaacaactacaagacaactcccccagtgctggacagcgacgggagcttcttcctctactccaagttgactgtagacaagtctagatggcagcaaggaaacgttttctcctgctcagtaatgcatgaggctctgcacaatcactatacccagaaatcactgtcccttagcccagggtga (SEQ ID NO:205) |

(continued)

| Affinity variants (Heavy Chain) | |
|---|---|
| Hu EpCam2 3HG2-1361-D Heavy Chain | caggtgcagctggtgcagagcggagctgaggtgaagaagccaggagcttccgtgaaggtgagctgtaaggcctctggcta caccttcacaaactacgacatccattgggtgaggcaggctccaggacagcggctggagtacatcggatggatctatcctggc aacgtgtacatccagtataatgagaagtttaagggcagggccaccctgacagctgacaagagcgcctctaccgcttacatgga gctgtccagcctgagatctgaggacacagccgtgtactattgcgctcgcgatggcccttggtttgcctattggggccagggca ccctggtgacagtgtcttccgcttccaccaagggcccatcagttttccccttggctccaagttctaaatccacaagcggtggaac agctgcactgggatgcctcgttaaagattatttccctgagcctgtgacagtgagctggaatagcggagcattgacttcaggtgt gcacacttttcccgctgtgttgcagtcctccggtctgtactcactgtccagtgtcgtaaccgtcccttctagcagcttgggaaccc agacctacatctgtaacgtcaaccataaaccatccaacacaaaggtggataagaaggttgaaccaaagagctgtgataagac acatacatgccctccttgtcctgcaccagagctcctcggaggtccatctgtgttcctgtttccccccaaacccaaggacactctta tgatctctcgtactccagaggtcacctgtgttgttgtcgacgtgagccatgaagatcccgaggttaaattcaactggtacgtggat ggagtcgaggttcacaatgccaagaccaagcccagggaggagcaatataattctacatatcgggtagtgagcgttctgaccg tgctccaccaagattggctcaatggaaaagagtacaagtgcaaggtgtccaacaaggctcttcccgctcccattgagaaaact atctccaaagccaaggggcagccacgggaaccccaggtgtatacattgcccccatctagagacgagctgaccaagaacca ggtgagtctcacttgtctggtcaaggggttttacccttctgacattgctgtagagtgggagtctaacggacagccagaaaacaa ctacaagacaactcccccagtgctggacagcgacgggagcttcttcctctactccaagttgactgtagacaagtctagatggca gcaaggaaacgttttctcctgctcagtaatgcatgaggctctgcacaatcactatacccagaaatcactgtcccttagcccaggg tga (SEQ ID NO:206) |
| Hu EpCam2 3HG2-1565-Y Heavy Chain | caggtgcagctggtgcagagcggagctgaggtgaagaagccaggagcttccgtgaaggtgagctgtaaggcctctggcta caccttcacaaactactatatccattgggtgaggcaggctccaggacagcggctggagtacatcggatggatctatcctggca acgtgtacatccagtataatgagaagtttaagggcagggccaccctgacagctgacaagagcgcctctaccgcttacatgga gctgtccagcctgagatctgaggacacagccgtgtactattgcgctcgcgatggctactggtttgcctattggggccagggca ccctggtgacagtgtcttccgcttccaccaagggcccatcagttttccccttggctccaagttctaaatccacaagcggtggaac agctgcactgggatgcctcgttaaagattatttccctgagcctgtgacagtgagctggaatagcggagcattgacttcaggtgt gcacacttttcccgctgtgttgcagtcctccggtctgtactcactgtccagtgtcgtaaccgtcccttctagcagcttgggaaccc agacctacatctgtaacgtcaaccataaaccatccaacacaaaggtggataagaaggttgaaccaaagagctgtgataagac acatacatgccctccttgtcctgcaccagagctcctcggaggtccatctgtgttcctgtttccccccaaacccaaggacactctta tgatctctcgtactccagaggtcacctgtgttgttgtcgacgtgagccatgaagatcccgaggttaaattcaactggtacgtggat ggagtcgaggttcacaatgccaagaccaagcccagggaggagcaatataattctacatatcgggtagtgagcgttctgaccg tgctccaccaagattggctcaatggaaaagagtacaagtgcaaggtgtccaacaaggctcttcccgctcccattgagaaaact atctccaaagccaaggggcagccacgggaaccccaggtgtatacattgcccccatctagagacgagctgaccaagaacca |

(continued)

| Affinity variants (Heavy Chain) | |
|---|---|
| | ggtgagtctcacttgtctggtcaaggggttttacccttctgacattgctgtagagtgggagtctaacggacagccagaaaacaactacaagacaactcccccagtgctggacagcgacgggagcttcttcctctactccaagttgactgtagacaagtctagatggcagcaaggaaacgttttctcctgctcagtaatgcatgaggctctgcacaatcactatacccagaaatcactgtcccttagcccagggtga (SEQ ID NO:207) |
| **Activatable antibody Light Chains (huEpCAMGv4.2 Antibody)** | |
| **Ep05 3014 Lv** | cagggacagtctggacagggatggtggccaccttgccagggaggagcttggtgtgagcagaggatcggaggaggctccagcggaggagctgtgggcctgctggctccaccaggaggactgtctggcagatccgacaacatcggcggctccgatatcgtgctgacccagacacccctgagcctgtctgtgacccctggccagccagcctccatcagctgcaggtcttcccggtccctgctgcatagcgacggcttcacctacctgtattggtttctgcagaagcccggccagagccctcagctgctgatctaccagacatctaatctggcttccggcgtgccagacagattcagctcttccggcagcggcaccgacttcaccctgaagatctctcgcgtggaggccgaggatgtgggcgtgtactattgtgctcagaacctggagctgcccaatacctttggccagggcacaaagctggagatcaagcgtacggtggctgcaccatctgtcttcatcttcccgccatctgatgagcagttgaaatctggaactgcctctgttgtgtgcctgctgaataacttctatcccagagaggccaaagtacagtggaaggtggataacgccctccaatcgggtaactcccaggagagtgtcacagagcaggacagcaaggacagcacctacagcctcagcagcaccctgacgctgagcaaagcagactacgagaaacacaaagtctacgcctgcgaagtcacccatcagggcctgagctcgcccgtcacaaagagcttcaacaggggagagtgt (SEQ ID NO:208) |
| **Ep05 2014 Lv** | cagggacagagcggacagggatggtggccaccttgccagggaggagcttggtgtgagcagaggatcggaggaggctccagcggaggctctatctcttccggcctgctgagcggcagatctgacaacatcggcggctccgatatcgtgctgacccagacacactgtccctgagcgtgaccccaggacagccagcctctatctcctgcaggagctctcggtctctgctgcattccgacggcttcacctacctgtattggtttctgcagaagcctggccagtctccacagctgctgatctaccagacaagcaatctggcttctggcgtgcccgacagattctccagctctggctccggcaccgacttcaccctgaagatcagccgcgtggaggctgaggatgtgggcgtgtactattgtgctcagaacctggagctgcctaatacctttggccagggcacaaagctggagatcaagcgtacggtggctgcaccatctgtcttcatcttcccgccatctgatgagcagttgaaatctggaactgcctctgttgtgtgcctgctgaataacttctatcccagagaggccaaagtacagtggaaggtggataacgccctccaatcgggtaactcccaggagagtgtcacagagcaggacagcaaggacagcacctacagcctcagcagcaccctgacgctgagcaaagcagactacgagaaacacaaagtctacgcctgcgaagtcacccatcagggcctgagctcgcccgtcacaaagagcttcaacaggggagagtgt (SEQ ID NO:209) |

In some embodiments, an EpBA of the disclosure comprises a heavy chain nucleic acid sequence of SEQ ID NO:201 and a light chain nucleic acid sequence of SEQ ID NO:202. In some embodiments, an EpBA of the disclosure comprises a heavy chain nucleic acid sequence of SEQ ID NO:203 and a light chain nucleic acid sequence of SEQ ID NO:204. In some embodiments, an EpBA of the disclosure comprises a heavy chain nucleic acid sequence of SEQ ID NO:205 and a light chain nucleic acid sequence of SEQ ID NO:204. In some embodiments, an EpBA of the disclosure comprises a heavy chain nucleic acid sequence of SEQ ID NO:206 and a light chain nucleic acid sequence of SEQ ID NO:204. In some embodiments, an EpBA of the disclosure comprises a heavy chain nucleic acid sequence of SEQ ID NO:207 and a light chain nucleic acid sequence of SEQ ID NO:204.

In some embodiments, an EpBA of the disclosure comprises a heavy chain nucleic acid sequence of SEQ ID NO:203 and a light chain nucleic acid sequence of SEQ ID NO:208. In some embodiments, an EpBA of the disclosure comprises a heavy chain nucleic acid sequence of SEQ ID NO:203 and a light chain nucleic acid sequence of SEQ ID NO:209.

In some embodiments, an EpBA of the disclosure comprises (i) a heavy chain variable region comprising the same amino acid sequence as the amino acid sequence of the heavy chain variable region encoded by the plasmid deposited with the American Type Culture Collection (ATCC®) as PTA-125343 and (ii) a light chain variable region comprising the same amino acid sequence as the amino acid sequence of the light chain variable region encoded by the plasmid deposited with the ATCC® as PTA-125342. Methods of making and using EpCAM antibodies and EpCAM-binding antibody fragment comprising the EpBA are also encompassed by the disclosure.

In some embodiments, the disclosure provides an EpCAM antibody comprising (i) a heavy chain comprising the same amino acid sequence as the amino acid sequence of the heavy chain encoded by the plasmid deposited with the ATCC® as PTA-125343 and (ii) a light chain comprising the same amino acid sequence as the amino acid sequence of the light chain variable region encoded by the plasmid deposited with the ATCC® as PTA-125342. Methods of making and using the EpCAM antibody are also encompassed by the disclosure.

In some embodiments, the disclosure provides an EpCAM activatable antibody or EpCAM-binding activatable antibody fragment comprising (i) a heavy chain variable region comprising the same amino acid sequence as the amino acid sequence of the heavy chain variable region encoded by the plasmid deposited with the ATCC® as PTA-125343 and (ii) a light chain variable region comprising the same amino acid sequence as the amino acid sequence of the light chain variable region encoded by the plasmid deposited with the ATCC® as PTA-125344. Methods of making and using the EpCAM activatable antibody or EpCAM-binding activatable antibody fragment are also encompassed by the disclosure.

In other embodiments, the disclosure provides an EpCAM activatable antibody or EpCAM-binding activatable antibody fragment comprising (i) a heavy chain variable region comprising the same amino acid sequence as the amino acid sequence of the heavy chain variable region encoded by the plasmid deposited with the ATCC® as PTA-125343 and (ii) a light chain variable region comprising the same amino acid sequence as the amino acid sequence of the light chain variable region encoded by the plasmid deposited with the ATCC® as PTA-125345. Methods of making and using the EpCAM activatable antibody or EpCAM-binding activatable antibody fragment are also encompassed by the disclosure.

Methods for the construction of recombinant vectors containing antibody coding sequences and appropriate transcriptional and translational control signals are well known in the art. Once an antibody has been recombinantly expressed, it may be purified by any method known in the art for purification of an immunoglobulin molecule, for example, by chromatography (*e.g.*, ion exchange, affinity, particularly by affinity for the specific antigen after Protein A, and sizing column chromatography), centrifugation, differential solubility, or by any other standard technique for the purification of proteins. In this regard, US Pat. No. 7,538,195 has been referred to in the present disclosure, the contents of which is hereby incorporated by reference in its entirety.

The disclosure also provides methods of producing an EpCAM antibody, EpCAM-binding antibody fragment, or EpCAM activatable antibody disclosed herein by culturing a cell under conditions that lead to expression of the antibody and/or EpCAM activatable antibody, wherein the cell comprises nucleic acid molecules encoding the antibody, antibody fragment or activatable antibody.

The disclosure also provides a method of manufacturing EpCAM activatable antibodies that in an activated state binds EpCAM by (a) culturing a cell comprising a nucleic acid construct that encodes the EpCAM activatable antibody, under conditions that lead to expression of the EpCAM activatable antibody, wherein the EpCAM activatable antibody, comprises a masking moiety (MM), a cleavable moiety (CM), and an Ab (*e.g.,* and EpCAM antibody or EpCAM-binding antibody fragment), (i) wherein the CM is a polypeptide that functions as a substrate for a protease; and (ii) wherein the CM is positioned in the EpCAM activatable antibody, such that, when the EpCAM activatable antibody, is in an uncleaved state, the MM interferes with specific binding of the Ab to EpCAM and in a cleaved state the MM does not interfere or compete with specific binding of the Ab to EpCAM; and (b) recovering the EpCAM activatable antibody. Suitable Ab, MM, and/or CM include any of the Ab, MM, and/or CM disclosed herein.

**Immunoconjugates**

In one embodiment, the disclosure provides immunoconjugates comprising an EpCAM antibody or EpCAM-binding antibody fragment, or an EpCAM activatable antibody, (*e.g.*, as disclosed herein) conjugated or covalently linked to a cytotoxic agent. Cytotoxic agents include any agent that is detrimental to cells such as, for example, Pseudomonas exotoxin, Diptheria toxin, a botulinum toxin A through F, ricin abrin, saporin, and cytotoxic fragments of such agents. Cytotoxic agents also include any agent having a therapeutic effect to prophylactically or therapeutically treat a disorder. Such therapeutic agents may be may be chemical therapeutic agents, protein or polypeptide therapeutic agents, and include therapeutic agents that possess a desired biological activity and/or modify a given biological response. Examples of therapeutic agents include without limitation, alkylating agents, angiogenesis inhibitors, anti-mitotic agents, hormone therapy agents, and antibodies useful for the treatment of cell proliferative disorders. In certain embodiments, the therapeutic agent is a maytansinoid compound, such as those described in US Pat. Nos. 5,208,020 and 7,276,497, the contents of each of which is herein incorporated by reference in its entirety. In certain embodiments, the therapeutic agents are benzodiazepine compounds, such as pyrrolobenzodiazepine (PBD) (such as those described in WO 2010/043880, WO 2011/130616, WO 2009/016516, WO 2013/177481 and WO 2012/112708) and indolinobenzodiazepine (IGN) compounds (such as those described in WO 2010/091150, and WO 2012/128868 and US 20170014522, the contents of each of which is herein incorporated by reference in its entirety.

A "pyrrolobenzodiazepine" (PBD) compound, as used herein is a compound having a pyrrolobenzodiazepine core structure. The pyrrolobenzodiazepine can be substituted or unsubstituted. A "pyrrolobenzodiazepine" compound can also include a compound having a two pyrrolobenzodiazepine core linked by a linker. The imine functionality (-C=N-) as part of indolinobenzodiazepine core can be reduced.

In certain embodiments, the pyrrolobenzodiazepine compound comprises a core structure represented by

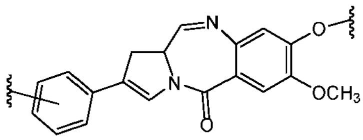

,

which optionally can be substituted.

In certain embodiments, the pyrrolobenzodiazepine compounds comprises a core structure represented by 4e3

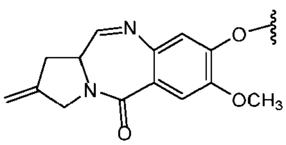

,

which optionally can be substituted.

A "indolinobenzodiazepine" (IGN) compound, as used herein, is a compound having an indolinobenzodiazepine core structure. The indolinobenzodiazepine can be substituted or unsubstituted. A "indolinobenzodiazepine" (IGN) compound can also include a compound having a two indolinobenzodiazepine core linked by a linker. The imine functionality (-C=N-) as part of indolinobenzodiazepine core can be reduced.

In certain embodiments, the indolinobenzodiazepine compound comprises a core structure represented by

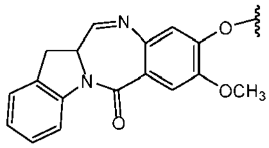

,

which can be optionally substituted.

In some embodiments, the indolinobenzodiazepine compound comprises a core structure represented by

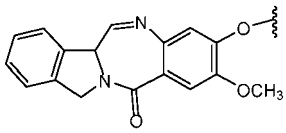
,

which can be further substituted.

The cytotoxic agent may be coupled or conjugated either directly to the EpCAM-binding agent (*e.g.,* an EpCAM antibody or EpCAM-binding antibody fragment or an EpCAM activatable antibody, disclosed herein) or indirectly, through a linker using techniques known in the art to produce an "immunoconjugate," "conjugate," "ADC," or "AADC."

**Linker Molecules**

Any suitable linkers known in the art can be used in preparing the disclosed immunoconjugates. In certain embodiments, the linkers are bifunctional linkers. As used herein, the term "bifunctional linker" refers to modifying agents that possess two reactive groups; one of which is capable of reacting with a cell binding agent while the other one reacts with the cytotoxic compound to link the two moieties together. Such bifunctional crosslinkers are well known in the art (*see,* for example, Isalm and Dent in Bioconjugation chapter 5, p218-363, Groves Dictionaries Inc. New York, 1999). For example, bifunctional crosslinking agents that enable linkage *via* a thioether bond include *N*-succinimidyl-4-(N-maleimidomethyl)-cyclohexane-1-carboxylate (SMCC) to introduce maleimido groups, or with N-succinimidyl-4-(iodoacetyl)-aminobenzoate (SICBA) to introduce iodoacetyl groups. Other bifunctional crosslinking agents that introduce maleimido groups or haloacetyl groups on to a cell binding agent are well known in the art (*see,* for example, US 2008/0050310 and US 20050169933, available from Pierce Biotechnology Inc. P.O. Box 117, Rockland, IL 61105, USA) and include, but not limited to, bis-maleimidopolyethyleneglycol (BMPEO), $BM(PEO)_2$, $BM(PEO)_3$, N-(β-maleimido-propyloxy)succinimide ester (BMPS), γ-maleimidobutyric acid N-succinimidyl ester (GMBS), 6-maleimidocaproic acid N-hydroxysuccinimide ester (EMCS), 5-maleimidovaleric acid NHS, HBVS, N-succinimidyl-4-(N-maleimidomethyl)-cyclo-hexane-1-carboxy-(6-amidocaproate), which is a "long chain" analog of SMCC (LC-SMCC), m-maleimidobenzoyl-N-hydroxysuccinimide ester (MBS), 4-(4-N-maleimidophenyl)-butyric acid hydrazide or HCl salt (MPBH), N-succinimidyl 3-(bromoacetamido)propionate (SBAP), N-succinimidyl iodoacetate (SIA), κ-maleimidoundecanoic acid N-succinimidyl ester (KMUA), N-succinimidyl 4-(p-maleimidophenyl)-butyrate (SMPB), succinimidyl-6-(β-maleimidopropionamido)hexanoate (SMPH), succinimidyl-(4-vinylsulfonyl)benzoate (SVSB), dithiobis-maleimidoethane (DTME), 1,4-bis-maleimidobutane (BMB), 1,4 bismaleimidyl-2,3-dihydroxybutane (BMDB), bis-maleimidohexane (BMH), bis-maleimidoethane (BMOE), sulfosuccinimidyl 4-(N-maleimidomethyl)cyclohexane-1-carboxylate (sulfo-SMCC), sulfosuccinimidyl(4-iodoacetyl)aminobenzoate (sulfo-SICBA), m-maleimidobenzoyl-N-hydroxysulfosuccinimide ester (sulfo-MBS), N-(γ-maleimidobutryl-oxy)sulfosuccinimide ester (sulfo-GMBS), N-(ε-maleimidocaproyloxy)sulfosuccimido ester (sulfo-EMCS), N-(κ-maleimidoundecanoyl-oxy)sulfosuccinimide ester (sulfo-KMUS), and sulfosuccinimidyl 4-(p-maleimidophenyl) butyrate (sulfo-SMPB).

Heterobifunctional crosslinking agents are bifunctional crosslinking agents having two different reactive groups. Heterobifunctional crosslinking agents containing both an amine-reactive N-hydroxysuccinimide group (NHS group) and a carbonyl-reactive hydrazine group can also be used to link the cytotoxic compounds disclosed herein with a cell-binding agent (*e.g.,* an EpCAM antibody, EpCAM-bindng antibody fragment, or EpCAM activatable antibody). Examples of such commercially available heterobifunctional crosslinking agents include succinimidyl 6-hydrazinonicotinamide acetone hydrazone (SANH), succinimidyl 4-hydrazidoterephthalate hydrochloride (SHTH) and succinimidyl hydrazinium nicotinate hydrochloride (SHNH). Conjugates bearing an acid-labile linkage can also be prepared using a hydrazine-bearing benzodiazepine derivative of the present disclosure. Examples of bifunctional crosslinking agents that can be used include succinimidyl-p-formyl benzoate (SFB) and succinimidyl-p-formylphenoxyacetate (SFPA).

Bifunctional crosslinking agents that enable the linkage of cell binding agent with cytotoxic compounds *via* disulfide bonds are known in the art and include *N*-succinimidyl-3-(2-pyridyldithio)propionate (SPDP), *N*-succinimidyl-4-(2-pyridyldithio)pentanoate (SPP), *N*-succinimidyl-4-(2-pyridyldithio)butanoate (SPDB), *N*-succinimidyl-4-(2-pyridyldithio)2-sulfo butanoate (sulfo-SPDB) to introduce dithiopyridyl groups. Other bifunctional crosslinking agents that can be used to introduce disulfide groups are known in the art and are disclosed in US Patents 6,913,748, 6,716,821, and US 20090274713 and 20100129314, the contents of each of which is herein incorporated by reference in its entirety. Alternatively, crosslinking agents such as 2-iminothiolane, homocysteine thiolactone or S-acetylsuccinic anhydride that introduce thiol groups can also be used.

In certain embodiments, the bifunctional linkers are represented by any one of the formula (a1L) - (a10L) described below.

**Cytotoxic Agents**

**A. Maytansinoid**

In certain embodiments, the cytotoxic agent is a maytansinoid compound, such as those described in US Pat. Nos. 5,208,020 and 7,276,497, the contents of each of which is herein incorporated by reference in its entirety. In certain embodiments, the maytansinoid compound is represented by the following formula:

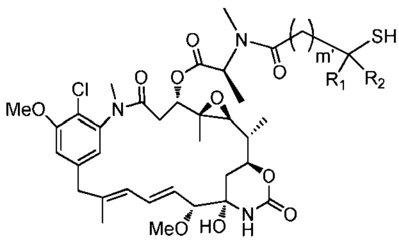

wherein the variables are as described above in any one of the 13th to 15th specific embodiments, of the first embodiment above and any more specific embodiments, described therein.

In a more specific embodiment, the maytansinoid compound is DM4:

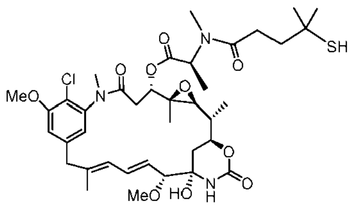

In another embodiment, the maytansinoid compound is DM1:

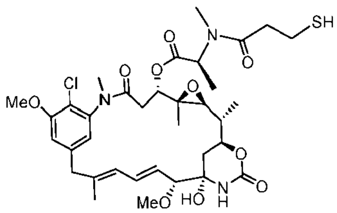

**B. Benzodiazepine**

In certain embodiments, the cytotoxic agent is a benzodiazepine compound, such as pyrrolobenzodiazepine (PBD) (such as those described in WO 2010/043880, WO 2011/130616, WO 2009/016516, WO 2013/177481 and WO 2012/112708) and indolinobenzodiazepine (IGN) compounds (such as those described in WO 2010/091150, and WO 2012/128868 and US20170014522. The entire teachings of each of these patents, patent publications and applications is herein incorporated by reference in its entirety.

As used herein, a "benzodiazepine" compound is a compound having a benzodiazepine core structure. The benzodiazepine core can be substituted or unsubstituted, and/or fused with one or more ring structures. It also includes a compound having two benzodiazepine core linked by a linker. The imine functionality (-C=N-) as part of benzodiazepine core can be reduced.

As used herein, a "pyrrolobenzodiazepine" (PBD) compound is a compound having a pyrrolobenzodiazepine core structure. The pyrrolobenzodiazepine can be substituted or unsubstituted. It also includes a compound having two pyrrolobenzodiazepine core linked by a linker. The imine functionality (-C=N-) as part of indolinobenzodiazepine core can be reduced.

In certain embodiments, the cytotoxic agent is an indolinobenzodiazepine compound represented by the following formula:

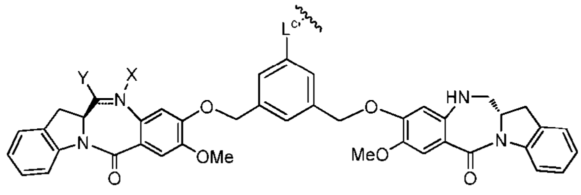
(L1a'),

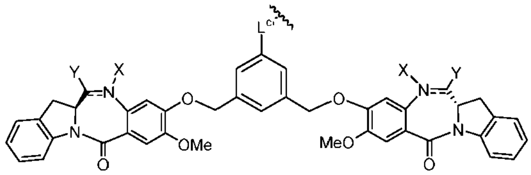
(L1a'1),

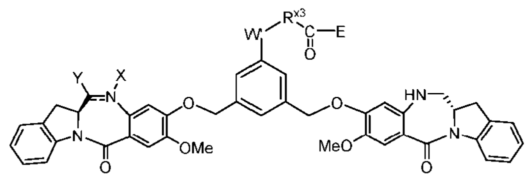
(L1b'),

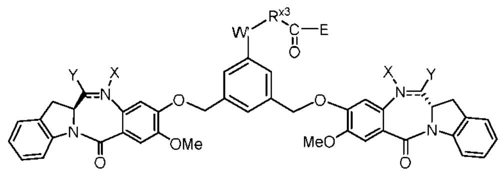
(L1b'1),

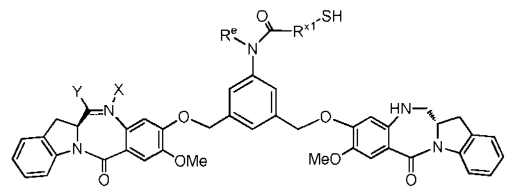
(L2a');

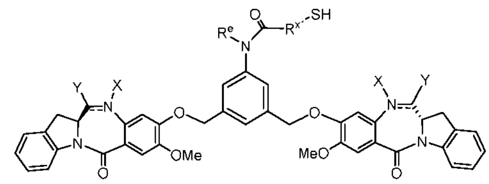
(L2a'1);

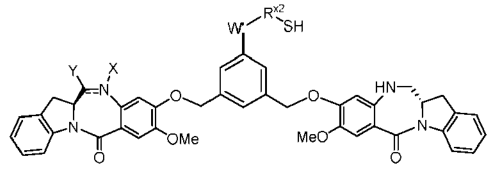
(L2b'); or

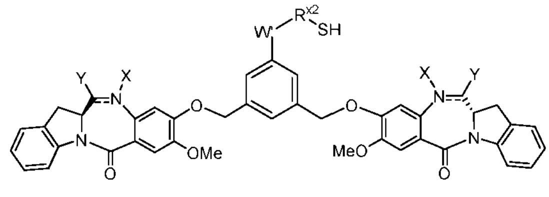
(L2b'1),

or a pharmaceutically acceptable salt thereof, wherein:

$L^{c,}$ is represented by the following formula:

$$-NR_5\text{-}P\text{-}C(=O)\text{-}(CR_aR_b)_m\text{-}C(=O)E \qquad (B1);$$

or

$$-NR_5\text{-}P\text{-}C(=O)\text{-}(CR_aR_b)_m\text{-}S\text{-}Z^s \qquad (B2)$$

C(=O)E is a reactive ester group, such as N-hydroxysuccinimide ester, N-hydroxy sulfosuccinimide ester, nitrophenyl (*e.g.,* 2 or 4-nitrophenyl) ester, dinitrophenyl (*e.g.,* 2,4-dinitrophenyl) ester, sulfo-tetraflurophenyl (*e.g.*, 4-sulfo-2,3,5,6-tetrafluorophenyl) ester, or pentafluorophenyl ester, preferably N-hydroxysuccinimide ester;
$Z^s$ is represented by the following formula:

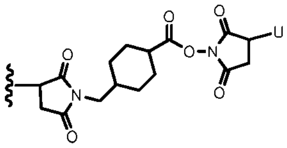 (a1); 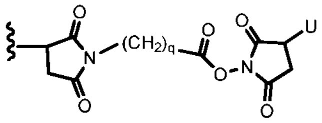 (a2); 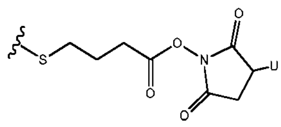 (a3);

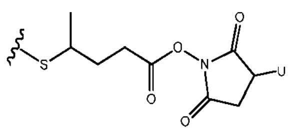 (a4); 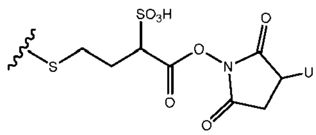 (a5),

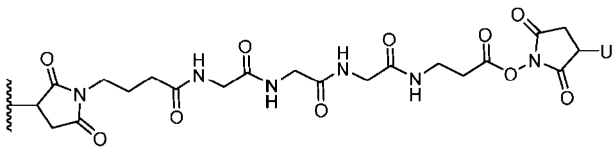 (a6),

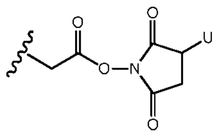 (a7); 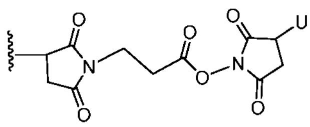 (a8);

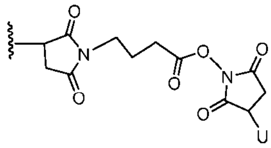 (a9); and 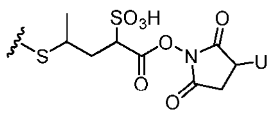 (a10),

wherein:

q is an integer from 1 to 5; and
U is -H or $SO_3H$ or a pharmaceutically acceptable salt thereof; and
the remaining variables are as described in any one of the 1st to 12th and 17th specific embodiments, of the first embodiment described above or any more specific embodiments, described therein.

In certain embodiments, the cytotoxic agent is an indolinobenzodiazepine compound represented by the following formula:

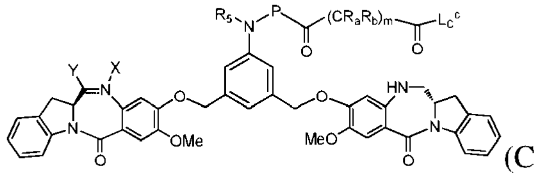
(C1a');

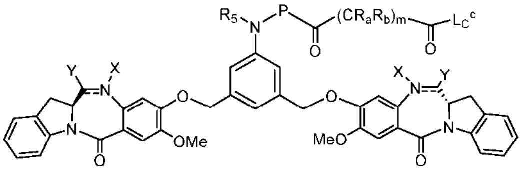
(C1a'1)

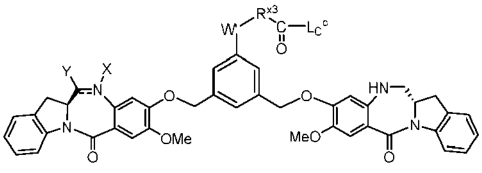
(C1b'),

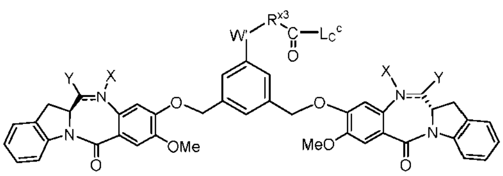
(C1b'1),

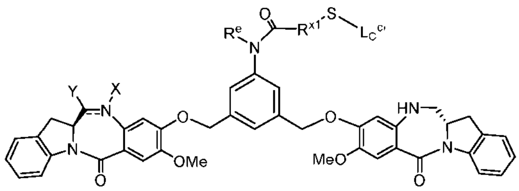
(C2a''),

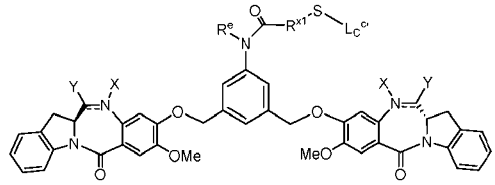
(C2a''1),

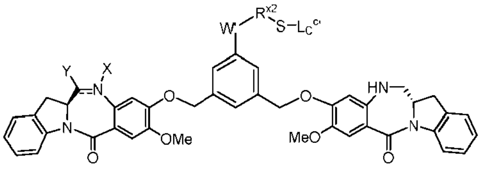
(C2b''), or

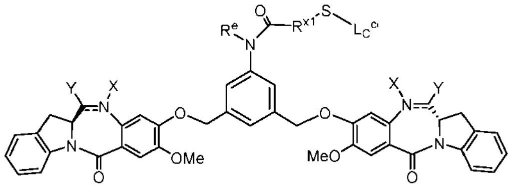
(C2b''1)

or a pharmaceutically acceptable salt thereof, wherein:

-$L_C^c$ for formulas (C1a'), (C1a'1), (C1b') and (C1b'1) is represented by the following formula:

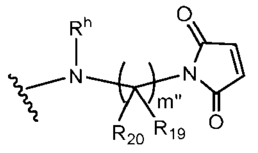

wherein the variables are as described above in any one of the 1st to 9th and 23rd specific embodiments, of the second embodiment or any more specific embodiments, described therein; and

$Lc^{c,}$ for formulas (C2a"), (C2a"1), (C2b") and (C2b"1) is represented by the following formula:

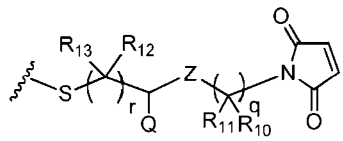

;

or

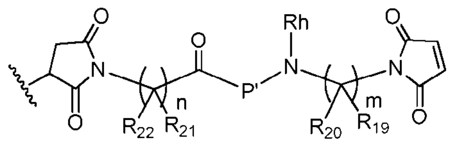

;

wherein the variables are as described above in any one of the 10th to 16th and 23rd specific embodiment of the second embodiment or any more specific embodiments, described therein.

In certain embodiments, the cytotoxic agent is an indolinobenzodiazepine compound of any one of the following or a pharmaceutically acceptable salt thereof:

| Compound No. | Structure |
|---|---|
| **D1** | 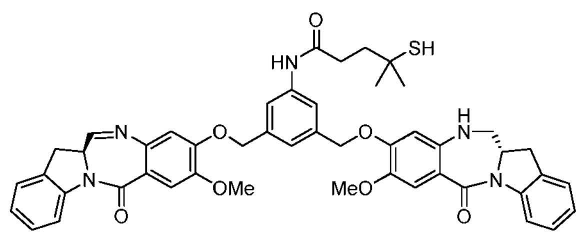 |
| **sD1** | 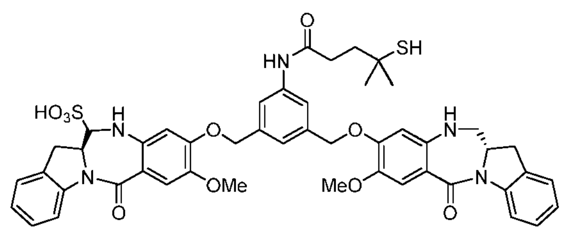 |

(continued)

| Compound No. | Structure |
|---|---|
| D2 | 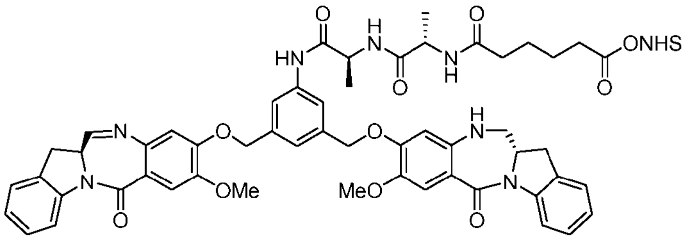 |
| sD2 | 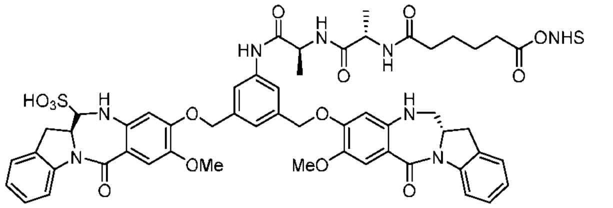 |
| DGN462 | 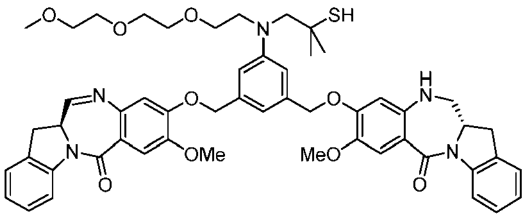 |
| sDGN462 | 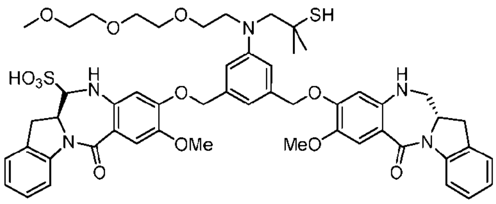 |
| D3 | 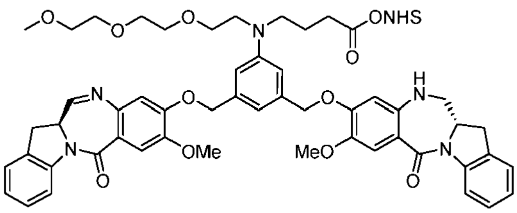 |
| sD3 | 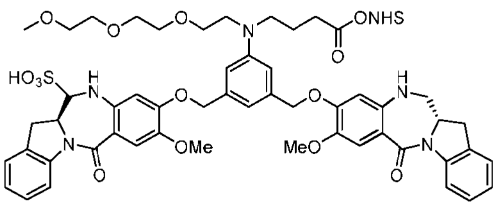 |
| D4 | 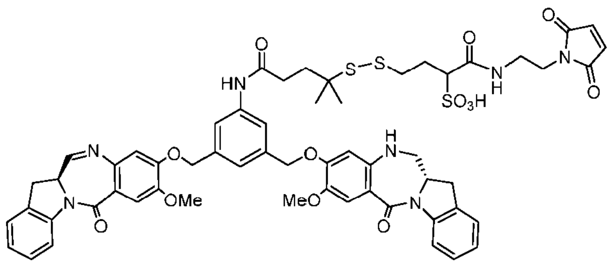 |

(continued)

| Compound No. | Structure |
|---|---|
| sD4 | 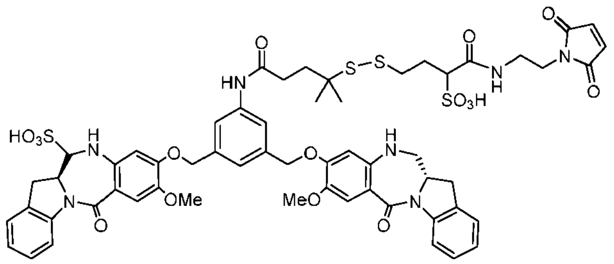 |
| D5 | 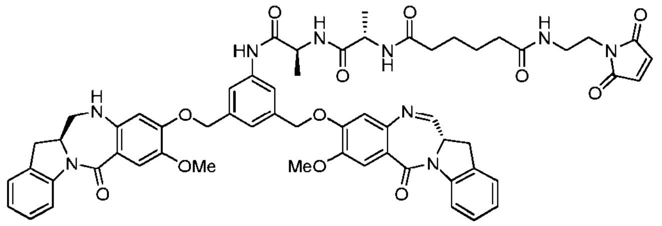 |
| sD5 | 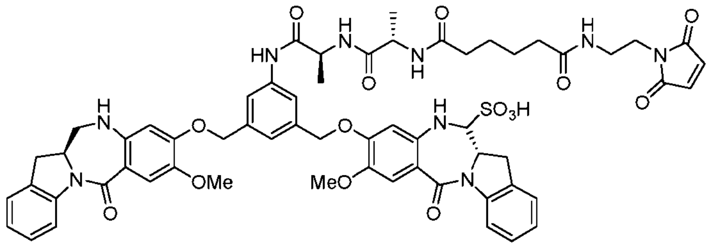 |
| D5' | 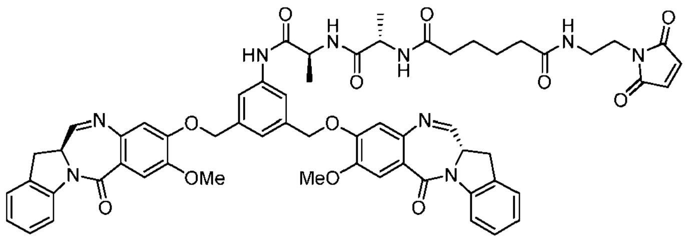 |
| sD5' | 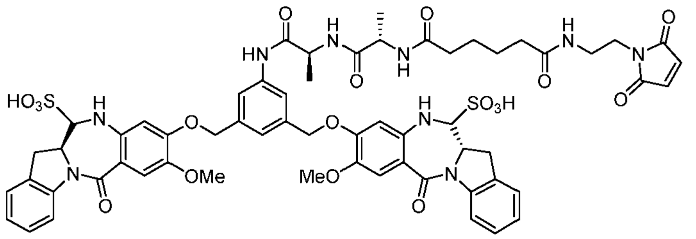 |
| D6 | 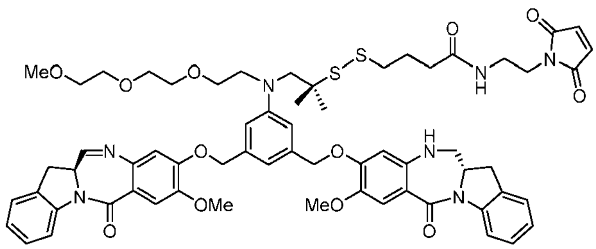 |

(continued)

| Compound No. | Structure |
|---|---|
| sD6 | 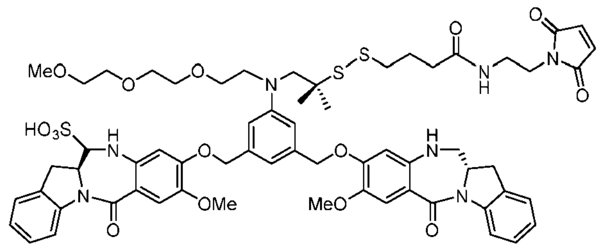 |
| D7 | 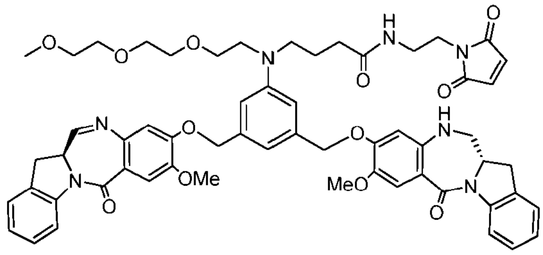 |
| sD7 | 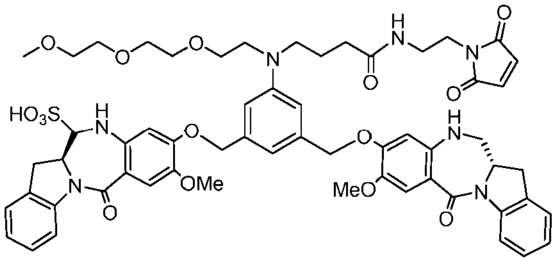 |

Compounds D1, sD1, D2, sD2, DGN462, sDGN462, D3 and sD3 shown above can be prepared according to procedures known in the art, for example, as described in US Pat. Nos. 9,381,256, 8,765,740, 8,426,402, and 9,353,127, and US 2016/0082114, the contents of each of which is herein incorporated by reference in its entirety.

In certain embodiments, the pharmaceutically acceptable salt of the compounds shown above (*e.g.,* sD1, sD2, sD4, sDGN462, sD3, sD4, sD5, sD5', sD6 or sD7) is a sodium or potassium salt. In particular embodiments, the pharmaceutically acceptable salt is a sodium salt.

In a specific embodiment, the cytotoxic agent is represented by the following formula:

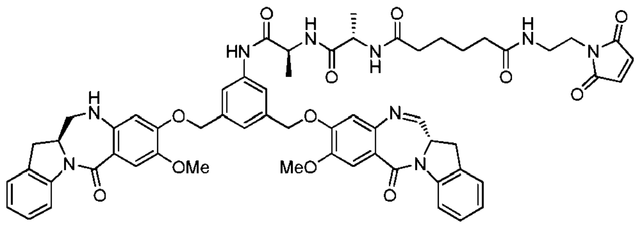

;

or

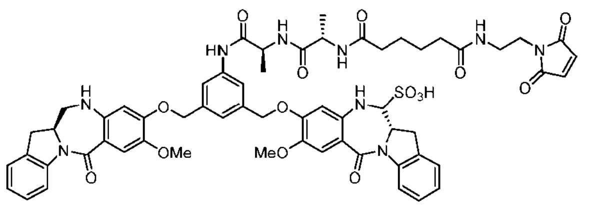

or a pharmaceutically acceptable salt thereof. In a specific embodiment, the pharmaceutically acceptable salt is a sodium

or a potassium salt.

In another specific embodiment, the cytotoxic agent is represented by the following formula:

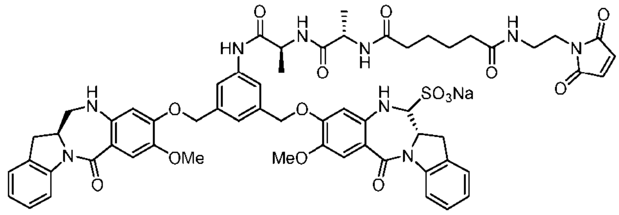

.

### Exemplary Immunoconjugates

In a first embodiment, the immunoconjugate comprises a EpCAM-binding agent (EpBA, *e.g.,* an EpCAM antibody or EpCAM-binding antibody fragment or an EpCAM activatable antibody disclosed herein) covalently linked to a cytotoxic agent disclosed herein through the ε-amino group of one or more lysine residues located on the EpBA.

In a 1st specific embodiment of the first embodiment, the immunoconjugate is represented by the following formula:

$$EpBA\left(Cy^{L1}\right)_{W_L} \quad (L1),$$

wherein:

EpBA (*e.g.,* an EpCAM antibody or EpCAM-binding antibody fragment or an EpCAM activatable antibody disclosed herein) that is covalently linked to $Cy^{L1}$ through a lysine residue;
$W_L$ is an integer from 1 to 20; and
$Cy^{L1}$ is a cytotoxic compound represented by the following formula:

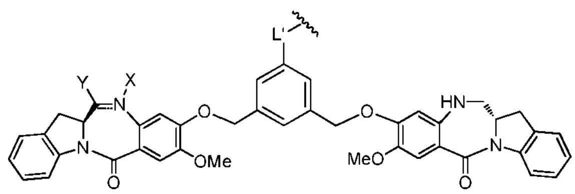
(L1a),

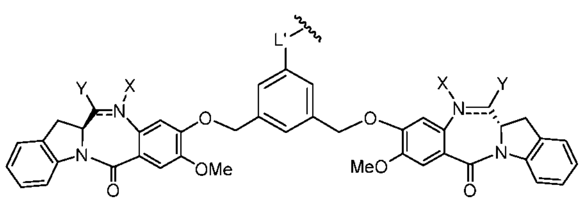
(L1a1),

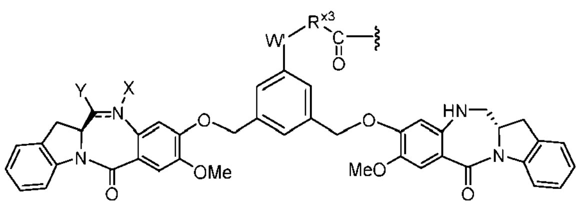
(L1b),

or

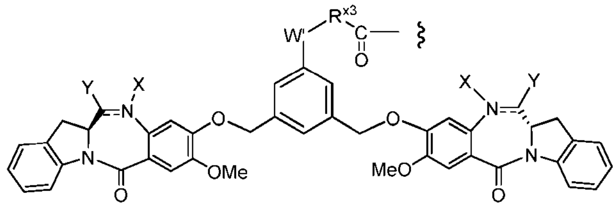 (L1b1);

or a pharmaceutically acceptable salt thereof, wherein:

the double line $\overline{--}$ between N and C represents a single bond or a double bond, provided that when it is a double bond, X is absent and Y is -H or a ($C_1$-$C_4$)alkyl; and when it is a single bond, X is -H or an amine protecting moiety, and Y is -OH or -$SO_3H$ or a pharmaceutically acceptable salt thereof;
W' is -$NR^{e'}$,
$R^{e'}$ is -($CH_2$-$CH_2$-O)$_n$-$R^k$;
n is an integer from 2 to 6;
$R^k$ is -H or -Me;
$R^{x3}$ is a ($C_1$-$C_6$)alkyl;
L' is represented by the following formula:

$$-NR_5-P-C(=O)-(CR_aR_b)_m-C(=O)- \quad (B1');$$

or

$$-NR_5-P-C(=O)-(CR_aR_b)_m-S-Z^{s1}- \quad (B2');$$

$R_5$ is -H or a ($C_1$-$C_3$)alkyl;
P is an amino acid residue or a peptide containing between 2 to 20 amino acid residues;
$R_a$ and $R_b$, for each occurrence, are each independently -H, ($C_1$-$C_3$)alkyl, or a charged substituent or an ionizable group Q;
m is an integer from 1 to 6; and
$Z^{s1}$ is selected from any one of the following formulas:

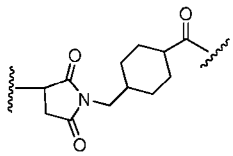 (b1); 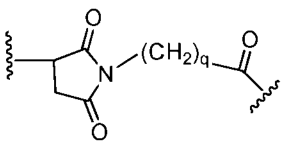 (b2); 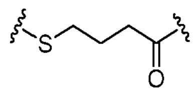 (b3);

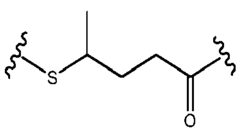 (b4); 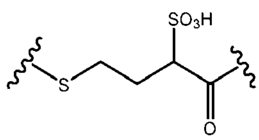 (b5),

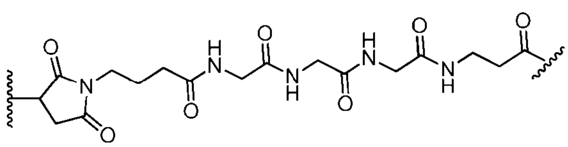 (b6), 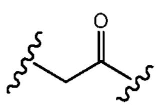 (b7);

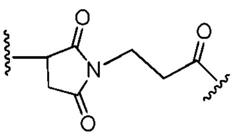 (b8); 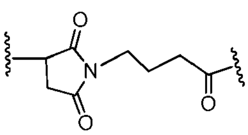 (b9); and 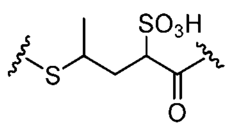 (b10),

wherein q is an integer from 1 to 5.

In a 2nd specific embodiment, for conjugates of formula (L1), $Cy^{L1}$ is represented by formula (L1a) or (L1a1); and the remaining variables are as described above in the 1st specific embodiment.

In a 3rd specific embodiment, for conjugates of formula (L1), $Cy^{L1}$ is represented by formula (L1b) or (L1b1); and the remaining variables are as described above in the 1st specific embodiment. More specifically, $R^{x3}$ is a $(C_2\text{-}C_4)$alkyl.

In a 4th specific embodiment, for conjugates of formula (L1), $Cy^{L1}$ is represented by formula (L1a); $R_a$ and $R_b$ are both H; $R_5$ is H or Me, and the remaining variables are as described above in the 1st specific embodiment.

In a 5th specific embodiment, P is a peptide containing 2 to 5 amino acid residues; and the remaining variables are described above in the 1st, 2nd or 4th specific embodiment. In a more specific embodiment, P is selected from: Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-$N^9$-tosyl-Arg, Phe-$N^9$-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:215), β-Ala-Leu-Ala-Leu (SEQ ID NO:216), Gly-Phe-Leu-Gly (SEQ ID NO:217), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, Met-Ala, Gln-Val, Asn-Ala, Gln-Phe and Gln-Ala. More specifically, P is Gly-Gly-Gly, Ala-Val, Ala-Ala, Ala-D-Ala, D-Ala-Ala, or D-Ala-D-Ala.

In a 6th specific embodiment, Q is -SO3H or a pharmaceutically acceptable salt thereof; and the remaining variables are as described above in the 1st, 2nd, 4th or 5th specific embodiment, or any other embodiment described therein.

In a 7th specific embodiment, the immunoconjugate of the first embodiment is represented by the following formula:

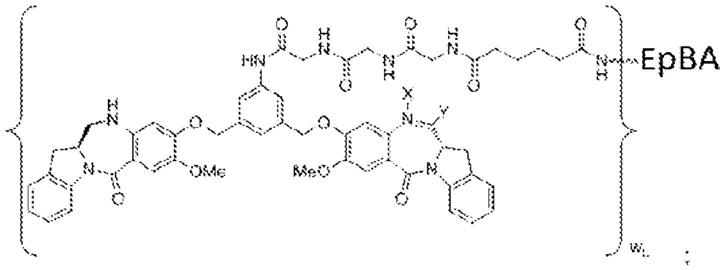
;

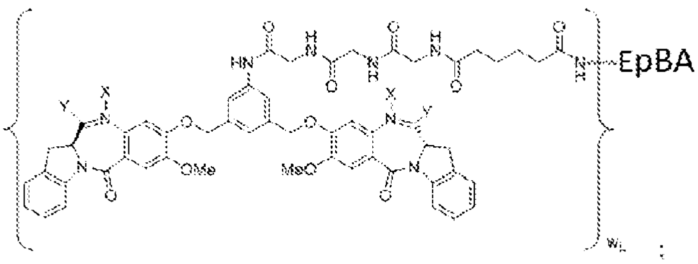
;

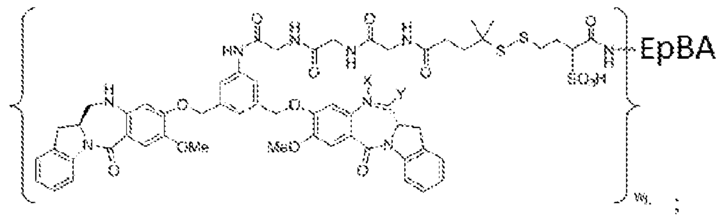
;

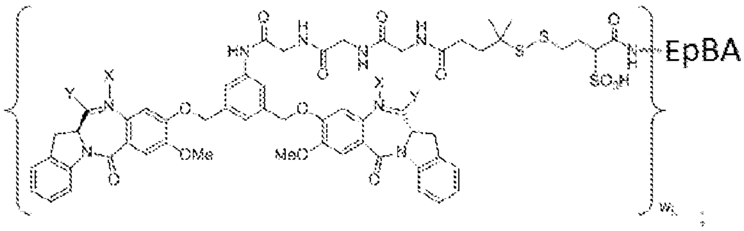
;

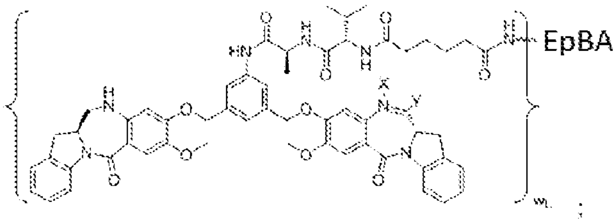

;

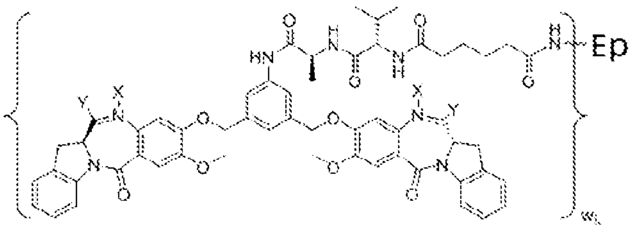

;

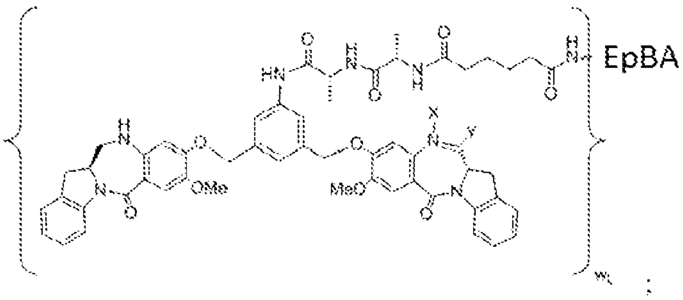

;

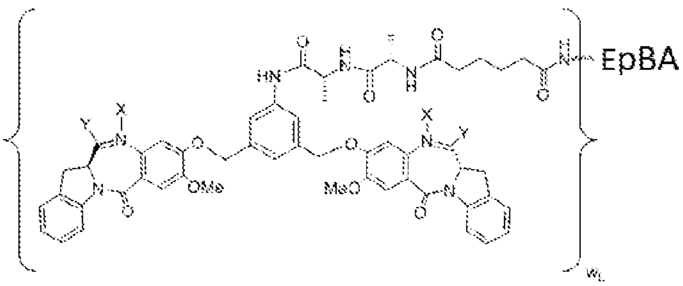

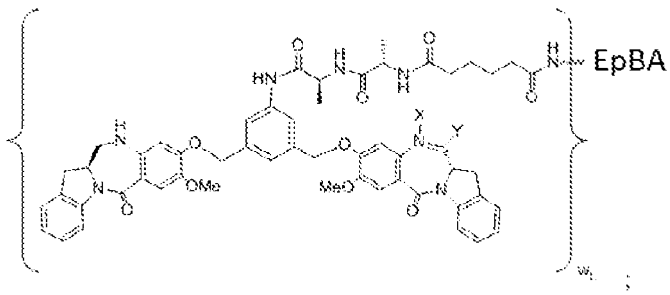

;

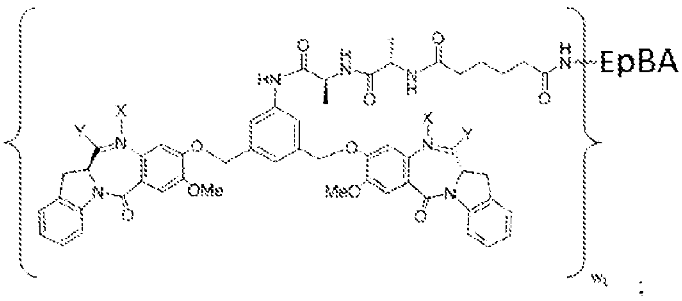

;

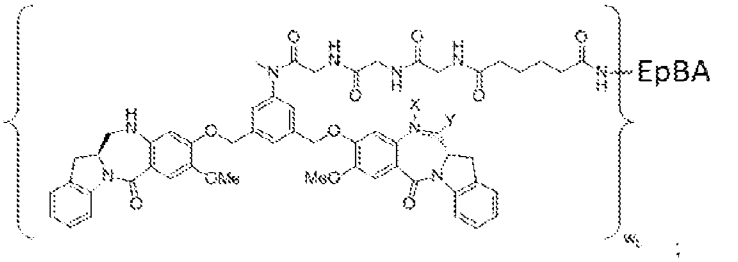

;

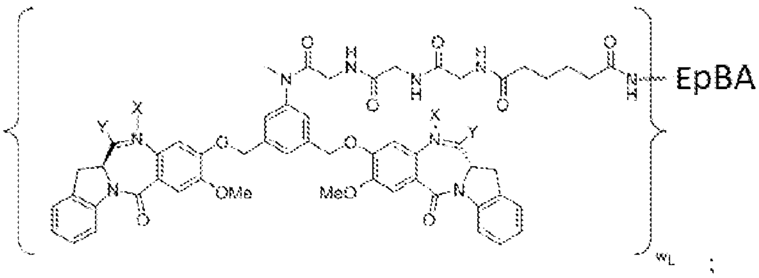

;

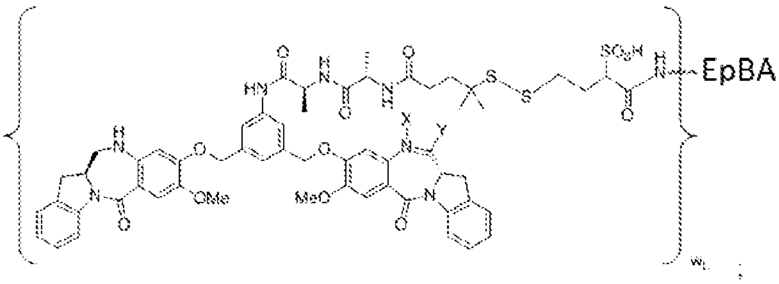

;

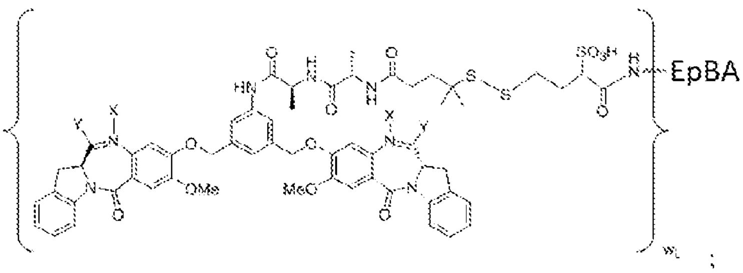

;

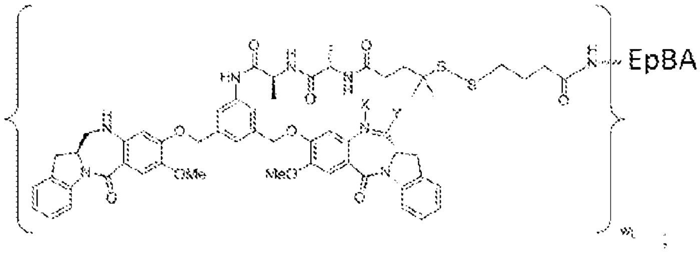

;

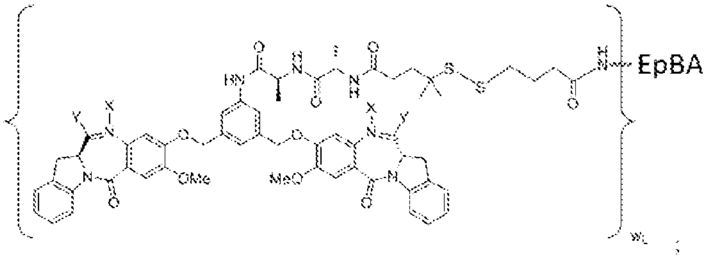

;

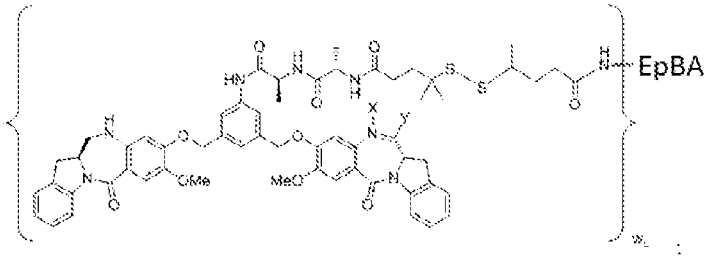

;

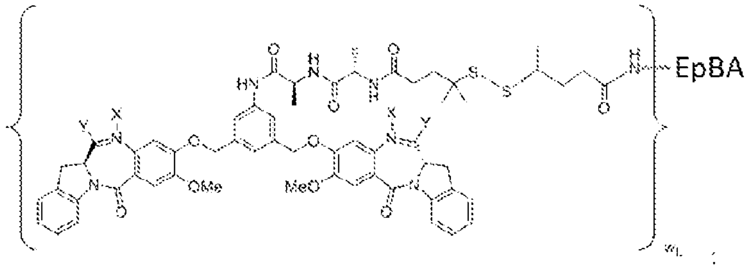

;

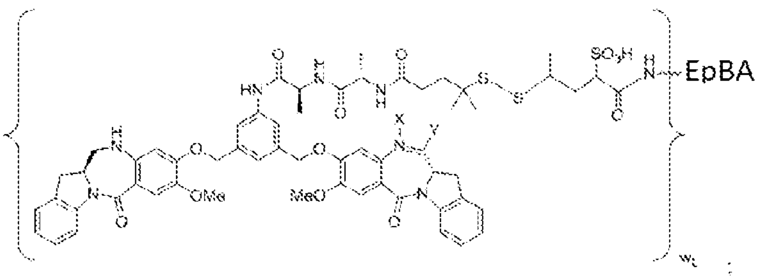

;

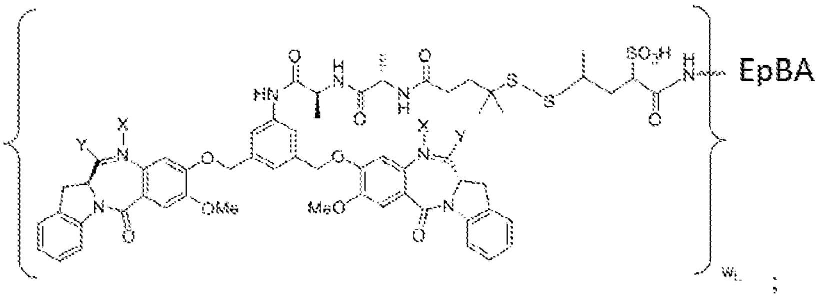

;

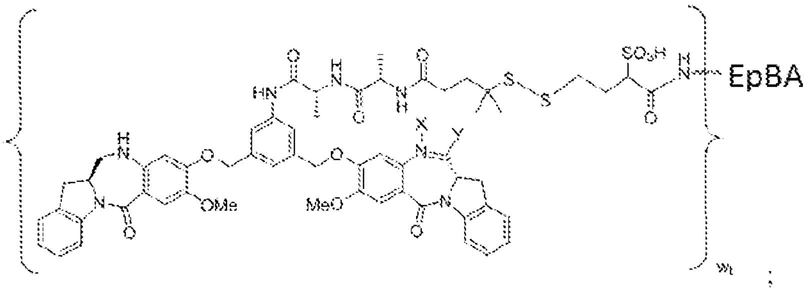

;

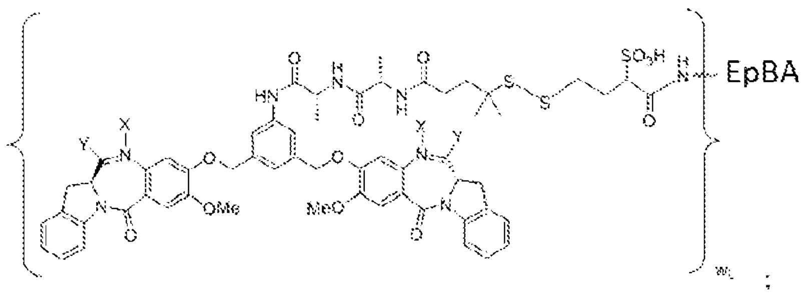

;

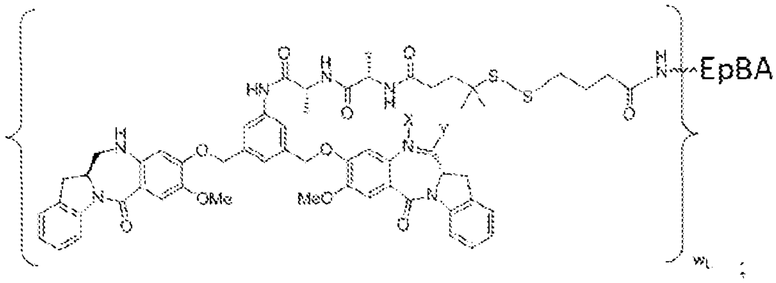

;

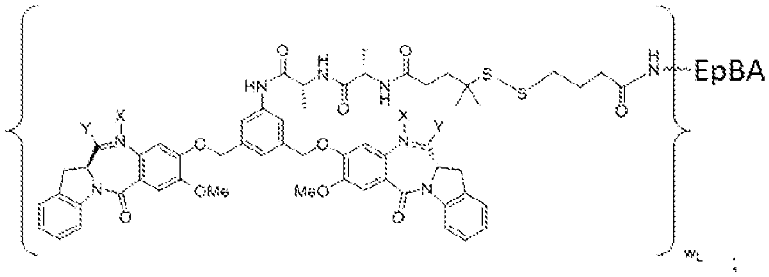

;

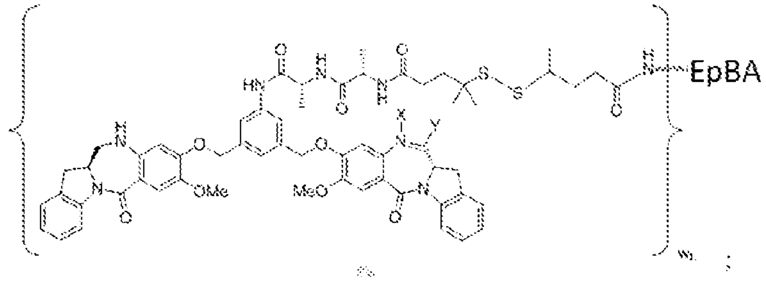

;

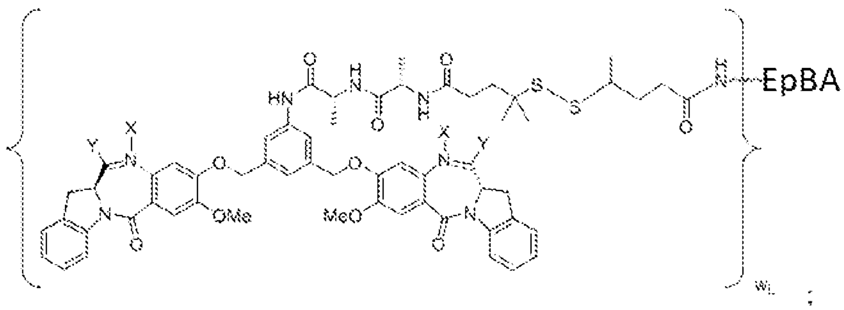

;

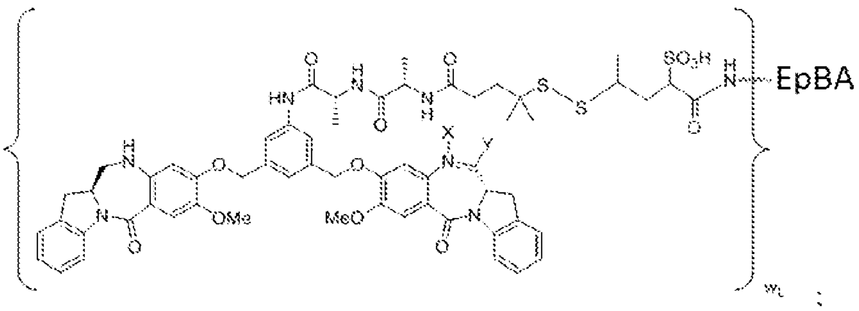

;

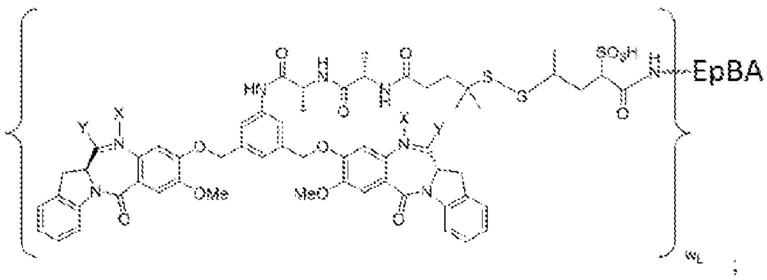

;

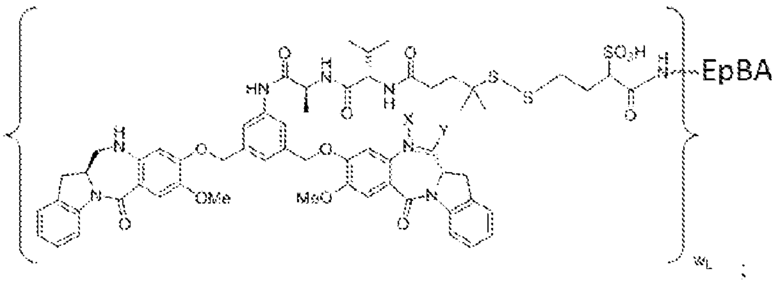

;

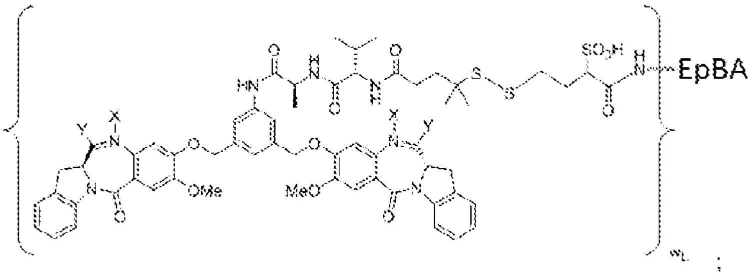

;

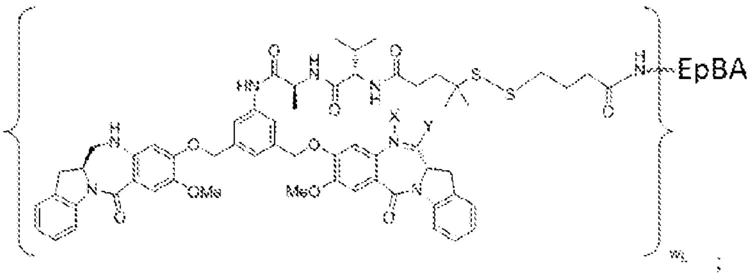

;

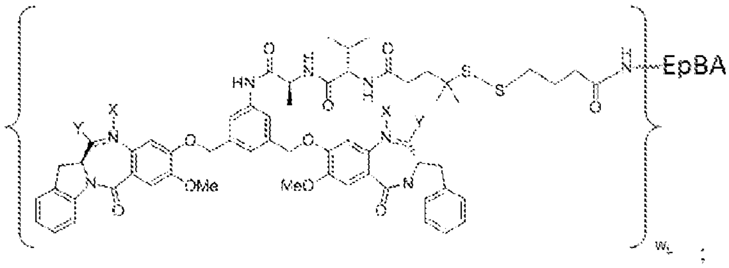

;

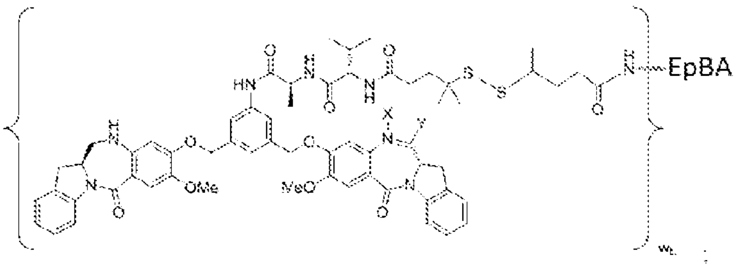

;

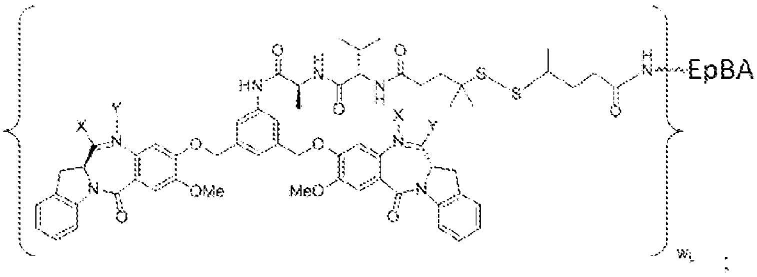

;

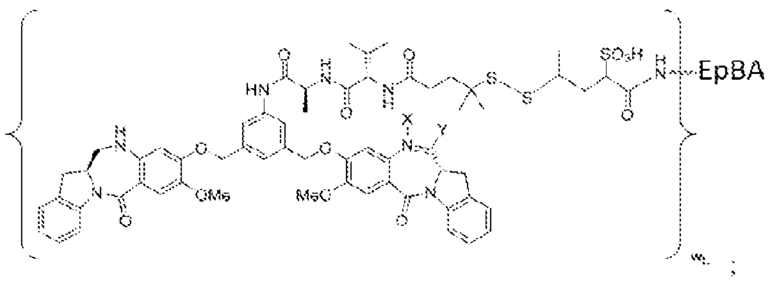

;

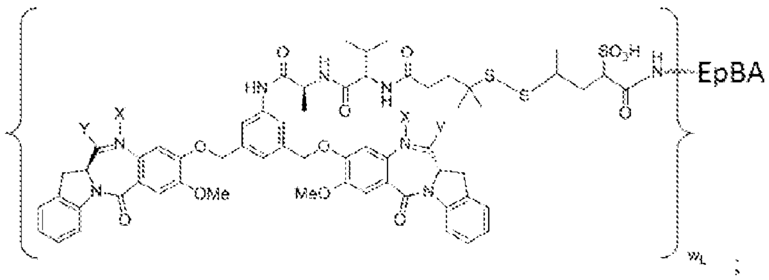

;

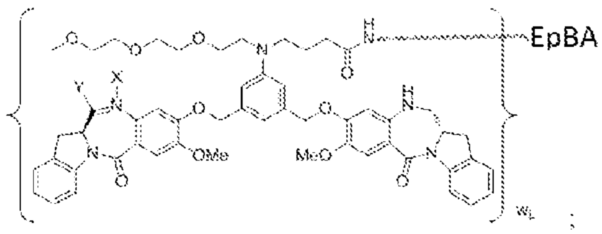

;

or

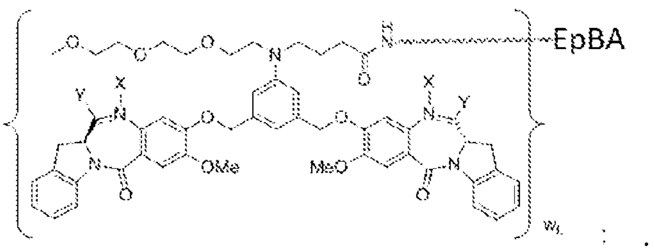

;

or a pharmaceutically acceptable salt thereof, wherein $W_L$ is an integer from 1 to 10; the double line $\overline{- -}$ between N and C represents a single bond or a double bond, provided that when it is a double bond, X is absent and Y is -H; and when it is a single bond, X is -H, and Y is -OH or $-SO_3H$ or a pharmaceutically acceptable salt thereof. In a more specific embodiment, the double line $\overline{- -}$ between N and C represents a double bond, X is absent and Y is -H. In another more specific embodiment, the double line $\overline{- -}$ between N and C represents a single bond, X is -H and Y is $-SO_3H$ or a pharmaceutically acceptable salt thereof.

In some embodiments, the EpBA of the $1^{st}$-$7^{th}$ specific embodiments comprises an EpCAM antibody, EpCAM-binding antibody fragment, or EpCAM activatable antibody disclosed herein. In some embodiments, the EpBA of the $1^{st}$-$7^{th}$ specific embodiments comprises a VH-CDR1 comprising $X_1YX_3X_4H$, wherein $X_1$ is selected from N and S, $X_3$ is selected from Y, N, F, S, H, D, L, I, and W, and $X_4$ is selected from I and M (SEQ ID NO:5); a VH-CDR2 comprising $WX_2X_3PGX_6VYIQYX_{12}X_{13}KFX_{17}G$, wherein $X_2$ is selected from I and F, $X_3$ is selected from Y and N, $X_6$ is selected from N and D, $X_{12}$ is selected from N and S, $X_{13}$ is selected from E and Q, and $X_{17}$ is selected from K and Q (SEQ ID NO:7); and a VH-CDR3 comprising $X_1GX_3X_4FAY$, wherein $X_1$ is selected from D and E, $X_3$ is selected from P, A, S, Y, F, G, T, and V, and $X_4$ is selected from Y and W (SEQ ID NO:8). In some embodiments, the EpBA of the $1^{st}$-$7^{th}$ specific embodiments comprises a VL-CDR1 comprising $RSSX_4SLLHSX_{10}GX_{12}TYLX_{16}$, wherein $X_4$ is selected from R and K, $X_{10}$ is selected from N and D, $X_{12}$ is selected from F and I, and $X_{16}$ is selected from Y and S (SEQ ID NO:10); a light chain VL-CDR2 comprising QTSNLAS (SEQ ID NO:40); and a VL-CDR3 comprising $X_1QX_3LELPX_8T$, wherein $X_1$ is selected from A, L, and Q, $X_3$ is selected from S, G, Y, and N, and $X_8$ is selected from N and W (SEQ ID NO: 11).

In some embodiments, the VH-CDR1 of the EpBA comprises the sequence NYX$_3$IH, wherein X$_3$ is selected from Y, N, F, S, H, D, L, I, and W (SEQ ID NO:6). In some embodiments, the VH-CDR3 of the EpBA comprises the sequence DGPX$_4$FAY, wherein X$_4$ is selected from Y and W (SEQ ID NO:9). In some embodiments, the VL-CDR3 of the EpBA comprises the sequence AQX$_3$LELPNT, wherein X$_3$ is selected from S, G, Y, and N (SEQ ID NO:12).

In some embodiments, the EpBA of the 1$^{st}$-7$^{th}$ specific embodiments comprises an EpCAM antibody, EpCAM-binding antibody fragment, or EpCAM activatable antibody comprising a VH-CDR1, VH-CDR2, VH-CDR3, VL-CDR1, VL-CDR2, and VL-CDR3 having the sequences of SEQ ID NOs: 13-15, 42, 40, and 41, respectively. In some embodiments, the EpBA of the 1$^{st}$-7$^{th}$ specific embodiments comprises an EpCAM antibody, EpCAM-binding antibody fragment, or EpCAM activatable antibody comprising a VH having the sequence of SEQ ID NO: 54 and a VL having the sequence of SEQ ID NO: 89. In some embodiments, EpBA of the 1$^{st}$-7$^{th}$ specific embodiments comprises an EpCAM antibody, EpCAM-binding antibody fragment, or EpCAM activatable antibody comprising a HC having the sequence of SEQ ID NO: 103 and a LC having the sequence of SEQ ID NO: 140.

In some embodiments, the EpBA of the 1$^{st}$-7$^{th}$ specific embodiments comprises an EpCAM activatable antibody comprising a MM of SEQ ID NO:155. In some embodiments, the EpBA of the 1$^{st}$-7$^{th}$ specific embodiments further comprises an EpCAM activatable antibody comprising a CM of SEQ ID NO:168. In alternative embodiments, the EpBA of the 1$^{st}$-7$^{th}$ specific embodiments comprises an EpCAM activatable antibody comprising a CM of SEQ ID NO:169. In one embodiment, the EpBA of the 1$^{st}$-7$^{th}$ specific embodiments comprises an EpCAM activatable antibody comprising a heavy chain having the sequence of SEQ ID NO: 103 and a light chain having the sequence of SEQ ID NO: 174. In one embodiment, the EpBA of the 1$^{st}$-7$^{th}$ specific embodiments comprises an EpCAM activatable antibody comprising a heavy chain having the sequence of SEQ ID NO:103 and a light chain having the sequence of SEQ ID NO:179.

In a 8$^{th}$ specific embodiment, the immunoconjugates of the first embodiment is represented by the following formula:

$$\text{EpBA} \left( -Cy^{L2} \right)_{W_L} \quad \text{(L2)},$$

wherein:

EpBA is an EpCAM-binding agent (*e.g.,* an EpCAM antibody, EpCAM-binding antibody fragment, or EpCAM activatable antibody) disclosed herein that is covalently linked to Cy$^{L2}$ through a Lys residue;
W$_L$ is an integer from 1 to 20; and

Cy$^{L2}$ is represented by the following formula:

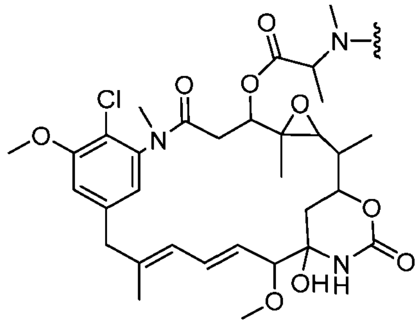

.

In a 9th specific embodiment, the immunoconjugates of the first embodiment is represented by the following formula:

$$\text{EpBA} \left( -Cy^{L2} \right)_{W_L} \quad \text{(L2)},$$

wherein:

EpBA is an EpCAM-binding agent (*e.g.,* an EpCAM antibody, EpCAM-binding antibody fragment, or EpCAM activatable antibody) disclosed herein that is covalently linked to Cy$^{L2}$ through a Lys residue;

$W_L$ is an integer from 1 to 20;
$Cy^{L2}$ is represented by the following formula:

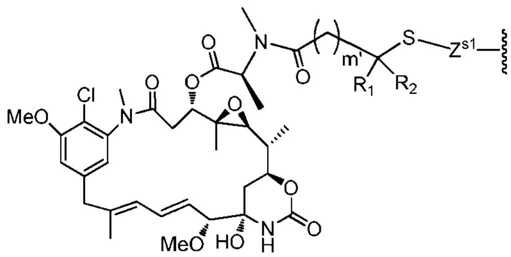
,

m' is 1 or 2;
$R_1$ and $R_2$, are each independently H or a $(C_1\text{-}C_3)$alkyl; and
$Z^{s1}$ is selected from any one of the following formulas:

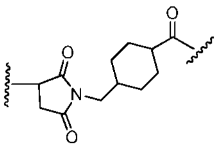 (b1); 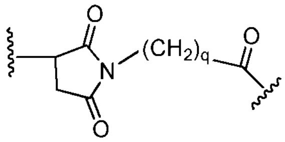 (b2); 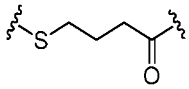 (b3);

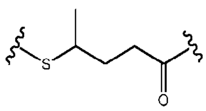 (b4); 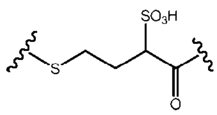 (b5),

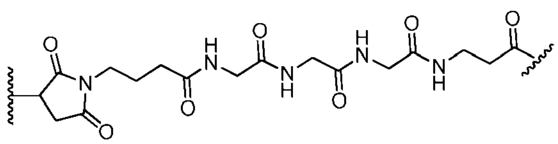 (b6), 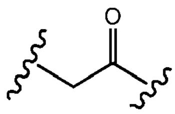 (b7);

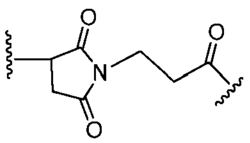 (b8); 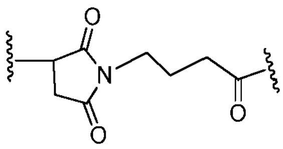 (b9), and 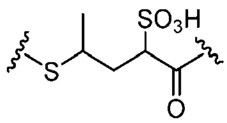 (b10),

wherein q is an integer from 1 to 5.

In a $10^{th}$ specific embodiment, for immunoconjugates of formula (L2), m' is 1, and $R_1$ and $R_2$ are both H; and the remaining variables are as described above in the 13th specific embodiment.

In a 11th specific embodiment, for immunoconjugates of formula (L2), m' is 2, and $R_1$ and $R_2$ are both Me; and the remaining variables are as described above in the 13th specific embodiment.

In a 12th specific embodiment, the immunoconjugates of the first embodiment is represented by the following formula.

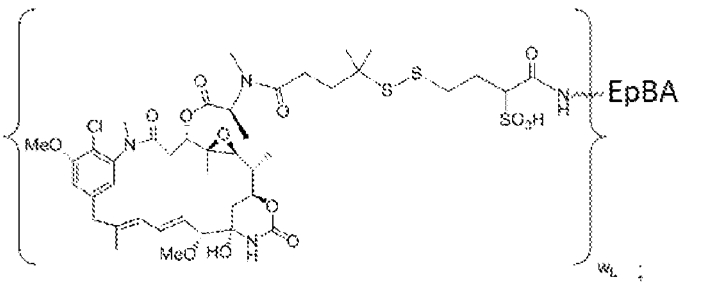
;

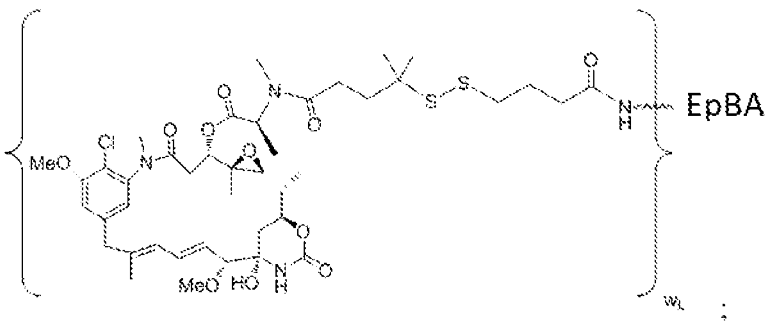
;

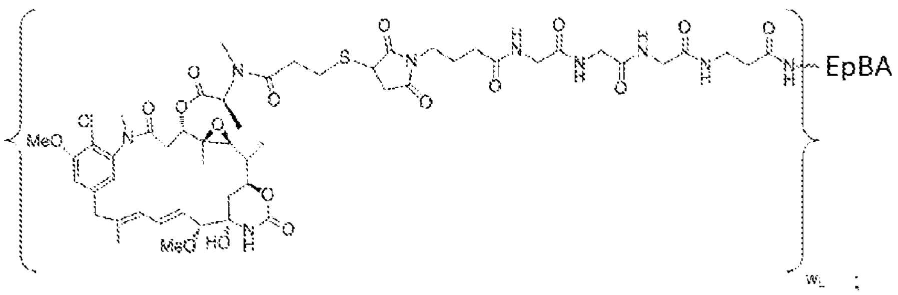
;

or

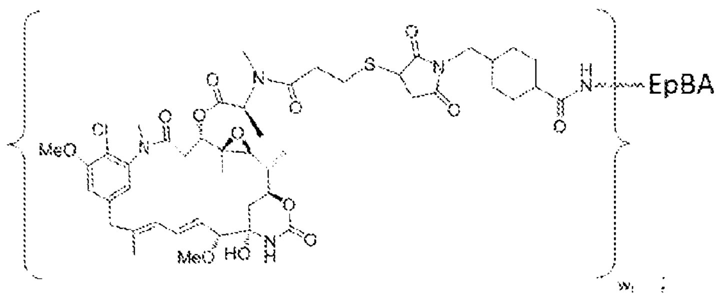
;

or a pharmaceutically acceptable salt thereof, wherein $W_L$ is an integer from 1 to 10.

In a 12th specific embodiment, for immunoconjugates of the first embodiment, Y is $-SO_3H$, $-SO_3Na$ or $-SO_3K$; and the remaining variables are as described above in any one of the 1st to 16th specific embodiment or any more specific embodiments, described therein. In one embodiment, Y is -SO3Na.

In some embodiments, the EpBA of the 8th-12th specific embodiments comprises an EpCAM antibody, EpCAM-binding antibody fragment, or EpCAM activatable antibody disclosed herein. In some embodiments, the EpBA of the 8th-12th specific embodiments comprises a VH-CDR1 comprising $X_1YX_3X_4H$, wherein $X_1$ is selected from N and S, $X_3$ is selected from Y, N, F, S, H, D, L, I, and W, and $X_4$ is selected from I and M (SEQ ID NO:5); a VH-CDR2 comprising $WX_2X_3PGX_6VYIQYX_{12}X_{13}KFX_{17}G$, wherein $X_2$ is selected from I and F, $X_3$ is selected from Y and N, $X_6$ is selected from N and D, $X_{12}$ is selected from N and S, $X_{13}$ is selected from E and Q, and $X_{17}$ is selected from K and Q (SEQ ID NO:7); and a VH-CDR3 comprising $X_1GX_3X_4FAY$, wherein $X_1$ is selected from D and E, $X_3$ is selected from P, A, S, Y, F, G, T, and V, and $X_4$ is selected from Y and W (SEQ ID NO:8). In some embodiments, the EpBA of the 8th-12th specific embodiments comprises a light chain CDR1 (VL-CDR1) comprising $RSSX_4SLLHSX_{10}GX_{12}TYLX_{16}$, wherein $X_4$ is selected from R and

K, $X_{10}$ is selected from N and D, $X_{12}$ is selected from F and I, and $X_{16}$ is selected from Y and S (SEQ ID NO:10); a light chain VL-CDR2 comprising QTSNLAS (SEQ ID NO:40); and a VL-CDR3 comprising $X_1QX_3LELPX_8T$, wherein $X_1$ is selected from A, L, and Q, $X_3$ is selected from S, G, Y, and N, and $X_8$ is selected from N and W (SEQ ID NO:11).

In some embodiments, the VH-CDR1 of the EpBA comprises the sequence $NYX_3IH$, wherein $X_3$ is selected from Y, N, F, S, H, D, L, I, and W (SEQ ID NO:6). In some embodiments, the VH-CDR3 of the EpBA comprises the sequence $DGPX_4FAY$, wherein $X_4$ is selected from Y and W (SEQ ID NO:9). In some embodiments, the VL-CDR3 of the EpBA comprises the sequence $AQX_3LELPNT$, wherein $X_3$ is selected from S, G, Y, and N (SEQ ID NO: 12).

In some embodiments, the EpBA of the 8th-12th specific embodiments comprises an EpCAM antibody, EpCAM-binding antibody fragment, or EpCAM activatable antibody comprising a VH-CDR1, VH-CDR2, VH-CDR3, VL-CDR1, VL-CDR2, and VL-CDR3 having the sequences of SEQ ID NOs: 13-15, 42, 40, and 41, respectively. In some embodiments, the EpBA of the 8th-12th specific embodiments comprises an EpCAM antibody, EpCAM-binding antibody fragment, or EpCAM activatable antibody comprising a VH having the sequence of SEQ ID NO: 54 and a VL having the sequence of SEQ ID NO: 89. In some embodiments, EpBA of the 8th-12th specific embodiments comprises an EpCAM antibody, EpCAM-binding antibody fragment, or EpCAM activatable antibody comprising a HC having the sequence of SEQ ID NO: 103 and a LC having the sequence of SEQ ID NO: 140.

In some embodiments, the EpBA of the 8th-12th specific embodiments comprises an EpCAM activatable antibody comprising a MM of SEQ ID NO:155. In some embodiments, the EpBA of the 8th-12th specific embodiments further comprises an EpCAM activatable antibody comprising a CM of SEQ ID NO:168. In alternative embodiments, the EpBA of the 8th-12th specific embodiments comprises an EpCAM activatable antibody comprising a CM of SEQ ID NO:169. In one embodiment, the EpBA of the 8th-12th specific embodiments comprises an EpCAM activatable antibody comprising a heavy chain having the sequence of SEQ ID NO: 103 and a light chain having the sequence of SEQ ID NO: 174. In one embodiment, the EpBA of the 8th-12th specific embodiments comprises an EpCAM activatable antibody comprising a heavy chain having the sequence of SEQ ID NO: 103 and a light chain having the sequence of SEQ ID NO: 179.

In certain embodiments, for compositions (*e.g.*, pharmaceutical compositions) comprising immunoconjugates of the first embodiment, or the 1st, 2nd, 3rd, 4th, 5th, 6th, 7th, 8th, 9th, 10th, 11th, or 12th specific embodiment, the average number of the cytotoxic agent per antibody molecule (*i.e.,* average value of wL), also known as Drug-Antibody Ratio (DAR) in the composition is in the range of 1.0 to 8.0. In some embodiments, DAR is in the range of 1.0 to 5.0, 1.0 to 4.0, 1.0 to 3.4, 1.0 to 3.0, 1.5 to 2.5, 2.0 to 2.5, or 1.8 to 2.2. In some embodiments, the DAR is less than 4.0, less than 3.8, less than 3.6, less than 3.5, less than 3.0 or less than 2.5.

In a second embodiment, the immunoconjugates comprise an EpBA covalently linked to a cytotoxic agent disclosed herein through the thiol group (-SH) of one or more cysteine residues located on the EpBA.

In a 1st specific embodiment, the immunoconjugate of the second embodiment is represented by the following formula:

$$EpBA\left(-Cy^{C1}\right)_{W_C} \quad (C1),$$

wherein:

EpBA is an EpCAM antibody, EpCAM-binding antibody fragment, or EpCAM activatable antibody, disclosed herein, covalently linked to $Cy^{C1}$ through a cysteine residue;

Wc is 1 or 2;

$Cy^{c1}$ is represented by the following formula:

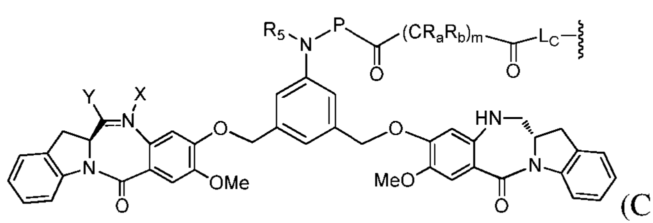

(C1a);

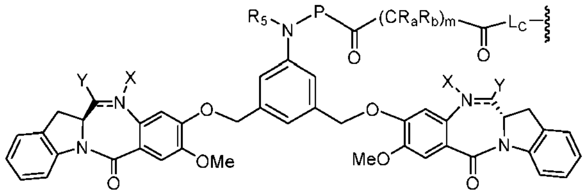
(C1a1);

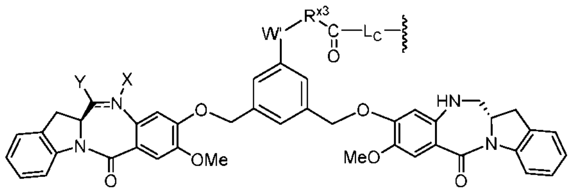
(C1b),

or

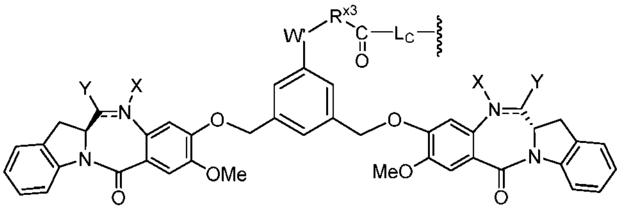
(C1b1)

or a pharmaceutically acceptable salt thereof, wherein:

the double line $\overline{--}$ between N and C represents a single bond or a double bond, provided that when it is a double bond, X is absent and Y is -H or a ($C_1$-$C_4$)alkyl; and when it is a single bond, X is -H or an amine protecting moiety, Y is -OH or -$SO_3H$ or a pharmaceutically acceptable salt thereof;
$R_5$ is -H or a ($C_1$-$C_3$)alkyl;
P is an amino acid residue or a peptide containing 2 to 20 amino acid residues;
$R_a$ and $R_b$, for each occurrence, are independently -H, ($C_1$-$C_3$)alkyl, or a charged substituent or an ionizable group Q;
m is an integer from 1 to 6;
W' is -$NR^{e'}$,
$R^{e'}$ is -($CH_2$-$CH_2$-O)$_n$-$R^k$;
n is an integer from 2 to 6;
$R^k$ is -H or -Me;
$R^{x3}$ is a ($C_1$-$C_6$)alkyl; and,
$L_C$ is represented by:

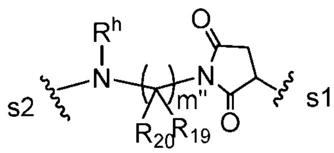
,

wherein s1 is the site covalently linked to EpBA, and s2 is the site covalently linked to the -C(=O)- group on $Cy^{c1}$; wherein:

$R_{19}$ and $R_{20}$, for each occurrence, are independently -H or a ($C_1$-$C_3$)alkyl;
m" is an integer between 1 and 10; and
$R^h$ is -H or a ($C_1$-$C_3$)alkyl.

In a 2nd specific embodiment, for immunoconjugate of formula (C1), CyC1 is represented by formula (C1a) or (C1a1); and the remaining variables are as described above in the 1st specific embodiment of the second embodiment.

In a 3rd specific embodiment, for immunoconjugate of formula (C1), CyC1 is represented by formula (C1b) or (C1b1); and the remaining variables are as described above in the 1st specific embodiment of the second embodiment.

In a 4th specific embodiment, for immunoconjugate of formula (C1), CyC1 is represented by formula (C1a) or (C1a1); Ra and Rb are both H; and R5 is H or Me; and the remaining variables are as described above in the 1st or 2nd specific embodiment of the second embodiment.

In a 5th specific embodiment, for immunoconjugate of formula (C1), P is a peptide containing 2 to 5 amino acid residues; and the remaining variables are as described above in the 1st, 2nd or 4th specific embodiment of the second embodiment. In a more specific embodiment, P is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-$N^9$-tosyl-Arg, Phe-$N^9$-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:215), β-Ala-Leu-Ala-Leu (SEQ ID NO:216), Gly-Phe-Leu-Gly (SEQ ID NO:217), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, Met-Ala, Gln-Val, Asn-Ala, Gln-Phe and Gln-Ala. In another more specific embodiment, P is Gly-Gly-Gly, Ala-Val, Ala-Ala, Ala-D-Ala, D-Ala-Ala, or D-Ala-D-Ala.

In a 6th specific embodiment, for immunoconjugates of formula (C1), Q is $-SO_3H$ or a pharmaceutically acceptable salt thereof; and the remaining variables are as describe above in the 1st, 2nd, 4th or 5th specific embodiment of the second embodiment or any more specific embodiments, described therein.

In a 7th specific embodiment, for immunoconjugates of formula (C1), R19 and R20 are both H; and m" is an integer from 1 to 6; and the remaining variables are as described above in the 1st, 2nd, 3rd, 4th, 5th or 6th specific embodiment of the second embodiment or any more specific embodiments, described therein.

In a 8th specific embodiment, for immunoconjugates of formula (C1), -L-LC- is represented by the following formula:

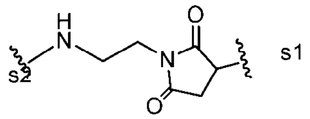

,

and the remaining variables are as described above in the 1st, 2nd, 3rd, 4th, 5th, 6th or 7th specific embodiment of the second embodiment or any more specific embodiments, described therein.

In a 9th specific embodiment, the immunoconjugate of the second embodiment is represented by the following formula:

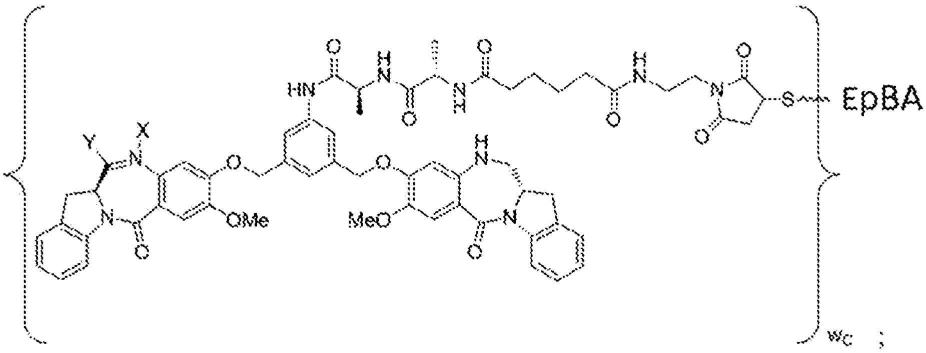

;

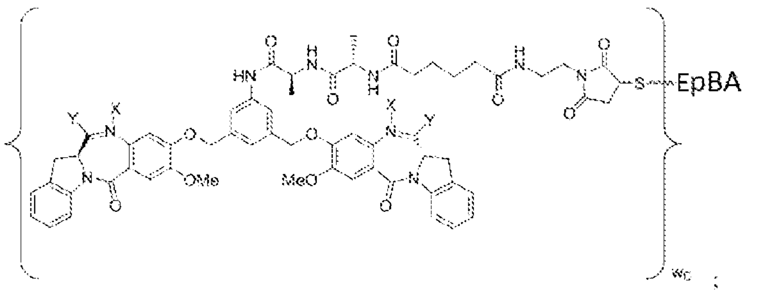

;

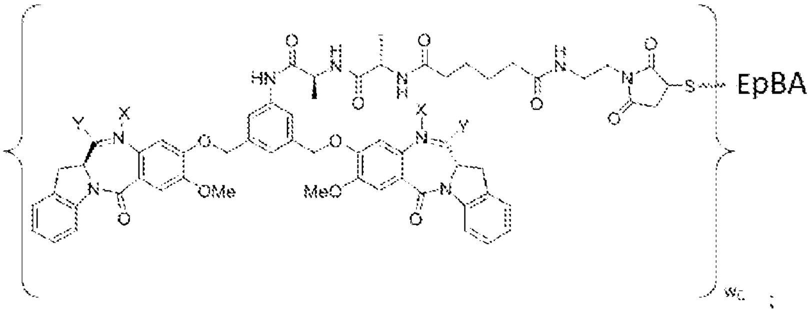

;

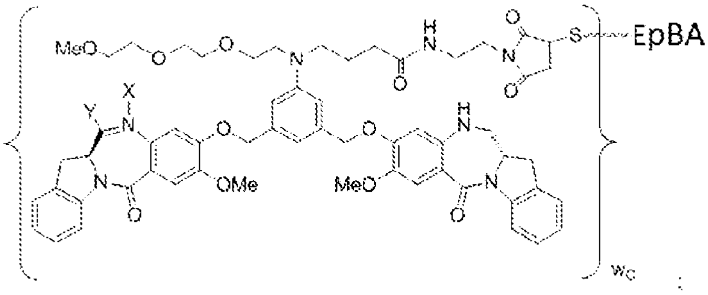

;

or

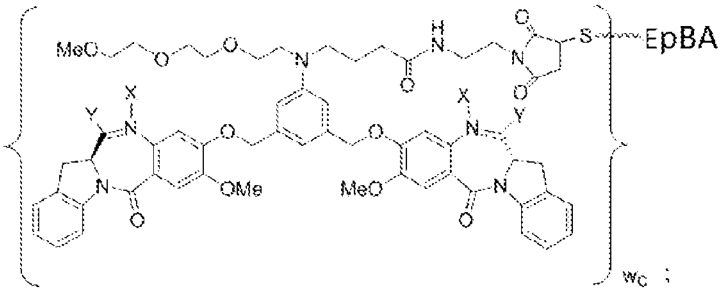

;

or a pharmaceutically acceptable salt thereof, wherein the double line between N and C represents a single bond or a double bond, provided that when it is a double bond, X is absent and Y is -H, and when it is a single bond, X is -H, and Y is -OH or -$SO_3H$ or a pharmaceutically acceptable salt thereof. In a more specific embodiment, the double line between N and C represents a double bond, X is absent and Y is -H. In another more specific embodiment, the double line between N and C represents a single bond, X is -H and Y is -$SO_3H$ or a pharmaceutically acceptable salt thereof.

In a 3rd embodiment, the immunoconjugate of the present invention is represented by the following formula:

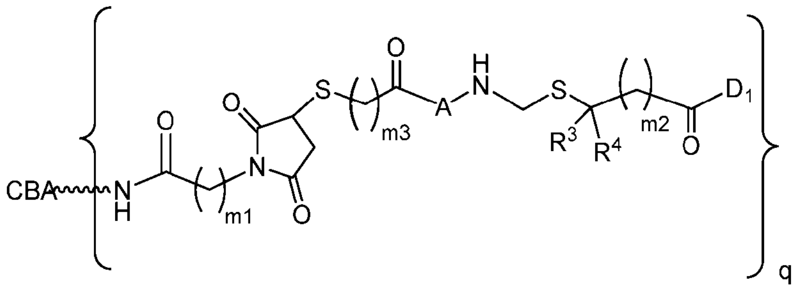

(Ia);

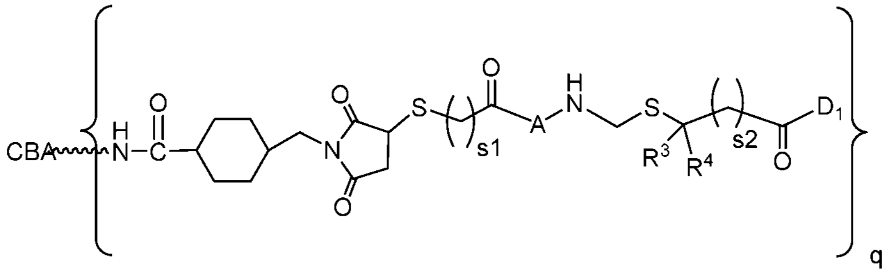

(Ib);

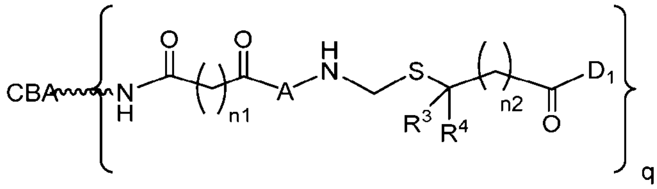

(Ic);

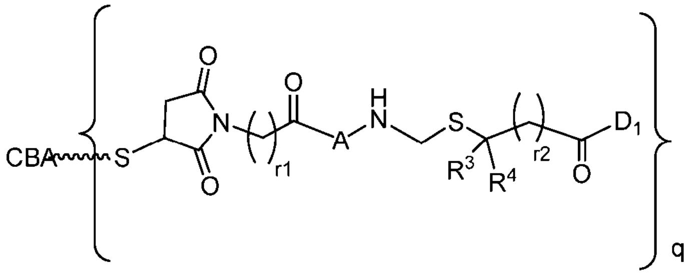

(Id);

or

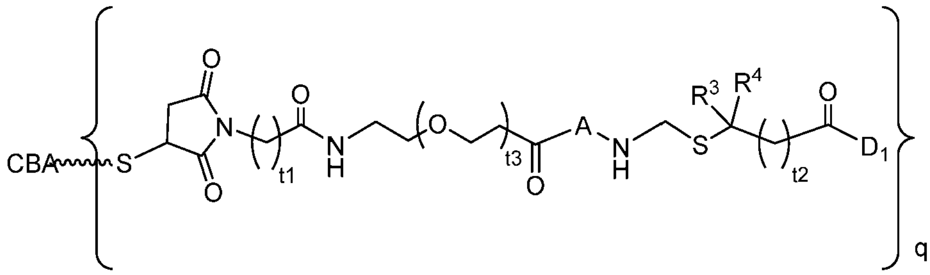

(Ie);

or a pharmaceutically acceptable salt thereof, wherein:

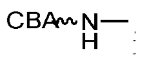

is EpBA connected to the $L_2$ group through a Lys amine group;

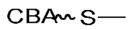

is the EpBA connected to the $L_2$ group through a Cys thiol group;
$R^3$ and $R^4$ are each independently H or Me;
m1, m3, n1, r1, s1 and t1 are each independently an integer from 1 to 6;
m2, n2, r2, s2 and t2 are each independently an integer from 1 to 7;
t3 is an integer from 1 to 12;
$D_1$ is represented by the following formula:

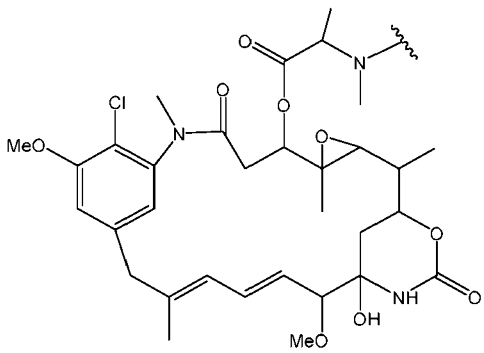

;

and

q is an integer from 1 to 20. In a more specific embodiment, $D_1$ is represented by the following formula:

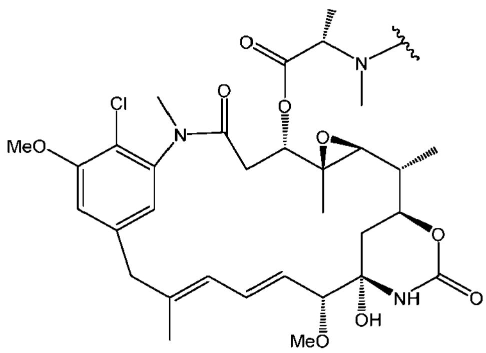

.

In an 1st specific embodiment of the 3rd embodiment, the immunoconjugate of the present invention is represented by the following formula:

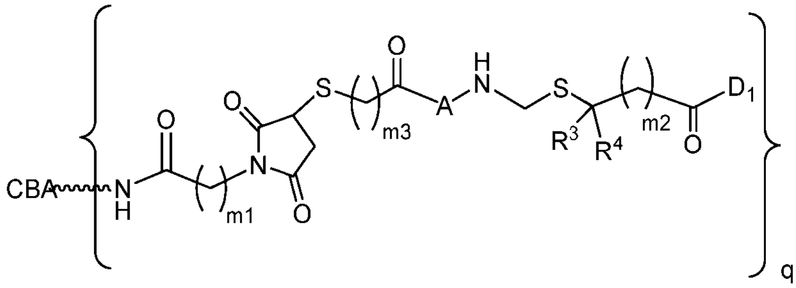

(Ia),

or

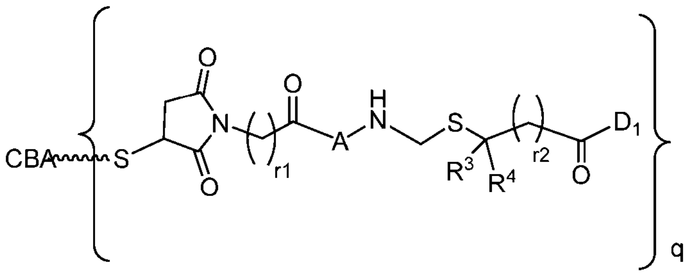

(Id);

wherein:

m1 and m3 are each independently an integer from 2 to 4;
m2 is an integer from 2 to 5;

r1 is an integer from 2 to 6;
r2 is an integer from 2 to 5; and

the remaining variables are as described in the 3rd embodiment.

In a 2nd specific embodiment, for the immunoconjugates described in the 3rd embodiment and 1st specific embodiment, A is Ala-Ala-Ala, Ala-D-Ala-Ala, Ala-Ala, D-Ala-Ala, Val-Ala, D-Val-Ala, D-Ala-Pro, or D-Ala-tBu-Gly. In a more specific embodiment, for the immunoconjugates described in the 3rd embodiment and 1st specific embodiment, A is L-Ala-D-Ala-L-Ala.

In a 3rd specific embodiment, the immunoconjugate of the present invention is represented by the following formula:

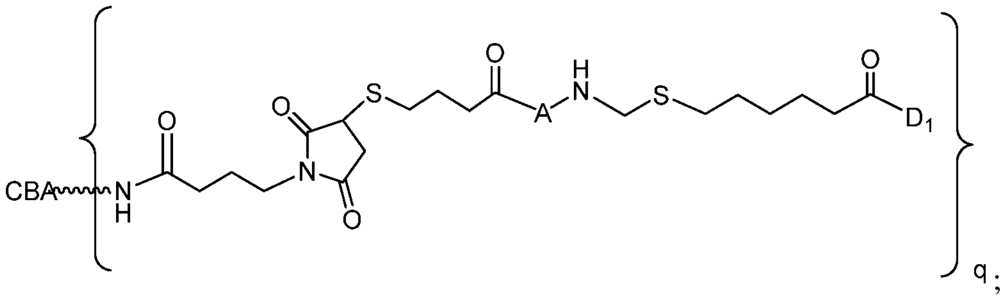

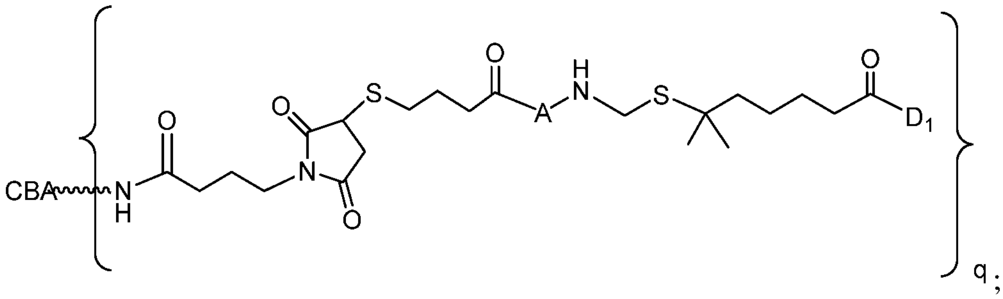

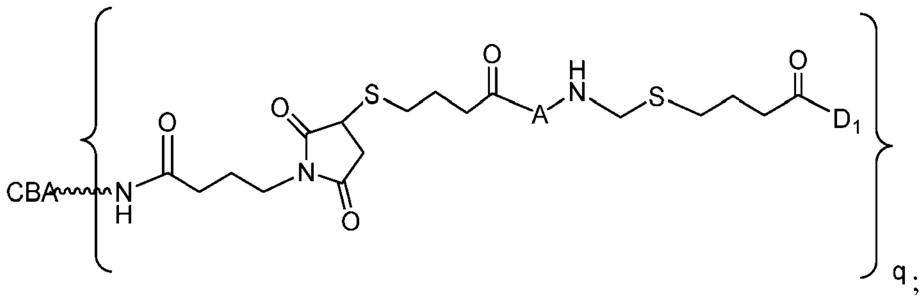

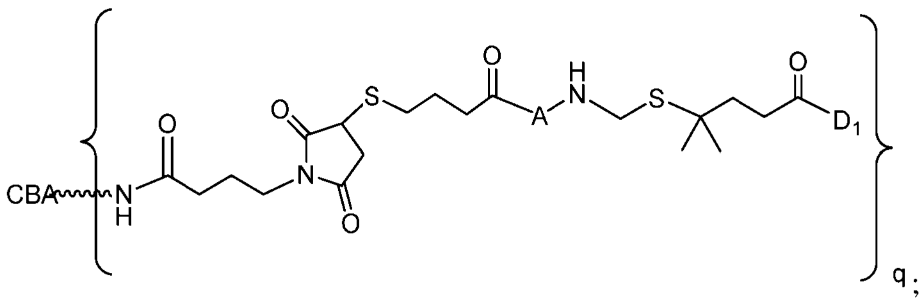

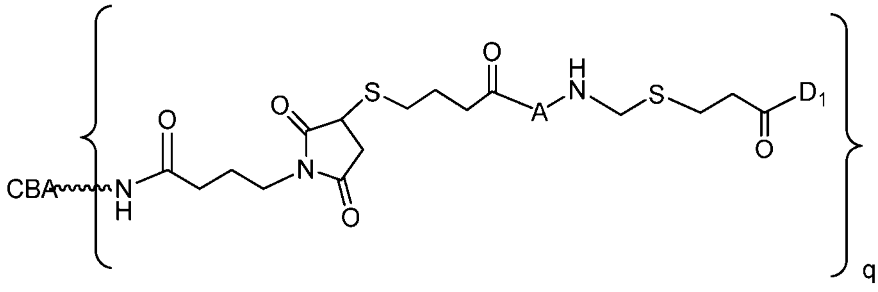
;

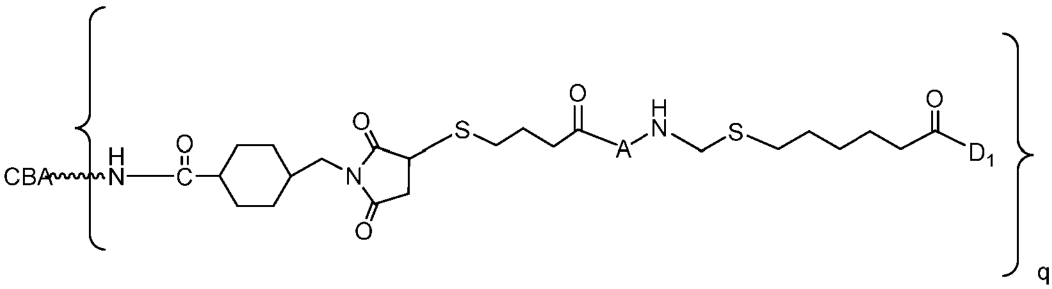
;

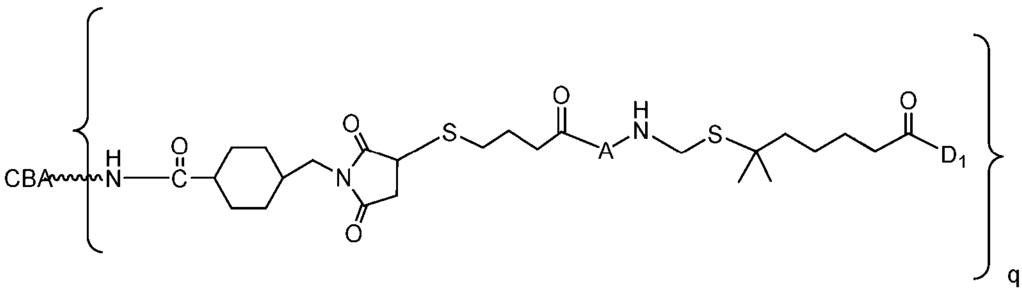
;

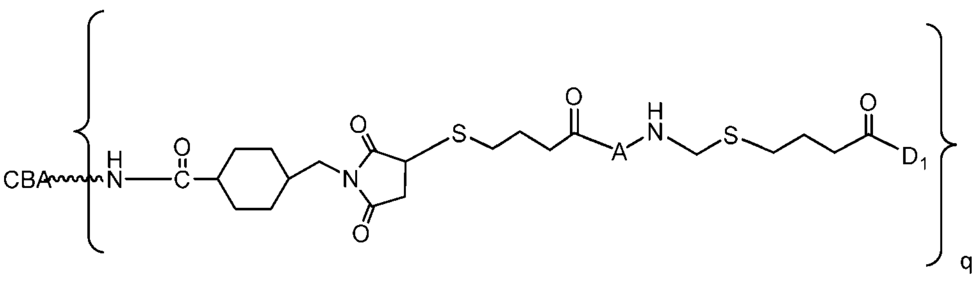
;

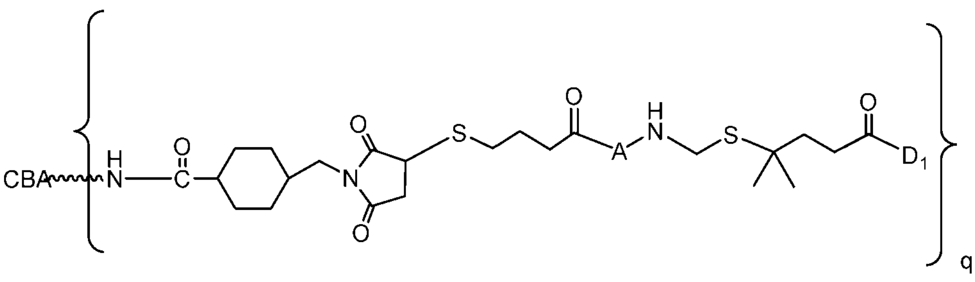
;

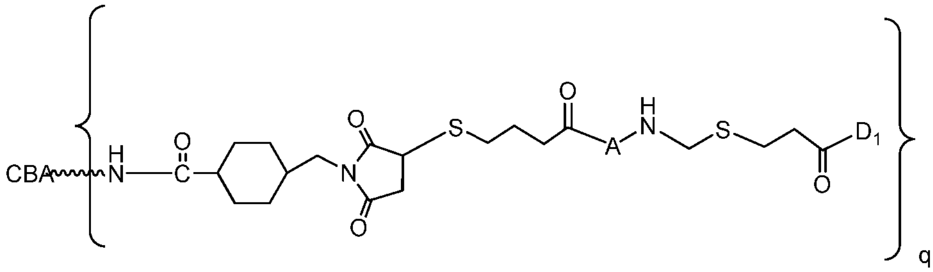
;

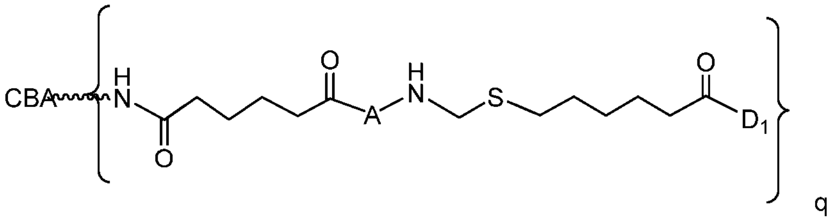
;

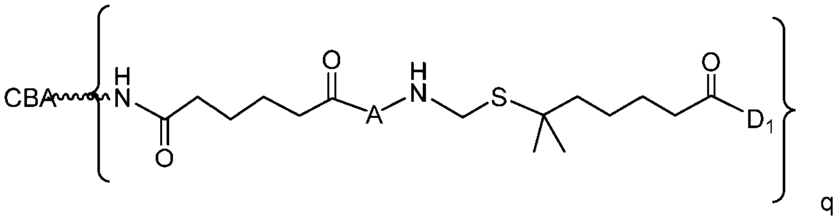
;

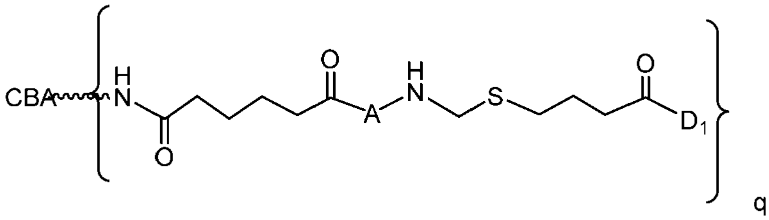
;

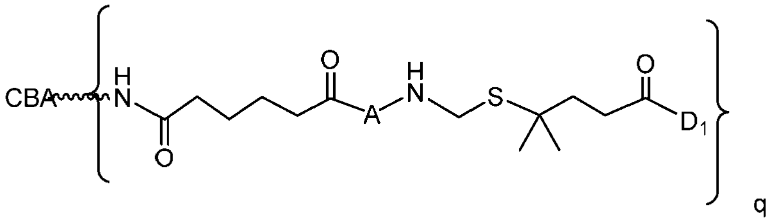
;

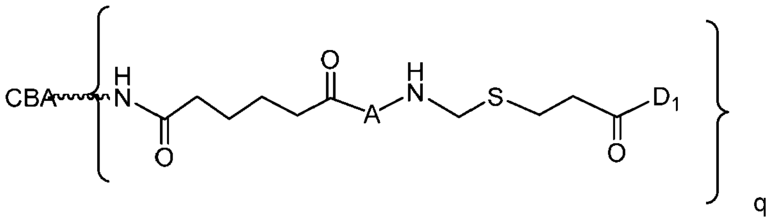
;

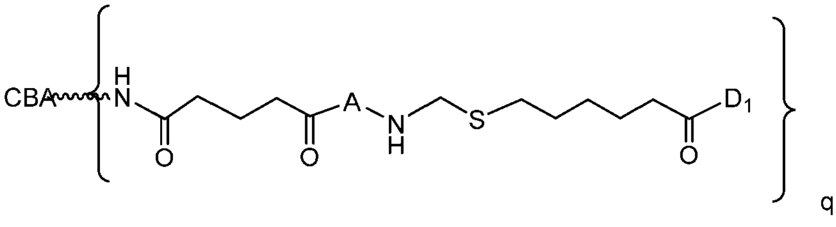
;

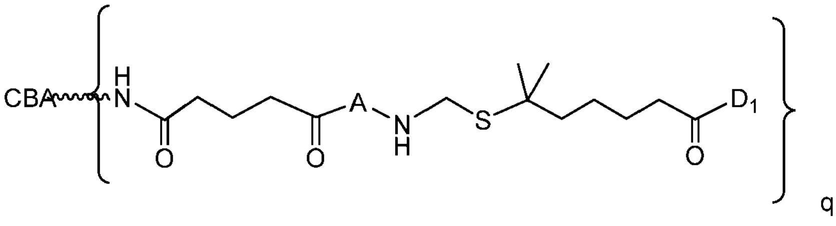
;

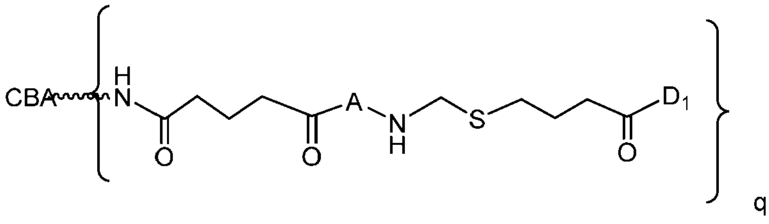
;

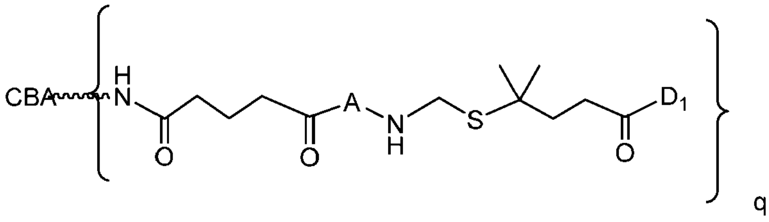
;

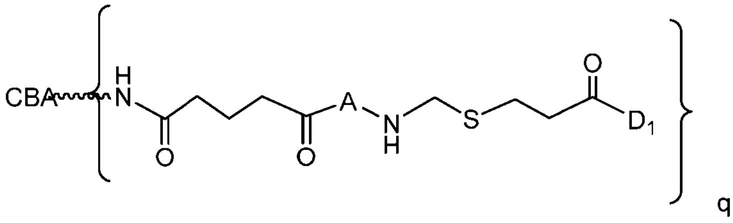
;

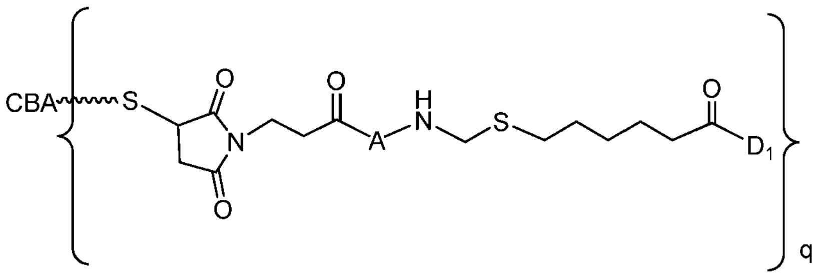
;

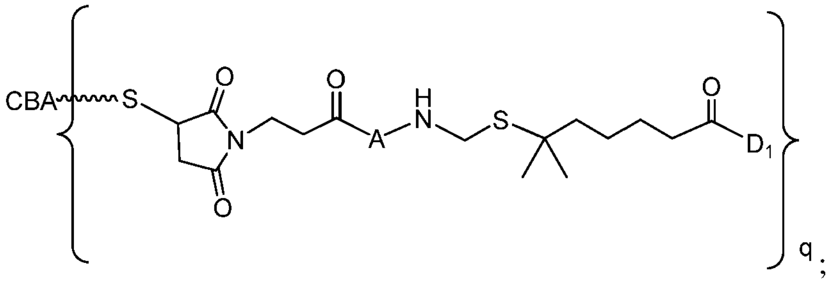

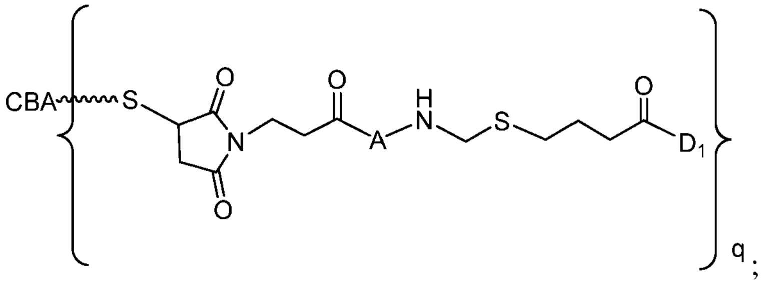

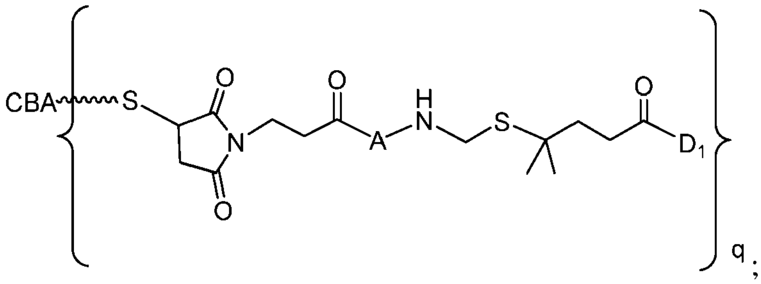

or

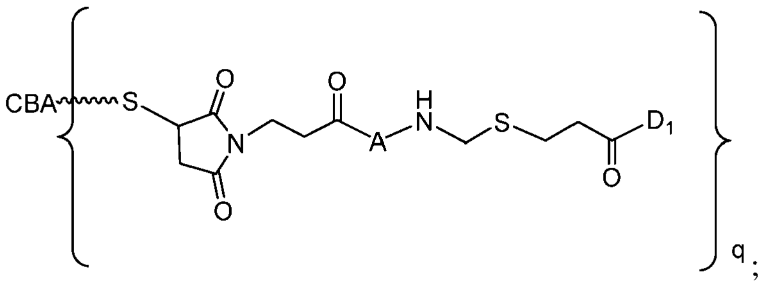

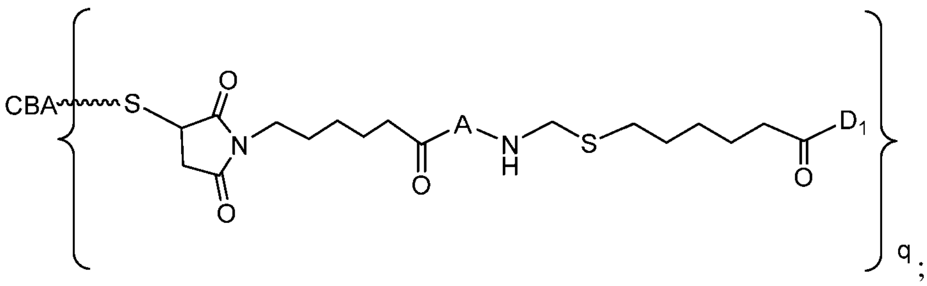

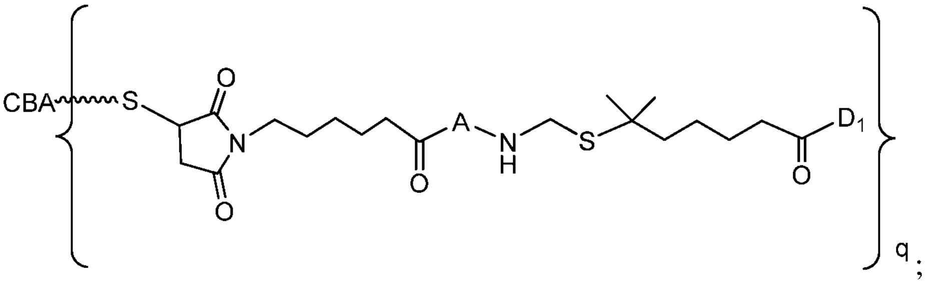
;

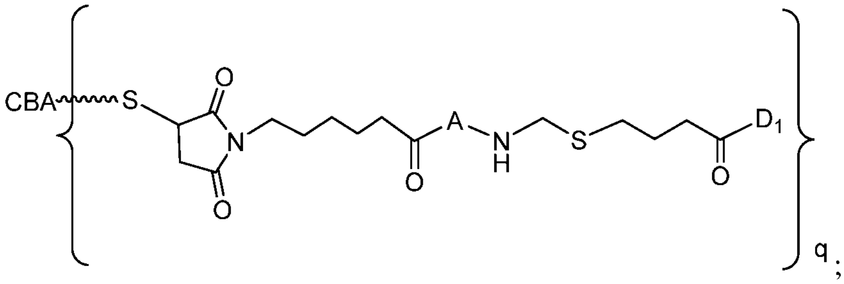
;

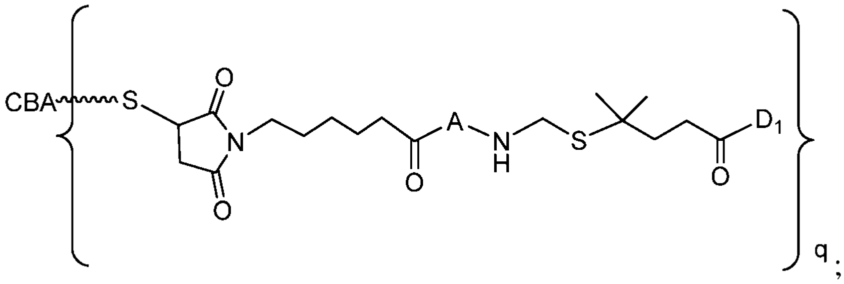
;

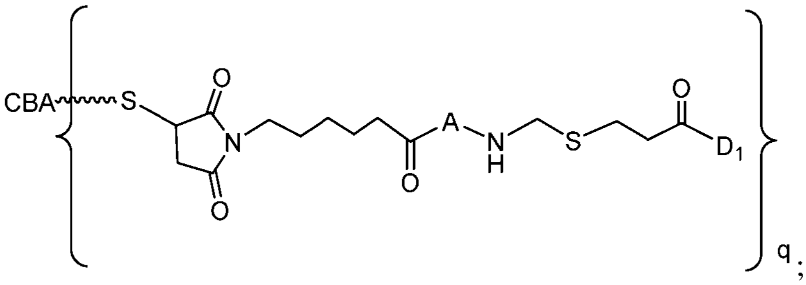
;

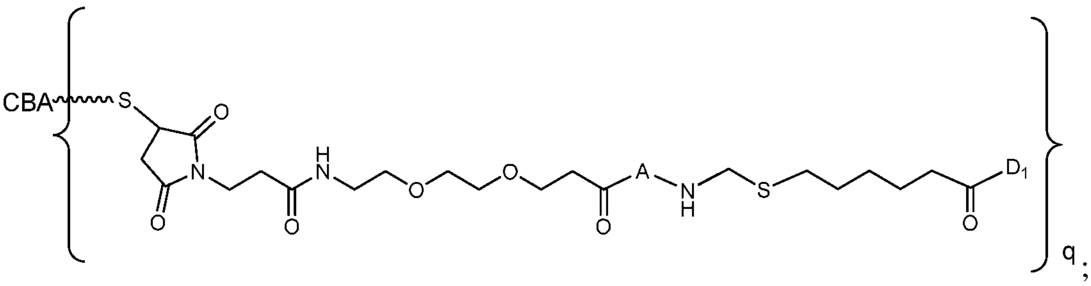
;

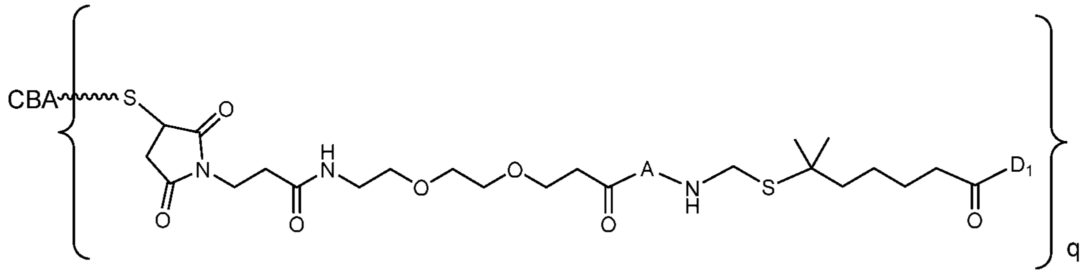

q ;

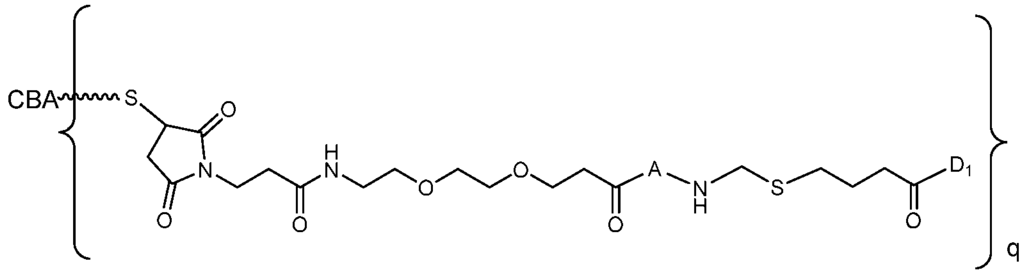

q ;

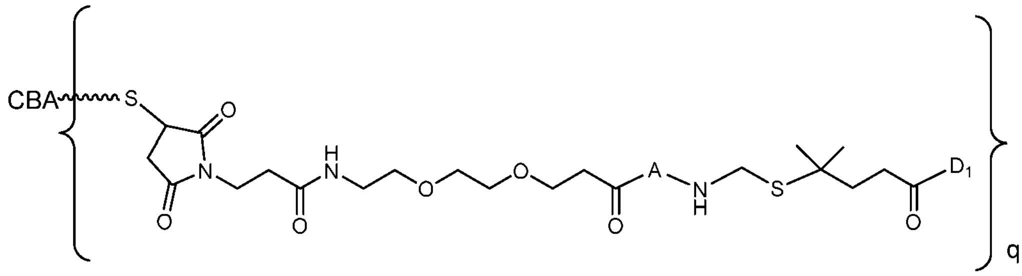

q ;

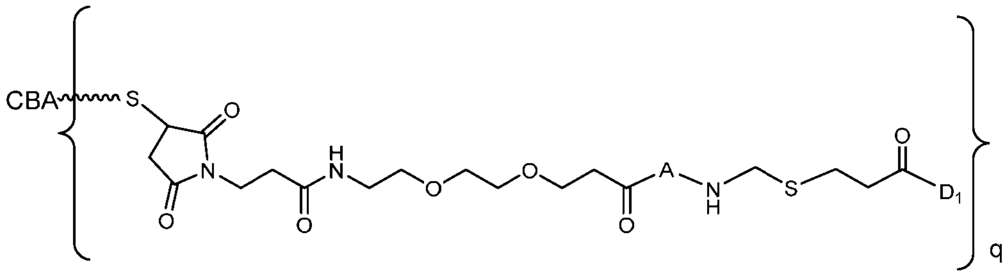

q ;

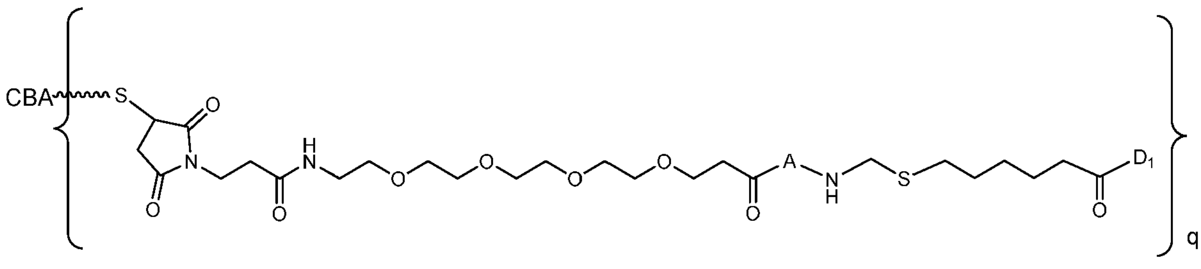

q ;

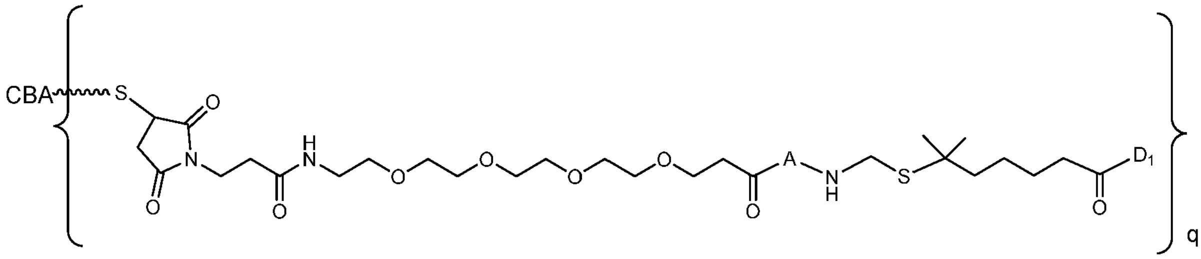

;

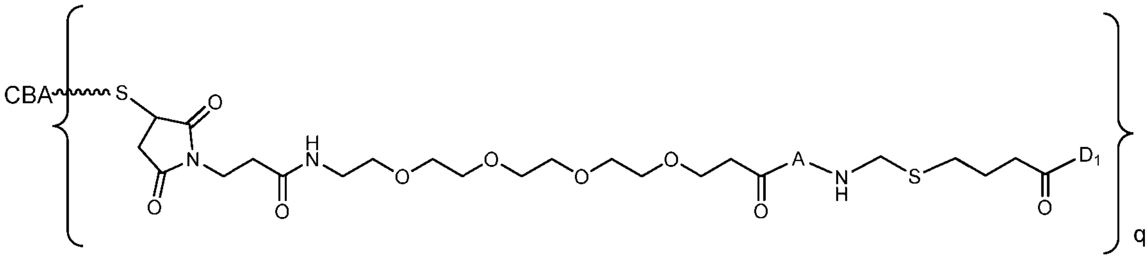

;

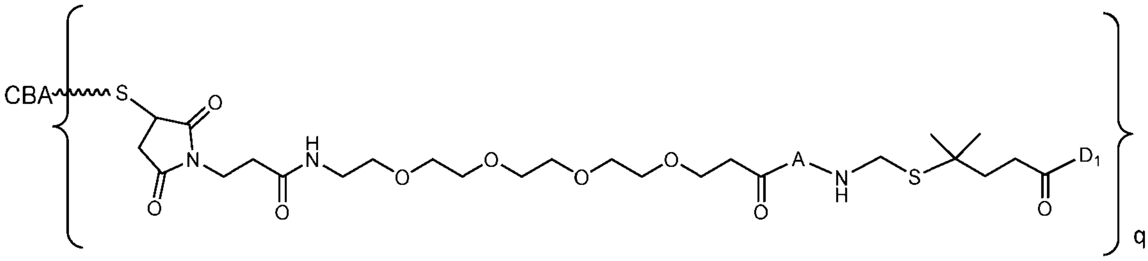

;

or

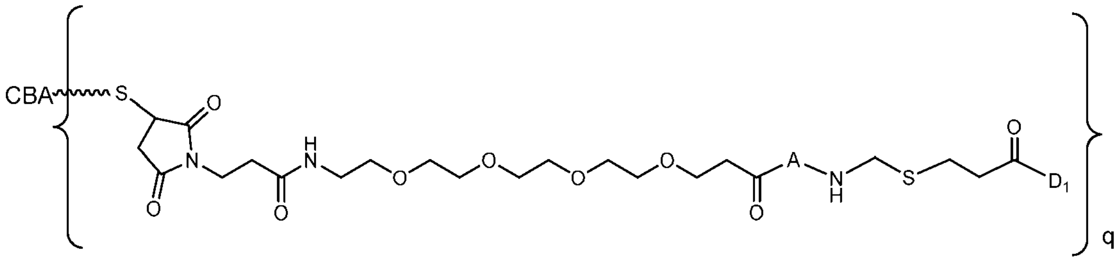

or a pharmaceutically acceptable salt thereof, wherein:

A is Ala-Ala-Ala, Ala-D-Ala-Ala, Ala-Ala, D-Ala-Ala, Val-Ala, D-Val-Ala, D-Ala-Pro, or D-Ala-tBu-Gly, and $D_1$ is represented by the following formula:

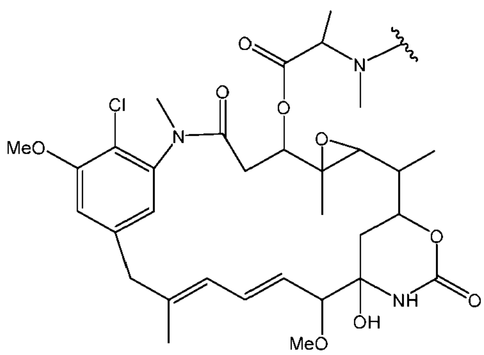

;

and the remaining variables are as described in the 3rd embodiment and its 1st and 2nd specific embodiments. In a more specific embodiment, A is L-Ala-D-Ala-L-Ala. In a more specific embodiment, $D_1$ is represented by the following formula:

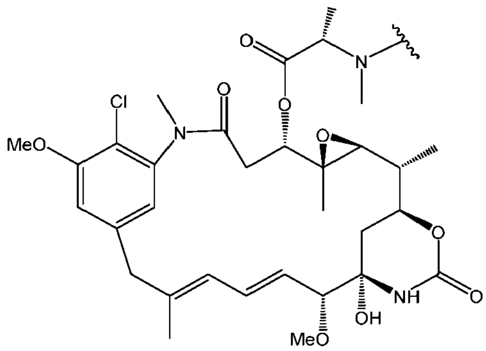

.

In a 4th specific embodiment, the immunoconjugate of the present invention is represented by the following formula:

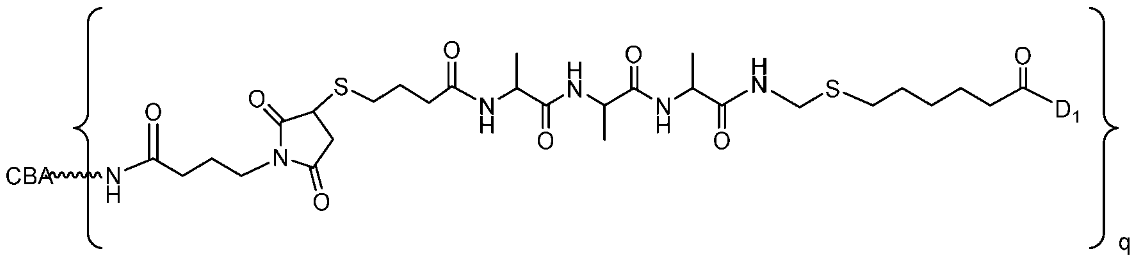

;

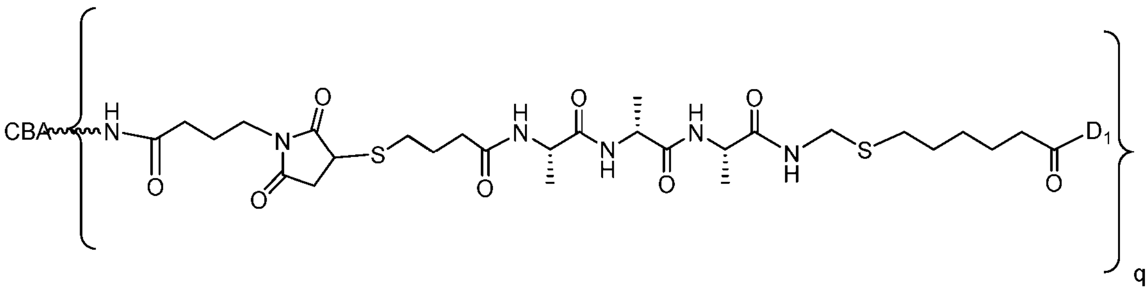

;

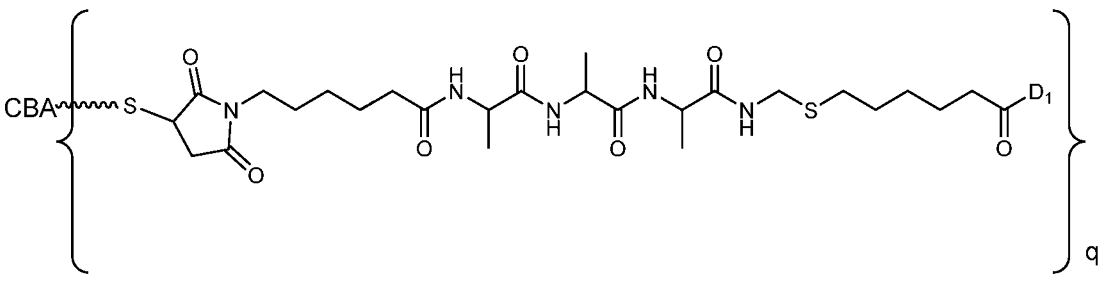

;

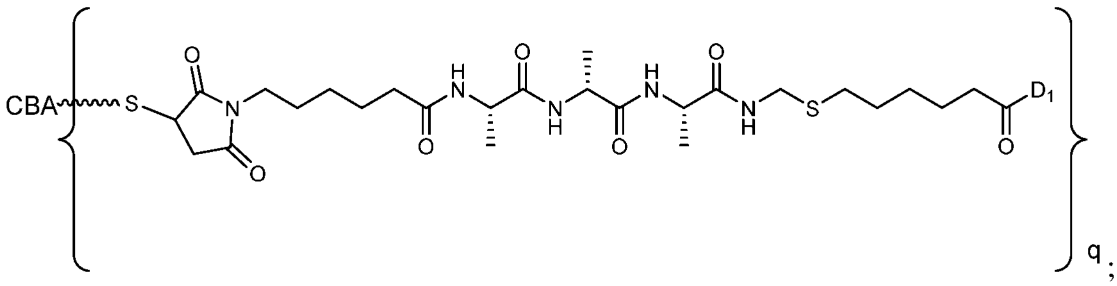

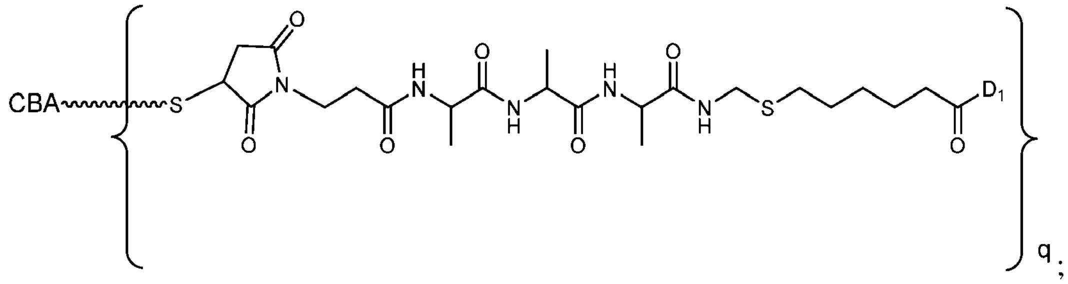

or

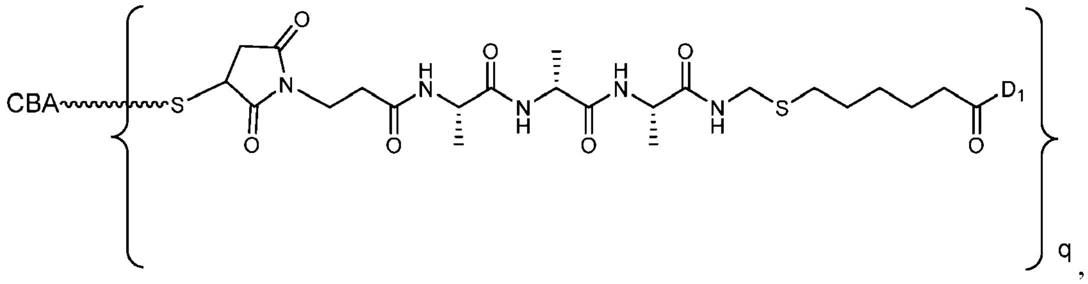

wherein $D_1$ is represented by the following formula:

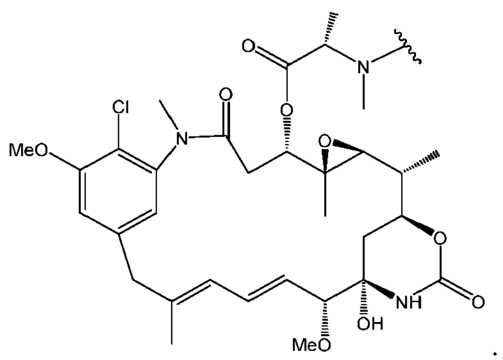

In a 5th specific embodiment, the immunoconjugate of the present invention is represented by the following formula:

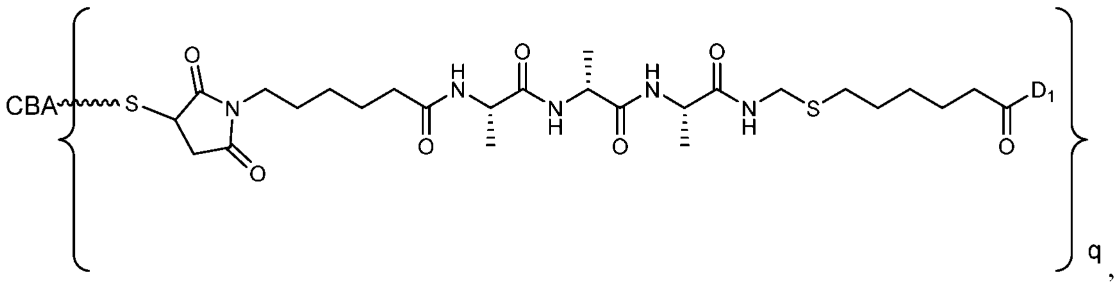

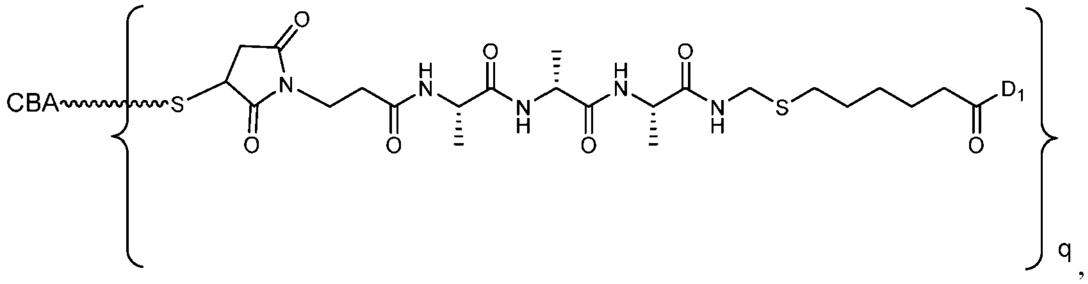

wherein:

CBA is an EpBA;
q is 1 or 2;
$D_1$ is represented by the following formula:

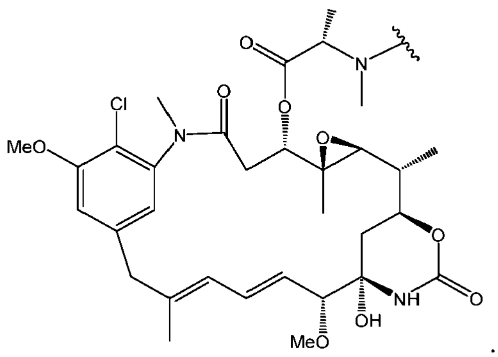

In a 6th specific embodiment, the immunoconjugate of the present invention is represented by the following formula:

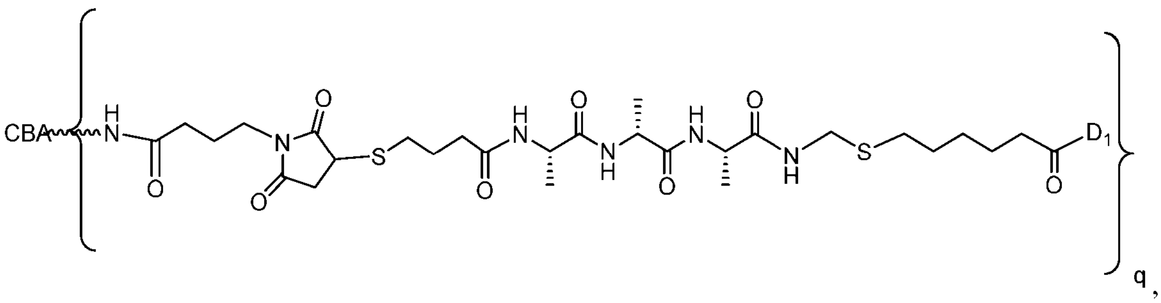

wherein:

CBA is EpBA;
q is an integer from 1 or 10;
$D_1$ is represented by the following formula:

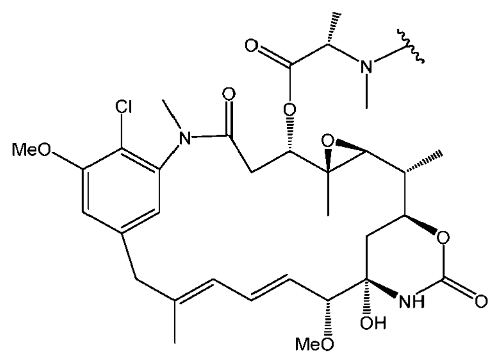

.

In another specific embodiment, the disclosure provides an EpBA immunoconjugate that comprises an EpBA *(e.g.,* EpCAM antibody, EpCAM-binding antibody fragment, or EpCAM activatable antibody) coupled to a maytansinoid compound DM21L (also referred to as LDL-DM) represented by the following formula:

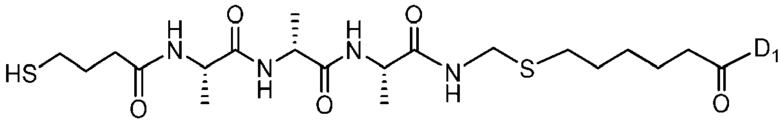
(D-2);

via γ-maleimidobutyric acid N-succinimidyl ester (GMBS) or a N-(γ-maleimidobutryloxy)sulfosuccinimide ester (sulfo-GMBS or sGMBS) linker. The GMBS and sulfo-GMBS (or sGMBS) linkers are known in the art and can be presented by the following structural formula:

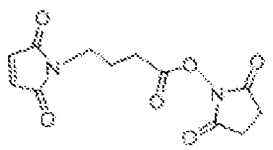
GMBS

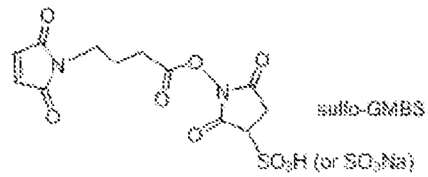
sulfo-GMBS

.

In one embodiment, the immunoconjugate is represented by the following formula:

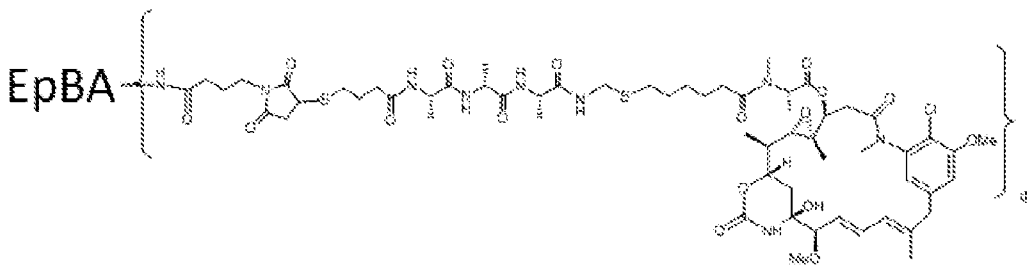

,

wherein:

EpBA is an EpCAM antibody, EpCAM-binding antibody fragment, or EpCAM activatable antibody connected to the maytansinoid compound through a Lys amine group, wherein q is an integer from 1 or 10.

In some embodiments, the EpBA of the EpBA-DM21L conjugate comprises an EpCAM antibody, EpCAM-binding antibody fragment, or EpCAM activatable antibody disclosed herein. In some embodiments, the EpBA of the EpBA-DM21L conjugate comprises a VH-CDR1 comprising $X_1YX_3X_4H$, wherein $X_1$ is selected from N and S, $X_3$ is selected from Y, N, F, S, H, D, L, I, and W, and $X_4$ is selected from I and M (SEQ ID NO:5); a VH-CDR2 comprising $WX_2X_3PGX_6VYIQYX_{12}X_{13}KFX_{17}G$, wherein $X_2$ is selected from I and F, $X_3$ is selected from Y and N, $X_6$ is selected from N and D, $X_{12}$ is selected from N and S, $X_{13}$ is selected from E and Q, and $X_{17}$ is selected from K and Q (SEQ ID NO:7); and a VH-CDR3 comprising $X_1GX_3X_4FAY$, wherein $X_1$ is selected from D and E, $X_3$ is selected from P, A, S, Y, F, G, T, and V, and $X_4$ is selected from Y and W (SEQ ID NO:8). In some embodiments, the EpBA of the EpBA-DM21L conjugate comprises a light chain CDR1 (VL-CDR1) comprising $RSSX_4SLLHSX_{10}GX_{12}TYLX_{16}$, wherein $X_4$ is selected from R and K, $X_{10}$ is selected from N and D, $X_{12}$ is selected from F and I, and $X_{16}$ is selected from Y and S (SEQ ID NO:10); a light chain VL-CDR2 comprising QTSNLAS (SEQ ID NO:40); and a VL-CDR3 comprising $X_1QX_3LELPX_8T$, wherein $X_1$ is selected from A, L, and Q, $X_3$ is selected from S, G, Y, and N, and $X_8$ is selected from N and W (SEQ ID NO:11).

In some embodiments, the VH-CDR1 of the EpBA comprises the sequence $NYX_3IH$, wherein $X_3$ is selected from Y, N, F, S, H, D, L, I, and W (SEQ ID NO:6). In some embodiments, the VH-CDR3 of the EpBA comprises the sequence $DGPX_4FAY$, wherein $X_4$ is selected from Y and W (SEQ ID NO:9). In some embodiments, the VL-CDR3 of the EpBA comprises the sequence $AQX_3LELPNT$, wherein $X_3$ is selected from S, G, Y, and N (SEQ ID NO: 12).

In some embodiments, the EpBA of the EpBA-DM21L conjugate comprises an EpCAM antibody, EpCAM-binding antibody fragment, or EpCAM activatable antibody comprising a VH-CDR1, VH-CDR2, VH-CDR3, VL-CDR1, VL-CDR2, and VL-CDR3 having the sequences of SEQ ID NOs: 13-15, 42, 40, and 41, respectively. In some embodiments, the EpBA of the EpBA-DM21L conjugate comprises an EpCAM antibody, EpCAM-binding antibody fragment, or EpCAM activatable antibody comprising a VH having the sequence of SEQ ID NO: 54 and a VL having the sequence of SEQ ID NO: 89. In some embodiments, EpBA of the EpBA-DM21L conjugate comprises an EpCAM antibody, EpCAM-binding antibody fragment, or EpCAM activatable antibody comprising a HC having the sequence of SEQ ID NO: 103 and a LC having the sequence of SEQ ID NO: 140.

In some embodiments, the EpBA of the EpBA-DM21L conjugate comprises an EpCAM activatable antibody comprising a MM of SEQ ID NO:155. In some embodiments, the EpBA of the EpBA-DM21L conjugate further comprises an EpCAM activatable antibody comprising a CM of SEQ ID NO:168. In alternative embodiments, the EpBA of the EpBA-DM21L conjugate comprises an EpCAM activatable antibody comprising a CM of SEQ ID NO:169. In one embodiment, the EpBA of the EpBA-DM21L conjugate comprises an EpCAM activatable antibody comprising a heavy chain having the sequence of SEQ ID NO: 103 and a light chain having the sequence of SEQ ID NO: 174. In one embodiment, the EpBA of the EpBA-DM21L conjugate comprises an EpCAM activatable antibody comprising a heavy chain having the sequence of SEQ ID NO: 103 and a light chain having the sequence of SEQ ID NO:179.

In certain embodiments, for compositions (e.g., pharmaceutical compositions) comprising EpBA-LDL-DM immunoconjugates, the average number of the cytotoxic agent per antibody molecule *(i.e.,* average value of q), also known as Drug-Antibody Ratio (DAR) DAR is in the range of 3.0 to 4.0, 3.2 to 3.8, or 3.4 to 3.7. In some embodiments, the DAR is 3.2, 3.3, 3.4, 3.5, 3.5, 3.7, or 3.8.

**Methods of Making Immunoconjugates**

The immunoconjugates comprising a EpCAM-binding agent (EpBA, *e.g.,* an EpCAM antibody, EpCAM-binding antibody fragment, or EpCAM activatable antibody) covalently linked to a cytotoxic agent through the ε-amino group of one or more lysine residues located on the EpBA as described the first embodiment above or any specific embodiments, descried therein can be prepared according to any methods known in the art, *see, e.g.,* WO 2012/128868 and WO 2012/112687, the entire contents of each of which is herein incorporated by reference in its entirety.

In certain embodiments, the immunoconjugates of the first embodiment are prepared by a first method comprising the steps of reacting the EpBA *(e.g.,* an EpCAM antibody, EpCAM-binding antibody fragment, or EpCAM activatable antibody, disclosed herein) with the cytotoxic agent having an amine reactive group.

In one embodiment, for the first method described above, the reaction is carried out in the presence of an imine reactive reagent, such as $NaHSO_3$.

In one embodiment, for the first method described above the cytotoxic agent having an amine reactive group is represented by the following formula:

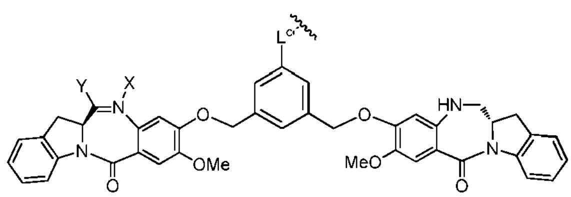
(L1a'),

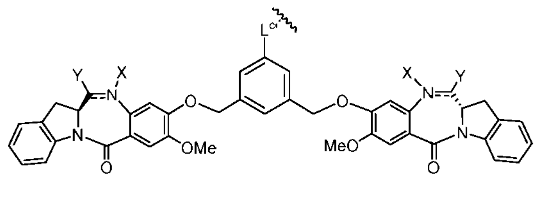
(L1a'1),

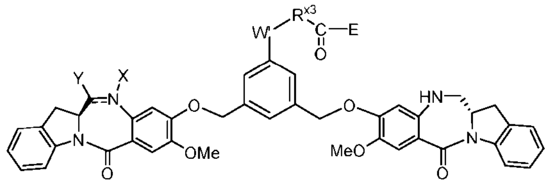

(L1b'),

or

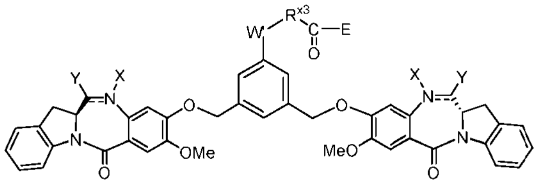

(L1b'1);

or a pharmaceutically acceptable salt thereof, wherein the definitions for the variables are described above for formulas (L1a'), (L1a'1), (L1b') and (L1b'1).

In certain embodiments, the immunoconjugates of the first embodiment is prepared by a second method comprising the steps of:

(a) reacting the cytotoxic agent with a linker compound having an amine reactive group and a thiol reactive group to form a cytotoxic agent-linker compound having the amine reactive group bound thereto; and

(b) reacting the EpBA with the cytotoxic agent-linker compound.

In one embodiment, the reaction in step (a) is carried out in the presence of an imine reactive reagent (*e.g.,* $NaHSO_3$). In one embodiment, the cytotoxic agent-linker compound is reacted with the EpBA without purification. Alternatively, the cytotoxic agent-linker compound is first purified before reacting with the EpBA.

In certain embodiments, the immunoconjugates of the first embodiment is prepared by a third method comprising the steps of:

(a) reacting the EpBA with a linker compound having an amine reactive group and a thiol reactive group to form a modified EpBA having a thiol reactive group bound thereto; and

(b) reacting the modified EpBA with the cytotoxic agent.

In one embodiment, the reaction in step (b) is carried out in the presence of an imine reactive reagent (*e.g.,* $NaHSO_3$).

In certain embodiments, the immunoconjugates of the first embodiment is prepared by a fourth method comprising the steps of reacting the EpBA, a cytotoxic compound and a linker compound having an amine reactive group and a thiol reactive group.

In one embodiment, the reaction is carried out in the presence of an imine reactive agent *(e.g.,* $NaHSO_3$).

In certain embodiments, for the second, third or fourth method, described above, the linker compound having an amine reactive group and a thiol reactive group is represented by the following formula:

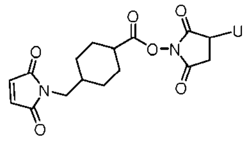

(a1L);

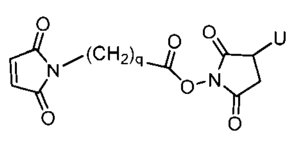

(a2L);

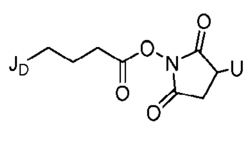

(a3L);

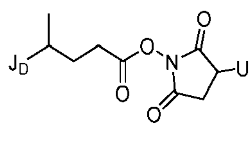

(a4L);

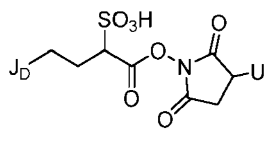

(a5L),

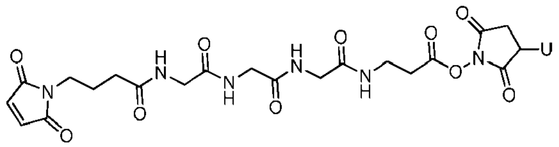 (a6L), 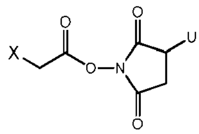 (a7L);

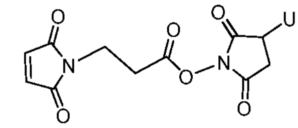 (a8L); 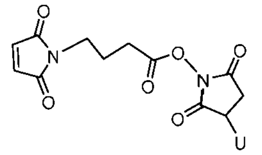 (a9L); and 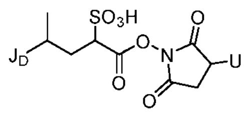 (a10L),

wherein X is halogen; $J_D$-SH, -SSR$^d$, or -SC(=O)R$^g$; R$^d$ is phenyl, nitrophenyl, dinitrophenyl, carboxynitrophenyl, pyridyl or nitropyridyl; R$^g$ is an alkyl; and the remaining variables are as described above for formula (a1) - (a10); and the cytotoxic agent is represented by the following formula:

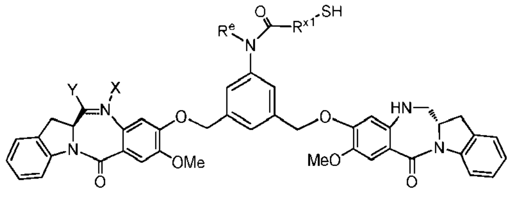 (L2a');

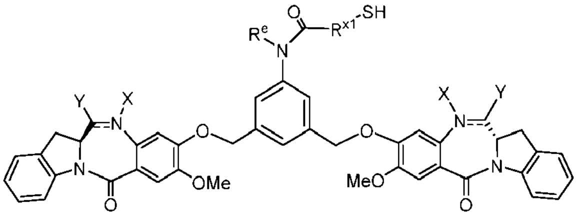 (L2a'1);

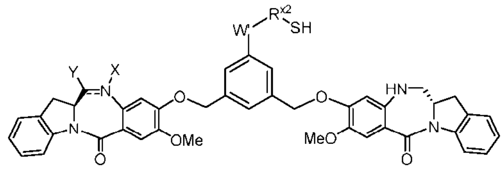 (L2b');

or

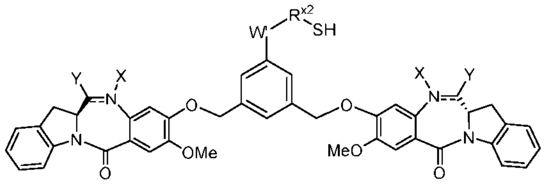 (L2b'1),

or a pharmaceutically acceptable salt thereof, wherein the variables are as described above for formulas (L1a'), (L1a'1), (L1b'), (L1b'1), (L2a'), (L2a'1), (L2b') and (L2b'1).

In certain embodiments, for the second, third or fourth methods described above, the linker compound having an amine reactive group and a thiol reactive group is represented by any one of the formula (a1L) - (a10L) and the cytotoxic agent is represented by the following formula:

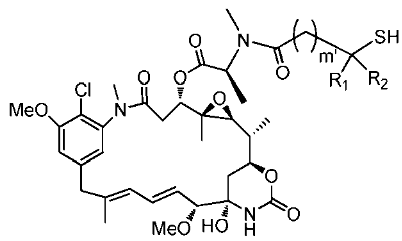

wherein the variables are as described above in any one of the 13th to 15th specific embodiments, of the first embodiment described above and any more specific embodiments, described therein.

In a specific embodiment, for the second, third or fourth methods described above, the linker is sulfo-SPDB, the cytotoxic agent is DM4 and the immunoconjugate is represented by the following formula:

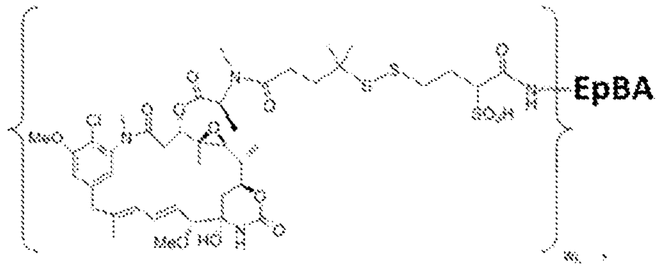

or a pharmaceutically acceptable salt thereof, wherein $W_L$ is an integer from 1 to 10.

The immunoconjugates comprising a EpCAM-binding agent covalently linked to a cytotoxic agent through the thiol group (-SH) of one or more cysteine residues located on the EpCAM-binding agent as described in the second embodiment above (e.g., immunoconjugates of any one of the 1st to 23rd specific embodiments, or any more specific embodiments, described therein) can be prepared by reacting the EpBA having one or more free cysteine with a cytotoxic agent having a thiol-reactive group disclosed herein.

In one embodiment, the cytotoxic agent having a thiol-reactive group is represented by the following formula:

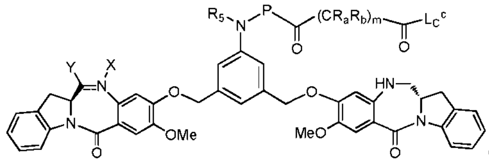

(C1a');

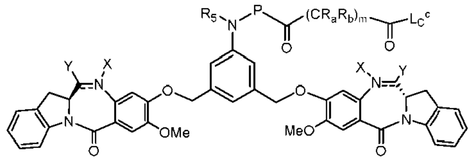

(C1a'1)

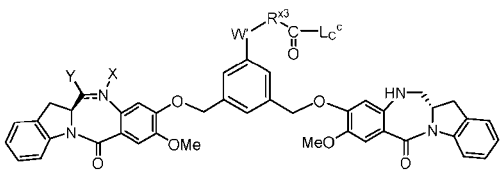

(C1b');

or

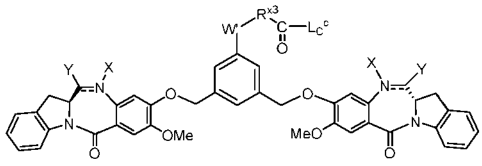
(C1b'1),

or a pharmaceutically acceptable salt thereof, wherein $-L_C{}^c$ is represented by the following formula:

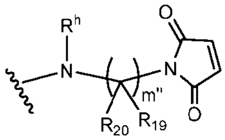

wherein the variables are as described above in any one of the 1st to 9th and 23rd specific embodiments, of the second embodiment or any more specific embodiments, described therein.

In another embodiment, the cytotoxic agent having a thiol-reactive group is represented by the following formula:

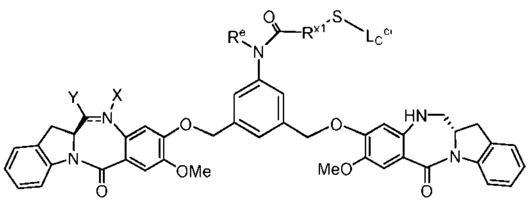
(C2a"),

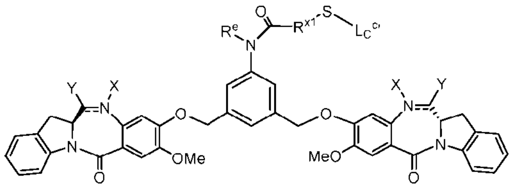
(C2a"1),

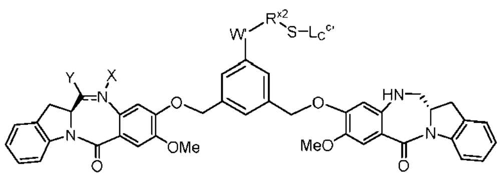
(C2b"),

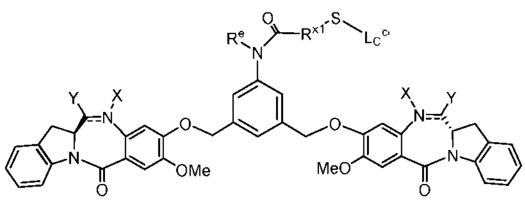
(C2b"1)

or a pharmaceutically acceptable salt thereof, wherein Lcc' is represented by the following formula:

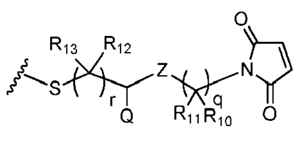
; or
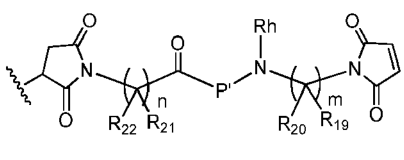
;

wherein the variables are as described above in any one of the 10th to 16th and 23rd specific embodiment of the second embodiment or any more specific embodiments, described therein.

In yet another embodiment, the cytotoxic agent having a thiol-reactive group is represented by the following formula:

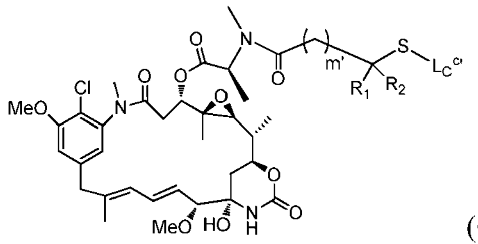

(C3a'),

or a pharmaceutically acceptable salt thereof, wherein LCc' is described above and the remaining variables are as described above in any one of the 17th to 23rd specific embodiments, of the second embodiment or any more specific embodiments, described therein.

In certain embodiments, organic solvents are used in the reaction of the EpBA and the cytotoxic agent to solubilize the cytotoxic agent. Exemplary organic solvents include, but are not limited to, dimethylacetamide (DMA), propylene glycol, *etc.* In one embodiment, the reaction of the EpBA and the cytotoxic agent is carried out in the presence of DMA and propylene glycol.

In a specific embodiment, the cytotoxic agent represented by the following formula:

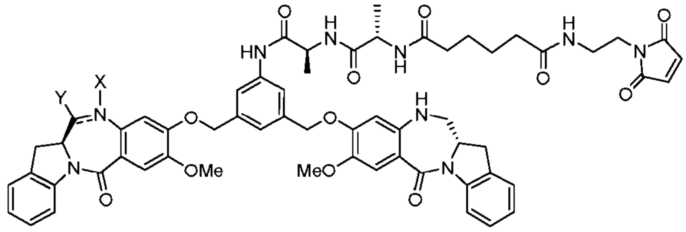

or a pharmaceutically acceptable salt thereof, is reacted with a EpBA *(e.g.,* an EpCAM antibody, EpCAM-binding antibody fragment, or an EpCAM activatable antibody, disclosed herein) to form the immunoconjugate represented by the following formula:

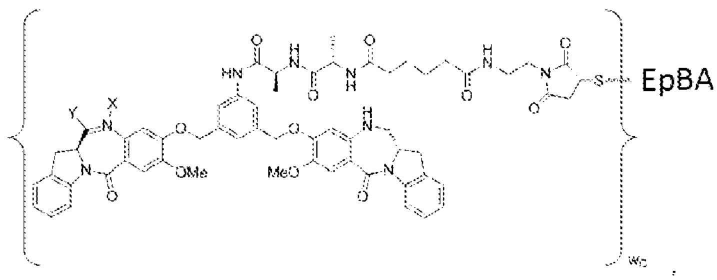

or a pharmaceutically acceptable salt thereof, wherein:

the double line between N and C represents a single bond or a double bond, provided that when it is a double bond, X is absent and Y is -H, and when it is a single bond, X is -H, and Y is - SO3H or a pharmaceutically acceptable salt thereof; and WC¬ is 1 or 2. In a more specific embodiment, the double line between N and C represents a double bond, X is absent and Y is -H. In another more specific embodiment, the double line between N and C represents a single bond, X is -H and Y is -SO3H or a pharmaceutically acceptable salt thereof. Even more specifically, the pharmaceutically acceptable salt is a sodium or a potassium salt.

In certain embodiments, when Y is -SO3H or a pharmaceutically acceptable salt thereof, the immunoconjugates are prepared by (a) reacting the imine-moiety in the imine-containing cytotoxic agent having a thiol-reactive group described above (*i.e.*, formula (C1a'), (C1a'1), (C1b'), (C1b'1), (C2a"), (C2a"1), (C2b") or (C2b"1), wherein the double line between N and C represents a double bond, X is absent and Y is -H) with a sulfur dioxide, bisulfite salt or a metabisulfite salt in an aqueous solution at a pH of 1.9 to 5.0 to form a modified cytotoxic agent comprising a modified imine moiety represented by the following formula:

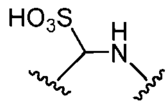

or a pharmaceutically acceptable salt thereof; and (b) reacting the modified cytotoxic agent with the EpCAM-binding agent *(e.g.,* an EpCAM antibody, EpCAM-binding antibody fragment, or an EpCAM activatable antibody, disclosed herein) disclosed herein to form the immunoconjugate.

In a 1st aspect, for the method described above, the reaction of step (a) is carried out at a pH of 1.9 to 5.0. More specifically, the pH is 2.5 to 4.9, 1.9 to 4.8, 2.0 to 4.8, 2.5 to 4.5, 2.9 to 4.5, 2.9 to 4.0, 2.9 to 3.7, 3.1 to 3.5, or 3.2 to 3.4. In another specific embodiment, the reaction of step (a) is carried out at a pH of 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.5, 4.6, 4.7, 4.8, 4.9 or 5.0. In yet another specific embodiment, the reaction of step (a) is carried out at a pH of 3.3. As used herein, a specific pH value means the specific value $\pm$ 0.05.

In some embodiments, the reaction of step (a) is carried out in the presence of a buffer solution. Any suitable buffer solution known in the art can be used in the provided methods. Suitable buffer solutions include, for example, but are not limited to, a citrate buffer, an acetate buffer, a succinate buffer, a phosphate buffer, a glycine-containing buffer (*e.g.,* glycine-HCl buffer), a phthalate buffer (*e.g.,* a buffer solution comprising sodium or potassium hydrogen phthalate), and a combination thereof. In some embodiments, the buffer solution is a succinate buffer. In some embodiments, the buffer solution is a phosphate buffer. In some embodiments, the buffer is a citrate-phosphate buffer. In some embodiments, the buffer is a citrate-phosphate buffer comprising citric acid and Na2HPO4. In other embodiments, the buffer is a citrate-phosphate buffer comprising citric acid and K2HPO4. In some embodiments, the concentration of the buffer solution described above can be in the range of 10 to 250 mM, 10 to 200 mM, 10 to 150 mM, 10 to 100 mM, 25 to 100 mM, 25 to 75 mM, 10 to 50 mM, or 20 to 50 mM.

In a 2nd aspect, the reaction step (a) is carried out in the absence of a buffer solution (*e.g.,* the buffers described in the 1st aspect). In some embodiments, the present method comprises the steps of: (a) reacting the imine-moiety in the imine-containing cytotoxic agent having a thiol-reactive group described above (*i.e*., formula (C1a’), (C1a’1), (C1b’), (C1b’1), (C2a"), (C2a"1), (C2b") or (C2b"1), wherein the double line between N and C represents a double bond, X is absent and Y is -H) with sulfur dioxide, a bisulfite salt or a metabisulfite salt in an aqueous solution to form a modified cytotoxic agent comprising a modified imine moiety represented by the following formula:

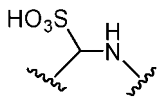

or a pharmaceutically acceptable salt thereof, wherein the aqueous solution does not comprise a buffer; and (b) reacting the modified cytotoxic agent with the EpCAM-binding agent *(e.g.,* an EpCAM antibody, EpCAM-binding antibody fragment, or an EpCAM activatable antibody, disclosed herein) disclosed herein to form the immunoconjugate. In some embodiments, the reaction of step (a) is carried out in a mixture of an organic solvent and water. More specifically, the reaction of step (a) is carried out in a mixture of dimethyacetamide (DMA) and water. In some embodiments, the mixture of DMA and water comprises less than 60% of DMA by volume. Even more specifically, the volume ratio of DMA and water is 1:1.

In a 3rd aspect, for the methods described above or in the 1st or 2nd aspect, 0.5 to 5.0 equivalents of the bisulfite salt or 0.25 or 2.5 equivalents of the metabisulfite salt is used for every 1 equivalent of the imine-containing cytotoxic agent in the reaction of step (a). In some embodiments, 0.5 to 4.5, 0.5 to 4.0, 0.5 to 3.5, 0.5 to 4.0, 0.5 to 3.5, 0.5 to 3.0, 0.5 to 2.5, 0.8 to 2.0, 0.9 to 1.8, 1.0 to 1.7, 1.1 to 1.6, or 1.2 to 1.5 equivalents of the bisulfite salt or 0.25 to 2.25, 0.25 to 2.0, 0.25 to 1.75, 0.25 to 2.0, 0.25 to 1.75, 0.25 to 1.5, 0.25 to 1.25, 0.4 to 1.0, 0.45 to 0.9, 0.5 to 0.85, 0.55 to 0.8, or 0.6 to 0.75 equivalents of the metabisulfite salt is used for every 1 equivalent of the imine-containing cytotoxic agent. In other embodiments, 0.5, 0.6, 0.7, 0.8, 0.9, 1.0, 1.1, 1.2, 1.3 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 4.0, 4.5 or 5.0 equivalents of the bisulfite salt or 0.25, 0.3, 0.35, 0.4, 0.45, 0.5, 0.55, 0.6, 0.65 0.7, 0.75, 0.8, 0.85, 0.9, 0.95, 1.0, 1.05, 1.1, 1.15, 1.2, 1.25, 1.3, 1.35, 1.4, 1.45, 1.5, 1.55, 1.6, 1.65, 1.7, 1.75, 2.0, 2.25 or 2.5 equivalents of the metabisulfite salt is used for every 1 equivalent of the imine-containing cytotoxic agent. In yet other embodiments, 1.4 equivalents of the bisulfite salt or 0.7 equivalent of the metabisulfite salt is used for every 1 equivalent of the imine-containing cytotoxic agent. In other embodiments, 1.2 equivalents of the bisulfite salt or 0.6 equivalent of the metabisulfite salt is used for every 1 equivalent of the imine-containing cytotoxic agent. As used herein, a specific equivalent means the specific value $\pm$ 0.05.

In a 4th aspect, for methods described above, the reaction of step (a) is carried out at a pH of 2.9 to 3.7 and 1.0 to 1.8 equivalents of the bisulfite salt or 0.5 to 0.9 equivalents of the metabisulfite salt is reacted with 1 equivalent of the imine-containing cytotoxic agent. In some embodiments, the reaction of step (a) is carried out at a pH of 3.1 to 3.5 and 1.1 to 1.6 equivalents of the bisulfite salt or 0.55 to 0.8 equivalents of the metabisulfite salt is reacted with 1 equivalent of the imine-containing cytotoxic agent. In other embodiments, the reaction of step (a) is carried out at a pH of 3.2 to 3.4 and 1.3 to 1.5 equivalents of the bisulfite salt or 0.65 to 0.75 equivalents of the metabisulfite is reacted with 1 equivalent of the imine-containing cytotoxic agent. In other embodiments, the reaction of step (a) is carried out at a pH of 3.3 and 1.4 equivalents of the bisulfite salt or 0.7 equivalent of the metabisulfite salt is reacted with 1 equivalent of the imine-containing cytotoxic agent. In yet other embodiments, the reaction of step (a) is carried out at a pH of 3.3 and 1.4 equivalents of sodium bisulfite is reacted with 1 equivalent of the imine-containing cytotoxic agent.

In a 5th aspect, for the methods described above or in the 1st, 2nd, 3rd or 4th aspect, the reaction of step (a) is carried out in a mixture of an organic solvent and water. Any suitable organic solvent can be used. Exemplary organic solvents include, but are not limited to, alcohols (e.g., methanol, ethanol, propanol, etc.), dimethylformamide (DMF), dimethylsulfoxide (DMSO), acetonitrile, acetone, methylene chloride, etc. In some embodiments, the organic solvent is miscible with water. In other embodiments, the organic solvent is not miscible with water, *i.e.,* the reaction of step (a) is carried out in a biphasic solution. In some embodiments, the organic solvent is dimethylacetamide (DMA). The organic solvent (e.g., DMA) can be present in the amount of 1%-99%, 1-95%, 10-80%, 20-70%, 30-70%, 1-60%, 5-60%, 10-60%, 20-60%, 30-60%, 40-60%, 45-55%, 10-50%, or 20-40%, by volume of the total volume of water and the organic solvent. In some embodiments, the reaction of step (a) is carried out in a mixture of DMA and water, wherein the volume ratio of DMA and water is 1:1.

In a 6th aspect, for the methods described above or in the 1st, 2nd, 3rd, 4th or 5th aspect, the reaction of step (a) can be carried out at any suitable temperature. In some embodiments, the reaction is carried out at a temperature from 0°C to 50°C, from 10°C to 50°C, from 10°C to 40°C, or from 10°C to 30°C. In other embodiments, the reaction is carried out at a temperature from 15°C to 30°C, from 20°C to 30°C, from 15°C to 25°C, from 16°C to 24°C, from 17°C to 23°C, from 18°C to 22°C or from 19°C to 21°C. In yet other embodiments, the reaction can be carried out at 15°C, 16°C, 17°C, 18°C, 19°C, 20°C, 21°C, 22°C, 23°C, 24°C or 25°C. In some embodiments, the reaction can be carried out from 0°C to 15°C, from 0°C to 10°C, from 1°C to 10°C, 5°C to 15°C, or from 5°C to 10°C.

In a 7th aspect, for the methods described above or in the 1st, 2nd, 3rd, 4th, 5th or 6th aspect, the reaction of step (a) is carried out for 1 minute to 48 hours, 5 minutes to 36 hours, 10 minutes to 24 hours, 30 minutes to 24 hours, 30 minutes to 20 hours, 1 hour to 20 hours, 1 hour to 15 hours, 1 hour to 10 hours, 2 hours to 10 hours, 3 hours to 9 hours, 3 hours to 8 hours, 4 hours to 6 hours, or 1 hour to 4 hours. In some embodiments, the reaction is allowed to proceed for 4, to 6 hours. In other embodiments, the reaction is allowed to proceed for 10 minutes, 15 minutes, 20 minutes, 30 minutes, 1 hours, 2 hours, 3 hours, 4 hours, 5 hours, 6 hours, 7 hours, 8 hours, 9 hours, 10 hours, 11 hours, 12 hours, 13 hours, 14 hours, 15 hours, etc. In other embodiments, the reaction is allowed to proceed for 4, hours. In yet other embodiments, the reaction is allowed to proceed for 2 hours.

In a 8th aspect, for the methods disclosed herein or in the 1st, 2nd, 3rd, 4th, 5th, 6th or 7th aspect, the reaction of step (b) is carried out at a pH of 4 to 9. In some embodiments, the reaction of step (b) is carried out at a pH of 4.5 to 8.5, 5 to 8.5, 5 to 8, 5 to 7.5, 5 to 7, 5 to 6.5, or 5.5 to 6.5. In other embodiments, the reaction of step (b) is carried out at pH 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0.

In some embodiments, for the methods described above or in the 1st, 2nd, 3rd, 4th, 5th, 6th, 7th or 8th aspect, the reaction of step (b) is carried out in an aqueous solution comprising a mixture of water and an organic solvent. Any suitable organic solvent described above can be used. More specifically, the organic solvent is DMA. In some embodiments, the aqueous solution comprises less than 50%, less than 40%, less than 30%, less than 25%, less than 20%, less than 15%, less than 10%, less than 5%, less than 3%, less than 2%, or less than 1% of the organic solvent (*e.g.,* DMA) by volume.

In some embodiments, for the methods disclosed herein or in the 1st, 2nd, 3rd, 4th, 5th, 6th, 7th or 8th aspect, the bisulfite salt is sodium or potassium bisulfite and the metabisulfite salt is sodium or potassium metabisulfite. In a specific embodiment, the bisulfite salt is sodium bisulfite and the metabisulfite salt is sodium metabisulfite.

In some embodiments, for the methods disclosed herein or in the 1st, 2nd, 3rd, 4th, 5th, 6th, 7th or 8th aspect, the modified cytotoxic agent is not purified before reacting with the cell-binding agent in step (b). Alternatively, the modified cytotoxic agent is purified before reacting with the cell-binding agent in step (b). Any suitable methods disclosed herein can be used to purify the modified cytotoxic agent.

In some embodiments, for the methods described above, the reaction of step (a) results in no substantial sulfonation of the maleimide group. In some embodiments, less than 50%, 40%, 30%, 20%, 10%, 9%, 8%, 7%, 6%, 5%, 4%, 3%, 2%, or 1% of the maleimide group is sulfonated. The percentage of maleimide sulfonation is equal to the total amount of the maleimide-sulfonated cytotoxic agent (the cytotoxic agent having sulfonation on the maleimide only) and the di-sulfonated cytotoxic agent (the cytotoxic agent having sulfonation on both the maleimide and the imine moieties) divided by the starting amount of the imine-containing cytotoxic agent before its reaction with the bisulfite salt or the metabisulfite salt.

In some embodiments, the immunoconjugates prepared by any methods described above is subject to a

purification step. In this regard, the immunoconjugate can be purified from the other components of the mixture using tangential flow filtration (TFF), non-adsorptive chromatography, adsorptive chromatography, adsorptive filtration, selective precipitation, or any other suitable purification process, as well as combinations thereof.

In some embodiments, the immunoconjugate is purified using a single purification step *(e.g.,* TFF). Preferably, the conjugate is purified and exchanged into the appropriate formulation using a single purification step (*e.g.,* TFF). In other embodiments, the immunoconjugate is purified using two sequential purification steps. For example, the immunoconjugate can be first purified by selective precipitation, adsorptive filtration, absorptive chromatography or non-absorptive chromatography, followed by purification with TFF. One of ordinary skill in the art will appreciate that purification of the immunoconjugate enables the isolation of a stable conjugate comprising the cell-binding agent chemically coupled to the cytotoxic agent.

Any suitable TFF systems may be utilized for purification, including a Pellicon type system (Millipore, Billerica, Mass.), a Sartocon Cassette system (Sartorius AG, Edgewood, N.Y.), and a Centrasette type system (Pall Corp., East Hills, N.Y.)

Any suitable adsorptive chromatography resin may be utilized for purification. Preferred adsorptive chromatography resins include hydroxyapatite chromatography, hydrophobic charge induction chromatography (HCIC), hydrophobic interaction chromatography (HIC), ion exchange chromatography, mixed mode ion exchange chromatography, immobilized metal affinity chromatography (IMAC), dye ligand chromatography, affinity chromatography, reversed phase chromatography, and combinations thereof. Examples of suitable hydroxyapatite resins include ceramic hydroxyapatite (CHT Type I and Type II, Bio-Rad Laboratories, Hercules, Calif.), HA Ultrogel hydroxyapatite (Pall Corp., East Hills, N.Y.), and ceramic fluoroapatite (CFT Type I and Type II, Bio-Rad Laboratories, Hercules, Calif.). An example of a suitable HCIC resin is MEP Hypercel resin (Pall Corp., East Hills, N.Y.). Examples of suitable HIC resins include Butyl-Sepharose, Hexyl-Sepharose, Phenyl-Sepharose, and Octyl Sepharose resins (all from GE Healthcare, Piscataway, N.J.), as well as Macro-prep Methyl and Macro-Prep t Butyl resins (Biorad Laboratories, Hercules, Calif.). Examples of suitable ion exchange resins include SP-Sepharose, CM-Sepharose, and Q-Sepharose resins (all from GE Healthcare, Piscataway, N.J.), and Unosphere S resin (Bio-Rad Laboratories, Hercules, Calif.). Examples of suitable mixed mode ion exchangers include Bakerbond CBAx resin (JT Baker, Phillipsburg N.J.) Examples of suitable IMAC resins include Chelating Sepharose resin (GE Healthcare, Piscataway, N.J.) and Profinity IMAC resin (Bio-Rad Laboratories, Hercules, Calif.). Examples of suitable dye ligand resins include Blue Sepharose resin (GE Healthcare, Piscataway, N.J.) and Affi-gel Blue resin (Bio-Rad Laboratories, Hercules, Calif.). Examples of suitable affinity resins include Protein A Sepharose resin (*e.g.,* MabSelect, GE Healthcare, Piscataway, N.J.), where the cell-binding agent is an antibody, and lectin affinity resins, *e.g.,* Lentil Lectin Sepharose resin (GE Healthcare, Piscataway, N.J.), where the cell-binding agent bears appropriate lectin binding sites. Alternatively an antibody specific to the cell-binding agent may be used. Such an antibody can be immobilized to, for instance, Sepharose 4 Fast Flow resin (GE Healthcare, Piscataway, N.J.). Examples of suitable reversed phase resins include C4, C8, and C18 resins (Grace Vydac, Hesperia, Calif.).

Any suitable non-adsorptive chromatography resin may be utilized for purification. Examples of suitable non-adsorptive chromatography resins include, but are not limited to, SEPHADEX™ G-25, G-50, G-100, SEPHACRYL™ resins (*e.g.,* S-200 and S-300), SUPERDEX™ resins (*e.g.,* SUPERDEX™ 75 and SUPERDEX™200), BIO-GEL® resins (*e.g.,* P-6, P-10, P-30, P-60, and P-100), and others known to those in the art.

The immunoconjugates comprising an EpBA covalently linked to a maytansinoid compound described in the 3rd embodiment herein can be prepared according to any suitable methods known in the art.

In certain embodiments, the immunoconjugates can be prepared by a first method comprising the steps of reacting the EpBA with the maytansinoid compound of formula (II):

$$L_2'\text{-A-NH-CR}^1\text{R}^2\text{-S-L}_1\text{-D} \qquad \text{(II).}$$

In certain embodiments, the immunoconjugates can be prepared by a second method comprising the steps of:

(a) reacting the maytansinoid compound of formula (III) or (IV) with a linker compound described herein to form a cytotoxic agent-maytansinoid compound having an amine-reactive group or a thiol-reactive group bound thereto that can be covalently linked to the EpBA, wherein formulas (III) and (IV) are represented by:

$$\text{A'-NH-CR}^1\text{R}^2\text{-S-L}_1\text{-D} \qquad \text{(III)}$$

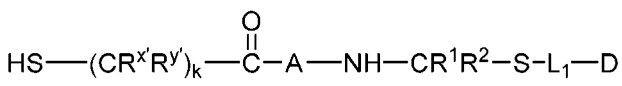
(IV);

and

(b) reacting the EpBA with the maytansinoid-linker compound to form the immunoconjugate.

In certain embodiments, the immunoconjugates can be prepared by a third method comprising the steps of:

(a) reacting the EpBA with a linker compound described herein to form a modified anti-EpBA having an amine-reactive group or a thiol-reactive group bound thereto (*e.g.,* compound of formula (II)) that can be covalently linked to the maytansinoid compound of formula (III) or (IV); and
(b) reacting the modified EpBA with the maytansinoid compound of formula (III) or (IV) to form the immunoconjugate.

In certain embodiments, the immunoconjugates can be prepared by a third method comprising reacting an EpBA, a linker compound and a maytansinoid compound of formula (III) or (IV) to form the immunoconjugates. In one embodiment, the EpBA and the maytansinoid compound of formula (III) or (IV) are mixed first, followed by the addition of the linker compound.
In certain embodiments, for the second, third or fourth methods described above, the linker compound is represented by any one of the formula (a1L) - (a10L):

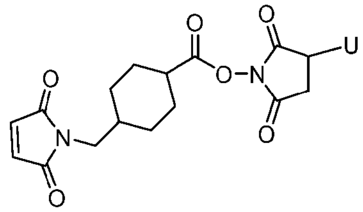 (a1L); 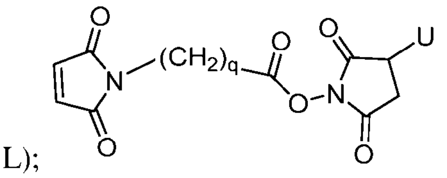 (a2L); 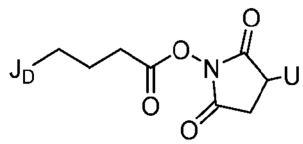 (a3L);

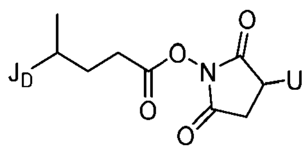 (a4L); 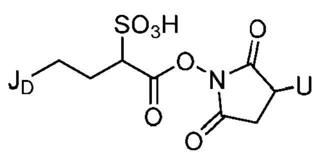 (a5L),

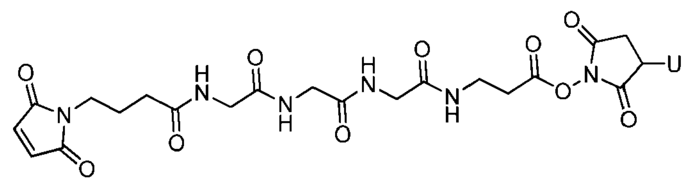 (a6L),

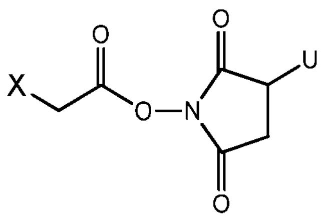 (a7L); 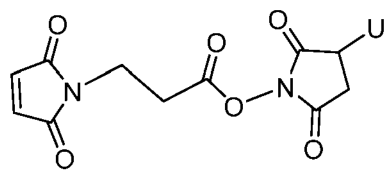 (a8L);

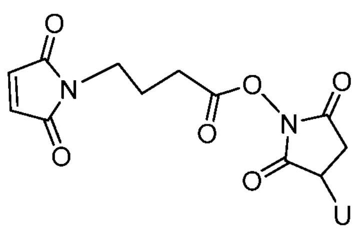 (a9L); and 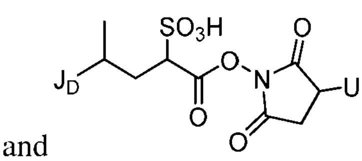 (a10L),

wherein X is halogen; $J_D$-SH, or -SSR$^d$; R$^d$ is phenyl, nitrophenyl, dinitrophenyl, carboxynitrophenyl, pyridyl or nitropyridyl; R$^g$ is an alkyl; and U is -H or $SO_3H$ or a pharmaceutically acceptable salt thereof.

In one embodiment, the linker compound is GMBS or sulfo-GMBS represented by represented by formula (a9L), wherein U is -H or $SO_3H$ or a pharmaceutically acceptable salt thereof.

In a specific embodiment, the immunoconjugate of the present invention is represented by the following formula:

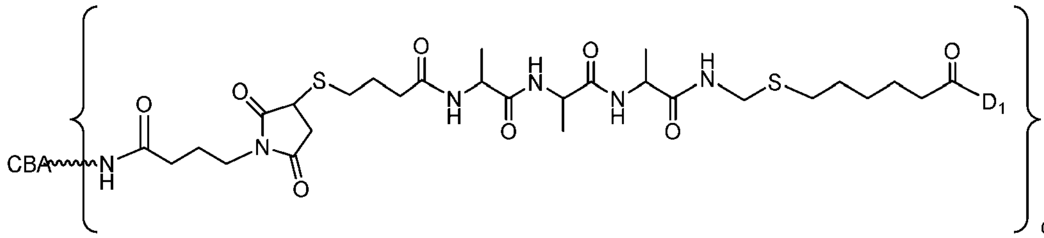

q (I-1);

and the immunoconjugate can be prepared by the second, third or fourth method described above, wherein the linker compound is GMBS or sulfo-GMBS represented by represented by formula (a9L), wherein U is -H or $SO_3H$ or a pharmaceutically acceptable salt thereof; and the maytansinoid compound is represented by formula (D-1):

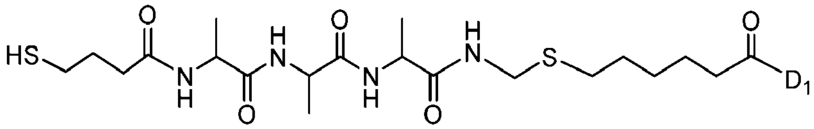

(D-1),

wherein $D_1$ is represented by the following formula:

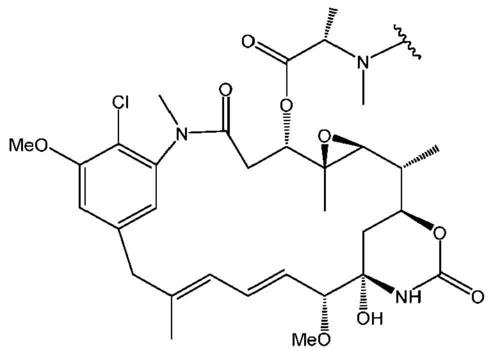

.

In a more specific embodiment, the immunoconjugate of formula (I-1) is prepared by reacting the maytansinoid compound of formula (D-1) with the linker compound GMBS or sulfo-GMBS to form a maytansinoid-linker compound, followed by reacting the EpBA with the maytansinoid-linker compound. In an even more specific embodiment, the maytansinoid linker compound is not purified before reacting with the EpBA.

In another specific embodiment, the immunoconjugate is represented by the following formula:

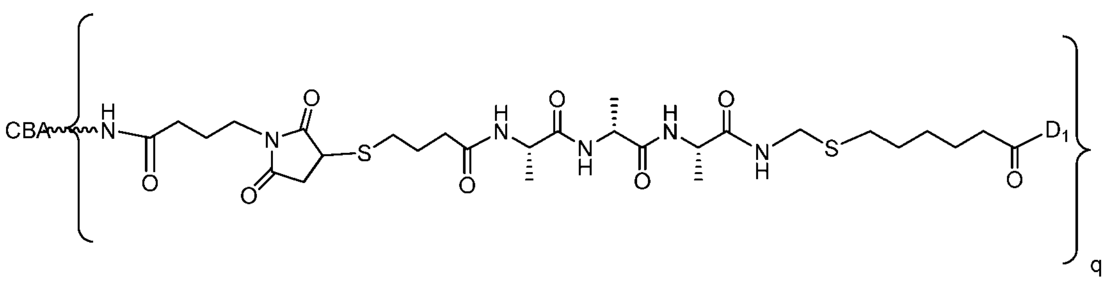

q (I-2);

and the immunoconjugate can be prepared by the second, third or fourth method described above, wherein the linker compound is GMBS or sulfo-GMBS represented by represented by formula (a9L), wherein U is -H or $SO_3H$ or a pharmaceutically acceptable salt thereof; and the maytansinoid compound is represented by formula (D-2) described

above. In a more specific embodiment, the immunoconjugate of formula (I-2) is prepared by reacting the maytansinoid compound of formula (D-2) with the linker compound GMBS or sulfo-GMBS to form a maytansinoid-linker compound, followed by reacting the EpBA with the maytansinoid-linker compound. In a even more specific embodiment, the maytansinoid linker compound is not purified before reacting with the EpBA.

In another specific embodiment, the immunoconjugate is represented by the following formula:

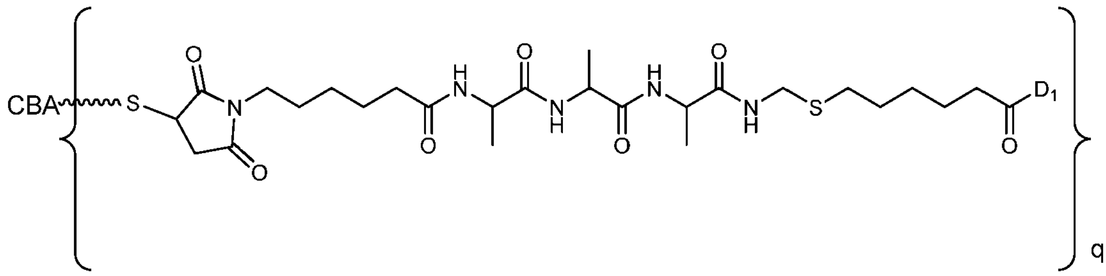

(I-3);

and the immunoconjugate is prepared according to the first method described above by reacting the EpBA with the maytansinoid compound of formula (D-3):

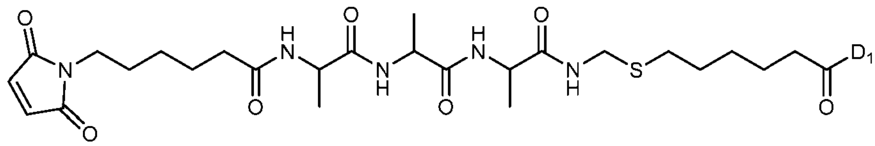

(D-3).

In another specific embodiment, the immunoconjugate is represented by the following formula:

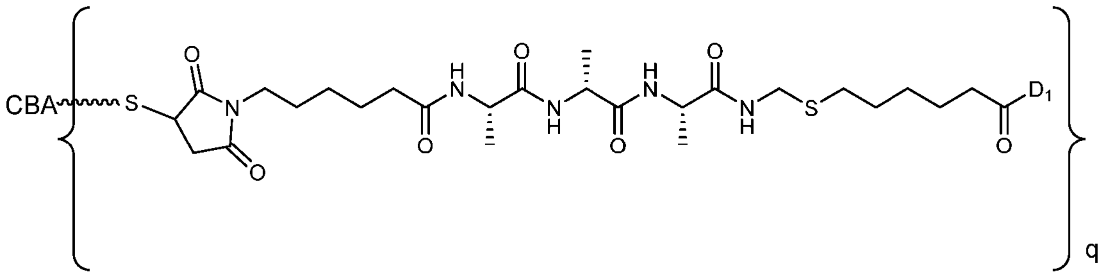

(I-4);

and the immunoconjugate is prepared according to the first method described above by reacting the EpBA with the maytansinoid compound of formula (D-4):

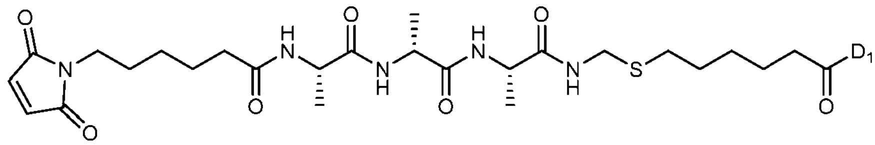

(D-4).

In another specific embodiment, the immunoconjugate is represented by the following formula:

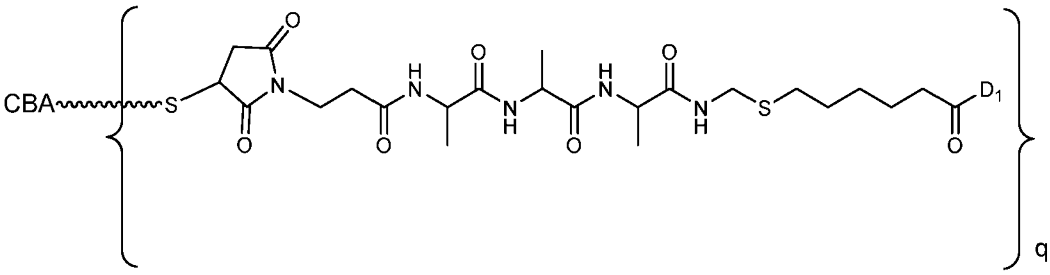

(I-5);

and the immunoconjugate is prepared according to the first method described above by reacting the EpBA with the maytansinoid compound of formula (D-5):

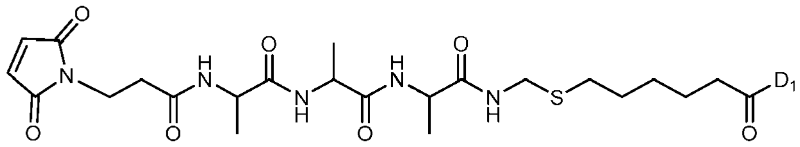

(D-5).

In another specific embodiment, the immunoconjugate is represented by the following formula:

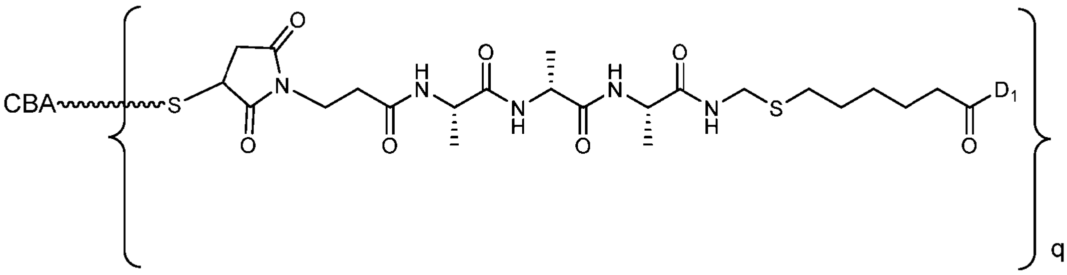

(I-6);

and the immunoconjugate is prepared according to the first method described above by reacting the EpBA with the maytansinoid compound of formula (D-6):

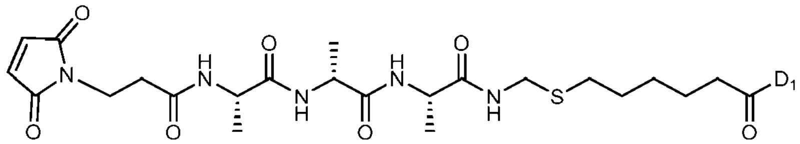

(D-6).

In some embodiments, the immunoconjugates prepared by any methods described above is subject to a purification step. In this regard, the immunoconjugate can be purified from the other components of the mixture using tangential flow filtration (TFF), non-adsorptive chromatography, adsorptive chromatography, adsorptive filtration, selective precipitation, or any other suitable purification process, as well as combinations thereof.

In some embodiments, the immunoconjugate is purified using a single purification step (e.g., TFF). Preferably, the conjugate is purified and exchanged into the appropriate formulation using a single purification step (e.g., TFF). In other embodiments of the invention, the immunoconjugate is purified using two sequential purification steps. For example, the immunoconjugate can be first purified by selective precipitation, adsorptive filtration, absorptive chromatography or non-absorptive chromatography, followed by purification with TFF. One of ordinary skill in the art will appreciate that purification of the immunoconjugate enables the isolation of a stable conjugate comprising the cell-binding agent chemically coupled to the cytotoxic agent.

Any suitable TFF systems may be utilized for purification, including a Pellicon type system (Millipore, Billerica, Mass.), a Sartocon Cassette system (Sartorius AG, Edgewood, N.Y.), and a Centrasette type system (Pall Corp., East Hills, N.Y.)

Any suitable adsorptive chromatography resin may be utilized for purification. Preferred adsorptive chromatography resins include hydroxyapatite chromatography, hydrophobic charge induction chromatography (HCIC), hydrophobic interaction chromatography (HIC), ion exchange chromatography, mixed mode ion exchange chromatography, immobilized metal affinity chromatography (IMAC), dye ligand chromatography, affinity chromatography, reversed phase chromatography, and combinations thereof. Examples of suitable hydroxyapatite resins include ceramic hydroxyapatite (CHT Type I and Type II, Bio-Rad Laboratories, Hercules, Calif.), HA Ultrogel hydroxyapatite (Pall Corp., East Hills, N.Y.), and ceramic fluoroapatite (CFT Type I and Type II, Bio-Rad Laboratories, Hercules, Calif.). An example of a suitable HCIC resin is MEP Hypercel resin (Pall Corp., East Hills, N.Y.). Examples of suitable HIC resins include Butyl-Sepharose, Hexyl-Sepharose, Phenyl-Sepharose, and Octyl Sepharose resins (all from GE Healthcare, Piscataway, N.J.), as well as Macro-prep Methyl and Macro-Prep t-Butyl resins (Biorad Laboratories, Hercules, Calif.). Examples of suitable ion exchange resins include SP-Sepharose, CM-Sepharose, and Q-Sepharose resins (all from GE Healthcare, Piscataway, N.J.), and Unosphere S resin (Bio-Rad Laboratories, Hercules, Calif.). Examples of suitable mixed mode ion exchangers include Bakerbond ABx resin (JT Baker, Phillipsburg N.J.) Examples of suitable IMAC resins include Chelating Sepharose resin (GE Healthcare, Piscataway, N.J.) and Profinity IMAC resin (Bio-Rad Laboratories, Hercules, Calif.). Examples of suitable

dye ligand resins include Blue Sepharose resin (GE Healthcare, Piscataway, N.J.) and Affi-gel Blue resin (Bio-Rad Laboratories, Hercules, Calif.). Examples of suitable affinity resins include Protein A Sepharose resin (e.g., MabSelect, GE Healthcare, Piscataway, N.J.), where the cell-binding agent is an antibody, and lectin affinity resins, e.g. Lentil Lectin Sepharose resin (GE Healthcare, Piscataway, N.J.), where the cell-binding agent bears appropriate lectin binding sites. Alternatively an antibody specific to the cell-binding agent may be used. Such an antibody can be immobilized to, for instance, Sepharose 4 Fast Flow resin (GE Healthcare, Piscataway, N.J.). Examples of suitable reversed phase resins include C4, C8, and C18 resins (Grace Vydac, Hesperia, Calif.).

Any suitable non-adsorptive chromatography resin may be utilized for purification. Examples of suitable non-adsorptive chromatography resins include, but are not limited to, SEPHADEXTM G-25, G-50, G-100, SEPHACRYLTM resins (e.g., S-200 and S-300), SUPERDEXTM resins (e.g., SUPERDEXTM 75 and SUPERDEXTM 200), BIO-GEL® resins (e.g., P-6, P-10, P-30, P-60, and P-100), and others known to those of ordinary skill in the art.

**Diagnostic and Research Applications**

In addition to the therapeutic uses of the antibodies discussed herein, the antibodies fragments, and activatable antibodies provided herein can be employed in many known diagnostic and research applications. The provided EpCAM antibodies and/or EpCAM-binding antibody fragments may be used, for example, in the purification, detection, and targeting of EpCAM, included in both *in vitro* and *in vivo* diagnostic methods. For example, the antibodies and/or fragments may be used in immunoassays for qualitatively and quantitatively measuring levels of EpCAM (*e.g.,* human EpCAM or cynomolgous EpCAM) expressed by cells in biological samples. *See, e.g.,* Harlow et al., Antibodies: A Laboratory Manual (Cold Spring Harbor Laboratory Press, 2nd ed. 1988), the entire contents of which is herein incorporated by reference.

The provided EpCAM Antibodies and/or EpCAM-binding antibody fragments may be used in, for example, competitive binding assays, direct and indirect sandwich assays, and immunoprecipitation assays (Zola, Monoclonal Antibodies: A Manual of Techniques, pp.147-158 (CRC Press, Inc., 1987)).

Detectably labeling an EpCAM antibody, EpCAM-binding antibody fragment, and/or EpCAM activatable antibody can be accomplished by linkage to an enzyme for use in an enzyme immunoassay (EIA), or enzyme-linked immunosorbent assay (ELISA). The linked enzyme reacts with the exposed substrate to generate a chemical moiety which can be detected, for example, by spectrophotometric, fluorometric or by visual means. Enzymes which can be used to detectably label for example the disclosed EpCAM antibodies, EpCAM-binding antibody fragments, and EpCAM activatable antibodies, include, but are not limited to, malate dehydrogenase, staphylococcal nuclease, delta-5-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase.

By radioactively labeling the EpCAM antibodies, EpCAM-binding antibody fragments, and EpCAM activatable antibodies, it is possible to detect EpCAM through the use of a radioimmunoassay (RIA) (*see, e.g.,* Work, et al., Laboratory Techniques and Biochemistry in Molecular Biology, North Holland Publishing Company, N.Y. (1978)). The radioactive isotope can be detected by such means as the use of a gamma counter or a scintillation counter or by autoradiography. Isotopes which are particularly useful for the purpose of the present disclosure are: $^{3}H$, $^{125}I$, $^{131}I$, $^{35}S$, $^{14}C$, and, preferably, $^{125}I$.

It is also possible to label the EpCAM antibodies, EpCAM-binding antibody fragments, and EpCAM activatable antibodies with a fluorescent compound. When the fluorescent labeled antibody, antibody fragment, or activatable antibody, is exposed to light of the proper wave length, its presence can then be detected due to fluorescence. Among the most commonly used fluorescent labelling compounds are fluorescein isothiocyanate, rhodamine, phycoerythrin, phycocyanin, allophycocyanin, o-phthaldehyde and fluorescamine.

The EpCAM antibodies, EpCAM-binding antibody fragments, and EpCAM activatable antibodies, can also be detectably labeled using fluorescence-emitting metals such as 125Eu, or others of the lanthanide series. These metals can be attached to the EpCAM antibodies, EpCAM-binding antibody fragments, and EpCAM activatable antibodies using such metal chelating groups as diethylenetriaminepentaacetic acid (DTPA) or ethylenediamine-tetraacetic acid (EDTA).

In additional embodiments, the EpCAM antibodies, EpCAM-binding antibody fragments, and EpCAM activatable antibodies are detectably labeled by coupling to a chemiluminescent compound. The presence of the chemiluminescently labeled antibody, or antibody fragment is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester. Likewise, a bioluminescent compound can be used to label the EpCAM antibodies, EpCAM-binding antibody fragments, EpCAM activatable antibodies, or derivatives thereof. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

EpCAM antibodies EpCAM-binding antibody fragments, and EpCAM activatable antibodies are useful for *in vivo* imaging, wherein an a EpCAM antibody, EpCAM-binding antibody fragment, or EpCAM activatable antibody, labeled with a detectable moiety such as a radio-opaque agent or radioisotope is administered to a subject, preferably into the bloodstream, and the presence and location of the labeled antibody or antibody fragment in the host is assayed. This imaging technique is useful in the staging and treatment of malignancies. The EpCAM antibody, EpCAM-binding antibody fragment, or EpCAM activatable antibody may be labeled with any moiety that is detectable in a host, whether by nuclear magnetic resonance, radiology, or other detection means known in the art.

The label used according to the disclosed methods can be any detectable moiety that is capable of producing, either directly or indirectly, a detectable signal. For example, the label may be a biotin label, an enzyme label (*e.g.,* luciferase, alkaline phosphatase, beta-galactosidase and horseradish peroxidase), a radio-label (*e.g.,* $^{3}H$, $^{14}C$, $^{32}P$, $^{35}S$, and $^{125}I$), a fluorophore such as fluorescent or chemiluminescent compound (*e.g.,* fluorescein isothiocyanate, rhodamine), an imaging agent (*e.g.,* Tc-$m^{99}$ and indium ($^{111}In$)) and a metal ion (*e.g.,* gallium and europium).

Any method known in the art for conjugating the EpCAM antibody, EpCAM-binding antibody fragment, or EpCAM activatable antibody, to the label may be employed, including those exemplary methods described by Hunter et al., Nature 144:945 (1962); David et al., Biochemistry 13:1014 (1974); Pain et al., J. Immunol. Meth. 40:219 (1981); Nygren, Histochem. and Cytochem. 30:407 (1982).

**Therapeutic Applications**

Also included are methods for inhibiting the growth of cells expressing EpCAM (*e.g.,* human EpCAM or cynomolgous EpCAM). As provided herein, the disclosed EpCAM antibodies, EpCAM-binding antibody fragments, EpCAM activatable antibodies, and and/or conjugates thereof, have the ability to bind EpCAM present on the surface of a cell (*e.g.,* a human cell or a cynomolgous cell) and mediate cell killing. In particular embodiments, the immunoconjugates comprise a cytotoxic payload, *e.g.,* a indolinobenzodiazepine DNA-alkylating agent, are internalized and mediate cell killing *via* the activity of the cytotoxic payload *e.g.,* a benzodiazepine, *e.g.,* an indolinobenzodiazepine DNA-alkylating agent. Such cell killing activity may be augmented by the immunoconjugate inducing antibody-dependent cell-mediated cytotoxicity (ADCC) and/or complement dependent cytotoxicity (CDC).

As used herein the terms "inhibit" and "inhibiting" include any inhibitory effect on cell growth, including cell death. The inhibitory effects include temporary effects, sustained effects and permanent effects.

The therapeutic applications provided herein include methods of treating a subject having a disease. The diseases treated with the provided methods are those characterized by the expression (*e.g.,* EpCAM overexpression). Such diseases include for example, breast cancer, lung cancer, stomach cancer, prostate cancer, ovarian cancer, colorectal cancer, colon cancer, esophageal cancer, tracheal cancer, gastric cancer, bladder cancer, uterine cancer, rectal cancer, or cancer of the small intestine, pancreatic cancer, or other epithelial cancer, or metastases associated therewith. The skilled artisan will understand that the methods of the present disclosure may also be used to treat other diseases yet to be described but characterized by the expression of EpCAM.

In other particular embodiments, the disclosed EpCAM antibodies, EpCAM-binding antibody fragments, EpCAM activatable antibodies, and and/or conjugates thereof, are useful in the treatment cancers expressing EpCAM. In some embodiments, the cancer is an epithelial or squamous cancer. In some embodiments, the cancer is breast cancer, lung cancer, stomach cancer, prostate cancer, ovarian cancer, colorectal cancer, colon cancer, esophageal cancer, tracheal cancer, gastric cancer, bladder cancer, uterine cancer, rectal cancer, pancreatic cancer, or cancer of the small intestine.

The therapeutic applications provided herein can also be practiced *in vitro* and *ex vivo.*

The disclosure also provides therapeutic applications of the disclosed EpCAM antibodies, EpCAM-binding antibody fragments, EpCAM activatable antibodies, and and/or conjugates thereof, wherein the antibodies, antibody fragments, activatable antibodies, or conjugates are administered to a subject, in a pharmaceutically acceptable dosage form. They can be administered intravenously as a bolus or by continuous infusion over a period of time, by intramuscular, subcutaneous, parenteral, intra-articular, intrasynovial, intrathecal, oral, topical, or inhalation routes. They may also be administered by intratumoral, peritumoral, intralesional, or perilesional routes, to exert local as well as systemic therapeutic effects.

Also provided are methods of treating, preventing and/or delaying the onset or progression of, or alleviating a symptom associated with aberrant expression and/or activity of EpCAM in a subject using EpCAM activatable antibodies that bind EpCAM, particularly EpCAM activatable antibodies that bind and neutralize or otherwise inhibit at least one biological activity of EpCAM and/or EpCAM-mediated signaling.

In some embodiments, the disclosure provides methods of treating, preventing and/or delaying the onset or progression of, or alleviating a symptom associated with the presence, growth, proliferation, metastasis, and/or activity of cells which are expressing EpCAM or aberrantly expressing EpCAM in a subject using EpCAM activatable antibodies that bind EpCAM, particularly EpCAM activatable antibodies that bind, target, neutralize, kill, or otherwise inhibit at least one biological activity of cells which are expressing or aberrantly expressing EpCAM. The disclosure also provides methods of

treating, preventing and/or delaying the onset or progression of, or alleviating a symptom associated with the presence, growth, proliferation, metastasis, and/or activity of cells which are expressing EpCAM in a subject using EpCAM activatable antibodies that bind EpCAM, particularly EpCAM activatable antibodies that bind, target, neutralize, kill, or otherwise inhibit at least one biological activity of cells which are expressing EpCAM.

The disclosure also provides methods of treating, preventing and/or delaying the onset or progression of, or alleviating a symptom associated with the presence, growth, proliferation, metastasis, and/or activity of cells which are aberrantly expressing EpCAM in a subject using EpCAM activatable antibodies that bind EpCAM, particularly EpCAM activatable antibodies that bind, target, neutralize, kill, or otherwise inhibit at least one biological activity of cells which are aberrantly expressing EpCAM.

The disclosure provides methods of preventing, delaying the progression of, treating, alleviating a symptom of, or otherwise ameliorating an EpCAM mediated disease in a subject by administering a therapeutically effective amount of an EpCAM antibody, conjugated EpCAM antibody, EpCAM activatable antibody, and/or conjugated EpCAM activatable antibody disclosed herein to a subject in need thereof.

The disclosure also provides methods of preventing, delaying the progression of, treating, alleviating a symptom of, or otherwise ameliorating cancer (*e.g.,* epithelial cancer and metastases thereof) in a subject by administering a therapeutically effective amount of an EpCAM antibody, conjugated EpCAM antibody, EpCAM activatable antibody, and/or conjugated EpCAM activatable antibody disclosed herein to a subject in need thereof. EpCAM is known to be expressed in a variety of cancers, including most cancers (and metastases) of epithelial origin.

In some embodiments, the cancer is an epithelial or squamous cancer.

In some embodiments, the cancer is breast cancer, lung cancer, stomach cancer, prostate cancer, ovarian cancer, colorectal cancer, colon cancer, esophageal cancer, tracheal cancer, gastric cancer, bladder cancer, uterine cancer, rectal cancer, pancreatic cancer, or cancer of the small intestine.

In some embodiments, the cancer is breast cancer, lung cancer, stomach cancer, colorectal cancer, colon cancer, rectal cancer, cancer of the small intestine, ovarian cancer, gastric cancer, or esophageal cancer.

In some embodiments, the cancer is ovarian cancer, uterine cancer, gastric cancers, pancreatic cancer, or colorectal cancer.

In some embodiments, the cancer is ovarian cancer.

In some embodiments, the cancer is uterine cancer.

In some embodiments, the cancer is gastric cancer.

In some embodiments, the cancer is pancreatic cancer.

In some embodiments, the cancer is colorectal cancer.

In some embodiments, the cancer is breast cancer. In certain embodiments, the cancer is triple negative breast cancer.

In some embodiments, the cancer is lung cancer. In some embodiments, the lung cancer is non-small cell lung cancer. In some embodiments, the non-small cell lung cancer is non-squamous non-small cell lung cancer.

An EpCAM antibody, a conjugated EpCAM antibody, an EpCAM activatable antibody, and/or a conjugated EpCAM activatable antibody, used in any of the embodiments, of these methods and uses can be administered at any stage of the disease. For example, such an EpCAM antibody, conjugated EpCAM antibody, EpCAM activatable antibody, and/or conjugated EpCAM activatable antibody, can be administered to a patient suffering cancer of any stage, from early to metastatic. The terms subject and patient are used interchangeably herein.

In some embodiments, the subject is a mammal, such as a human, non- human primate, companion animal (*e.g.,* cat, dog, horse), farm animal, work animal, or zoo animal. In some embodiments, the subject is a human. In some embodiments, the subject is a companion animal. In some embodiments, the subject is an animal in the care of a veterinarian.

The EpCAM antibody, conjugated EpCAM antibody, EpCAM activatable antibody, and/or conjugated EpCAM activatable antibody, and therapeutic formulations thereof are administered to a subject suffering from or susceptible to a disease or disorder associated with aberrant EpCAM expression and/or activity, such as cancer. A subject suffering from or susceptible to a disease or disorder associated with aberrant EpCAM expression and/or activity is identified using any of a variety of methods known in the art. For example, subjects suffering from cancer or other neoplastic condition are identified using any of a variety of clinical and/or laboratory tests such as, physical examination and blood, urine and/or stool analysis to evaluate health status. For example, subjects suffering from inflammation and/or an inflammatory disorder are identified using any of a variety of clinical and/or laboratory tests such as physical examination and/or bodily fluid analysis, *e.g.*, blood, urine and/or stool analysis, to evaluate health status.

Administration of an EpCAM antibody, conjugated EpCAM antibody, EpCAM activatable antibody, and/or conjugated EpCAM activatable antibody, to a patient suffering from a disease or disorder associated with aberrant EpCAM expression and/or activity (*e.g.,* a cancer such as a carcinoma) is considered successful if any of a variety of laboratory or clinical objectives is achieved. For example, administration of an EpCAM antibody, conjugated EpCAM antibody, EpCAM activatable antibody, and/or conjugated EpCAM activatable antibody, to a patient suffering from a

disease or disorder associated with aberrant EpCAM expression and/or activity is considered successful if one or more of the symptoms associated with the disease or disorder is alleviated, reduced, inhibited or does not progress to a further, *i.e.,* worse, state. Administration of an EpCAM antibody, conjugated anti- EpCAM antibody, EpCAM activatable antibody, and/or conjugated EpCAM activatable antibody, to a patient suffering from a disease or disorder associated with aberrant EpCAM expression and/or activity is considered successful if the disease or disorder enters remission or does not progress to a further, *i.e.*, worse, state.

In some embodiments, the EpCAM antibody, conjugated EpCAM antibody, EpCAM activatable antibody, and/or conjugated EpCAM activatable antibody, and therapeutic formulations thereof are administered to a subject suffering from or susceptible to a disease or disorder, such as subjects suffering from cancer or other neoplastic condition, wherein the subject's diseased cells are expressing EpCAM. In some embodiments, the diseased cells are associated with aberrant EpCAM expression and/or activity. In some embodiments, the diseased cells are associated with normal EpCAM expression and/or activity. A subject suffering from or susceptible to a disease or disorder wherein the subject's diseased cells express EpCAM is identified using any of a variety of methods known in the art. For example, subjects suffering from cancer or other neoplastic condition are identified using any of a variety of clinical and/or laboratory tests such as, physical examination and blood, urine and/or stool analysis to evaluate health status. For example, subjects suffering from inflammation and/or an inflammatory disorder are identified using any of a variety of clinical and/or laboratory tests such as physical examination and/or bodily fluid analysis, *e.g.*, blood, urine and/or stool analysis, to evaluate health status.

In some embodiments, the EpCAM antibody, conjugated EpCAM antibody, EpCAM activatable antibody, and/or conjugated EpCAM activatable antibody, and therapeutic formulations thereof are administered to a subject suffering from or susceptible to a disease or disorder associated with cells expressing EpCAM or the presence, growth, proliferation, metastasis, and/or activity of such cells, such as subjects suffering from cancer or other neoplastic conditions. In some embodiments, the cells are associated with aberrant EpCAM expression and/or activity. In some embodiments, the cells are associated with normal EpCAM expression and/or activity. A subject suffering from or susceptible to a disease or disorder associated with cells that express EpCAM is identified using any of a variety of methods known in the art. For example, subjects suffering from cancer or other neoplastic condition are identified using any of a variety of clinical and/or laboratory tests such as, physical examination and blood, urine and/or stool analysis to evaluate health status. For example, subjects suffering from inflammation and/or an inflammatory disorder are identified using any of a variety of clinical and/or laboratory tests such as physical examination and/or bodily fluid analysis, *e.g.*, blood, urine and/or stool analysis, to evaluate health status.

Administration of an EpCAM antibody, conjugated EpCAM antibody, EpCAM activatable antibody, and/or conjugated EpCAM activatable antibody, to a patient suffering from a disease or disorder associated with cells expressing EpCAM *(e.g.,* a cancer such as a carcinoma) is considered successful if any of a variety of laboratory or clinical objectives is achieved. For example, administration of an EpCAM antibody, conjugated EpCAM antibody, EpCAM activatable antibody, and/or conjugated EpCAM activatable antibody, to a patient suffering from a disease or disorder associated with cells expressing EpCAM is considered successful if one or more of the symptoms associated with the disease or disorder is alleviated, reduced, inhibited or does not progress to a further, *i.e.,* worse, state. Administration of an EpCAM antibody, conjugated EpCAM antibody, EpCAM activatable antibody, and/or conjugated EpCAM activatable antibody, to a patient suffering from a disease or disorder associated with cells expressing EpCAM is considered successful if the disease or disorder enters remission or does not progress to a further, *i.e.,* worse, state.

The disclosure provides antibodies and antibody fragments that specifically bind human EpCAM, EpCAM activatable antibodies, and conjugated EpCAM antibodies, antibody fragments, or activatable antibodies that are useful in methods of treating, preventing, delaying the progression of, ameliorating and/or alleviating a symptom of a disease or disorder associated with aberrant EpCAM expression and/or activity. For example, the EpCAM activatable antibodies are used in methods of treating, preventing, delaying the progression of, ameliorating and/or alleviating a symptom of a cancer or other neoplastic condition.

The disclosure provides antibodies and antibody fragments that specifically bind human EpCAM, EpCAM activatable antibodies, and conjugated EpCAM antibodies, antibody fragments, or activatable antibodies that are useful in methods of treating, preventing, delaying the progression of, ameliorating and/or alleviating a symptom of a disease or disorder associated with cells expressing EpCAM. In some embodiments, the cells are associated with aberrant EpCAM expression and/or activity. In some embodiments, the cells are associated with normal EpCAM expression and/or activity. For example, the EpCAM activatable antibodies can be used in methods of treating, preventing, delaying the progression of, ameliorating and/or alleviating a symptom of a cancer or other neoplastic condition.

The disclosure provides antibodies and antibody fragments that specifically bind human EpCAM, EpCAM activatable antibodies, conjugated EpCAM antibodies and antibody fragments, and/or conjugated EpCAM activatable antibodies, that are useful in methods of treating, preventing, delaying the progression of, ameliorating and/or alleviating a symptom of a disease or disorder in which diseased cells express EpCAM. In some embodiments, the diseased cells are associated with aberrant EpCAM expression and/or activity. In some embodiments, the diseased cells are associated with normal EpCAM expression and/or activity. For example, the EpCAM activatable antibodies are used in methods of

treating, preventing, delaying the progression of, ameliorating and/or alleviating a symptom of a cancer or other neoplastic condition.

**Pharmaceutical Compositions**

The provided compositions include bulk drug compositions useful in the manufacture of pharmaceutical compositions (*e.g.,* impure or non-sterile compositions) and pharmaceutical compositions (*i.e.,* compositions that are suitable for administration to a subject or patient) that can be used in the preparation of unit dosage forms. Such compositions comprise a prophylactically or therapeutically effective amount of the provided immunoconjugates and a pharmaceutically acceptable carrier.

Preferably, provided compositions comprise a prophylactically or therapeutically effective amount of a disclosed immunoconjugate and a pharmaceutically acceptable carrier.

In a specific embodiment, the term "pharmaceutically acceptable" means approved by a regulatory agency of the Federal or a state government or listed in the US Pharmacopeia or other generally recognized pharmacopeia for use in animals, and more particularly in humans. The term "carrier" refers to a diluent, adjuvant (*e.g.*, Freund's adjuvant (complete and incomplete), excipient, or vehicle with which the therapeutic is administered. Generally, the ingredients of the compositions provide herein are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The disclosure also provides a pharmaceutical pack or kit comprising one or more containers filled with an immunoconjugate provided herein, alone or with such pharmaceutically acceptable carrier. The disclosure also provides a pharmaceutical pack or kit comprising one or more containers filled with one or more of the ingredients of the pharmaceutical compositions provided herein. Optionally associated with such container(s) can be a notice in the form prescribed by a governmental agency regulating the manufacture, use or sale of pharmaceuticals or biological products, which notice reflects approval by the agency of manufacture, use or sale for human administration.

The disclosure provides kits that can be used in the above methods. A kit can comprise any of the immunoconjugates disclosed herein.

**Methods of Administration**

The disclosed compositions may be provided for the treatment, prophylaxis, and amelioration of one or more symptoms associated with a disease, disorder by administering to a subject a therapeutically effective amount an immunoconjugate provided herein. In a preferred aspect, such compositions are substantially purified (*i.e.,* substantially free from substances that limit its effect or produce undesired side effects). In a specific embodiment, the subject is an animal, preferably a mammal such as non-primate (*e.g.,* bovine, equine, feline, canine, rodent, *etc*.) or a primate (*e.g.,* monkey such as, a cynomolgus monkey, human, *etc*.). In a preferred embodiment, the subject is a human.

Methods of administering an immunoconjugate provided herein include, but are not limited to, parenteral administration (*e.g.,* intradermal, intramuscular, intraperitoneal, intravenous and subcutaneous), epidural, and mucosal (*e.g.,* intranasal and oral routes). In a specific embodiment, the immunoconjugates provided herein are administered intramuscularly, intravenously, or subcutaneously. The compositions may be administered by any convenient route, for example, by infusion or bolus injection, and may be administered together with other biologically active agents. Administration can be systemic or local.

The disclosure also provides that preparations of the disclosed immunoconjugates are packaged in a hermetically sealed container such as an ampoule or sachette indicating the quantity of the molecule. In one embodiment, such molecules are supplied as a dry sterilized lyophilized powder or water free concentrate in a hermetically sealed container and can be reconstituted, *e.g.*, with water or saline to the appropriate concentration for administration to a subject. Preferably, the immunoconjugates are supplied as a dry sterile lyophilized powder in a hermetically sealed container.

The lyophilized preparations of the immunoconjugates provided herein should be stored at between 2°C and 8°C in their original container and the molecules should be administered within 12 hours, preferably within 6 hours, within 5 hours, within 3 hours, or within 1 hour after being reconstituted. In an alternative embodiment, such molecules are supplied in liquid form in a hermetically sealed container indicating the quantity and concentration of the molecule, fusion protein, or conjugated molecule. Preferably, such immunoconjugates when provided in liquid form are supplied in a hermetically sealed container.

As used herein, an "therapeutically effective amount" of a pharmaceutical composition is an amount sufficient to effect beneficial or desired results including, without limitation, clinical results such as decreasing a symptom of cancer (*e.g.,* the proliferation, of cancer cells, tumor presence, tumor metastases, *etc*.), thereby increasing the quality of life of

those suffering from the disease, decreasing the dose of other medications required to treat the disease, enhancing the effect of another medication such as *via* targeting and/or internalization, delaying the progression of the disease, and/ or prolonging survival of individuals.

A therapeutically effective amount can be administered in one or more administrations. For purposes of this disclosure, a therapeutically effective amount of drug, compound, or pharmaceutical composition is an amount sufficient to reduce the proliferation of (or the effect of) viral presence and to reduce and /or delay the development of the viral disease, either directly or indirectly.

## Examples

### Example 1: Generation of Mouse Monoclonal Antibodies against Human and Cynomolgus EpCAM (CD326) Antigen

To generate monoclonal antibodies against human and cynomolgus (cyno) EpCAM, three different immunization protocols were used. In the first immunization protocol, wild type BALB/c female mice were injected subcutaneously three times with the cyno-EpCAM expressing 300-19 cell line, which is a BALB/c derived pre-B cell, and were then injected two times with the human-EpCAM expressing 300-19 cell line. In the second immunization protocol, wild type BALB/c female mice were injected subcutaneously four times with the NSCLC cell line H1568, and were then injected four times with cyno primary kidney epithelial cells. In the third immunization protocol, FcgammaR2b ko/ko BALB/c female mice (model # 579, Taconic) were injected subcutaneously with human-EpCAM expressing 300-19 cells three times, and were then injected with cyno-EpCAM expressing 300-19 cells two times. In all three protocols, cells were prepared in PBS and injected into mice at a dose of 5 X $10^6$ cells/mouse/injection with two weeks interval between injections. To boost the immune response, anti GITR Ab (clone DTA-1) was injected one week after the first immunization. Three days before being sacrificed for *Hybridoma* generation, the immunized mice received intraperitoneal injection of another dose of the human-EpCAM expressing 300-19 cells. Mouse spleens were collected according to standard animal protocols and were ground between two sterile, frosted microscopic slides to obtain a single cell suspension in RPMI-1640 medium. After the red blood cells were lysed with ACK lysing buffer, the spleen cells were then mixed with murine myeloma P363Ag8.653 cells (P3 cells) at the ratio of 1 P3 cell: 3 spleen cells. The mixture of spleen cells and P3 cells was washed and treated with pronase in fusion media (0.3 M mannitol/D-sorbitol, 0.1 mM CaCl2, 0.5 mM MgCl2 and 1 mg/mL BSA) at room temperature for 3 min. The reaction was stopped by addition of Fetal Bovine Serum (FBS), and cells were then washed, re-suspended in 2 mL cold fusion media and fused using a BTX ECM 2001 electrofusion machine. The fused cells were added gently to RPMI-1640 selection medium containing hypoxanthine-aminopterin-thymidine (HAT), incubated for 20 min at 37°C, and then seeded into ten flat bottom 96-well plates at 200 μL/well. The plates were then incubated in a 5% CO2 incubator at 37°C until hybridoma clones were ready for antibody screening. Other techniques of immunization and *Hybridoma* production can also be used, including those described in J. Langone and H. Vunakis (Eds., Methods in Enzymology, Vol. 121, Immunochemical Techniques, Part I, Academic Press, Florida); and E. Harlow and D. Lane (Antibodies: A Laboratory Manual, 1988, Cold Spring Harbor Laboratory Press, New York, NY).

#### *Hybridoma* Screening and Subcloning

*Hybridoma* screening was performed using a flow cytometry binding assay with human EpCAM expressing 300-19 cells and wild-type 300-19 cells. In brief, the wild-type 300-19 cells were first labeled with CELLTRACE™ far red DDAO-SE, mixed with untreated cells at 1:1 ratio and incubated with the *Hybridoma* supernatant for 2 hours on ice. Cells were then washed, incubated with PE-labeled anti mouse IgG, washed, fixed with formalin and analyzed using FACS array. The *Hybridomas* with specific reactivity to human-EpCAM antigen were expanded and the supernatants were rescreened by flow cytometric binding assay using three independent cell lines: human-EpCAM expressing 300-19 cells, cyno-EpCAM expressing 300-19 cells and wild type 300-19 cells. The *Hybridomas* with positive binding to human and cyno EpCAM antigens but negative on wild type 300-19 cells were further subcloned by limiting dilution. One subclone from each *Hybridoma,* which showed specific binding to human and cyno EpCAM antigens, was selected for subsequent analysis.

A total of 20 fusions were conducted over the course of this investigation. 63 *Hybridomas* specific for human and cynomolgus EpCAM antigens were generated and 29 *Hybridomas* were subcloned. Stable subclones were cultured and the isotype of the monoclonal antibody was identified using commercial mouse IgG isotyping reagents.

#### Murine Antibody Purification

The filtered supernatant from the *Hybridoma* subclones was purified using a scheme that essentially consists of two chromatography steps: protein A affinity and ceramic hydroxyapatite (CHT). Briefly, the filtered supernatant was

neutralized by the addition of 1:10 volume of 1 M Tris/HCl buffer (pH 8.0). The neutralized supernatant was loaded on a protein A column (HiTrap Protein A HP, 1 mL) which had been pre-equilibrated with 1 PBS (pH 7.3±0.1). The column was washed with 1X PBS (pH 7.3±0.1) to reduce non-specific host cell proteins. The bound antibody was then eluted using 25mM acetic acid containing 50mM sodium chloride (pH 3.2) and neutralized immediately with 1M Tris-base to a pH of 7.0 ±0.2. The neutralized pool was diluted 1:10 in CHT binding buffer (15mM sodium phosphate, pH 7.0±0.1) and loaded onto a Type II CHT column (40 μm particle size) pre-equilibrated with CHT binding buffer. The bound protein was then eluted using a linear gradient (15mM to 160mM sodium phosphate in 10 column volumes), and fractions of interest (high percent monomers by size exclusion chromatography, SEC) were pooled, dialyzed against 1X PBS (pH 7.3±0.1), and filter sterilized. The final antibody concentration was determined by measuring absorbance at 280 nm and an extinction coefficient of 1.44 mL $mg^{-1}$ $cm^{-1}$.

All purification experiments were conducted on an AKTA purification system which was equipped with in-line UV, conductivity and pH probes. The SEC analysis was performed using an Agilent HPVL 1100 system by injecting 40 μg of a sample on a TSKgel G3000SWXL column (7.8 x 300 mm), which also had an in-line guard column (6.0 x 40 mm) to extend column life. The mobile phase contained 50 mm sodium phosphate buffer and 400 mm sodium perchlorate, the flow rate was 1.0 mL/min, and the elution isocratic.

**Example 2: Binding Affinity of Murine EpCAM Antibodies**

Binding affinity was assayed by a flow cytometry binding assay using purified antibody mEpCAM23 (obtained using the third immunization protocol described in Example 1) and performed with HSC2 cells **(FIG. 1A)** or with cyno primary kidney epithelial cells **(FIG. 1B).** In the binding assay, $2\times10^4$ cells per sample were incubated with varying concentrations of mEpCAM23 in 200 μL FACS buffer (RPMI 1640 medium supplemented with 2% normal goat serum) on ice for 2hrs. The cells were then pelleted, washed twice, and incubated for 30 min with 100 μL of goat anti-mouse IgG-antibody conjugated with ALexa Fluor Plus 488. The cells were pelleted again, washed with FACS buffer and re-suspended in 200 μL of PBS containing 1% formaldehyde. Samples were acquired using a FACSCalibur™ flow cytometer with the HTS multiwell sampler and analyzed using CellQuest™ Pro. For each sample, the geomean fluorescence intensity for FL1 was calculated and plotted against the antibody concentration in a semi-log plot. A dose-response curve was generated by non-linear regression and the $EC_{50}$ value of each curve, which corresponds to the apparent dissociation constant ($K_d$) of each antibody, was calculated using GraphPad Prism v4.

As shown in **FIGs. 1A and 1B,** the apparent $K_d$ of the mEpCAM23 antibody ranged from $3.8 \times 10^{-10}$ to $7.9 \times 10^{-10}$. The sequence of mEpCAM23 was identified, and an mEpCAM23 clone was selected for chimerization, humanization and further evaluation.

**Example 3: Cloning and Sequencing of the VL and VH of the EpCAM Antibodies** <u>Cloning of the $V_L$ and $V_H$ Regions</u>

Total cellular RNA was prepared from $5 \times 10^6$ cells of the EpCAM *Hybridomas* using a Quick-RNA® mini-prep kit according to the manufacturer's protocol. cDNA was subsequently synthesized from total RNA using the SuperScript III® cDNA synthesis kit according to the manufacturer's instructions.

The PCR procedures for amplifying the antibody variable region cDNAs derived from *Hybridoma* cells were based on methods described in Wang et al. (J. Immunol. Methods 233:167-177 (2000)) and Co et al. (J. Immunol. 148:1149-54 (1992)). Briefly, the $V_L$ and $V_H$ sequences were amplified by degenerate primers on the 5'-end and either murine kappa or $IgG_1$ constant region specific primers, respectively, on the 3'-end. The purified amplicons were sent to Genewiz for Sanger sequencing.

Because the degenerate primers used to clone the $V_L$ and $V_H$ cDNA sequences can alter the 5'-end, additional sequencing efforts were needed to verify the complete cDNA sequences. The preliminary sequences were entered into a search query of the NCBI IgBlast site to identify the murine germline sequences from which the antibody sequences had been derived. PCR primers were then designed to anneal to the germline linked leader sequence of the murine antibody so that the new PCR reaction would yield a complete variable region cDNA sequence, unaltered by the PCR primers.

<u>Mass Determination for Sequence Confirmation</u>

The variable region cDNA sequences obtained for each of the EpCAM antibodies were combined with germline constant region sequences to obtain full length antibody cDNA sequences. To confirm these sequencing results, the molecular weights of the full length heavy chain and light chain cDNA sequences were compared with the molecular weights obtained by LC/MS analyses of the purified murine EpCAM antibodies.

Chimeric Antibody Expression and Purification

The variable region amino acid sequences for the murine EpCAM antibodies were codon-optimized, synthesized and cloned in-frame with human IgG1 constant regions by GenScript (New Jersey) to build chimeric versions of the EpCAM antibodies. Briefly, the light chain variable region was cloned into the EcoRI and BsiWI sites of the in-house pAbKZeo plasmid and the heavy chain variable region was cloned into the HindIII and Apa1 sites of the in-house pAbG1Neo plasmid. These expression constructs were transiently produced in suspension adapted HEK-293T cells using PEI as transfection reagent in shake flasks. The PEI transient transfections were performed as previously described (*see* Durocher et al., Nucleic Acids Res. 30(2):E9 (2002)), except that the HEK-293T cells were grown in Freestyle 293 (Invitrogen) and the culture volume was left undiluted after the addition of the PEI-DNA complexes. The transfections were incubated for a week, harvested, and the filtered supernatants were then purified using a combination of protein A and CHT chromatography using procedures as described in Example 1.

**Example 4: Antibody Humanization**

Antibody humanization was performed using complementarity determining region (CDR) grafting procedures essentially as described in Jones et al., Nature 321: 604-608 (1986), Verhoeyen et al., Science 239:1534-1536 (1988), US Pat. Nos. 5,225,539 and 5,585,089. CDR grafting generally consists of replacing the Fv framework regions (FRs) of a mouse antibody with human antibody Fv framework regions while preserving the mouse CDR residues critical for the specific antigen-binding properties of the parent antibody. Exemplary CDRs of the murine EpCAM-23 antibody following the Kabat CDR definitions are indicated in Table 13 below.

**Table 13.**

| **Murine EpCAM-23 CDRs** |
|---|
| **Heavy Chain** |
| CDR-H1: NYYIH (SEQ ID NO:13)<br>CDR-H2: WIYPGNVYIQYNEKFKG (SEQ ID NO:14)<br>CDR-H3: DGPWFAY (SEQ ID NO:15 |
| **Light Chain** |
| CDR-L1: RSSKSLLHSDGFTYLY (SEQ ID NO:39)<br>CDR-L2: QTSNLAS (SEQ ID NO:40)<br>CDR-L3: AQNLELPNT (SEQ ID NO:41) |

The CDR-grafting process begins by selecting appropriate human acceptor frameworks, typically those derived from human antibody genes sharing the highest sequence homology to the parent murine antibody. These acceptor frameworks are generally identified utilizing the interactive tool, DomainGapAlign, of the International ImMunoGeneTics information system® (IMGT (http://imgt.cines.fr/)) as described in Ehrenmann et al., Nucleic Acids Res. 38: D301-307 (2010). After selection of the $V_L$ and $V_H$ human germline sequences that share the highest sequence identity with the murine sequences, a humanized version of the antibody is generated by grafting the murine CDRs into the human germline sequences. Reduced or abolished antigen-binding affinity after CDR grafting is often observed as a consequence of incompatibilities between murine CDRs and human frameworks, Foote and Winter, J. Mol. Biol. 224:487-499 (1992); however, platform of residues directly underneath the CDRs, referred to as vernier zone residues, may help to preserve the conformation of the CDR loops that direct the specificity and affinity of the antibody. Therefore, back mutations of these vernier zone residues to murine residues are often needed to restore binding affinity.

**FIGs. 2A and 2B** show sequence alignments between the variable regions of the original muEpCAM-23 light chain and heavy chain sequences and their closest human germline matches. Based on the alignment results, the human germline sequences selected as the acceptor frameworks for the $V_L$ and $V_H$ domains of EpCAM-23 antibody were IGKV2D-29*02 **(FIG. 2A)** and IGHV1-3*01 **(FIG. 2B),** respectively.

An initial CDR graft of muEpCAM-23 was created by grafting Kabat positions 24-34 (CDR-L1), 50-56 (CDR-L2), and 89-97 (CDR-L2) of the VL, and Kabat positions 31-35 (CDR-H1), 50-65 (CDR-H2), and 95-102 (CDR-H3) of the VH, into the corresponding human germline IGKV2D-29*01 and IGHV1-3*01 frameworks. The initial CDR grafted version contained 12 framework residue substitutions in $V_L$ and 27 framework residue changes in $V_H$. Additionally, variants containing one or more back-mutations of the vernier zone residues were also made, and were subsequently evaluated for EpCAM-binding. The tested vernier zone residue back-mutations included 3 residues in the $V_L$ (position 4 in FW-L1, and positions 36, 64 in FW-L2) and 6 residues in the $V_H$ (positions 47, 48 in FW-H2, positions 67, 69, 71 and 73 in FW-H3).

Furthermore, to minimize the potential impact of conjugating lysine residues that fall in CDRs, lysine residues were replaced with arginine in the CDR regions of some versions. Additionally, in some grafted versions certain CDRH2 residues were changed to the IGHV1-3*01 germline residues to increase the degree of humanness. Table 14 and Table 15 list all the residue changes between the various humanized VL and VH versions, respectively. The humanized DNA constructs were synthesized, expressed, and the recombinant antibodies were purified essentially using procedures described in Example 2 for subsequent human EpCAM and cyno EpCAM-binding analysis.

**Table 14. CDR-grafting of EpCAM-23 antibody $V_L$**

| | | EpCAM-23-$V_L$ | | | |
|---|---|---|---|---|---|
| Kabat position | Murine residue | VL Gv1 residues | VL Gv2 residues | VL Gv3 residues | VL Gv4 residues |
| L4 | L | M | L | M | L |
| L9 | F | L | L | L | L |
| L11 | N | L | L | L | L |
| L12 | P | S | S | S | S |
| L15 | L | P | P | P | P |
| L17 | T | Q | Q | Q | Q |
| L18 | S | P | P | P | P |
| L27 | K | R | K | K | R |
| L36 | F | Y | F | F | F |
| L45 | H | Q | Q | Q | Q |
| L64 | S | G | S | G | S |
| L74 | R | K | K | K | K |
| L100 | G | Q | Q | Q | Q |

**Table 15. CDR-grafting of EpCAM-23 antibody $V_H$**

| | EpCAM-23-$V_H$ | | | | |
|---|---|---|---|---|---|
| Kabat position | Murine residue | VH Gv1 residues | VH Gv2 residues | VH Gv3 residues | VH Gv4 residues |
| H1 | E | Q | Q | Q | Q |
| H3 | K | Q | Q | Q | Q |
| H5 | E | V | V | V | V |
| H6 | E | Q | Q | Q | Q |
| H9 | P | A | A | A | A |
| H10 | A | E | E | E | E |
| H11 | L | V | V | V | V |
| H12 | V | K | K | K | K |
| H19 | R | K | K | K | K |
| H20 | I | V | V | V | V |
| H38 | K | R | R | R | R |
| H40 | R | A | A | A | A |
| H44 | G | R | R | R | R |
| H46 | D | E | E | E | E |
| H47 | Y | W | Y | W | Y |
| H48 | I | M | I | M | I |

(continued)

| | EpCAM-23-$V_H$ | | | | |
|---|---|---|---|---|---|
| Kabat position | Murine residue | VH Gv1 residues | VH Gv2 residues | VH Gv3 residues | VH Gv4 residues |
| H60 | N | S | N | N | S |
| H61 | E | Q | E | E | Q |
| H64 | K | Q | K | K | Q |
| H66 | K | R | R | R | R |
| H67 | A | V | A | V | V |
| H69 | L | I | L | I | I |
| H71 | A | R | A | R | A |
| H73 | K | T | K | T | K |
| H75 | S | A | A | A | A |
| H79 | F | Y | Y | Y | Y |
| H81 | Q | E | E | E | E |
| H83 | T | R | R | R | R |
| H87 | S | T | T | T | T |
| H91 | F | Y | Y | Y | Y |

Binding affinity of chimerized/humanized EpCAM antibodies

Flow cytometry binding assays were carried out and analyzed as described in Example 2 using secondary Alexa Fluor Plus 488 conjugated goat-anti-human antibodies (Invitrogen).

The chimerized antibody chEpCAM23 was assayed for its binding affinity to HSC2 cells **(FIG. 3A)** and cyno primary kidney epithelial cells **(FIG. 3B).**

Several humanized versions of EpCAM23 retained binding to human HSC2 cells **(FIG. 3C).** However, only version Gv2.2 (VL Gv2 and VH Gv2) and version Gv4.2 (VL Gv4 and VH Gv2) also retained binding to cyno primary kidney epithelial cells **(FIG. 3D).** In addition, another version of Gv4.2 with cysteine specific conjugation sites, huEpCAM23Gv4.2-C442, was made and evaluated. As shown in **FIGs. 3E** and **3F,** this antibody binds to HSC2 and cyno primary kidney epithelial cells with a similar Kd to that of huEpCAM23Gv4.2. **FIGs. 4A** and **4B** show the sequence alignment of the variable regions of the original murine EpCAM-23 antibody, grafted version Gv 2.2 and grafted version Gv 4.2. Payloads are generally conjugated to the antibody either using lysine or cysteine residues, and the grafted version Gv4.2 differed from version Gv2.2 by only a single amino acid (lysine to arginine in position 23). Therefore, for ease of conjugation, Gv4.2 was selected for further evaluation.

**Example 5: Affinity modulation of huEpCAM-23Gv4.2 antibody**

For durable tumor retention, monoclonal antibodies must bind to antigens with high affinity. However, it has been hypothesized that if the interaction between antibody and tumor antigen is too high, efficient tumor penetration of the monoclonal antibodies could be impaired, resulting in diminished *in vivo* efficacy. The optimal affinity is likely a function of many variables such as antigen expression and heterogeneity; tumor size; rate of receptor internalization and recycling; vasculature; bystander killing activity; and drug to antibody ratio (DAR) (Vasalou et al., PLoS ONE 10(3):e0118977(2015) doi:10.1371/journal.pone. 0118977). Therefore, to better understand the interplay of all these variables, affinity variants of the humanized antibody Gv4.2 were created. The variants were created either by mutagenizing solvent exposed CDR residues or VH/VL framework residues that rarely occur in the mouse/human natural repertoire (<5% frequency). The solvent exposed positions were identified by creating a homology model of the Gv4.2 antibody using the antibody-modeling feature of the commercial software MOE (Chemical computing group). Table 16 lists all the positions that were explored, and Table 17 shows the binding KD of various variants to Human HSC2 cells and cyno primary kidney epithelial cells. All of the variants were expressed, purified, and characterized for FACS binding essentially using procedures described in Examples 2 and 4.

**Table 16. Solvent exposed positions of Gv4.2 antibody.**

| Name | Variable region | Original Residue | Mutated Residue | Kabat Position |
|---|---|---|---|---|
| huEpCAM23HCGv2a | $V_H$ | Q | E | 6 |
| huEpCAM23HCGv2b | $V_H$ | N | S | 31 |
| huEpCAM23HCGv2c | $V_H$ | Y | N | 33 |
| huEpCAM23HCGv2d | VH | N | D | 54 |
| huEpCAM23HCGv2e | $V_H$ | Y | F | 33 |
| huEpCAM23HCGv2f | VH | Y | S | 33 |
| huEpCAM23HCGv2g | $V_H$ | Y | W | 33 |
| huEpCAM23HCGv2h | $V_H$ | I | F | 51 |
| huEpCAM23HCGv2i/2o | $V_H$ | Y | W | 47 |
| huEpCAM23HCGv2j | $V_H$ | Y | N | 52 |
| huEpCAM23HCGv2k | $V_H$ | D | E | 95 |
| huEpCAM23HCGv2l | $V_H$ | W | Y | 98 |
| huEpCAM23HCGv2m | $V_H$ | P | A | 97 |
| huEpCAM23HCGv2n | $V_H$ | I | M | 34 |
| huEpCAM23HCGv2p | $V_H$ | I | V | 51 |
| huEpCAM23HCGv2q | $V_H$ | L | I | 69 |
| huEpCAM23HCGv2r | $V_H$ | A | R | 71 |
| huEpCAM23HCGv2s | $V_H$ | A | V | 67 |
| huEpCam23HG2-1361-H | $V_H$ | Y | H | 33 |
| huEpCam23HG2-1361-D | $V_H$ | Y | D | 33 |
| huEpCam23HG2-1361-1 | $V_H$ | Y | I | 33 |
| huEpCam23HG2-1361-L | $V_H$ | Y | L | 33 |
| huEpCam23HG2-1565-Y | $V_H$ | P | Y | 97 |
| huEpCam23HG2-1565-S | $V_H$ | P | S | 97 |
| huEpCam23HG2-1565-A | $V_H$ | P | A | 97 |
| huEpCam23HG2-1565-F | $V_H$ | P | F | 97 |
| huEpCam23HG2-1565-G | $V_H$ | P | G | 97 |
| huEpCam23HG2-1565-T | $V_H$ | P | T | 97 |
| huEpCam23HG2-1565-V | $V_H$ | P | V | 97 |
| huEpCAM23LCGv4a | $V_L$ | D | N | 30c |
| huEpCAM23LCGv4b | $V_L$ | F | I | 30e |
| huEpCAM23LCGv4c | $V_L$ | N | W | 96 |
| huEpCAM23LCGv4e | $V_L$ | Y | S | 34 |
| huEpCAM23LCGv4f | $V_L$ | F | Y | 36 |
| huEpCAM23LCGv4g | $V_L$ | A | Q | 89 |
| huEpCAM23LCGv4h | $V_L$ | A | L | 89 |
| huEpCAM23LCGv4i | $V_L$ | N | Y | 91 |
| huEpCAM23LCGv4j | $V_L$ | N | G | 91 |
| huEpCAM23LCGv4k | $V_L$ | N | S | 91 |

**Table 17. Binding of select affinity variants to human HSC2 cells and cyno primary kidney epithelial cells.**

| **Variant name** | **Heavy chain** | **Light chain** | **KD on HSC2 cells (nM)** (NS for not saturated) | **KD on cyno primary kidney epithelial cells (nM)** (NS for not saturated) |
|---|---|---|---|---|
| huEpCAM23 Gv4.2a | huEpCAM23 HCGv2a | huEpCAM 23 LCGv4 | Not Tested | Not Tested |
| huEpCAM23 Gv4.2b | huEpCAM23 HCGv2b | huEpCAM 23 LCGv4 | Not Tested | Not Tested |
| huEpCAM23 Gv4.2c | huEpCAM23 HCGv2c | huEpCAM 23 LCGv4 | NS | NS |
| huEpCAM23 Gv4.2d | huEpCAM23 HCGv2d | huEpCAM 23 LCGv4 | Not Tested | Not Tested |
| | | | | |
| huEpCAM23Gv4a.2 | huEpCAM23HCGv2 | huEpCAM 23 LCGv4a | 0.1 | 1.1 |
| huEpCAM23Gv4b.2 | huEpCAM23HCGv2 | huEpCAM 23 LCGv4b | Not Tested | Not Tested |
| huEpCAM23Gv4c.2 | huEpCAM23HCGv2 | huEpCAM 23 LCGv4c | Not Tested | Not Tested |
| | | | | |
| huEpCAM23Gv4a.2a | huEpCAM23HCGv2 a | huEpCAM 23 LCGv4a | NS | NS |
| huEpCAM23Gv4a.2b | huEpCAM23HCGv2 b | huEpCAM 23 LCGv4a | 0.5 | 8 |
| huEpCAM23Gv4a.2d | huEpCAM23HCGv2 d | huEpCAM 23 LCGv4a | 0.6 | NS |
| huEpCAM23Gv4b.2a | huEpCAM23HCGv2 a | huEpCAM 23 LCGv4b | NS | NS |
| huEpCAM23Gv4b.2b | huEpCAM23HCGv2 b | huEpCAM 23 LCGv4b | 0.7 | 6.6 |
| huEpCAM23Gv4b.2d | huEpCAM23HCGv2 d | huEpCAM 23 LCGv4b | 1.4 | NS |
| huEpCAM23Gv4b.2a | huEpCAM23HCGv2 a | huEpCAM 23 LCGv4b | NS | NS |
| huEpCAM23Gv4b.2b | huEpCAM23HCGv2 b | huEpCAM 23 LCGv4b | 1.3 | 46 |

(continued)

| Variant name | Heavy chain | Light chain | KD on HSC2 cells (nM) (NS for not saturated) | KD on cyno primary kidney epithelial cells (nM) (NS for not saturated) |
|---|---|---|---|---|
| huEpCAM23Gv4b.2d | huEpCAM23HCGv2 d | huEpCAM 23 LCGv4b | 2.5 | NS |
| huEpCAM23Gv4.2f | huEpCAM23HCGv2f | huEpCAM 23 LCGv4 | NS | NS |
| huEpCAM23Gv4.2g | huEpCAM23HCGv2 g | huEpCAM 23 LCGv4 | 0.94 | NS |
| huEpCAM23Gv4.2h | huEpCAM23HCGv2 h | huEpCAM 23 LCGv4 | 2.5 | NS |
| huEpCAM23Gv4.2i | huEpCAM23HCGv2i | huEpCAM 23 LCGv4 | 0.95 | ~15 |
| huEpCAM23Gv4.2j | huEpCAM23HCGv2j | huEpCAM 23 LCGv4 | 0.6 | NS |
| huEpCAM23Gv4.2k | huEpCAM23HCGv2 k | huEpCAM 23 LCGv4 | 0.59 | 6.3 |
| huEpCAM23Gv4.2l | huEpCAM23HCGv2l | huEpCAM 23 LCGv4 | 2.7 | NS |
| huEpCAM23Gv4.2m | huEpCAM23HCGv2 m | huEpCAM 23 LCGv4 | 2.1 | NS |
| huEpCAM23Gv4.2o | huEpCAM23HCGv2 o | huEpCAM 23 LCGv4 | 1.0 | 11.4 |
| huEpCAM23 Gv4.2p | huEpCAM23HCGv2 p | huEpCAM 23 LCGv4 | 1.1 | 8.6 |
| huEpCAM23Gv4.2q | huEpCAM23HCGv2 q | huEpCAM 23 LCGv4 | NS | NS |
| huEpCAM23Gv4.2r | huEpCAM23HCGv2r | huEpCAM 23 LCGv4 | NS | 7.3 |
| huEpCAM23Gv4.2s | huEpCAM23HCGv2 s | huEpCAM 23 LCGv4 | 0.6 | 4.7 |
| huEpCAM23Gv4.2-1361A | huEpCam23HG2-1361-A | huEpCAM 23 LCGv4 | NS | NS |
| huEpCAM23Gv4.2-1361D | huEpCam23HG2-1361-D | huEpCAM 23 LCGv4 | ~6 | NS |
| huEpCAM23Gv4.2-1361G | huEpCam23HG2-1361-G | huEpCAM 23 LCGv4 | NS | NS |
| huEpCAM23Gv4.2-1361H | huEpCam23HG2-1361-H | huEpCAM 23 LCGv4 | 1.3 | NS |
| huEpCAM23Gv4.2-1361I | huEpCam23HG2-1361-I | huEpCAM 23 LCGv4 | NS | NS |
| huEpCAM23Gv4.2-1361L | huEpCam23HG2-1361-L | huEpCAM 23 LCGv4 | 0.75 | NS |
| huEpCAM23Gv4.2-1361N | huEpCam23HG2-1361-N | huEpCAM 23 LCGv4 | NS | NS |

(continued)

| Variant name | Heavy chain | Light chain | **KD on HSC2 cells (nM)** (NS for not saturated) | **KD on cyno primary kidney epithelial cells (nM)** (NS for not saturated) |
|---|---|---|---|---|
| huEpCAM23Gv4.2-1361P | huEpCam23HG2-1361-P | huEpCAM 23 LCGv4 | NS | NS |
| huEpCAM23Gv4.2-1361R | huEpCam23HG2-1361-R | huEpCAM 23 LCGv4 | NS | NS |
| huEpCAM23Gv4.2-1361V | huEpCam23HG2-1361-V | huEpCAM 23 LCGv4 | 1.7 | NS |
| huEpCAM23Gv4.2-1565A | huEpCam23HG2-1565-A | huEpCAM 23 LCGv4 | 1.5 | NS |
| huEpCAM23Gv4.2-1565F | huEpCam23HG2-1565-A | huEpCAM 23 LCGv4 | 1.5 | NS |
| huEpCAM23Gv4.2-1565G | huEpCam23HG2-1565-G | huEpCAM 23 LCGv4 | 1.1 | NS |
| huEpCAM23Gv4.2-1565H | huEpCam23HG2-1565-H | huEpCAM 23 LCGv4 | NS | NS |
| huEpCAM23Gv4.2-1565L | huEpCam23HG2-1565-L | huEpCAM 23 LCGv4 | NS | NS |
| huEpCAM23Gv4.2-1565R | huEpCam23HG2-1565-R | huEpCAM 23 LCGv4 | NS | NS |
| huEpCAM23Gv4.2-1565S | huEpCam23HG2-1565-S | huEpCAM 23 LCGv4 | 4.2 | NS |
| huEpCAM23Gv4.2-1565T | huEpCam23HG2-1565-T | huEpCAM 23 LCGv4 | 1.6 | NS |
| huEpCAM23Gv4.2-1565V | huEpCam23HG2-1565-V | huEpCAM 23 LCGv4 | 1.3 | NS |
| huEpCAM23Gv4.2-1565T | huEpCam23HG2-1565-T | huEpCAM 23 LCGv4 | 1.2 | ~5.2 |
| * NS = not saturated | | | | |

**Example 6: Binding affinity of the affinity variants of humanized anti-EpCAM23Gv4.2 antibody**

An initial set of humanized anti-EpCAM23Gv4.2 affinity variants was tested in an enzyme-linked immunosorbent assay (ELISA) using mFc-tagged huEpCAM or cynoEpCAM protein. Briefly, each mFc-tagged EpCAM protein was diluted to 0.5 ug/mL in 50 mM sodium bicarbonate buffer pH 9.6, and 100 μL was added to each well. After a 16 hr incubation at 4°C, the plates were washed with Tris-buffered saline with 0.1% Tween-20 (TBST), then blocked with 200 μL blocking buffer (TBS with 1% BSA). Next, 100 μL of primary antibody, the affinity variant serially diluted in blocking buffer, was added in duplicate to the ELISA wells and incubated at room temperature for 1 hr. The plates were then washed 3 times with TBST before adding 100 μL of anti-human IgG (H+L)-HRP to each well. The plates were again incubated for 1 hr at room temperature, followed by three washes with TBST. Finally, 100 μL of TMB one component HRP microwell substrate was added to each well and incubated for 5 min. The reaction was stopped by adding 100 μL stopping solution and absorbances were read at 450 nm in a multiwell plate reader. OD450 was plotted against the antibody concentration in a semi-log plot. A dose-response curve was generated by non-linear regression and the EC50 value of each curve was calculated using GraphPad Prism v6.

The results from the ELISA assay are show in **FIGs. 5A and 5B** (for variants comprising a mutation in the light chain) and in **FIGs. 5C and 5D** (for variants comprising a mutation in the heavy chain). The seven variants shown in **FIGs. 5A-5D** exhibited different binding profiles, with particularly distinct binding exhibited by light chain variant Gv4c.2 and heavy chain variant Gv4.2c. Variant Gv4c.2 showed the weakest binding to the cynoEpCAM-mFc protein among the three

light chain mutation variants, with about a 3-fold weaker affinity than its parent antibody **(FIG. 5B).** However, variant Gv4c.2 showed a similar KD to huEpCAM-mFc as the parent antibody **(FIG. 5A).** Among the four heavy chain mutation variants, variant Gv4.2c showed significantly reduced binding to cynoEpCAM-mFc **(FIG. 5D),** but showed similar binding to huEpCAM-mFc **(FIG. 5C)** compared to the parent antibody. Using different combinations of these heavy chain and light chain variants, double mutation variants were made in addition to the above-referenced single mutation variants.

The binding affinity of additional humanized anti-EpCAM23Gv4.2 affinity variants to HSC2 cells and to cyno primary kidney epithelial cells was compared to that of the parent antibody. Flow cytometry binding assays were carried out and analyzed as described in Example 2 and 4, and a total of 60 variants were evaluated. **FIGs. 5E-5H** show the binding curves for double mutant variants, and **FIGs. 5I-5K** show the binding curves of certain affinity variants containing single mutations in the huEpCAM23Gv4.2 heavy chain. As shown in **FIGs. 5E-5K,** variants were generated with a variety of binding profiles. In particular, the double mutation variants Gv4a.2a, Gv4b.2a, and Gv4c.2a exhibited very high fluorescence intensities and failed to saturate both cells types: HSC2 **(FIG. 5E)** and cyno primary kidney epithelial cells **(FIG. 5F).** Additionally, variants Gv4a.2b and Gv4b.2b exhibited about a 4-fold decrease in affinity compared to the parent antibody on cyno primary kidney epithelial cells **(FIG. 5H),** but showed similar binding on HSC2 cells **(FIG. 5G).** The double mutation variants Gv4b.2d, Gv4c.2b and Gv4c.2d had very poor binding on cyno primary kidney epithelial cells but similar binding on HSC2 cells relative to the parent antibody. As shown in **FIGs. 5I-5K,** the apparent dissociation constant ($K_d$) of the single mutation affinity variants ranged from 0.8 nM to about 6 nM, which represents a 2- to 15-fold increase compared to the parent antibody huEpCAM23Gv4.2 ($K_d$ 0.4 nM) on HSC2 cells. These single mutation variants were also tested with cyno primary kidney epithelial cells, with the data shown in **FIGs. 5L-5N.**

In particular, huEpCAM23Gv4.2Q exhibited a high fluorescence intensity on both HSC2 cells and cyno primary kidney cells, while huEpCAM23Gv4.2R only exhibited a high fluorescence intensity on HSC2 cells (**FIGs. SI** and **5L**). huEpCAM23Gv4.2H and huEpCAM23Gv4.2L exhibited similar $K_d$ values but were associated with very poor binding on cyno primary kidney epithelial cells. The other clones shown in **FIGs. 5I** and **5L** saturated on both cell types and were associated with various $K_d$ values.

**FIGs. 5J** and **5M** show the binding characteristics for affinity variants containing a particular amino acid substitution in the same position of CDR1 (*i.e.,* substitution of the original Y residue at position 33 for another amino acid). All of the variants shown in **FIGs. 5J** and **5M** had reduced $K_d$ values on HSC2 cells compared that of the parent antibody and exhibited very poor binding (with the exception huEpCAM23Gv4.2-1361-I) on cyno primary kidney epithelial cells. **FIGs. 5K** and **5N** show the binding characteristics for affinity variants containing a particular amino acid substitution in the same position of CDR3 (i.e., substitution of the original P residue at position 97 for another amino acid). The huEpCAM23Gv4.2-1565-Y variant saturated both HSC2 cells and cyno primary epithelial cells with reduced $K_d$ value compared to that of the parent antibody, while the rest of variants saturated only on HSC2 cells and not on cyno primary kidney epithelial cells.

Collectively, these data show that affinity variants were generated with various geomean fluorescence binding intensities and with a range of $K_d$. Based on these results, two affinity variants, huEpCAM23Gv4.2-1361-H and huEpCAM23Gv4.2-1565-Y, were selected for further evaluation. In particular, these variants were selected due to their somewhat lower binding affinities on HSC2 cells relative to the parent antibody (*i.e.,* about a 3-fold reduced $K_d$ compared to the parent antibody on HSC2 cells) and their different mutation sites.

## Example 7: Mask Discovery

The studies provided herein were designed to identify and characterize masking moieties (MM or "masks") for use in activatable anti-EpCAM antibodies ("activatable antibodies").

A humanized anti-human EpCAM monoclonal antibody (EpCAM23Gv4.2) that is cross-reactive with human and cynomolgus EpCAM was used to screen a random $X_{15}$ peptide library where X is any amino acid, using a method similar to that described in PCT International Publication Number WO 2010/081173, published 15 July 2010. The screening consisted of one round of MACS and three rounds of FACS sorting. The initial MACS sorting was done with protein-A Dynabeads (Invitrogen) with the anti-EpCAM antibody. For MACS, anti-EpCAM antibody was conjugated with Dy-Light-488 (ThermoFisher), EpCAM binding activity was confirmed, and anti-EpCAM-488 was used as a fluorescent probe for all FACS rounds. Individual peptide clones were identified by sequence analysis from each FACS round.

Two heavy chain variants of the humanized anti-human EpCAM monoclonal antibody (EpCAM23(1361-H) and EpCAM23(1565-Y)) were generated and used to screen a random $X_{15}$ peptide library where X is any amino acid, using a method similar to that described in PCT International Publication Number WO 2010/081173, published 15 July 2010. Masks EP101 to EP 104 were identified using the EpCAM23(1565-Y) heavy chain variant. Masks EP105 to EP 110 were identified using the EpCAM23(1361-H) heavy chain variant. Mutations of the lysine residue in the Ep107 masking moiety were also generated

The sequences of selected anti-EpCAM masking moieties are listed in Table 18.

**Table 18. Masking Moieties for EpCAM Activatable Antibodies.**

| Anti-EpCAM Masking Moietv | Amino Acid Sequence |
|---|---|
| **Masks for Gv4.2 Humanized Antibody** | |
| EP01 | PLMTCSDYYTCKNNL (SEQ ID NO:218) |
| EP01-1 | PLMTCSDYYTCHNNL (SEQ ID NO:219) |
| EP01-2 | PLMTCSDYYTCLNNL (SEQ ID NO:151) |
| EP01-3 | PLMTCSDYYTCRNNL (SEQ ID NO:220) |
| EP02 | LSCTHSRYDMHCPHM (SEQ ID NO:152) |
| EP03 | HYCHSRTDTITHCNA (SEQ ID NO:153) |
| EP04 | WCPRLFDRPSMGCPT (SEQ ID NO:154) |
| EP05 | WWPPCQGGAWCEQRI (SEQ ID NO:155) |
| EP06 | KFSCTTMPHKNCMKP (SEQ ID NO:221) |
| EP07 | HSGCPRLFDRCSAPA (SEQ ID NO:156) |
| EP08 | PLMTCTKYNNCINNL (SEQ ID NO:222) |
| EP09 | PNYMCKLNTCTHHIA (SEQ ID NO:223) |
| EP10 | AACAGGVLFDRCSVD (SEQ ID NO:224) |
| EP11 | FICPTLYDRPHCMHT (SEQ ID NO:157) |
| EP12 | NMPACQGGSWCRPGY (SEQ ID NO:225) |
| EP13 | TLCARPPSYQLCSHV (SEQ ID NO:226) |
| EP14 | NLCPPGETRTQCQIT (SEQ ID NO:227) |
| EP15 | LYCALYSVQLFCVDL (SEQ ID NO:228) |
| EP16 | KMAPCDAWYCHQQTA (SEQ ID NO:229) |
| EP17 | QDECSNKEAENCSDV (SEQ ID NO:230) |
| EP18 | QTCYGWSWSGSCLWY (SEQ ID NO:231) |
| EP19 | SNPCISTGHTHCQKI (SEQ ID NO:232) |
| EP20 | QICFGGSKNRYDCFM (SEQ ID NO:233) |
| EP21 | PTAPCHFNPPCRNTG (SEQ ID NO: 234) |
| EP22 | VCSGSSPGHPRVCGD (SEQ ID NO:235) |
| EP23 | LLPCPTSMPSYCFNY (SEQ ID NO:236) |
| EP24 | RGTPCTINTCTHHFS (SEQ ID NO: 237) |
| EP25 | KNGGCKVPIPCKSWS (SEQ ID NO:238) |
| **Masks for Affinity Variants 1361-H and 1565-Y** | |
| EP101 | SWCHSATDTILPCSN (SEQ ID NO:158) |
| EP102 | SPACSDRYYQTCVLN (SEQ ID NO:159) |
| EP103 | MSCVVDRFDRQCPHL (SEQ ID NO:160) |
| EP 104 | TTRCEHYWFTCPLSP (SEQ ID NO:161) |
| EP105 | DCTGYSPSVLPACRV (SEQ ID NO:162) |
| EP 106 | FCSGYSPSVLPSCLM (SEQ ID NO:163) |
| EP 107 | SKPCSYMHPYCFYNS (SEQ ID NO:164) |
| EP107-1 | SRPCSYMHPYCFYNS (SEQ ID NO:239) |
| EP107-2 | SHPCSYMHPYCFYNS (SEQ ID NO:240) |

(continued)

| Masks for Affinity Variants 1361-H and 1565-Y | |
|---|---|
| EP107-3 | SLPCSYMHPYCFYNS (SEQ ID NO:241) |
| EP 108 | LTRCTIAHPYCYYNY (SEQ ID NO:165) |
| EP 109 | PNTCMSERRICSLTY (SEQ ID NO:166) |
| EP110 | PRPHCAILRQCLAAT (SEQ ID NO:167) |

These masking peptides were used to generate anti-EpCAM activatable antibodies of the disclosure. The amino acid sequences for certain of these anti-EpCAM activatable antibodies are shown below in Table 19. In some embodiments, these anti-EpCAM activatable antibodies of the present disclosure include ISSGLLSGRSDNH (SEQ ID NO:242), AVGLLAPPGGLSGRSDNH (SEQ ID NO:243), ISSGLLSGRSDIH (SEQ ID NO:244), ISSGLLSGRSDQH (SEQ ID NO:245), ISSGLLSGRSDNP (SEQ ID NO:246), ISSGLLSGRSANP (SEQ ID NO:247), ISSGLLSGRSANI(SEQ ID NO:248), ISSGLLSGRSDNI (SEQ ID NO:169), AVGLLAPPGGLSGRSDIH (SEQ ID NO:249), AVGLLAPPGGLSGRSDQH (SEQ ID NO:250), AVGLLAPPGGLSGRSDNP(SEQ ID NO:251), AVGLLAPPGGLSGRSANP (SEQ ID NO:252), AVGLLAPPGGLSGRSANI (SEQ ID NO:253), or AVGLLAPPGGLSGRSDNI (SEQ ID NO:168). In some embodiments, certain anti-EpCAM activatable antibodies of the present disclosure include a non-cleavable moiety "NSUB" which is not expected to be cleaved.

While certain light chain sequences of activatable antibodies of the present disclosure shown below include the N-terminal spacer sequence of QGQSGQG (SEQ ID NO:254), those of ordinary skill in the art appreciate that the activatable anti-EpCAM antibodies of the disclosure can include any suitable spacer sequence, such as, for example, a spacer sequence selected from the group consisting of QGQSGQ (SEQ ID NO:255), QGQSG (SEQ ID NO:256), QGQS (SEQ ID NO:257), QGQ, QG, GQSGQG (SEQ ID NO:258), QSGQG (SEQ ID NO:259), SGQG (SEQ ID NO:260), GQG, G, or Q. In some embodiments, the light chains of activatable anti-EpCAM antibodies of the disclosure can have no spacer sequence joined to the N-terminus.

**Table 19. Variable light chain amino acid sequences of activatable antibodies of the disclosure.**

| Activatable antibody Light Chain Variable Domain | Amino Acid Sequence |
|---|---|
| EP01-2014-Gv4 | QGQSGQGPLMTCSDYYTCKNNLGGGSSGGSISSGLLSGRSDNIG GSDIVLTQTPLSLSVTPGQPASISCRSSRSLLHSDGFTYLYWFLQKP GQSPQLLIYQTSNLASGVPDRFSSSGSGTDFTLKISRVEAEDVGVYY CAQNLELPNTFGQGTKLEIK (SEQ ID NO:261) |
| EP01-1-2014-Gv4 | QGQSGQGPLMTCSDYYTCHNNLGGGSSGGSISSGLLSGRSDNIG GSDIVLTQTPLSLSVTPGQPASISCRSSRSLLHSDGFTYLYWFLQKP GQSPQLLIYQTSNLASGVPDRFSSSGSGTDFTLKISRVEAEDVGVYY CAQNLELPNTFGQGTKLEIK (SEQ ID NO:262) |
| EP01-2-2014-Gv4 | QGQSGQGPLMTCSDYYTCLNNLGGGSSGGSISSGLLSGRSDNIGG SDIVLTQTPLSLSVTPGQPASISCRSSRSLLHSDGFTYLYWFLQKPG QSPQLLIYQTSNLASGVPDRFSSSGSGTDFTLKISRVEAEDVGVYYC AQNLELPNTFGQGTKLEIK (SEQ ID NO:263) |
| EP01-3-2014-Gv4 | QGQSGQGPLMTCSDYYTCRNNLGGGSSGGSISSGLLSGRSDNIG GSDIVLTQTPLSLSVTPGQPASISCRSSRSLLHSDGFTYLYWFLQKP GQSPQLLIYQTSNLASGVPDRFSSSGSGTDFTLKISRVEAEDVGVYY CAQNLELPNTFGQGTKLEIK (SEQ ID NO:264) |

(continued)

| Activatable antibody Light Chain Variable Domain | Amino Acid Sequence |
|---|---|
| EP02-2014-Gv4 | QGQSGQGLSCTHSRYDMHCPHMGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:265) |
| EP03-2014-Gv4 | QGQSGQGHYCHSRTDTITHCNAGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:266) |
| EP04-2014-Gv4 | QGQSGQGWCPRLFDRPSMGCPTGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:267) |
| EP05-2014-Gv4 | QGQSGQGWWPPCQGGAWCEQRIGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:268) |
| EP06-2014-Gv4 | QGQSGQGKFSCTTMPHKNCMKPGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:269) |
| EP07-2014-Gv4 | QGQSGQGHSGCPRLFDRCSAPAGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:270) |
| EP08-2014-Gv4 | QGQSGQGPLMTCTKYNNCINNLGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:271) |
| EP09-2014-Gv4 | QGQSGQGPNYMCKLNTCTHHIAGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:272) |
| EP10-2014-Gv4 | QGQSGQGAACAGGVLFDRCSVDGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:273) |

(continued)

| Activatable antibody Light Chain Variable Domain | Amino Acid Sequence |
|---|---|
| EP11-2014-Gv4 | QGQSGQGFICPTLYDRPHCMHTGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:274) |
| EP12-2014-Gv4 | QGQSGQGNMPACQGGSWCRPGYGGGSSGGSISSGLLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:275) |
| EP01-3014-Gv4 | QGQSGQGPLMTCSDYYTCKNNLGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:276) |
| EP01-1-3014-Gv4 | QGQSGQGPLMTCSDYYTCHNNLGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:277) |
| EP01-2-3014-Gv4 | QGQSGQGPLMTCSDYYTCLNNLGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:278) |
| EP01-3-3014-Gv4 | QGQSGQGPLMTCSDYYTCRNNLGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:279) |
| EP02-3014-Gv4 | QGQSGQGLSCTHSRYDMHCPHMGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:280) |
| EP03-3014-Gv4 | QGQSGQGHYCHSRTDTITHCNAGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:281) |
| EP04-3014-Gv4 | QGQSGQGWCPRLFDRPSMGCPTGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:282) |

(continued)

| Activatable antibody Light Chain Variable Domain | Amino Acid Sequence |
|---|---|
| EP05-3014-Gv4 | QGQSGQGWWPPCQGGAWCEQRIGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:283) |
| EP06-3014-Gv4 | QGQSGQGKFSCTTMPHKNCMKPGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:284) |
| EP07-3014-Gv4 | QGQSGQGHSGCPRLFDRCSAPAGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:285) |
| EP08-3014-Gv4 | QGQSGQGPLMTCTKYNNCINNLGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:286) |
| EP09-3014-Gv4 | QGQSGQGPNYMCKLNTCTHHIAGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:287) |
| EP10-3014-Gv4 | QGQSGQGAACAGGVLFDRCSVDGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:288) |
| EP11-3014-Gv4 | QGQSGQGFICPTLYDRPHCMHTGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:289) |
| EP12-3014-Gv4 | QGQSGQGNMPACQGGSWCRPGYGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:290) |
| **Variable Light Chains for Affinity Variants 1361-H and 1565-Y** | |
| EP101-3014-Gv4 | QGQSGQGSWCHSATDTILPCSNGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:291) |

(continued)

| Variable Light Chains for Affinity Variants 1361-H and 1565-Y | |
|---|---|
| EP102-3014-Gv4 | QGQSGQGSPACSDRYYQTCVLNGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:292) |
| EP103-3014-Gv4 | QGQSGQGMSCVVDRFDRQCPHLGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:293) |
| EP104-3014-Gv4 | QGQSGQGTTRCEHYWFTCPLSPGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:294) |
| EP105-3014-Gv4 | QGQSGQGDCTGYSPSVLPACRVGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:295) |
| EP106-3014-Gv4 | QGQSGQGFCSGYSPSVLPSCLMGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:296) |
| EP107-3014-Gv4 | QGQSGQGSKPCSYMHPYCFYNSGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:297) |
| EP108-3014-Gv4 | QGQSGQGLTRCTIAHPYCYYNYGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:298) |
| EP109-3014-Gv4 | QGQSGQGPNTCMSERRICSLTYGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:299) |
| EP110-3014-Gv4 | QGQSGQGPRPHCAILRQCLAATGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:300) |

(continued)

| Variable Light Chains for Affinity Variants 1361-H and 1565-Y | |
|---|---|
| EP107-1-3014-Gv4 | QGQSGQGSRPCSYMHPYCFYNSGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:301) |
| EP107-2-3014-Gv4 | QGQSGQGSHPCSYMHPYCFYNSGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:302) |
| EP107-3-3014-Gv4 | QGQSGQGSLPCSYMHPYCFYNSGGGSSGGAVGLLAPPGGLSGRSDNIGGSDIVLTQTPLSLSVTPGQPASISCRSSRSLLHSDGFTYLYWFLQKPGQSPQLLIYQTSNLASGVPDRFSSSGSGTDFTLKISRVEAEDVGVYYCAQNLELPNTFGQGTKLEIK (SEQ ID NO:303) |

Exemplary anti-EpCAM activatable antibodies having the following heavy and light chains are shown below in Table 20.

**Table 20. Anti-EpCAM Activatable Antibodies.**

| Anti-EpCAM Activatable Antibody | Heavy Chain | Light Chain |
|---|---|---|
| EP01-2014 | EpCAM23Gv4.2 HC | EP01-2014 LC |
| EP02-2014 | EpCAM23Gv4.2 HC | EP02-2014 LC |
| EP03-2014 | EpCAM23Gv4.2 HC | EP03-2014 LC |
| EP04-2014 | EpCAM23Gv4.2 HC | EP04-2014 LC |
| EP05-2014 | EpCAM23Gv4.2 HC | EP05-2014 LC |
| EP06-2014 | EpCAM23Gv4.2 HC | EP06-2014 LC |
| EP07-2014 | EpCAM23Gv4.2 HC | EP07-2014 LC |
| EP08-2014 | EpCAM23Gv4.2 HC | EP08-2014 LC |
| EP09-2014 | EpCAM23Gv4.2 HC | EP09-2014 LC |
| EP10-2014 | EpCAM23Gv4.2 HC | EP10-2014 LC |
| EP11-2014 | EpCAM23Gv4.2 HC | EP11-2014 LC |
| EP12-2014 | EpCAM23Gv4.2 HC | EP12-2014 LC |
| EP01-1-2014 | EpCAM23Gv4.2 HC | EP01-1-2014 LC |
| EP01-2-2014 | EpCAM23Gv4.2 HC | EP01-2-2014 LC |
| EP01-3-2014 | EpCAM23Gv4.2 HC | EP01-3-2014 LC |
| EP01-3014 | EpCAM23Gv4.2 HC | EP01-3014 LC |
| EP02-3014 | EpCAM23Gv4.2 HC | EP02-3014 LC |
| EP03-3014 | EpCAM23Gv4.2 HC | EP03-3014 LC |
| EP04-3014 | EpCAM23Gv4.2 HC | EP04-3014 LC |
| EP05-3014 | EpCAM23Gv4.2 HC | EP05-3014 LC |
| EP06-3014 | EpCAM23Gv4.2 HC | EP06-3014 LC |
| EP07-3014 | EpCAM23Gv4.2 HC | EP07-3014 LC |

(continued)

| Anti-EpCAM Activatable Antibody | Heavy Chain | Light Chain |
|---|---|---|
| EP08-3014 | EpCAM23Gv4.2 HC | EP08-3014 LC |
| EP09-3014 | EpCAM23Gv4.2 HC | EP09-3014 LC |
| EP10-3014 | EpCAM23Gv4.2 HC | EP10-3014 LC |
| EP11-3014 | EpCAM23Gv4.2 HC | EP11-3014 LC |
| EP12-3014 | EpCAM23Gv4.2 HC | EP12-3014 LC |
| EP01-1-3014 | EpCAM23Gv4.2 HC | EP01-1-3014 LC |
| EP01-2-3014 | EpCAM23Gv4.2 HC | EP01-2-3014 LC |
| EP01-3-3014 | EpCAM23Gv4.2 HC | EP01-3-3014 LC |
| EP01-NSUB | EpCAM23Gv4.2 HC | EP01-NSUB LC |
| EP02-NSUB | EpCAM23Gv4.2 HC | EP02-NSUB LC |
| EP03-NSUB | EpCAM23Gv4.2 HC | EP03-NSUB LC |
| EP04-NSUB | EpCAM23Gv4.2 HC | EP04-NSUB LC |
| EP05-NSUB | EpCAM23Gv4.2 HC | EP05-NSUB LC |
| EP06-NSUB | EpCAM23Gv4.2 HC | EP06-NSUB LC |
| EP07-NSUB | EpCAM23Gv4.2 HC | EP07-NSUB LC |
| EP08-NSUB | EpCAM23Gv4.2 HC | EP08-NSUB LC |
| EP09-NSUB | EpCAM23Gv4.2 HC | EP09-NSUB LC |
| EP10-NSUB | EpCAM23Gv4.2 HC | EP10-NSUB LC |
| EP11-NSUB | EpCAM23Gv4.2 HC | EP11-NSUB LC |
| EP12-NSUB | EpCAM23Gv4.2 HC | EP12-NSUB LC |
| EP01-1-NSUB | EpCAM23Gv4.2 HC | EP01-1-NSUB LC |
| EP01-2-NSUB | EpCAM23Gv4.2 HC | EP01-2-NSUB LC |
| EP01-3-NSUB | EpCAM23Gv4.2 HC | EP01-3-NSUB LC |
| EP101-3014 (1565-Y) | EpCAM23(1565-Y) HC | EP101-3014 LC |
| EP102-3014 (1565-Y) | EpCAM23(1565-Y) HC | EP102-3014 LC |
| EP103-3014 (1565-Y) | EpCAM23(1565-Y) HC | EP103-3014 LC |
| EP104-3014 (1565-Y) | EpCAM23(1565-Y) HC | EP104-3014 LC |
| EP105-3014 (1361-H) | EpCAM23(1361-H) HC | EP105-3014 LC |
| EP106-3014 (1361-H) | EpCAM23(1361-H) HC | EP106-3014 LC |
| EP107-3014 (1361-H) | EpCAM23(1361-H) HC | EP107-3014 LC |
| EP108-3014 (1361-H) | EpCAM23(1361-H) HC | EP108-3014 LC |
| EP109-3014 (1361-H) | EpCAM23(1361-H) HC | EP109-3014 LC |
| EP110-3014 (1361-H) | EpCAM23(1361-H) HC | EP110-3014 LC |
| EP107-1-3014 (1361-H) | EpCAM23(1361-H) HC | EP107-1-3014 LC |
| EP107-1-3014 (1361-H) | EpCAM23(1361-H) HC | EP107-2-3014 LC |
| EP107-1-3014 (1361-H) | EpCAM23(1361-H) HC | EP107-3-3014 LC |
| EP01-3014 (1565-Y) | EpCAM23(1565-Y) HC | EP01-3014 LC |
| EP02-3014 (1565-Y) | EpCAM23(1565-Y) HC | EP02-3014 LC |
| EP03-3014 (1565-Y) | EpCAM23(1565-Y) HC | EP03-3014 LC |

(continued)

| Anti-EpCAM Activatable Antibody | Heavy Chain | Light Chain |
|---|---|---|
| EP04-3014 (1565-Y) | EpCAM23(1565-Y) HC | EP04-3014 LC |
| EP05-3014 (1565-Y) | EpCAM23(1565-Y) HC | EP05-3014 LC |
| EP06-3014 (1565-Y) | EpCAM23(1565-Y) HC | EP06-3014 LC |
| EP07-3014 (1565-Y) | EpCAM23(1565-Y) HC | EP07-3014 LC |
| EP08-3014 (1565-Y) | EpCAM23(1565-Y) HC | EP08-3014 LC |
| EP09-3014 (1565-Y) | EpCAM23(1565-Y) HC | EP09-3014 LC |
| EP10-3014 (1565-Y) | EpCAM23(1565-Y) HC | EP10-3014 LC |
| EP11-3014 (1565-Y) | EpCAM23(1565-Y) HC | EP11-3014 LC |
| EP12-3014 (1565-Y) | EpCAM23(1565-Y) HC | EP12-3014 LC |
| EP01-3014 (1361-H) | EpCAM23(1361-H) HC | EP01-3014 LC |
| EP02-3014 (1361-H) | EpCAM23(1361-H) HC | EP02-3014 LC |
| EP03-3014 (1361-H) | EpCAM23(1361-H) HC | EP03-3014 LC |
| EP04-3014 (1361-H) | EpCAM23(1361-H) HC | EP04-3014 LC |
| EP05-3014 (1361-H) | EpCAM23(1361-H) HC | EP05-3014 LC |
| EP06-3014 (1361-H) | EpCAM23(1361-H) HC | EP06-3014 LC |
| EP07-3014 (1361-H) | EpCAM23(1361-H) HC | EP07-3014 LC |
| EP08-3014 (1361-H) | EpCAM23(1361-H) HC | EP08-3014 LC |
| EP09-3014 (1361-H) | EpCAM23(1361-H) HC | EP09-3014 LC |
| EP10-3014 (1361-H) | EpCAM23(1361-H) HC | EP10-3014 LC |
| EP11-3014 (1361-H) | EpCAM23(1361-H) HC | EP11-3014 LC |
| EP12-3014 (1361-H) | EpCAM23(1361-H) HC | EP12-3014 LC |

A solid-phase binding assay was used to demonstrate the binding of anti-human EpCAM antibodies of the present disclosure. Briefly, recombinant human EpCAM-mFC protein (Immunogen) was coated on ELISA plates (50 μL of 1 μg/mL), and then incubated with serially-diluted anti-EpCAM antibody (starting at 62.5 nM) or activatable anti-EpCAM antibodies (starting at 1 μM), where in the activatable antibodies were assayed in their uncleaved form. The amount of bound antibody was detected using anti-human IgG (anti-Fab) conjugated to horseradish peroxidase (Sigma) with Ultra TMB-ELISA reagent (Thermo Fisher Scientific) and the OD was measured at 450 nM. The $K_D$ were measured for each antibody and activatable antibody, and the ELISA masking efficiency (ME) for each activatable antibody relative to the unmasked antibody was calculated, with exemplary results are shown in Tables 21 and 22. Collectively, these data show that anti-human EpCAM activatable antibodies of the present disclosure demonstrate a shifted binding affinity to recombinant EpCAM protein compared to the parental anti-EpCAM antibody of the present disclosure.

**Table 21. Anti-EpCAM Activatable Antibodies Masking Efficiencies.**

| Anti-EpCAM Activatable Antibody | ELISA $K_D$ (nM) | ELISA masking efficiency |
|---|---|---|
| EP01-2014 | 36 | 99 |
| EP02-2014 | 7 | 21 |
| EP03-2014 | 11 | 31 |
| EP04-2014 | 12 | 34 |
| EP05-2014 | 17 | 50 |
| EP06-2014 | 1 | 3 |
| EP07-2014 | 12 | 38 |
| EP08-2014 | 15 | 48 |

(continued)

| Anti-EpCAM Activatable Antibody | ELISA $K_D$ (nM) | ELISA masking efficiency |
|---|---|---|
| EP09-2014 | 22 | 67 |
| EP 10-2014 | 15 | 42 |
| EP11-2014 | 21 | 59 |
| EP12-2014 | 16 | 46 |
| EP01-1-2014 | 53 | 81 |
| EP01-2-2014 | 46 | 100 |
| EP01-3-2014 | 52 | 86 |
| EP01-3014 | 35 | 100 |
| EP02-3014 | 7 | 19 |
| EP03-3014 | 9 | 25 |
| EP04-3014 | 11 | 31 |
| EP05-3014 | 16 | 44 |
| EP06-3014 | 1 | 4 |
| EP07-3014 | 10 | 27 |
| EP08-3014 | 13 | 37 |
| EP09-3014 | 18 | 52 |
| EP10-3014 | 13 | 35 |
| EP11-3014 | 20 | 53 |
| EP12-3014 | 14 | 38 |
| EP01-1-3014 | 59 | 76 |
| EP01-2-3014 | 46 | 102 |
| EP01-3-3014 | 64 | 80 |
| EP101-3014 (1565-Y) | 389 | 930 |
| EP102-3014 (1565-Y) | 72 | 173 |
| EP103-3014 (1565-Y) | 140 | 335 |
| EP104-3014 (1565-Y) | 126 | 302 |
| EP105-3014 (1361-H) | 201 | 343 |
| EP106-3014 (1361-H) | 348 | 592 |
| EP107-3014 (1361-H) | 147 | 250 |
| EP108-3014 (1361-H) | 58 | 99 |
| EP109-3014 (1361-H) | 512 | 872 |
| EP110-3014 (1361-H) | 336 | 571 |

**Table 22. Anti-EpCAM Activatable Antibodies Masking Efficiencies.**

| Anti-EpCAM Activatable Antibody | ELISA $K_D$(nM) | ELISA masking efficiency |
|---|---|---|
| EP01-3014 (1565-Y) | 0.66 | 1 |
| EP02-3014 (1565-Y) | 34.5 | 67 |
| EP03-3014 (1565-Y) | 64.4 | 125 |
| EP04-3014 (1565-Y) | 26.9 | 52 |

(continued)

| Anti-EpCAM Activatable Antibody | ELISA $K_D$(nM) | ELISA masking efficiency |
|---|---|---|
| EP05-3014 (1565-Y) | 8.6 | 17 |
| EP07-3014 (1565-Y) | 37.6 | 73 |
| EP11-3014 (1565-Y) | 38.0 | 74 |
| EP101-3014 (1565-Y) | 388.8 | 756 |
| EP102-3014 (1565-Y) | 72.5 | 141 |
| EP103-3014 (1565-Y) | 140.2 | 272 |
| EP104-3014 (1565-Y) | 126.4 | 246 |
| EP01-3014 (1361-H) | 18.2 | 32 |
| EP02-3014 (1361-H) | 0.44 | 1 |
| EP03-3014 (1361-H) | 4.0 | 7 |
| EP04-3014 (1361-H) | 1.9 | 3 |
| EP05-3014 (1361-H) | 0.46 | 1 |
| EP07-3014 (1361-H) | 1.5 | 3 |
| EP11-3014 (1361-H) | 30.5 | 54 |
| EP105-3014 (1361-H) | 201 | 358 |
| EP106-3014 (1361-H) | 348 | 619 |
| EP107-3014 (1361-H) | 147 | 261 |
| EP108-3014 (1361-H) | 58 | 103 |
| EP109-3014 (1361-H) | 512 | 911 |
| EP110-3014 (1361-H) | 336 | 597 |
| EP107-1-3014 (1361-H) | 31 | 87 |
| EP107-2-3014 (1361-H) | 44 | 122 |
| EP107-3-3014 (1361-H) | 44 | 123 |

As depicted in Tables 21 and 22, these exemplary results demonstrated that the anti-human EpCAM activatable antibodies showed a range of masking efficiencies relative to an unmasked anti-EpCAM antibody.

**Example 8: Binding affinity of anti-EpCAM23Gv4.2 Activatable Antibodies, Antibody-Drug Conjugates, and Activatable antibody-Drug Conjugates**

The binding affinities of activatable antibodies and activatable antibody-drug conjugates ("AADC") comprising huEpCAM23Gv4.2 and one of 7 different masks were evaluated by flow cytometry. In particular, activatable antibodies and activatable antibody drug conjugates were generated using the antibody huEpCAM23Gv4.2, the enzyme sensitive substrate 3014, and a mask selected from Ep1-2, Ep-2, Ep03, Ep04, Ep05, Ep07 and Ep11. activatable antibodies comprising a mask linked to the antibody by a non-cleavable peptide moiety ("NSUB" or "Non-substrate") were also generated for use as a negative control. NSUB is not a substrate for proteases, and masks cannot be removed from a Activatable antibody comprising NSUB. Accordingly, activatable antibodies comprising NSUB were expected to remain inactive even after treatment with uPA.

*In vitro* EpCAM Activatable antibody/AADC Activation

To activate the substrated EpCAM activatable antibodies in vitro, a desired amount of activatable antibody was prepared in PBS at 1mg/ml, and uPA was added to the solution to a final concentration of 1 μM (based on protein concentration). The solution was then incubated at 37°C in a humidified 5% CO2 incubator overnight.

EpCAM Activatable antibody Binding Affinity

The binding affinity of EpCAM activatable antibodies to HSC2 cells was compared to that of the parent antibody huEpCAM23Gv4.2 and between activatable antibodies in the activated and non-activated forms. The activatable antibodies were activated as described above. Flow cytometry binding assays were carried out and analyzed as described in Example 2 using secondary Alexa Flour plus 488 conjugated goat-anti-human antibodies. As shown in **FIG. 6A,** activatable antibodies without activation had very poor binding to the cells, with an apparent Kd > $1X10^{-7}$ M, while the activated activatable antibodies retained binding affinity at a level similar to that of the parent antibody. Further tests were performed for 5 of the activatable antibodies using cyno primary kidney cells, and similar results were observed (see **FIG. 6B**). The apparent Kd values of the activated versus non-activated activatable antibodies were different by more than 10 fold in both HSC2 and cyno primary kidney epithelial cells.

Binding affinity of sSPDB-DM4 and DGN549 conjugated with anti-huEpCAM23Gv4.2 antibody and with anti-huEpCAM23Gv4.2-Ep05-3014 Activatable antibody

The binding affinities of the sSPDB-DM4 and DGN549 ADC conjugates of huEpCAM23Gv4.2 and the activated AADCs of huEpCAM23Gv4.2 substrated with 3014/NSUB and various masks were assayed using HSC2 cells. The activatable antibodies were activated as described above. Flow cytometry binding assays were carried out and analyzed as described in Example 2 using secondary Alexa Flour plus 488 conjugated goat-anti-human antibodies. In particular, the binding affinities of the activated DGN549 AADCs substrated with 3014 were compared with that of the DGN549 ADC, and the activated sSPDB-DM4 AADCs substrated with 3014 were compared with the sSPDB-DM4 ADC and with their NSUB form counterparts. The binding curves for the sSPDB-DM4 conjugates are provided in **FIG. 7A,** and the binding curves for the DGN549 conjugates are provided in **FIG. 7B.** As shown in **FIGs. 7A** and **7B,** the activated AADCs had similar binding to HSC2 cells as the corresponding ADCs. In contrast, the NSUB AADCs exhibited poor binding to the cells. These data demonstrate that the activatable antibody's binding ability to the cells can be restored by treatment with uPA, which removes the mask from the activatable antibody via the 3014 substrate but not via the NSUB substrate.

**Example 9: Binding affinity of sSPDB-DM4, DM21 and DGN549 conjugated with huEpCAM23Gv4.2-Ep05-3014 and huEpCAM23Gv4.2-Ep05-2014**

Additional activatable antibodies comprising huEpCAM23Gv4.2 with the Ep05 mask were made with another substrate, 2014. AADCs were generated for further evaluation by conjugating sSPDB-DM4, DM21L, and DGN549 to huEpCAM23Gv4.2-Ep05-3014 and by conjugating sSPDB-DM4 and DM21L to huEpCAM23Gv4.2-Ep05-2014. The binding affinity of the conjugates to HSC2 cells was assayed in both activated and non-activated forms, and the affinity of the AADCs were compared to both the corresponding activatable antibodies and wild type antibody. Flow cytometry binding assays were carried out and analyzed as described in Example 2 using secondary PE-conjugated goat-anti-human antibodies. Activatable antibodies and AADCs were activated as described in Example 8. The binding affinity of huEpCAM23Gv4.2-Ep05-2014-sSPDB-DM4, huEpCAM23Gv4.2-Ep05-3014-sSPDB-DM4 and huEpCAM23Gv4.2-Ep05-3014-DGN549 is shown in **FIGs. 8A, 8B and 8C,** respectively. The binding affinity of huEpCAM23Gv4.2-Ep05-3014-DM21L and huEpCAM23Gv4.2-Ep05-2014-DM21L is shown in **FIG. 8D.** The activated Activatable antibodies and AADCs had similar binding affinity to HSC2 cells as the wild type antibody, while the inactivated forms did not bind well to the cells. These results are consistent with the results described in Examples 4-6 and 8.

**Example 10: Epitope Mapping**

The human CD326 antigen, EpCAM (epithelial cell adhesion molecule), is composed of 314 amino acids, containing a 265-amino acid extra-cellular domain, a 23-amino acid transmembrane domain, and a cytoplasmic tail of 26 amino acids. The extracellular domain can be further divided into three domains (D1, D2 and D3). The extracellular domain contains two cysteine rich epidermal growth factor like (EGF-like) repeats, which include a first domain comprising a region from the glutamine at position 24 of the mature protein *(i.e.,* prior to signal peptide cleavage) to the cysteine at position 59 (see SEQ ID NO:2), and a second domain comprising a region from cysteine at position 66 to the cysteine at position 135 (see SEQ ID NO:3). Then, in tandem with the first two domains, there is also a cysteine free third domain (D3) which includes amino acid residues 136-243 (see SEQ ID NO:4).

| | |
|---|---|
| >NP_002345.2 EpCAM D1 domain | QEECVCENYKLAVNCFVNNNRQCQCTSVGAQNTVIC (SEQ ID NO:2) |

(continued)

| | |
|---|---|
| EpCAM D2 domain | CLVMKAEMNGSKLGRRAKPEGALQNNDGLYDAADCDESGLFKAKQ CNGTSMCWCVNTAGVRRTDKDTEITC (SEQ ID NO:3) |
| EpCAM D3 domain | SERVRTYWIIIELKHKAREKPYDSKSLRTALQKEITTRYQLDPKFITSILY ENNVITIDLVQNSSQKTQNDVDIADVAYYFEKDVKGESLFHSKKMDLT VNGEQLDLDPGQTLIYYVDEKAPEFSMQGLK (SEQ ID NO:4) |

Epitopes for the humanized EpCAM antibody Gv4.2 were mapped by engineering chimeric proteins utilizing combinations of the extracellular domains of human and mouse EpCAM. Because human and mouse EpCAM share over 82% amino acid sequence identity and 85% similarity (*see* **FIG. 9**), the topology of the chimeric proteins should remain intact.

EpCAM chimeric Variants Cloning and Expression

The extracellular region of human EpCAM (residues 1-265) was codon optimized, synthesized and cloned in-frame into a vector (pGSmuFc2ANL) containing the mouse IgG2a Fc region utilizing HindIII and BamHI restriction sites at Genscript. Similarly, other expression vectors containing various chimeric variants of the human/mouse EpCAM extracellular domain were synthesized by replacing residues corresponding to human EpCAM domain D1 (24-59), domain D2 (66-135), domain D3 (136-265) or a combination thereof with corresponding mouse residues. **FIGs. 10A and 10B** show the alignment of the extracellular region of the various chimerized variants. These expression constructs were transiently produced in suspension adapted HEK-293T cells using PEI as a transfection reagent in shake flasks. The transfections were incubated for one week, harvested, and the filtered supernatants purified using a combination of protein A and CHT chromatography essentially using procedures described in Example 1.

Antibody Binding to Various EpCAM Constructs

The humanized EpCAM antibody huEpCAM23Gv4.2 was tested in an enzyme-linked immunosorbent assay (ELISA) format for binding to the EpCAM proteins described above. Briefly, each mFc-tagged EpCAM protein was purified using a combination of protein A and CHT chromatography essentially as described in Example 1. Each mFc-tagged EpCAM protein was diluted to 0.5 ug/mL in 50 mM sodium bicarbonate buffer pH 9.6, and 100 μL was added to each well. After a 16 hr incubation at 4°C, the plates were washed with Tris-buffered saline with 0.1% Tween-20 (TBST), then blocked with 200 μL blocking buffer (TBS with 1% BSA). Next, 100 μL of primary antibody, huEpCAM23Gv4.2 serially diluted in blocking buffer, was added in duplicate to the ELISA wells and incubated at room temperature for 1 hr. The plates were then washed 3 times with TBST before adding 100 μL of anti-human IgG (H+L)-HRP to each well. The plates were again incubated for 1 hr at room temperature followed by three washes with TBST. Finally, 100 μL of TMB one component HRP microwell substrate was added to each well and incubated for 5 min. The reaction was stopped by adding 100 μL stopping solution and absorbances were read at 450 nm in a multiwell plate reader. OD450 was plotted against the antibody concentration in a semi-log plot. A dose-response curve was generated by non-linear regression and the $EC_{50}$ value of each curve was calculated using GraphPad Prism v6.

Binding of the huEpCAM23Gv4.2 antibody to the chimeric EpCAM proteins was evaluated in comparison to the wild type EpCAM. **FIG. 11** demonstrates that the huEpCAM23Gv4.2 antibody binds to both EpCAM-D2 (66-135) and EpCAM-D3 (136-265) with similar affinities as to that of the wild type EpCAM. Conversely, the huEpCAM23Gv4.2 antibody does not bind to the EpCAM-D1 and D1/D2 constructs and binding is all but eliminated for the chimeric protein EpCAM-D1(24-59) construct. These results indicate that the epitope of the huEpCAM23Gv4.2 antibody is located primarily within the D1 (24-59) domain of EpCAM.

## Example 11: Preparation of EpCAM Antibody Drug Conjugates and Activatable antibody Drug Conjugates

Preparation of huEpCAM23Gv4.2-sulfo-SPDB-DM4 conjugates

The molar concentration of huEpCAM23Gv4.2, sulfo-SPDB and DM4 were calculated according to Beer's law using the UV/Vis absorbance values at 280, 343 and 412nm and the extinction coefficients respectively. The linker concentration was determined by reacting the linker with 50mM DTT in 50mM potassium phosphate buffer pH 7.5 and measuring thiopyridine release at 343nm. The drug concentration is determined by reacting DM4 with 10mM DTNB [5,5-dithiobis-(2-nitrobenzoic acid)] in 50mM potassium phosphate buffer at pH 7.5 and measuring absorbance at 412nm.

Prior to antibody conjugation, sulfo-SPDB-DM4 in-situ mixture was prepared by reacting 5.0mM sulfo-SPDB with 6.3mM DM4 in 30% aqueous [50mM EPPS pH 8.0 (4-(2-hydroxylethyl)-piperazinepropanesulfonic acid)] and 70% organic [(N-N-dimethylacetamide, DMA, SAFC)] at 20°C for 90min. During the conjugation reaction, a solution of 8-10 mg/mL of antibody was reacted with 5 to 6 -fold molar excess of sulfo-SPDB-DM4 over antibody in 50 mM EPPS pH8.0 with 8% DMA (v/v), for 15-20 hours at 25°C. The reaction was purified into 10 mM Histidine, 250 mM Glycine, 1% Sucrose, and 0.01% Tween-20, pH 5.0 formulation buffer using NAP desalting columns and filtered through a syringe filter with a 0.22 μm PVDF membrane.

The molar ratio of DM4 conjugated to antibody (DAR) and the percentage of unconjugated maytansinoid species were determined as described below. The purified conjugate was found to have 3.7 mol DM4/mol antibody by UV-Vis, 95% monomer by SEC, and below 1 % free drug by HPLC Hisep column analysis.

DAR was determined by measuring the UV/Vis absorbance at 252 and 280 nm and calculating the [Ab] and [DM4] using binomial equations that account for the contribution of each component. The amount of unbound maytansinoid present in the final huEpCAM23Gv4.2-sulfo-SPDB-DM4 conjugates was calculated from the resulting peak areas observed in samples analyzed via HISEP column (25 cm x 4.6 mm, 5 μm). The percent free maytansinoid (% FM) present in the conjugate sample was calculated using the following equation: % Free Maytansinoid = (Reverse-phase PA 252 due to DM4) / (Reverse-phase PA 252 due to DM4 + Flow through PA 252 due to DM4) x 100%.

Preparation of huEpCAM23Gv4.2 Ep05-3014-sulfo-SPDB-DM4 AADC conjugate

Prior to antibody conjugation, sulfo-SPDB-DM4 in-situ mixture was prepared by reacting 5.0mM sulfo-SPDB with 6.3mM DM4 in 30% aqueous [50mM EPPS pH 8.0 (4-(2-hydroxylethyl)-piperazinepropanesulfonic acid)] and 70% organic [(N-N-dimethylacetamide, DMA, SAFC)] at 20°C for 90min. During the conjugation reaction, a solution of 8-10 mg/mL of antibody was reacted with 5 to 6 -fold molar excess of sulfo-SPDB-DM4 over antibody in 50 mM EPPS pH8.0 with 8% DMA (v/v), for 4-5 hours at 25°C. The reaction was purified into 10 mM Histidine, 250 mM Glycine, 1% Sucrose, and 0.01% Tween-20, pH 5.0 formulation buffer using NAP desalting columns and filtered through a syringe filter with a 0.22 μm PVDF membrane. The purified conjugate was found to have 3.6 mol DM4/mol activatable antibody by UV-Vis, 98% monomer by SEC, and below 1% free drug by HPLC Hisep column analysis.

Preparation of huEpCAM23Gv4.2 Ep05-2014-sulfo-SPDB-DM4 AADC conjugate

Prior to antibody conjugation, sulfo-SPDB-DM4 in-situ mixture was prepared by reacting 5.0mM sulfo-SPDB with 6.3mM DM4 in 30% aqueous [50mM EPPS pH 8.0 (4-(2-hydroxylethyl)-piperazinepropanesulfonic acid)] and 70% organic [(N-N-dimethylacetamide, DMA, SAFC)] at 20°C for 90min. During the conjugation reaction, a solution of 8-10 mg/mL of antibody was reacted with 5 to 6 -fold molar excess of sulfo-SPDB-DM4 over antibody in 50 mM EPPS pH8.0 with 8% DMA (v/v), for 4-5 hours at 25°C. The reaction was purified into 10 mM Histidine, 250 mM Glycine, 1% Sucrose, and 0.01% Tween-20, pH 5.0 formulation buffer using NAP desalting columns and filtered through a syringe filter with a 0.22 μm PVDF membrane. The purified conjugate was found to have 3.6 mol DM4/mol activatable antibody by UV-Vis, 98% monomer by SEC, and below 1 % free drug by HPLC Hisep column analysis.

Preparation of huEpCAM23Gv4.2 Ep05-NSUB-sulfo-SPDB-DM4 AADC conjugate

Prior to antibody conjugation, sulfo-SPDB-DM4 in-situ mixture was prepared by reacting 5.0mM sulfo-SPDB with 6.3mM DM4 in 30% aqueous [50mM EPPS pH 8.0 (4-(2-hydroxylethyl)-piperazinepropanesulfonic acid)] and 70% organic [(N-N-dimethylacetamide, DMA, SAFC)] at 20°C for 90min. During the conjugation reaction, a solution of 8-10 mg/mL of antibody was reacted with 5 to 6 -fold molar excess of sulfo-SPDB-DM4 over antibody 50 mM EPPS pH8.0 with 8% DMA (v/v), for 4-5 hours at 25°C. The reaction was purified into 10 mM Histidine, 250 mM Glycine, 1% Sucrose, and 0.01% Tween-20, pH 5.0 formulation buffer using NAP desalting columns and filtered through a syringe filter with a 0.22 μm PVDF membrane. The purified conjugate was found to have 3.8 mol DM4/mol activatable antibody by UV-Vis, 99% monomer by SEC, and below 1 % free drug by HPLC Hisep column analysis.

Preparation of huEpCAM23Gv4.2-GMBS-DM21L conjugates

The molar concentration of huEpCAM23Gv4.2, sulfo-GMBS and DM21 were calculated according to Beer's law using the UV/Vis absorbance values at 280, 343 and 412nm and extinction coefficients respectively. The linker concentration was determined by reacting the linker with 50mM DTT in 50mM potassium phosphate buffer pH 7.5 and measuring thiopyridine release at 343nm. The drug concentration is determined by reacting DM21 with 10mM DTNB [5,5-dithiobis-(2-nitrobenzoic acid)] in 50mM potassium phosphate buffer at pH 7.5 and measuring absorbance at 412nm.

Prior to conjugation, sulfo-GMBS-DM21 in-situ mixture was prepared by reacting 3mM sulfo-GMBS with 3.9mM

DM21 in 60/40 (v/v) DMA and succinate buffer pH 5.0 respectively. The conjugation was carried out with 6-7 linker excess of sulfo-GMBS-DM21 over antibody at 5mg/mL in 60mM 4-(2-Hydroxyethyl)-1-piperazinepropanesulfonic acid (EPPS) pH 8.5 with 15% DMA (v/v). After a 4-5 hour incubation at 25°C, the reaction was purified into 10 mM Histidine, 250 mM Glycine, 1% Sucrose, and 0.01% Tween-20, pH 5.0 using NAP desalting columns and filtered through a 0.22 μm PVDF membrane filter.

The molar ratio of DM21 conjugated to antibody (DAR) and the percentage of unconjugated maytansinoid species were determined as described below. The purified conjugate was found to have 3.7 mol DM21/mol antibody by UV-Vis, 95% monomer by SEC, and below 1 % free drug by HPLC Hisep column analysis.

The molar ratio of DM21 conjugated to antibody (DAR) was determined by measuring the UV/Vis absorbance at 252 and 280 nm and calculating the [Ab] and [DM21] using binomial equations that account for the contribution of each component. The amount of unbound maytansinoid present in the final huEpCAM23Gv4.2-GMBS-DM21 conjugates was calculated from the resulting peak areas observed in samples analyzed via HISEP column (25 cm x 4.6 mm, 5 μm). The percent free maytansinoid (% FM) present in the conjugate sample was calculated using the following equation: % Free Maytansinoid = (Reverse-phase PA 252 due to DM21) / (Reverse-phase PA 252 due to DM21 + Flow through PA 252 due to DM21) x 100%.

<u>Preparation of huEpCAM23Gv4.2 Ep05-3014-GMBS-DM21L conjugates</u>

Prior to conjugation, sulfo-GMBS-DM21 in-situ mixture was prepared by reacting 3mM sulfo-GMBS with 3.9mM DM21 in 60/40 (v/v) DMA and succinate buffer pH 5.0 respectively. The conjugation was carried out with 6-7 linker excess of sulfo-GMBS-DM21 over activatable antibody at 5mg/mL in 60mM 4-(2-Hydroxyethyl)-1-piperazinepropanesulfonic acid (EPPS) pH 8.5 with 15% DMA (v/v). After a 4-5 hour incubation at 25°C, the reaction was purified into 10 mM Histidine, 250 mM Glycine, 1% Sucrose, and 0.01% Tween-20, pH 5.0 using NAP desalting columns and filtered through a 0.22 μm PVDF membrane filter. The purified conjugate was found to have 3.6 mol DM21/mol activatable antibody by UV-Vis, 99% monomer by SEC, and below 1 % free drug by HPLC Hisep column analysis.

<u>Preparation of huEpCAM23Gv4.2 Ep05-2014-GMBS-DM21L conjugates</u>

Prior to conjugation, sulfo-GMBS-DM21 in-situ mixture was prepared by reacting 3mM sulfo-GMBS with 3.9mM DM21 in 60/40 (v/v) DMA and succinate buffer pH 5.0 respectively. The conjugation was carried out with 6-7 linker excess of sulfo-GMBS-DM21 over activatable antibody at 5mg/mL in 60mM 4-(2-Hydroxyethyl)-1-piperazinepropanesulfonic acid (EPPS) pH 8.5 with 15% DMA (v/v). After a 4-5 hour incubation at 25°C, the reaction was purified into 10 mM Histidine, 250 mM Glycine, 1% Sucrose, and 0.01% Tween-20, pH 5.0 using NAP desalting columns and filtered through a 0.22 μm PVDF membrane filter. The purified conjugate was found to have 3.6 mol DM21/mol activatable antibody by UV-Vis, 99% monomer by SEC, and below 1 % free drug by HPLC Hisep column analysis.

<u>Preparation of huEpCAM23Gv4.2-DGN549 conjugates (lysine linked)</u>

The molar concentration of huEpCAM23Gv4.2 and $SO_3$-DGN549-NHS were calculated according to Beer's law using the UV/Vis absorbance values at 280 and 330nm and their extinction coefficients respectively. The DGN549-NHS drug stock was made in DMA and diluted in ethanol for measuring 330nm absorbance. The DGN549-NHS sulfonation reaction was carried out by adding 5-10 molar excess of $NaHSO_3$ over DGN549-NHS in 90% DMA and 10% 50mM succinate pH 5.0 at room temperature for 3-4 hours.

The huEpCAM23Gv4.2-DGN549 conjugates were made by reacting 4-5 molar excess of sulfonated DGN549-NHS reagent (D2) over antibody at 2mg/mL in 50mM EPPS pH 8.0, 15% DMA (v/v). The reaction was carried out at 25°C for 3-5 hours. The reaction mixture was purified via Sephadex G-25 columns equilibrated in a buffer containing 10 mM Histidine, 250 mM Glycine, 1% Sucrose, and 0.01% Tween-20, 50 μM sodium bisulfite, pH 5.0, and filtered over a 0.22 um PVDF filter. The molar ratio of DGN549 per antibody (DAR) and the percentage of total free DGN549 species were determined as described below. huEpCAM23Gv4.2-DGN549 conjugate with 2.7 DGN549 molecules per antibody was obtained with <1% present as unconjugated DGN549.

For DGN conjugates, the DAR was determined by measuring the UV/Vis absorbance at 280 and 330 nm and calculating the [Ab] and [DGN549] according to Beer's law. To determine the amount of unconjugated DGN549, the conjugate was passed through a dual-column system (TOSOH SEC QC-PAK GFC 300 and Agilent Zorbax C18 columns) to calculate total AUC for free DGN549. The free DGN549 was reported as a ratio of unconjugated DGN549 over total DGN549.

Preparation of huEpCAM23Gv4.2-Ep05-3014-DGN549 conjugates (lysine linked)

The huEpCAM23Gv4.2-DGN549 conjugates were made by reacting 4-5 molar excess of sulfonated DGN549-NHS reagent (D2) over activatable antibody at 2mg/mL in 50mM EPPS pH 8.0, 15% DMA (v/v). The reaction was carried out at 25°C for 3-5 hours. The reaction mixture was purified via Sephadex G-25 columns equilibrated in a buffer containing 10 mM Histidine, 250 mM Glycine, 1% Sucrose, and 0.01% Tween-20, 50 μM sodium bisulfite, pH 5.0, and filtered over a 0.22 um PVDF filter. Conjugates with 2.6 DGN549 molecules per activatable antibody was obtained with <1% present as unconjugated DGN549.

Preparation of huEpCAM23Gv4.2-Ep05-2014-DGN549 conjugates (lysine linked)

The huEpCAM23Gv4.2-DGN549 conjugates were made by reacting 4-5 molar excess of sulfonated DGN549-NHS reagent (D2) over activatable antibody at 2mg/mL in 50mM EPPS pH 8.0, 15% DMA (v/v). The reaction was carried out at 25°C for 3-5 hours. The reaction mixture was purified via Sephadex G-25 columns equilibrated in a buffer containing 10 mM Histidine, 250 mM Glycine, 1% Sucrose, and 0.01% Tween-20, 50 μM sodium bisulfite, pH 5.0, and filtered over a 0.22 um PVDF filter. Conjugates with 2.8 DGN549 molecules per activatable antibody was obtained with <1% present as unconjugated DGN549.

Preparation of huEpCAM23Gv4.2-Ep05-NSUB-DGN549 conjugates (lysine linked)

The huEpCAM23Gv4.2-DGN549 conjugates were made by reacting 4-5 molar excess of sulfonated DGN549-NHS reagent (D2) over activatable antibody at 2mg/mL in 50mM EPPS pH 8.0, 15% DMA (v/v). The reaction was carried out at 25°C for 3-5 hours. The reaction mixture was purified via Sephadex G-25 columns equilibrated in a buffer containing 10 mM Histidine, 250 mM Glycine, 1% Sucrose, and 0.01% Tween-20, 50 μM sodium bisulfite, pH 5.0, and filtered over a 0.22 um PVDF filter. Conjugates with 2.9 DGN549 molecules per activatable antibody was obtained with <1% present as unconjugated DGN549.

Preparation of huEpCAM23Gv4.2-C442-DGN549 conjugates

huEpCAM23Gv4.2-C442 antibody bearing two unpaired cysteine residues in the reduced state was prepared using standard procedures. The conjugation reaction was carried out using this intermediate at a final antibody concentration of 1 mg/mL in PBS containing 5 mM EDTA, pH 6.0 and 10 molar equivalents of Mal-DGN549 (or D5, as a 8.2 mM stock solution in DMA) with 2% v/v DMA and 38% v/v propylene glycol. The conjugation reaction was carried out for 15-20 hours in a water bath at 25 °C. The conjugate was purified into 10 mM Histidine, 250 mM Glycine, 1% Sucrose, 0.01% Tween-20 and 50 μM sodium bisulfite, pH 5.0 buffer via Sephadex G-25columns, and filtered through a 0.22 μm PVDF syringe filter. Conjugates with 2.0 DGN549 molecules per antibody were obtained with <1% present as unconjugated DGN549.

**Example 12. *In vitro* Cytotoxicity of EpCAM Antibody and EpCAM Activatable Antibody Conjugates**

The ability of EpCAM-targeting antibody-drug conjugates (ADCs) and Activatable antibody-drug conjugates (AADCs) to kill tumor cells was measured using in vitro cytotoxicity assays. To evaluate the specific killing, the potency of the anti-EpCAM ADC was measured with and without blocking antibody. Briefly, target cells were plated at 2000 cells per well in 100 μL of complete cell culture media recommended by the ATCC. 50ul/well of media only (non-blocking condition) or naked EpCAM antibody (for blocking) at 500 nM was added to the solution. Conjugates were serially diluted in complete cell culture media and 50 μL of each dilution was added per well. The final concentration of the conjugates typically ranged from 1.5 x $10^{-13}$ M to 5 x $10^{-8}$ M. The cells were then incubated at 37°C in a humidified 5% CO2 incubator for 5 to 6 days, and the viability of the remaining cells was determined by colorimetric WST-8 assay. WST-8 is reduced by dehydrogenases in living cells to an orange formazan product that is soluble in tissue culture medium, and the amount of formazan produced is directly proportional to the number of living cells. WST-8 was added to 10% of the final volume and plates were incubated at 37°C in a humidified 5% CO2 incubator for an additional 2 to 4 hours. Plates were analyzed by measuring the absorbance at 450 nm (A450) in a multiwell plate reader, and background A450 absorbance of wells with media and WST-8 only was subtracted from all values. The percent viability was calculated by dividing each treated sample value by the average value of wells with untreated cells. Percent viability = 100* (A450 treated sample - A450 background)/ (A450 untreated sample - A450 background). The percent viability value was plotted against the antibody concentration in a semi-log plot for each treatment, a dose-response curve was generated by non-linear regression, and the $EC_{50}$ value of each curve was calculated using GraphPad Prism 6.

EpCAM ADC *in vitro* cytotoxic activity in NSCLC, CRC, and HNC

The in vitro cytotoxicity of the huEpCAM23Gv4.2-sSBDP-DM4 conjugate was evaluated with and without EpCAM antibody blocking in the EpCAM-expressing NSCLC cell lines H1568, H292, and H2110, the CRC cell line LoVo, and the HNC cell line Detroit562. The results from the cytotoxicity assays are shown in **FIGs. 12A-12E.** In particular, huEpCAM23Gv4.2-sSBDP-DM4 had specific cell killing in all five cell lines tested. The EC50 values for the huEpCAM23Gv4.2-sSPDB-DM4 conjugate without blocking were 0.06 nM in H1568 cells, 0.07 nM in H292 cells, 0.09 nM in H2110 cells, 0.02 nM in Lovo cells and 0.03 nM in Detroit562 cells. In contrast, EpCAM antibody blocking of the same conjugate resulted in cell killing with an EC50 value of 3.9 nM in H1568 cells, 2.2 nM in H292 cells, 2.8 nM in H2110 cells, 20 nM in Lovo cells, and 1.4 nM in Detroit562 cells.

In addition, DGN549 was conjugated to huEpCAM23Gv4.2 at the Lys and site specific C442 sites, respectively. The conjugates were evaluated and compared to a non-targeting isotype control IgG1 conjugate, chKTI-DGN549, with H2110 cells. As shown in **FIG. 12F,** both conjugates, huEpCAM23Gv4.2-lys-DGN549 and -C442-DGN549, had similar EC50 and were >100 fold more potent than chKTI-DGN549.

In additional experiments, DM21 was conjugated to huEpCAM23Gv4.2. The potency of the huEpCAM23Gv4.2-DM21L conjugate was assayed with and without huEpCAM23Gv4.2 blocking in four NSCLC cell lines and one head-and-neck cell line. The results shown in **FIGs. 12G-12K** indicate the huEpCAM23Gv4.2-DM21L conjugate is potent to all five of the cell lines tested and that the cytotoxicity can be blocked by addition of the parental antibody.

*In vitro* cytotoxic activity EpCAM AADC in NSCLC and ovarian cancer cell lines

To expand upon the *in vitro* cytotoxicity activity observed with the huEpCAM23Gv4.2-sSPDB-DM4, DM21 and DGN549 conjugates, EpCAM AADCs were prepared with the same payloads. In particular, sSPDB-DM4 and DM21L were conjugated to the activatable antibodies huEpCAM-Ep05-3014 and huEpCAM-Ep05-2014, and DGN549 was conjugated to huEpCAM23Gv4.2-3014. The activity of the DM4, DM21 and DGN549 conjugates was assayed in the NSCLC cell lines Calu3, EBC-1 and H2110. In addition, DM21L AADCs were tested in Detroit562 cells, and DM4 and DGN549 AADCs in OV90 cells, respectively. The potency of the AADCs was evaluated in activated and non-activated forms and compared to their counterpart EpCAM-targeted ADC or to the non-targeting isotype control IgG1 conjugate, chKTI ADC. The *in vitro* AADC activation was carried out as described in Example 8. The results from typical cytotoxicity assays are shown in **FIGs. 13-15.** In particular, the *in vitro* activity of the DM4 conjugates is shown in **FIGs. 13A-13D,** the *in vitro* activity of the DM21L conjugates is shown in **FIGs. 14A-14D,** and the *in vitro* activity of the DGN549 conjugates is shown in **FIGs. 15A-15D.** As expected, the activated AADCs had similar activity as the EpCAM-specific ADC, and the non-activated AADCs are much less potent to the cells. The EC50 values ranged from 0.06 to 0.46 nM for the sSPDB-DM4 ADC and from 0.08 nM to 1.4 nM for the activated sSPDB-DM4 AADC (see Table 23). The EC50 for the activated DM21 AADCs ranged from 0.3 to 2 nM (see Table 24). Additionally, the huEpCAM-DGN549 ADC and activated huEpCAM-Ep05-3014-DGN549 AADC were very potent, with EC50 values ranging from 0.006 to 0.02 nM for the ADC and from 0.017 to 0.06 nM for the AADC (see Table 25). These results show that a good specificity window is observed for both the ADC and AADC conjugates, suggesting that cytotoxicity is a result of anti-EpCAM antibody binding to target cells.

**Table 23. $EC_{50}$ and specificity window for cytotoxicity of sSPDB-DM4 conjugated with huEpCAM23Gv4.2 antibody and activatable antibodies in NSCLC cell lines, Calu3, EBC-1 and H2110, and ovarian cancer cell line OV90.**

| Cells | huEpCAM-sSPDB-DM4 ADC IC50 nM | chKTI-sSPDB-DM4 IC50 nM | specificity window | EP05-2014-DM4 + uPA IC50 nM | EP05-2014-DM4 no uPA IC50 nM | specificity window | EP05-3014-DM4 IC50 nm | EP05-3014-DM4 IC50 nm | specificity window |
|---|---|---|---|---|---|---|---|---|---|
| | | | | uPA | No uPA | | uPA | No uPA | |
| Calu3 | 0.46 | 25.3 | 55 | 0.31 | 5.6 | 18 | 1.4 | 34 | 24 |
| EBC-1 | 0.08 | 2.1 | 26 | 0.15 | 3.3 | 22 | 0.1 | 3.6 | 36 |
| H2110 | 0.094 | 1.3 | 13 | 0.13 | 2.9 | 22 | 0.14 | 3.5 | 25 |
| OV90 | 0.06 | 2.5 | 42 | 0.08 | 1.4 | 18 | 0.1 | 2.6 | 26 |

**Table 24. $EC_{50}$ and specificity window for cytotoxicity of huEpCAM23Gv4.2-Ep05-3014-DM21and huEpCAM23Gv4.2-Ep05-2014-DM21 in NSCLC cell lines, Calu3, EBC-1 and H2110, and HNC cancer cell line Detroit562.**

| Cells | EpCAM23Gv4.2-Ep05-3014-DM21 | | specificity window | EpCAM23Gv4.2-Ep05-2014-DM21 | | specificity window |
|---|---|---|---|---|---|---|
| | Non-activated | Activated | | Non-activated | Activated | |
| Calu3 | 39 | 2 | 20 | 33 | 0.8 | 41 |
| EBC-1 | 11 | 0.6 | 18 | 12 | 0.4 | 30 |
| H2110 | 27 | 1.1 | 25 | 26 | 0.4 | 65 |
| Detroit562 | 40 | 0.8 | 50 | 34 | 0.29 | 117 |

**Table 25. $EC_{50}$ and specificity window for cytotoxicity of huEpCAM23Gv4.2-DGN549 ADC and huEpCAM23Gv4.2-Ep05-3014-DGN549 in NSCLC cell lines, Calu3, EBC-1 and H2110, and ovarian cancer cell line OV90.**

| Cells | EpCAM-DGN549 ADC EC50 nM | chKTI-DGN549 EC50 nM | specificity window | Ep05-3014-DGN549 EC50 nM | Ep05-3014-DGN549 EC50 nM | specificity window |
|---|---|---|---|---|---|---|
| Lot#E | | | | uPA | No uPA | |
| Calu3 | 0.01 | 3.6 | 360 | 0.04 | 0.91 | 23 |
| EBC-1 | 0.004 | 13.1 | 3275 | 0.01 | 0.68 | 68 |
| H2110 | 0.02 | 21.1 | 1055 | 0.06 | 2 | 33 |
| OV90 | 0.006 | 6.3 | 1050 | 0.017 | 0.59 | 35 |

The in vitro potency of huEpCAM23Gv4.2-DGN549 AADCs comprising additional masks was also evaluated and compared to individual mask efficiencies. As shown in Table 26, the window of in vitro activity of huEpCAM23Gv4.2 AADCs correlates well with mask efficiency.

**Table 26. In vitro activity of anti-EpCAM AADCs compared to mask efficiency (ME).**

<table>
<tr><th rowspan="2">ADC/AADC</th><th rowspan="2">ME</th><th colspan="3">DGN549 conjugates</th></tr>
<tr><th colspan="2">IC50 (nM)</th><th>Specificity window</th></tr>
<tr><td>huEpCAMGv4.2-DGN549</td><td>-</td><td colspan="2">0.025</td><td rowspan="2">176</td></tr>
<tr><td>Non-targeting-DGN549 ADC</td><td>-</td><td colspan="2">4.4</td></tr>
<tr><td colspan="2"></td><td>Activated</td><td>Intact</td><td>Specificity window</td></tr>
<tr><td>huEpCAM23Gv4.2-Ep01-2-3014-DGN549</td><td>100</td><td>0.019</td><td>2.5</td><td>132</td></tr>
<tr><td>huEpCAM23Gv4.2-Ep11-3014-DGN549</td><td>53</td><td>0.018</td><td>1.6</td><td>89</td></tr>
<tr><td>huEpCAM23Gv4.2-Ep05-3014-DGN549</td><td>44</td><td>0.056</td><td>1.3</td><td>23</td></tr>
<tr><td>huEpCAM23Gv4.2-Ep04-3014-DGN549</td><td>31</td><td>0.076</td><td>1.7</td><td>23</td></tr>
<tr><td>huEpCAM23Gv4.2-Ep07-3014-DGN549</td><td>27</td><td>0.043</td><td>1</td><td>23</td></tr>
<tr><td>huEpCAM23Gv4.2-Ep03-3014-DGN549</td><td>25</td><td>0.04</td><td>0.61</td><td>15</td></tr>
<tr><td>huEpCAM23Gv4.2-Ep02-3014-DGN549</td><td>19</td><td>0.039</td><td>0.2</td><td>5</td></tr>
</table>

**Example 13: Activity of EpCAM ADCs and AADCs in CB17 SCID Mice Bearing NCI-H2110 Human NSCLC Xenografts**

The antitumor activity of varying doses of EpCAMG23v4.2-DGN549 and huEpCAMG23v4.2-s-SPDB-DM4 antibody-drug conjugates (ADC), as well as their derived activatable antibody-drug-conjugates (AADC) comprised of masked antibodies with either the 3014 or 2014 substrates, were evaluated in a series of in vivo studies. The studies were performed in 6-8 weeks old female CB17 SCID mice (CB17/lcr-PrdxSCID) bearing NCI-H2110 tumors, a human NSCLC sub-cutaneous xenograft model. Animals were obtained from Charles River Laboratories and showed no signs of disease or illness during the 5 day observation period before being assigned to a study.

NCI-H2110 cells were harvested from tissue culture with a consistent > 98% cell viability, determined by trypan blue exclusion. Each mouse was inoculated with $10^7$ NCI-H2110 cells in 0.1 mL of a 1:1 solution of serum free medium and Matrigel (SFM:Mat) by subcutaneous injection on the right flank, using a 27-gauge needle. Mice were randomized into treatment groups prior to administration of articles (test agents and vehicle control) based on their tumor volume (TV). The animals were administered articles the next day, based on individual body weight (BW). Articles were administered as a single i.v. bolus, using a 1.0 mL syringe fitted with a 27-gauge needle.

Tumor volume (TV) and body weight (BW) measurements were recorded in StudyDirector software twice per week. Tumor volume ($mm^3$) was estimated from caliper measurements, following the formula for the cylindrical volume, as: TV ($mm^3$) = (L x W x H)/2, where L, W and H are the respective orthogonal tumor length, width and height measurements (in mm). Body weights (g) of mice were used to follow body weight change (BWC) in the treated animals, which is expressed as percent of body weight at a given measurement time (BWt) compared to the pre-treatment, initial body weight (BWi), calculated as follows: % BWC = [(BWt / BWi) - 1] x 100.

The primary endpoints used to evaluate the efficacy of the treatments were tumor growth inhibition and tumor regressions. Tumor growth inhibition (TGI) is the ratio of the median TV of the treated group (T) at the time when the median TV of the control Placebo group (C) reaches a size close to 1000 $mm^3$, and is expressed as a percentage of the initial TV at time of randomization, using the formula: TGI = T/C x 100. According to NCI standards, a TGI ≤ 42% is the minimum level of anti-tumor activity (marked A for Active), a TGI < 10% is considered a high anti-tumor activity level (marked HA, for Highly Active), while a treatment with a TGI > 42% is considered inactive (IA). A mouse was defined to have a partial regression

(PR) when its TV was reduced by 50% or greater compared to the TV at time of treatment, complete tumor regression (CR) when no palpable tumor could be detected and be a tumor-free survivor (TFS) if tumor free at the end of the study (EoS).

A secondary end-point defining compound efficacy is tumor growth delay, as measured by LCK (log cell kill) activity and increased life span (ILS). LCK activity is calculated following the formula: LCK = (T-C)/(Td x 3.32), where T is the median survival of a group (days), TFS excluded, C is the median survival of the Control group (days), and Td is the Tumor doubling time (days), as determined on the Control (Vehicle) group's median TV through time. According to NCI standards, a LCK >2.8 defines the compound as highly active (++++), a LCK in the [0.7; 2.8] is active (3 levels defined), while a LCK< 0.7 defines inactivity (-). Increased life span (ILS) is expressed as a percentage of the median survival of the treated group (T) (all animals included) compared to the Control group (C), following the formula

ILS = (T-C)/C x 100. According to NCI standards an ILS ≥ 25% is defines a minimum level of activity, while an ILS ≥ 50% denotes high level activity.

Mice were euthanized if tumors exceeded 1000 mm3; if the animals lost more than 20% of their initial body weight (% BWC < -20%), which is a rough index of toxicity; if tumors become necrotic; or if mice became moribund at any point during the study.

*In vivo* Efficacy of huEpCAMG23v4.2-DGN549

Mice were inoculated and randomized 6 days post-inoculation (dpi) into groups of 6 mice. The mean tumor volumes (TV) for each group were between 118 and 122.1 $mm^3$. The study was ended at 116 days post inoculation (EOS = 116 dpi).

The treatment groups included a Placebo control group administered vehicle (200 μl/ms) and three huEpCAMG23v4.2-DGN549 ADC groups, administered at 0.5, 1.5 and 3 μg/kg respectively, with all of the doses based on drug concentration.

In this study, TGI was determined at 27 dpi, when the median TV of the control Placebo group reached 1070.4 $mm^3$.

The results of the study are shown in **FIG. 16.** All treatments were well tolerated at the indicated doses, and no body weight loss was observed. The ADC huEpCAM23Gv4.2-DGN549 was highly active (HA) at 3 μg/kg, with a TGI value of 1.8%, 6/6 PR, 3/6 CR and 1/6 TSF. Furthermore, this group also showed a LCK = 1.77, qualifying the treatment as active (++), and a 179% ILS (highly active), demonstrating good tumor growth delay. Tumor regressions in the 3 μg/kg regimen started at early time points following ADC administration and resulted in multiple partial regressions as early as 7 days post treatment. In particular, the 1.5 μg/kg dose was active, with a TGI value of 31.6%, a 1/6 PR, and an ILS of 46% (active). In contrast, the 0.5 μg/kg dose of the huEpCAM23Gv4.2-DGN549 was inactive. Accordingly, these data show that treatment with huEpCAM23Gv4.2-DGN549 induces a high incidence of tumor regressions in this tumor model and results in potent anti-tumor activity at doses as low as 1.5 μg/kg.

Efficacy of masked-EpCAM-DGN549 bearing the Ep02, Ep01-02, Ep11 and Ep04 masking moieties with the 3014 cleavable moiety

Mice were inoculated and randomized 6 days post-inoculation (dpi) into groups of 6 mice. The mean tumor volumes (TV) for each group were between 105.8 and 115.4 $mm^3$. The study was ended at 120 days post inoculation (EOS = 120 dpi).

The treatment groups included a Placebo control group administered vehicle (200 μl/ms) and four masked-huEpCAMG23v4.2-DGN549 AADC groups (i.e., AADCs bearing masks Ep02, Ep01-02, Ep11 and Ep04 linked to the 3014 substrate) administered at 3 and 5 μg/kg, based on drug concentration. A positive control group treated with the huEpCAM23Gv4.2-DGN549 ADC at 5 μg/kg was also included in this study.

In this study, TGI was determined at 25 dpi, when the median TV of the control Placebo (vehicle) group reached 1072.4 $mm^3$.

The results of the study are shown in **FIG. 17A.** All treatments were well tolerated at the indicated doses, and no body weight loss was observed in this study.

The ADC huEpCAM23Gv4.2-DGN549 was highly active (HA) at 5 μg/kg, with aTGI value of 0.3%, a 6/6 PR, a 6/6 CR, 6/6 animals tumor free at the end-of-study, and a 120 dpi, which resulted in 380% ILS.

The Ep01-02-3014-DGN549 showed minimal activity at 5 μg/kg, with 34.5% TGI and 48% ILS. The Ep11-3014-DGN549 was active at the 5 μg/kg dose, with 10.1% TGI, 1/6 PR, 0.89 LCK (+), and 78% ILS, but was inactive at a lower dose. The Ep02-3014-DGN549 AADC was active at 3 μg/kg (38.6% TGI, 1/6 PR) and highly active at 5 ug/kg (9.9% TGI, 2/6 PR, 66% ILS). The Ep04-3014-DGN549 AADC was active at 3 μg/kg (19.7% TGI, 60% ILS) and was highly active at 5 μg/kg (4.2% TGI), inducing multiple regressions (6/6 PR, 1/6 CR) and significantly increasing the group's lifespan (LCK = 1.48, 144% ILS). Accordingly, these data show that treatment with the Ep04-DGN549 AADC is highly efficient in this tumor model at a dose of 5 μg/kg, while treatment with Ep02- and Ep01-02- and Ep11- 3014-DGN549 AADCs shows less anti-tumor activity.

Efficacy of masked-EpCAM-DGN549 bearing the Ep03, Ep05, Ep07 and Ep04 masks with the 3014 cleavable moiety

Mice were inoculated and randomized 6 days post-inoculation (dpi) into groups of 6 mice. The mean tumor volumes (TV) for each group were between 98.5 and 104.8 $mm^3$. The study was ended at 118 days post inoculation (EOS = 118 dpi).

The treatment groups included a placebo Control group administered vehicle (200 μl/ms) and four masked-huEpCAMG23v4.2-DGN549 AADC groups (i.e., AADCs bearing masks Ep03, Ep05, Ep07 and Ep04 linked to the 3014 substrate) administered at 3 and 5 μg/kg, based on drug concentration. A negative control group administered an AADC bearing a non-cleavable Ep04 mask, Ep04-NSUB-DGN549, was also included, as was a positive control group treated with the huEpCAM23Gv4.2-DGN549 ADC at 5 μg/kg.

In this study, TGI was determined at 25 dpi, when the median TV of the control Placebo (vehicle) group reached 1293.4 $mm^3$.

The results of the study are shown in **FIG. 17B.** All treatments were well tolerated at the indicated doses, and no body weight loss was observed in this study.

The huEpCAM23Gv4.2-DGN549 ADC used as a positive control was highly active (HA) at 5 μg/kg, based on tumor growth inhibition (TGI = 0.0%), multiple regressions (6/6 PR, 6/6 CR and 6/6 tumor free animals at the end-of-study, 118 dpi) and overall tumor growth delay (372% ILS), consistent with previous results.

As expected, the non-cleavable Ep04-NSUB-DGN549 was inactive (TGI = 43.6%, LCK = 0.5), and induced no regressions, with a slight inhibition of tumor growth likely attributed to the payload rather than a specific, antigen-directed anti-tumor response. Consistent with previous results, Ep04-3014-DGN549 AADC administered at 5 μg/kg demonstrated activity, as defined by tumor growth inhibition (TGI = 3.2 %) and tumor growth delay (LCK = 2.37, +++, and 210% ILS), and induced multiple regressions (5/6 PR, 3/6 CR and 2/6 TFS).

The Ep03- and Ep07- 3014-DGN549 AADCs were active at 3μg/kg, with tumor growth inhibition rates of 21.7% and 35.9% respectively, and LCK values of 0.79 (+) and 44% ILS, but induced no regressions at this dose. At 5 μg/kg, both Ep03- and Ep07- 3014-DGN549 AADCs were highly active (TGI of 7.6% and 6.4% respectively, LCK = 1.69 (++), 94% ILS) and induced a few regressions (2/6 and 3/6 PR respectively).

The Ep05-3014-DGN549 AADC was active at 3 μg/kg (17.5% TGI, 1/6 PR, LCK = 1.4 (++) and 78% ILS), and was highly active at 5 ug/kg, similar to the Ep04-3014-DGN549 AADC. Specifically, the Ep05-3014-DGN549 AADC administered at 5 ug/kg demonstrated tumor growth inhibition to 3.7%, an LCK = 2.4 (+++), a 134% ILS, and multiple regressions (6/6 PR, 2/6 CR). Accordingly, these data show that treatment with Ep04- and Ep05-3014-EpCAM AADCs results in high activity in this model at 5 μg/kg. Both Ep04- and Ep05-3014-DGN549 induced multiple tumor regression events at this dose, and remained active at a lower dose of 3 μg/kg.

Efficacy of Ep05-2014-DM4 and Ep05-2014-DGN549

Mice were inoculated and randomized into groups of 6 mice 7 days post-inoculation (dpi). The mean tumor volumes (TV) for each group were between 96.7 and 102.3 $mm^3$. The study was ended at 58 days post inoculation (EOS = 58 dpi).

In Part 1 of this study, the treatment groups included a placebo Control group administered vehicle (200 μl/ms) and two masked-huEpCAMG23v4.2-DGN549 AADC groups (i.e., a AADC with a cleavable mask bearing the 3014 substrate, and a AADC in the non-cleavable-NSUB (no substrate) form) administered at 5 μg/kg, based on drug concentration. In Part 2 of this study, the treatment groups included a placebo Control group administered vehicle (200 μl/ms), huEpCAMG23v4.2-s-SPDB-DM4 ADC groups administered at 15, 30 and 45 μg/kg (based on drug concentration), two Ep01-02-2014-s-SPDB AADC groups (administered at 45 and 30 μg/kg), and a non-cleavable variant Ep01-02-NSUB-s-SPDB-DM4 group.

In this study, TGI was determined at 23 dpi, when the median TV of the control Placebo (vehicle) group reached 1156.9 $mm^3$.

The results of the study are shown in **FIGs. 17C** and **17D.** All treatments were well tolerated at the indicated doses, and no body weight loss was observed in this study.

*A. Efficacy of masked-EpCAM-3014-DGN549 AADCs bearing the Ep05 and Ep07 masks.*

The results of Part A of this study are shown in **FIG. 17C.** The Ep05-3014-DGN549 and Ep07-3014-DGN549 AADCs administered at 5 μg/kg were highly active highly active (HA) based on tumor growth inhibition (2.9 and 3.9%), with multiple partial regressions (6/6 and 5/6 respectively), but with no CR and no TFS at the end-of-study (58 dpi). Median survival was > 58d for the group treated with the Ep05-3014-DGN549 AADC, and 56d for the group treated with the

Ep07-3014-DGN549 AADC, which resulted in LCK values of 2.56 and 2.41, respectively (+++ activity), and a corresponding ILS > 152% and 143%, respectively. As expected, the non-cleavable Ep05-NSUB-DGN549 and Ep07-NSUB-DGN549 AADCs were inactive (TGI > 70%, LCK = 0.29, and ILS = 17%).

*B. Efficacy of huEpCAMG23v4.2-s-SPDB-DM4, and the Ep01-02-2014-s-SPDB-DM4.*

The results of Part B of this study are shown in **FIG. 17D.** The efficacy of the maytansinoid payload was tested with huEpCAMG23Gv4.2-s-SPDB-DM4 ADC (parental EpCAM-DM4) administered at 45, 30 and 15 μg/kg based on drug. The parental EpCAM-DM4 ADC was highly active at doses as low as 30 μg/kg (TGI < 1%, ILS > 152%), and induced multiple regressions (6/6 PR, 6/6 and 5/6 CR and TFS, respectively, for the 45 and 30 μg/kg doses). At the dose of 15 μg/kg, the parental EpCAM-DM4 ADC remained active (TGI = 20.3%, LCK = 1.02), and while the multiple PRs (6/6) did not all progress to CRs and TFS (1/6), the group's lifespan was increased by 70% compared to the Control (Vehicle) group. The efficacy of the maytansoid payload was also tested in an AADC format, using the Ep01-02 mask and the 2014 substrate: Ep01-02-2014-DM4. The dose of 45 μg/kg showed minimal activity as per tumor growth inhibition (35.6%), but did not induce any regressions and only slightly increased the lifespan of the group. Collectively, these data show that the anti-tumor activity of the EpCAM-DGN549 AADCs is tumor-specific (as AADCs containing Abs with non-cleavable masks are inactive), and that the cleavable DM4 payload is very active in the ADC format, with limited activity of the DM4 payload in the AADC format using the Ep01-02 mask and the 2014 substrate.

<u>Efficacy of Ep05-2014-DM4 and Ep05-2014-DGN549</u>

Mice were inoculated and randomized into groups of 6 mice 8 days post-inoculation (dpi). The mean tumor volumes (TV) for each group were between 90.8 and 99.1 $mm^3$. The study was ended at 62 days post inoculation (EOS = 62 dpi).

The treatment groups included a placebo Control group administered vehicle (200 μl/ms) and two Ep05 AADCs with the 3014 substrate (the Ep05-3014-DM4 AADC administered at 1.5 mg/kg based on Ab (~ 25 μg/kg based on drug) and the Ep05-3014-DGN549 AADC administered at 5 μg/kg based on drug). Two groups were dosed similarly with the corresponding non cleavable -NSUB AADCs, as negative controls. Three additional groups were administered the Ep05-2014-DM4 AADC at 1.5, 2.5 and 5 mg/kg (based on Ab), and two additional groups were administered the Ep05-2014-DGN549 AADC at 3 and 5 μg/kg (based on drug). All animals were dosed 5 days post-randomization (13dpi).

In this study, TGI was determined at 30 dpi, when the median TV of the control Placebo (vehicle) group reached 1241.8 $mm^3$.

The results of the study are shown in **FIGs. 17E** and **17F.** All treatments were well tolerated at the indicated doses, and no body weight loss was observed in this study.

As expected, all of the Ep05-NSUB AADCs were inactive, independently of the payload used. The Ep05-2014-DM4 AADC was highly active at 5 mg/kg (TGI = 0%), inducing CRs in 6/6 mice, all defined as TFS at EoS. The Ep05-2014-DM4 AADC administered at 2.5 mg/kg was also highly active based on TGI (7.2%), but induced only 3 PR and 1 CR, 1 TSF. The Ep05-2014-DM4 AADC administered at 1.5 mg/kg was inactive, as was the same dose of the Ep05-3014-DM4 AADC **(FIG. 17E).** The Ep05-3014-DGN549 AADC was highly active (HA) at 5 μg/kg (TGI = 5.5%), but induced only 2 PRs. The 2014-substrated AADC, Ep05-2014-DGN549, was inactive at both doses used, and did not induce regressions of tumor growth **(FIG. 17F).** Accordingly, these data show that the anti-tumor activity of the Ep05 AADCs is tumor-specific, as AADCs containing Abs with non-cleavable masks are inactive independently of the payload. Additionally, the Ep05-DGN549 AADC at 5 μg/kg was highly active when used with the 3014 substrate, but lost activity if the 2014 substrate was used. In contrast, the Ep05-2014-DM4 AADC bearing the cleavable -s-SPDB-DM4 payload is highly active and induced multiple tumor regressions, in a dose-dependent manner.

**Example 14: Efficacy of Ep05- 3014-DM4 and 2014-DM4 in C.B-17 SCID mice bearing the Calu-3 NSCLC xenograft**

Female mice, 6-8 weeks old, were inoculated subcutaneously in the area of the right flank with $5x10^6$ Calu-3 cells/mouse in 0.2 mL of SFM:Matrigel. Mice were randomized into groups of 6 mice 10 days post-inoculation (dpi). The mean tumor volumes (TV) for each group were between 97.6 and 106.4 $mm^3$. The study was ended at 119 days post inoculation (EOS = 119 dpi).

The treatment groups included a placebo Control group administered vehicle (200 μl/ms), a parental huEpCAMG23v4.2-s-SPDB-DM4 ADC group as a positive control, a non-cleavable Ep05-NSUB-DM4 AADC group as a negative control, a Ep05-3014-DM4 AADC group, and a Ep05-2014-DM4 AADC group, with all AADC compounds tested at 5 and 2.5 mg/kg (based on activatable antibody concentration).

In this study, TGI was determined at 73 dpi, when the median TV of the control placebo (vehicle) group reached

1138 $mm^3$.

The results of the study are shown in **FIG. 18.** All treatments were well tolerated at the indicated doses, and no body weight loss was observed in this study.

As expected, the Ep05-NSUB-DM4 AADC was inactive, while the parental EpCAM-DM4 ADC was active at a dose of 5 mg/kg, with a TGI = 15.6% at 5 mg/kg, and 21% at 2.5 mg/kg. Moreover, the EpCAM-DM4 ADC delayed tumor growth, but did not induce any regressions. Similarly, no regressions were observed with the E05-3014-DM4 and Ep05-2014-DM4 AADCs. The Ep05-3014-DM4 AADC was active at both 5 and 2.5 mg/kg (TGI values of 15.6% and 21%, respectively), while the Ep05-2014-DM4 AADC was inactive (TGI > 40%). Accordingly, these data show that treatment with an EpCAM-DM4 ADC or Ep05-DM4 AADC results in some tumor growth delay in the Calu-3 tumor model, but exhibits a minimal amount of overall anti-tumor activity.

**Example 15: Efficacy of Ep05- 3014-DM4 and 2014-DM4 in C.B-17 SCID mice bearing the NCI-H292 NSCLC xenograft**

Female mice, 6-8 weeks old, were inoculated subcutaneously in the area of the right flank with $2.5 \times 10^6$ NCI-H292 cells/mouse in 0.1 mL of SFM. Mice were randomized into groups of 6 mice 13 days post-inoculation (dpi). The mean tumor volumes (TV) for each group were between 88.7 and 94.1 $mm^3$. The study was terminated at 64 dpi.

The treatment groups included a placebo Control group administered vehicle (200 $\mu$l/ms), a parental huEp-CAMG23v4.2-s-SPDB-DM4 ADC group as a positive control, a non-cleavable Ep05-NSUB-DM4 AADC group as a negative control administered at 2.5 mg/kg, and two groups each of Ep05-3014-DM4 AADC and Ep05-2014-DM4 AADC (both administered at 5 and 2.5 mg/kg based on activatable antibody concentration).

In this study, TGI was determined at 28 dpi, when the median TV of the control placebo (vehicle) group reached 939.8 $mm^3$.

The results of the study are shown in **FIG.19.** All treatments were well tolerated at the indicated doses, and while several animals had to be sacrificed due to body weight loss, this was attributed to characteristics of the xenograft model rather than the treatments.

As expected, the Ep05-NSUB-DM4 AADC was inactive, while the parental EpCAM-DM4 ADC was active at 2.5 mg/kg, with a TGI of 22.7%. The EpCAM-DM4 ADC delayed the tumor growth (ILS= 100% (28.5 days)), but did not induce any regressions. Regressions were not observed in this model except for a short, non-maintained PR in the E05-3014-DM4 group dosed at 2.5 mg/kg. However, both the Ep05-3014-DM4 and the Ep05-2014-DM4 AADCs were highly active at doses as low as 2.5 mg/kg (TGI of 10% and 11.45% respectively) and increased the lifespan of the corresponding groups more than two-fold (100%). Accordingly, these data show that NCI-H292 tumors are sensitive to the EpCAM-DM4 ADC, as well as the derived Ep05-DM4 AADCs. While induction of significant tumor regression was not observed, there was significant tumor growth delay and an increased lifespan in the treated groups. Moreover, responses to both the 3014-substrated AADC and the 2014-substrated AADC were similar.

**Example 16: Efficacy of Ep05- 3014-DM4, Ep05-2014-DM4, Ep05-3014-DM21, and Ep05-2014-DM21 in C.B-17 SCID mice bearing the Detroit 562 HNSCC xenograft**

Female mice, 6-8 weeks old, were inoculated subcutaneously in the area of the right flank with $10^7$ Detroit 562 cells/mouse in 0.2 mL of 1:1 solution of SFM:Matrigel. Mice were randomized into groups of 8 mice 4 days post-inoculation (dpi). The mean tumor volumes (TV) for each group were between 99.2 and 106 $mm^3$. The study was terminated at 90 dpi.

The treatment groups included a placebo Control group administered vehicle (200 $\mu$l/ms), a Ep05-3014-DM4 AADC group administered at 2.5 mg/kg, a Ep05-2014-DM4 AADC group administered at 2.5 mg/kg, a Ep05-3014-DM21L AADC group administered at 2.5 mg/kg, a Ep05-3014-DM21L AADC group administered at 5 mg/kg, a Ep05-2014-DM21L AADC group administered at 2.5 mg/kg, and a Ep05-2014-DM21L AADC group administered at 5 mg/kg. All AADCs were administered based on activatable antibody concentration. Additionally, a positive Control ADC group was administered huEpCAM23Gv4.2-DM21L at 2.5 mg/kg based on antibody concentration.

In this study, TGI was determined at 28 dpi, when the median TV of the control placebo (vehicle) group reached 1004.2 $mm^3$.

The results of the study are shown in **FIG. 20.** All treatments were well tolerated at the indicated doses.

All AADCs used in this study (Ep05-3014-DM4, Ep05-2014-DM4, Ep05-3014-DM21L, and Ep05-2014-DM21L), as well as the huEpCAM23Gv4.2-DM21L ADC, were highly active at all tested doses (TGI of 0% for all groups except Ep05-3014-DM21L at 2.5 mg/kg, which had a TGI of 0.7%) and increased the lifespan of the corresponding groups more than three-fold (200%). All groups induced 100% partial regressions except for the Ep05-2014-DM21L group at 2.5 mg/kg, which induced 87.5% partial regressions. These partial regressions mostly progressed to complete regressions (i.e., >75% of PRs progressed to CRs) which were maintained to study termination, resulting in multiple tumor free survivors. Additional details regarding the TGI, PRs, CRs, and TFS associated with each group are provided in **FIG. 20.**

Accordingly, these data show that Detroit 562 tumors are highly sensitive to the Ep05-DM4 AADCs, resulting in significant tumor regressions, tumor growth delay, and an increased lifespan in the treated groups.

### Example 17: Efficacy of Ep05-3014-DM21 and Ep05-2014-DM21 in C.B-17 SCID mice bearing the NCI-H441 NSCLC xenograft

Female mice, 6-8 weeks old, were inoculated subcutaneously in the area of the right flank with $10^7$ NCI-H441 cells/mouse in 0.2 mL of SFM. Mice were randomized into groups of 8 mice 8 days post-inoculation (dpi). The mean tumor volumes (TV) for each group were between 95.1 and 101.4 $mm^3$. The study was terminated at 92 dpi.

The treatment groups included a placebo Control group administered vehicle (200 μl/ms), a parental huEpCAM23Gv4.2-DM21L ADC group dosed at 2.5 mg/kg (based on antibody concentration), an Ep05-3014-DM21L AADC group administered at 2.5 mg/kg, an Ep05-3014-DM21L AADC group administered at 5 mg/kg, an Ep05-2014-DM21L AADC group administered at 2.5 mg/kg, and an Ep05-2014-DM21L AADC group administered at 5 mg/kg. All AADCs were administered based on activatable antibody concentration.

In this study, TGI was determined at 37 dpi, when the median TV of the control placebo (vehicle) group reached 1109.7 $mm^3$.

The results of the study are shown in **FIG. 21.** All treatments were well tolerated at the indicated doses.

All treatments were active to highly active, increased the group's life-span by > 149% and induced 100% PRs in each group. Multiple CRs were reached and were consistently maintained to end of study, resulting in multiple TFSs. The huEpCAM23Gv4.2-DM21 ADC was highly active at 2.5 mg/kg, with a TGI of 7.7%, 8/8 PRs, and 7/8 CRs. Similarly, the Ep05-2014-DM21 AADC was highly active at both 2.5 and 5 mg/kg doses, with a TGI of 6.2% and 4.2 % respectively, and 6/8 and 5/8 CRs respectively. The Ep05-3014-DM21 AADC was active at the 5 mg/kg dose, with TGI of 10.1% and 5/8 CRs. At the 2.5 mg/kg dose, the Ep05-3014-DM21 AADC was highly active, with a TGI of 6.3%, and 6/8 CRs.

Accordingly, these data show that NCI-H441 tumors are sensitive to the Ep05-DM21L AADCs, resulting in significant tumor regressions, tumor growth delay, and an increased lifespan in the treated groups.

### Example 18: Efficacy of Ep05-2014-DM21 in C.B-17 SCID mice bearing the OV-90 EOC xenograft

Female mice, 6-8 weeks old, were inoculated subcutaneously in the area of the right flank with $10^7$ OV-90 cells/mouse in 0.1 mL of SFM:Matrigel. Mice were randomized into groups of 6 mice each, 7 days post-inoculation (dpi). The mean tumor volumes (TV) for each group were between 92.2 and 98.5 $mm^3$. The study was terminated at 71 dpi.

The treatment groups included a placebo Control group administered vehicle (200 μl/ms), a parental huEpCAM23Gv4.2-DM21 ADC group dosed at 1.25 mg/kg (based on antibody concentration), an Ep05-2014-DM21L AADC group administered at 1.25 mg/kg (based on activatable antibody concentration), an Ep05-2014-DM21L AADC group administered at 2.5 mg/kg (based on activatable antibody concentration), a chKTI-DM21L non-targeted negative control ADC group administered at 1.25 mg/kg (based on antibody concentration), and a chKTI-DM21L non-targeted negative control ADC group administered at 2.5 mg/kg (based on antibody concentration).

In this study, TGI was determined at 34 dpi, when the median TV of the control placebo (vehicle) group reached 1436.7 $mm^3$.

The results of the study are shown in **FIG. 22.** All treatments were well tolerated at the indicated doses.

All EpCAM targeting treatments used in this study (the huEpCAM23Gv4.2-DM21 ADC group at 1.25 mg/kg and the Ep05-2014-DM21L AADC groups at both 2.5 and 1.25 mg/kg) were highly active, with TGI < 5%, > 109% increase in the group's life-span compared to the non-treated animals, and 100% PRs in each group. All PRs in these groups progressed to complete regression (CRs) and were maintained to end of study, resulting in TFSs. The non-targeted chKTI-DM21 ADC was not active against OV-90 tumors at 2.5 and 1.25 mg/kg, with TGI > 74.5% and no tumor regressions.

Accordingly, these data show that the OV-90 tumors are sensitive to the Ep05-2014-DM21L AADC, resulting in significant tumor regressions and an increased lifespan in the treated groups.

### Example 19: Efficacy of Ep05-2014-DM21 in C.B-17 SCID mice bearing the Calu-3 NSCLC xenograft

Female mice, 6-8 weeks old, were inoculated subcutaneously in the area of the right flank with $5x10^6$ Calu-3 cells/mouse in 0.2 mL of SFM:Matrigel. Mice were randomized into groups of 6 mice each, 6 days post-inoculation (dpi). The mean tumor volumes (TV) for each group were between 110.3 and 116.5 $mm^3$.

The treatment groups included a placebo Control group administered vehicle (200 μl/ms), a parental huEpCAM23Gv4.2-DM21L ADC group dosed at 2.5 mg/kg (based on antibody concentration), an Ep05-2014-DM21L AADC group administered at 2.5 mg/kg (based on activatable antibody concentration), an Ep05-2014-DM21L AADC group administered at 5 mg/kg (based on activatable antibody concentration), a chKTI-DM21L non-targeted negative control ADC group administered at 2.5 mg/kg (based on antibody concentration), and a chKTI-DM21L non-targeted negative

control ADC group administered at 5 mg/kg (based on antibody concentration).

In this study, TGI was determined at 62 dpi, when the median TV of the control placebo (vehicle) group reached 963.8 $mm^3$.

The results of the study are shown in **FIG. 23.** All treatments were well tolerated at the indicated doses.

The huEpCAM23Gv4.2-DM21 ADC at 2.5 mg/kg was highly active, with a TGI of 7.8%, 6/6 PRs, 5/6 CR, and 1/6 mice tumor free at latest measurement (75 dpi). The Ep05-2014-DM21L AADC was highly active at 5 mg/kg, with 4.4% TGI, 5/6 PRs progressing into CRs, and 2/6 mice tumor free at the latest measurement (75 dpi). The Ep05-2014-DM21L AADC was active at 2.5 mg/kg, with 30.4% TGI and 3/6 PRs. The non-targeted chKTI-DM21L ADC control group was inactive against Calu-3 tumors at 5 and 2.5 mg/kg, with TGI > 90% at the dose of 5 mg/kg and no tumor regressions.

Accordingly, these data show that the Calu-3 tumors are sensitive to the Ep05-2014-DM21L AADC, resulting in significant tumor regressions and tumor growth delay.

**Example 20: Analysis of EpCAM-targeting AADC pharmacokinetics and tolerability in cynomolgus monkeys**

*Pharmacokinetics and tolerability of DM4 conjugates*

To further assess the tolerability and pharmacokinetics associated with EpCAM-targeting ADCs and AADCs, male cynomolgus monkeys were administered huEpCAM23 Gv4.2-s-SPDB-DM4, huEpCAM23Gv4.2-Ep05-2014-s-SPDB-DM4, or huEpCAM23Gv4.2-Ep05-3014-s-SPDB-DM4 according to the study design shown in Table 27.

**Table 27. Pharmacokinetic and tolerability study design in cynomolgus monkeys.**

| Group | Test Material | Dose Level | Number of animals |
|---|---|---|---|
| 1 | huEpCAM23Gv4.2-s-SPDB-DM4 | 8 mg Ab/kg | 2M |
| 2 | huEpCAM23Gv4.2-Ep05-2014-s-SPDB-DM4 | 8 mg Ab/kg | 2M |
| 2b | huEpCAM23Gv4.2-Ep05-2014-s-SPDB-DM4 | 12 mg Ab/kg | 2M |
| 3 | huEpCAM23Gv4.2-Ep05-3014-s-SPDB-DM4 | 8 mg Ab/kg | 2M |
| 3b | huEpCAM23Gv4.2-Ep05-3014-s-SPDB-DM4 | 12 mg Ab/kg | 2M |

Following a single IV infusion of the ADC or AADC, the cynomolgus monkeys were observed for 28 days and samples were collected for pharmacokinetic analysis. In particular, the monkeys were monitored for changes in body weight, clinical observations, and clinical pathology evaluation (e.g., lipase and amylase).

As shown in **FIG. 24,** an EpCAM-targeting AADC was well tolerated at 8 mg/kg in cynomolgus monkeys. In particular, clinical observations in monkeys receiving 8 mg/kg of huEpCAM23Gv4.2-Ep05-2014-s-SPDB-DM4 or huEpCAM23Gv4.2-Ep05-3014-s-SPDB-DM4 were limited to reddened skin at the injection site, and the fecal output was considered normal. Similar results were obtained for monkeys receiving 12 mg/kg of huEpCAM23Gv4.2-Ep05-2014-s-SPDB-DM4. In contrast, monkeys receiving 8 mg/kg of the ADC were considered moribund and clinical observations consisted of abnormal gait, decreased activity, reduced appetite, cold to the touch, mild skin findings (reddish or flaking), liquid stool, and dehydration was suspected.

Cynomolgus monkeys receiving an EpCAM-targeting DM4 AADC also showed improved serum chemistry relative to monkeys receiving the EpCAM-targeting DM4 ADC. In particular, changes in albumin, TP, A/G ratio **(FIG. 25A),** urea nitrogen **(FIG. 25B),** and creatinine **(FIG. 25C)** were observed only in the ADC group, and increases in lipase **(FIG. 25D)** and amylase **(FIG. 25E)** serum concentrations were similarly associated only with the ADC group.

As shown in **FIG. 26** and Table 28, administration of an AADC was also associated with improved conjugate exposure. Collectively, these data suggest that effective masking of the DM4 AADC reduces the impact of normal tissue expression of EpCAM on pharmacokinetic and toxicity profiles.

**Table 28. Pharmacokinetic profile of cynomolgus monkeys receiving huEpCAM23Gv4.2-s-SPDB-DM4, huEpCAM23Gv4.2-Ep05-2014-s-SPDB-DM4, or huEpCAM23Gv4.2-Ep05-3014-s-SPDB-DM4.**

| Test Article | Dose (mg Ab/kg) | $T_{1/2}$ (hr) | $C_{max}$ (μg/mL) | $AUC_\infty$ (hr*μg/mL) | $V_{ss}$ (mL/kg) | Cl (mL/hr/kg) |
|---|---|---|---|---|---|---|
| huEpCAM23Gv4 .2-s-SPDB-DM4 | 8 | 33.9 + 6.4 | 275 ± 134 | 5510 ± 947 | 53.3 ± 3.1 | 1.47 ± 0.25 |
| huEpCAM23Gv4 .2-Ep05-2014-s-SPDB-DM4 | 8 | 94.9 ± 12.4 | 200 ± 21 | 19000 ± 61 | 56.6 ± 3.4 | 0.421 ± 0.001 |

(continued)

| Test Article | Dose (mg Ab/kg) | $T_{1/2}$ (hr) | $C_{max}$ (μg/mL) | $AUC_\infty$ (hr*μg/mL) | $V_{ss}$ (mL/kg) | Cl (mL/hr/kg) |
|---|---|---|---|---|---|---|
| huEpCAM23Gv4. 2-Ep05-3014-s-SPDB-DM4 | 8 | 64.3 + 16.9 | 177 + 3.82 | 13600 ± 874 | 58.2 ± 5.33 | 0.59 + 0.038 |

*Pharmacokinetics and tolerability of DM21 conjugates*

In a separate study, male cynomolgus monkeys were administered huEpCAM23Gv4.2-Ep05-2014-DM21L or huEpCAM23Gv4.2-Ep05-3014-DM21L according to the study design shown in Table 29.

**Table 29. Pharmacokinetic and tolerability study design in cynomolgus monkeys.**

| Group | Test Material | Dose Level | Number of animals |
|---|---|---|---|
| 1 | huEpCAM23Gv4.2-Ep05-2014-DM21L | 12 mg Ab/kg | 2M |
| 2 | huEpCAM23Gv4.2-Ep05-3014-DM21L | 12 mg Ab/kg | 2M |

Following a single IV infusion of the ADC or AADC, the cynomolgus monkeys were observed for 28 days and samples were collected for pharmacokinetic analysis. In particular, the monkeys were monitored for changes in body weight, clinical observations, and clinical pathology evaluation (*e.g.*, lipase and amylase).

As shown in **FIG. 27,** an huEpCAM23Gv4.2-Ep05-2014-DM21L was well tolerated at 12 mg/kg in cynomolgus monkeys. In particular, clinical observations were limited to reddened/darkened skin, liquid feces, and sunken eyes (dehydration). In contrast, monkeys receiving 12 mg/kg of huEpCAM23Gv4.2-Ep05-3014-DM21L exhibited clinical observations similar to those receiving huEpCAM23Gv4.2-s-SPDB-DM4 in the previous study.

Cynomolgus monkeys receiving 12 mg/kg huEpCAM23Gv4.2-Ep05-2014-DM21L also showed similar serum chemistry to monkeys receiving 12 mg/kg of huEpCAMGv4.2-Ep05-2014-s-SPDB-DM4 in the previous study. In particular, changes in A/G ratio **(FIG. 28A),** urea nitrogen **(FIG. 28B),** and creatinine **(FIG.** 28C), lipase **(FIG. 28D),** and amylase **(FIG. 28E)** serum concentrations were similar for these two groups, while serum chemistry parameters for the huEpCAM23Gv4.2-Ep05-3014-DM21L group trended toward those obtained for the DM4 ADC group in the previous study.

As shown in **FIG. 29** and Table 30, administration of huEpCAM23Gv4.2-Ep05-2014-DM21L was also associated with improved conjugate exposure compared to all other tested conjugates (including both DM4 and DM21L conjugates). Collectively, these data suggest that effective masking of the DM21 AADC reduces the impact of normal tissue expression of EpCAM on pharmacokinetic and toxicity profiles.

**Table 30. Pharmacokinetic profile of cynomolgus monkeys receiving huEpCAM23Gv4.2-Ep05-2014-DM21L.**

| Test Article | Dose (mg Ab/kg) | $T_{1/2}$ (hr) | $C_{max}$ (μg/mL) | $AUC_\infty$ (hr*μg/mL) | $V_{ss}$ (mL/kg) | Cl (mL/hr/kg) |
|---|---|---|---|---|---|---|
| huEpCAM23Gv4.2-Ep05-2014-DM21L | 12 | 111 ± 40.8 | 426 + 33.9 | 42300 ± 3650 | 45.6 + 11.1 | 0.285 + 0.0246 |

**Example 21: Analysis of EpCAM Expression**

EpCAM Tumor Expression

To evaluate EpCAM expression across different indications, tissue microarrays (TMA) representing 10 different tumor types were first evaluated using an EpCAM immunohistochemistry (IHC) assay developed at ImmunoGen for preliminary research use.

All tumors analyzed were FFPE (Formalin fixed & paraffin embedded) samples. In particular, two multi-carcinoma TMAs representing 10 different indications were analyzed, as well as indication-specific TMAs representing triple-negative breast cancer (TNBC), ovarian cancer, and bladder cancer. A colorectal cancer (CRC) TMA was also analyzed, as were whole tumor tissue FFPE blocks for lung cancer and ovarian cancer.

Immunohistochemical staining for EpCAM was carried out using the Ventana Discovery Ultra autostainer. The primary antibody for EpCAM was a commercially available rabbit monoclonal antibody. All samples were evaluated and

scored by a board-certified pathologist trained in the scoring algorithm. The presence of at least 100 viable tumor cells was required for scoring. Staining intensity was scored on a semi-quantitative integer scale from 0 to 3, with 0 representing no staining, 1 representing weak staining, 2 representing moderate and 3 representing strong staining. The percentage of cells staining positively at each intensity level was recorded. Scoring was based on localization of EpCAM to the cell membrane only. The staining results were analyzed by H-score, which combines components of staining intensity with the percentage of positive cells. It has a value between 0 and 300 and is defined as:

$$1 * (\text{percentage of cells staining at } 1+ \text{ intensity}) \\ + 2 * (\text{percentage of cells staining at } 2+ \text{ intensity}) \\ + 3 * (\text{percentage of cells staining at } 3+ \text{ intensity}) \\ = \text{H score}$$

Table 31 below summarizes the prevalence of EpCAM based on membrane staining for all 10 indications from the multi-carcinoma TMAs.

**Table 31. Prevalence of EpCAM in 10 different indications based on membrane staining.**

| Tumor type (n) | Total positivity (H ≥ 1) | % of tumors with H = 1-100 | % of tumors with H = 101-200 | % of tumors with H = 201-300 |
|---|---|---|---|---|
| Stomach (n = 39) | 100% | 21% | 67% | 13% |
| Ovary (n =39) | 100% | 8% | 51% | 41% |
| Uterus (n = 40) | 98% | 20% | 58% | 20% |
| Colon (n = 38) | 95% | 0% | 71% | 24% |
| Breast (n = 38) | 95% | 71% | 24% | 0% |
| Pancreas (n =37) | 95% | 49% | 41% | 5% |
| Prostate (n = 36) | 94% | 28% | 53% | 14% |
| Lung (n = 40) | 88% | 58% | 18% | 13% |
| Head/Neck (n = 40) | 53% | 45% | 8% | 0% |
| Liver (n = 39) | 41% | 31% | 8% | 3% |

Following upon the results from the multi carcinoma TMA, five indication-specific TMAs were chosen for expanded prevalence analysis: colorectal cancer (CRC) TMA with 42 cases (40 evaluable), ovarian cancer TMA with 37 cores, triple-negative breast cancer (TNBC) TMA with 81 cores, bladder cancer TMA with 60 carcinoma cores and a non-small cell lung cancer (NSCLC) TMA with 80 cores for adenocarcinoma and 80 cores for squamous cell carcinoma (79 evaluable for each). **FIG. 30** describes EpCAM expression with a breakdown of H-scores within each tumor indication.

Additionally, whole tumor tissue sections of NSCLC-adenocarcinoma (n = 86), NSCLC-squamous cell carcinoma (n =19) and ovarian carcinoma (n = 29) were analyzed by IHC for EpCAM expression. All of these samples were scored for membrane staining and the results summarized in Table 32.

**Table 32. Prevalence of EpCAM in additional samples for NSCLC and Ovarian Cancer.**

| Tumor type (n) | Total positivity (H ≥ 1) | H = 1-100 | H = 101-200 | H = 201-300 |
|---|---|---|---|---|
| NSCLC-all (n = 105) | 93% | 39% | 42% | 12% |
| NSCLC-adenocarcinoma (n =86) | 100% | 38% | 48% | 15% |
| NSCLC-squamous (n = 19) | 64% | 48% | 16% | 0% |
| Ovarian (n = 29) | 100% | 17% | 76% | 7% |

The results of these preliminary studies show that EpCAM is expressed in a wide range of solid cancers and support the use of anti-EpCAM9 drug conjugates in many different EpCAM-expressing solid tumors.

EpCAM Expression in Normal Human Tissues

Normal human tissue expression of EpCAM was evaluated by IHC in a manner similar to that described above using the same anti-EpCAM rabbit monoclonal antibody and Ventana Discovery Ultra autostainer.

All tissues analyzed were FFPE (Formalin fixed & paraffin embedded) samples using a multi-tissue microarray TMA representing 35 different organs/anatomic sites from 3 separate individuals. All samples were evaluated and scored by a board-certified pathologist trained in the scoring algorithm. The presence of at least 100 viable tumor cells was required for scoring. As for tumor samples, staining intensity was scored on a semi-quantitative integer scale from 0 to 3, with 0 representing no staining, 1 representing weak staining, 2 representing moderate and 3 representing strong staining. However, unlike for tumors, H-scores were not calculated due to the various structures normally found in different tissues which make H-scores an inaccurate reflection of staining. The pathologist's description of any staining pattern observed in each organ were recorded, with the results summarized in Table 33.

Significant, strong staining is observed in a number of normal human tissues, notably the small intestine, colon and rectum. Accordingly, alternative targeting approaches, such as activatable antibody-based technologies, represent an attractive option for overcoming potential toxicity risks.

**Table 33. Prevalence of EpCAM in Normal Human Tissues.**

| Organ/site | % cores positive | Cell type/structure | Intensity |
|---|---|---|---|
| Adrenal gland | 0% | n/a | negative |
| Bladder | 5 0% | Superficial transitional epithelium | very weak |
| Bone marrow | 0% | n/a | negative |
| Breast | 100% | Acini | 1+/2+ |
| Cerebellum | 0% | n/a | negative |
| Cerebral cortex | 0% | n/a | negative |
| Colon/rectum | 100% | Surface, villi and crypt epithelium | 3+ |
| Esophagus | 0% | n/a | negative |
| Eye | 0% | n/a | negative |
| Fallopian Tube | 100% | Fallopian epithelium | 3+ |
| Heart | 0% | n/a | negative |
| Kidney (cortex, medulla) | 100% | Distal tubules | 1+ |
| Liver | 100% | Bile ducts | 1+ |
| Lung | 100% | Type II pneumocytes | 1+ |
| Ovary | 0% | n/a | negative |
| Pancreas | 100% | Acini | 1+ |
| Parathyroid | 100% | Parathyroid cells | 2+ |
| Peripheral nerve | 0% | n/a | negative |
| Pituitary Gland | 100% | Pituitary cells | 1+/2+ |
| Placenta | 0% | n/a | negative |
| Prostate | 100% | Prostate glands | 1+ |
| Salivary gland | 100% | Acini | 1+/2+ |
| Skeletal muscle | 0% | n/a | negative |
| Skin | 0% | n/a | negative |
| Small intestine | 100% | Surface, villi and crypt epithelium | 3+ |
| Spinal cord | 0% | n/a | negative |
| Spleen | 0% | n/a | negative |
| Stomach | 33% | Fundic glands | 1+, cytoplasmic |

(continued)

| Organ/site | % cores positive | Cell type/structure | Intensity |
|---|---|---|---|
| Testis | 33% | Spermatids | Patchy granular |
| Thymus | 0% | n/a | negative |
| Thyroid | 100% | Follicular thyroid epithelium | 1+, 2+, 3+ |
| Tonsil | 0% | n/a | negative |
| Ureter | 0% | n/a | negative |
| Uterus-cervix | 0% | n/a | negative |
| Uterus-endometrium | 100% | Endometrial glands | 1+, 2+, 3+ |

| | |
|---|---|
| **Exemplary IgG1 Fc Region** | APELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTP PVLDSDGSFFLYSKLTVDKSRWQQGNVFSCSVMHEALHNHYTQKSLSLSPG (**SEQ ID NO:304**) |
| **Exemplary IgG2 Fc Region** | APPVAGPSVFLFPPKPKDTLMISRTPEVTCVVVDVSHEDPEVQFNWYVDG VEVHNAKTKPREEQFNSTFRVVSVLTVVHQDWLNGKEYKCKVSNKGLPAP IEKTISKTKGQPREPQVYTLPPSREEMTKNQVSLTCLVKGFYPSDIAVEW ESNGQPENNYKTTPPMLDSDGSFFLYSKLTVDKSRWQQGNVFSCSVMHEA LHNHYTQKSLSLSPG (**SEQ ID NO:305**) |
| **Exemplary IgG4 Fc Region** | APEFLGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLPS SIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHE ALHNHYTQKSLSLSLG (**SEQ ID NO:306**) |
| **Exemplary L234A/L235A IgG1 Fc Region** | APEAAGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTP PVLDSDGSFFLYSKLTVDKSRWQQGNVFSCSVMHEALHNHYTQKSLSLSPG (**SEQ ID NO:307**) |
| **Exemplary N297A IgG1 Fc Region** | APELLGGPSVFLFPPKPKDTLMISRTPEVTCVVVDVSHEDPEVKFNWYVDGVEVH NAKTKPREEQY**A**STYRVVSVLTVLHQDWLNGKEYKCKVSNKALPAPIEKTISKAK CQPREPQVYTLPPSRDELTKNQVSLTCLVKCFYPSDIAVEWESNCQPENNYKTTP PVLDSDGSFFLYSKLTVDKSRWQQGNVFSCSVMHEALHNHYTQKSLSLSPG (**SEQ ID NO: 308**) |
| **Exemplary N297Q IgG1 Fc Region** | APELLGGPSVFLFPPKPKDTLMISRTPEVTCVVVDVSHEDPEVKFNWYVDGVEVH NAKTKPREEQY**Q**STYRVVSVLTVLHQDWLNGKEYKCKVSNKALPAPIEKTISKAK GQPREPQVYTLPPSRDELTKNQVSLTCLVKGFYPSDIAVEWESNGQPENNYKTTP PVLDSDGSFFLYSKLTVDKSRWQQGNVFSCSVMHEALHNHYTQKSLSLSPG (**SEQ ID NO: 309**) |

It is to be appreciated that the Detailed Description section, and not the Summary and Abstract sections, is intended to be used to interpret the claims. The Summary and Abstract sections may set forth one or more but not all exemplary embodiments of the present disclosure as contemplated by the inventor(s), and thus, are not intended to limit the present disclosure and the appended claims in any way.

The present disclosure has been described above with the aid of exemplary headings and other functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments, will so fully reveal the general *Nature* of the encompassed compositions and methods that others can, by applying knowledge within the skill of the art, readily modify and/or adapt for various applications such specific embodiments, without undue experimentation, without departing from the general

concept of the present disclosure. Therefore, such adaptations and modifications are intended to be within the meaning and range of equivalents of the disclosed embodiments, based on the teaching and guidance presented herein. The phraseology or terminology herein is for the purpose of description and not of limitation, such that the terminology or phraseology of the present specification is to be interpreted by the skilled artisan in light of the teachings and guidance.

The breadth and scope of the present disclosure should not be limited by any of the above-described exemplary embodiments, but should be defined only in accordance with the following claims and their equivalents.

**THE INVENTION ALSO PROVIDES THE FOLLOWING NUMBERED EMBODIMENTS:**

1. An EpCAM antibody or EpCAM-binding antibody fragment, wherein the antibody or antibody fragment comprises:

(a) a heavy chain CDR1 (VH-CDR1) comprising the sequence $X_1YX_3X_aH$, wherein $X_1$ is selected from N and S, $X_3$ is selected from Y, N, F, S, H, D, L, I, and W, and $X_4$ is selected from I and M (SEQ ID NO:5);
(b) a heavy chain CDR2 (VH-CDR2) comprising the sequence $WX_2X_3PGX_6VYIQYX_{12}X_{13}KFX_{17}G$, wherein $X_2$ is selected from I and F, $X_3$ is selected from Y and N, $X_6$ is selected from N and D, $X_{12}$ is selected from N and S, $X_{13}$ is selected from E and Q, and $X_{17}$ is selected from K and Q (SEQ ID NO:7);
(c) heavy chain CDR3 (VH-CDR3) comprising the sequence $X_1GX_3X_4FAY$, wherein $X_1$ is selected from D and E, $X_3$ is selected from P, A, S, Y, F, G, T, and V, and $X_4$ is selected from Y and W (SEQ ID NO:8);
(d) a light chain CDR1 (VL-CDR1) comprising the sequence $RSSX_4SLLHSX_{10}GX_{12}TY LX_{16}$, wherein $X_4$ is selected from R and K, $X_{10}$ is selected from N and D, $X_{12}$ is selected from F and I, and $X_{16}$ is selected from Y and S (SEQ ID NO: 10);
(e) a light chain CDR2 (VL-CDR2) comprising the sequence QTSNLAS (SEQ ID NO:40); and
(f) a light chain CDR3 (VL-CDR3) comprising the sequence $X_1QX_3LELPX_8T$, wherein $X_1$ is selected from A, L, and Q, $X_3$ is selected from S, G, Y, and N, and $X_8$ is selected from N and W (SEQ ID NO:11).

2. The antibody or antibody fragment of embodiment 1, wherein the antibody or antibody fragment comprises:

(a) a heavy chain CDR1 (VH-CDR1) comprising the sequence $NYX_3IH$, wherein $X_3$ is selected from Y, N, F, S, H, D, L, I, and W (SEQ ID NO:6);
(b) a heavy chain CDR2 (VH-CDR2) comprising the sequence $WX_2X_3PGX_6VYIQYX_{12}X_{13}KFX_{17}G$, wherein $X_2$ is selected from I and F, $X_3$ is selected from Y and N, $X_6$ is selected from N and D, $X_{12}$ is selected from N and S, $X_{13}$ is selected from E and Q, and $X_{17}$ is selected from K and Q (SEQ ID NO:7);
(c) heavy chain CDR3 (VH-CDR3) comprising the sequence $DGPX_4FAY$, wherein $X_4$ is selected from Y and W (SEQ ID NO:9);
(d) a light chain CDR1 (VL-CDR1) comprising the sequence $RSSX_4SLLHSX_{10}GX_{12}TYLX_{16}$, wherein $X_4$ is selected from R and K, $X_{10}$ is selected from N and D, $X_{12}$ is selected from F and I, and $X_{16}$ is selected from Y and S (SEQ ID NO:10);
(e) a light chain CDR2 (VL-CDR2) comprising the sequence QTSNLAS (SEQ ID NO:40); and
(f) a light chain CDR3 (VL-CDR3) comprising the sequence $AQX_3LELPNT$, wherein $X_3$ is selected from S, G, Y, and N (SEQ ID NO:12).

3. The EpCAM antibody or EpCAM-binding antibody fragment of embodiment 1 or 2, wherein the antibody or antibody fragment comprises a VH-CDR1, VH-CDR2, VH-CDR3, VL-CDR1, VL-CDR2, and VL-CDR3 having the sequences selected from the group:

(a) SEQ ID NOs: 13-15, 42, 40, and 41, respectively;
(b) SEQ ID NOs: 13-15, and 39-41, respectively;
(c) SEQ ID NOs: 13, 26, 15, and 39-41, respectively; and
(d) SEQ ID NOs: 13, 24, 15, 42, 40, and 41, respectively.

4. The EpCAM antibody or EpCAM-binding antibody fragment of any one of embodiments 1-3, wherein the antibody or antibody fragment binds with a $K_D$ of 3.0 nM or less to both human EpCAM and cynomolgus EpCAM.

5. The EpCAM antibody or EpCAM-binding antibody fragment of embodiment 4, wherein the antibody or antibody

fragment specifically binds to an epitope within the extracellular domain of human EpCAM having the amino acid sequence of SEQ ID NO:2.

6. The antibody or antibody fragment of embodiment 5, wherein the antibody or antibody fragment comprises:

(a) a VH-CDR1 comprising the sequence NYYIH (SEQ ID NO:13);
(b) a VH-CDR2 comprising the sequence WIYPGNVYIQYNEKFKG (SEQ ID NO:14);
(c) a VH-CDR3 comprising the sequence DGPWFAY (SEQ ID NO:15);
(d) a VL-CDR1 comprising the sequence RSSRSLLHSDGFTYLY (SEQ ID NO:42);
(e) a VL-CDR2 comprising the sequence QTSNLAS (SEQ ID NO:40); and
(f) and a VL-CDR3 comprising the sequence AQNLELPNT (SEQ ID NO:41).

7. The antibody or antibody fragment of embodiment 4, wherein the antibody or antibody fragment comprises:

(a) a VH-CDR1 comprising the sequence NYYIH (SEQ ID NO:13);
(b) a VH-CDR2 comprising the sequence WIYPGNVYIQYNEKFKG (SEQ ID NO:14);
(c) a VH-CDR3 comprising the sequence DGPWFAY (SEQ ID NO:15);
(d) a VL-CDR1 comprising the sequence RSSKSLLHSDGFTYLY (SEQ ID NO:39);
(e) a VL-CDR2 comprising the sequence QTSNLAS (SEQ ID NO:40); and
(f) a VL-CDR3 comprising the sequence AQNLELPNT (SEQ ID NO:41).

8. The EpCAM antibody or EpCAM-binding antibody fragment of embodiment 3, wherein the antibody or antibody fragment comprises a VH and a VL selected from:

(a) a VH having a sequence of SEQ ID NO:54, and a VL having a sequence of SEQ ID NO:89;
(b) a VH having a sequence of SEQ ID NO:54, and a VL having a sequence of SEQ ID NO:87;
(c) a VH having a sequence of SEQ ID NO:55, and a VL having a sequence of SEQ ID NO:87;
(d) a VH having a sequence of SEQ ID NO:56, and a VL having a sequence of SEQ ID NO:88;
(e) a VH having a sequence of SEQ ID NO:55, and a VL having a sequence of SEQ ID NO:89; and
(f) a VH having a sequence of SEQ ID NO:56, and a VL having a sequence of SEQ ID NO:89.

9. The antibody or antibody fragment of embodiment 8, wherein the antibody or antibody fragment comprises a VH of SEQ ID NO:54 and a VL of SEQ ID NO:89.

10. The antibody or antibody fragment of embodiment 8, wherein the antibody or antibody fragment comprises a VH of SEQ ID NO:54 and a VL of SEQ ID NO:87.

11. The EpCAM antibody or EpCAM-binding antibody fragment of embodiment 8, wherein the antibody or antibody fragment comprises a heavy chain and a light chain selected from

(a) a HC having a sequence of SEQ ID NO:103, and a LC having a sequence of SEQ ID NO:140;
(b) a HC having a sequence of SEQ ID NO:103, and a LC having a sequence of SEQ ID NO:138;
(c) a HChaving a sequence of SEQ ID NO:105, and a LC having a sequence of SEQ ID NO:139;
(d) a HC having a sequence of SEQ ID NO:106, and a LC having a sequence of SEQ ID NO:139;
(e) a HC having a sequence of SEQ ID NO:105, and a LC having a sequence of SEQ ID NO:140; and
(f) a HC having a sequence of SEQ ID NO:106, and a LC having a sequence of SEQ ID NO:140.

12. The antibody or antibody fragment of embodiment 11, wherein the antibody or antibody fragment comprises a HC of SEQ ID NO:103 and a LC of SEQ ID NO:140.

13. The antibody or antibody fragment of embodiment 11, wherein the antibody or antibody fragment comprises a HC of SEQ ID NO: 103 and a LC of SEQ ID NO: 138.

14. The EpCAM antibody or EpCAM-binding antibody fragment of embodiment 1 or 2, wherein the antibody or antibody fragment comprises a VH-CDR1, VH-CDR2, VH-CDR3, VL-CDR1, VL-CDR2, and VL-CDR3 having the sequences selected from the group consisting of:

(a) SEQ ID NOs: 22, 14, 15, 42, 40, and 41, respectively;

(b) SEQ ID NOs: 13, 14, 33, 42, 40, and 41, respectively;
(c) SEQ ID NOs: 23, 14, 15, 42, 40, and 41, respectively; and
(d) SEQ ID NOs: 25, 14, 15, 42, 40, and 41, respectively.

15. The antibody or antibody fragment of embodiment 14, wherein the antibody or antibody fragment comprises:

(a) a VH-CDR1 comprising the sequence NYHIH (SEQ ID NO:22);
(b) a VH-CDR2 comprising the sequence WIYPGNVYIQYNEKFKG (SEQ ID NO:14);
(c) a VH-CDR3 comprising the sequence DGPWFAY (SEQ ID NO:15);
(d) a VL-CDR1 comprising the sequence RSSRSLLHSDGFTYLY (SEQ ID NO:42);
(e) a VL-CDR2 comprising the sequence QTSNLAS (SEQ ID NO:40); and
(f) a VL-CDR3 comprising the sequence AQNLELPNT (SEQ ID NO:41).

16. The antibody or antibody fragment of embodiment 14, wherein the antibody or antibody fragment comprises:

(a) a VH-CDR1 comprising the sequence NYYIH (SEQ ID NO:13);
(b) a VH-CDR2 comprising the sequence WIYPGNVYIQYNEKFKG (SEQ ID NO:14);
(c) a VH-CDR3 comprising the sequence DGYWFAY (SEQ ID NO:33);
(d) a VL-CDR1 comprising the sequence RSSRSLLHSDGFTYLY (SEQ ID NO:42);
(e) a VL-CDR2 comprising the sequence QTSNLAS (SEQ ID NO:40); and
(f) a VL-CDR3 comprising the sequence AQNLELPNT (SEQ ID NO:41).

17. The EpCAM antibody or EpCAM-binding antibody fragment of embodiment 14, wherein the antibody or antibody fragment comprises a VH and a VL selected from:

(a) a VH having a sequence of SEQ ID NO:75, and a VL having a sequence of SEQ ID NO:89;
(b) a VH having a sequence of SEQ ID NO:77, and a VL having a sequence of SEQ ID NO:89;
(c) a VH having a sequence of SEQ ID NO:76, and a VL having a sequence of SEQ ID NO:89; and
(d) a VH having a sequence of SEQ ID NO:84, and a VL having a sequence of SEQ ID NO:89.

18. The antibody or antibody fragment of embodiment 17, wherein the antibody or antibody fragment comprises a VH of SEQ ID NO: 75 and a VL of SEQ ID NO: 89.

19. The antibody or antibody fragment of embodiment 17, wherein the antibody or antibody fragment comprises a VH of SEQ ID NO: 77 and a VL of SEQ ID NO: 89.

20. The EpCAM antibody or EpCAM-binding antibody fragment of embodiment 17, wherein the antibody or antibody fragment comprises a heavy chain and a light chain selected from

(a) a HC having a sequence of SEQ ID NO:125, and a LC having a sequence of SEQ ID NO:140;
(b) a HC having a sequence of SEQ ID NO:127, and a LC having a sequence of SEQ ID NO:140;
(c) a HC having a sequence of SEQ ID NO:126, and a LC having a sequence of SEQ ID NO:140; and
(d) a HC having a sequence of SEQ ID NO:134, and a LC having a sequence of SEQ ID NO:140.

21. The antibody or antibody fragment of embodiment 20, wherein the antibody or antibody fragment comprises a HC of SEQ ID NO: 125 and a LC of SEQ ID NO: 140.

22. The antibody or antibody fragment of embodiment 20, wherein the antibody or antibody fragment comprises a HC of SEQ ID NO: 127 and a LC of SEQ ID NO: 140.

23. The antibody or antibody fragment of any one of embodiments 1-22, wherein the antibody or antibody fragment is a murine, non-human mammal, chimeric, humanized, or human antibody.

24. The antibody or antibody fragment of any one of embodiments 1-23, wherein the antibody is a full-length antibody.

25. The full-length antibody of embodiment 24, wherein the antibody is human IgG1.

26. The antibody or antibody fragment of any one of embodiments 1-23, wherein antibody fragment is an Fab, Fab',

$F(ab')_2$, $F_d$, single chain Fv or scFv, disulfide linked $F_v$, V-NAR domain, IgNar, intrabody, IgG$\Delta CH_2$, minibody, $F(ab')_3$, tetrabody, triabody, diabody, single-domain antibody, DVD-Ig, Fcab, $mAb_2$, $(scFv)_2$, or scFv-Fc.

27. An activatable antibody comprising the antibody or antibody fragment of any one of embodiments 1-26, wherein the activatable antibody further comprises

(a) a cleavable moiety coupled to the antibody or antibody fragment, wherein the cleavable moiety is a polypeptide that functions as a substrate for a protease; and
(b) a masking moiety coupled to the antibody or antibody fragment, wherein the masking moiety inhibits the binding of the antibody or antibody fragment to EpCAM when the activatable antibody is in an uncleaved state,

wherein the activatable antibody in the uncleaved state has the structural arrangement from N-terminus to C-terminus as follows: (masking moiety)-(cleavable moiety)-(antibody or antibody fragment) or (antibody or antibody fragment)-(cleavable moiety)-(masking moiety).

28. An EpCAM activatable antibody comprising:

(a) an EpCAM antibody or EpCAM-binding antibody fragment comprising a VH-CDR1, VH-CDR2, VH-CDR3, VL-CDR1, VL-CDR2, and VL-CDR3 having the sequences of a member selected from the group:

(i) SEQ ID NOs: 13-15, 42, 40, and 41, respectively;
(ii) SEQ ID NOs: 13-15, and 39-41, respectively;
(iii) SEQ ID NOs: 13, 26, 15, and 39-41, respectively; and
(iv) SEQ ID NOs: 13, 24, 15, 42, 40, and 41, respectively;

(b) a masking moiety coupled to the EpCAM antibody or EpCAM-binding antibody fragment, wherein the masking moiety inhibits the binding of the antibody or antibody fragment to EpCAM when the activatable antibody is in an uncleaved state; and
(c) a cleavable moiety coupled to the EpCAM antibody or EpCAM-binding antibody fragment, wherein the cleavable moiety is a polypeptide that functions as a substrate for a protease;

wherein the activatable antibody in the uncleaved state has the structural arrangement from N-terminus to C-terminus as follows: (masking moiety)-(cleavable moiety)-(antibody or antibody fragment) or (antibody or antibody fragment)-(cleavable moiety)-(masking moiety).

29. The EpCAM activatable antibody of embodiment 28, wherein the activatable antibody comprises an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 13-15, 42, 40, and 41, respectively.

30. The EpCAM activatable antibody of embodiment 29, wherein the activatable antibody comprises an EpCAM antibody or EpCAM-binding antibody fragment comprising a VH having the sequence of SEQ ID NO: 54 and a VL having the sequence of SEQ ID NO: 89.

31. The EpCAM activatable antibody of embodiment 30, wherein the activatable antibody comprises an EpCAM antibody or EpCAM-binding antibody fragment comprising a heavy chain having the sequence of SEQ ID NO: 103 and a LC having the sequence of SEQ ID NO: 140.

32. The EpCAM activatable antibody of any one of embodiments 29-31, wherein the activatable antibody comprises a masking moiety having an amino acid sequence selected from SEQ ID NOs: 151-157.

33. The EpCAM activatable antibody of embodiment 32, wherein the activatable antibody comprises a masking moiety having the amino acid sequence of SEQ ID NO: 155.

34. The EpCAM activatable antibody of any one of embodiments 29-33, wherein the activatable antibody comprises a cleavable moiety comprising the amino acid sequence of SEQ ID NO:168 or 169.

35. An EpCAM activatable antibody comprising:

(a) an EpCAM antibody or EpCAM-binding antibody fragment comprising a VH-CDR1, VH-CDR2, VH-CDR3, VL-CDR1, VL-CDR2, and VL-CDR3 having the sequences selected from the group consisting of:

(i) SEQ ID NOs: 22, 14, 15, 42, 40, and 41, respectively;
(ii) SEQ ID NOs: 13, 14, 33, 42, 40, and 41, respectively;
(iii) SEQ ID NOs: 23, 14, 15, 42, 40, and 41, respectively, and;
(iv) SEQ ID NOs: 25, 14, 15, 42, 40, and 41, respectively.

(b) a masking moiety coupled to the EpCAM antibody or EpCAM-binding antibody fragment, wherein the masking moiety inhibits the binding of the antibody or antibody fragment to EpCAM when the activatable antibody is in an uncleaved state; and
(c) a cleavable moiety coupled to the EpCAM antibody or EpCAM-binding antibody fragment, wherein the cleavable moiety is a polypeptide that functions as a substrate for a protease;

wherein the activatable antibody in the uncleaved state has the structural arrangement from N-terminus to C-terminus as follows: (masking moiety)-(cleavable moiety)-(antibody or antibody fragment) or (antibody or antibody fragment)-(cleavable moiety)-(masking moiety).

36. The EpCAM activatable antibody of embodiment 35, wherein the activatable antibody comprises an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 22, 14, 15, 42, 40, and 41, respectively.

37. The EpCAM activatable antibody of embodiment 36, wherein the activatable antibody comprises an EpCAM antibody or EpCAM-binding antibody fragment comprising a VH having the sequence of SEQ ID NO: 75 and a VL having the sequence of SEQ ID NO: 89.

38. The EpCAM activatable antibody of embodiment 37, wherein the activatable antibody comprises an EpCAM antibody or EpCAM-binding antibody fragment comprising a heavy chain having the sequence of SEQ ID NO: 125 and a light chain having the sequence of SEQ ID NO: 140.

39. The EpCAM activatable antibody of any one of embodiments 36-38, wherein the activatable antibody comprises a masking moiety having an amino acid sequence selected from SEQ ID NOs: 151-157 and 162-167.

40. The EpCAM activatable antibody of embodiment 39, wherein the activatable antibody comprises a masking moiety having an amino acid sequence selected from SEQ ID NO: 162-167.

41. The EpCAM activatable antibody of any one of embodiments 36-40, wherein the activatable antibody comprises a cleavable moiety comprising the amino acid sequence of SEQ ID NO:168 or 169.

42. The EpCAM activatable antibody of embodiment 35, wherein the activatable antibody comprises an EpCAM antibody or EpCAM-binding antibody fragment comprising a VH-CDR1, VH-CDR2, VH-CDR3, VL-CDR1, VL-CDR2, and VL-CDR3 having the sequences of SEQ ID NOs: 13, 14, 33, 42, 40, and 41, respectively.

43. The EpCAM activatable antibody of embodiment 42, wherein the activatable antibody comprises an EpCAM antibody or EpCAM-binding antibody fragment comprising a VH having the sequence of SEQ ID NO: 77 and a VL having the sequence of SEQ ID NO: 89.

44. The EpCAM activatable antibody of embodiment 43, wherein the activatable antibody comprises an EpCAM antibody or EpCAM-binding antibody fragment comprising a heavy chain having the sequence of SEQ ID NO: 127 and a light chain having the sequence of SEQ ID NO: 140.

45. The EpCAM activatable antibody of any one of embodiments 42-44, wherein the activatable antibody comprises a masking moiety having an amino acid sequence selected from SEQ ID NOs: 151-161.

46. The EpCAM activatable antibody of embodiment 45, wherein the activatable antibody comprises a masking moiety having an amino acid sequence selected from of SEQ ID NO: 158-161.

47. The EpCAM activatable antibody of any one of embodiments 42-46, wherein the activatable antibody comprises a

cleavable moiety comprising the amino acid sequence of SEQ ID NO:168 or 169.

48. A cell producing the antibody or EpCAM-binding antibody fragment of any one of embodiments 1-26 or the activatable antibody of any one of embodiments 27-47.

49. A method of producing an EpCAM antibody or EpCAM-binding antibody fragment, or an EpCAM activatable antibody, comprising:

(a) culturing the cell of embodiment 48; and,
(b) isolating the EpCAM antibody, EpCAM-binding antibody fragment, or EpCAM activatable antibody from the cell or cell culture.

50. The method of embodiment 49, wherein the cell is a eukaryotic cell.

51. The method of embodiment 50, wherein the cell is a CHO cell.

52. A diagnostic reagent comprising the antibody or antibody fragment of any one of embodiments 1-26 or the EpCAM activatable antibody of any one of embodiments 27-47.

53. The diagnostic reagent of embodiment 52, wherein the antibody, antibody fragment, or activatable antibody is labeled.

54. The diagnostic reagent of embodiment 53, wherein the label is selected from: a radiolabel, a fluorophore, a chromophore, an imaging agent and a metal ion.

55. A polynucleotide or set of polynucleotides encoding the antibody or antibody fragment of any of embodiments 1-26 or the activatable antibody of any one of embodiments 27-47.

56. A vector or set of vectors comprising the polynucleotide of embodiment 55.

57. A host cell comprising the polynucleotide or set of polynucleotides of embodiment 55 or the vector of embodiment 56.

58. An immunoconjugate represented by the following formula:

$$\text{EpBA}\left(\text{Cy}^{L1}\right)_{W_L},$$

wherein:

EpBA is an EpCAM antibody, EpCAM antibody fragment, or EpCAM activatable antibody according to any of embodiments 1-47, that is covalently linked to $Cy^{L1}$ through a lysine residue;
$W_L$ is an integer from 1 to 20; and
$Cy^{L1}$ is represented by the following formula:

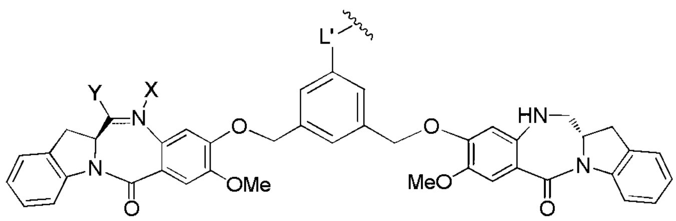
,

or

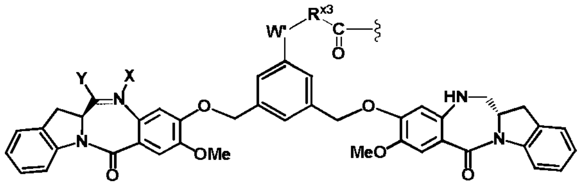

or a pharmaceutically acceptable salt thereof, wherein:

the double line $\overline{- -}$ between N and C represents a single bond or a double bond, provided that when it is a double bond, X is absent and Y is -H or a $(C_1-C_4)$alkyl; and when it is a single bond, X is -H or an amine protecting moiety, and Y is -OH or $-SO_3H$ or a pharmaceutically acceptable salt thereof;
W' is $-NR^{e'}$,
$R^{e'}$ is $-(CH_2-CH_2-O)_n-R^k$;
n is an integer from 2 to 6;
$R^k$ is -H or -Me;
$R^{x3}$ is a $(C_1-C_6)$alkyl;
L' is represented by the following formula:

$$-NR_5-P-C(=O)-(CR,R_b)_m-C(=O)- \quad (B1');$$

or

$$-NR_5-P-C(=O)-(CR_aR_b)_m-S-Z^{s1}- \quad (B2');$$

$R_5$ is -H or a $(C_1-C_3)$alkyl;
P is an amino acid residue or a peptide containing between 2 to 20 amino acid residues;
$R_a$ and $R_b$, for each occurrence, are each independently -H, $(C_1-C_3)$alkyl, or a charged substituent or an ionizable group Q;
m is an integer from 1 to 6; and
$Z^{s1}$ is selected from any one of the following formulas:

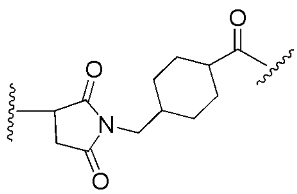
(b1);

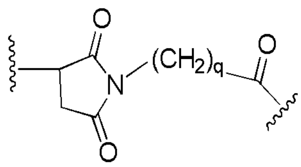
(b2);

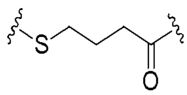
(b3);

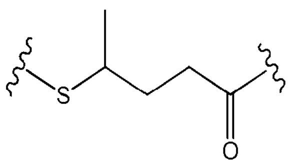
(b4);

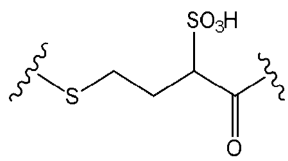
(b5),

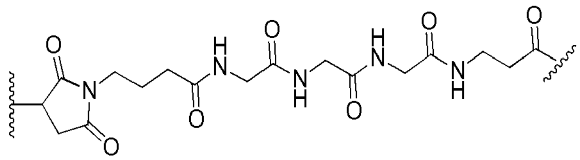
(b6),

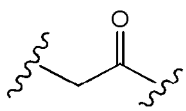 (b7); 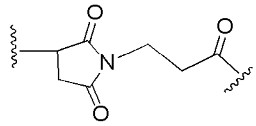 (b8); 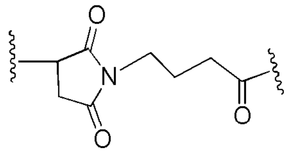 (b9);

and

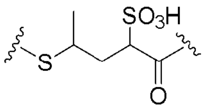 (b10),

wherein q is an integer from 1 to 5.

59. The immunoconjugate of embodiment 58, wherein $R_a$ and $R_b$ are both H; and $R_5$ is H or Me.

60. The immunoconjugate of embodiment 58 or 59, wherein P is a peptide containing 2 to 5 amino acid residues.

61. The immunoconjugate of any one of embodiments 58-60, wherein P is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-$N^9$-tosyl-Arg, Phe-$N^9$-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:215), β-Ala-Leu-Ala-Leu (SEQ ID NO:216), Gly-Phe-Leu-Gly (SEQ ID NO:217), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, Met-Ala, Gln-Val, Asn-Ala, Gln-Phe and Gln-Ala.

62. The immunoconjugate of embodiment 61, wherein P is Gly-Gly-Gly, Ala-Val, Ala-Ala, Ala-D-Ala, D-Ala-Ala, or D-Ala-D-Ala.

63. The immunoconjugate of any one of embodiments 58-62, wherein Q is $-SO_3H$ or a pharmaceutically acceptable salt thereof.

64. The immunoconjugate of embodiment 58, wherein the immunoconjugate is represented by the following formula:

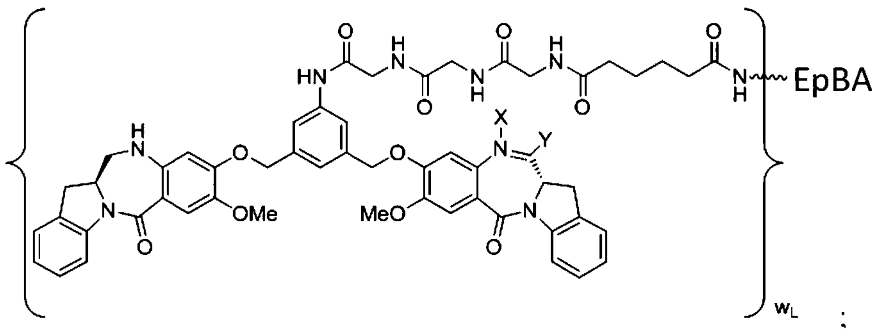 ;

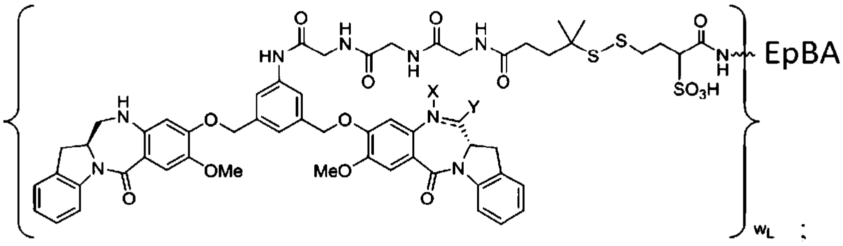 ;

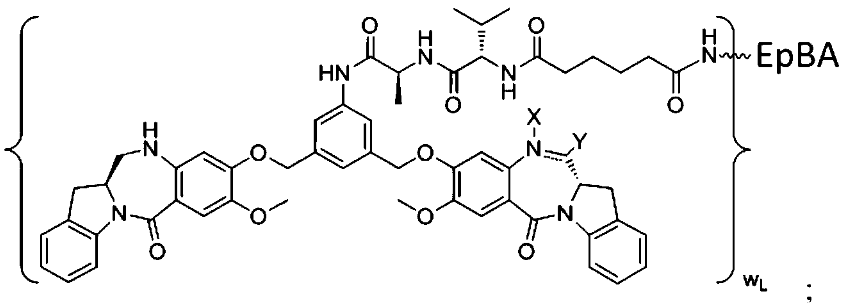
;

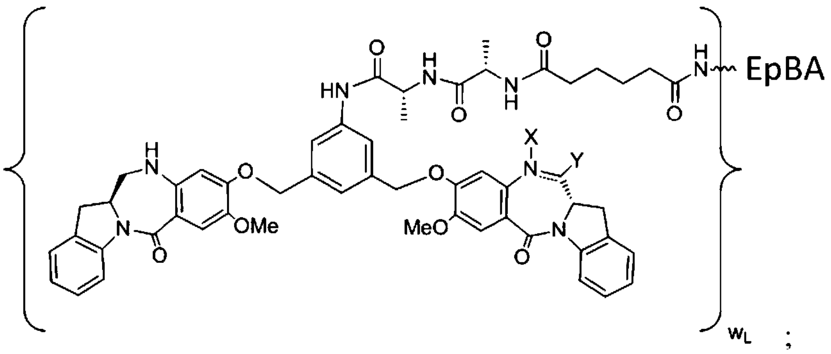
;

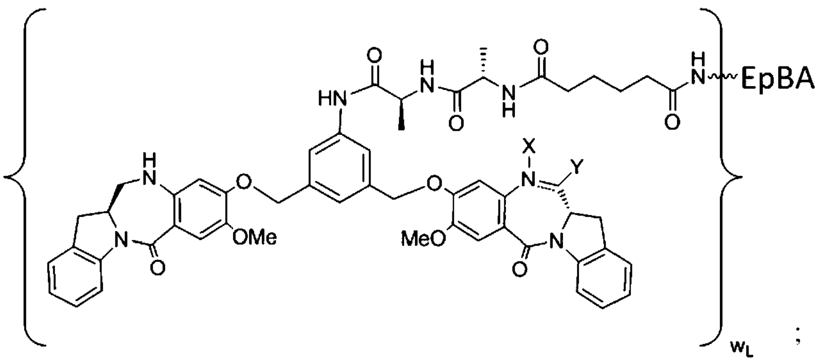
;

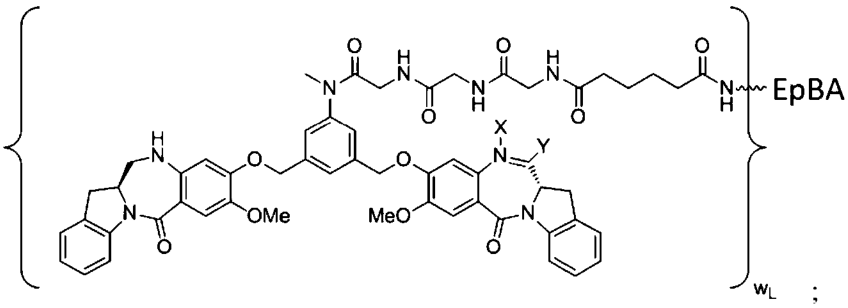
;

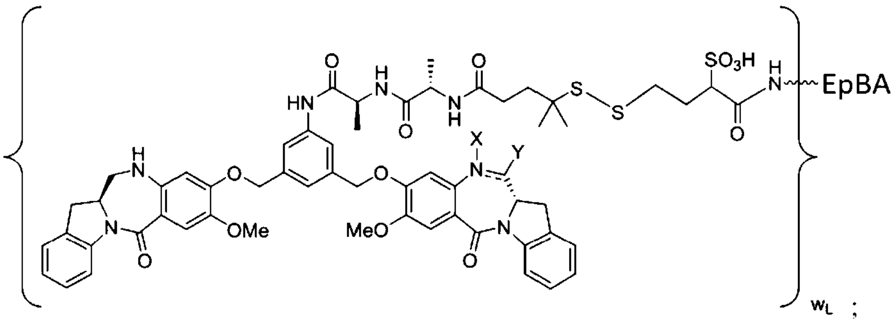
;

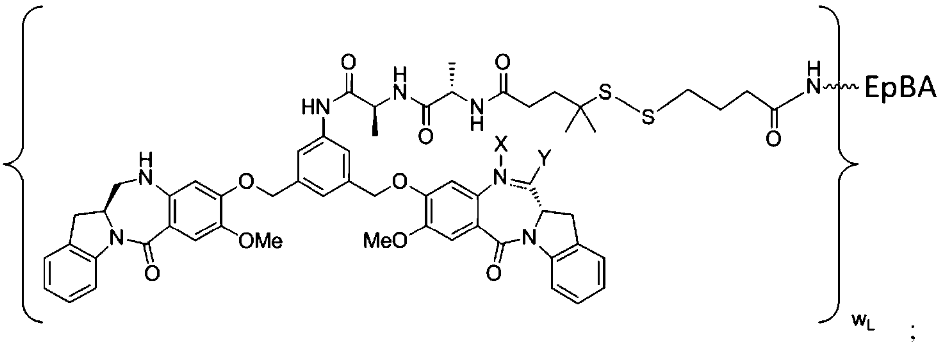
;

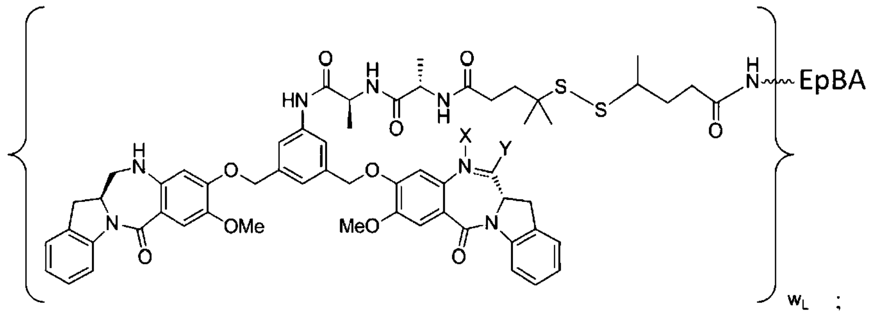
;

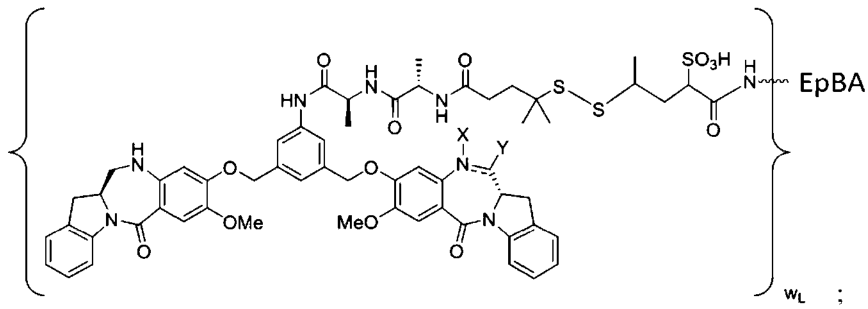
;

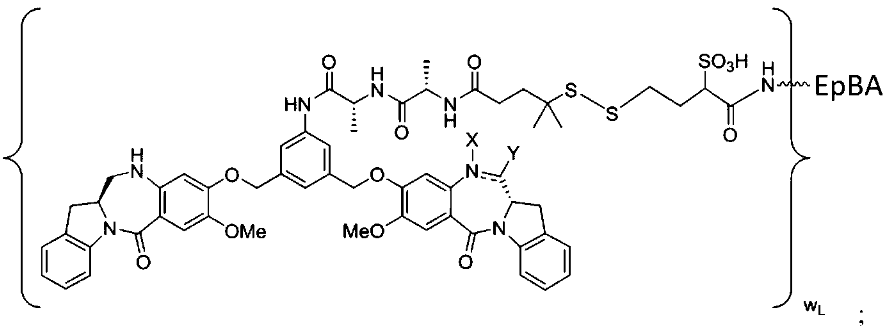
;

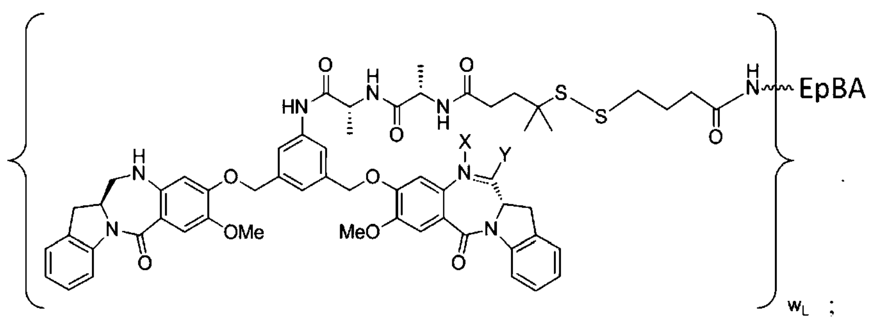
;

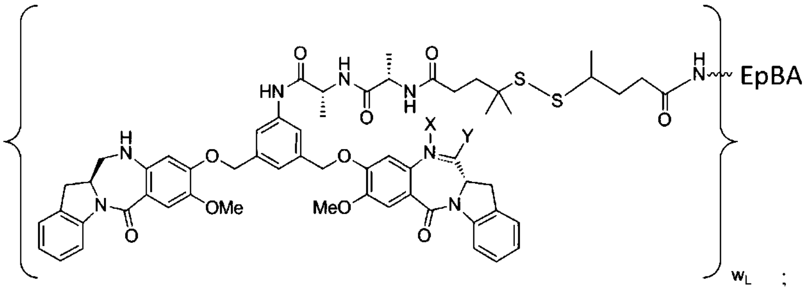

;

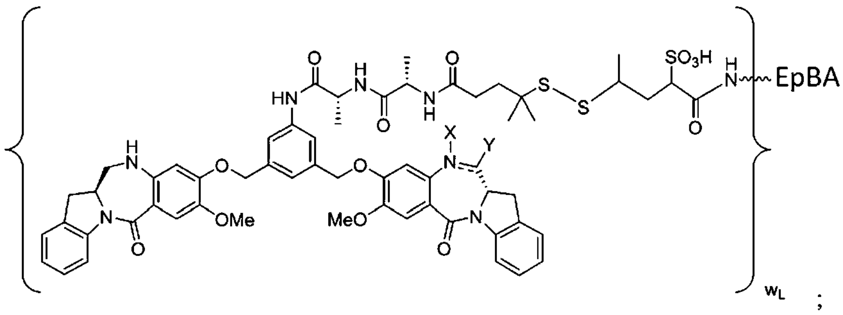

;

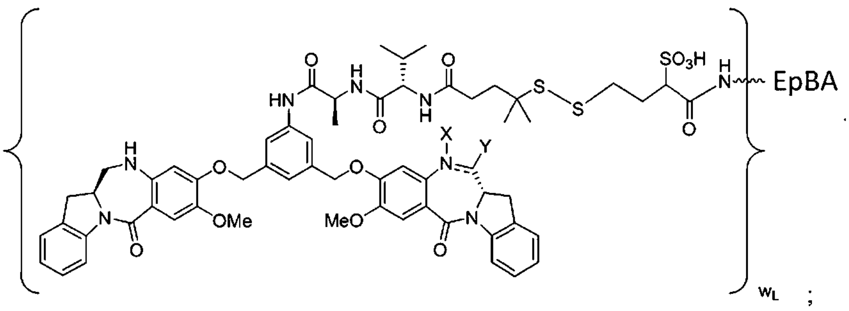

;

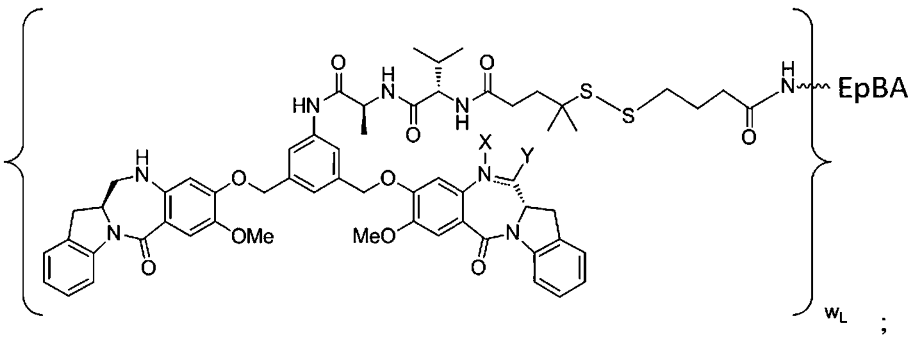

;

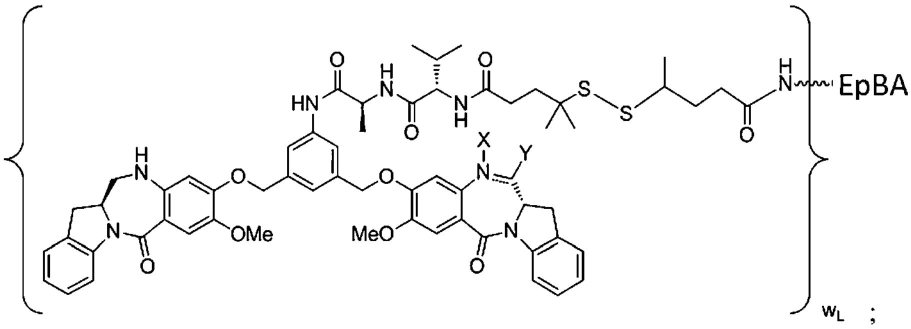

;

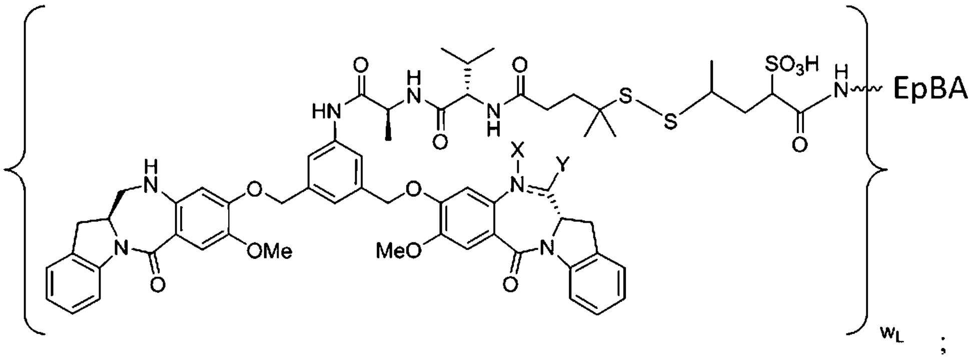

;

or

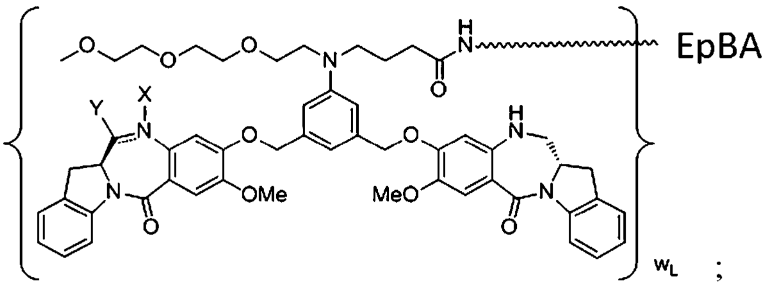

;

or a pharmaceutically acceptable salt thereof, wherein

$W_L$ is an integer from 1 to 10; the double line ⎓ between N and C represents a single bond or a double bond, provided that when it is a double bond, X is absent and Y is -H; and when it is a single bond, X is -H, and Y is -OH or $-SO_3H$ or a pharmaceutically acceptable salt thereof.

65. The immunoconjugate of any one of embodiments 58-64, wherein the double line ⎓ between N and C represents a double bond, X is absent and Y is -H.

66. The immunoconjugate of any one of embodiments 58-64, wherein the double line ⎓ between N and C represents a single bond, X is -H, and Y is $-SO_3H$ or a pharmaceutically acceptable salt thereof.

67. The immunoconjugate of embodiment 66, wherein Y is $-SO_3H$, $-SO_3Na$ or $-SO_3K$.

68. The immunoconjugate of embodiment 66, wherein Y is $-SO_3Na$.

69. An immunoconjugate represented by the following formula:

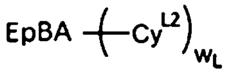

,

wherein:

EpBAis an EpCAM antibody, EpCAM antibody fragment, or EpCAM activatable antibody according to any of embodiments 1-47 that is covalently linked to $Cy^{L2}$ through a lysine residue;

$W_L$ is an integer from 1 to 20;

$Cy^{L2}$ is represented by the following formula:

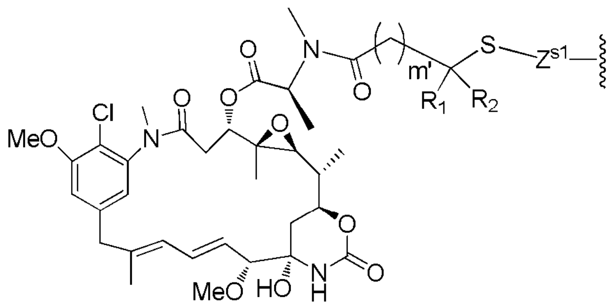

,

m' is 1 or 2;

$R_1$ and $R_2$, are each independently H or a $(C_1\text{-}C_3)$alkyl; and

$Z^{s1}$ is selected from any one of the following formulas:

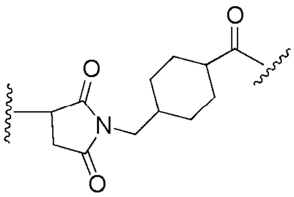 (b1); 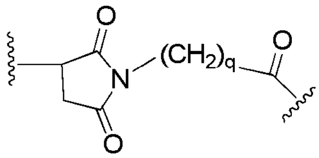 (b2); 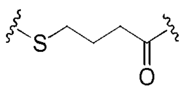 (b3);

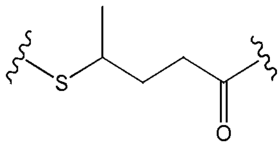 (b4); 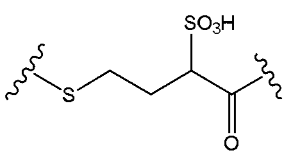 (b5),

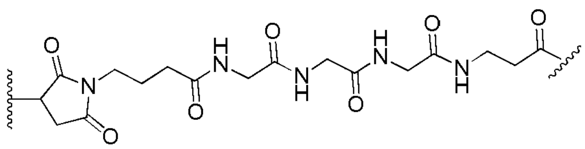 (b6),

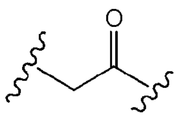 (b7); 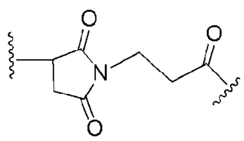 (b8); 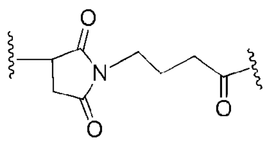 (b9), and 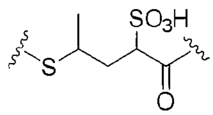 (b10),

wherein q is an integer from 1 to 5.

70. The immunoconjugate of embodiment 69, wherein m' is 1, and $R_1$ and $R_2$ are both H.

71. The immunoconjugate of embodiment 69, wherein m' is 2, and R1 and R2 are both Me.

72. The immunoconjugate of embodiment 69 wherein the immunoconjugate is represented by the following formula:

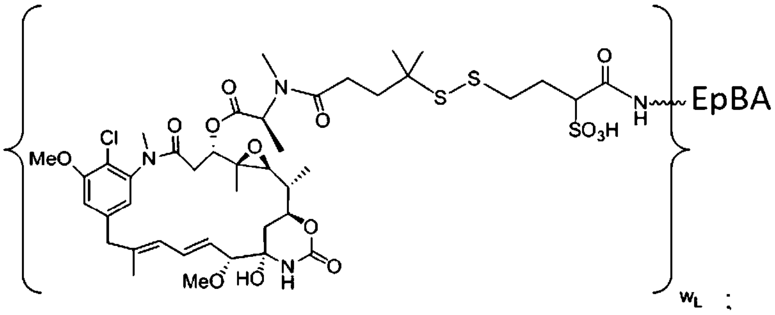

;

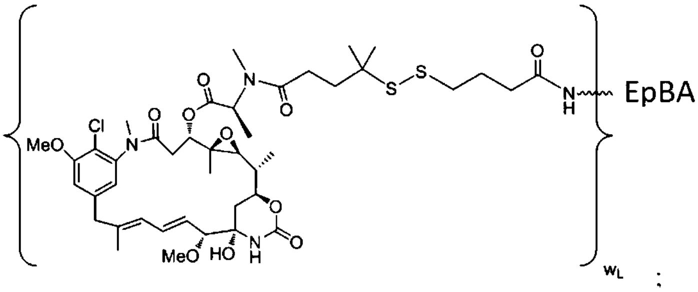

;

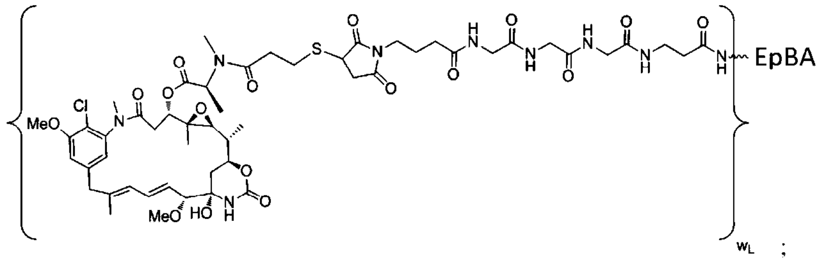

;

or

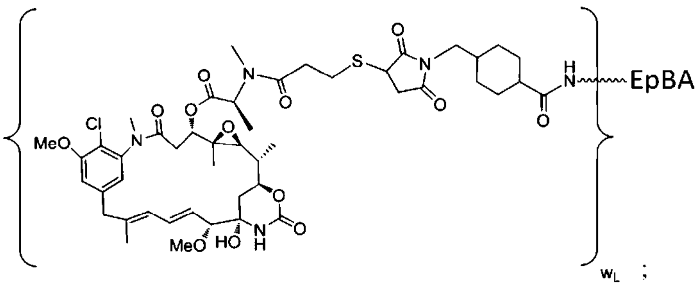

;

or a pharmaceutically acceptable salt thereof, wherein $W_L$ is an integer from 1 to 10.

73. The immunoconjugate of embodiment 69, wherein the immunoconjugate is represented by the following formula:

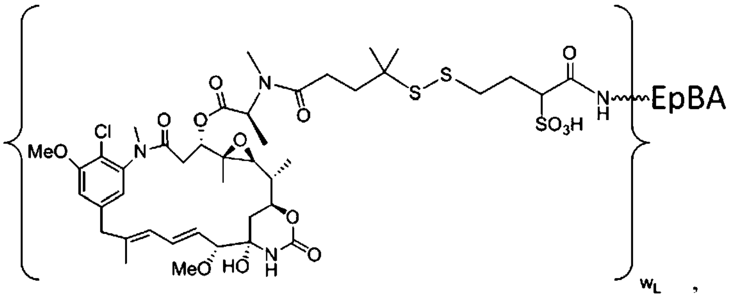

,

or a pharmaceutically acceptable salt thereof.

74. An immunoconjugate represented by the following formula:

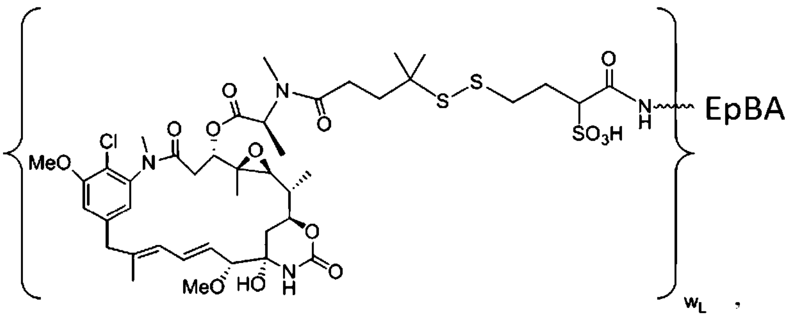

or a pharmaceutically acceptable salt thereof, wherein:

$W_L$ is an integer from 1 to 10;
EpBA is an EpCAM antibody, EpCAM antibody fragment, or EpCAM activatable antibody comprising a VH-CDR1, VH-CDR2, VH-CDR3, VL-CDR1, VL-CDR2, and VL-CDR3 having the sequences of SEQ ID NOs:13-15, 42, 40, and 41, respectively.

75. The immunoconjugate of embodiment 74, wherein the isolated antibody, or EpCAM-binding antibody fragment comprises a heavy chain variable region (VH) and a light chain variable region (VL) having sequences of SEQ ID NO:54 and SEQ ID NO:89, respectively.

76. The immunoconjugate of embodiment 74, wherein the isolated antibody comprises a heavy chain and a light chain having the sequences of SEQ ID NO: 103 and SEQ ID NO:140, respectively.

77. The immunoconjugate of any one of embodiments 74-76, wherein the immunoconjugate comprises a activatable antibody comprising a masking moiety having an amino acid sequence selected from SEQ ID NOs:151-157.

78. The immunoconjugate of embodiment 77, wherein the masking moiety has the amino acid sequence of SEQ ID NO:155.

79. The immunoconjugate of embodiment 77 or 78, wherein the immunoconjugate comprises a activatable antibody further comprising a cleavable moiety of SEQ ID NO:168 or SEQ ID NO:169.

80. An immunoconjugate comprising an EpBA coupled to a maytansinoid compound DM21L (also referred to as LDL-DM) represented by the following structural formula:

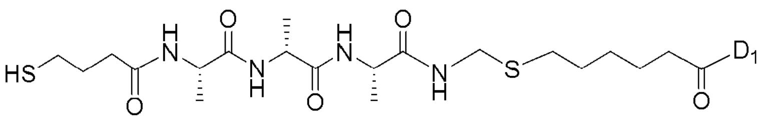 (D-2);

via □ -maleimidobutyric acid N-succinimidyl ester (GMBS) or a
N-(y-maleimidobutryloxy)sulfosuccinimide ester (sulfo-GMBS or sGMBS) linker, wherein EpBA is an EpCAM antibody, EpCAM antibody fragment, or EpCAM activatable antibody according to any of embodiments 1-47.

81. The immunoconjugate of embodiment 80, wherein the GMBS and sulfo-GMBS (or sGMBS) linkers are represented by the following formula:

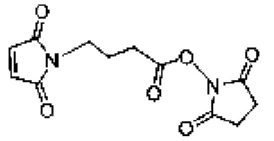 GMBS 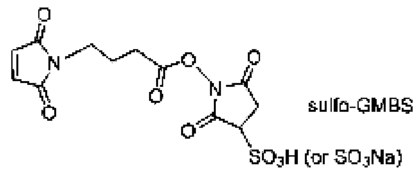 sulfo-GMBS

.

82. The immunoconjugate of embodiment 80 or 81, represented by the following formula:

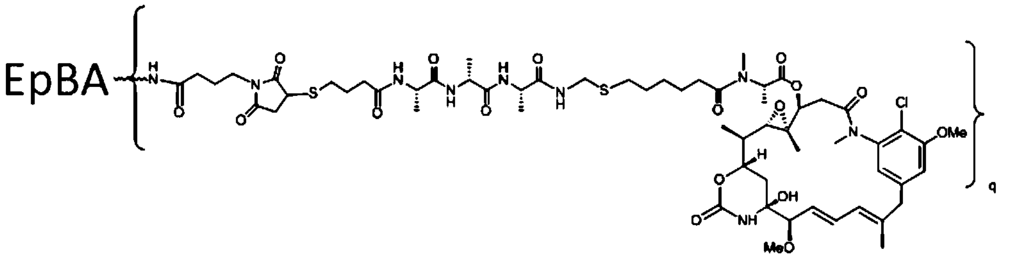

,

wherein:
EpBA is connected to the maytansinoid compound through a Lys amine group, wherein q is an integer from 1 or 10.

83. The immunoconjugate of any of embodiments embodiment 80-82, wherein the EpBA comprises a VH-CDR1, VH-CDR2, VH-CDR3, VL-CDR1, VL-CDR2, and VL-CDR3 comprising the sequences of SEQ ID NOs: 13-15, 42, 40, and 41, respectively.

84. The immunoconjugate of embodiment 83, wherein the EpBA comprises a heavy chain variable region (VH) and a light chain variable region (VL) having sequences of SEQ ID NO:54 and SEQ ID NO:89, respectively.

85. The immunoconjugate of embodiment 84, wherein the EpBA comprises a heavy chain and a light chain having the sequences of SEQ ID NO: 103 and SEQ ID NO: 140, respectively.

86. The immunoconjugate of any one of embodiments 83-85, wherein the immunoconjugate comprises a activatable antibody comprising a masking moiety having an amino acid sequence selected from SEQ ID NOs:151-157.

87. The immunoconjugate of embodiment 86, wherein the masking moiety has the amino acid sequence of SEQ ID NO:155.

88. The immunoconjugate of embodiment 86 or 87, wherein the immunoconjugate comprises a activatable antibody further comprising a cleavable moiety of SEQ ID NO:168 or SEQ ID NO:169.

89. The immunoconjugates of any one of embodiments 58-88, wherein the pharmaceutically acceptable salt is a sodium or a potassium salt.

90. A pharmaceutical composition comprising the antibody, antigen-binding fragment, or activatable antibody of any one of embodiments 1-47 and a pharmaceutically acceptable carrier.

91. A pharmaceutical composition comprising the immunoconjugate of any of embodiments 58-88 and a pharmaceutically acceptable carrier.

92. A method of killing a cancer cell that comprises contacting the cancer cell with an effective amount of an antibody, antigen-binding fragment, or activatable antibody of any one of embodiments 1-47, immunoconjugate of any of embodiments 58-88, or pharmaceutical composition of embodiment 90 or 91.

93. The method of embodiment 92 wherein the cancer cell is an epithelial cancer cell, a breast cancer cell, lung cancer cell, stomach cancer cell, colorectal cancer, prostate cancer cell, ovarian cancer cell, colon cancer cell, rectal cancer

cell or a cancer stem cell.

94. The method of embodiment 92 wherein the cancer cell is an ovarian cancer cell, a uterine cancer cell, a gastric cancer cell, a pancreatic cancer cell, or a colorectal cancer cell.

95. A method of treating cancer that expresses EpCAM, the method comprising administering a therapeutically effective amount of an antibody or antigen-binding fragment of any one of embodiments 1-47, immunoconjugate of any of embodiments 58-88, or pharmaceutical composition of embodiment 90 or 91, to a subject in need thereof.

96. The method of treatment according to embodiment 95, wherein the cancer is an epithelial cancer.

97. The method of treatment according to embodiment 95, wherein the cancer is breast cancer, lung cancer, stomach cancer, colorectal cancer, prostate cancer, ovarian cancer, colon cancer, esophageal cancer, tracheal cancer, gastric cancer bladder cancer, uterine cancer, rectal cancer, cancer of the small intestine, or a metastases thereof.

98. The method of treatment according to embodiment 95, wherein the cancer is ovarian cancer, uterine cancer, gastric cancer, pancreatic cancer, colorectal cancer, or a metastases thereof.

99. The method of treatment of embodiment 97, wherein the cancer is lung cancer.

100. The method of treatment of embodiment 99, wherein the lung cancer is non-small cell lung cancer.

101. The method of treatment of embodiment 100, wherein the non-small cell lung cancer is non-squamous non-small cell lung cancer.

102. The method of treatment of embodiment 95, wherein the cancer is ovarian cancer.

103. The method of treatment of embodiment 95, wherein the cancer is triple negative breast cancer.

104. The method of treatment of embodiment 95, wherein the cancer is colorectal cancer.

105. The method of treatment of embodiment 95, wherein the cancer is esophageal cancer.

106. The method of treatment of embodiment 95, wherein the cancer is gastric cancer.

107. The method of treatment of embodiment 95, wherein the cancer is uterine cancer.

108. The method of treatment of embodiment 95, wherein the cancer is pancreatic cancer.

## Claims

**1.** A method of producing an immunoconjugate comprising an EpCAM-binding agent (EpBA) comprising the step of reacting the EpBA with a cytotoxic agent-linker compound to produce the immunoconjugate, wherein the EpBA is an EpCAM antibody, EpCAM-binding antibody fragment, or EpCAM activatable antibody comprising a heavy chain CDR1 (VH-CDR1), a heavy chain CDR2 (VH-CDR2), a heavy chain CDR3 (VH-CDR3), a light chain CDR1 (VL-CDR1), a light chain CDR2 (VL-CDR2), and a light chain CDR3 (VL-CDR3) having the sequences selected from the group consisting of:

(a) SEQ ID NOs: 13-15, 42, 40, and 41, respectively;
(b) SEQ ID NOs: 13-15, and 39-41, respectively;
(c) SEQ ID NOs: 13, 26, 15, and 39-41, respectively; and
(d) SEQ ID NOs: 13, 26, 15, 42, 40, and 41, respectively.

**2.** The method of claim 1, wherein the cytotoxic agent-linker compound is formed by the step of reacting the cytotoxic agent with a linker compound.

**3.** The method of claim 2, wherein the cytotoxic agent comprises an amine reactive group.

4. The method of claim 2, wherein the linker compound comprises an amine reactive group.

5. The method of claim 2, wherein the linker compound comprises a thiol reactive group.

6. The method of claim 2, wherein the linker compound comprises an amine reactive group and a thiol reactive group.

7. The method of claim 1, wherein the immunoconjugate produced is represented by the following formula:

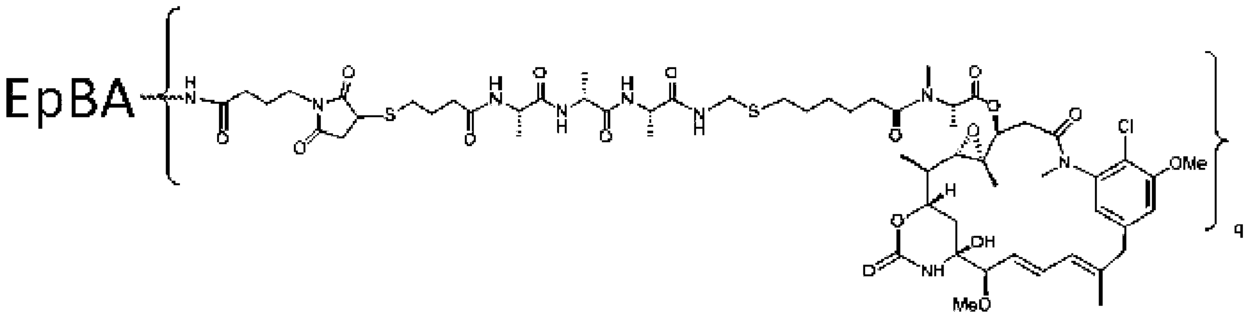

wherein q is an integer from 1 to 10, and wherein q is conjugated to lysine residues of EpBA.

8. The method of claim 7, wherein q is 3 or 4.

9. The method of claim 1, wherein the cytotoxic agent-linker compound is formed by the step of reacting GMBS, represented by the formula a9L, with a maytansinoid compound represented by the formula (D-1)

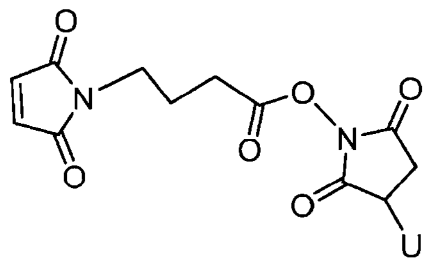

(a9L)

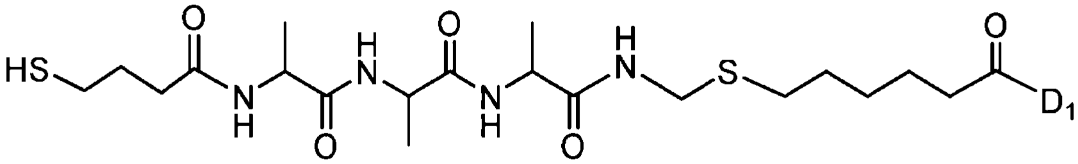

(D-1),

wherein $D_1$ is represented by the formula:

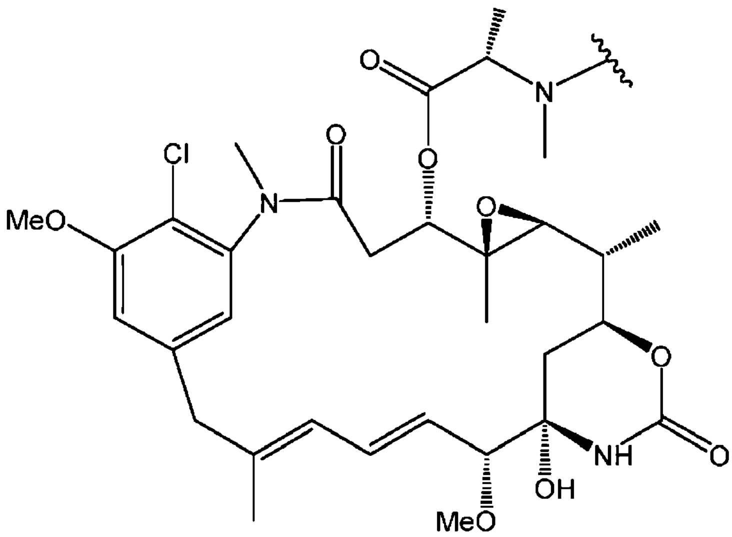

wherein U is -H, wherein a maytansinoid-linker compound is formed as the cytotoxic agent-linker compound.

**10.** The method of any one of claims 1 to 9, wherein the EpBA is:

(a) an EpCAM activatable antibody;
(b) an EpCAM antibody; or
(c) an EpCAM-binding antibody fragment.

**11.** The method of any one of claims 1 to 9, wherein the EpBA comprises:

(a) a VH-CDR1 comprising the sequence NYYIH (SEQ ID NO:13);
(b) a VH-CDR2 comprising the sequence WIYPGNVYIQYNEKFKG (SEQ ID NO:14);
(c) a VH-CDR3 comprising the sequence DGPWFAY (SEQ ID NO:15);
(d) a VL-CDR1 comprising the sequence RSSRSLLHSDGFTYLY (SEQ ID NO:42);
(e) a VL-CDR2 comprising the sequence QTSNLAS (SEQ ID NO:40); and
(f) a VL-CDR3 comprising the sequence AQNLELPNT (SEQ ID NO:41)

**12.** The method of claim 11, wherein the EpBA is an EpCAM activatable antibody.

**13.** The method of claim 11, wherein the EpBA is an EpCAM antibody.

**14.** The method of claim 11, wherein the EpBA is an EpCAM-binding antibody fragment.

**15.** The method of claim 12, wherein the EpCAM activatable antibody comprises:

(a) a masking moiety (MM) coupled to the EpCAM antibody or EpCAM-binding antibody fragment thereof, wherein the masking moiety comprises the sequence WWPPCQGGAWCEQRI (SEQ ID NO:155); and
(b) a cleavable moiety (CM) coupled to the EpCAM antibody or EpCAM-binding antibody fragment thereof, wherein the cleavable moiety comprises the sequence ISSGLLSGRSDNI (SEQ ID NO: 169).

FIG. 1A

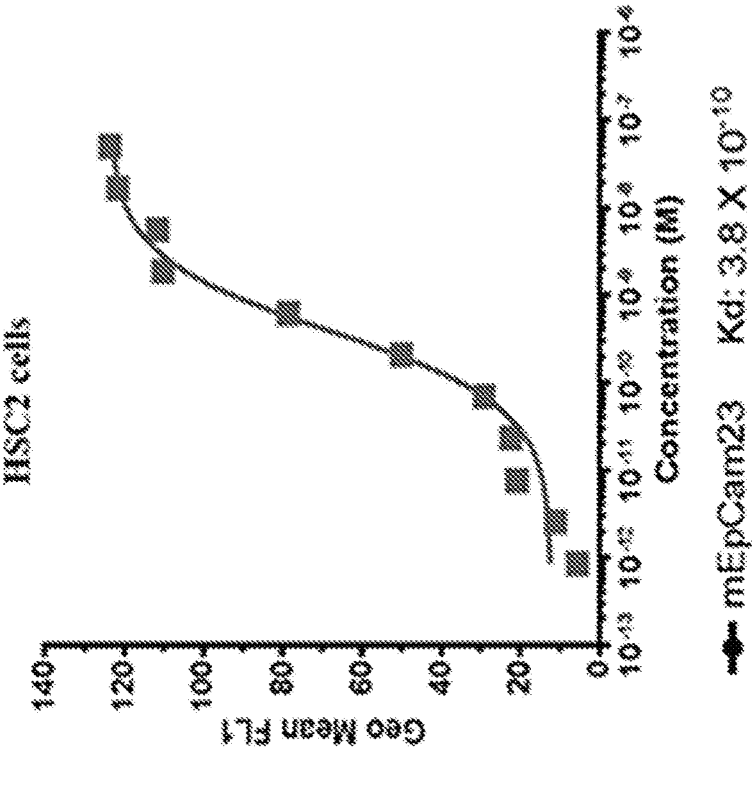
HSC2 cells
Geo Mean FL1
0
20
40
60
80
100
120
140
Concentration (M)
mEpCam23 Kd: 3.8 X 10-10

FIG. 1B

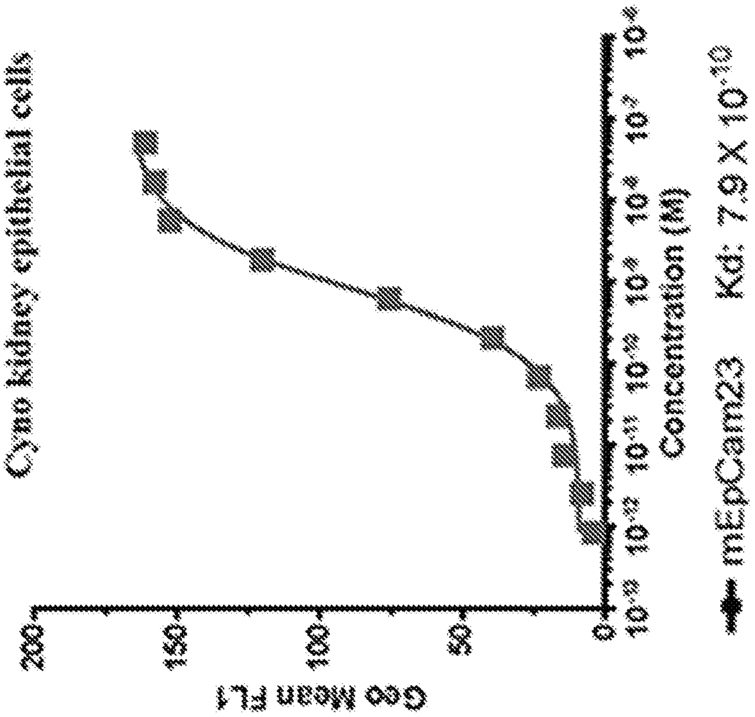
Cyno kidney epithelial cells
Geo Mean FL1
0
50
100
150
200
Concentration (M)
mEpCam23 Kd: 7.9 X 10-10

# FIG. 2A

```
                <---------FR1-IMGT--------><CDR1-IMGT><---FR2-IMGT---><C><-------------FR3-IMGT-----------
muEpCAM23     1 DIVLTQTPFSNPVTLGTSASISCRSSKSLLHSDGFTYLYWFLQKPGQSPHLLIYQTSNLASGVPDRFSSSGSGTDFTLRISRVEAEDVGV  90
IGKV2D29*02   1 ...M....L.LS..P.QP.....K..Q.......K....Y........Q....EV..RF........G.........K...........  90
IGKV2D29*01   1 ...M....L.LS..P.QP.....K..Q.......K....Y......P.Q....EV..RF........G.........K...........  90
IGKV229*02    1 ...M....L.LS..P.QP.....K..Q.......K....Y........Q....EV.SRF........G.........K...........  90

                   -->
muEpCAM23     91  YYCAQNLELP  100
IGKV2D29*02   91  ...M.SIQ..  100
IGKV2D29*01   91  ...M.SIQ..  100
IGKV229*02    91  ...M.GIH..  100
```

# FIG. 2B

```
              <-------FR1-IMGT--------><CDR1-I><---FR2-IMGT----><CDR2-I><--------------FR3-IMGT-------------->
muEpCAM23     EVKLEESGPALVKPGASVRISCKASGYTFTNYYIHWVKQRPGQGLDYIGWIYPGNVYIQYNEKFKGKATLTADKSSSTAFMQLSSLTSEDSAVYFCAR 98
IGHV13*01     Q.Q.VQ..AEVK......KV..........S.AM...R.A...R.EWM...NA..GNTK.SQ..Q.RV.I.R.T.A...Y.E....R...T...Y... 98
IGHV12*02     Q.Q.VQ..AEVK......KV..........G..M...R.A.....EWM...N.NSGGTN.AQ..Q.RV.M.R.T.I...Y.E..R.R.D.T...Y... 98
IGHV12*04     Q.Q.VQ..AEVK......KV..........G..M...R.A.....EWM...N.NSGGTN.AQ..Q.WV.M.R.T.I...Y.E..R.R.D.T...Y... 98
```

FIG. 3A

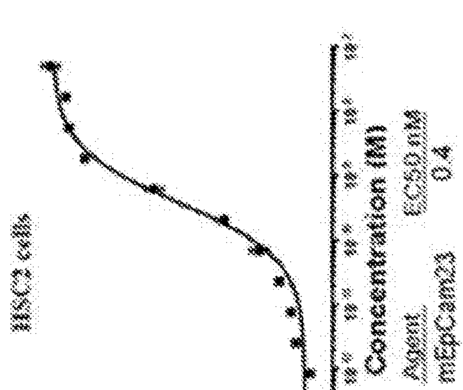


FIG. 3B

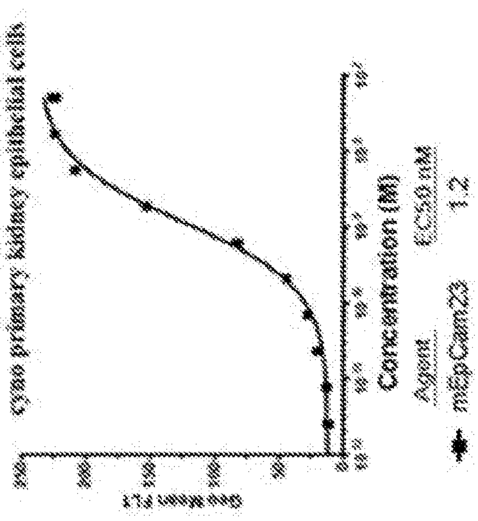


FIG. 3C

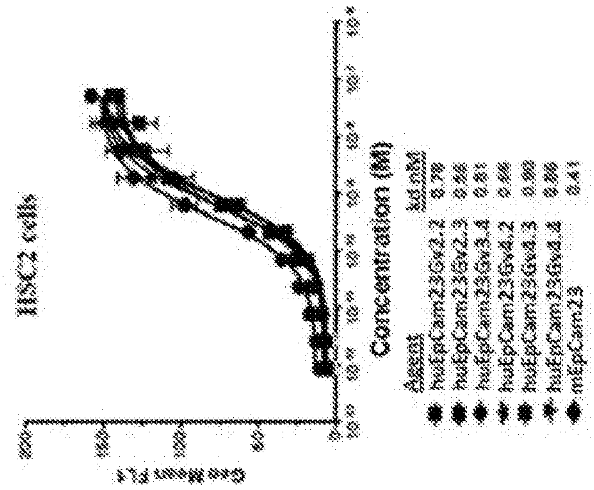


FIG. 3D

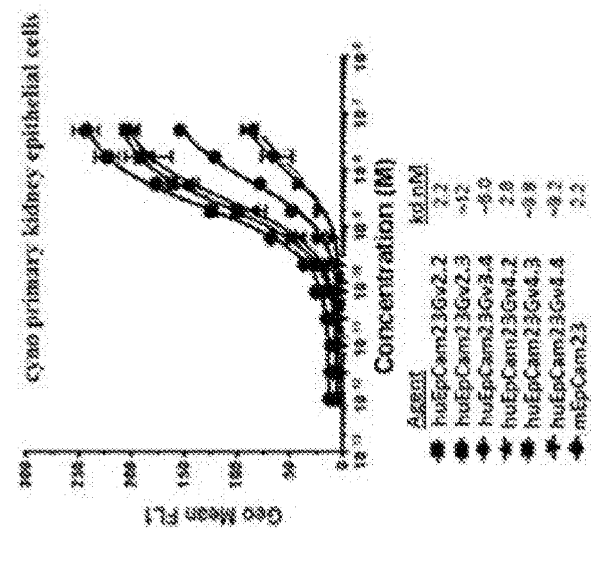

FIG. 3F

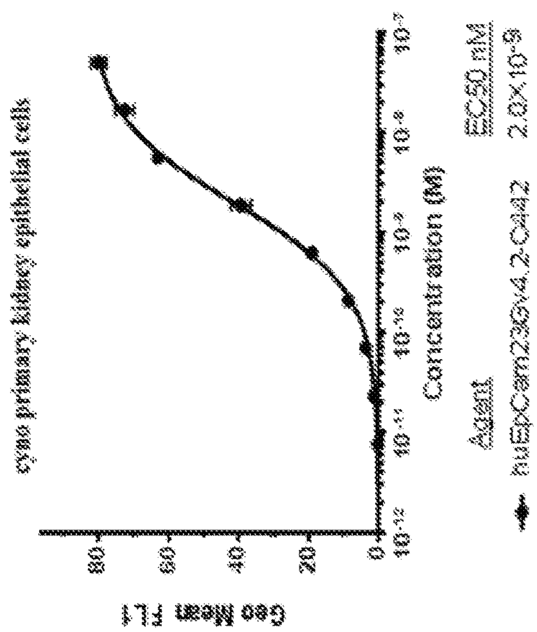
cyno primary kidney epithelial cells
Geo Mean FL1
80
60
40
20
0
10-12
10-11
10-10
10-9
10-8
10-7
Concentration (M)
Agent
huEpCam23Gv4.2-C442
EC50 nM
2.0X10-9

FIG. 3E

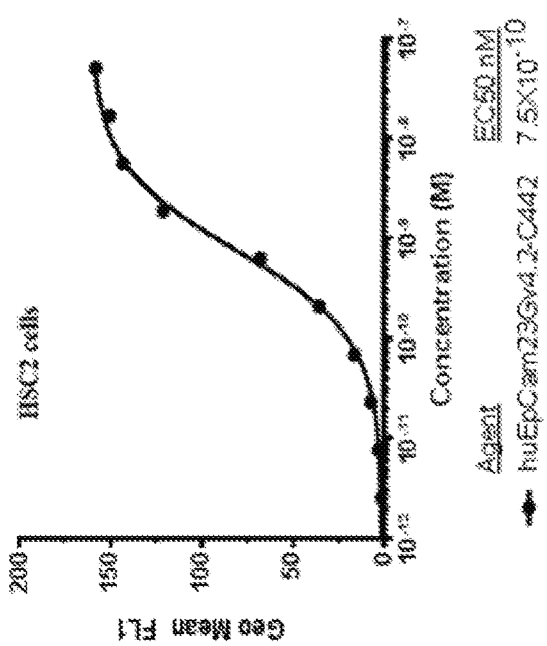
HSC2 cells
Geo Mean FL1
200
150
100
50
0
10-12
10-11
10-10
10-9
10-8
10-7
Concentration (M)
Agent
huEpCam23Gv4.2-C442
EC50 nM
7.5X10-10

**FIG. 4A**

```
              1                                                          60
    VL Gv4    DIVLTQTPLSLSVTPGQPASISCRSSRSLLHSDGFTYLYWFLQKPGQSPQLLIYQTSNLA
    VL Gv2    DIVLTQTPLSLSVTPGQPASISCRSSKSLLHSDGFTYLYWFLQKPGQSPQLLIYQTSNLA
mVL EpCAM23   DIVLTQTPFSNPVTLGTSASISCRSSKSLLHSDGFTYLYWFLQKPGQSPHLLIYQTSNLA
              ******** *  ** *  ******** ********************** **********

              61                                                  112
    VL Gv4    SGVPDRFSSSGSGTDFTLKISRVEAEDVGVYYCAQNLELPNTFGQGTKLEIK (SEQ ID NO:89)
    VL Gv2    SGVPDRFSSSGSGTDFTLKISRVEAEDVGVYYCAQNLELPNTFGQGTKLEIK (SEQ ID NO:87)
mVL EpCAM23   SGVPDRFSSSGSGTDFTLRISRVEAEDVGVYYCAQNLELPNTFGGGTKLEIK (SEQ ID NO:85)
              ****************** ************************* *******
```

**FIG. 4B**

```
            1                                                          60
  VH Gv2    QVQLVQSGAEVKKPGASVKVSCKASGYTFTNYYIHWVRQAPGQRLEYIGWIYPGNVYIQY
mEpCAM23    EVKLEESGPALVKPGASVRISCKASGYTFTNYYIHWVKQRPGQGLDYIGWIYPGNVYIQY
             * *  **    ******  ***************** * *** * **************

            61                                                     116
  VH Gv2    NEKFKGRATLTADKSASTAYMELSSLRSEDTAVYYCARDGPWFAYWGQGTLVTVSS (SEQ ID NO:54)
mEpCAM23    NEKFKGKATLTADKSSSTAFMQLSSLTSEDSAVYFCARDGPWFAYWGQGTLVTVSS (SEQ ID NO:52)
            ****** ******** *** * **** *** *** *********************
```

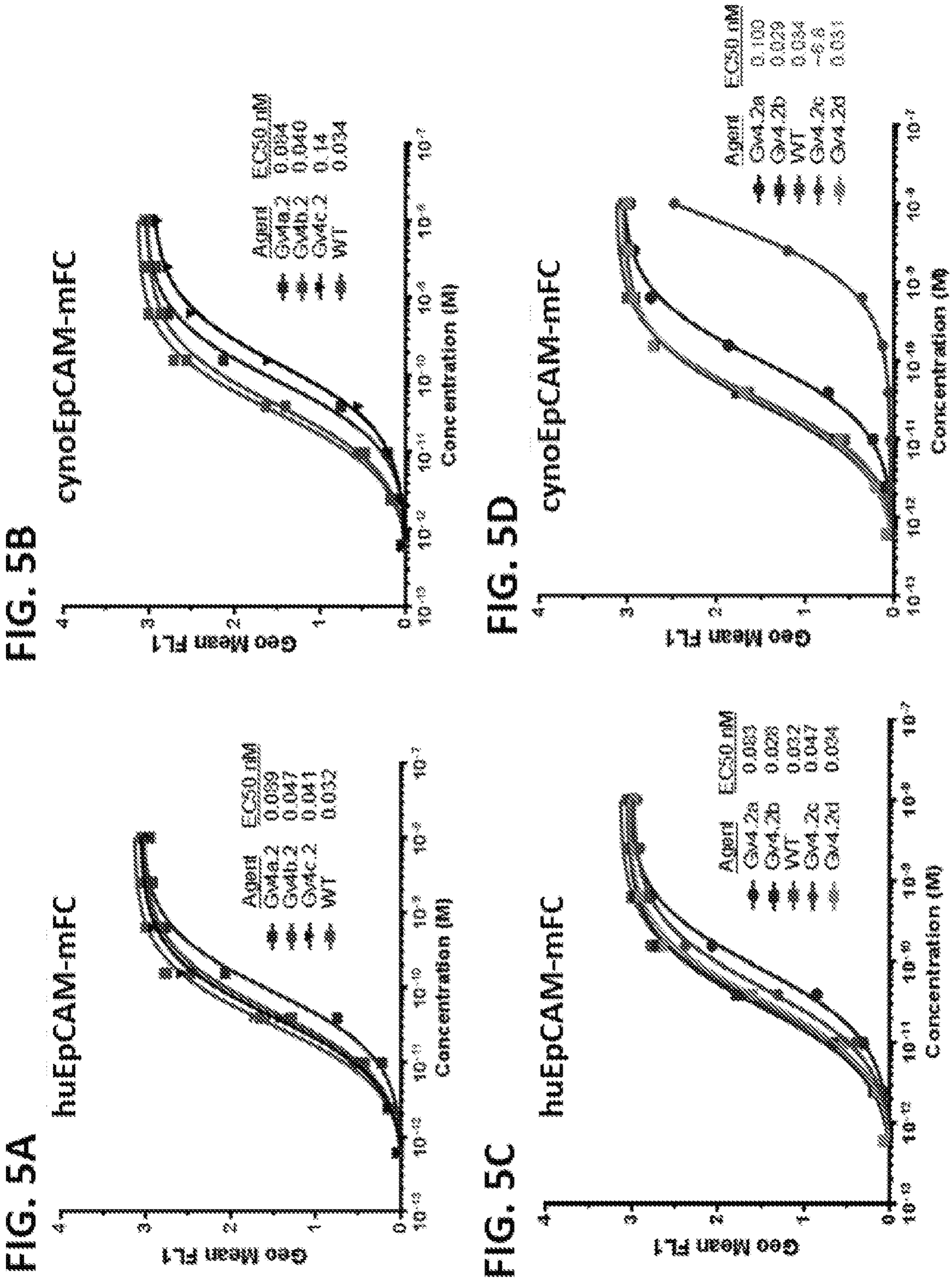


FIG. 5A FIG. 5B FIG. 5C FIG. 5D

FIG. 5E

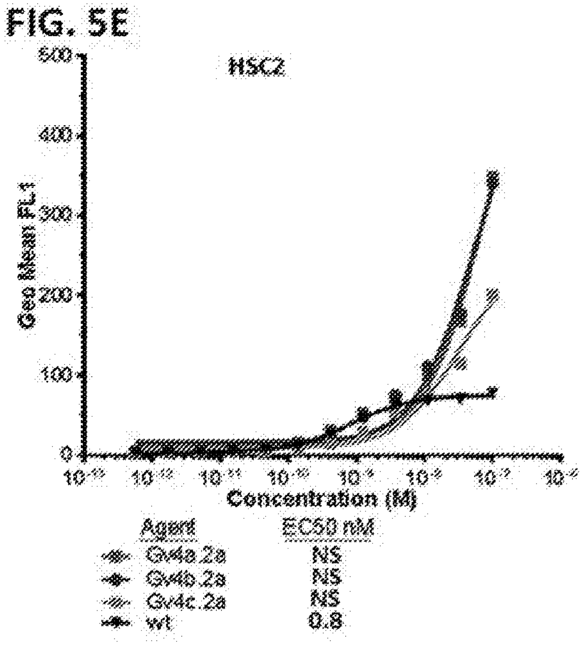


FIG. 5F

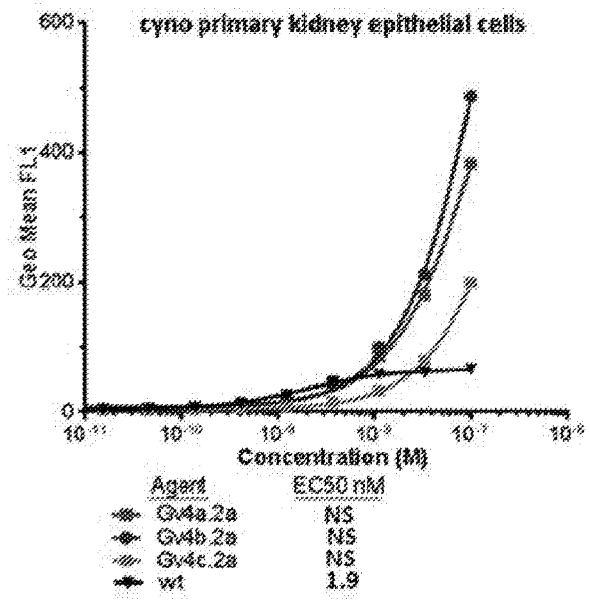


FIG. 5G

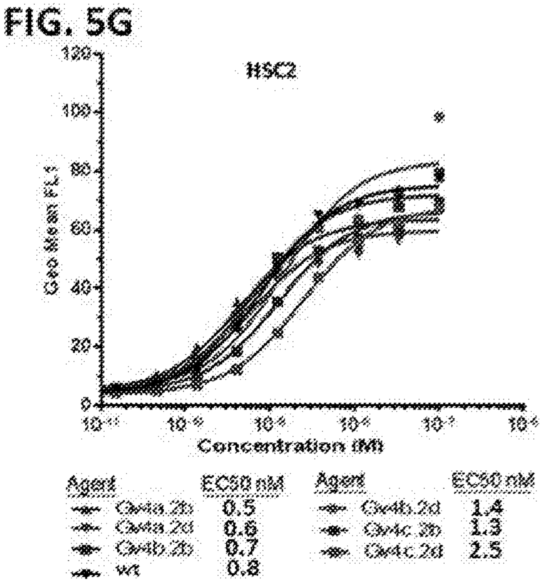


FIG. 5H

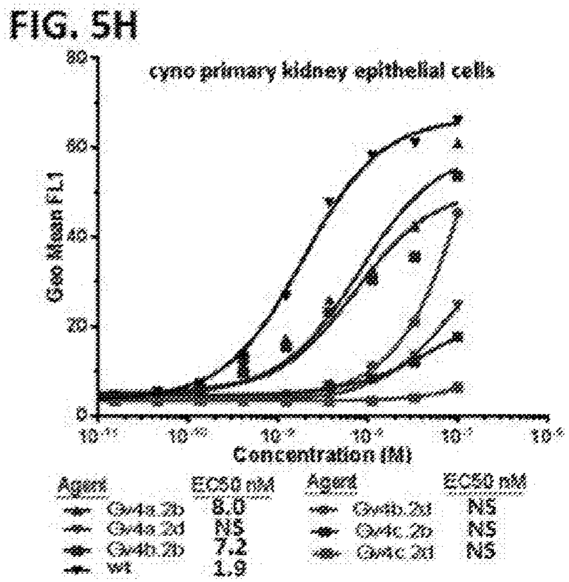

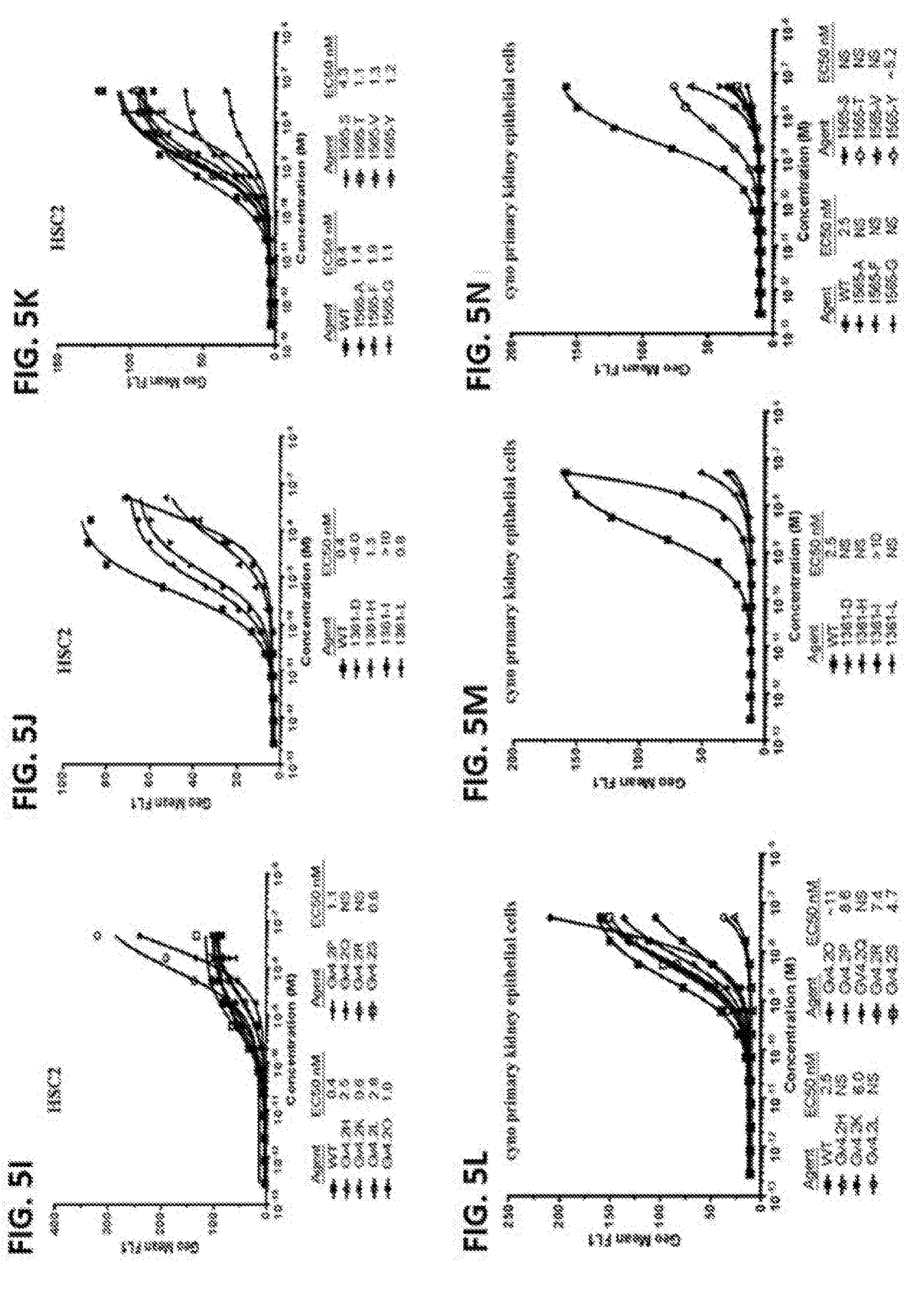


FIG. 5I

FIG. 5J

FIG. 5K

FIG. 5L

FIG. 5M

FIG. 5N

FIG. 6A

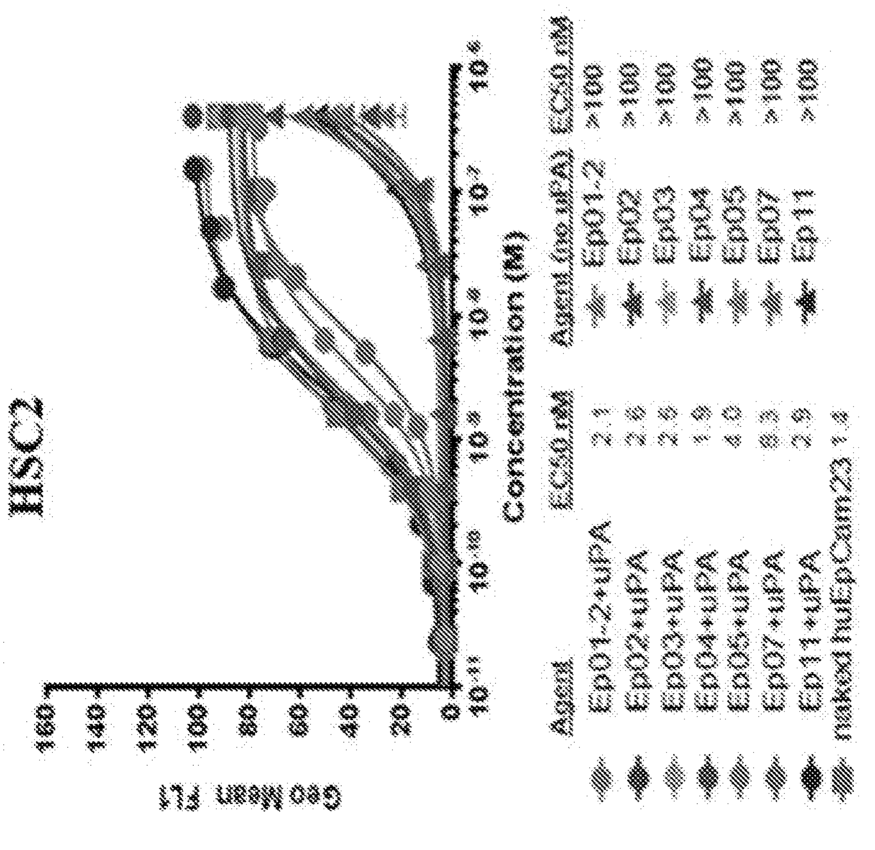
HSC2
Geo Mean FL1
Concentration (M)
Agent
Ep01-2+uPA
Ep02+uPA
Ep03+uPA
Ep04+uPA
Ep05+uPA
Ep07+uPA
Ep11+uPA
naked huEpCam23
EC50 nM
2.1
2.6
2.6
1.9
4.0
6.3
2.9
1.4
Agent (no uPA)
Ep01-2
Ep02
Ep03
Ep04
Ep05
Ep07
Ep11
EC50 nM
>100
>100
>100
>100
>100
>100
>100

FIG. 6B

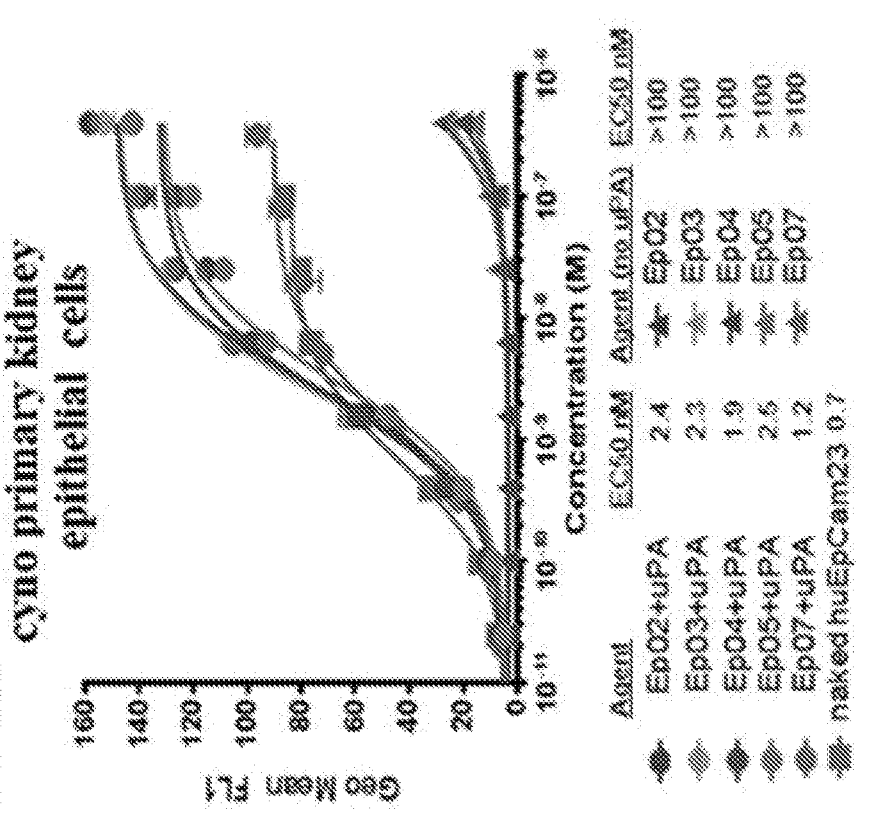
cyno primary kidney epithelial cells
Geo Mean FL1
Concentration (M)
Agent
Ep02+uPA
Ep03+uPA
Ep04+uPA
Ep05+uPA
Ep07+uPA
naked huEpCam23
EC50 nM
2.4
2.3
1.9
2.5
1.2
0.7
Agent (no uPA)
Ep02
Ep03
Ep04
Ep05
Ep07
EC50 nM
>100
>100
>100
>100
>100

FIG. 7B

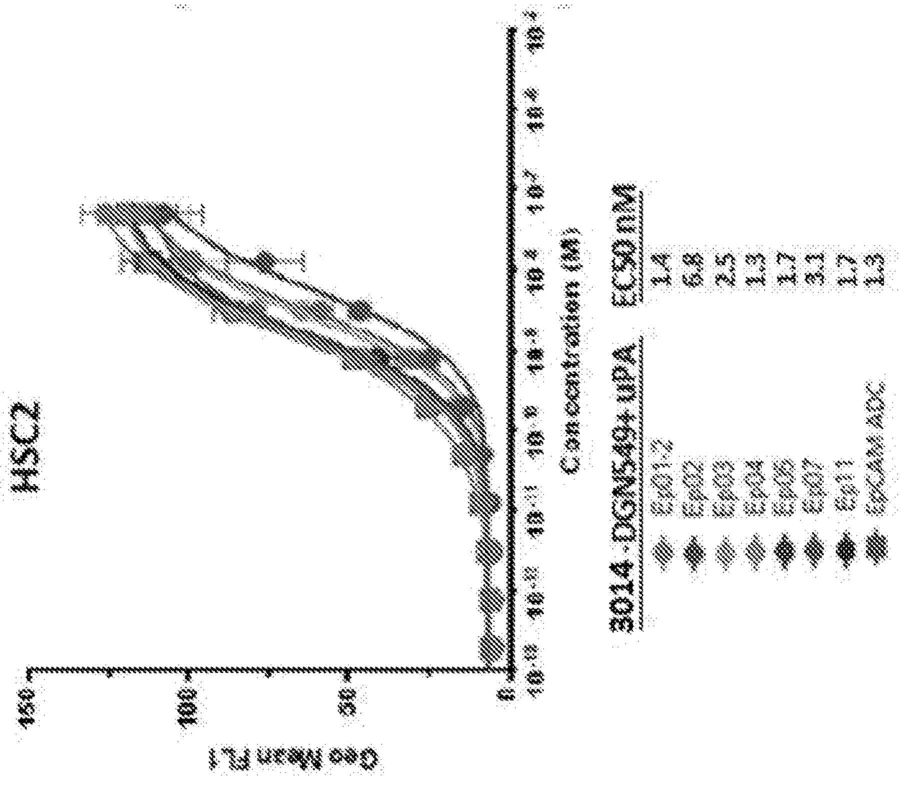
HSC2
Geo Mean FL1
Concentration (M)
3014-DGN549+ uPA
Ep01-2
Ep02
Ep03
Ep04
Ep05
Ep07
Ep11
EpCAM ADC
EC50 nM
1.4
6.8
2.5
1.3
1.7
3.1
1.7
1.3

FIG. 7A

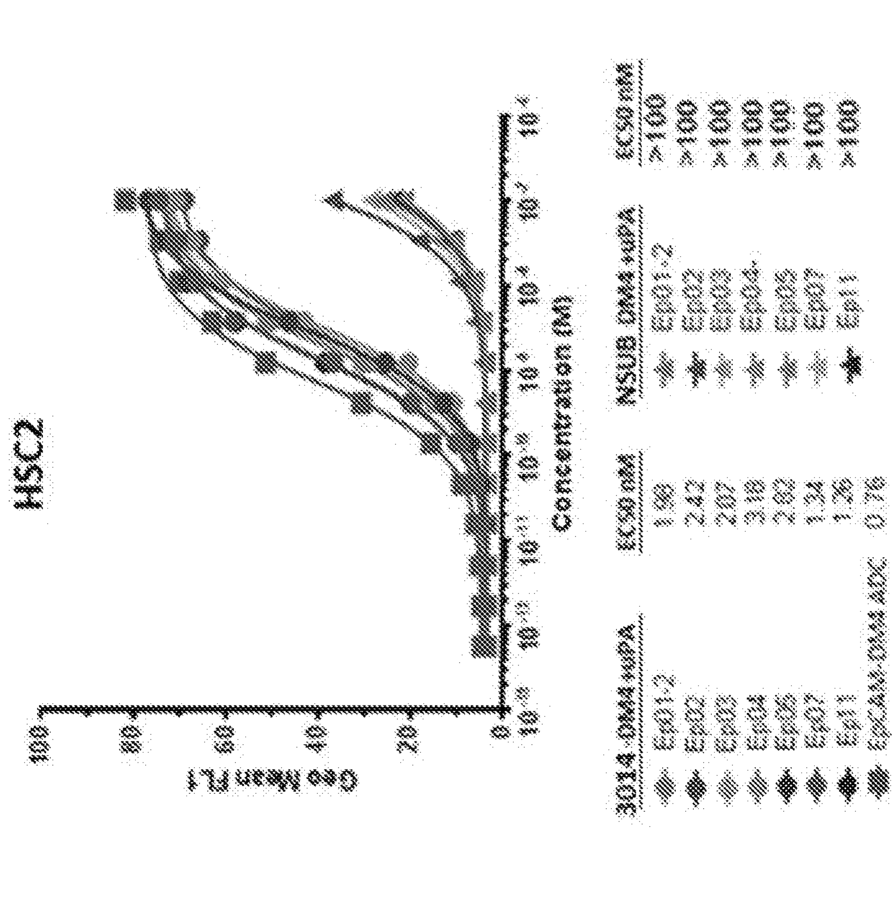
HSC2
Geo Mean FL1
Concentration (M)
3014_DM4+uPA
Ep01-2
Ep02
Ep03
Ep04
Ep05
Ep07
Ep11
EpCAM-DM4 ADC
EC50 nM
1.98
2.42
2.07
3.18
2.02
1.34
1.26
0.76
NSUB_DM4+uPA
Ep01-2
Ep02
Ep03
Ep04
Ep05
Ep07
Ep11
EC50 nM
>100
>100
>100
>100
>100
>100
>100

FIG. 8A

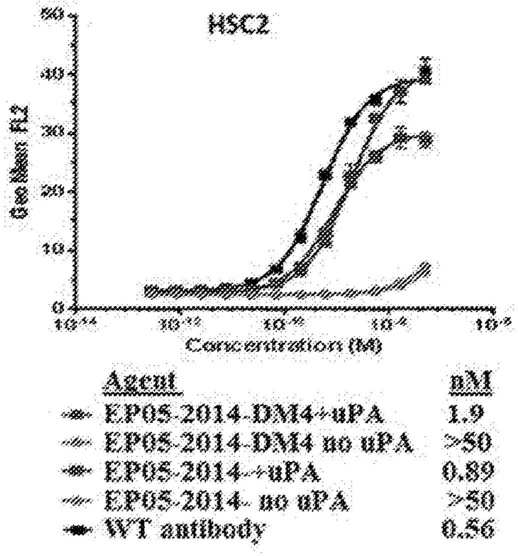


FIG. 8B

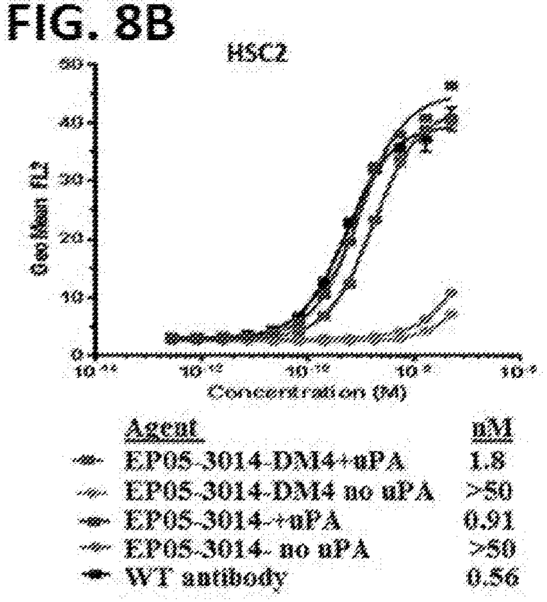


FIG. 8C

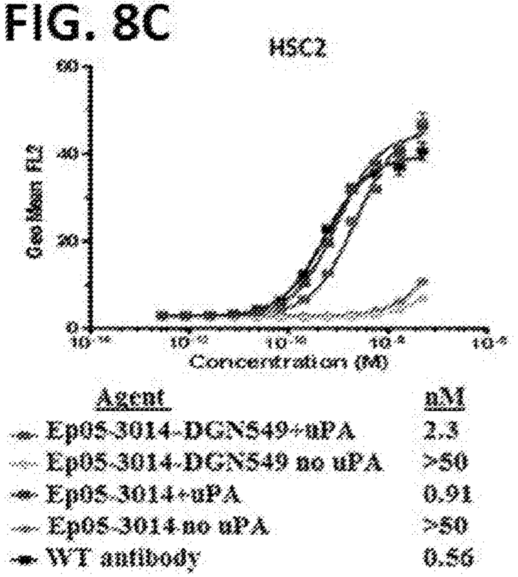


FIG. 8D

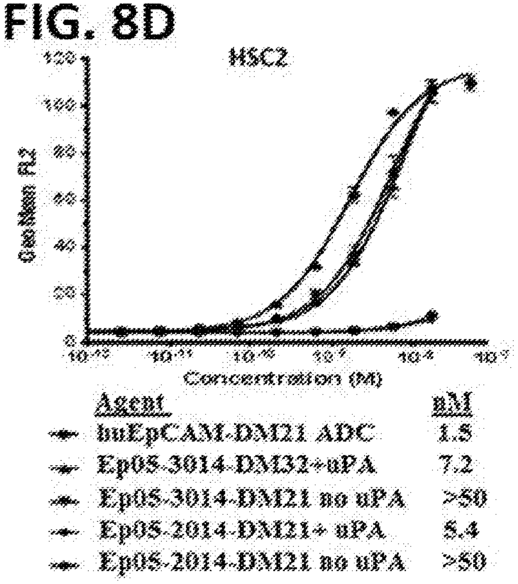

FIG. 9

```
                     D1
                  1                                  36     43
Human EpCAM       QEECVCENYKLAVNCFVNNNRQCQCTSVGAQNTVICSKLAAKCLVMKAEM
Mouse EpCAM       QRDCVCDNYKLATSCSLNEYGECQCTSYGTQNTVICSKLASKCLAMKAEM
                  *  *** *****  *  *    ***** * ********** *** *****

                     D2
                 51                                               100
Human EpCAM       NGSKLGRRAKPEGALQNNDGLYDPDCDESGLFKAKQCNGTSMCWCVNTAG
Mouse EpCAM       THSKSGRRIKPEGAIQNNDGLYDPDCDEQGLFKAKQCNGTATCWCVNTAG
                    ** *** ***** ************* *********** ********

                     D3
                101       112                                     150
Human EpCAM       VRRTDKDTEITCSERVRTYWIIIELKHKAREKPYDSKSLRTALQKEITTR
Mouse EpCAM       VRRTDKDTEITCSERVRTYWIIIELKHKERESPYDHQSLQTALQEAFTSR
                  **************************** ** ***  ** ****   * *

                151                                               200
Human EpCAM       YQLDPKFITSILYENNVITIDLVQNSSQKTQNDVDIADVAYYFEKDVKGE
Mouse EpCAM       YKLNQKFIKNIMYENNVITIDLMQNSSQKTQDDVDIADVAYYFEKDVKGE
                  * *  ***  * ********** ***************************

                201                                       243
Human EpCAM       SLFHS-KKMDLTVNGEQLDLDPGQTLIYYVDEKAPEFSMQGLK (SEQ ID NO:1)
Mouse EpCAM       SLFHSSKSMDLRVNGEPLDLDPGQTLIYYVDEKAPEFSMQGLT (SEQ ID NO:210)
                  ***** * *** **** *************************
```

# FIG. 10A

```
                                   1                                      40
              Human EpCAM          QEECVCENYKLAVNCFVNNNRQCQCTSVGAQNTVICSKLA
   Chimeric EpCAM-D1 swapped       QRDCVCDNYKLATSCSLNEYGECQCTSYGTQNTVICSKLA
   Chimeric EpCAM-D2 swapped       QEECVCENYKLAVNCFVNNNRQCQCTSVGAQNTVICSKLA
Chimeric EpCAM-D1&D2 swapped       QRDCVCDNYKLATSCSLNEYGECQCTSYGTQNTVICSKLA
   Chimeric EpCAM-D3 Swapped       QEECVCENYKLAVNCFVNNNRQCQCTSVGAQNTVICSKLA
              Mouse EpCAM          QRDCVCDNYKLATSCSLNEYGECQCTSYGTQNTVICSKLA
                                   *  *** *****  *  *    ***** * **********

                                   41                                     80
              Human EpCAM          AKCLVMKAEMNGSKLGRRAKPEGALQNNDGLYDPDCDESG
   Chimeric EpCAM-D1 swapped       AKCLVMKAEMNGSKLGRRAKPEGALQNNDGLYDPDCDESG
   Chimeric EpCAM-D2 swapped       AKCLAMKAEMTHSKSGRRIKPEGAIQNNDGLYDPDCDEQG
Chimeric EpCAM-D1&D2 swapped       SKCLAMKAEMTHSKSGRRIKPEGAIQNNDGLYDPDCDEQG
   Chimeric EpCAM-D3 Swapped       AKCLVMKAEMNGSKLGRRAKPEGALQNNDGLYDPDCDESG
              Mouse EpCAM          SKCLAMKAEMTHSKSGRRIKPEGAIQNNDGLYDPDCDEQG
                                    *** *****  ** *** ***** ************* *

                                   81                                    120
              Human EpCAM          LFKAKQCNGTSMCWCVNTAGVRRTDKDTEITCSERVRTYW
   Chimeric EpCAM-D1 swapped       LFKAKQCNGTSMCWCVNTAGVRRTDKDTEITCSERVRTYW
   Chimeric EpCAM-D2 swapped       LFKAKQCNGTATCWCVNTAGVRRTDKDTEITCSERVRTYW
Chimeric EpCAM-D1&D2 swapped       LFKAKQCNGTATCWCVNTAGVRRTDKDTEITCSERVRTYW
   Chimeric EpCAM-D3 Swapped       LFKAKQCNGTSMCWCVNTAGVRRTDKDTEITCSERVRTYW
              Mouse EpCAM          LFKAKQCNGTATCWCVNTAGVRRTDKDTEITCSERVRTYW
                                   **********  ****************************

                                   121                                   160
              Human EpCAM          IIIELKHKAREKPYDSKSLRTALQKEITTRYQLDPKFITS
   Chimeric EpCAM-D1 swapped       IIIELKHKAREKPYDSKSLRTALQKEITTRYQLDPKFITS
   Chimeric EpCAM-D2 swapped       IIIELKHKAREKPYDSKSLRTALQKEITTRYQLDPKFITS
Chimeric EpCAM-D1&D2 swapped       IIIELKHKAREKPYDSKSLRTALQKEITTRYQLDPKFITS
   Chimeric EpCAM-D3 Swapped       IIIELKHKERESPYDHQSLQTALQEAFTSRYKLNQKFIKN
              Mouse EpCAM          IIIELKHKERESPYDHQSLQTALQEAFTSRYKLNQKFIKN
                                   ******** ** ***  ** ****   * ** *  ***
```

# FIG. 10B

```
                                  161                                    200
                    Human EpCAM   ILYENNVITIDLVQNSSQKTQNDVDIADVAYYFEKDVKGE
   Chimeric EpCAM-D1 swapped      ILYENNVITIDLVQNSSQKTQNDVDIADVAYYFEKDVKGE
   Chimeric EpCAM-D2 swapped      ILYENNVITIDLVQNSSQKTQNDVDIADVAYYFEKDVKGE
Chimeric EpCAM-D1&D2 swapped      ILYENNVITIDLVQNSSQKTQNDVDIADVAYYFEKDVKGE
   Chimeric EpCAM-D3 Swapped      IMYENNVITIDLMQNSSQKTQDDVDIADVAYYFEKDVKGE
                    Mouse EpCAM   IMYENNVITIDLMQNSSQKTQDDVDIADVAYYFEKDVKGE
                                  * ********** ******** ******************

                                  201                                    240
                    Human EpCAM   SLFHS-KKMDLTVNGEQLDLDPGQTLIYYVDEKAPEFSMQ
   Chimeric EpCAM-D1 swapped      SLFHS-KKMDLTVNGEQLDLDPGQTLIYYVDEKAPEFSMQ
   Chimeric EpCAM-D2 swapped      SLFHS-KKMDLTVNGEQLDLDPGQTLIYYVDEKAPEFSMQ
Chimeric EpCAM-D1&D2 swapped      SLFHS-KKMDLTVNGEQLDLDPGQTLIYYVDEKAPEFSMQ
   Chimeric EpCAM-D3 Swapped      SLFHSSKSMDLRVNGEPLDLDPGQTLIYYVDEKAPEFSMQ
                    Mouse EpCAM   SLFHSSKSMDLRVNGEPLDLDPGQTLIYYVDEKAPEFSMQ
                                  ***** * *** **** ***********************

                                  241
                    Human EpCAM   GLK  (SEQ ID NO:1)
   Chimeric EpCAM-D1 swapped      GLK  (SEQ ID NO:211)
   Chimeric EpCAM-D2 swapped      GLK  (SEQ ID NO:212)
Chimeric EpCAM-D1&D2 swapped      GLK  (SEQ ID NO:213)
   Chimeric EpCAM-D3 Swapped      GLT  (SEQ ID NO:214)
                    Mouse EpCAM   GLT  (SEQ ID NO:210)
                                  **
```

FIG. 11

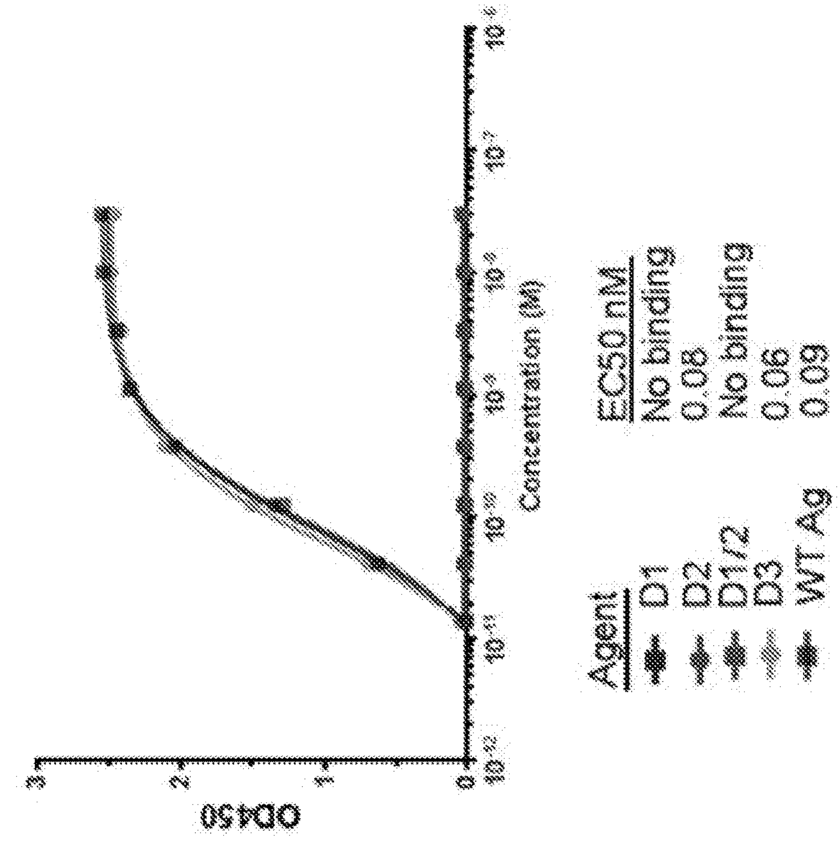
OD450
Concentration (M)
Agent
D1
D2
D1/2
D3
WT Ag
EC50 nM
No binding
0.08
No binding
0.06
0.09

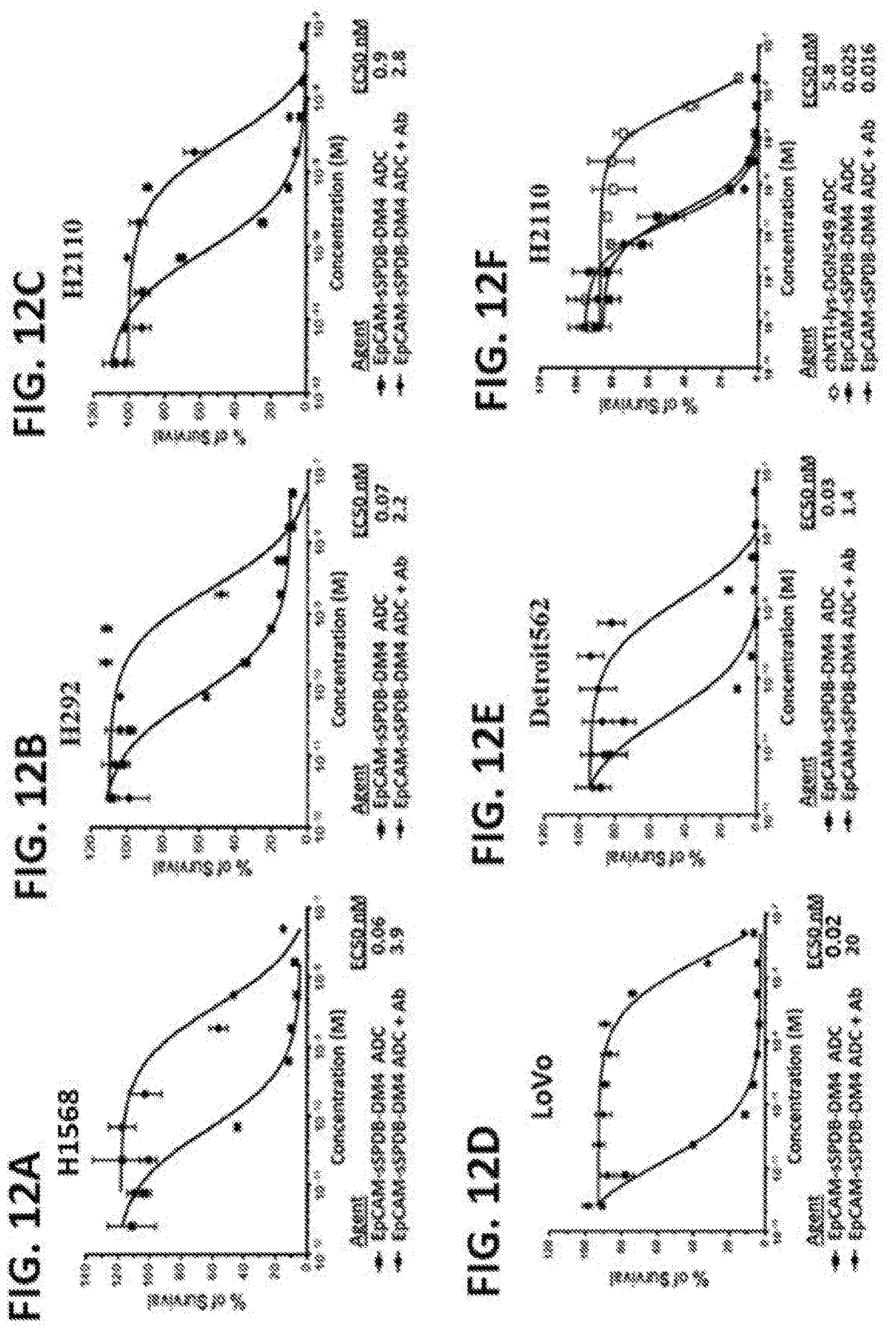
FIG. 12A
H1568
% of Survival
Concentration (M)
Agent
EpCAM-sSPDB-DM4 ADC
EpCAM-sSPDB-DM4 ADC + Ab
EC50 nM
0.06
3.9
FIG. 12B
H292
% of Survival
Concentration (M)
Agent
EpCAM-sSPDB-DM4 ADC
EpCAM-sSPDB-DM4 ADC + Ab
EC50 nM
0.07
2.2
FIG. 12C
H2110
% of Survival
Concentration (M)
Agent
EpCAM-sSPDB-DM4 ADC
EpCAM-sSPDB-DM4 ADC + Ab
EC50 nM
0.9
2.8
FIG. 12D
LoVo
% of Survival
Concentration (M)
Agent
EpCAM-sSPDB-DM4 ADC
EpCAM-sSPDB-DM4 ADC + Ab
EC50 nM
0.02
20
FIG. 12E
Detroit562
% of Survival
Concentration (M)
Agent
EpCAM-sSPDB-DM4 ADC
EpCAM-sSPDB-DM4 ADC + Ab
EC50 nM
0.03
1.4
FIG. 12F
H2110
% of Survival
Concentration (M)
Agent
chKTI-lys-DGN549 ADC
EpCAM-sSPDB-DM4 ADC
EpCAM-sSPDB-DM4 ADC + Ab
EC50 nM
5.8
0.025
0.016

FIG. 12G

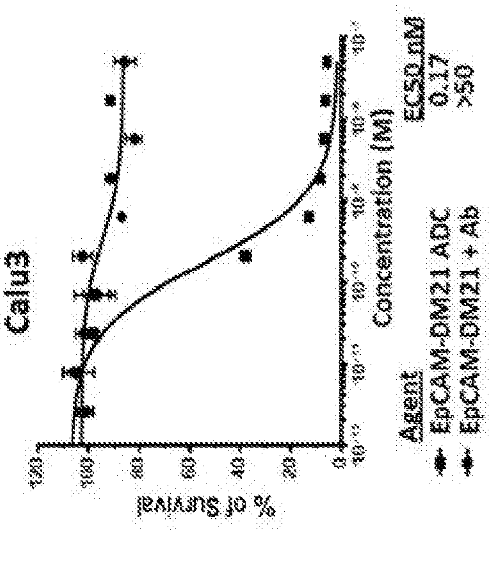


FIG. 12H

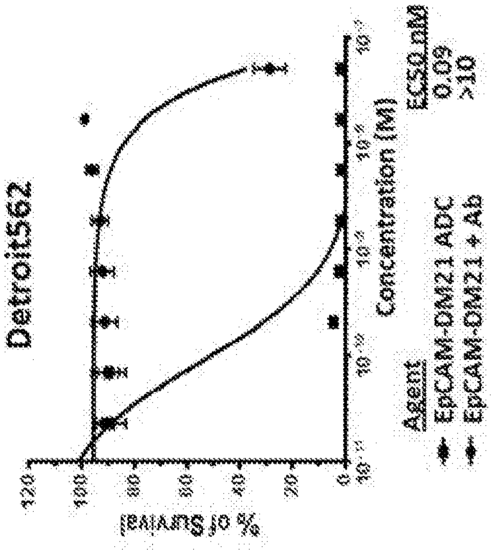


FIG. 12I

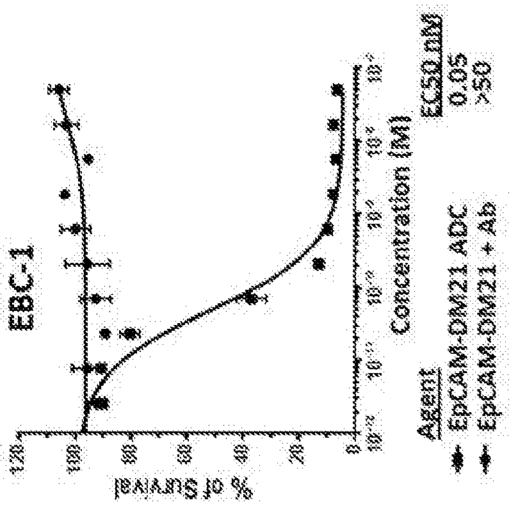


FIG. 12J

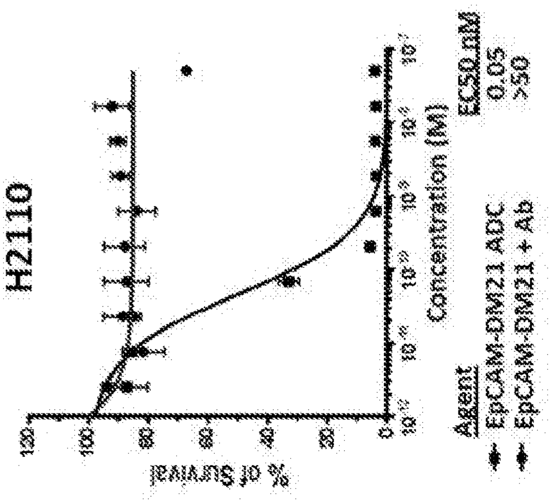


FIG. 12K

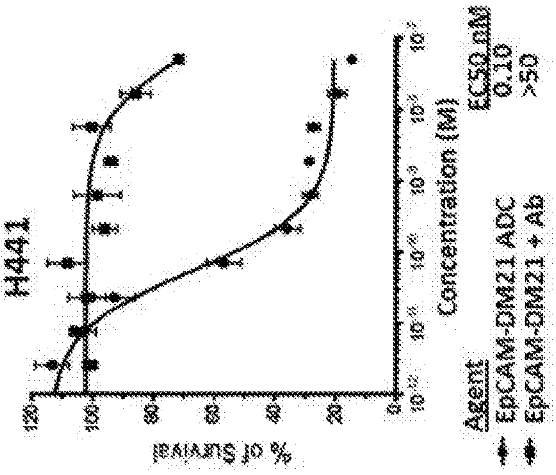

FIG. 13A

Calu3

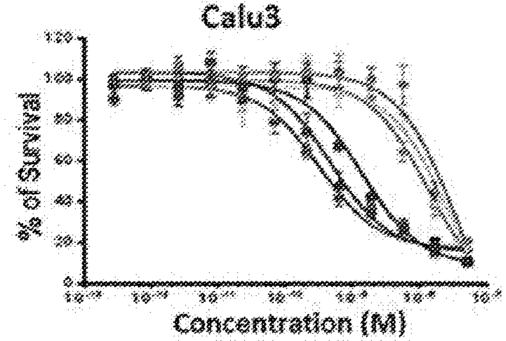


| Agent | EC50 nM |
|---|---|
| EpCAM-sSPDB-DM4 ADC | 0.46 |
| chKTI-sSPDB-DM4 ADC | 25.3 |
| Ep05-2014-DM4+uPA | 0.31 |
| Ep05-2014-DM4 no uPA | 5.6 |
| EP05-3014-DM4+uPA | 1.4 |
| EP05-3014-DM4 no uPA | 34 |

FIG. 13B

OV90

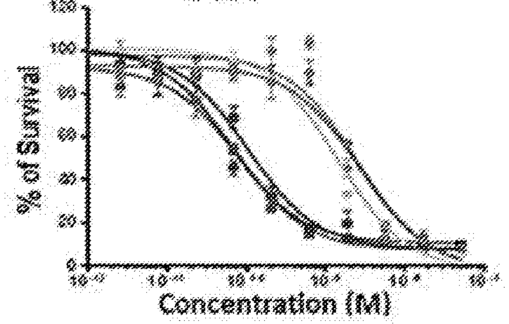


| Agent | EC50 nM |
|---|---|
| EpCAM-sSPDB-DM ADC | 0.06 |
| chKTI-sSPDB-DM4 ADC | 2.5 |
| Ep05-2014-DM4+uPA | 0.08 |
| Ep05-2014-DM4 no uPA | 1.4 |
| EP05-3014-DM4+uPA | 0.1 |
| EP05-3014-DM4 no uPA | 28 |

FIG. 13C

EBC-1

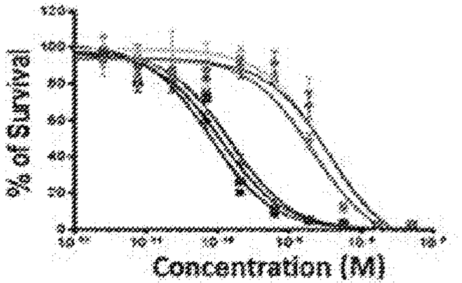


| Agent | EC50 nM |
|---|---|
| EpCAM-sSPDB-DM4 ADC | 0.08 |
| chKTI-sSPDB-DM4 ADC | 2.1 |
| Ep05-2014-DM4+uPA | 0.15 |
| Ep05-2014-DM4 no uPA | 3.3 |
| EP05-3014-DM4+uPA | 0.1 |
| EP05-3014-DM4 no uPA | 3.6 |

FIG. 13D

H2110

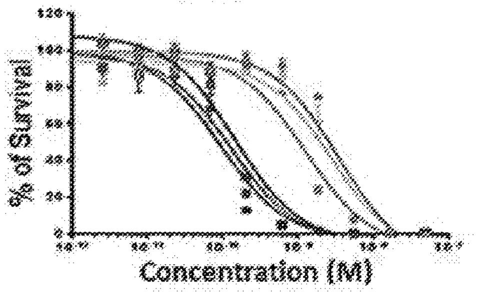


| Agent | EC50 nM |
|---|---|
| EpCAM-sSPDB-DM4 ADC | 0.094 |
| chKTI-sSPDB-DM4 ADC | 1.3 |
| Ep05-2014-DM4+uPA | 0.13 |
| Ep05-2014-DM4 no uPA | 2.9 |
| EP05-3014-DM4+uPA | 0.14 |
| EP05-3014-DM4 no uPA | 3.5 |

FIG. 14A

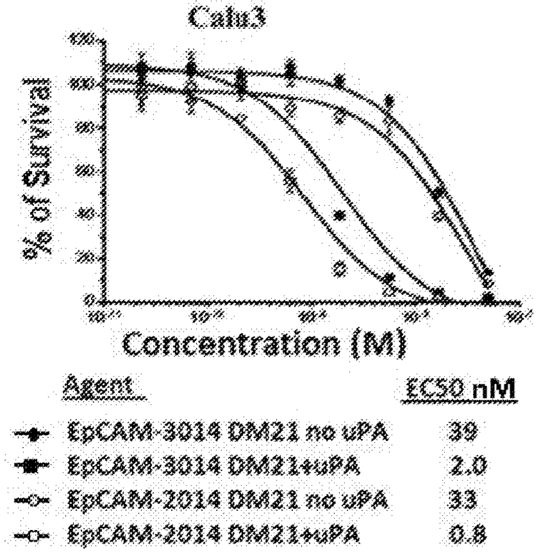


FIG. 14B

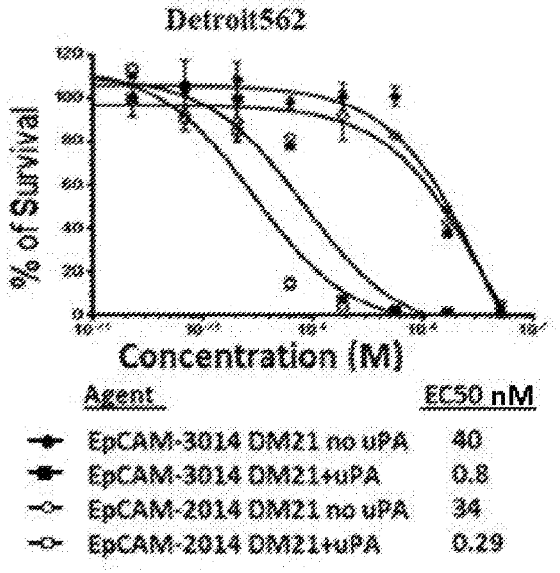


FIG. 14C

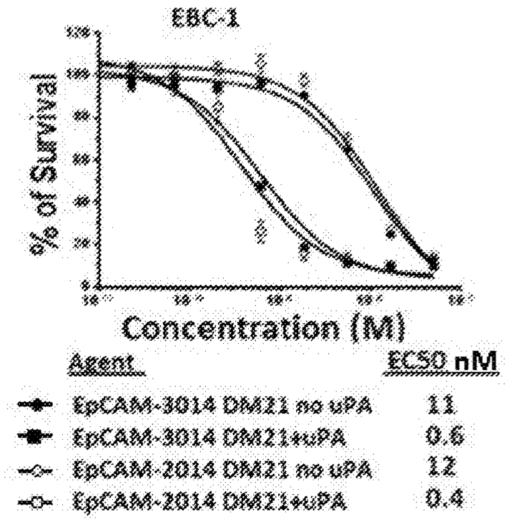


FIG. 14D

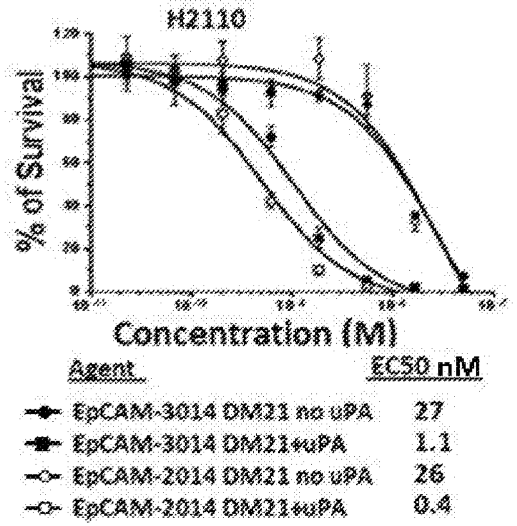

FIG. 15A

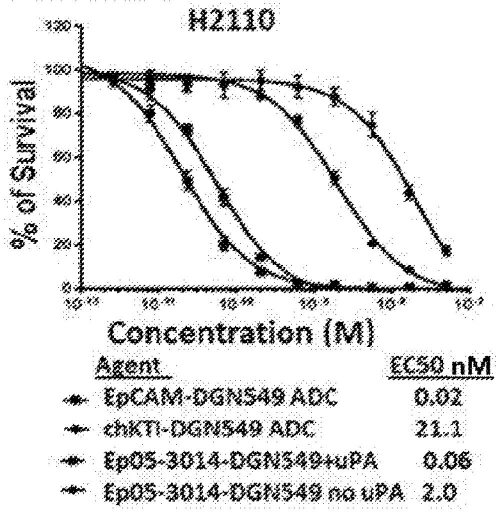


FIG. 15B

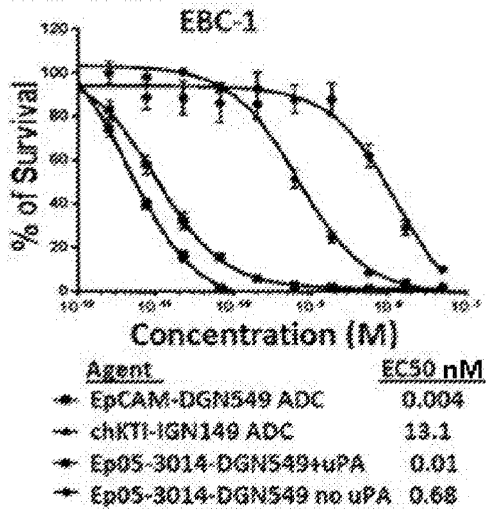


FIG. 15C

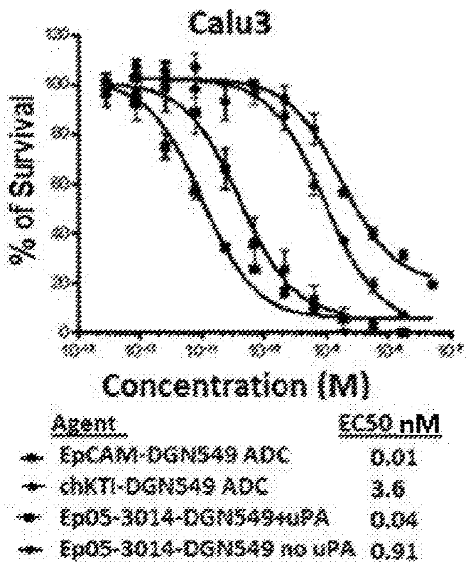


FIG. 15D

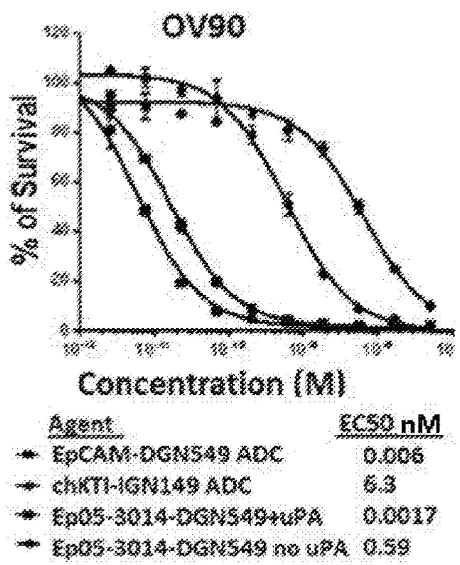

FIG. 16

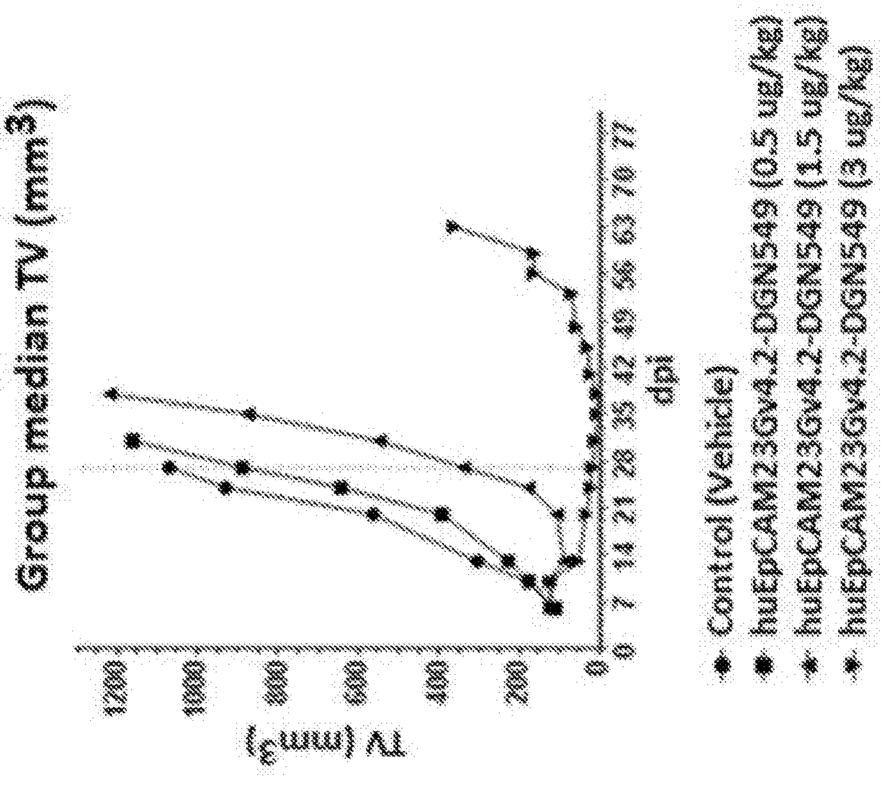
Group median TV (mm3)
TV (mm3)
0
200
400
600
800
1000
1200
0 7 14 21 28 35 42 49 56 63 70 77
dpi
Control (Vehicle)
huEpCAM23Gv4.2-DGN549 (0.5 ug/kg)
huEpCAM23Gv4.2-DGN549 (1.5 ug/kg)
huEpCAM23Gv4.2-DGN549 (3 ug/kg)

FIG. 17A

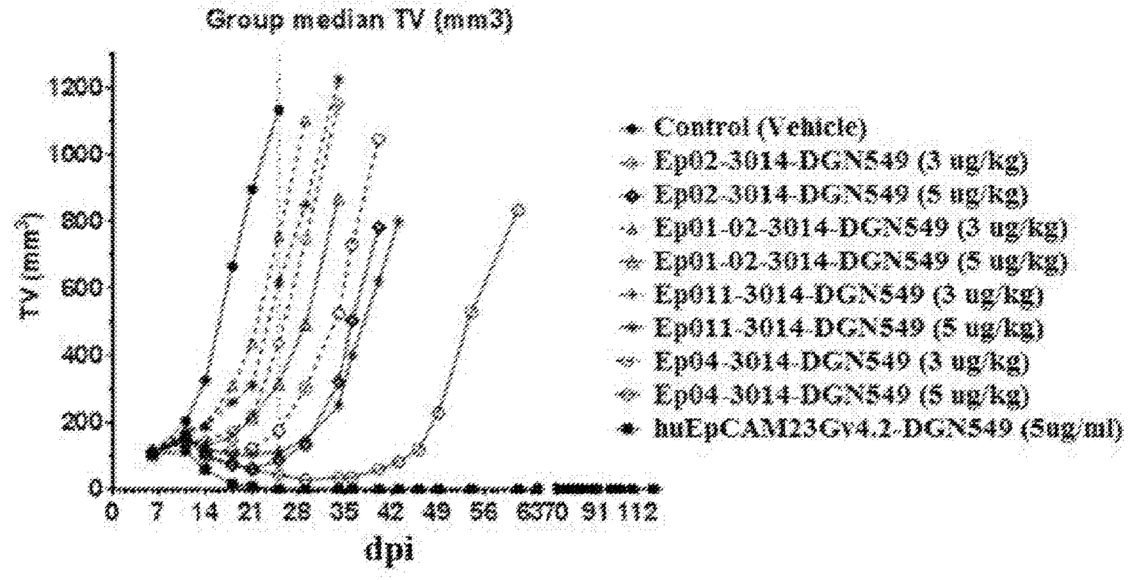


FIG. 17B

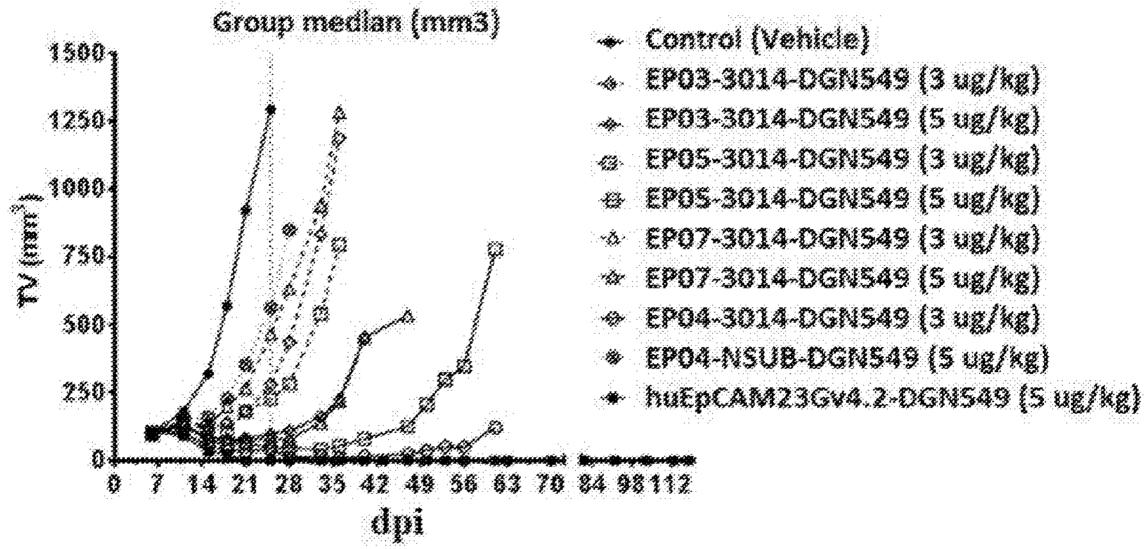

# FIG. 17C

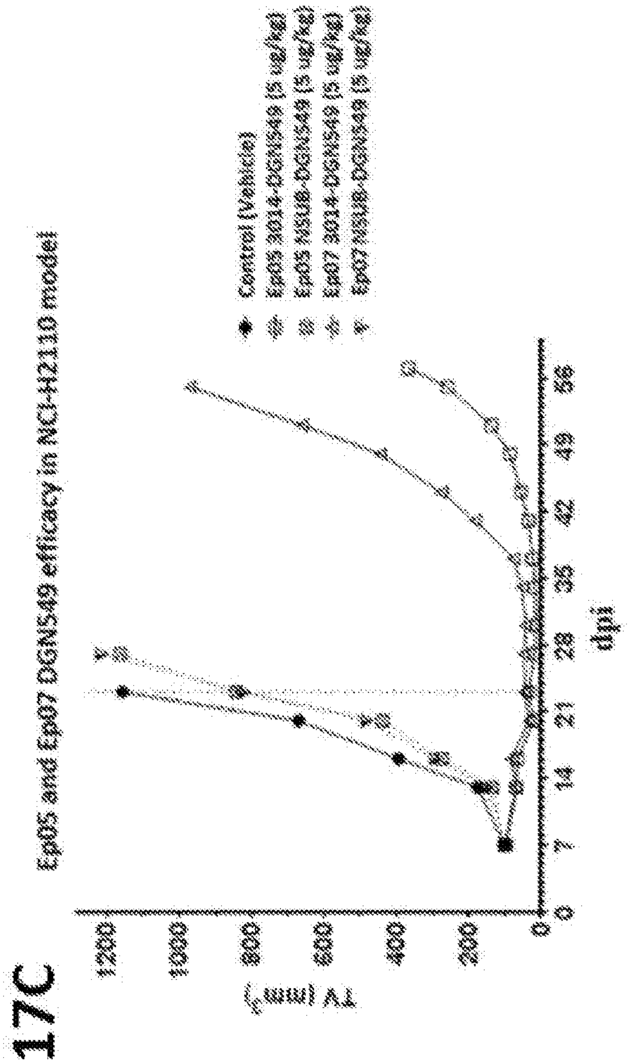


# FIG. 17D

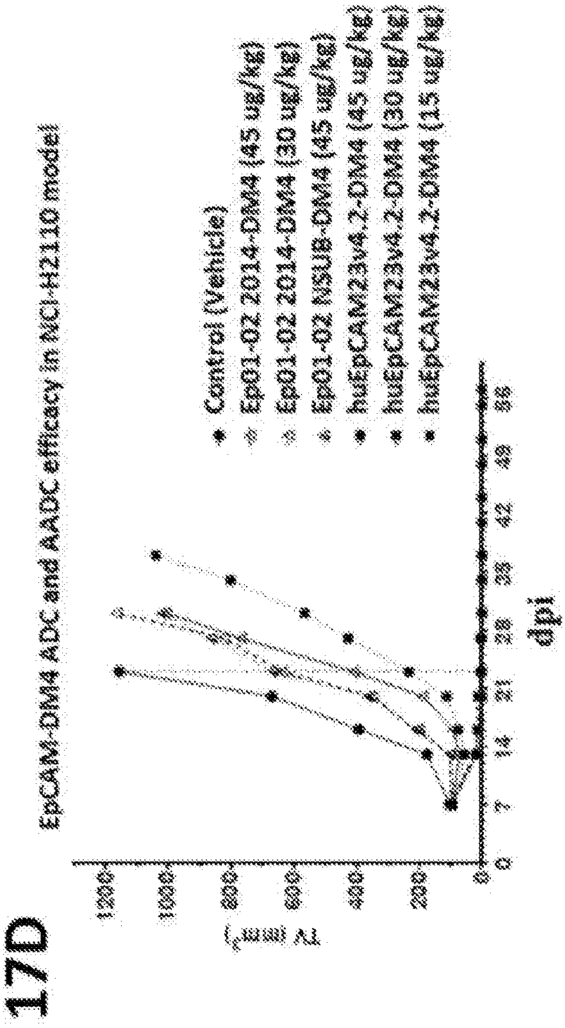

FIG. 17E

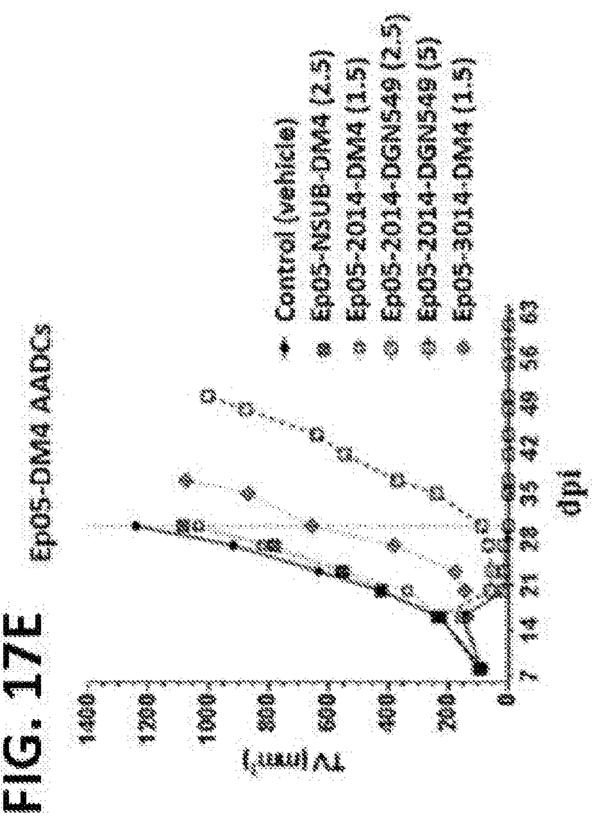


FIG. 17F

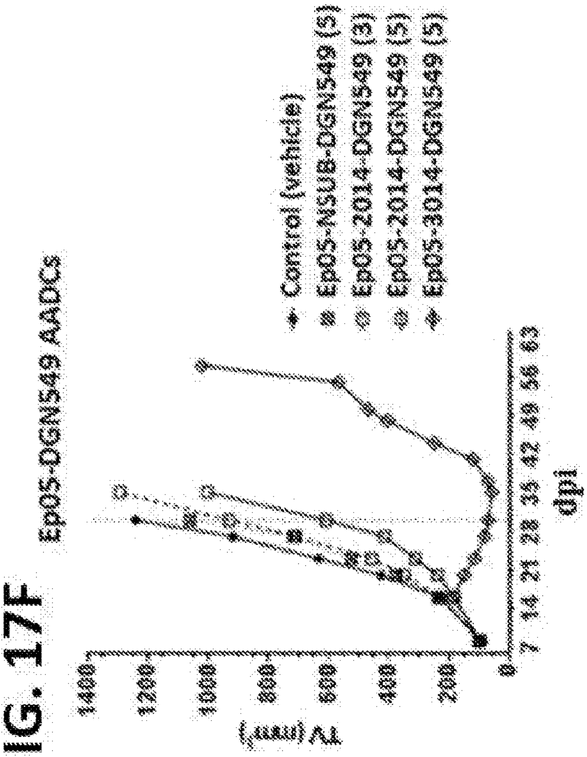

FIG. 18

Ep05-DM4 AADCs in the Calu-3 Model

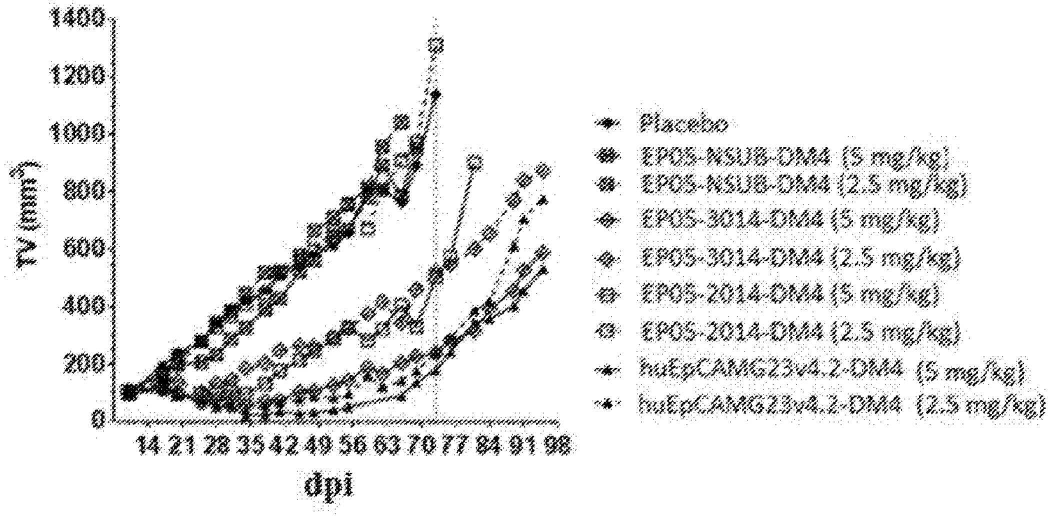


FIG. 19

Ep05 AADCs in NCI-H292 model

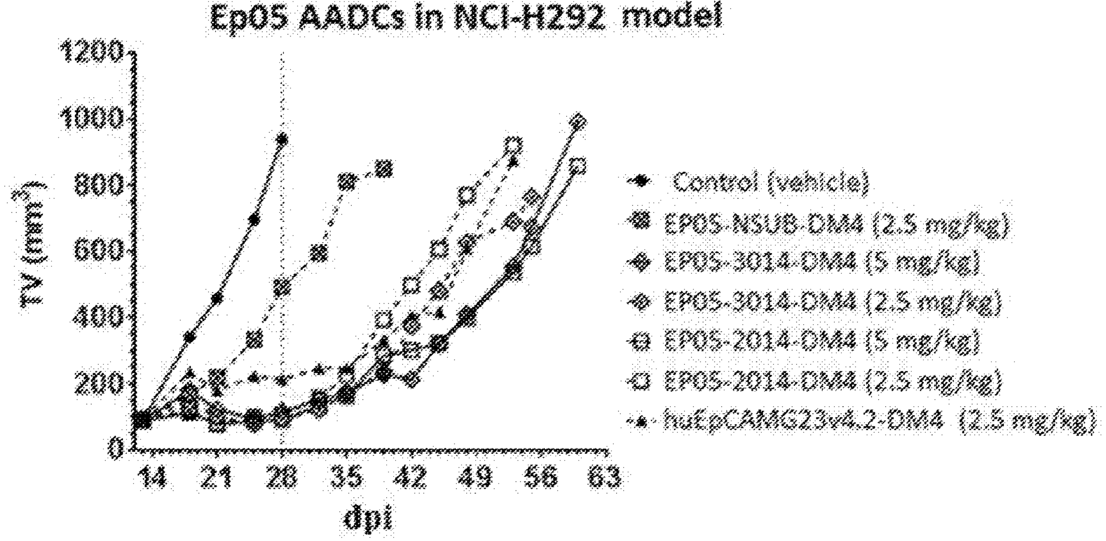

FIG. 20

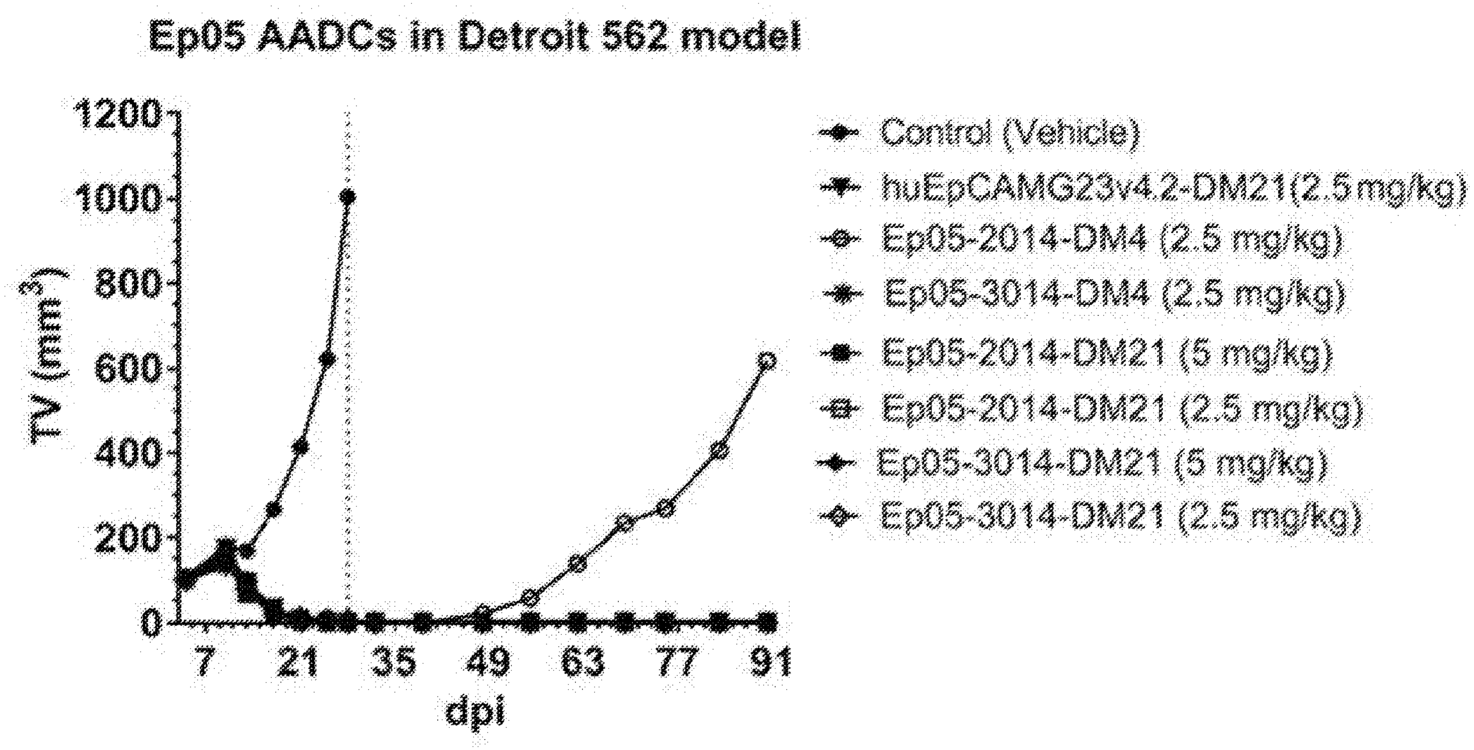


| Group (dose, mg/kg) | TGI (%T/C) | PR | CR | TFS |
|---|---|---|---|---|
| Control (Vehicle) | na | 0/8 | 0/8 | 0/8 |
| huEpCAM27Gv4.2-DM21 ADC (2.5) | 0 % | 8/8 | 8/8 | 8/8 |
| Ep05-3014-DM4 (2.5) | 0 % | 8/8 | 8/8 | 7/8 |
| Ep05-2014-DM4 (2.5) | 0 % | 8/8 | 6/8 | 0/8 |
| Ep05-3014-DM21 (5) | 0 % | 8/8 | 7/8 | 7/8 |
| Ep05-3014-DM21 (2.5) | 0.7 % | 8/8 | 7/8 | 6/8 |
| Ep05-2014-DM21 (5) | 0 % | 8/8 | 8/8 | 8/8 |
| Ep05-2014-DM21 (2.5) | 0 % | 7/8 | 7/8 | 6/8 |

FIG. 21 H441

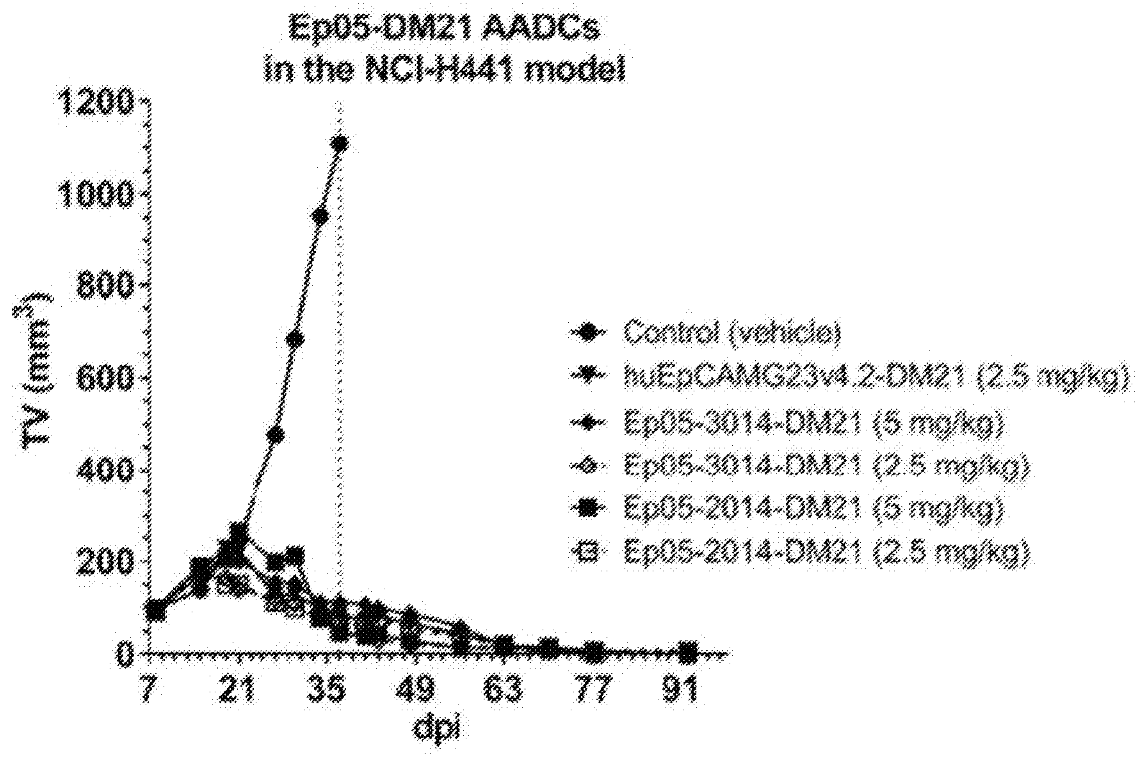


| Group (dose, mg/kg) | TGI (%T/C) | PR | CR | TFS |
|---|---|---|---|---|
| Control (Vehicle) | na | 0/8 | 0/8 | 0/8 |
| huEpCAM27Gv4.2-DM21 ADC (2.5) | 7.7 % | 8/8 | 7/8 | 7/8 |
| Ep05-3014-DM21 (5) | 10.1 % | 8/8 | 5/8 | 5/8 |
| Ep05-3014-DM21 (2.5) | 6.3 % | 8/8 | 6/8 | 6/8 |
| Ep05-2014-DM21 (5) | 4.2 % | 8/8 | 7/8 | 7/8 |
| Ep05-2014-DM21 (2.5) | 6.2 % | 8/8 | 4/8 | 4/8 |

FIG. 22 OV-90

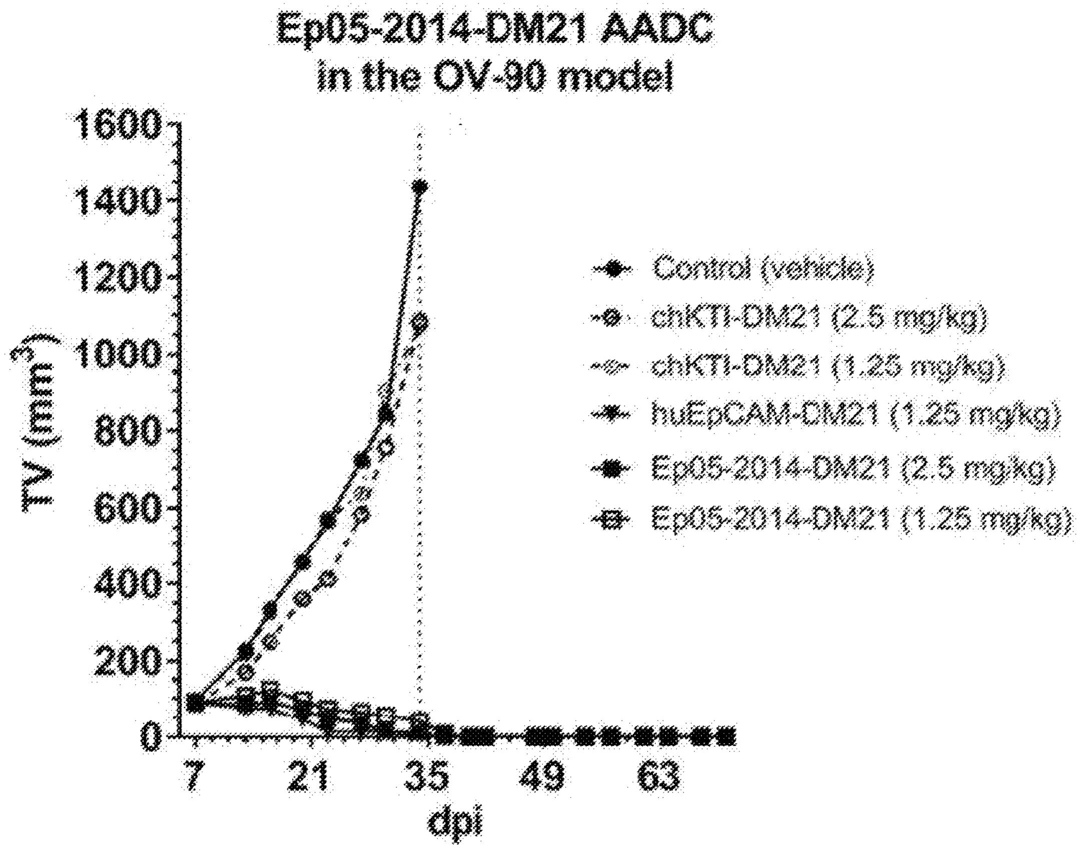


| Group (dose, mg/kg) | TGI (%T/C) | PR | CR | TFS |
|---|---|---|---|---|
| Control (Vehicle) | na | 0/6 | 0/6 | 0/6 |
| chKTI-DM21 (2.5) | 75.5 % | 0/6 | 0/6 | 0/6 |
| chKTI-DM21 (1.25) | 74.8 % | 0/6 | 0/6 | 0/6 |
| huEpCAM27Gv4.2-DM21 ADC (1.25) | 0.5 % | 6/6 | 6/6 | 6/6 |
| Ep05-2014-DM21 (2.5) | 0.8 % | 6/6 | 6/6 | 6/6 |
| Ep05-2014-DM21 (1.25) | 2.9 % | 6/6 | 6/6 | 6/6 |

FIG.23 Calu-3

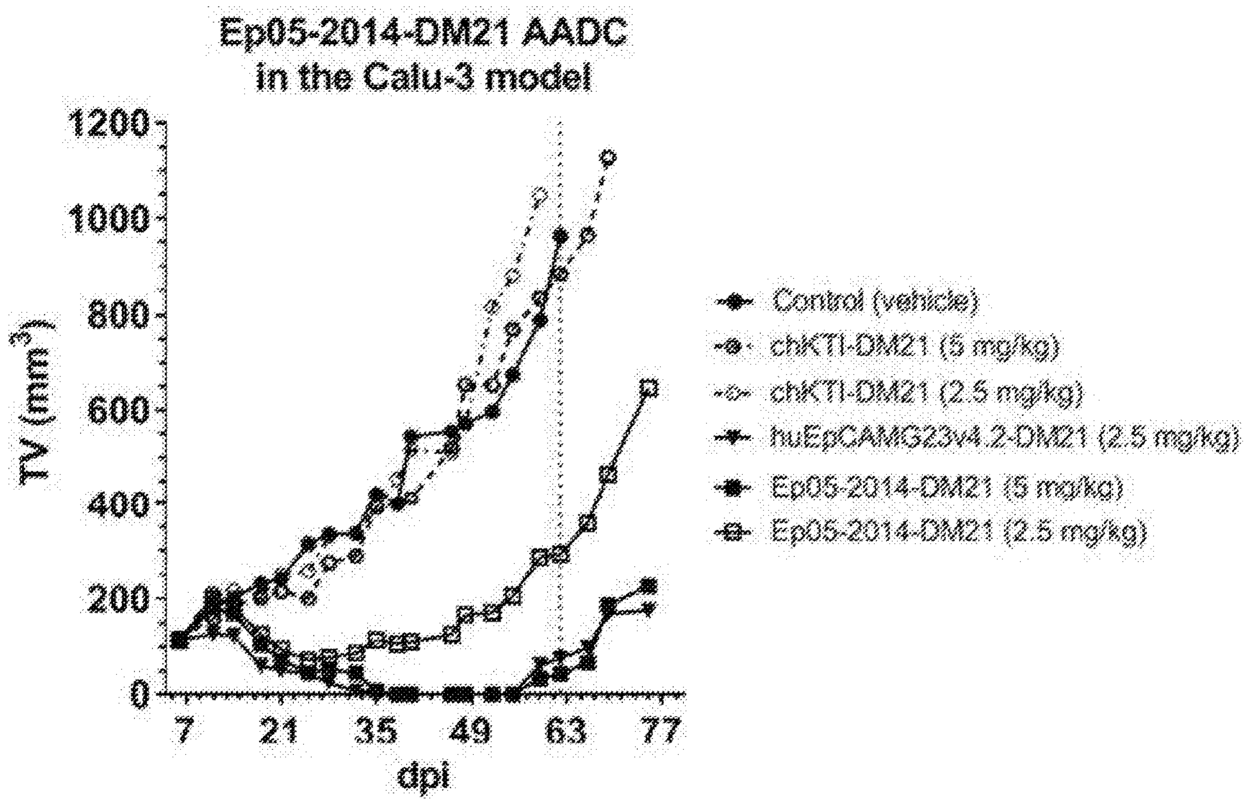


| Group (dose, mg/kg) | TGI (%T/C) | PR | CR | TF (75 dpi) |
|---|---|---|---|---|
| Control (Vehicle) | na | 0/6 | 0/6 | 0/6 |
| chKTI-DM21 (5) | 91.7 % | 0/6 | 0/6 | 0/6 |
| chKTI-DM21 (2.5) | >110 % | 0/6 | 0/6 | 0/6 |
| huEpCAM27Gv4.2-DM21 ADC (2.5) | 7.8 % | 6/6 | 5/6 | 1/6 |
| Ep05-2014-DM21 (5) | 4.4 % | 5/6 | 5/6 | 2/6 |
| Ep05-2014-DM21 (2.5) | 30.4 % | 3/6 | 0/6 | 0/6 |

FIG. 24

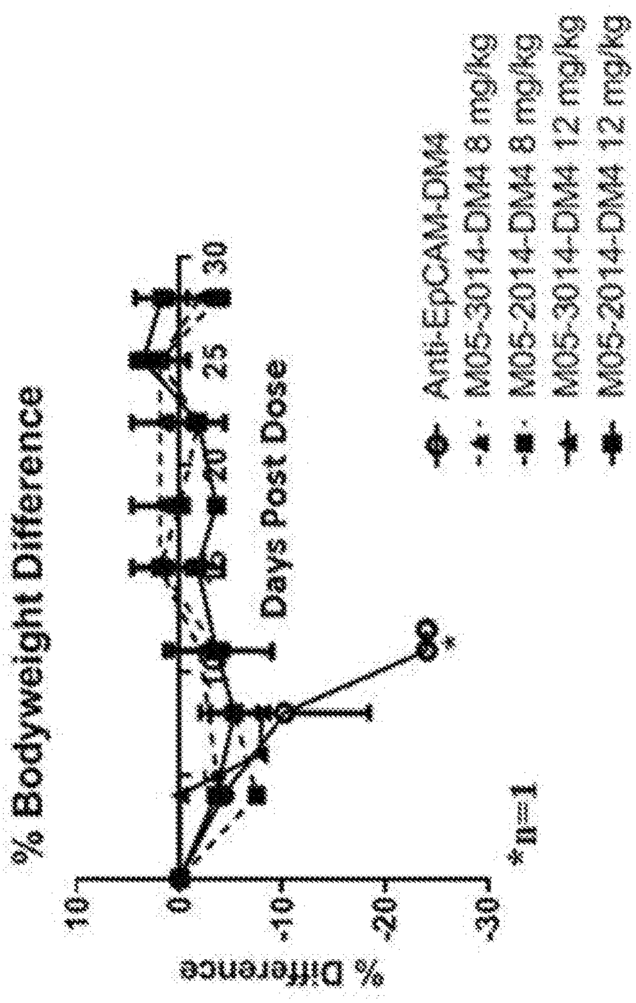
% Bodyweight Difference
% Difference
10
0
-10
-20
-30
5
10
15
20
25
30
Days Post Dose
*n=1
Anti-EpCAM-DM4
M05-3014-DM4 8 mg/kg
M05-2014-DM4 8 mg/kg
M05-3014-DM4 12 mg/kg
M05-2014-DM4 12 mg/kg

FIG. 25A

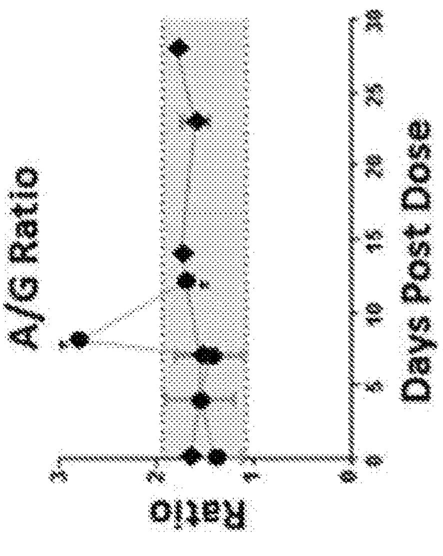


FIG. 25B

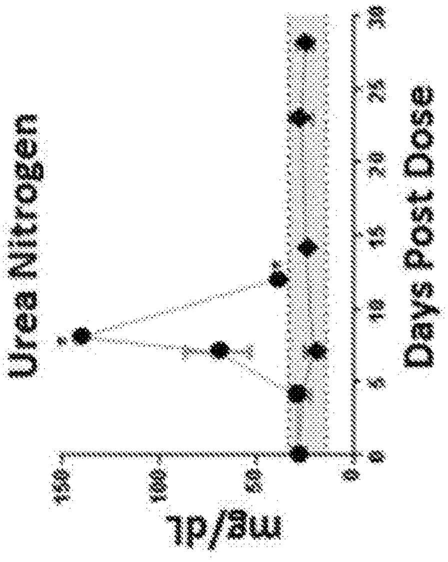


FIG.25C

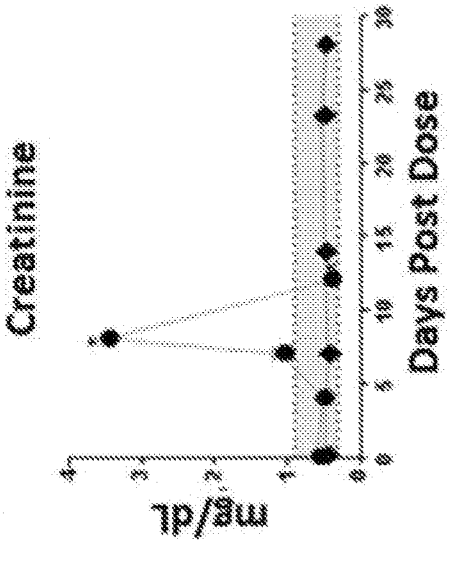


● anti-EpCAM ADC-DM4 8 mg/kg ◆ M05-DM4 AADC 8 mg/kg

FIG. 25D

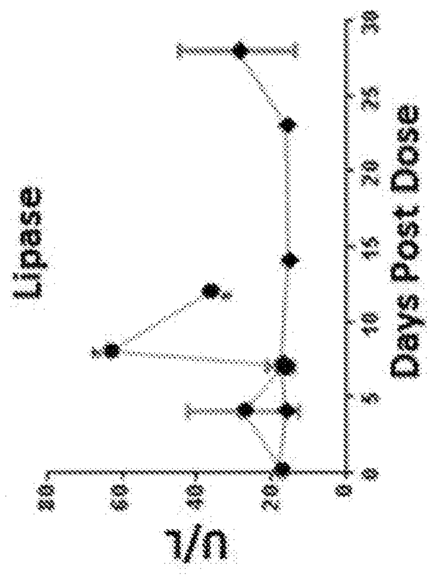


FIG. 25E

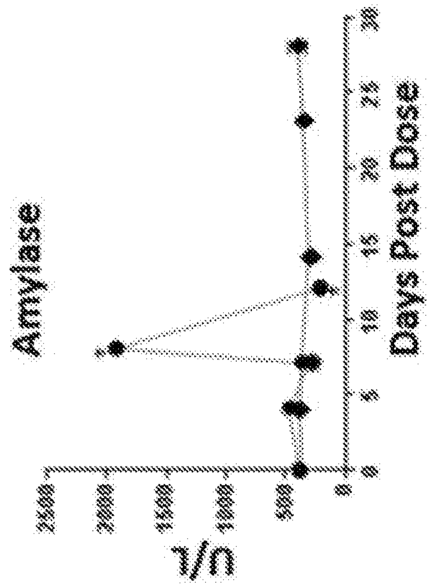


● anti-EpCAM ADC-DM4 8 mg/kg ◆ M05-DM4 AADC 8 mg/kg

FIG. 26

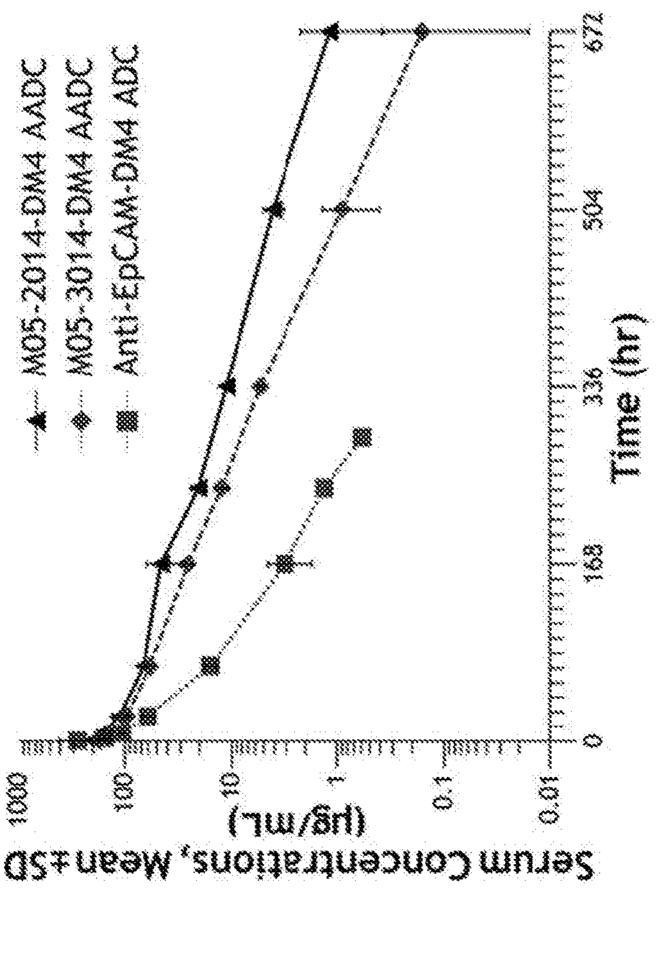

Serum Concentrations, Mean±SD (µg/mL)
1000
100
10
1
0.1
0.01
0
168
336
504
672
Time (hr)
M05-2014-DM4 AADC
M05-3014-DM4 AADC
Anti-EpCAM-DM4 ADC


FIG. 27

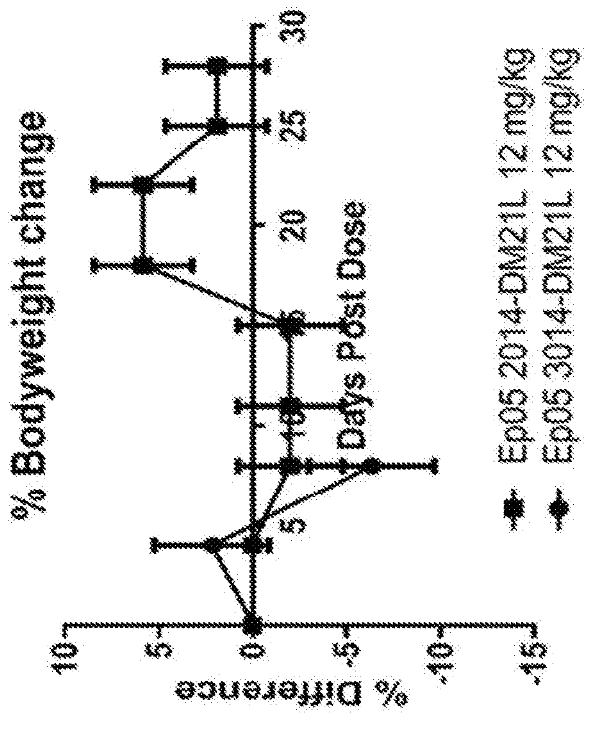

% Bodyweight change
% Difference
10
5
0
-5
-10
-15
5
10
15
20
25
30
Days Post Dose
Ep05 2014-DM21L 12 mg/kg
Ep05 3014-DM21L 12 mg/kg

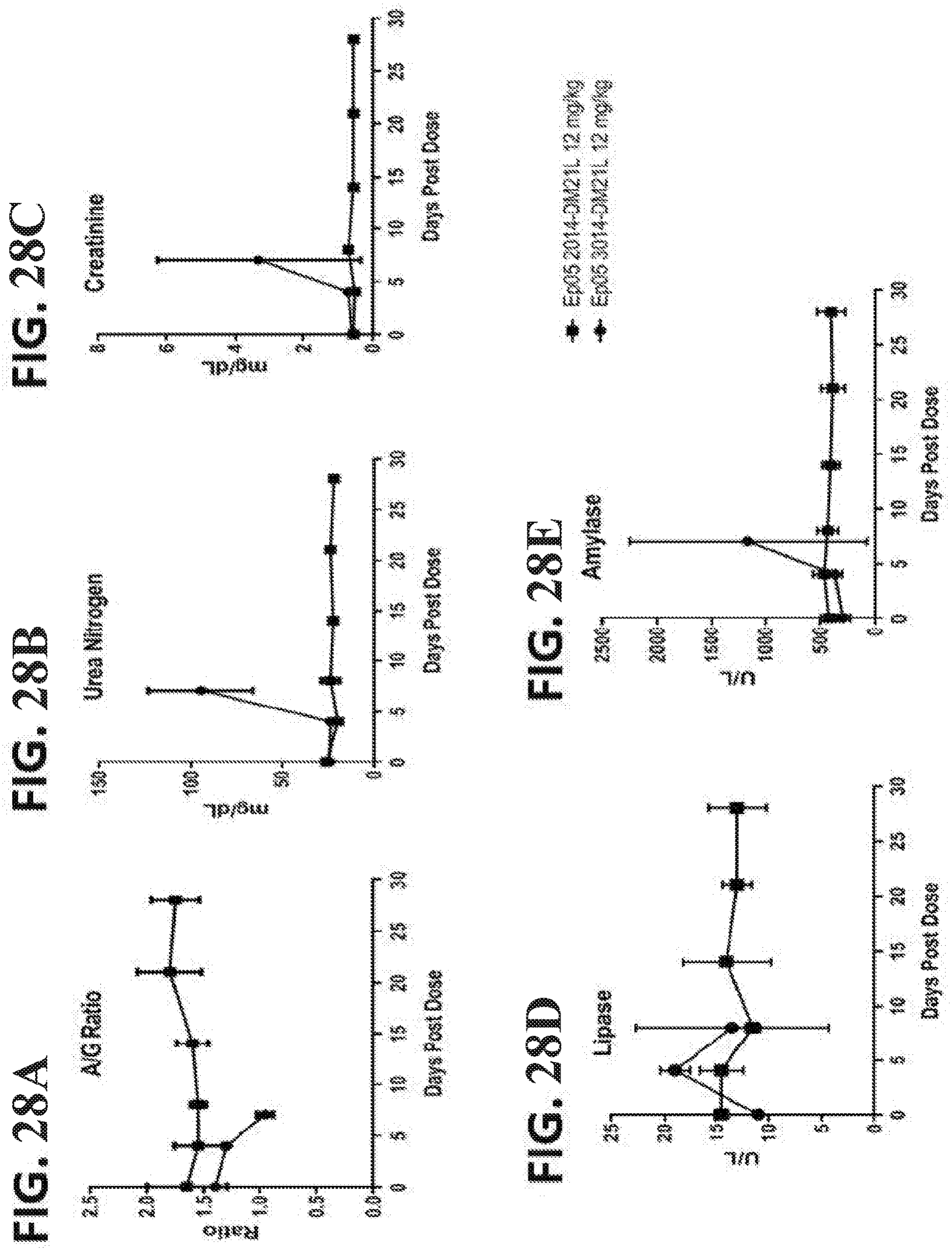


FIG. 28A

FIG. 28B

FIG. 28C

FIG. 28D

FIG. 28E

FIG. 29

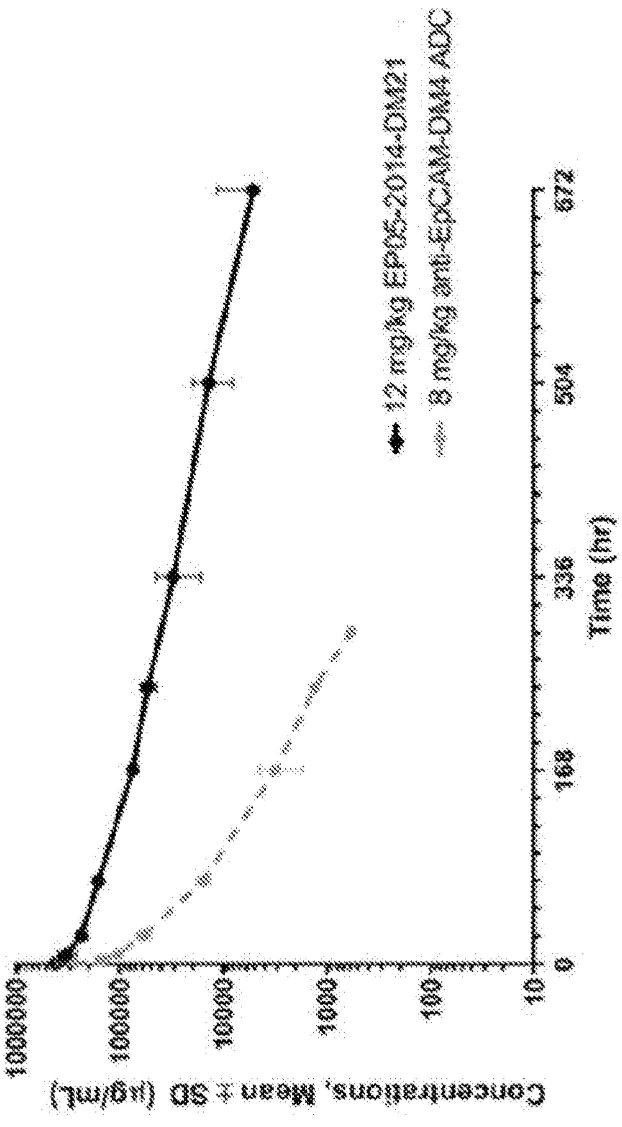
12 mg/kg EPO5-2014-DM21
8 mg/kg anti-EpCAM-DM4 ADC
Concentrations, Mean ± SD (µg/mL)
1000000
100000
10000
1000
100
10
0
168
336
504
672
Time (hr)

FIG. 30

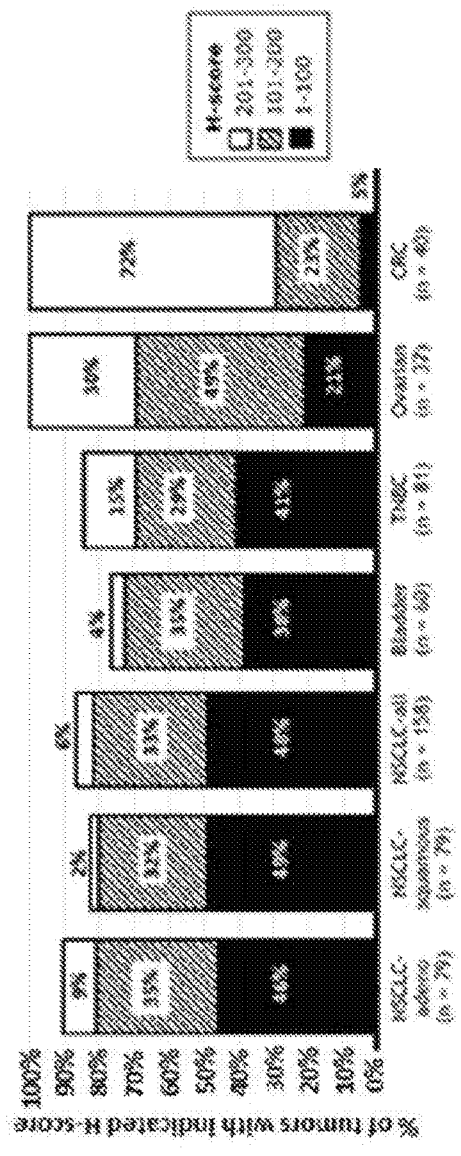
% of tumors with indicated H-score
100%
90%
80%
70%
60%
50%
40%
30%
20%
10%
0%
NSCLC-adeno (n = 79)
NSCLC-squamous (n = 79)
NSCLC-all (n = 158)
Bladder (n = 60)
TNBC (n = 81)
Ovarian (n = 37)
CRC (n = 40)
H-score
201-300
101-200
1-100

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5225539 A
- WO 9711971 A
- WO 07106585 A
- US 20070148167 A1
- WO 199711971 A
- WO 2007106585 A
- US 6277375 B
- US 7083784 B
- US 7217797 B
- US 8088376 B
- US 20020147311 A
- US 20070148164 A
- WO 199823289 A
- WO 2009058492 A
- WO 2010033279 A
- WO 2009025846 A
- WO 2010081173 A
- US 7666817 B
- US 8563269 B
- WO 2014026136 A
- WO 2016179285 A
- US 7538195 B
- US 5208020 A
- US 7276497 B
- WO 2010043880 A
- WO 2011130616 A
- WO 2009016516 A
- WO 2013177481 A
- WO 2012112708 A
- WO 2010091150 A
- WO 2012128868 A
- US 20170014522 A
- US 20080050310 A
- US 20050169933 A
- US 6913748 B
- US 6716821 B
- US 20090274713 A
- US 20100129314 A
- US 9381256 B
- US 8765740 B
- US 8426402 B
- US 9353127 B
- US 20160082114 A
- WO 2012112687 A
- US 5585089 A


### Non-patent literature cited in the description


- **BALZAR et al.** *J. Mol. Med.*, 1999, vol. 77, 699-712
- **MOMBURG et al.** *Cancer Res*, 1987, vol. 47, 2883-2891
- **WENT et al.** *Br. J. Cancer*, 2006, vol. 94, 128-35
- **HERLYN et al.** *Proc Natl. Acad. Sci. USA*, 1979, vol. 76, 1438-1442
- **WENT et al.** *Hum. Pathol.*, 2004, vol. 35, 122-128
- **PASSLICK**. *Int. J. Cancer*, 2000, vol. 87, 548-552
- **MARTIN**. *J. Clin. Pathol.*, 1999, vol. 52, 701-704
- **SZALA**. *Proc. Natl. Acad. Sci. USA*, 1990, vol. 87, 3542-3546
- **PACKEISEN**. *Hybridoma*, 1999, vol. 18, 37-40
- **O'BRIEN et al.** *Nature*, 2007, vol. 445, 106-110
- **MARHABA et al.** *Curr. Mol. Med.*, 2008, vol. 8, 784-804
- **BAEUERLE**. *Br. J. Cancer*, 2007, vol. 96, 417-423
- **KABAT et al.** Sequences of Proteins of Immunological Interest. National Institutes of Health, 1991
- **AL-LAZIKANI et al.** *J. Molec. Biol.*, 1997, vol. 273, 927-948
- **KABAT et al.** Sequences of Immunological Interest. Public Health Service, National Institutes of Health, 1991
- **KABAT et al.** Sequences of Proteins of Immunological Interest. Public Health Service, National Institutes of Health, 1991
- **CHOTHIA et al.** *J. Mol. Biol.*, 1987, vol. 196, 901-917
- **JONES et al.** *Nature*, 1986, vol. 321, 522-525
- **RIECHMANN et al.** *Nature*, 1988, vol. 332, 323-327
- **VERHOEYEN et al.** *Science*, 1988, vol. 239, 1534-1536
- **KARLIN et al.** *Proc. Natl. Acad. Sci.*, 1990, vol. 87, 2264-2268

- **KARLIN et al.** *Proc. Natl. Acad. Sci.*, 1993, vol. 90, 5873-5877
- **ALTSCHUL et al.** *Nucleic Acids Res.*, 1991, vol. 25, 3389-3402
- **ALTSCHUL et al.** *Nucleic Acids Res.*, 1997, vol. 25, 3389-3402
- **ALTSCHUL et al.** *Meth. Enzym.*, 1996, vol. 266, 460-480
- **NEEDLEMAN** ; **WUNSCH**. *J. Mol. Biol.*, 1970, vol. 48, 444-453
- **MYERS** ; **MILLER**. *CABIOS*, 1989, vol. 4, 11-17
- **SMITH** ; **WATERMAN**. *Advances in Applied Mathematics*, 1981, vol. 2, 482-489
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc. Natl. Acad. Sci. USA*, 1997, vol. 94, 412-417
- **P. WUTS** ; **T. GREENE**. Protective Groups in Organic Synthesis. J. Wiley & Sons, 2007
- **P. G.M. WUTS** ; **T. W. GREENE**. Protective Groups in Organic Synthesis. John Wiley & Sons, 2007
- Antibody-Antigen Interactions. **BERZOFSKY et al.** Fundamental Immunology. Raven Press, 1984
- **KUBY**. Janis Immunology. W. H. Freeman and Company, 1992
- **SCATCHARD et al.** *Ann. N.Y. Acad. Sci.*, 1949, vol. 51, 660-672
- **STAVENHAGEN et al.** *Cancer Res.*, 2007, vol. 57 (18), 8882-8890
- **IDUSOGIE et al.** *J. Immunol.*, 2001, vol. 166, 2571-2575
- **SAZINSKY et al.** *PNAS USA*, 2008, vol. 105, 20167-20172
- **DAVIS et al.** *J. Rheumatol.*, 2007, vol. 34, 2204-2210
- **BOLT et al.** *Eur. J. Immunol.*, 1993, vol. 23, 403-411
- **ALEGRE et al.** *Transplantation*, 1994, vol. 57, 1537-1543
- **XU et al.** *Cell Immunol.*, 2000, vol. 200, 16-26
- **COLE et al.** *Transplantation*, 1999, vol. 68, 563-571
- **HUTCHINS et al.** *PNAS USA*, 1995, vol. 92, 11980-11984
- **REDDY et al.** *J. Immunol.*, 2000, vol. 164, 1925-1933
- **MCEARCHERN et al.** *Blood*, 2007, vol. 109, 1185-1192
- **STROHL**. *Curr. Op. Biotechnol.*, 2009, vol. 20, 685-691
- **KUMAGAI et al.** *J. Clin. Pharmacol.*, 2007, vol. 47, 1489-1497
- **HAYDEN-LEDBETTER et al.** *Clin. Cancer*, 2009, vol. 15, 2739-2746
- **LAZAR et al.** *PNAS USA*, 2006, vol. 103, 4005-4010
- **BRUCKHEIMER et al.** *Neoplasia*, 2009, vol. 11, 509-517
- **BOULWARE et al.** *Biotechnol Bioeng.*, 2010, vol. 106 (3), 339-346
- **KUTMEIER et al.** *BioTechniques*, 1994, vol. 17, 242
- **ISALM** ; **DENT**. Bioconjugation. Groves Dictionaries Inc., 1999, 218-363
- **HARLOW et al.** Antibodies: A Laboratory Manual. Cold Spring Harbor Laboratory Press, 1988
- **ZOLA**. Monoclonal Antibodies: A Manual of Techniques. CRC Press, Inc., 1987, 147-158
- **WORK et al.** Laboratory Techniques and Biochemistry in Molecular Biology. North Holland Publishing Company, 1978
- **HUNTER et al.** *Nature*, 1962, vol. 144, 945
- **DAVID et al.** *Biochemistry*, 1974, vol. 13, 1014
- **PAIN et al.** *J. Immunol. Meth.*, 1981, vol. 40, 219
- **NYGREN**. *Histochem. and Cytochem.*, 1982, vol. 30, 407
- Immunochemical Techniques. Methods in Enzymology. Academic Press, vol. 121
- **E. HARLOW** ; **D. LANE**. Antibodies: A Laboratory Manual. Cold Spring Harbor Laboratory Press, 1988
- **WANG et al.** *J. Immunol. Methods*, 2000, vol. 233, 167-177
- **CO et al.** *J. Immunol.*, 1992, vol. 148, 1149-54
- **DUROCHER et al.** *Nucleic Acids Res.*, 2002, vol. 30 (2), E9
- **JONES et al.** *Nature*, 1986, vol. 321, 604-608
- **EHRENMANN et al.** *Nucleic Acids Res.*, 2010, vol. 38, D301-307
- **FOOTE** ; **WINTER**. *J. Mol. Biol.*, 1992, vol. 224, 487-499
- **VASALOU et al.** *PLoS ONE*, 2015, vol. 10 (3), e0118977